# EUROPEAN PATENT APPLICATION

(11) **EP 3 978 515 A1**
(43) Date of publication of application: **06.04.2022**
(21) Application number: 20199856.4
(22) Date of filing: 02.10.2020
(51) Int. Cl.: C07K 14/34, C07K 14/195, C12N 1/20, C12N 15/09, C12P 1/04

(54) **GENETICALLY MODIFIED METHYLOBACILLUS BACTERIA HAVING IMPROVING PROPERTIES**

(71) Applicant: Acies Bio d.o.o., 1000 Ljubljana (SI)
(72) Inventor: VIRANT, David, 1233 Dob (SI); KRUIS, Aleksander Johannes, 4260 Bled (SI); NAGODE, Toni, 1000 Ljubljana (SI); HORVAT, Jaka, 4000 Kranj (SI); FUJS, Stefan, 1000 Ljubljana (SI)
(74) Representative: Zacco GmbH

(57) **Abstract**

The present invention generally relates to the biotechnology engineering, and specifically to genetically modified bacteria of the genus *Methylobacillus* which have improved properties making them particularly useful in large scale methanol fermentations. More specifically, the present invention provides a bacterium of the genus *Methylobacillus* which has been modified to have a decreased production of exopolysaccharides (EPS) compared to an otherwise identical bacterium that does not carry said modification. The present invention further provides a method for producing a biochemical compound using a genetically modified bacterium of the present invention.

SEQUENCE LISTING

<110>  Acies Bio d.o.o.

<120>  Genetically modified Methylobacillus bacteria having improving
       properties

<130>  P49906EP00

<160>  184

<170>  PatentIn version 3.5

<210>  1
<211>  257
<212>  PRT
<213>  Methylobacillus flagellatus

<400>  1

Met Ser Leu Asn Lys Asn Leu Asn Asp Ile Glu Lys Ser Gln Leu Ile
1               5                   10                  15

Glu Leu Met His Glu Ser Leu Arg Ile Arg Ser His Phe Glu Leu Phe
            20                  25                  30

Leu Trp Met Gln Gly Lys Leu Gln Val Phe Leu Pro His Glu Ile Met
        35                  40                  45

Ile Thr Ala Trp Gly Asp Phe Ser Leu Gly Val Ile Tyr Phe Asn Ile
    50                  55                  60

Val Ser Pro Leu Pro Gly Ile Arg Ser Asp Lys Ile Ala Ser His Glu
65                  70                  75                  80

Ile Asn Gln Phe Leu Gln Asn Leu Phe Asn Tyr Trp Leu Asn His Ala
                85                  90                  95

Lys Ala Pro Phe Thr Leu Ala Ala Ala Asp Asn Ile Leu His Asp Cys
            100                 105                 110

Glu Phe Ser Asn Lys Glu Ser Ser Tyr Tyr Leu Lys Asn Met Lys Ser
        115                 120                 125

Ala Leu Val His Ala Ile Lys Asp His Arg Gly Arg His Asp Cys Leu
    130                 135                 140

Tyr Val Leu Leu Ser Ser Asn Leu Ser Phe Pro Pro Ala Ser Arg Pro
145                 150                 155                 160

Met Leu Glu Ala Leu Leu Pro Tyr Ile Asp Ser Ala Leu Arg Gln Ile
                165                 170                 175

Glu His Leu Pro Glu Gln Gln Pro Thr Leu Ile Lys Ala Glu Pro Glu
            180                 185                 190

Pro Lys Asp Glu Ser Leu Asn Ile Leu Ser Gly Arg Glu Leu Glu Ile
        195                 200                 205

Met Asp Trp Val Thr Lys Gly Lys Thr Asn Gln Glu Ile Gly Met Ile
    210                 215                 220

Leu Asp Ile Ser Ala Phe Thr Val Lys Asn His Leu Gln Arg Ile Phe
225                 230                 235                 240

Lys Lys Leu Asn Val Met Asn Arg Ala Gln Ala Val Ala Lys Phe Arg
                245                 250                 255

Lys

<210> 2
<211> 168
<212> PRT
<213> Methylobacillus flagellatus

<400> 2

Met Tyr Pro Asp Leu Val His Leu Gly Gly Ala Asp Lys Tyr Phe Glu
1               5                   10                  15

Glu Ile Leu Glu Ile Val Asn Lys Ile Lys Leu Phe Gly Asp Phe Ser
            20                  25                  30

Asn Glu Glu Val Arg Tyr Leu Cys Ser Tyr Met Gln Cys Tyr Ala Ala
        35                  40                  45

Pro Arg Asp Cys Gln Leu Leu Thr Glu Gly Asp Pro Gly Asp Tyr Leu
    50                  55                  60

Leu Leu Ile Leu Thr Gly Glu Val Asn Val Ile Lys Asp Ile Pro Asn
65                  70                  75                  80

Lys Gly Ile Gln Thr Ile Ala Lys Val Gly Ala Gly Ala Ile Ile Gly
                85                  90                  95

Glu Met Ser Met Ile Asp Gly Met Pro Arg Ser Ala Ser Cys Val Ala
            100                 105                 110

Ser Leu Pro Thr Asp Phe Ala Val Leu Ser Arg Asp Ala Leu Tyr Gln
        115                 120                 125

Leu Leu Ala Asn Met Pro Lys Leu Gly Asn Lys Val Leu Ile Arg Leu
    130                 135                 140

Leu Gln Leu Leu Thr Ala Arg Phe Arg Glu Ser Tyr Asp Arg Ile Leu
145                 150                 155                 160

Pro Lys Thr Leu Gly Glu Leu Ile
                165

<210>  3
<211>  225
<212>  PRT
<213>  Methylobacillus flagellatus

<400>  3

Met Phe Leu Ser Arg Leu His Thr Pro Ile Leu Ser Arg Val Ala Pro
1               5                   10                  15

Phe Ala Leu Tyr Met Leu Phe Met Ala Val Val Asp Leu Val Val Arg
            20                  25                  30

Ile Gly Leu Val Asp Ala Asp Thr Arg Trp Leu Tyr Gly Ile Lys Val
        35                  40                  45

Gly Leu Val Ala Leu Leu Leu Trp Tyr Leu Arg Lys Gln Tyr Val Glu
    50                  55                  60

Leu Gln Gly Leu Ala Ala Leu Gly Ala Arg Ser Trp Leu Ser Ala Ile
65                  70                  75                  80

Ala Val Gly Met Leu Val Phe Ile Ala Trp Ile Asn Leu Asp Ala Pro
                85                  90                  95

Trp Met Leu Ile Gly Asp Gln Ser Glu Gly Phe Asp Pro Gly Ser Gly
            100                 105                 110

Pro Asp Ala Arg Leu Leu Met Ala Thr Arg Val Met Gly Ala Ala Leu
        115                 120                 125

Val Val Pro Leu Met Glu Glu Leu Phe Trp Arg Ser Phe Leu Met Arg
    130                 135                 140

Trp Ile His Asn Gln His Phe Leu Asn Val Asp Pro Arg Gln Val Ser
145                 150                 155                 160

Val Leu Ala Leu Leu Val Ser Ser Ala Leu Phe Ala Ile Glu His Thr
                165                 170                 175

Glu Trp Phe Ala Gly Leu Val Ala Gly Leu Ala Tyr Gly Leu Leu Tyr
            180                 185                 190

Ile Arg Ser Gly Lys Ile Trp Leu Pro Ile Ile Ala His Ala Val Thr
        195                 200                 205

Asn Gly Ile Leu Gly Leu Trp Ile Ile Tyr Thr Gly Asn Trp His Tyr
    210                 215                 220

Trp
225

<210>  4
<211>  410
<212>  PRT
<213>  Methylobacillus flagellatus

<400>  4

Met Arg Leu Ser Gln Ser Thr Asn Leu Ile Lys Ile Ala Leu Val Ala
1               5                   10                  15

Gly Leu Gly Ile Glu Leu Ala Gly Phe Val Val Pro Ala Cys Ala Leu
            20                  25                  30

Glu Gly Asp Val Leu Lys Pro Tyr Ala Ser Leu Thr Tyr Ser Tyr Asp
        35                  40                  45

Asp Asn Leu Arg Arg Phe Ala Ser Lys Gln Leu Ala Gln Met Ser Thr
    50                  55                  60

Gly Ser Ser Asp Thr Ser Asp Thr Met Leu Val Lys Ser Ala Gly Ile
65                  70                  75                  80

Val Leu Asp His Thr Ile Ser Arg Gln Lys Met Tyr Val Asp Phe Arg
                85                  90                  95

Leu Asn Lys Ser Thr Phe Asn Lys Asn Ser Glu Leu Asp Asn Asp Gly
            100                 105                 110

Arg Asp Val Ile Ala Arg Trp Asp Trp Glu Leu Gly Asn Arg Leu Glu
        115                 120                 125

Gly Lys Val Glu Phe Phe His Lys Lys Ala Leu Val Pro Phe Ser Glu
    130                 135                 140

Phe Gln Pro Leu Gln Gly Thr Met Leu Ser Leu Asn Thr Arg Thr Gln
145                 150                 155                 160

Asp Arg Arg Leu Leu Glu Ala Arg Trp Met Leu His Pro Arg Trp Arg
                165                 170                 175

Val Arg Gly Ala Phe Ile Tyr Ser Glu Met Glu Tyr Gly Ala Glu Ala
            180                 185                 190

Gln Arg Ala Ala Asn Leu Lys Glu Asp Ile Gln Glu Ile Gly Phe Asp
        195                 200                 205

Tyr Leu Ser Pro Thr Lys Ser Ile Ile Gly Val Leu Tyr Arg Asn Thr
    210                 215                 220

Lys Gly Ser Lys Pro Gln Gln Ala Phe Gln Gly Ser Phe Ile Ser Asn
225                 230                 235                 240

Asp Tyr Asp Gln Asn Glu Leu Lys Leu Asn Val Asp Trp Ala Val Thr
                245                 250                 255

Gly Lys Ser Arg Leu Gln Phe Leu Gly Gly Leu Val Asp Arg Gln His
            260                 265                 270

Glu Gln Leu Ser Gln Arg Asp Phe Arg Gly Val Asn Ala Arg Gly Asn
        275                 280                 285

Phe Asn Trp Met Thr Ser Gly Lys Thr Asn Leu Asn Ala Ala Val Trp
    290                 295                 300

Arg Glu Asn Asn Ala Gln Ser Phe Val Thr Thr Ser Tyr Thr Leu Asn
305                 310                 315                 320

Arg Gly Thr Ser Leu Thr Ala Asn Leu Gln Ala Thr Gly Lys Ile Ala
                325                 330                 335

Val Gln Gly Val Leu Lys Tyr Glu Lys Arg Asp Phe Glu Gly Asp Gln
            340                 345                 350

Ile Phe Gly Gln Phe Arg Ser Asp Lys Asp Lys Ser Phe Met Leu Ser
        355                 360                 365

Leu Met Tyr Asn Pro Val Thr Ser Phe Arg Ile Thr Thr Ser Phe Ile
    370                 375                 380

His Ser Asp Arg Glu Ser Thr Ser Asp Met Phe Glu Phe Arg Ser Asn
385                 390                 395                 400

Ser Val Ser Val Thr Gly Gln Tyr Glu Phe
                405                 410

<210>  5
<211>  461
<212>  PRT
<213>  Methylobacillus flagellatus

<400>  5

Met Thr Val Asn Asp Ile Pro Leu Val Ala Phe Phe Lys Gln Leu Leu
1               5                   10                  15

Asp Pro Leu Ile Ile Leu Gly Thr Leu Tyr Gly Phe Thr Leu Leu Tyr
            20                  25                  30

Gly Glu Pro Phe Asn Gly Tyr Tyr Leu Ile Leu Ala Leu Leu Ala Tyr
        35                  40                  45

Phe Val Ser Ser Asn Val Tyr Glu Arg Leu Asp Leu Tyr Arg Thr Trp
    50                  55                  60

Arg Ser Gly Lys Val Leu Gly Tyr Ile Arg Asp Ile Leu Phe Gly Trp
65                  70                  75                  80

Ala Ile Val Val Thr Ile Leu Ala Phe Ile Gly Tyr Ala Ala Asp Leu
                85                  90                  95

Thr Asp Gln Tyr His Glu Glu Met Val Tyr Thr Trp Phe Val Ala Val
            100                 105                 110

Pro Ile Ile Phe Thr Val Ala His Leu Leu Ile Arg Lys Ile Ala Ser
        115                 120                 125

Asp Leu His Lys Asn Gly Glu Val Arg Ser Val Val Ile Val Gly Ala
    130                 135                 140

Asn Asp Ile Gly Val Arg Leu Val Asn Arg Ile Asn Glu Asp Pro Tyr
145                 150                 155                 160

Leu Phe Met Glu Val Lys Gly Leu Phe Asp Asp Arg Glu Ile Ser Arg
                165                 170                 175

His Pro Lys Gly Met Arg His Val Pro Leu Leu Gly Thr Met Ala Asp
            180                 185                 190

Val Ala Ala Tyr Val Arg Ala Asn Asn Thr Gln Ile Ile Phe Ile Ser
        195                 200                 205

Leu Pro Met Ser Ala Gln Pro Arg Ile Arg Gln Leu Phe Glu Glu Leu
    210                 215                 220

Gln Asp Thr Thr Ala Ser Val Tyr Phe Val Pro Asp Ile Tyr Ile Phe
225                 230                 235                 240

Asp Leu Val Gln Ala Arg Phe Asp Asp Val Ala Gly Val Pro Val Val
                245                 250                 255

Ala Ile Cys Glu Thr Pro Phe Thr Gly Phe Asn Ser Leu Ile Lys Arg
            260                 265                 270

Thr Ser Asp Ile Ile Leu Ala Ser Leu Ile Leu Leu Leu Leu Ser Pro
        275                 280                 285

Ile Met Leu Thr Leu Ala Leu Ala Val Lys Leu Thr Ser Lys Gly Pro
    290                 295                 300

Val Ile Phe Lys Gln Arg Arg Tyr Gly Leu Asn Gly Glu Glu Ile Ile
305                 310                 315                 320

Val Tyr Lys Phe Arg Ser Met Thr Val Met Glu Asp Gly Asp Lys Val
                325                 330                 335

Val Gln Ala Gln Lys Arg Asp Gln Arg Leu Thr Pro Ile Gly Gly Phe
            340                 345                 350

Leu Arg Arg Asn Ser Leu Asp Glu Leu Pro Gln Phe Ile Asn Val Leu
        355                 360                 365

Gln Gly Arg Met Ser Ile Val Gly Pro Arg Pro His Ala Val Ala His
    370                 375                 380

Asn Glu Gln Tyr Arg Lys Leu Ile Arg Gly Tyr Met Leu Arg His Lys
385                 390                 395                 400

Val Lys Pro Gly Ile Thr Gly Trp Ala Gln Val Asn Gly Leu Arg Gly
                405                 410                 415

Glu Thr Asp Thr Leu Glu Lys Met Glu Ala Arg Ile Glu Tyr Asp Leu
            420                 425                 430

Asp Tyr Leu Arg His Trp Ser Leu Ala Phe Asp Leu Trp Ile Ile Ile
        435                 440                 445

Arg Thr Val Leu Val Val Phe Lys Lys Asp Asn Ala Tyr
    450                 455                 460

<210>  6

<211>  314
<212>  PRT
<213>  Methylobacillus flagellatus

<400>  6

Met Asn Thr Glu Tyr Gly Ser Thr Ile Met Gln Trp Ser Lys Met Thr
1               5                   10                  15

Lys Gly Ala Leu Val Ala Leu Met Ile Ser Ile Gly Leu Ser Gly Cys
            20                  25                  30

Gly Asp Lys Ser Asn Glu Thr Lys Ala Ser Gln Ser Leu Ala Arg Val
        35                  40                  45

Asn Gly Lys Glu Ile Thr Val His Gln Leu Asn Glu Glu Leu Ala Arg
    50                  55                  60

Ala Asn Val Gln Pro Ala Gln Lys Glu Ala Ala Thr Lys Gln Ile Leu
65                  70                  75                  80

Gln Ser Leu Ile Asp Arg Gln Leu Leu Glu Gln Ala Ala Leu Lys Ala
                85                  90                  95

Lys Val Asp Arg Asp Pro Asn Val Met Gln Ala Ile Glu Arg Ala Lys
            100                 105                 110

Ser Gln Met Ile Ala Gln Ala Tyr Leu Gln Ser Arg Val Ala Asn Ile
        115                 120                 125

Gly Lys Pro Asp Lys Thr Glu Val Glu Asn Phe Tyr Lys Gln Asn Pro
    130                 135                 140

Gln Leu Phe Ser Glu Arg Lys Leu Val Glu Met Glu Gln Ile Leu Ile
145                 150                 155                 160

Asp Ser Arg Tyr Val Asn Asp Glu Phe Lys Ser Ala Leu Gly Ser Phe
                165                 170                 175

Lys Thr Leu Gln Glu Val Ala Ala Trp Leu Asp Ala Lys Gly Ile Gln
            180                 185                 190

Tyr Asp Arg Gly Glu Val Ala Arg Ser Val Ala Glu Leu Pro Pro Gln
        195                 200                 205

Ile Ser Glu Arg Leu Lys Asp Ala Arg Arg Gly Gln Leu Phe Val Ile
    210                 215                 220

Gly Met Gly Pro Arg Thr Met Leu Val Leu Met Asp Ser Val Lys Glu

225                 230                 235                 240

Ser Pro Val Ser Leu Ala Asp Ala Thr Pro Gln Ile Glu Gln Met Leu
                245                 250                 255

Leu Asn Lys Lys Arg Lys Glu Ala Gly Glu Ala Glu Ile Lys Arg Leu
            260                 265                 270

Arg Ala Glu Ala Lys Ile Glu Tyr Leu Asp Lys Ser Ala Glu Val Pro
        275                 280                 285

Gln Gln Pro Glu Lys Ala Glu Gln Ser Lys Pro Ser Ser Val Asp Asp
    290                 295                 300

Asp Ile Ala Arg Gly Val Ser Gly Leu Lys
305                 310

<210>  7
<211>  270
<212>  PRT
<213>  Methylobacillus flagellatus

<400>  7

Met Tyr Arg Cys Ser Ala Met Thr Lys Leu Ile Lys Trp Phe Val Thr
1               5                   10                  15

Val Val Leu Thr Ile Val Val Phe Asn Val Asn Ala Ala Asp Ser Asp
            20                  25                  30

Ile Val Leu Gly Pro Gly Asp Val Leu Arg Ile Ser Val Phe Glu Gln
        35                  40                  45

Pro Asp Leu Ser Leu Glu Val Arg Val Ser Glu Ser Gly Thr Ile Thr
    50                  55                  60

Tyr Pro Leu Ile Gly Glu Val Arg Val Gly Gly Glu Thr Pro Ala Ala
65                  70                  75                  80

Ala Glu Arg Lys Ile Ala Ser Met Leu Glu Ser Gly Gly Phe Leu Lys
                85                  90                  95

Asn Pro His Val Asn Ile Ile Val Ala Gln Leu Gln Ser Gln Gln Val
            100                 105                 110

Ser Val Leu Gly Gln Val Asn Arg Pro Gly Arg Tyr Pro Leu Asp Ser
        115                 120                 125

Ala Lys Ser Leu Ala Asp Val Leu Ala Leu Ala Gly Gly Val Ala Pro

130                 135                 140

Asp Gly Gly Asp Leu Ile Ser Leu Val Arg Lys Ile Glu Gly Lys Ser
145                 150                 155                 160

Val Arg Glu Ile Ile Asp Phe Ala Glu Met Met Arg Asp Gly Asp Leu
                165                 170                 175

Asn Lys Asn Ile Leu Leu Thr Ala Gly Asp Ile Ile Tyr Val Asp Arg
            180                 185                 190

Ala Pro Arg Phe Tyr Ile Tyr Gly Glu Val Gln Arg Pro Gly Gln Tyr
        195                 200                 205

Arg Leu Glu Tyr His Met Thr Val Leu Gln Ala Leu Ala Val Gly Gly
    210                 215                 220

Gly Leu Ser Leu Arg Gly Thr Glu Lys Gly Ile Lys Val Lys Arg Leu
225                 230                 235                 240

Asp Asp Glu Gly Gly Val Lys Ile Ile Lys Val Lys His Asp Asp Phe
                245                 250                 255

Ile Gln Pro Asn Asp Val Val Tyr Val Gln Glu Ser Val Phe
            260                 265                 270

<210>  8
<211>  469
<212>  PRT
<213>  Methylobacillus flagellatus

<400>  8

Met Ser Phe Ile Arg Phe Ile Ser Ile Ile Lys Ala Arg Tyr Lys Ile
1               5                   10                  15

Ile Ile Phe Thr Leu Phe Ile Thr Val Leu Thr Thr Ala Ile Val Thr
            20                  25                  30

Ile Leu Leu Pro Lys Asn Tyr Lys Ala Thr Ala Ser Val Leu Val Asn
        35                  40                  45

Tyr Lys Gly Ala Asp Pro Val Thr Gly Val Met Leu Pro Ala Gln Leu
    50                  55                  60

Met Pro Gly Tyr Met Ala Thr Gln Val Asp Ile Ile Ser Ser Lys Asn
65                  70                  75                  80

Val Ala Lys Lys Val Ile Asp Lys Leu Gly Leu Ala Asp Val Pro Ala

                85                  90                  95

Val Lys Glu Asp Phe Tyr Gln Ala Thr Ser Gly Lys Gly Glu Ile Lys
            100                 105                 110

Glu Trp Leu Ala Asp Leu Leu Leu Lys Lys Leu Gln Val Lys Pro Ser
        115                 120                 125

Arg Glu Ser Ser Ile Ile Asp Ile Ser Phe Phe Gly Val Asn Pro Gln
    130                 135                 140

Phe Ala Ala Thr Ile Ala Asn Glu Phe Ala Glu Ser Tyr Met Gln Thr
145                 150                 155                 160

Ser Ile Gln Leu Lys Val Glu Pro Ser Gln Lys Ala Ala Leu Tyr Phe
                165                 170                 175

Val Glu Gln Leu Lys Glu Leu Arg Glu Asp Leu Ala Gln Ala Gln Ser
            180                 185                 190

Lys Leu Ser Glu Tyr Gln Gln Glu Lys Gly Ile Val Ser Val Asp Glu
        195                 200                 205

Arg Leu Asp Val Glu Arg Ala Arg Leu Asn Glu Leu Ser Ser Gln Leu
    210                 215                 220

Val Ile Ala Gln Ala Gln Val Met Glu Ala Arg Ser Arg Glu Lys Asn
225                 230                 235                 240

Ser Ser Gly Lys Glu Ala Tyr Glu Ser Pro Asp Val Ala Ala Asn Pro
                245                 250                 255

Ile Ile Gln Asn Leu Lys Thr Glu Ile Ala Arg Ala Glu Ser Arg Phe
            260                 265                 270

Ala Asp Val Ser Gln Lys Phe Glu Arg Asn His Pro Phe Tyr Leu Gly
        275                 280                 285

Ala Lys Ala Glu Ile Glu Asn Leu Lys Ser Glu Leu Asp Arg Gln Ile
    290                 295                 300

Lys Ser Val Ser Ser Asn Val Ala Ser Asn Ser Arg Ile Leu Gln Gln
305                 310                 315                 320

Arg Glu Ala Glu Ile Arg Leu Ala Leu Asn Asn Gln Lys Glu Lys Val
                325                 330                 335

Leu Gln Leu Asn Arg Asp Arg Asp Ile Leu Ser Val Leu Thr Arg Glu
            340                 345                 350

Val Glu Asn Ala Gln Lys Ala Tyr Asp Met Ala Met Gln Arg Tyr Thr
        355                 360                 365

Gln Thr Asn Ile Glu Gly Gln Ser Asn Gln Ser Asp Ile Ser Leu Leu
    370                 375                 380

Asn Pro Ala Val Ala Pro Leu Thr Pro Thr Ser Pro Asn Leu Leu Leu
385                 390                 395                 400

Asn Ile Ile Leu Ser Ile Leu Leu Gly Met Phe Leu Gly Ile Gly Phe
                405                 410                 415

Gly Val Leu Ala Glu Phe Val Asp Arg Arg Val Arg Thr Ala Asp Asp
            420                 425                 430

Leu Ile Glu Val Leu Gln Ala Pro Leu Leu Gly Thr Ile Ser Lys Glu
        435                 440                 445

Lys Arg Lys Leu Arg Phe Phe Lys Asn Thr Ser Asn Leu Ile Thr Leu
    450                 455                 460

Gly Lys Ala Glu Leu
465

<210>  9
<211>  298
<212>  PRT
<213>  Methylobacillus flagellatus

<400>  9

Met Ile Ser Phe Thr Pro Ser Asn Ser Asn Ser Glu Lys Val Glu Asn
1               5                   10                  15

Ala Pro Leu Val Asn Glu Phe Ser Glu Phe Thr Ile Gly Ser Leu Leu
            20                  25                  30

Leu Arg Met Gly Lys Ile Thr Pro Glu Asp Ala Glu Arg Ile Met Arg
        35                  40                  45

Leu His Lys Glu Lys Gly Leu Arg Phe Gly Glu Ala Ala Gln Ala Leu
    50                  55                  60

Gly Leu Ile Thr Glu Asp Asp Ile Gln Gln Val Leu Ser Arg Gln Phe
65                  70                  75                  80

Asn Tyr Pro Tyr Leu Ser Pro Asp Gln Asn Ser Tyr Leu Asn Glu Leu
                85                  90                  95

Val Ala Ala Tyr Gln Pro Phe Ser Asn Lys Ala Glu Val Leu Arg Ala
            100                 105                 110

Val Arg Ser Gln Leu Met Leu His Trp Phe Ser Pro Glu Arg Lys Ala
        115                 120                 125

Leu Ala Val Ser Ser Val Ser Ser Ser Asp Asn Ala Ser Ser Phe Val
    130                 135                 140

Ala Asn Leu Ala Val Val Phe Ala Gln Leu Gly Glu Asn Thr Leu Leu
145                 150                 155                 160

Ile Asp Ala Asn Leu Arg Tyr Pro Arg Gln His Glu Ile Tyr Asn Leu
                165                 170                 175

Ser Asn Lys Arg Gly Leu Ser Asp Ile Leu Ala Gly Arg Ala Gly Tyr
            180                 185                 190

Asp Val Ile Ser Lys Ile Asn Phe Phe Glu Asn Leu Ser Val Leu Thr
        195                 200                 205

Ala Gly Thr Leu Pro Pro Asn Pro Gln Glu Leu Val Ser Lys Pro Thr
    210                 215                 220

Phe Gly Asn Leu Ile Lys Glu Leu Ala Cys Val Tyr Asp Val Ile Leu
225                 230                 235                 240

Leu Asp Thr Pro Glu Leu Ser Asn Tyr Ser Asp Gly Tyr Ala Ile Ala
                245                 250                 255

Phe Thr Val Gly Gly Ile Val Leu Val Ala Arg Gln Asn His Thr Arg
            260                 265                 270

Val Asn Asp Leu Ser Ile Ala Val Glu Gln Cys Lys Asn Asn Gly Thr
        275                 280                 285

Lys Val Ile Gly Ser Ile Leu Val Asp Leu
    290                 295

<210>  10
<211>  295
<212>  PRT
<213>  Methylobacillus flagellatus

<400>  10

Met Asn Thr Val Glu Val Ile Glu Gln Asp Asn Pro Gly Ile Trp Glu
1               5                   10                  15

Val Ile Asn Arg Asn Trp Met Leu Leu Leu Gly Leu Phe Ile Met Tyr
            20                  25                  30

Thr Pro Thr Tyr Leu Asn Leu Leu Asn Thr Val Trp Gln Ser Glu Asp
        35                  40                  45

Gln Ala His Gly Pro Leu Ile Val Gly Val Val Leu Tyr Leu Val Trp
    50                  55                  60

Val Gln Arg His Gln Leu Phe Lys Ala Glu Asn Asn Ile Ile Gly Arg
65                  70                  75                  80

Pro Val Ile Gly Gly Ile Val Leu Val Phe Gly Leu Leu Leu Tyr Ile
                85                  90                  95

Leu Gly Arg Ser Gln Asp Ile Ala Pro Leu Asp Ile Ala Ser Gln Ile
            100                 105                 110

Pro Val Phe Ala Gly Leu Leu Leu Ile Phe Arg Gly Tyr Ser Gly Leu
        115                 120                 125

Arg Ala Leu Trp Phe Pro Leu Phe Phe Leu Ile Phe Met Val Pro Leu
    130                 135                 140

Pro Gly Val Phe Leu Asp Ala Val Thr Leu Pro Met Lys Ile Ala Val
145                 150                 155                 160

Ser Tyr Val Ala Glu Asn Ile Leu Tyr Trp Ile Gly Tyr Pro Ile Ser
                165                 170                 175

Arg Ser Gly Val Ile Leu Gln Ile Gly Gln Tyr Gln Leu Leu Val Ala
            180                 185                 190

Asp Ala Cys Ala Gly Leu His Thr Leu Ile Ser Leu Glu Ala Leu Gly
        195                 200                 205

Leu Leu Tyr Leu Asn Val Val Lys His Asp Ser Phe Ile Arg Asn Val
    210                 215                 220

Ile Leu Gly Ile Leu Ile Ile Pro Ile Ser Phe Thr Ala Asn Val Ile
225                 230                 235                 240

Arg Val Met Val Leu Thr Leu Val Thr Tyr His Phe Gly Asp Ala Ala
                245                 250                 255

Gly Gln Gly Phe Val His Gly Phe Ala Gly Met Leu Leu Phe Val Val
            260                 265                 270

Ala Leu Ile Leu Ile Ile Ser Val Asp Thr Leu Leu Gln Tyr Leu Val
        275                 280                 285

Lys Lys Trp Gln Leu Lys Ala
    290                 295

<210>  11
<211>  226
<212>  PRT
<213>  Methylobacillus flagellatus

<400>  11

Met Asp Gly Phe Thr Leu Arg Asn Leu Ile Ile Phe Leu Leu Leu Leu
1               5                   10                  15

Val Ala Thr Leu Gly Gly Val Ile Leu Lys Pro Thr Lys Phe Val Asn
            20                  25                  30

Ala Ala Glu Asp Ile Asn Leu Glu Ala Met Ile Pro Lys Glu Phe Asp
        35                  40                  45

Glu Trp Lys Ile Asp Pro Thr Ile Thr Pro Ile Gln Val Ser Pro Glu
    50                  55                  60

Val Gln Gln Asn Leu Asn Lys Val Tyr Asp Gln Thr Leu Ser Arg Thr
65                  70                  75                  80

Tyr Ile Asn Lys Gln Gly Met Arg Ile Met Leu Ser Ile Ala Tyr Gly
                85                  90                  95

Gly Asp Gln Ser Arg Gly Leu Gln Ala His Lys Pro Glu Thr Cys Tyr
            100                 105                 110

Thr Ala Gln Gly Phe Asn Val Asp Leu Leu Gly Asn Ala Ile Leu Asn
        115                 120                 125

Phe Asp Gly Tyr Glu Ile Lys Val Arg His Leu Leu Ala Gln His Gly
    130                 135                 140

Ala Arg Tyr Glu Pro Ile Leu Tyr Trp Met Arg Val Gly Glu Thr Thr
145                 150                 155                 160

Val Tyr Gly Gly Leu Gly Gln Thr Ile Lys Arg Phe Glu Leu Gly Leu
                165                 170                 175

Gln Gly Tyr Ile Pro Asp Gly Leu Leu Phe Arg Val Ser Ser Ile Ser
            180                 185                 190

Arg Asp Asn Ile Ser Ala Phe Thr Leu Gln Glu Glu Phe Val Lys Ser
        195                 200                 205

Leu Leu Lys Asn Val Asp Ser Lys Ser Arg Lys Lys Leu Ile Gly Thr
    210                 215                 220

Phe Ser
225

<210>  12
<211>  428
<212>  PRT
<213>  Methylobacillus flagellatus

<400>  12

Met Ile Lys His Thr Ala Phe Met Ala Leu Ser Thr Phe Ser Arg Leu
1               5                   10                  15

Leu Ser Ala Val Val Leu Phe Ile Ile Leu Ala Arg Phe Leu Gly Pro
            20                  25                  30

Arg Asp Phe Gly Ile Leu Met Tyr Asn Phe Thr Leu Ala Ser Ile Ala
        35                  40                  45

Val Leu Leu Val Glu Tyr Gly Phe Ser Asn Gln Leu Leu Arg Asp Ile
    50                  55                  60

Gly Arg Tyr Pro Asp Asn Ile Asn Gln Leu Met Gly Arg Val Phe Leu
65                  70                  75                  80

Ala Lys Leu Tyr Leu Thr Val Val Met Gly Val Cys Cys Leu Ala Gly
                85                  90                  95

His Tyr Leu Phe Tyr Thr Asn Asp Leu Asp Arg Ala Ile Phe Trp Ile
            100                 105                 110

Leu Leu Leu Ser Cys Leu Leu Thr Ser Phe Gly Asp Phe Phe Asn Ile
        115                 120                 125

Ala Phe Arg Gly Ile Gly Lys Phe His Glu Glu Thr Lys Val Ala Thr
    130                 135                 140

Ile Gly Ser Leu Leu His Phe Gly Leu Ile Val Leu Met Val Ile Asn
145                 150                 155                 160

Gly Ala Glu Leu Leu Leu Ile Ser Leu Gly Phe Val Leu Ser Arg Phe
                165                 170                 175

Val Tyr Phe Ile Leu Ser Trp Arg Ala Tyr Lys Arg Val Val Gly Asp
            180                 185                 190

Leu Asn Thr Leu Ser Ala Ser Pro Val Ala Val Leu Lys Ala Leu Lys
        195                 200                 205

Ala Gly Phe Pro Tyr Ala Val Asp Ala Gly Phe Thr Asn Phe Phe Tyr
    210                 215                 220

Gln Val Asp Thr Met Leu Val Lys Tyr Tyr Leu Gly Ala Thr Gly Val
225                 230                 235                 240

Gly Ile Tyr Gln Ala Gly Met Arg Phe Leu Asn Gly Ala Met Gln Phe
                245                 250                 255

Ala Pro Val Leu Gly Asn Val Phe Leu Pro Val Ile Ala Ala Lys His
            260                 265                 270

Thr Asn Thr Arg Glu Phe Val Glu Leu Ala Lys Lys Leu Asn Leu Gln
        275                 280                 285

Met Leu Ala Val Gly Thr Val Cys Trp Ala Ile Phe Trp Phe Ala Gly
    290                 295                 300

Met His Ile Thr Glu Phe Val Phe Gly Asp Glu Tyr Val Glu Leu Asn
305                 310                 315                 320

Ser Ile Trp Pro Tyr Ile Gly Leu Leu Val Phe Thr Arg Tyr Leu Ala
                325                 330                 335

Ala Pro Gln Gly Val Leu Leu Thr Ala Ser Gly Ser Gln Thr Ile Arg
            340                 345                 350

Val Trp Ala Gln Ile Phe Ala Leu Ile Ile Leu Leu Ile Ser Ala Pro
        355                 360                 365

Leu Leu Met Lys Val Ile Gly Leu Lys Gly Met Val Ile Ala Leu Ile
    370                 375                 380

Ile Thr Leu Leu Thr Leu Phe Leu Ile Phe Phe Ser Ala Leu Arg Leu
385                 390                 395                 400

Arg Lys Ile Pro Thr Gly Phe Gly Gly Val Asn Phe Thr Leu Thr Val
                405                 410                 415

Ile Val Phe Ser Leu Ser Ile Phe Leu Leu Val Tyr
                420                 425

<210>  13
<211>  392
<212>  PRT
<213>  Methylobacillus flagellatus

<400>  13

Met Leu Lys Ile Gly Leu Leu Trp His Ser Thr Thr Ser Asp Asn Leu
1               5                   10                  15

Gly Val Gly Ala Leu Thr Glu Ala Gln Ile Ser Ile Ile Asn Lys Ala
            20                  25                  30

Ala Val Gln Ala Gly Val Glu Val Glu Tyr Arg Ile Phe Gly Thr Asn
        35                  40                  45

Gly Asn Lys Asp Tyr Phe Glu Gly Asn Pro Leu Ile Glu Arg Gly Thr
    50                  55                  60

Asn Val Ser Leu Lys Arg Ile Leu Ile Gly Ala Ser Asp Tyr Pro Lys
65                  70                  75                  80

Lys Ile Arg Glu Cys Asp Val Val Phe Asp Ile Gly Glu Gly Asp Ser
                85                  90                  95

Phe Ala Asp Ile Tyr Gly Phe Ser Arg Phe Leu Ile Gln Ile Val Ser
            100                 105                 110

Lys Leu Val Val Leu Phe Gln Arg Lys Pro Leu Ile Leu Ser Pro Gln
        115                 120                 125

Thr Ile Gly Pro Phe Glu Ser Arg Phe Asn Leu Lys Val Ala Asn Asn
    130                 135                 140

Val Met Gln Arg Cys Thr Lys Ile Phe Val Arg Asp Gly Leu Ser Phe
145                 150                 155                 160

Gly Tyr Leu Gln Lys Asn Asn Leu Ser Val Asn Ala Glu Glu Val Ile
                165                 170                 175

Asp Val Ala Phe Arg Leu Pro Phe Gln Arg Pro Ser Arg Asp Ser Ser
            180                 185                 190

Lys Val Lys Ile Gly Leu Asn Val Ser Gly Leu Leu Tyr Asn Gly Gly
        195                 200                 205

Tyr Thr Gly Lys Asn Gln Phe Gly Leu Ser Val Asn Tyr Pro Glu Leu
    210                 215                 220

Thr Arg Ser Leu Leu Asp Gln Phe Thr Arg Ile Glu Gly Cys Glu Val
225                 230                 235                 240

Trp Leu Val Pro His Val Leu Thr Thr Ser Phe Pro Ile Glu Asp Asp
                245                 250                 255

Val Asn Val Cys Val Gln Leu Gln Glu Leu Tyr Pro Ala Ala Lys Leu
            260                 265                 270

Ala Pro Ser Phe Ser Ser Pro Ser Gln Ala Lys Ser Phe Ile Ser Gly
        275                 280                 285

Met Asp Phe Phe Leu Gly Ala Arg Met His Ala Cys Ile Ala Ala Phe
    290                 295                 300

Ser Ser Gly Val Pro Val Val Pro Leu Ala Tyr Ser Arg Lys Phe Asn
305                 310                 315                 320

Gly Leu Phe Ser Ala Leu Asp Tyr Pro Tyr Leu Gly Asp Cys Lys Val
                325                 330                 335

Asn Thr Asn Glu Gln Ile Val Gly Leu Val Met Asp Cys Phe Asn Ser
            340                 345                 350

Arg Glu Gln Leu Lys Phe Ala Val Glu Glu Gly Asn Lys Ile Ala Glu
        355                 360                 365

Lys Lys Leu Leu Arg Tyr Glu Gln Tyr Ile Ala Glu Leu Leu Thr Arg
    370                 375                 380

Ile Ser Asn Lys Lys Ile Val Leu
385                 390

<210>  14
<211>  340
<212>  PRT
<213>  Methylobacillus flagellatus

<400>  14

Met Tyr Ser Cys Lys Met Lys Lys Tyr Ile Ile Gly Phe Leu Pro Leu
1               5                   10                  15

Ser Ile Lys Trp Leu Leu Arg Phe Cys Leu Arg Gly Pro Ser Leu Leu
            20                  25                  30

Arg Asp Phe Cys Tyr Asp Ala Lys Arg Tyr Ile Lys Trp Ser Ser Val
        35                  40                  45

Ile Ser Phe Glu Lys Asp Arg Glu Lys Ile Leu Ala Val Ile Thr Ala
    50                  55                  60

Thr Tyr His Asn Ile Glu Lys Gly Met Ser Leu Pro Asn Pro Arg Pro
65                  70                  75                  80

Leu Phe Gly Arg Val Val Ile Gly Gln Leu Val Gly Ser Val Asp Asn
                85                  90                  95

Phe His Leu Asn Phe Gly Pro Asp Pro Gln Leu Asp Val Pro Ile Asn
            100                 105                 110

Val Leu Ala Arg Tyr Cys Gln Phe Asn Arg Ser Leu Gly Gln Pro Asp
        115                 120                 125

Ser Gln Leu Glu Val Ile Val Asp Arg Leu Lys Lys Asn Asn Gln Ser
    130                 135                 140

Thr Leu Leu Arg Ile Asn Asp Gly Gly Val Ile Leu Arg Ser Lys Ala
145                 150                 155                 160

Glu Val Leu Asp Ala Ile Ser Gly Val Lys Glu Asp Phe Phe Leu Met
                165                 170                 175

Arg His Ser Val Arg Gln Phe Thr Lys Asp Pro Ile Glu His Ser Val
            180                 185                 190

Ile Glu Lys Ala Val Arg Ile Ala Gln Lys Ser Pro Ala Val Cys Asn
        195                 200                 205

Arg Gln Ser Gly Lys Val Arg Ala Ile Leu Asn Pro Glu Leu Ile Arg
    210                 215                 220

Ser Thr Leu Gln Ile Gln Gly Gly Ala Arg Gly Phe Tyr Glu Glu Val
225                 230                 235                 240

Gln Ala Leu Phe Cys Ile Thr Val Asp Leu Ser Asn Phe Asn Gly Ala
                245                 250                 255

Glu Arg Tyr Gln Gly Trp Ile Asp Gly Gly Met Phe Ala Met Ser Phe
            260                 265                 270

Ile Tyr Gly Leu His Leu Gln Gly Leu Gly Ser Cys Ala Leu Asn Trp

275                 280                 285

Ser Lys Thr Arg Lys Gln Asp Ile Glu Met Arg Asn His Leu Arg Ile
    290                 295                 300

Pro Glu Asn Glu Leu Ile Ile Met Phe Val Ala Ala Gly Tyr Leu Arg
305                 310                 315                 320

Glu Ser Phe Val Val Pro Gln Ser Tyr Arg Lys Pro Leu Gly Glu Val
                325                 330                 335

Leu Ser Tyr Ile
            340

<210>  15
<211>  490
<212>  PRT
<213>  Methylobacillus flagellatus

<400>  15

Met Lys Asn Asn Ser Ser Leu Leu Ser Phe Asn Arg Arg Asp Ala Asn
1               5                   10                  15

Phe Val Ser Leu Lys Ile Gly Ser Ile Phe Phe Val Ile Met Ala Ala
            20                  25                  30

Ile Val Ala Leu Ile Phe Gly Phe Val Ser Ala Ile Phe Pro Trp Trp
        35                  40                  45

Trp Ile Val Ala Ile Ile Leu Ser Ile Leu Phe Ile Phe Ile Gly Phe
    50                  55                  60

Thr Tyr Pro Leu Val Ala Leu Ile Ala Val Ile Ala Ile Ser Ser Arg
65                  70                  75                  80

Leu Ile Pro Asp Thr Phe Leu Pro Ser Ile Arg Leu Leu Gly Gly Thr
                85                  90                  95

Ile Pro Ser Ser Asp Met Ala Leu Phe Ala Thr Phe Ile Phe Cys Phe
            100                 105                 110

Phe Lys Tyr Asn Ser Glu Phe Ser Phe Thr Lys Gln Ala Leu Ser Pro
        115                 120                 125

Leu Ala Ile Pro Leu Phe Val Val Leu Leu Leu Leu Leu Phe Ser Val
    130                 135                 140

Phe Arg Ala Val Phe Glu Ile Arg Leu Pro Leu Lys Asp Ile Leu Gly

145                 150                 155                 160

Glu Ser Arg His Phe Leu Tyr Trp Leu Leu Ile Pro Leu Ile Ala Leu
                165                 170                 175

Thr Cys Ser Asp Arg Gln Arg Phe Lys Phe Ile Val Phe Gly Phe Leu
            180                 185                 190

Phe Val Ala Phe Leu Phe Ser Ile Gly Gln Ile Leu Gln Gly Phe Leu
        195                 200                 205

Gly Val Asn Val Phe Gly Arg Glu Leu Thr Ser Leu Glu Thr Leu Gly
    210                 215                 220

Ser Ile Asn Ser Asp Val Thr Arg Ser Met Thr Ser Gly Thr His Ile
225                 230                 235                 240

Ile Val Trp Gly Phe Leu Phe Val Leu Ala Leu Phe Ala Asn Arg Ile
                245                 250                 255

Gly Phe Ser Lys Thr Leu Ile Ile Leu Cys Ile Phe Thr Gly Ala Gly
            260                 265                 270

Ile Phe Leu Thr Tyr Gly Arg Ala Val Trp Gly Gly Thr Phe Leu Ala
        275                 280                 285

Val Leu Phe Leu Cys Trp Lys Leu Gly Thr Lys Lys Met Leu Arg Ser
    290                 295                 300

Val Gly Leu Ile Ala Ile Val Ala Val Phe Gly Leu Ser Ala Met Leu
305                 310                 315                 320

Ala Ile Lys Pro Gly Ala Ile Glu Gly Ala Met Gln Arg Leu Phe Ser
                325                 330                 335

Val Glu Glu Glu Val Ser His Gly Ser Ser Leu Gly Trp Arg Phe Tyr
            340                 345                 350

Glu Asn Lys Met Ala Trp Ala Ala Ile Gln Arg Ser Pro Leu Leu Gly
        355                 360                 365

Ile Gly Leu Gly Ala Pro Tyr Arg Pro Pro Ala Lys Ser Asp Val Thr
    370                 375                 380

Ala Glu Gln Val Arg Tyr Val His Ser Ala Tyr Ile Tyr Leu Thr Leu
385                 390                 395                 400

Lys Thr Gly Ile Ile Val Phe Met Ser Val Ile Phe Leu Ile Phe Lys
                405                 410                 415

Ile Leu His Leu Thr Tyr Ile Gln Thr Arg Ser Asn Asp Leu Phe Ile
            420                 425                 430

Ser Ser Val Ala Ile Ala Ser Phe Ala Ala Met Ile Glu Tyr Phe Leu
        435                 440                 445

Ala Cys Ile Thr Gln Pro Glu Leu Met Gln Thr Pro Gly Ile Ala Phe
    450                 455                 460

Leu Ala Thr Leu Ala Gly Leu Met Cys Ala Ala Asn Tyr Leu Asn Gln
465                 470                 475                 480

Lys Asn Leu Thr Lys Gly Lys Leu Thr Ser
                485                 490

<210>  16
<211>  268
<212>  PRT
<213>  Methylobacillus flagellatus

<400>  16

Met Asn Val Lys Thr Ser Pro Leu Ile Ser Ile Ile Thr Ala Thr Tyr
1               5                   10                  15

Asn Ser Met Lys Tyr Phe Glu Asp Thr Lys Lys Ser Val Leu Glu Gly
            20                  25                  30

Asp Val Glu Asp Phe Glu Trp Ile Ile Val Asp Asp Gly Ser Thr Asp
        35                  40                  45

Gly Thr Arg Lys Tyr Leu Gln Glu Ile Asp His Pro Asn Ile Lys Leu
    50                  55                  60

His Phe Lys Ser Ile Asn Ala Gly Ile Glu Asp Ser Tyr Arg Thr Gly
65                  70                  75                  80

Ile Glu Leu Ala Thr Gly Lys Tyr Leu Leu Ile Leu Asp His Asp Asp
                85                  90                  95

Thr Ile Pro Leu Gly Ser Leu Ser Lys Arg Leu Asp Leu Leu Glu Ser
            100                 105                 110

Asn Pro Asn Ala Leu Ala Ala Phe Gly Ala Val Ala Tyr Met Asp Glu
        115                 120                 125

Ser Gly Arg Ile Tyr Lys Glu Ser Arg Phe Pro Phe Val Ser Asp Asp
    130                 135                 140

Cys Val Leu Ser Ser Leu Phe Thr Leu Phe Gly Ile Phe Ile Leu Pro
145                 150                 155                 160

Val Tyr Pro Leu Lys Gln Gly Cys Val Val Leu Arg Ser Ser Phe Val
                165                 170                 175

Lys Asn Asn Pro Gly Leu Tyr Asp Ile Gln Leu Phe Leu Gln Ala Ala
            180                 185                 190

Lys Leu Gly Pro Val Ile Phe Ile Lys Gln Ala Cys Leu Asn Tyr Arg
        195                 200                 205

Thr Phe Arg Thr Gln Phe Ser Ser Ser Arg Lys Met Arg Leu Ile Arg
    210                 215                 220

Phe Phe Gln Phe Val Trp Ala Lys Tyr Ala Phe Lys Phe Leu Pro Trp
225                 230                 235                 240

Tyr Ile Ser Pro Phe Phe Ala Ile Tyr Lys Thr Ile Pro Glu Phe Phe
                245                 250                 255

Lys Val Leu Trp Ser Phe Val Ser Ser Lys Arg Ile
            260                 265

<210>  17
<211>  322
<212>  PRT
<213>  Methylobacillus flagellatus

<400>  17

Met Met Thr Asp Phe Ser Ser Lys Lys Lys Leu Asp Leu Ser Val Ala
1               5                   10                  15

Ile Val Phe Val Asn Tyr Asn Thr Ser Asp Gln Ile Ile Ser Cys Ile
            20                  25                  30

Tyr Ser Leu Arg Glu Glu Arg Tyr Ser Lys Ile Ile Val Val Asp Asn
        35                  40                  45

Ala Ser Pro Asp Asp Cys Pro Glu Cys Ile Val Glu Glu Phe Pro Asp
    50                  55                  60

Val Val Leu Ile Lys Ser Glu Lys Asn Leu Gly Phe Gly Glu Gly Cys
65                  70                  75                  80

Asn Ile Gly Lys Asp Trp Val Ile Val Asn Thr Asp Cys Glu Tyr Ile
                85                  90                  95

Phe Phe Leu Asn Pro Asp Thr Leu Val Glu Pro Gly Leu Leu Gln Leu
            100                 105                 110

Leu His Asp Gly Phe Phe Ser Pro Glu Val Gly Leu Val Thr Pro Arg
        115                 120                 125

Ile Thr Met Met Gly Asp Gly Leu Asp Ile Leu Trp Tyr Gly Gly Gly
    130                 135                 140

Tyr Met Asn Trp Leu Thr Gly Ser Ala Arg Val Pro Gly Cys Gly Leu
145                 150                 155                 160

Ser Ala Val Ser Lys Ile Ala Leu Glu Asp Arg Phe Val Glu Phe Ala
                165                 170                 175

Ser Gly Cys Ala Phe Met Ile Arg Arg Ser Val Leu Glu Glu Cys Gly
            180                 185                 190

Gly Phe Asp Pro Arg Tyr Phe Met Tyr Glu Glu Asp Val Glu Leu Ser
        195                 200                 205

Leu Arg Val Ile Ser Tyr Gly Tyr Lys Ile Tyr Tyr Met Pro Lys Ala
    210                 215                 220

Val Val Arg His Leu Ala Gln Gly Ser Gln Gly Lys Glu Val Glu Ala
225                 230                 235                 240

Val Gly Met Phe Lys Gly Arg Asn Pro Lys Leu Pro Phe Phe Val Tyr
                245                 250                 255

Gln Thr Ala Lys Asn Arg Phe Leu Thr Val Phe Thr His Gly Thr Val
            260                 265                 270

Leu Lys Lys Gly Leu Phe Leu Ile Gly Phe Met Leu Trp Val Phe Arg
        275                 280                 285

Asn Ser Phe Glu Trp Val Arg Tyr Lys Arg Leu Asp Ala Tyr Asn Ala
    290                 295                 300

Leu Phe Leu Gly Ile Lys Asp Phe Val Lys Tyr Thr Tyr Val Ser Gly
305                 310                 315                 320

Lys Lys

<210>  18
<211>  382
<212>  PRT
<213>  Methylobacillus flagellatus

<400>  18

Met Lys Glu Ile Asn Val Ala Phe Val Glu Pro Val Gly Ser His Gly
1               5                   10                  15

Gly Met Asp Tyr Tyr Asp Ser Gly Leu Cys Ala Gly Leu Leu His Gln
            20                  25                  30

Gly Val Ser Ala Thr Trp Tyr Thr Cys Asp Ile Ser Lys Pro Cys Gly
        35                  40                  45

Asn Arg Pro Ile Phe Met Gln Leu Thr Phe Val Asn Ile Trp Gly Lys
    50                  55                  60

Asp Ala Thr Trp Arg Arg Gly Ala Arg Tyr Ile Leu Gly Leu Cys Arg
65                  70                  75                  80

Ser Ile Lys His Ala Lys Lys Asn Tyr Ser Asn Ile Ala His Phe His
                85                  90                  95

Phe Phe His Val Gly Val Leu Glu Phe Leu Ser Val Leu Phe Phe Arg
            100                 105                 110

Leu Phe Gly Phe Lys Val Val Ala Thr Val His Asp Val Glu Ser Phe
        115                 120                 125

Lys Pro Gly Leu Thr Phe Ser Ile Leu Leu Lys Trp Thr Tyr Phe Leu
    130                 135                 140

Cys Asn Gln Leu Ile Val His Asn Gln Val Ser Lys Asp Glu Leu Ile
145                 150                 155                 160

Asn Arg Ser Asn Val Ser Ile Asn Lys Ile His Val Ile Pro His Gly
                165                 170                 175

Ser Tyr Ile Gly Leu Val Ala Pro Ser Met Pro Lys Ala Asp Ala Arg
            180                 185                 190

Gln Ala Leu Asn Val Ser Ser Asp Glu Lys Val Ile Leu Phe Phe Gly
        195                 200                 205

Gln Ile Lys Glu Val Lys Gly Leu Asp Leu Leu Ile Glu Ala Leu Gly
    210                 215                 220

Leu Val Lys Asp Lys Leu Lys Pro Phe Lys Leu Val Ile Ala Gly Lys
225                 230                 235                 240

Val Trp Lys Asp Asn Phe Ser Lys Tyr Gln Glu Leu Ile Ser Lys Asn
                245                 250                 255

Gly Leu Asn Asp Phe Cys Lys Leu Asp Ile Arg Tyr Ile Pro Asp Gln
            260                 265                 270

Glu Ile Ser Met Phe Tyr Ser Ala Ala Asp Leu Ile Val Leu Pro Tyr
        275                 280                 285

Arg Lys Ile Tyr Gln Ser Gly Val Leu Leu Met Ala Met Ser Tyr Gly
    290                 295                 300

Thr Pro Val Leu Ala Ser Asp Leu Pro Gly Met Ala Gln Ile Ile Asn
305                 310                 315                 320

Asp Gly Glu Asn Gly Phe Leu Phe Ser Ala Gly Asn Tyr Ile Asp Leu
                325                 330                 335

Ala Glu Lys Leu Val Lys Ile Phe Ser Tyr His Asp Leu Ser Arg Ile
            340                 345                 350

Thr Leu Gln Ala Glu Asn Asp Met Arg Thr Arg Phe Ser Trp Asp Glu
        355                 360                 365

Ile Ala Ile Lys Thr Ile Ser Val Tyr Gln Lys Ser Ile Ser
    370                 375                 380

<210>  19
<211>  501
<212>  PRT
<213>  Methylobacillus flagellatus

<400>  19

Met Gln Leu Ile Asn Ser Arg Arg Ile Val Leu Leu Ser Leu Phe Ser
1               5                   10                  15

Leu Met Pro Leu Val Gly Ile Ala Glu Val Tyr Lys Ser Thr Asp Val
            20                  25                  30

Asp Gly Ser Thr Thr Tyr Ser Asp Thr Pro Gln Ala Asn Ser Gln Lys
        35                  40                  45

Ile Lys Val Phe Pro Ser Asp Tyr Lys Lys Asn Val Lys Ser Glu Ala
    50                  55                  60

Ala Ala Asn Lys Ser Arg Ser Asn Ser Ile Ser Ala Ala Asn Val Thr
65                  70                  75                  80

Glu Ala Pro Ile Asp Glu Ser Ser Ser Asp Ser Asn Val Thr Ile Lys
                85                  90                  95

His Ser Thr Ser Asn Lys Ser Ile Asn Ser Ser Asn Pro Ser Ala Ala
            100                 105                 110

Gly Gly Ser Ser Ser Ala Gly Gly Ser Ser Ser Ala Gly Gly Ser Ser
        115                 120                 125

Ser Ala Gly Gly Ser Ser Ser Ala Gly Gly Ser Ser Ser Ala Gly Gly
    130                 135                 140

Ser Ser Ser Ala Gly Gly Ser Ser Ser Ala Gly Gly Ser Ser Ser Ala
145                 150                 155                 160

Gly Gly Ser Ser Ser Glu Thr Thr Thr Ala Ala Ser Val Asp Lys Val
                165                 170                 175

Ser Ala Ala Leu Gln Gly Pro Lys Ala Phe Leu Asn Ser Asp Asp Phe
            180                 185                 190

Lys Glu Asn Ser Tyr Arg Gly Phe Asn Val Leu Gly Arg Lys Leu Ser
        195                 200                 205

Ser Ala Asp Met Glu Asp Leu Glu Lys Phe Asn Val Asn Leu Ile Arg
    210                 215                 220

Ser Val Ile His Leu Glu Lys Cys Lys Lys Cys Asn Asp Tyr Glu Ile
225                 230                 235                 240

Ser Pro Val Glu Leu Ser Gln Met Asp Phe Leu Val Glu Ala Gly Arg
                245                 250                 255

Lys Leu Gly Phe Tyr Val Ile Phe Ala Phe Asp Pro Lys Pro Ala Gly
            260                 265                 270

Asp Gln Ala Ile Tyr Trp Asn Asn Gln Gln Leu Arg Glu Ser Ile Lys
        275                 280                 285

Asn Asn Trp Val Thr Ile Ala Lys Arg Tyr Lys Asn Glu Thr Val Val
    290                 295                 300

Ala Gly Phe Asp Leu Ile Asn Glu Pro Val Val Arg Pro Gly Ser Val
305                 310                 315                 320

Trp Glu Lys Tyr Ala Thr Glu Leu Ile Thr Ala Ile Arg Lys Val Asp
                325                 330                 335

Glu Asn His Ile Ile Phe Leu Glu Pro Ser Pro Trp Gly Leu Ala Ser
            340                 345                 350

Ser Leu Glu Gln Phe Thr Pro Val Pro Phe Asn Asn Ile Val Tyr Ser
        355                 360                 365

Phe His Phe Tyr Ser Pro Gln Glu Leu Thr His Gln Gly Leu Ala Ala
    370                 375                 380

Tyr Pro His Ala Tyr Ile Tyr Pro Ser Glu Lys Trp Asp Lys Glu Tyr
385                 390                 395                 400

Leu Asn Asn Arg Leu Asp Ile Ala Arg Asn Trp Ala Asn Lys Tyr Asn
                405                 410                 415

Val Pro Leu Tyr Ile Gly Glu Phe Ser Ile Ile Arg Trp Ala Pro Ser
            420                 425                 430

Gly Ser Arg Leu Arg Tyr Leu Thr Asp Val Val Glu Arg Phe Glu Gln
        435                 440                 445

Glu Lys Trp Ser Trp Thr Tyr His Ser Tyr Arg Glu Tyr Ser Gly Trp
    450                 455                 460

Asp Ala Glu Met Pro Asp Ser Ile Phe Lys Asn Asp Ala Ile Val Glu
465                 470                 475                 480

Arg Gln Asn Asn Val Pro Val Leu Gln Leu Leu Met Lys Asn Phe Asn
                485                 490                 495

Lys Asn Lys Lys Tyr
            500

<210>  20
<211>  368
<212>  PRT
<213>  Methylobacillus flagellatus

<400>  20

Met His Lys Lys Ile Ala Ile Leu Gly Thr Arg Gly Ile Pro Ala Ala
1               5                   10                  15

His Gly Gly Phe Glu Thr Phe Ala Glu His Leu Ala Ile Tyr Leu Glu
            20                  25                  30

Arg Lys Gly Trp Lys Val Thr Val Tyr Cys Gln Val Asp Gly Asn Gly
        35                  40                  45

Pro Met Tyr Glu Asp Met Trp Asn Gly Ile Lys Arg Val Asn Ile Pro
    50                  55                  60

Ile Ser Ile Gln Gly Ser Leu Gly Thr Ile Leu Phe Asp Trp Lys Ser
65                  70                  75                  80

Thr Ile His Ala Ser Asn Thr Tyr His Leu Ile Leu Thr Leu Gly Tyr
                85                  90                  95

Asn Thr Ala Leu Phe Cys Gly Leu Tyr Arg Thr Lys Gly Ile Thr Asn
            100                 105                 110

Leu Ile Asn Met Asp Gly Ile Glu Trp His Arg Lys Lys Trp Gly Ala
        115                 120                 125

Val Ala Lys Leu Trp Phe Trp Ile Asn Asp Trp Ala Gly Ser Ile Leu
    130                 135                 140

Gly Asn His Leu Ile Ala Asp His Pro Gln Ile Ala Arg His Leu Glu
145                 150                 155                 160

Thr Arg Val Arg Pro Ser Lys Ile Thr Met Ile Pro Tyr Gly Ala Asp
                165                 170                 175

Lys Val Ile Glu Ala Asn Gln Ala Val Leu Glu Glu Tyr Gly Leu Lys
            180                 185                 190

Pro Phe Ser Tyr Ala Ile Leu Ile Ala Arg Ala Glu Pro Glu Asn Ser
        195                 200                 205

Ile Leu Glu Val Val Lys Ala Trp Ser Ala Lys Pro Arg Gly Met Arg
    210                 215                 220

Leu Val Val Leu Gly Lys Tyr Asn Pro Asp His Ala Tyr Gln Val Ala
225                 230                 235                 240

Val Lys Ala Ala Gly Ser Asp Glu Val Leu Phe Pro Gly Ala Ile Tyr
                245                 250                 255

Asp Lys His Val Val Glu Ser Leu Arg Tyr Phe Ser Arg Leu Tyr Ile
            260                 265                 270

His Gly His Gln Val Gly Gly Thr Asn Pro Ser Leu Val Glu Ala Leu

275                 280                 285

Gly Ala Gly Asn Ala Val Leu Ala His Asp Asn Arg Phe Asn Arg Trp
    290                 295                 300

Val Ala Gly Asp Lys Ala Ala Tyr Phe Ser Gly Glu Lys Asp Cys Glu
305                 310                 315                 320

Ile Thr Leu Asp Glu Leu Leu Asn Asp Glu Val Arg Leu Arg Glu Met
                325                 330                 335

Arg Ala Gly Ser Leu Ala Gln Tyr Asp Thr Leu Phe Thr Trp Asp Ser
            340                 345                 350

Val Leu Gln Gln Tyr Glu Ala Leu Leu Thr Arg Phe Leu Pro Asn Lys
        355                 360                 365

<210>  21
<211>  479
<212>  PRT
<213>  Methylobacillus flagellatus

<400>  21

Met Gln Ile Gln Ala Val Ile Leu Ser Gly Gly Ser Gly Thr Arg Leu
1               5                   10                  15

Trp Pro Leu Ser Arg Glu Asn Tyr Pro Lys Gln Leu Leu Thr Leu Leu
            20                  25                  30

Asp Asn Asp Ser Leu Leu Gln Ser Thr Leu Arg Arg Val Asp Pro Leu
        35                  40                  45

Ile Gly Thr Glu Cys Ser Pro Ile Leu Val Cys Asn Glu Glu Tyr Arg
    50                  55                  60

Phe Val Ile Ala Glu Gln Leu Arg Met Met Gly Arg Arg Gly Ser Ile
65                  70                  75                  80

Leu Leu Glu Pro Val Gly Lys Asn Thr Ala Pro Ala Leu Thr Leu Ala
                85                  90                  95

Ala Leu Glu Ala Ala Ser His Gly Gln Asp Pro Ile Leu Leu Val Met
            100                 105                 110

Pro Ala Asp His Ala Ile Ile Asn Val Gln Asp Phe Gln Asp Thr Val
        115                 120                 125

Arg Thr Gly Ala Glu His Ala Ala Ala Gly Lys Ile Val Thr Phe Gly

130                 135                 140

Ile Val Pro Asp Arg Ala Glu Thr Gly Tyr Gly Tyr Ile Lys Ser Gly
145                 150                 155                 160

Ala Val Leu Glu Gly Phe Gly Ser Ala Phe Ser Ile Glu Gln Phe Val
                165                 170                 175

Glu Lys Pro Asp Glu Ala Thr Ala Gln Gln Tyr Val Val Ser Gly Asn
            180                 185                 190

Tyr Phe Trp Asn Ser Gly Leu Phe Met Met Arg Ala Thr Val Trp Leu
        195                 200                 205

Asn Ala Ile Ala Lys Thr Arg Pro Asp Ile Leu Ser Ser Cys Lys Gln
    210                 215                 220

Ala Trp Asp Gly Gln Gln Arg Asp Gly Asp Phe Val Arg Val Asp Lys
225                 230                 235                 240

Leu Lys Phe Gln Gln Val Pro Ser Asp Ser Ile Asp Tyr Ala Val Met
                245                 250                 255

Glu His Val Ala Arg Gly Ala Asp Asn Leu Pro Ala Gly Ile Val Ile
            260                 265                 270

Pro Leu Asn Ala Gly Trp Ser Asp Val Gly Ala Trp Asp Ser Phe Gly
        275                 280                 285

Lys Ile Leu Pro Gln Asp Asn Ala Gly Asn Ser His Leu Gly Asp Val
    290                 295                 300

Leu Phe Asn Asp Cys Ser Asn Thr Leu Ala Val Ser Ser Gly Arg Leu
305                 310                 315                 320

Leu Ala Cys Val Gly Leu Gln Asn Ile Ala Val Ile Glu Thr Ala Asp
                325                 330                 335

Ala Val Leu Val Ile Asp Lys Ser Lys Thr Gln Asp Val Lys Lys Ile
            340                 345                 350

Val Glu Gln Leu Lys Val Gln Lys Arg Pro Glu Ala Lys Thr His Arg
        355                 360                 365

Lys Val His Arg Pro Trp Gly Trp Tyr Asp Ser Ile Asp Met Gly Ala
    370                 375                 380

Arg Phe Gln Val Lys Arg Ile Val Val Asn Pro Gly Ala Thr Leu Ser
385                 390                 395                 400

Leu Gln Met His His His Arg Ala Glu His Trp Ile Val Val Thr Gly
                405                 410                 415

Thr Ala Lys Val Thr Arg Gly Asp Glu Glu Phe Leu Val Ser Glu Asn
            420                 425                 430

Gln Ser Thr Tyr Ile Pro Leu Gly Val Thr His Met Leu Ala Asn Pro
        435                 440                 445

Gly Lys Leu Pro Leu Glu Met Ile Glu Val Gln Ser Gly Ala Tyr Leu
    450                 455                 460

Gly Glu Asp Asp Ile Val Arg Phe Asn Asp Gln Tyr Gly Arg Ser
465                 470                 475

<210>  22
<211>  306
<212>  PRT
<213>  Methylobacillus flagellatus

<400>  22

Met Ala Thr Phe Asn Lys Ile Leu Leu Thr Gly Ile Asn Gly Gln Val
1               5                   10                  15

Gly His Ala Leu Tyr Pro Lys Leu Gln Ser Leu Gly Glu Val Val Ala
            20                  25                  30

Leu Asp Arg Ser Gln Leu Asp Leu Ser Lys Pro Asp His Ile Arg Asp
        35                  40                  45

Val Val Arg Gln Thr Lys Pro Asp Leu Ile Ile Asn Pro Ala Ala Tyr
    50                  55                  60

Thr Ala Val Asp Lys Ala Glu Ser Glu Pro Glu Leu Ala Tyr Ala Ile
65                  70                  75                  80

Asn Gly Thr Ala Pro Gly Ile Leu Ala Glu Glu Ala Ala Lys Leu Asn
                85                  90                  95

Ala Leu Leu Val His Tyr Ser Thr Asp Tyr Val Phe Asp Gly Thr Lys
            100                 105                 110

Thr Thr Pro Tyr Thr Glu Leu Asp Glu Thr Asn Pro Leu Ser Val Tyr
        115                 120                 125

Gly Ala Ser Lys Leu Ala Gly Glu Lys Ala Ile Gln Glu Val Gly Ala
    130                 135                 140

Asn His Leu Ile Phe Arg Thr Ser Trp Val Tyr Gly Ala Tyr Gly Lys
145                 150                 155                 160

Asn Phe Met Arg Thr Ile Leu Arg Leu Ala Ala Glu Arg Asp Ser Leu
                165                 170                 175

Asn Ile Val Ala Asp Gln Phe Gly Ala Pro Thr Ser Thr Glu Ser Ile
            180                 185                 190

Ala Asp Ala Thr Ile Thr Ala Leu His Ala Trp Asp Gly Thr Gln Arg
        195                 200                 205

Gly Val Tyr His Leu Val Asn Thr Gly Glu Thr Ser Trp His Gly Phe
    210                 215                 220

Ala Leu Glu Ile Ile Arg Gln Tyr Gly Glu Ile Ala Gln Lys Lys Asn
225                 230                 235                 240

Leu Ala Lys Leu Lys Ser Thr Val Glu Asn Val Val Gly Ile Thr Thr
                245                 250                 255

Ala Glu Tyr Pro Thr Pro Ala His Arg Pro Ala Asn Ser Lys Met Ser
            260                 265                 270

Thr Glu Lys Phe Met Ser Thr Phe Lys Thr Glu Leu Ala Pro Trp Glu
        275                 280                 285

Leu Ala Leu Arg Ser Thr Val Thr His Leu Ala Thr Phe Ser Val Glu
    290                 295                 300

Asn His
305

<210>  23
<211>  182
<212>  PRT
<213>  Methylobacillus flagellatus

<400>  23

Met Asn Val Thr Pro Thr His Ile Pro Asp Val Ile Val Phe Glu Pro
1               5                   10                  15

Lys Ile Phe Gly Asp Ala Arg Gly Phe Phe Phe Glu Ser Phe Asn Gln
            20                  25                  30

Lys Ala Phe Glu Glu Ala Val Gly Arg Lys Tyr Asp Phe Val Gln Asp
        35                  40                  45

Asn His Ser Lys Ser Ser Lys Gly Val Leu Arg Gly Leu His Tyr Gln
    50                  55                  60

Ile Gln Gln Ala Gln Gly Lys Leu Val Arg Val Val Ala Gly Glu Val
65                  70                  75                  80

Phe Asp Val Ala Val Asp Ile Arg Lys Ser Ser Pro Thr Phe Gly Gln
                85                  90                  95

Trp Val Gly Gln His Leu Ser Ala Glu Asn Lys Lys Gln Met Trp Val
            100                 105                 110

Pro Pro Gly Phe Ala His Gly Phe Leu Val Leu Ser Glu Thr Ala Glu
        115                 120                 125

Phe Leu Tyr Lys Thr Thr Asp Phe Tyr Ala Pro Ala His Glu Arg Cys
    130                 135                 140

Leu Ser Trp Asn Asp Pro Val Val Gly Ile Glu Trp Pro Asp Ile Gly
145                 150                 155                 160

Thr Pro Pro Leu Leu Ser Ser Lys Asp Gln Gln Gly Leu Gly Leu His
                165                 170                 175

Gln Thr Glu Leu Phe Ala
            180

<210> 24
<211> 292
<212> PRT
<213> Methylobacillus flagellatus

<400> 24

Met Arg Lys Gly Ile Ile Leu Ala Gly Gly Ser Gly Thr Arg Leu Tyr
1               5                   10                  15

Pro Val Thr Gln Ala Val Ser Lys Gln Leu Leu Pro Val Tyr Asp Lys
            20                  25                  30

Pro Met Ile Tyr Tyr Pro Leu Ser Thr Leu Met Leu Ala Gly Ile Arg
        35                  40                  45

Asp Ile Leu Leu Ile Ser Thr Pro Gln Asp Thr Pro Arg Phe Glu Gln
    50                  55                  60

Leu Leu Gly Asp Gly Ser Gln Trp Gly Leu Asn Ile Ala Tyr Ala Val
65                  70                  75                  80

Gln Pro Ser Pro Asp Gly Leu Ala Gln Ala Phe Ile Ile Gly Lys Lys
                85                  90                  95

Phe Ile Gly Asn Asp Pro Ser Ala Leu Val Leu Gly Asp Asn Ile Phe
            100                 105                 110

Tyr Gly His Asp Leu Ala His Asp Leu Ala His Ala Asp Gln Arg Gln
        115                 120                 125

His Gly Ala Thr Val Phe Ala Tyr Pro Val His Asp Pro Glu Arg Tyr
    130                 135                 140

Gly Val Val Glu Phe Asn Asp Ala Gly Gln Ala Ile Ser Leu Glu Glu
145                 150                 155                 160

Lys Pro Lys Glu Pro Lys Ser Arg Tyr Ala Val Thr Gly Leu Tyr Phe
                165                 170                 175

Tyr Asp Asn Gln Val Val Asp Ile Ala Ala Ser Leu Lys Pro Ser Pro
            180                 185                 190

Arg Gly Glu Leu Glu Ile Thr Asp Val Asn Lys Ile Tyr Leu Glu Arg
        195                 200                 205

Arg Ser Leu Asn Val Glu Leu Met Gly Arg Gly His Ala Trp Leu Asp
    210                 215                 220

Thr Gly Thr His Glu Ser Leu Leu Glu Ala Ser Leu Phe Ile Glu Thr
225                 230                 235                 240

Ile Glu Lys Arg Gln Gly Leu Lys Val Ala Cys Pro Glu Glu Ile Ala
                245                 250                 255

Tyr Arg Lys Gly Tyr Ile Asp Ala Glu Gln Val Glu Lys Ile Ala Gln
            260                 265                 270

Pro Tyr Leu Lys Asn Gly Tyr Gly Gln Tyr Leu Ile Arg Met Leu Lys
        275                 280                 285

Glu Lys Val Phe
    290

<210>  25
<211>  366
<212>  PRT

<213>  Methylobacillus flagellatus

<400>  25

Met Thr Thr Leu Asn Asn Thr Ile Ile Val Thr Gly Gly Ala Gly Phe
1               5                   10                  15

Ile Gly Gly Asn Phe Val Leu Asp Trp Val Lys Ala Gly Leu Gly Thr
            20                  25                  30

Val Ile Asn Leu Asp Lys Leu Thr Tyr Ala Gly Asn Leu Glu Asn Leu
        35                  40                  45

Ala Asp Ile Glu Gly Asn Pro Asn His Ile Phe Val His Gly Asp Ile
    50                  55                  60

Gly Asp Arg Ala Leu Val Ala Lys Leu Leu Ala Glu His Lys Pro Arg
65                  70                  75                  80

Ala Val Ile Asn Phe Ala Ala Glu Ser His Val Asp Arg Ser Ile His
                85                  90                  95

Gly Pro Glu Asp Phe Ile Gln Thr Asn Val Val Gly Thr Phe His Leu
            100                 105                 110

Leu Glu Glu Val Arg Ala Tyr Trp Asn Ser Val Val Lys Thr His Pro
        115                 120                 125

Glu Glu Lys Glu Asn Pro Gln Phe Arg Phe Leu His Val Ser Thr Asp
    130                 135                 140

Glu Val Tyr Gly Thr Leu Gly Pro Lys Asp Gln Ala Phe Thr Glu Thr
145                 150                 155                 160

Thr Pro Tyr Ala Pro Asn Ser Pro Tyr Ser Ala Ser Lys Ala Ala Ser
                165                 170                 175

Asp His Leu Val Arg Ala Tyr His His Thr Tyr Gly Leu Pro Val Leu
            180                 185                 190

Thr Thr Asn Cys Ser Asn Asn Tyr Gly Pro Tyr His Phe Pro Glu Lys
        195                 200                 205

Leu Ile Pro Leu Cys Leu Leu Asn Ala Leu Ala Gly Lys Pro Leu Pro
    210                 215                 220

Ile Tyr Gly Asp Gly Gln Gln Ile Arg Asp Trp Leu Phe Val Lys Asp
225                 230                 235                 240

His Cys Ser Ala Ile Arg Arg Val Leu Glu Ala Gly Gln Leu Gly Glu
                245                 250                 255

Thr Tyr Asn Val Gly Gly Trp Asn Glu Lys Ala Asn Leu Asp Val Val
            260                 265                 270

Gln Thr Leu Cys Ser Ile Leu Asp Glu Leu Ser Pro Lys Ala Asp Gly
        275                 280                 285

Ser Ser Tyr Lys Asp Gln Ile Thr Phe Val Lys Asp Arg Pro Gly His
    290                 295                 300

Asp Arg Arg Tyr Ala Ile Asp Ala Ser Lys Leu Glu Arg Glu Leu Gly
305                 310                 315                 320

Trp Lys Pro Ala Glu Thr Phe Glu Ser Gly Ile Arg Lys Thr Val Glu
                325                 330                 335

Trp Tyr Leu Gln Asn Gln Ala Trp Val His Asn Val Thr Ser Gly Glu
            340                 345                 350

Tyr Arg Asn Trp Val Ser Lys Gln Tyr Ser Gly Ser Ala Ala
        355                 360                 365

<210>  26
<211>  334
<212>  PRT
<213>  Methylobacillus flagellatus

<400>  26

Met Leu Leu Val Thr Gly Gly Thr Gly Tyr Ile Gly Ser His Ala Cys
1               5                   10                  15

Val Glu Leu Ile Asn Ala Gly Tyr Glu Val Val Val Leu Asp Asn Phe
            20                  25                  30

Ser Asn Ser Gln Ala His Ile Val Asp Arg Ile Lys Gln Ile Thr Gly
        35                  40                  45

Lys Ala Pro Val Leu Val Glu Gly Asp Val Arg Asp Glu Ala Leu Leu
    50                  55                  60

Ser Asp Leu Phe Ser Arg Tyr Pro Phe Gln Ala Val Met His Phe Ala
65                  70                  75                  80

Gly Leu Lys Ala Val Gly Glu Ser Val Ala Gln Pro Leu Arg Tyr Tyr
                85                  90                  95

Asp Asn Asn Val Ala Gly Ser Val Thr Leu Leu Arg Val Met Gln Ala
        100                 105                 110

His Asp Val Lys Arg Leu Val Phe Ser Ser Ser Ala Thr Val Tyr Gly
    115                 120                 125

Asp Pro Ala Ser Val Pro Ile Arg Glu Asp Phe Pro Leu Ser Ala Thr
    130                 135                 140

Asn Pro Tyr Gly Ala Ser Lys Leu Met Val Glu Asp Ile Leu Arg Asp
145                 150                 155                 160

Leu Ala Val Ser Asp Pro Glu Trp Arg Ile Ala Ile Leu Arg Tyr Phe
                165                 170                 175

Asn Pro Val Gly Ala His Glu Ser Gly Leu Ile Gly Glu Asn Pro Asn
            180                 185                 190

Gly Met Pro Asn Asn Leu Met Pro Tyr Ile Thr Gln Val Ala Gln Gly
        195                 200                 205

Gln Arg Glu Ser Leu Ser Val Phe Gly Ser Asp Tyr Pro Thr Val Asp
    210                 215                 220

Gly Thr Gly Val Arg Asp Tyr Ile His Val Val Asp Leu Val Val Gly
225                 230                 235                 240

His Leu Arg Ala Leu Glu Arg Leu Asp Lys Glu Ala Gly Val Phe Thr
                245                 250                 255

Cys Asn Leu Gly Thr Gly Gln Gly Tyr Ser Val Leu Glu Met Val Lys
            260                 265                 270

Ala Phe Glu Asp Ala Ser Gly Gln Ala Val Pro Tyr Gln Leu Val Ala
        275                 280                 285

Arg Arg Pro Gly Asp Val Ala Ala Ser Tyr Thr Asp Pro Ala Tyr Ala
    290                 295                 300

Glu Gln Ala Leu Gly Trp Lys Ala Thr Arg Gly Ile Glu Gln Met Cys
305                 310                 315                 320

Lys Asp Ser Trp His Trp Gln Gln Asn Leu Ala Gln Arg Gly
                325                 330

<210>  27
<211>  472

<212>  PRT
<213>  Methylobacillus flagellatus

<400>  27

Met Leu Leu Pro Val Ile Met Ser Gly Gly Ala Gly Ser Arg Leu Trp
1               5                   10                  15

Pro Val Ser Arg Glu Thr Leu Thr Lys Pro Phe Ile Glu Leu Pro Asp
            20                  25                  30

Gly Arg Thr Leu Leu Gln Lys Thr Leu Gln Arg Thr Ala Cys Leu Asp
        35                  40                  45

Gly Val Ser Glu Val Leu Thr Val Thr Asn Lys Glu Tyr Tyr Phe Leu
    50                  55                  60

Thr Arg Asp Val Tyr Glu Ala Thr Asp Thr Asn Val Ser His Ser Tyr
65                  70                  75                  80

Leu Leu Glu Thr Ile Gly Arg Asn Thr Ala Pro Ala Ile Ala Val Ala
                85                  90                  95

Ala Leu His Ala Ala Ala Lys His Gly Glu Asp Val Val Leu Leu Val
            100                 105                 110

Met Pro Ala Asp His Leu Ile Gly Asn Gln Lys Val Phe Ser Lys Ala
        115                 120                 125

Val Thr Val Ala Thr Asp Met Ala Val Asn Gly Trp Ile Val Thr Phe
    130                 135                 140

Gly Val Glu Pro Ser Thr Pro Glu Thr Gly Tyr Gly Tyr Ile Glu Gly
145                 150                 155                 160

Gly Glu Ser Ile Gly Asp Ser Gly Ala Arg Arg Val Val Arg Phe Val
                165                 170                 175

Glu Lys Pro Asp Leu Glu Thr Ala Glu Lys Tyr Ile Ala Leu Gly Arg
            180                 185                 190

Phe Thr Trp Asn Ala Gly Ile Phe Cys Phe Thr Ala Lys Thr Ile Ile
        195                 200                 205

Ala Ala Leu Ala Glu Tyr Val Pro Glu Val Leu Glu Ile Ala Lys Lys
    210                 215                 220

Thr Leu Ala Ser Ser Asp Ser Lys Thr Glu Pro Val Val Leu Gly Glu
225                 230                 235                 240

Glu His Phe Ala Ser Ser Pro Asp Ile Ser Ile Asp Tyr Ala Val Met
                245                 250                 255

Glu Arg Ala Ser Lys Val Ala Val Val Ala Ala Asp Phe Asp Trp Ser
            260                 265                 270

Asp Ile Gly Ser Trp Ser Ala Met Ala Glu Leu Val Gln Ser Asp Glu
        275                 280                 285

Arg Gly Asn Arg Val Asn Gly Glu Ala Leu Leu Val Asp Ala Asn Asn
    290                 295                 300

Cys Tyr Ile Gln Ser Asp Ser Arg Leu Val Ala Gly Ile Gly Ile Asp
305                 310                 315                 320

Asn Leu Leu Val Val Asp Thr Pro Asp Ala Leu Leu Val Ala His His
                325                 330                 335

Ser Arg Ala Gln Asp Val Lys Thr Val Val Lys Gln Leu Lys Leu Glu
            340                 345                 350

Ser His Ser Ala Ala Asn Tyr His Arg Lys Val His Arg Pro Trp Gly
        355                 360                 365

Thr Tyr Thr Val Leu Glu Glu Gly Ala Asn Phe Lys Ile Lys Cys Ile
    370                 375                 380

Lys Val Lys Pro Gly Ala Ser Leu Ser Leu Gln Met His His His Arg
385                 390                 395                 400

Ser Glu His Trp Val Val Val Ser Gly Ile Ala Lys Val Val Asn Gly
                405                 410                 415

Glu Arg Glu Leu Leu Ile Asn Ala Asn Glu Ser Thr Tyr Ile Pro Ala
            420                 425                 430

Gly His Lys His Arg Leu Glu Asn Pro Gly Leu Leu Asp Leu Val Ile
        435                 440                 445

Ile Glu Val Gln Ser Gly Glu Tyr Leu Gly Glu Asp Asp Ile Val Arg
    450                 455                 460

Phe Gln Asp Ile Tyr Gly Arg Thr
465                 470

<210>  28

<211>  264
<212>  PRT
<213>  Methylobacillus flagellatus

<400>  28

Met Ile Ser Met Leu Lys Ala Leu Trp Leu Tyr Arg Gly Phe Val Leu
1               5                   10                  15

Gly Ser Val Lys Arg Glu Phe Gln Ser Lys Tyr Arg Asn Ser Leu Leu
            20                  25                  30

Gly Ala Val Trp Thr Val Leu Asn Pro Leu Ala Met Ile Val Val Tyr
        35                  40                  45

Thr Val Ile Phe Ser Gln Val Met Lys Ala Arg Leu Pro Gly Val Asp
    50                  55                  60

Ser Thr Phe Ala Phe Ser Ile His Leu Cys Ala Gly Ile Leu Thr Trp
65                  70                  75                  80

Gly Leu Phe Ala Glu Ile Val Gly Arg Ser Gln Thr Thr Phe Ile Asp
                85                  90                  95

Asn Ala Asn Leu Leu Lys Lys Leu Ser Phe Pro Arg Leu Cys Leu Pro
            100                 105                 110

Val Ile Val Thr Ala Thr Ala Leu Val Asn Phe Ala Ile Val Phe Gly
        115                 120                 125

Leu Phe Thr Ala Phe Leu Leu Leu Ile Gly Met Phe Ser Gly Trp Pro
    130                 135                 140

Tyr Leu Val Leu Ile Pro Val Leu Ile Leu Met Ile Val Phe Ala Leu
145                 150                 155                 160

Gly Leu Gly Ile Ile Leu Gly Val Leu Asn Val Phe Phe Arg Asp Val
                165                 170                 175

Gly Gln Phe Phe Gly Ile Val Leu Ser Phe Trp Phe Trp Leu Thr Pro
            180                 185                 190

Ile Val Tyr Pro Val Ser Ile Leu Pro Glu Arg Val Gln Asp Tyr Met
        195                 200                 205

Arg Leu Asn Pro Met Ala Arg Leu Met Glu Ala Phe Gln Gly Ile Leu
    210                 215                 220

Val Gln Ala Lys Trp Pro Asp Trp Tyr Ser Leu Trp Pro Val Phe Ala

225                 230                 235                 240

Leu Ser Ile Val Leu Cys Leu Leu Gly Leu Ser Leu Phe Arg Lys His
                245                 250                 255

Ala Gly Asp Met Val Asp Glu Leu
            260

<210>  29
<211>  178
<212>  PRT
<213>  Methylobacillus flagellatus

<400>  29

Met Asp Ser Ala Arg Ser Lys Phe Ser Tyr Pro Ala Arg Arg Asp Gly
1               5                   10                  15

Gly Ser Phe Ala Val Thr Ala Ala Lys Arg Ser Asn Thr Ser Ile Lys
            20                  25                  30

Pro Asn Leu Ala Val Ile Asn Trp Met Leu Glu Gln Glu Asp Arg Met
        35                  40                  45

Gly Leu Ser Thr Pro Arg Pro Tyr Arg Glu Phe Glu Glu Arg Val Tyr
    50                  55                  60

Arg His Arg Asp Asp Leu Arg Arg Leu Ile His Ser Leu Val Ala Asp
65                  70                  75                  80

Gly Lys Lys Ile Tyr Gly Tyr Gly Ala Ser Ala Lys Gly Asn Val Val
                85                  90                  95

Leu Gln Phe Cys Gly Phe Thr Ser Glu Glu Ile Pro Ala Ile Ala Glu
            100                 105                 110

Val Asn Glu Glu Lys Phe Gly Arg Val Thr Pro Gly Thr His Ile Pro
        115                 120                 125

Ile Ile Ser Glu Lys Glu Ala Arg Ala Ala Lys Pro Asp Tyr Phe Leu
    130                 135                 140

Val Leu Pro Trp His Phe Lys Asp Gly Ile Leu Arg Arg Glu Lys Asp
145                 150                 155                 160

Phe Leu Ala Asp Gly Gly Arg Phe Ile Phe Pro Phe Pro Glu Ile Glu
                165                 170                 175

Ile Val

<210>  30
<211>  346
<212>  PRT
<213>  Methylobacillus flagellatus

<400>  30

Met Thr Ile Lys Thr Ala Ile Ile Thr Gly Ile Thr Gly Gln Asp Gly
1               5                   10                  15

Ala Tyr Leu Ala Glu Leu Leu Leu Ser Lys Gly Tyr Lys Val Tyr Gly
            20                  25                  30

Thr Phe Arg Arg Thr Ser Ser Val Asn Phe Trp Arg Ile Glu Glu Leu
        35                  40                  45

Gly Ile Glu Asn Asp Pro Asn Leu Leu Leu Val Glu Tyr Asp Leu Thr
    50                  55                  60

Asp Leu Ser Ala Ser Ile Arg Leu Leu Gln Ser Thr Gly Ala Thr Glu
65                  70                  75                  80

Val Tyr Asn Leu Ala Ala Gln Ser Phe Val Gly Val Ser Phe Glu Gln
                85                  90                  95

Pro Ile Thr Thr Ala Glu Ile Thr Gly Val Gly Ala Leu Asn Leu Leu
            100                 105                 110

Glu Ala Ile Arg Leu Val Asn Pro Asn Ile Arg Tyr Tyr Gln Ala Ser
        115                 120                 125

Thr Ser Glu Met Phe Gly Lys Val Gln Ala Ile Pro Gln Thr Glu Asp
    130                 135                 140

Thr Pro Phe Tyr Pro Arg Ser Pro Tyr Gly Val Ala Lys Leu Tyr Ala
145                 150                 155                 160

His Trp Met Thr Val Asn Tyr Arg Glu Ser Tyr Gly Ile Phe Gly Ser
                165                 170                 175

Ser Gly Ile Leu Phe Asn His Glu Ser Pro Leu Arg Gly Arg Glu Phe
            180                 185                 190

Val Thr Arg Lys Ile Thr Asp Ser Val Ala Lys Ile Lys Leu Gly Lys
        195                 200                 205

Leu Asp Cys Leu Glu Leu Gly Asn Leu Asp Ala Lys Arg Asp Trp Gly

210                 215                 220

Tyr Ala Lys Asp Tyr Val Glu Gly Met Trp Arg Ile Leu Gln Ala Asp
225                 230                 235                 240

Glu Pro Asp Thr Phe Val Leu Ala Thr Asn Arg Thr Glu Thr Val Arg
                245                 250                 255

Asp Phe Val Ser Met Ala Phe Lys Gly Ala Gly Ile Ala Val Glu Phe
            260                 265                 270

Lys Gly Lys Gly Glu Asp Glu Val Ala Val Asp Thr Ala Thr Gly Lys
        275                 280                 285

Ile Val Val Arg Val Asn Pro Lys Phe Tyr Arg Pro Ala Glu Val Asp
    290                 295                 300

Leu Leu Ile Gly Ser Pro Ala Lys Ala Lys Ala Lys Leu Gly Trp Glu
305                 310                 315                 320

Pro Lys Thr Thr Leu Glu Gln Leu Cys Gln Met Met Val Glu Ala Asp
                325                 330                 335

Leu Arg Arg Asn Thr Glu Gly Phe Ser Phe
            340                 345

<210>  31
<211>  288
<212>  PRT
<213>  Methylobacillus flagellatus

<400>  31

Met Arg Ile Leu Leu Thr Gly Ala Asp Gly Phe Thr Gly Arg His Phe
1               5                   10                  15

Leu Thr Ala Ala Lys Ala Ala Gly His Glu Val Val Ala Leu Arg Ala
            20                  25                  30

Asp Leu Thr Asp Lys Ala Val Leu Gln Arg Glu Val Glu Ala Ala Arg
        35                  40                  45

Pro Glu Ala Val Val His Leu Ala Ala Ile Ser Phe Val Gly His Ala
    50                  55                  60

Asp Val Ser Ala Phe Tyr Asp Val Asn Val Ile Gly Thr Leu Asn Leu
65                  70                  75                  80

Leu Asp Ala Leu Ala Gly Leu Ser Val Thr Pro Arg Ser Val Leu Leu

85                  90                  95

Ala Ser Ser Ala Asn Val Tyr Gly Asn Ala Ala His Ser Pro Ile Thr
            100                 105                 110

Glu Glu Gln Ala Pro Ala Pro Val Asn His Tyr Ala Met Ser Lys Leu
        115                 120                 125

Ala Met Glu His Met Ala Arg Thr Tyr Leu Asp Arg Leu Pro Leu Phe
    130                 135                 140

Phe Val Arg Pro Phe Asn Tyr Thr Gly Pro Gly Gln Ala Glu Ser Phe
145                 150                 155                 160

Val Ile Pro Lys Leu Val Ala His Phe Ala Cys Lys Ala Gln Thr Val
                165                 170                 175

Glu Leu Gly Asn Leu Asp Val Glu Arg Glu Phe Asn Asp Val Ala Phe
            180                 185                 190

Val Cys Glu Ala Tyr Leu Arg Leu Leu Asp Lys Ala Val Pro Gly Glu
        195                 200                 205

Val Phe Asn Ile Cys Ser Gly Ala Pro Val Thr Leu Arg Ala Val Ile
    210                 215                 220

Gly Met Leu Arg Glu Leu Thr Ala His Asp Ile Glu Val Lys Val Asn
225                 230                 235                 240

Pro Gln Phe Val Arg Asn Asn Glu Ile Lys Thr Leu Cys Gly Asp Pro
                245                 250                 255

Ser Lys Leu Phe Arg Thr Ile Gly Glu Gly Arg Leu Val Pro Leu Ala
            260                 265                 270

Leu Thr Leu Lys Ile Met Leu Glu His Ala Thr Arg Lys Glu Lys Lys
        275                 280                 285

<210>  32
<211>  380
<212>  PRT
<213>  Methylobacillus flagellatus

<400>  32

Met Met Lys Val Gly Phe Gly Val Thr Val Leu Thr Arg Cys Leu Ala
1               5                   10                  15

Leu Gly Gly Ile Asp Gly Ile Gly Ser Tyr Thr Arg Glu Leu Met Lys

20                  25                  30

Arg Leu Glu Ala Asp Pro Ala Val Glu Leu Phe Pro Ile Ser Tyr Gly
        35                  40                  45

Tyr Ser Pro Ser Gln Phe Pro Ile Gly Ser Arg Ala Pro Thr Thr Phe
    50                  55                  60

Gly Arg Tyr Ala Pro Leu Ala Ala Val Ser Ala Val Ser Gly Phe Pro
65                  70                  75                  80

Phe Leu Gly Thr Ala Gln Leu Lys Glu Arg Val Asp Leu Ile His Ala
                85                  90                  95

Pro Asp His Leu Ile Pro Asn Tyr Gly Lys Val Pro Val Ile Ala Ser
            100                 105                 110

Ile Met Asp Ala Ile Pro Leu Ser His Pro Glu Trp Val Ser Pro Lys
        115                 120                 125

Leu Arg Lys Met Lys Asn Ala Leu Trp Lys Arg Ser Ala His Trp Ala
    130                 135                 140

Ser His Ile Ile Thr Ile Ser Glu Tyr Ser Lys Gln Glu Ile Ser Gln
145                 150                 155                 160

His Phe Gly Ile Glu Ala Gln Lys Ile Ser Ala Ile Pro Leu Gly Val
                165                 170                 175

Asp Glu Arg Trp Phe Gln Pro Leu Glu Glu Glu Ala Met Lys Val Val
            180                 185                 190

Leu Arg Arg Tyr Asn Leu Pro Gln Tyr Tyr Phe Leu Val Val Gly Thr
        195                 200                 205

Leu Gln Pro Arg Lys Asn Val Gly Arg Val Ile Glu Ala Tyr Arg Ser
    210                 215                 220

Leu Pro Gln Gly Ile Arg Asn Glu Val Pro Leu Val Ile Val Gly Arg
225                 230                 235                 240

Ala Gly Trp Arg Cys Glu Asp Val Val Glu Met Leu Thr Ser Ser Ser
                245                 250                 255

Ser Gly Asn Ser Leu Arg Trp Leu Lys His Leu Pro Asp Ala Asp Leu
            260                 265                 270

Leu Ala Val Leu Lys Gly Ala Thr Ala Leu Val Phe Pro Ser Leu Tyr
        275                 280                 285

Glu Gly Phe Gly Leu Pro Val Val Glu Ala Phe Ala Ala Gly Thr Pro
    290                 295                 300

Val Ile Thr Ser Asn Thr Thr Ser Leu Pro Glu Val Ala Gly Asp Ala
305                 310                 315                 320

Ala Met Leu Val Asn Pro Leu Asn Val Ser Asp Ile Ala Ala Ala Met
                325                 330                 335

Gln Ser Met Leu Glu Gln Pro Glu Leu Ala Glu Ser Phe Arg Asn Lys
            340                 345                 350

Gly Gln Glu Arg Ala Leu Ala Tyr Ser Trp Asp Lys Thr Ala Ser Met
        355                 360                 365

Thr Leu Asp Val Tyr Lys Lys Thr Arg Glu Ala Ala
    370                 375                 380

<210>  33
<211>  399
<212>  PRT
<213>  Methylobacillus flagellatus

<400>  33

Met Ile Ile Arg Phe Leu Gly Ala Gln Trp Leu Ala Val Gly Phe Val
1               5                   10                  15

Gly Val Ile Ser Leu Ala Val Ser Ile Leu Ile Ala Arg Thr Leu Gly
            20                  25                  30

Pro Asp Ser Phe Gly Val Tyr Ser Val Val Leu Ser Gly Gly Ala Ile
        35                  40                  45

Ala Ala Ile Leu Leu Asp Gly Gly Phe Gly Arg Leu Leu Gln Arg Glu
    50                  55                  60

Arg Ala His Pro Thr Pro Ala Leu Leu Arg Phe Leu Pro Ile Leu Pro
65                  70                  75                  80

Gln Ala Ala Leu Gly His Val Ile Ile Ala Ala Ser Ile Phe Ser Leu
                85                  90                  95

Ile Ala Leu Leu Val Leu Pro Lys His Ala Leu Thr Ile Val Ala Val
            100                 105                 110

Ile Trp Phe Phe Ala Ala Ser Val Thr Asn Gln Phe Gly Leu Asn Ile
        115                 120                 125

Leu Arg Gly Asp Gly Arg Met Val Arg Asp Ala Ser Trp Gln Met Gly
    130                 135                 140

Asn Arg Thr Phe Thr Ala Ala Cys Val Cys Ile Val Ile Leu Met Gly
145                 150                 155                 160

Ala Glu Lys Pro Trp Gln Val Phe Phe Ala Gln Phe Val Gly Ala Thr
                165                 170                 175

Ala Phe Gly Leu Leu Ile Met His Tyr Leu Arg Thr Arg Pro Leu Trp
            180                 185                 190

Lys Ile Pro Pro Thr Leu Tyr Ala Ala Thr Leu Pro Phe Val Trp Leu
        195                 200                 205

Asp Leu Ala Thr Val Leu Tyr Phe Arg Ala Asp Met Leu Leu Leu Glu
    210                 215                 220

Trp Leu Asp Val Ser Lys Phe Glu Val Gly Lys Tyr Gly Val Ala Cys
225                 230                 235                 240

Arg Leu Ile Glu Ala Val Ile Leu Leu Ala Ser Pro Leu Cys Ile Ile
                245                 250                 255

Leu Phe Arg Lys Phe Arg Leu Gly Ser Ala Ser Pro Arg Leu Met Leu
            260                 265                 270

Arg Arg Met Leu Pro Val Leu Ala Gly Ala Leu Met Val Gly Val Phe
        275                 280                 285

Phe Met Val Phe Phe Trp Leu Cys Ser Thr Pro Leu Ile Ala Val Ala
    290                 295                 300

Tyr Gly Glu Ala Tyr Ala Gly Ala Asp His Ile Leu Ala Ile Leu Gly
305                 310                 315                 320

Cys Ala Leu Leu Phe Ile Leu Pro Asn Gly Val Leu Asn Gln Ala Ala
                325                 330                 335

Leu Ala Leu Gly Ile Glu Arg Trp Leu Ala Met Ser Ala Ser Phe Ala
            340                 345                 350

Ala Val Leu Asn Ile Ala Gly Asn Leu Trp Leu Ile Pro Gln Tyr Gly
        355                 360                 365

Leu Ile Gly Ala Ala Trp Met Thr Val Val Thr Glu Ala Val Leu Gly
    370                 375                 380

Cys Cys Val Ala Leu Gly Leu Trp Arg His Arg Asn Arg Ser Val
385                 390                 395

<210>  34
<211>  377
<212>  PRT
<213>  Methylobacillus flagellatus

<400>  34

Met Gln Asp Leu Ala Val Ala His Val Tyr Arg Thr Tyr Phe Pro Asp
1               5                   10                  15

Pro Pro Gly Gly Leu Gln Glu Ala Ile Arg Gln Ile Ala Leu Thr Thr
            20                  25                  30

Gly Met Gln Gly Val Asp Asn Thr Val Phe Thr Leu Ser Pro Gln Pro
        35                  40                  45

Arg Pro Ala Ile Leu Gln Arg Pro Glu Ala Cys Val Val Arg Cys Arg
    50                  55                  60

Ser Trp Ala Ala Pro Ala Ser Cys Asp Leu Gly Gly Ile Gly Ala Phe
65                  70                  75                  80

Leu Ala Phe Ser Arg Leu Ala Glu Lys Ser Asp Val Leu His Tyr Leu
                85                  90                  95

Phe Pro Trp Pro Phe Ala Asp Val Leu His Thr Val Val Arg Pro Gly
            100                 105                 110

Cys Pro Ala Val Met Thr Tyr Ile Ser Asp Val Val Arg Gln Gln Leu
        115                 120                 125

Leu Gly Arg Leu Tyr Ala Pro Leu Met Trp Lys Val Leu Arg Gln Met
    130                 135                 140

Arg Leu Val Val Ala Asn Ser Pro Ala Tyr Ala Arg Thr Ser Pro Val
145                 150                 155                 160

Leu Ser His Pro Glu Ile Arg Glu Lys Val Arg Val Ile Pro Leu Gly
                165                 170                 175

Ile Glu Glu Ser Ser Tyr Pro Lys Ser Gly Asp Glu Glu Ile Leu Gln
            180                 185                 190

Arg Leu Gly Ile Gly Lys Asp Glu Pro Tyr Phe Leu Phe Ile Gly Val
        195                 200                 205

Leu Arg Tyr Tyr Lys Gly Ala His Phe Leu Val Lys Ala Ala Lys Arg
    210                 215                 220

Ile Gly Ala Lys Val Val Phe Ala Gly Ala Gly Pro Glu Gly Ala Arg
225                 230                 235                 240

Leu Lys Ala Leu Ala Gly Glu Val Gly Ala Glu Asn Val Ile Phe Ala
                245                 250                 255

Gly Met Val Thr Asn Ala Glu Lys Val Thr Leu Leu Gln Cys Cys Arg
            260                 265                 270

Ala Leu Val Leu Pro Ser His Leu Arg Ser Glu Ala Tyr Gly Met Val
        275                 280                 285

Leu Val Glu Ala Ala Met Phe Gly Lys Pro Leu Ile Ser Cys Glu Ile
    290                 295                 300

Gly Thr Gly Thr Ser Tyr Val Asn His His Glu Glu Thr Gly Phe Val
305                 310                 315                 320

Val Glu Pro Ala Ser Pro Glu Ala Leu Ala Ser Ala Met Ala Val Leu
                325                 330                 335

Leu Ser Glu Asp Arg Leu Ala Thr Glu Met Gly Ala Ala Ala Arg Gln
            340                 345                 350

Arg Tyr Glu Leu Leu Phe Ser Gly Pro Ala Leu Gly Lys Ala Tyr Ala
        355                 360                 365

Ala Leu Phe Arg Glu Val Asp Ala Ser
    370                 375

<210>  35
<211>  313
<212>  PRT
<213>  Methylobacillus flagellatus

<400>  35

Met Met Gln Leu Leu Val Thr Gly Ala Thr Gly Phe Val Gly Lys Thr
1               5                   10                  15

Leu Cys Ser Ala Leu Arg Arg Gln Ser Phe Pro Val Lys Ala Ile Gly
            20                  25                  30

Arg Ala Gly Thr Ile Arg Ala Asp Ala Leu Gly Asp Gln Val Ala Ser
        35                  40                  45

Ile Asp Gly Gln Thr Asp Trp Ser Leu Ile Leu Asp Gly Ile Asn Ala
    50                  55                  60

Val Ile His Leu Ala Ala Arg Val His Val Met Arg Glu Gln Leu Ala
65                  70                  75                  80

Asp Pro Leu Ala Ala Tyr Arg Glu Thr Asn Val Glu Gly Thr Leu Asn
                85                  90                  95

Leu Ala Arg Gln Ala Ala Lys Ala Gly Val Lys Arg Phe Val Phe Ile
            100                 105                 110

Ser Ser Ile Lys Val Asn Gly Glu Gln Thr Leu Pro Gly Lys Pro Phe
        115                 120                 125

Thr Ala Tyr Asp Val Pro Ala Pro Glu Asp Ala Tyr Gly Ile Ser Lys
    130                 135                 140

Phe Glu Ala Glu Gln Gly Leu Leu Ala Leu Ala Arg Glu Thr Gly Met
145                 150                 155                 160

Asp Val Val Val Ile Arg Pro Pro Leu Val Tyr Gly Pro Gly Val Lys
                165                 170                 175

Gly Asn Phe Ala Ser Met Met Lys Leu Val Lys Ser Gly Ile Pro Leu
            180                 185                 190

Pro Leu Gly Ala Ile His Asn Gln Arg Ser Leu Val Ala Ile Asp Asn
        195                 200                 205

Leu Val Asn Leu Ile Val Thr Cys Ile Asp His Pro Ala Ala Ala Asn
    210                 215                 220

Gln Val Phe Leu Val Ser Asp Gly Glu Asp Leu Ser Thr Ser Asp Leu
225                 230                 235                 240

Leu Arg Arg Leu Ala Lys Ala Ala Gly Val Ser Ser Arg Leu Met Pro
                245                 250                 255

Val Ser Ala Arg Trp Leu Glu Met Gly Leu Arg Met Cys Gly Lys Arg
            260                 265                 270

Leu Ile Ala Gln Arg Leu Leu Gly Ser Leu Gln Val Asp Ile Ser Lys
        275                 280                 285

Thr Arg Glu Leu Leu Gly Trp Val Pro Pro Ile Ser Val Asp Glu Gly
    290                 295                 300

Leu Lys Arg Cys Phe Ser Thr Lys Gly
305                 310

<210>  36
<211>  183
<212>  PRT
<213>  Methylobacillus flagellatus

<400>  36

Met Ile Arg Phe Phe Asp Ile Ile Phe Ser Val Ile Gly Leu Val Leu
1               5                   10                  15

Gly Phe Pro Leu Leu Leu Ser Ile Tyr Thr Leu Gly Leu Phe Asp Thr
            20                  25                  30

Gly Ala Pro Leu Phe Arg Gln Val Arg Val Gly Arg Tyr Arg Gln Pro
        35                  40                  45

Phe Thr Leu Val Lys Phe Arg Thr Met Lys Val Asp Thr Ala Ser Val
    50                  55                  60

Ala Ser His Leu Ala Ser Thr Asn Ser Ile Thr Arg Leu Gly Arg Phe
65                  70                  75                  80

Leu Arg Arg Thr Lys Leu Asp Glu Leu Pro Gln Leu Trp Asn Val Leu
                85                  90                  95

Lys Gly Glu Met Ser Leu Val Gly Pro Arg Pro Asn Leu Phe Asn Gln
            100                 105                 110

Glu Glu Leu Met Ala Glu Arg Glu Leu Arg Gly Val Tyr His Ala Arg
        115                 120                 125

Pro Gly Ile Thr Gly Leu Ala Gln Val Asn Asp Ile Asp Met Ser Thr
    130                 135                 140

Pro Arg Leu Leu Ala Glu Thr Asp Ala Arg Met Leu Arg Glu Met Asn
145                 150                 155                 160

Val Arg Leu Tyr Phe Lys Leu Leu Ile Gln Thr Val Leu Gly Arg Gly
                165                 170                 175

Gln Gly Asp Arg Val Val Arg
            180

<210>  37
<211>  637
<212>  PRT
<213>  Methylobacillus flagellatus

<400>  37

Met Leu His Tyr Phe Pro Ser Phe Lys Trp Ser Asn Tyr Ile Ser Leu
1               5                   10                  15

Lys Asn Pro Arg Ser Leu Ser Ala Trp Ile Tyr Asp Ile Val Ala Ala
            20                  25                  30

Ala Leu Ala Trp Trp Ala Ala Phe Leu Leu Arg Phe Asn Phe Asp Ile
        35                  40                  45

Pro Ala Asn Phe Val Gln Ser Met Leu Asp Ser Ala Ala Trp Val Ile
    50                  55                  60

Pro Leu Gln Ala Phe Val Phe Leu Leu Ala Gly Leu Tyr Lys Gly Val
65                  70                  75                  80

Trp Arg Phe Ala Ser Leu Pro Asp Leu Gln Arg Leu Val Arg Ala Ile
                85                  90                  95

Ala Ile Ser Ala Val Leu Val Val Ala Ile Leu Phe Met Phe Lys Pro
            100                 105                 110

His Gly Ile Val Pro Arg Ser Val Leu Ile Leu Asp Pro Met Leu Leu
        115                 120                 125

Leu Leu Leu Met Gly Gly Ser Arg Phe Ala Tyr Arg Ala Trp Lys Glu
    130                 135                 140

His Arg Leu Tyr Gly Ser Ala Gln Phe Ser Gly Lys Pro Val Leu Val
145                 150                 155                 160

Leu Gly Ala Gly Glu Ala Ala Ile Ala Leu Leu Lys Asp Leu Ser Lys
                165                 170                 175

Ser Arg Glu Trp His Val Val Gly Leu Leu Asp Asp Lys Lys Thr Met
            180                 185                 190

His Gly Arg Glu Leu Leu Gly Val Asn Val Leu Gly Ser Ile Ala Gln
        195                 200                 205

Leu Pro Gln Ile Ala Ala Asp Leu Ser Val Asn His Val Ile Val Ala
    210                 215                 220

Met Pro Ser Ala Arg His Gln Arg Arg Arg Glu Ile Val Glu Leu Ala
225                 230                 235                 240

Ser Arg Asn Gly Leu Thr Ala Leu Thr Val Pro Ser Phe Asp Asp Leu
                245                 250                 255

Met Ser Gly His Leu Ser Met Ala Gln Ile Arg Pro Val Glu Val Glu
            260                 265                 270

Asp Leu Leu Gly Arg Glu Pro Val Ser Leu Asp Gly Glu Gly Leu Gln
        275                 280                 285

His Leu Ile Glu Gly Gln Val Leu Leu Ile Thr Gly Ala Gly Gly Ser
    290                 295                 300

Ile Gly Ser Glu Leu Cys Arg Gln Val Leu Lys Tyr Arg Pro Ala Cys
305                 310                 315                 320

Leu Val Cys Leu Asp Gln Ser Glu Tyr Ala Leu Tyr Arg Leu Glu Gln
                325                 330                 335

Glu Phe Asp Asn Leu Glu Phe Asp Thr Arg Ile Glu Tyr Val Val Gly
            340                 345                 350

Asp Val Arg Asn Ala Arg Arg Val Gly Gln Val Leu Gly Arg Tyr Met
        355                 360                 365

Pro Lys Leu Val Phe His Ala Ala Ala Tyr Lys His Val Pro Leu Met
    370                 375                 380

Glu Asn Arg Asn Val Ala Glu Ser Leu Ser Asn Asn Val Ser Gly Thr
385                 390                 395                 400

Tyr Thr Leu Ala Gln Ala Cys Lys Thr Ala Gly Val Ala Lys Phe Val
                405                 410                 415

Leu Ile Ser Thr Asp Lys Ala Val Asn Pro Thr Asn Val Met Gly Ala
            420                 425                 430

Ser Lys Arg Leu Ala Glu Met Ile Cys Gln Gly Leu Gln Glu Glu Ser
        435                 440                 445

Gly Thr Arg Phe Ile Val Val Arg Phe Gly Asn Val Leu Gly Ser Ser
    450                 455                 460

Gly Ser Val Ile Pro Lys Phe Arg Glu Gln Ile Ala Ala Gly Gly Pro

465                 470                 475                 480

Ile Thr Val Thr His Pro Glu Ile Thr Arg Tyr Phe Met Ser Ile Pro
                485                 490                 495

Glu Ala Cys Gln Leu Val Met Gln Ala Gly Leu Met Gly Gly Gly Gly
            500                 505                 510

Glu Ile Phe Val Leu Asp Met Gly Asp Pro Val Arg Ile Val Asp Leu
        515                 520                 525

Ala Arg Asp Met Ile Arg Leu Ser Gly Leu Asn Glu Asp Asp Ile Gln
    530                 535                 540

Ile Val Tyr Ser Gly Leu Arg Pro Gly Glu Lys Leu Tyr Glu Glu Leu
545                 550                 555                 560

Leu Ala Asp Asp Glu Leu Thr Val Pro Thr Pro His Asp Lys Leu Arg
                565                 570                 575

Ile Ala Val Ala Arg Ser Val Asp Gly Ala Trp Val Gln Ala Leu Leu
            580                 585                 590

Lys Trp Ile Asp Arg Ala Ser Leu Ile Asp Glu Ser Val Ile Lys Gln
        595                 600                 605

Glu Leu Gly Met Trp Val Glu Glu Tyr Ala Gly Asp Ile His Ala Asn
    610                 615                 620

Pro Ser Met Met Phe Pro Pro Glu Ser Ser Thr Ile His
625                 630                 635

<210>  38
<211>  291
<212>  PRT
<213>  Methylobacillus flagellatus

<400>  38

Met Ala Lys Lys Ile Thr Lys Ala Val Phe Pro Val Ala Gly Leu Gly
1               5                   10                  15

Thr Arg Phe Leu Pro Ala Thr Lys Ala Ser Pro Lys Glu Met Leu Pro
            20                  25                  30

Ile Val Asp Lys Pro Leu Ile Gln Tyr Ala Ala Glu Glu Ala Val Ala
        35                  40                  45

Ala Gly Ala Thr Glu Leu Ile Phe Ile Ile Gly Arg Asn Lys Arg Thr

50                  55                  60

Ile Pro Asp His Phe Asp Lys Ser Phe Glu Leu Glu Ala Thr Leu Glu
65                  70                  75                  80

Ala Ser Lys Lys Thr Lys Val Leu Glu Glu Ile Arg Asn Ile Leu Pro
                85                  90                  95

Lys Asn Val Ser Cys Ile Tyr Ile Arg Gln Ala Glu Ala Leu Gly Leu
            100                 105                 110

Gly His Ala Val Leu Leu Ala Lys Ser Val Val Gly Ser Glu Pro Phe
        115                 120                 125

Ser Val Ile Leu Ala Asp Asp Leu Ile Asp Ala Asp Lys Pro Ala Met
    130                 135                 140

Gln Gln Met Thr Glu Leu Phe Asp Gln Glu Gly Val Ser Ile Leu Gly
145                 150                 155                 160

Val Glu Asp Val Glu Pro Ser Glu Thr Ala Ser Tyr Gly Ile Val Asp
                165                 170                 175

Ala Thr Glu Val Ser Gly Pro Asn Val Leu Gln Leu Asn Gly Ile Val
            180                 185                 190

Glu Lys Pro Gln Pro Glu Glu Ala Pro Ser Asn Leu Ala Val Val Gly
        195                 200                 205

Arg Tyr Ile Leu Thr Pro Glu Ile Phe Glu Cys Leu Glu Asn Val Lys
    210                 215                 220

Pro Gly Lys Gly Gly Glu Ile Gln Leu Thr Asp Gly Ile Ala Ala Leu
225                 230                 235                 240

Met Glu Lys Gln Lys Ile Leu Ala Tyr Arg Phe Asp Gly Lys Arg Tyr
                245                 250                 255

Asp Cys Gly Ser Lys Leu Gly Tyr Val Lys Ala Asn Val Glu Tyr Ser
            260                 265                 270

Leu Lys His Glu Glu Phe Gly Lys Glu Phe Ala Gln Tyr Leu Arg Asp
        275                 280                 285

Leu Thr Lys
    290

<210>  39
<211>  272
<212>  PRT
<213>  Methylobacillus glycogenes

<400>  39

Met Leu Gln Gln Phe Ser Ile Gly Val Arg Glu His His His Lys Glu
1               5                   10                  15

Leu Thr Pro Asp Gln Ala Gly Ala Phe Leu Glu Val Val Asp Ala Ser
            20                  25                  30

Leu Arg Ile Lys Lys Lys Ala Gln Phe Phe Ser Trp Leu Gln Gly Tyr
        35                  40                  45

Phe Gln Phe Leu Leu Pro His Glu Val Leu Leu Cys Gly Leu Ser Ile
    50                  55                  60

Gly Ala Gly Arg Pro Phe Thr Leu Glu Thr Phe Ser Ser Thr Arg Tyr
65                  70                  75                  80

Val Thr Asp Thr His Thr Ala Val Ser Met Gln Glu Thr Asp Gly Val
                85                  90                  95

Val Leu Arg Ala Ile Gln Ser Trp Gln Glu Thr Ser Leu Pro Val Leu
            100                 105                 110

Val Asp Pro Tyr Leu Arg Pro Gly Ser Phe Gly Asn Phe Thr Val Pro
        115                 120                 125

Glu Ile Asp Gln Ala Asn Leu Gln Ala Ser Glu Leu Arg Asn Leu Ala
    130                 135                 140

Ala Tyr Gly Leu Ser Ser Ala Asp Gly Ser Ile Ser Ser Phe Phe Cys
145                 150                 155                 160

Phe Ser Arg Val Ala Gly Glu Leu Asn Gly Asn His Ala Tyr Leu Leu
                165                 170                 175

Glu Leu Leu Val Pro Tyr Leu His Ser Val Leu Met Arg Val Ser Asp
            180                 185                 190

Ser Ala Thr Ala Ser Ala Asp Met Leu Gly Asp Ser Ser Val Ala Gly
        195                 200                 205

Leu Ile Thr Met Arg Glu Lys Glu Ile Leu His Trp Val His Thr Gly
    210                 215                 220

Lys Ser Asn Trp Glu Ile Ala Thr Ile Leu Gln Ile Ser Pro Leu Thr
225                 230                 235                 240

Val Lys Asn His Met Gln Asn Ile Leu Arg Lys Leu Asp Val Gln Asn
                245                 250                 255

Arg Ser His Ala Ala Ser Lys Ala Phe Arg Leu Gly Leu Ile Arg Asn
            260                 265                 270

<210>  40
<211>  379
<212>  PRT
<213>  Methylobacillus glycogenes

<400>  40

Met Gly Tyr Ser Asp Pro Val Leu Ala Arg Glu Gly Asp Val Ile Arg
1               5                   10                  15

Pro Tyr Val Ser Ala Thr Tyr Val Tyr Asp Asp Asn Leu Arg Arg Arg
            20                  25                  30

Ser Asp Lys Glu Ser Asp Thr Ser Val Val Gly Arg Val Gly Ile Ile
        35                  40                  45

Leu Asp Lys Asp Ile Ser Gln Gln Asn Phe Tyr Ala Asn Ile Gly Val
    50                  55                  60

Asn Glu Thr Lys Tyr Asn Lys Asn Ser Glu Leu Asp Asn Thr Gly Lys
65                  70                  75                  80

Asp Ile Val Ala Arg Trp Asn Trp Ala Val Gly Ser Gln Phe Arg Gly
                85                  90                  95

Lys Val Glu Tyr Tyr Lys Asn Glu Ala Met Leu Pro Phe Ala Asp Phe
            100                 105                 110

Arg Gly Val Glu Leu Asn Met Arg Ile Gln Glu Arg Lys Thr Phe Glu
        115                 120                 125

Ala Asn Trp Arg Phe His Ser Ser Trp Arg Ala Arg Phe Gly Val Gln
    130                 135                 140

Arg Gln Asp Val Glu Tyr Asp Ser Gln Thr Leu Asn Ala Ala Ser Phe
145                 150                 155                 160

Gln Glu Asn Ser Gln His Val Gly Leu Asp Tyr Leu Ala Glu Ser Gly
                165                 170                 175

Ser Lys Val Gly Leu Val Phe Thr Asn Gly Arg Gly Thr Arg Pro Phe
        180                 185                 190

Asp Arg Pro Ala Phe Phe Pro Gly Ile Gly Thr Val Phe Val Asn Asn
    195                 200                 205

Asp Tyr Arg Gln Gln Ser Leu Lys Ala Asp Ile Leu Trp Leu Tyr Ser
    210                 215                 220

Ala Lys Thr Arg Phe Glu Phe Leu Gly Gly Tyr Val Asp Arg Lys His
225                 230                 235                 240

Asp Ser Phe Ser Gln Arg Asp Phe Ser Gly Phe Asn Ala His Gly Asn
                245                 250                 255

Met Val Trp Ser Ile Thr Asp Lys Thr Ser Phe Lys Thr Ala Ala Trp
        260                 265                 270

Arg Asp Ala Asn Ala Ile Ser Ile Val Thr Ala Ser Tyr Val Ile Asp
    275                 280                 285

Ser Gly Ala Ser Ala Ser Ile Ser Tyr Ser Ala Thr Ser Lys Ile Leu
    290                 295                 300

Leu Ser Thr Ser Ala Arg Tyr Gln Gln Leu Lys Phe Glu Gly Asp Ile
305                 310                 315                 320

Thr Gly Gln Asp Gln Gly Arg Thr Asp Lys Asn Lys Val Phe Ser Phe
                325                 330                 335

Gly Val Thr Tyr Ser Pro Leu Glu Ser Leu Ser Phe Thr Thr Gly Leu
        340                 345                 350

Asn Arg Ser Leu Arg Asp Ser Thr Ser Pro Ile Leu Asn Tyr Asn Ser
    355                 360                 365

Thr Ser Ile Ser Leu Thr Gly Arg Tyr Glu Phe
    370                 375

<210>  41
<211>  449
<212>  PRT
<213>  Methylobacillus glycogenes

<400>  41

Met Thr Val Asn Asp Ile Pro Leu Val Ala Phe Phe Lys Gln Leu Leu
1               5                   10                  15

Asp Pro Leu Ile Ile Leu Gly Thr Leu Tyr Val Phe Thr Leu Leu Phe
            20                  25                  30

Gly Glu Pro Phe Asn Gly Tyr Tyr Leu Ile Leu Ala Leu Leu Ala Tyr
        35                  40                  45

Phe Val Ser Ser Asn Val Tyr Glu Arg Leu Asp Leu Tyr Arg Thr Trp
    50                  55                  60

Arg Ser Gly Lys Ile Leu Gly Tyr Ile Arg Asp Ile Leu Phe Gly Trp
65                  70                  75                  80

Gly Ile Val Ile Ala Val Leu Met Phe Ile Gly Tyr Ala Ala Asp Leu
                85                  90                  95

Ser Ser Gln Tyr Leu Asp Glu Met Ile Tyr Ala Trp Phe Ala Ser Val
            100                 105                 110

Pro Met Ile Phe Ile Val Thr His Leu Ala Ile Arg Lys Ile Ala Ser
        115                 120                 125

Asp Leu His Lys Asn Gly Glu Val Arg Ser Val Val Ile Ile Gly Ala
    130                 135                 140

Asn Asp Ile Gly Val Arg Leu Ile Asn Arg Ile Thr Asp Asp Pro Tyr
145                 150                 155                 160

Leu Phe Met Glu Val Lys Gly Ile Phe Asp Asp Arg Glu Val Ala Arg
                165                 170                 175

His Pro Lys Gly Met Arg Asn Leu Pro Leu Leu Gly Ser Met Ser Glu
            180                 185                 190

Ile Ala Pro Tyr Val Arg Ala Asn Asn Thr Gln Ile Ile Phe Ile Ser
        195                 200                 205

Leu Pro Met Ser Ala Gln Pro Arg Ile Arg Gln Leu Phe Glu Glu Leu
    210                 215                 220

Gln Asp Thr Thr Ala Ser Ile Tyr Phe Val Pro Asp Ile Tyr Ile Phe
225                 230                 235                 240

Asp Leu Val Gln Ala Arg Phe Asp Asp Val Ala Gly Val Pro Val Val
                245                 250                 255

Ala Ile Leu Glu Thr Pro Phe Thr Gly Phe Asn Ser Leu Ile Lys Arg
            260                 265                 270

Thr Ser Asp Ile Ile Leu Ala Ser Leu Ile Leu Leu Met Leu Ser Pro
        275                 280                 285

Ile Met Leu Thr Leu Ala Leu Ala Val Lys Leu Thr Ser Pro Gly Pro
    290                 295                 300

Val Ile Phe Lys Gln Arg Arg Tyr Ala Glu Trp Gly Asn His Cys Leu
305                 310                 315                 320

Ile Pro Leu His Glu Ser Asn Gly Arg Trp Gln Gly Ser Thr Gly Ala
                325                 330                 335

Glu Ala Arg Ser Ala Ile Asp Thr Asp Trp Trp Phe Leu Ala Gln Glu
            340                 345                 350

Leu Thr Gly Ala Ala Ala Val His Gln Cys Val Ala Arg Ser Asp Glu
        355                 360                 365

Tyr Arg Gly Ala Thr Ala Cys Arg Phe Ser Ala Ile Ser Gln Ala Asp
    370                 375                 380

Ser Trp Leu Tyr Ala Ala Ala Gln Gly Gln Ala Trp Asn Asn Arg Leu
385                 390                 395                 400

Gly Ala Gly Glu Trp Phe Thr Trp Asn Tyr Leu Gly Gln Asp Gly Val
                405                 410                 415

Pro Tyr Arg Val Leu Gly Leu Phe Ala Pro Leu Val Pro Gly Phe Leu
            420                 425                 430

Leu Asp Tyr Arg Gln Asp Gly Ala Gly Cys Val Gln Lys Arg Arg Leu
        435                 440                 445

Leu

<210>  42
<211>  310
<212>  PRT
<213>  Methylobacillus glycogenes

<400>  42

Met Asn Asn Gly Phe Val Thr Asn Phe Lys Gln Ser Arg Thr Leu Cys
1               5                   10                  15

Val Gly Ala Val Ile Val Leu Ala Phe Ala Leu Ser Gly Cys Gly Asp
            20                  25                  30

Lys His Glu Ser Lys Ala Ser Gln Ser Leu Val Lys Val Asn Gly Lys
        35                  40                  45

Glu Ile Thr Val His Gln Leu Asn Gln Glu Leu Gly Arg Ala Asn Ile
    50                  55                  60

Gln Glu Asn Gln Arg Glu Gln Ala Thr Gln Gln Ile Leu Thr Ala Leu
65                  70                  75                  80

Ile Asp Arg Gln Leu Leu Glu Glu Ala Ala Leu Lys Ala Lys Val Asp
                85                  90                  95

Arg Asp Pro Asn Val Met Thr Thr Ile Glu Arg Ala Lys Ser Gln Ile
            100                 105                 110

Ile Ala Gln Ala Tyr Leu Gln Ser Lys Leu Gly Ser Val Ala Lys Pro
        115                 120                 125

Ser Lys Thr Glu Ile Glu Asn Phe Tyr Asn Glu Asn Pro Gln Phe Phe
    130                 135                 140

Ser Lys Arg Arg Met Val Glu Met Asp Gln Leu Leu Ile Asp Thr Lys
145                 150                 155                 160

Phe Ala Asn Asp Glu Leu Lys Thr Ala Leu Gly Lys Leu Lys Thr Leu
                165                 170                 175

Gln Glu Val Ala Ala Trp Met Asp Ala His Asn Ile Gln Tyr Asp Gln
            180                 185                 190

Gly Gln Val Ser Arg Ser Val Ala Glu Leu Pro Pro Gln Ile Ser Glu
        195                 200                 205

Arg Leu Lys Ala Glu Asp Lys Gly Lys Leu Phe Val Ile Gly Met Gly
    210                 215                 220

Pro Arg Thr Met Leu Ile Met Met Asn Glu Ile Lys Asp Ser Pro Val
225                 230                 235                 240

Ser Leu Ala Glu Ala Thr Pro Gln Ile Glu Asn Asn Leu Leu Asn Lys
                245                 250                 255

Ala Arg Lys Glu Val Ala Glu Asn Glu Val Lys Arg Leu Lys Ala Asp
            260                 265                 270

Ala Lys Ile Glu Tyr Ile Asp Lys Lys Met Glu Thr Ala Thr Glu Thr
        275                 280                 285

Lys Ala Ala Lys Lys Lys Pro Ser Ser Thr Glu Asp Asp Ile Ala Ser
    290                 295                 300

Gly Val Ser Gly Leu Lys
305                 310

<210>  43
<211>  264
<212>  PRT
<213>  Methylobacillus glycogenes

<400>  43

Met Lys Lys Leu Met Thr Trp Phe Phe Leu Leu Met Leu Ser Leu Thr
1               5                   10                  15

Pro Phe Val Thr His Ala Ala Glu Gly Asp Ile Leu Leu Gly Ala Gly
            20                  25                  30

Asp Val Leu Lys Ile Ser Val Phe Glu Gln Pro Asp Leu Ser Leu Glu
        35                  40                  45

Val Arg Val Ser Glu Ser Gly Ser Ile Thr Tyr Pro Leu Ile Gly Glu
    50                  55                  60

Val Arg Val Gly Gly Gly Thr Val Ala Ala Ala Glu Gln Arg Ile Ala
65                  70                  75                  80

Thr Met Leu Glu Lys Gly Gly Phe Leu Lys Ser Ala His Val Asn Ile
                85                  90                  95

Ile Val Ala Gln Leu Gln Ser Leu Gln Val Ser Ile Leu Gly Gln Val
            100                 105                 110

Asn Arg Pro Gly Arg Tyr Pro Ile Asp Ser Ala Lys Thr Leu Ala Asp
        115                 120                 125

Val Leu Ala Leu Ala Gly Gly Val Ser Pro Glu Gly Gly Asp Leu Ile
    130                 135                 140

Thr Leu Val His Asn Val Asp Gly Lys Ala Val Arg Asp Gln Ile Asp
145                 150                 155                 160

Leu Ala Asp Met Met Arg Thr Gly Asp Leu Ser Lys Asn Ile Arg Val
                165                 170                 175

Ala Gly Gly Asp Ile Ile Tyr Val Glu Arg Phe Asp Lys Phe Tyr Ile
            180                 185                 190

Tyr Gly Gln Val Gln Arg Ala Ser His Tyr Arg Leu Glu His Glu Met
        195                 200                 205

Thr Val Leu Gln Ala Leu Ser Val Gly Gly Gly Leu Ser Pro Arg Gly
    210                 215                 220

Thr Glu Lys Gly Val Lys Ile Lys Arg Arg Asp Ala Ser Gly His Ile
225                 230                 235                 240

Glu Val Ile Thr Ala Lys His Asp Asp Leu Ile Lys Pro Asn Asp Val
                245                 250                 255

Ile Tyr Val Gln Glu Ser Phe Phe
            260

<210>  44
<211>  462
<212>  PRT
<213>  Methylobacillus glycogenes

<400>  44

Met Ser Phe Ser Gln Leu Leu Leu Ile Phe Lys Ala Arg Tyr Lys Ile
1               5                   10                  15

Leu Leu Ser Thr Ile Val Val Ala Leu Val Val Ala Ile Ala Leu Asn
            20                  25                  30

Ile Phe Leu Pro Asn Ser Tyr Lys Ala Thr Thr Ser Ile Leu Val Asn
        35                  40                  45

Tyr Lys Gly Val Asp Pro Val Thr Gly Met Ala Val Pro Ala Gln Leu
    50                  55                  60

Leu Pro Gly Tyr Met Gly Thr Gln Val Asp Ile Ile Thr Ser Lys Lys
65                  70                  75                  80

Ile Ala Lys Arg Val Val Thr Thr Leu Lys Leu Asn Glu Ser Ser Glu
                85                  90                  95

Val Val Glu Ser Phe Asn Asp Ser Thr Gly Gly Glu Gly Asp Ile Val
            100                 105                 110

Asp Trp Leu Ala Asp Leu Leu Ile Ser Arg Ile Glu Val Gln Pro Ala
        115                 120                 125

Arg Asn Ser Ser Val Leu Gln Leu Glu Phe Asp Gly Ala Asn Pro Lys
    130                 135                 140

Phe Ala Ala Val Val Ala Asn Ser Phe Ala Glu Glu Tyr Ile Lys Ala
145                 150                 155                 160

Thr Ile Glu Leu Lys Val Glu Pro Ser Lys Lys Ala Ala Glu Phe Tyr
                165                 170                 175

Thr Gly Gln Leu Lys Asp Leu Arg Ala Asp Ile Thr Arg Ala Gln Gln
            180                 185                 190

Lys Leu Ser Asp Tyr Gln Gln Asp Lys Ser Ile Val Ser Val Asp Glu
        195                 200                 205

Arg Leu Asp Ile Glu Arg Ala Lys Leu Asn Glu Leu Ser Ser Gln Leu
    210                 215                 220

Val Met Ser Gln Gly Gln Thr Leu Glu Ala Gln Ser Arg Gln Thr Gly
225                 230                 235                 240

Ala Gln Gly Ser Asn Ala Tyr Gly Ser Pro Asp Val Ala Ser Asn Pro
                245                 250                 255

Met Val Leu His Leu Lys Thr Glu Ile Ala Arg Ser Glu Ser Arg Phe
            260                 265                 270

Ala Asp Ile Ser Gln Lys Tyr Glu Arg Asn His Pro Leu Tyr Gln Gly
        275                 280                 285

Ala Lys Ala Glu Leu Asp Asn Leu Lys Ser Glu Leu Ser Lys Gln Ile
    290                 295                 300

Gln Ala Ala Thr Val Asn Ile Ser Ser Asn Tyr Arg Ile Leu Lys Gln
305                 310                 315                 320

Arg Glu Ser Glu Ile Arg Asn Ala Leu Ala Glu Gln Lys Thr Lys Val
                325                 330                 335

Met Leu Leu Asn Lys Asp Arg Asp Ala Leu Thr Val Leu Thr Arg Glu
            340                 345                 350

Val Glu Gly Ala Gln Arg Ala Tyr Glu Leu Ala Thr Gln Arg Tyr Thr
        355                 360                 365

Gln Ile Asn Leu Glu Gly Gln Ser Asn Gln Ala Asp Val Ser Ile Leu
    370                 375                 380

Asn Pro Ala Thr Pro Pro Leu Lys Pro Ser Ser Pro Lys Lys Ile Leu

385                 390                 395                 400

Asn Val Ala Val Ala Leu Val Leu Gly Thr Ile Phe Gly Leu Leu Phe
                405                 410                 415

Ser Leu Leu Ala Glu Leu Thr Asn Glu Arg Ile Arg Ser Glu Arg Val
            420                 425                 430

Leu Val Glu Ile Val Gly Ala Pro Leu Leu Gly Val Ile Asp Lys Asn
        435                 440                 445

Lys Thr His Ser Arg Ile Gln Arg Leu Leu Ala Lys Lys Pro
    450                 455                 460

<210>  45
<211>  292
<212>  PRT
<213>  Methylobacillus glycogenes

<400>  45

Met Thr Asn Asp Val Ser Asn Thr Ser Ser Ile Pro Ala Leu Asn Ala
1               5                   10                  15

Asp Phe Asn Ile Gly Gly Met Leu Leu Lys Met Gly Lys Ile Thr Pro
            20                  25                  30

Gln Asp Ala Glu Arg Ile Leu Arg Val Gln Lys Glu Arg Gly Val Arg
        35                  40                  45

Phe Gly Glu Ala Ala Glu Ser Leu Gly Leu Val Ser Ser Ala Asp Ile
    50                  55                  60

Asn Tyr Val Leu Ser Lys Gln Phe Ala Tyr Pro Tyr Leu Met Asp Gly
65                  70                  75                  80

Arg Gly Asp Phe Ser Glu Asn Leu Val Ala Ala Tyr Asp Pro Phe Ser
                85                  90                  95

Ile Asn Val Glu Lys Tyr Arg Ala Ile Arg Ser Gln Leu Met Leu Tyr
            100                 105                 110

Trp Phe Ala Gln Gly Lys Lys Ser Leu Ser Phe Ser Ser Val Tyr Asp
        115                 120                 125

Lys Asp Asp Thr Ser Asp Leu Val Ala Asn Leu Gly Val Val Phe Ser
    130                 135                 140

Gln Leu Gly Glu Lys Thr Leu Ile Ile Asp Ala His Leu Asp Ser Pro

145                 150                 155                 160

Arg Gln Asn Arg Ile Phe Asn Ile Lys Ser Thr Ala Gly Leu Ser Asp
                165                 170                 175

Ile Leu Ala Gly Arg Val Thr Asp Tyr Ser Ser Ser Ile Thr Arg Leu
            180                 185                 190

Val Glu Phe Glu His Leu Ser Ile Leu Ser Ser Gly Thr Ile Pro Pro
        195                 200                 205

Asn Pro Arg Glu Leu Leu Gly Arg Gly Phe Leu Lys Gln Phe Ile Thr
    210                 215                 220

Ala Ile Ala Asn Asp Tyr Asp Val Ile Leu Val Asp Ala Pro Ser Phe
225                 230                 235                 240

Val Arg His Ser Glu Pro Leu Ser Val Ala Ser Ala Thr Glu Gly Val
                245                 250                 255

Val Leu Leu Ala Lys Arg His Val Ser Lys Ala Asn Asp Leu Ser Lys
            260                 265                 270

Val Val Asp Gln Leu Ser Lys Ile Arg Val Pro Val Val Gly Thr Val
        275                 280                 285

Leu Thr Glu Asn
    290

<210>  46
<211>  299
<212>  PRT
<213>  Methylobacillus glycogenes

<400>  46

Met Ser Leu Asn Gln Ser Ile Ala Val Ala Ser Thr Gln Leu Arg Arg
1               5                   10                  15

Tyr Gln His Val Ile Pro Leu Leu Val Gly Leu Leu Phe Leu Phe Val
            20                  25                  30

Pro Thr Tyr Leu Asn Leu Thr Asn Thr Val Trp Gln Met Gln Asp Gln
        35                  40                  45

Ala His Gly Pro Leu Ile Leu Met Val Val Leu Tyr Leu Val Trp Val
    50                  55                  60

Asp Lys Glu Ile Leu Leu Asp Thr Ala Asn Leu Arg Gln Ser Gly Arg

65                  70                  75                  80

Gly Val Gly Leu Leu Leu Phe Leu Phe Gly Leu Leu Val Tyr Ile Ile
                85                  90                  95

Gly Arg Ser Gln Asp Leu Leu Val Phe Asp Val Gly Ser Gln Ile Pro
            100                 105                 110

Val Leu Ala Gly Leu Leu Leu Ile Phe Tyr Lys Ala Lys Thr Leu Lys
        115                 120                 125

Arg Phe Trp Phe Pro Leu Phe Phe Leu Phe Phe Met Ile Pro Leu Pro
    130                 135                 140

Gly Ser Met Leu Asp Val Val Thr Leu Pro Met Lys Val Ala Val Ser
145                 150                 155                 160

Tyr Val Ala Glu His Val Leu Tyr Trp Phe Asp Tyr Pro Ile Ala Arg
                165                 170                 175

Ser Gly Val Ile Ile Gln Ile Gly Gln Tyr Gln Leu Leu Val Ala Asp
            180                 185                 190

Ala Cys Ala Gly Met His Thr Leu Ile Ser Leu Glu Ala Leu Gly Leu
        195                 200                 205

Leu Tyr Leu Asn Ile Val Arg His Asp Ser Leu Ala Arg Asn Leu Leu
    210                 215                 220

Leu Ala Leu Leu Ile Ile Pro Ile Ser Phe Ser Ala Asn Val Ile Arg
225                 230                 235                 240

Val Ile Val Leu Thr Leu Ile Thr Tyr Tyr Phe Gly Asp Glu Val Gly
                245                 250                 255

Gln Gly Phe Val His Gly Phe Ala Gly Ile Leu Leu Phe Val Val Ala
            260                 265                 270

Leu Thr Leu Ile Ile Ser Val Asp Ser Leu Ile Gln Tyr Phe Val Gly
        275                 280                 285

Lys His Thr Gln Arg Lys Ala Arg Leu Gln Ser
    290                 295

<210>  47
<211>  228
<212>  PRT
<213>  Methylobacillus glycogenes

<400>  47

Met Ser Val Arg Leu Ile Asn Ile Leu Ala Val Cys Met Val Val Ser
1               5                   10                  15

Ser Gly Leu Ala Leu Ala Leu Thr Pro Asp Leu Ser Ser Val Arg Asp
            20                  25                  30

Asp Pro His Leu Glu Ser Phe Val Pro Ser Ser Phe Gly Gln Trp Gln
        35                  40                  45

Glu Val Lys Thr Asn Gln Val Gln Met Asp Leu Thr Val Arg Arg Asp
    50                  55                  60

Gly Glu Pro Ser Ile Asp Asn Pro Tyr Asp Glu Val Leu Met Arg Ser
65                  70                  75                  80

Tyr Ala Asn Glu Lys Gly Gln Val Ile Met Leu Ala Leu Ala Tyr Gly
                85                  90                  95

Lys Leu Gln Arg Gln Glu Val Lys Val His Arg Pro Glu Leu Cys Tyr
            100                 105                 110

Thr Ala Gln Gly Phe Ser Ile Arg Asn Gln Gln Asp Thr Ala Val Asn
        115                 120                 125

Val Gly Ala Gly Ala Thr Pro Ile Pro Ser Lys Ala Leu Val Ala Ala
    130                 135                 140

Ser Arg Gln Arg Glu Glu Leu Val Leu Tyr Trp Ile Arg Ile Gly Asp
145                 150                 155                 160

Met Ile Ser Lys Ser Ala Trp Glu Thr Arg Leu Tyr Ile Val Glu Gln
                165                 170                 175

Gly Leu Lys His Gln Leu Val Asp Gly Ile Leu Val Arg Thr Ser Gln
            180                 185                 190

Ile Leu Gly Glu Asp Asp Ala Glu Gln Ser Leu Gln Leu Gln Gln Gln
        195                 200                 205

Phe Leu His Asp Leu Val Gln Ala Val Pro Arg Asp Lys Arg Lys Tyr
    210                 215                 220

Leu Ile Pro Ser
225

<210>  48
<211>  378
<212>  PRT
<213>  Methylobacillus glycogenes

<400>  48

Met Lys Ile Leu Phe Leu Ser His Thr Ala Ala Gly Gly Glu Phe Val
1               5                   10                  15

Val Gly Ser His Gln Leu Ser Lys Gln Phe Ala Leu Gln Gly His Glu
            20                  25                  30

Val Val His Leu Ser Pro Pro Val Thr Pro Ala His Leu Thr Leu Leu
        35                  40                  45

Gln Lys Thr Pro Phe Glu Arg Val Arg Phe Lys Arg Trp Met Lys Ala
    50                  55                  60

Gly Asp Val Ile Asn His Val Thr Asp Ile Ile Pro Phe Ser Leu Ile
65                  70                  75                  80

Pro Trp Gly Ile Ala Thr Arg Leu Gly Ser Gly Ser Asp Ile Phe Gly
                85                  90                  95

Tyr Phe Leu Lys Phe Ser Ala Leu Arg Gln Leu Arg Lys His Gln Phe
            100                 105                 110

Ser Asn Pro Asp Val Val Leu Ile Asp Glu Pro Arg Leu Gly Tyr Leu
        115                 120                 125

Leu Lys Tyr Phe Pro Lys Ser Lys Ile Val Tyr Arg Pro Thr Asp Ile
    130                 135                 140

Tyr His Glu Ile Arg Asn Asp Pro Ser Ile Val Asp Ala Glu Lys Gly
145                 150                 155                 160

Leu Ile Ser Gln Val His Gly Phe Ile Ala Thr Ser Glu Pro Val Arg
                165                 170                 175

Gln His Leu Asn Arg Leu Gly Val Asp Glu Val Leu Leu Leu Gln Asn
            180                 185                 190

Gly Val Asp Leu Asp His Tyr Leu Asn Phe Thr Lys Ser Lys Leu Asp
        195                 200                 205

Leu Pro Pro Glu Pro Arg Ala Ile Tyr Ala Gly Ala Leu Asp His Arg
    210                 215                 220

Phe Gly Phe Asp Val Leu Lys Ser Ala Ala Thr Lys His Gln Lys Ile
225                 230                 235                 240

Ser Phe Leu Leu Ala Gly Pro Val Glu Glu Lys Tyr Lys Ala Ala Phe
                245                 250                 255

Ser Asp Leu Ala Asn Val His Phe Leu Gly His Val Gln Tyr Lys Ala
            260                 265                 270

Leu Pro Asn Leu Leu Ser Gln Cys Gln Ile Gly Leu Leu Pro Met Ser
        275                 280                 285

Asp His Pro Ser Asn Leu Gly Arg Ser Pro Met Lys Leu Phe Glu Tyr
    290                 295                 300

Ala Ala Val Gly Leu Pro Val Val Ser Thr Ser Thr Pro Glu Leu Glu
305                 310                 315                 320

Arg Arg Ser Leu Pro Phe Val Phe Leu Ala Glu Asn Ala Gln Ile Phe
                325                 330                 335

Thr Asp Lys Leu Gly Asp Val Cys Asn Ala Asp Ile Lys Ile Leu Ala
            340                 345                 350

Gln Gln Ala Arg Asp Asn Ala Lys His Glu Ser Trp Ala Ser Lys Thr
        355                 360                 365

Ile Thr Ile Leu Asn Tyr Ile Tyr Ser Lys
    370                 375

<210>  49
<211>  420
<212>  PRT
<213>  Methylobacillus glycogenes

<400>  49

Met Asn Lys Thr Thr Glu Pro Gln Asp Ser Gln Thr Asn Val Pro Trp
1               5                   10                  15

Trp Leu Ser Pro His Gly Leu Cys Leu Gly Phe Leu Leu Pro Met Ile
            20                  25                  30

Leu Leu Ile Trp Trp Leu Gly Ser Val Arg Ser Pro Thr Leu Thr Ile
        35                  40                  45

Arg Gly Tyr Ile Tyr Leu Ser Ser Glu Tyr Ile Ala Leu Ala Val Leu
    50                  55                  60

Ile Val Val Leu Ser Ala Ser Ala Ala Trp Met Gly Ser Phe Val Lys
65                  70                  75                  80

Thr Lys Val Arg Val Pro Pro Asp Ser Gln Val Asn Met Ala Leu Arg
                85                  90                  95

Val Leu Gly Tyr Val Ala Val Ala Ala Tyr Ile Tyr Trp Phe Lys Glu
            100                 105                 110

Leu Ile Ile Asn Pro Met Asn Ile Ile Asn Ile Phe Thr Gly Thr Gly
        115                 120                 125

Leu Ser Arg Gln Glu Ile Gly Ala Thr Gln Gly Ile Thr Ser Leu Val
    130                 135                 140

Asn Phe Thr Pro Ile Phe Ile Ser Ile Tyr Ile Tyr Ala Arg Tyr Ile
145                 150                 155                 160

Ala Lys Leu Pro Leu Arg Gln Asp Val Val Leu Leu Phe Tyr Ala Ile
                165                 170                 175

Val Leu Leu Thr Leu Leu Arg Val Phe Ala Trp Ala Glu Arg Leu Ala
            180                 185                 190

Leu Ile Glu Ile Ile Leu Pro Phe Ala Leu Leu Ile Thr Arg Gln Gln
        195                 200                 205

Val Phe Ser Lys Ile Lys Ile Leu Gln Phe Phe Ile Lys Ala Gly Pro
    210                 215                 220

Phe Ala Gly Leu Pro Leu Leu Val Leu Tyr Phe Gly Met Ala Glu Tyr
225                 230                 235                 240

Phe Arg Ser Trp Ser Ser Thr Thr Tyr Ala Gly Lys Ser Ser Phe Trp
                245                 250                 255

Asp Phe Ala Leu Gly Arg Ile Ala Ser Tyr Tyr Tyr Thr Ser Leu Asn
            260                 265                 270

Asn Gly Ala Gly Met Leu Ala Thr Val Asn Trp Pro Thr Tyr Glu Phe
        275                 280                 285

Glu Asn Val Leu Asn Trp Met His Lys Phe Pro Ile Phe Gly Gly Val
    290                 295                 300

Phe Arg Tyr Tyr Val Asp Leu His Thr Met Arg Met Gly Glu Phe Leu
305                 310                 315                 320

Ala Gln Tyr Gly Asp Glu Glu Phe Asn Asn Pro Ser Gly Ile Phe Ser
                325                 330                 335

Val Ile Leu Asp Leu Gly Leu Pro Phe Gly Ile Ile Tyr Phe Phe Ile
            340                 345                 350

Thr Ala Phe Leu Ala Gly Ile Phe Tyr Lys Gly Tyr Leu Arg Gly Asn
        355                 360                 365

Phe Leu Ser Val Ile Phe Tyr Pro Leu Phe Phe Ile Thr Leu Leu Glu
    370                 375                 380

Ile Phe Arg Tyr Pro Tyr Leu Gly Thr Ser Arg Ala Phe Thr Glu Ile
385                 390                 395                 400

Ile Gly Val Ile Ile Ala Phe Phe Ile Val Lys Gly Arg Thr Glu Asn
                405                 410                 415

Asp Arg Lys Ser
            420

<210>  50
<211>  454
<212>  PRT
<213>  Methylobacillus glycogenes

<400>  50

Met Ile Glu Asn Arg Arg His Leu Val Lys Ser Leu Phe Leu Met Leu
1               5                   10                  15

Phe Ser Ser Trp Leu Pro Lys Val Ala Ser Ala Ala Pro Lys Val Val
            20                  25                  30

Gly Gly Asn Ala Asp Phe Thr Arg Ala Leu Asp Asp Ala Leu Ala Gly
        35                  40                  45

Arg Thr Glu Val Leu Ser Gly Tyr Tyr Glu Ile Ser Asn Pro Leu Arg
    50                  55                  60

Phe Ser Leu Lys Ser Gly Thr Val Lys Phe Ala Pro Asp Phe Gln Leu
65                  70                  75                  80

Lys Phe Leu Asn Asp Lys Leu Ser Gly Leu Val Phe Ser Ser Cys Ser
                85                  90                  95

Asp Leu Ser Leu Ile Gly Gly Asp Phe Ser Tyr Lys Asn Pro Pro Ala
            100                 105                 110

Arg Arg Ile Gly His Gly Gly Ile Tyr Glu Phe Ser Arg Cys Thr Lys
        115                 120                 125

Val Ile Val Arg Asp Gly Phe Tyr Tyr Asp Thr Pro Gly Thr Gly Ile
    130                 135                 140

Ile Phe Thr Leu Cys Asp Asp Cys Ala Val Ile Lys Ala Thr Val Trp
145                 150                 155                 160

Asn Ser Trp Ala Asp Gly Leu His Phe Ala Asn Cys Asn Asn Cys Ile
                165                 170                 175

Ala Leu Asp Tyr Gln Ser Thr Asn Thr Gly Asp Asp Gly Leu Ala Phe
            180                 185                 190

Val Asn Tyr Lys Lys His Pro Asp Ala Val Gly Gly Tyr Ala Asn Asn
        195                 200                 205

Ile Val Ile Asn Gly Ser Tyr Ala Arg Gly Ile Ala Ile Ile Gly Gln
    210                 215                 220

Ser Gly Val Thr Ile Asp Lys Phe Glu Ile Arg His Thr Ser Ala Glu
225                 230                 235                 240

Gly Leu Lys Ile Gly Ser Glu Gln Tyr Tyr Asp Thr Arg Ile Pro Ser
                245                 250                 255

Asn Val Thr Ile Lys Asn Gly Ile Val Glu Glu Ala Gly Thr Trp Cys
            260                 265                 270

Val Ala Lys Ala Thr Gly Asn Gly Phe Leu Val Gln Gly Ala Thr Asp
        275                 280                 285

Lys Ile Val Val Glu Asn Leu Thr Val Asn Ala Pro Arg Val His Gly
    290                 295                 300

Ile Val Phe Asp Arg His Gly Gly Asn Val Lys Met Lys Asp Ile Val
305                 310                 315                 320

Val Lys Asn Thr Lys Ala Ser Gly Ile Thr Met Met Ala Asp Asn Ile
                325                 330                 335

Glu Leu Val Asn Gly Glu Phe Leu Asn Ile Gly Thr Thr Pro Leu Tyr
            340                 345                 350

Val Asn Lys Ala Leu Thr Ala Asn Phe Ser Lys Ile Asn Val Asn Thr
        355                 360                 365

Phe Gly Phe Ala Asp Lys Ser Ser Trp Pro Ile Phe Trp Ala Val Glu
    370                 375                 380

Ala Lys Asn Val Leu Ile Asn Gln Met Lys Val Glu Ser Thr Thr Leu
385                 390                 395                 400

Val Arg Ile Gly Gly Lys Asn Val Ile Ser His Gln Ile Thr Gly Val
                405                 410                 415

Gln Ile Ser Gly Val Gln Leu Ser Gly Leu Leu Ser Asp Gly Leu Asn
            420                 425                 430

Ser Lys Thr Thr Ser Ala Val Ser Ala Ile Thr Ser Thr Ala Val Lys
        435                 440                 445

Phe Ile Glu Gln Trp Asn
    450

<210>  51
<211>  368
<212>  PRT
<213>  Methylobacillus glycogenes

<400>  51

Met Asp Ala Gly Ala Arg Leu Arg Ile Ser Trp Ile Asp Val Cys Arg
1               5                   10                  15

Gly Phe Ala Met Leu Leu Val Val Val Gly His Asn Gln Ile Leu Met
            20                  25                  30

Gly Gln Ser Leu Ser Leu Val Lys Phe Ile Tyr Leu Phe His Met Pro
        35                  40                  45

Leu Phe Phe Phe Ile Ser Gly Ile Thr Leu Lys Pro Asp Gln Gly Leu
    50                  55                  60

Thr Tyr Tyr Phe Thr Arg Ala Ile Asn Val Ile Ala Val Tyr Phe Val
65                  70                  75                  80

Phe Ser Phe Leu Val Leu Pro Phe Val Leu Met Lys Asn Pro Asp Asp
                85                  90                  95

Gly Val Phe Asn Ile Val Leu Gly Ile Leu Tyr Gly Thr Gly His Thr
            100                 105                 110

Ile Glu Val Ala Pro Leu Trp Phe Ile Pro Cys Leu Phe Phe Ser Ile
        115                 120                 125

Val Leu Val Gly Leu Ala Leu Val Ile Ser Asn His Phe Asn Asn Thr
    130                 135                 140
Asp Arg Glu Ser Asn Arg Ser Pro Ile Phe Phe Leu Ala Leu Leu Phe
145                 150                 155                 160
Ile Gly Val Gly Val Phe Leu Ile Asn Ser Ile His Tyr Tyr Leu Ile
                165                 170                 175
Asp Asn Leu Ala Trp Gly Ser Leu Lys Asn Ser Gly Met Ile Trp Ser
            180                 185                 190
Ile Asp Ile Ala Ile Ile Gly Ala Gly Phe Ile Leu Leu Gly Met Tyr
        195                 200                 205
Ala Lys Val Phe Val Asp Lys Tyr Lys Ile Thr Glu Ile Lys Ser Ile
    210                 215                 220
Tyr Leu Ile Val Ser Ile Val Cys Ala Ile Phe Val Met Leu Gly Leu
225                 230                 235                 240
Phe Ser Phe Gly Val Asp Val Asp Leu Asn Phe Arg Lys Ile Ala His
                245                 250                 255
Tyr Pro Leu Ala Met Leu Cys Met Ile Leu Gly Ile Phe Phe Ser Ile
            260                 265                 270
Leu Phe Ser Ile Phe Leu Asp Lys Tyr Thr Tyr Leu Ser Thr Ile Leu
        275                 280                 285
Ser Ser Ile Gly Lys Ser Thr Leu Leu Ile Leu Leu Phe His Asn Ala
    290                 295                 300
Ile Gln Asn His Leu Ile Phe Thr Ile Gly Asp Ser Lys Ile Tyr Ser
305                 310                 315                 320
Ala Val Ile Gly Ile Val Ile Ala Ile Leu Ile Pro Ile Val Leu Asp
                325                 330                 335
Lys Phe Val Val Asn Lys Tyr Glu Leu Phe Arg Lys Ile Val Tyr Pro
            340                 345                 350
Lys Leu Ser Ile Asn Leu Ala Ser Lys Ile Met Val Gly Lys Arg Phe
        355                 360                 365
<210> 52

<211>  365
<212>  PRT
<213>  Methylobacillus glycogenes

<400>  52

Met Ile Val Tyr Ile Asn Gly Arg Phe Leu Gly Arg Arg Ile Thr Gly
1               5                   10                  15

Val Asp Arg Tyr Ala Ile Glu Val Leu Lys Glu Leu Asn Thr Leu Ile
            20                  25                  30

Ala Glu Asn His Pro Ser Val Ala Gly Val Glu Tyr Lys Ile Val Ile
        35                  40                  45

Pro Pro Arg Ile Lys Thr Asp Leu His Leu Thr His Ile Pro Ile Val
    50                  55                  60

Ala Tyr Gly Phe Phe Ser Gly His Ala Trp Glu Gln Leu Ser Leu Leu
65                  70                  75                  80

Met Leu Ser Leu Ser Ser Lys Ser Met Val Leu Asn Leu Cys Asn Thr
                85                  90                  95

Gly Ser Ile Phe Val Arg Ser Gln Ile Val Val Ile His Asp Ala Ala
            100                 105                 110

Val Val Arg Ile Pro Ser Thr Phe Ser Leu Ala Phe Arg Val Trp Tyr
        115                 120                 125

Lys Thr Leu Met Pro Val Leu Gly Arg Val Ala Lys Lys Ile Val Thr
    130                 135                 140

Val Ser Glu Tyr Ser Lys Lys Asp Ile Ile Asn Phe Phe Gly Val His
145                 150                 155                 160

Pro Glu Lys Val Gln Val Val Gly Ala Gly Gly Glu His Ile Leu Ser
                165                 170                 175

Gln Gln Pro Asp Ala Gly Ile Leu Glu Lys Phe Gln Val His Pro Gly
            180                 185                 190

Ser Tyr Val Leu Ala Val Ser Ser His Ala Pro His Lys Asn Phe Ser
        195                 200                 205

Leu Thr Ile Glu Ser Leu Lys Leu Ser Lys Asn Ala Asn Ile Pro Tyr
    210                 215                 220

Leu Ile Ala Gly Gly Ala Asn Gln Lys Ile Phe Gly Glu Asn Ser Gln

225                 230                 235                 240

Leu Asp Ser Ser Lys Val Thr Leu Leu Gly Tyr Val Ser Asp Asn Glu
                245                 250                 255

Leu Arg Ala Leu Tyr Glu His Ala Leu Cys Phe Val Phe Pro Ser Leu
            260                 265                 270

Tyr Glu Gly Phe Gly Leu Pro Pro Leu Glu Ala Met Asn Cys Gly Cys
        275                 280                 285

Pro Val Leu Ala Ser Asn Ala Ala Ser Ile Pro Glu Val Cys Ser Asp
    290                 295                 300

Ala Ala Leu Tyr Phe Ser Pro His Asp Pro Gln Glu Leu Ser Lys Leu
305                 310                 315                 320

Ile Asp Asn Val Ala His Asp Ala Asp Leu Arg Lys Ser Leu Val Asn
                325                 330                 335

Gln Gly Leu Lys Arg Ala Lys Asp Phe Ser Trp Lys Asn Ser Ala Ile
            340                 345                 350

Asp Ile Ala Arg Met Cys Lys Glu Ile Phe Thr Ile Lys
        355                 360                 365

<210>  53
<211>  384
<212>  PRT
<213>  Methylobacillus glycogenes

<400>  53

Met Thr Asn Lys Tyr Thr Arg His Ser Arg Leu His Pro Lys Lys Val
1               5                   10                  15

Ala Ile Val Val Asp Trp Leu Asn Glu Tyr Ala Gly Ala Glu Arg Val
            20                  25                  30

Leu Glu Gln Leu Val Leu Cys Tyr Pro Asp Ala Asp Ile Phe Ala Val
        35                  40                  45

Ile Glu Phe Leu Pro Asp Asn Leu Arg Gly Phe Ile Gln Asn Lys Lys
    50                  55                  60

Val Thr Thr Thr Phe Ile Gln Asn Leu Pro Lys Ala Lys Thr His Tyr
65                  70                  75                  80

Arg Asn Tyr Leu Pro Leu Met Pro Leu Ala Ile Glu Gln Leu Asp Leu

                85                  90                  95

Ser Gln Tyr Asp Leu Ile Ile Ser Ser Ser His Ala Val Ala Lys Gly
            100                 105                 110

Val Leu Thr Gly Pro Asp Gln Val His Val Ser Tyr Val His Ser Pro
        115                 120                 125

Met Arg Tyr Ala Trp Asp Leu Gln His Gln Tyr Leu Arg Glu Ser Asn
    130                 135                 140

Leu Val Ser Gly Phe Lys Ser Trp Ile Val Lys Trp Leu Leu His Lys
145                 150                 155                 160

Leu Arg Ile Trp Asp Leu Arg Thr Ala Asn Gly Val Asp Val Phe Ile
                165                 170                 175

Ala Asn Ser Ser Phe Ile Ala Lys Arg Ile Lys Lys Val Tyr Gly Arg
            180                 185                 190

Glu Ala Arg Val Ile Tyr Pro Pro Val Asp Thr Asn Ser Phe Ala Phe
        195                 200                 205

Gln Glu Lys Lys Asp Asp Phe Phe Leu Thr Ala Ser Arg Leu Val Pro
    210                 215                 220

Tyr Lys Arg Val Asp Leu Ile Val Glu Ala Phe Ser Lys Ile Pro Asp
225                 230                 235                 240

Gln Lys Leu Val Val Ile Gly Asp Gly Pro Asp Met Ser Lys Val Arg
                245                 250                 255

Ala Lys Ala Gly Pro Asn Val Glu Ile Leu Gly Tyr Gln Ser His Ala
            260                 265                 270

Val Leu Arg Asp Tyr Met Gln Arg Ala Lys Gly Phe Val Phe Ala Ala
        275                 280                 285

Glu Glu Asp Phe Gly Ile Ser Pro Val Glu Ala Gln Ala Cys Gly Thr
    290                 295                 300

Pro Val Ile Ala Phe Ala Lys Gly Gly Ala Leu Glu Thr Val Ile Pro
305                 310                 315                 320

Glu Glu Arg Gly Asp Pro Ala Thr Gly Leu Phe Phe His Glu Gln Thr
                325                 330                 335

Ala Asp Ala Ile Val Asp Ala Ile Thr Arg Phe Asn Leu Leu Ser Gln
            340                 345                 350

Lys Ile Ser Pro Phe Ala Cys Arg Glu Asn Ala Leu Arg Phe Ser Pro
        355                 360                 365

Asp Arg Phe Arg Asn Glu Ile Ile Ser Thr Val Asn Lys Ala Leu Asp
    370                 375                 380

<210>  54
<211>  388
<212>  PRT
<213>  Methylobacillus glycogenes

<400>  54

Met Lys Ile Ala Val Ala Gly Thr Gly Tyr Val Gly Leu Ser Ile Ala
1               5                   10                  15

Val Leu Leu Ala Gln Lys Asn Glu Val Val Ala Leu Asp Ile Val Pro
            20                  25                  30

Asp Lys Val Asp Leu Ile Asn Asn Lys Ile Ser Pro Ile Val Asp Lys
        35                  40                  45

Glu Ile Glu His Tyr Leu Lys Asn Ile Pro Leu Asn Leu Arg Ala Thr
    50                  55                  60

Thr Asn Lys His Asp Ala Tyr Glu Asn Ala Asp Phe Val Val Ile Ala
65                  70                  75                  80

Thr Pro Thr Asp Tyr Asp Pro Val Thr Ser Tyr Phe Asn Thr Ser Ser
                85                  90                  95

Val Glu Ala Val Ile Lys Asp Val Gln Ala Ile Ala Pro Asn Ala Thr
            100                 105                 110

Met Val Ile Lys Ser Thr Ile Pro Val Gly Tyr Thr Lys Met Ile Ser
        115                 120                 125

Glu Lys Leu Gly Val Asp Asn Leu Ile Phe Ser Pro Glu Phe Leu Arg
    130                 135                 140

Glu Gly Lys Ala Leu Tyr Asp Asn Leu Tyr Pro Ser Arg Ile Val Ile
145                 150                 155                 160

Gly Glu Ile Ser Asp Arg Ala Gln Val Phe Ala Asp Leu Leu Asn Ala
                165                 170                 175

Gly Ala Val Lys Glu Asn Ile Pro Val Leu Leu Thr Asn Ser Ala Glu
            180                 185                 190

Ala Glu Ala Ile Lys Leu Phe Ala Asn Thr Phe Leu Ala Met Arg Val
        195                 200                 205

Ala Tyr Phe Asn Glu Leu Asp Thr Tyr Ala Ala Thr His Gly Leu Asn
    210                 215                 220

Thr Arg Gln Ile Ile Asp Gly Val Cys Leu Asp Pro Arg Val Gly Asp
225                 230                 235                 240

His Tyr Asn Asn Pro Ser Phe Gly Tyr Gly Gly Tyr Cys Leu Pro Lys
                245                 250                 255

Asp Thr Lys Gln Leu Leu Ala Asn Tyr Asp Asn Val Pro Gln Lys Leu
            260                 265                 270

Ile Asn Ala Ile Val Asp Ala Asn Thr Thr Arg Lys Asp Phe Ile Ala
        275                 280                 285

Gln Ser Ile Ile Lys Arg Asp Pro Lys Val Val Gly Ile Tyr Arg Leu
    290                 295                 300

Ile Met Lys Ala Gly Ser Asp Asn Phe Arg Ala Ser Ser Ile Gln Gly
305                 310                 315                 320

Val Met Lys Arg Ile Lys Ala Arg Gly Ile Lys Val Val Val Phe Glu
                325                 330                 335

Pro Glu Leu Lys Glu Asp Gln Phe Phe Asn Ser Glu Val Ile Arg Asn
            340                 345                 350

Leu Glu Glu Phe Lys Lys Ile Ser Asp Val Ile Val Ala Asn Arg Arg
        355                 360                 365

Ala Gln Asp Leu Ser Asp Val Asp Glu Lys Val Tyr Thr Arg Asp Leu
    370                 375                 380

Phe Gly Asn Asp
385

<210>  55
<211>  323
<212>  PRT
<213>  Methylobacillus glycogenes

<400>  55

Met Arg Ile Leu Phe Ser Pro Ala Thr Ala Ser Asn Gln Tyr Val Glu
1               5                   10                  15

Asn Thr Ile Ala Leu Ile Lys Thr Leu Asp Ala Thr His Val Glu Thr
            20                  25                  30

Gln Ala Arg Leu Asn Ala Gly Phe Ile Leu Arg Asn Ala Phe Asn Arg
        35                  40                  45

Tyr Asp Cys Ile Ile Ile Asn Trp Leu Glu Asn Asn Leu Val Gly Lys
    50                  55                  60

Asn Lys Ser Leu Asp Ile Trp Ala Val Ala Lys Phe Leu Phe Lys Phe
65                  70                  75                  80

Phe Ile Tyr Arg Val Met Gly Arg Lys Leu Ile Phe Val Arg His Asn
                85                  90                  95

Ile Tyr Pro His Ala Leu Lys Gly Arg Gln Ala Ser Ile Ala Thr Trp
            100                 105                 110

Ile Ile Glu Ala Ala Ile Lys Leu Ser Thr Ile Ser Val Val His Ser
        115                 120                 125

Gly His Leu Glu Asn Glu Asn Ala Lys Tyr Val Pro His Pro Leu Tyr
    130                 135                 140

Arg Val Asp Ser Gln Val Glu Gly Gln Gly Ser Asp Tyr Tyr Val Ile
145                 150                 155                 160

Phe Gly Arg Ile Val Ala Tyr Lys Ala Ile His Lys Leu Leu Glu Ser
                165                 170                 175

Trp Gly Ser Gln Arg Leu Ile Ile Ala Gly Ile Val Glu Asp His Ala
            180                 185                 190

Tyr Ala Ala Ser Leu Gln Ser Ile Ile Gln Ser Arg Gly Leu Ser Asn
        195                 200                 205

Val Glu Leu Arg Ala Glu Phe Leu Ser Glu Ala Glu Ala Glu Gln Leu
    210                 215                 220

Val Ala Asn Ser Lys Gly Leu Ile Leu Ser His Cys Asp Ala Asp Met
225                 230                 235                 240

Ile Val Ser Gly Ser Phe Phe Phe Ala Ala Ser Leu Gly Val Pro Val
                245                 250                 255

Phe Ala Met Asp Thr Pro Phe Phe Gln Trp Leu Lys Asn Glu Gln Gly
            260                 265                 270

Tyr Gln Gly Leu Gln Val Phe Thr Ser Leu Gln Ala Met Thr Ala Glu
        275                 280                 285

Leu Ala Thr Ala Ala Ala Val Glu Lys Asn Thr Ile Ala Ala Ser Ala
    290                 295                 300

Gln Arg Leu Phe Gly Asn Asp Arg Val Arg Ser Ala Trp Gly Ser Val
305                 310                 315                 320

Leu His Gly

<210> 56
<211> 470
<212> PRT
<213> Methylobacillus glycogenes

<400> 56

Met Thr Thr Ile Gly Gln Asp Val Asn Gln Pro Ala Gly Ile Pro Glu
1               5                   10                  15

Val Phe Thr Ile Asn Asp Val Val Asp Arg Gly Leu Cys Val Gly Cys
            20                  25                  30

Gly Ala Cys Ala Val Val Asp Ser Asn Val Lys Leu Thr Arg Asn Gln
        35                  40                  45

Tyr Gly Ser Met Val Ala Ala Ile Asp Ala Asn Ile Leu Pro Gly Ser
    50                  55                  60

Ile Ala Asp Arg Val Cys Pro Phe Ser Asp Asn Ser Leu Asn Glu Asp
65                  70                  75                  80

Val Val Gly Glu Arg Val Phe Ala Ser Cys Glu Asn Phe Asp Gln Arg
                85                  90                  95

Thr Gly Tyr Tyr Thr Asn Leu Tyr Tyr Gly Arg Arg Phe Thr Glu Ala
            100                 105                 110

Glu Leu Leu Lys Ser Ser Ser Gly Gly Leu Thr Ser Tyr Val Ala Ile
        115                 120                 125

Glu Leu Leu Lys Arg His His Val Asp Gly Ile Ile His Val Gly Arg
    130                 135                 140

Asp Gln Ser Asp Asp Arg Asp Ala Leu Phe Lys Phe Met Val Ser Tyr
145                 150                 155                 160

Thr Glu Glu Asp Ala Asn Ala Cys Lys Lys Ser Gln Tyr Tyr Ser Leu
                165                 170                 175

Ser Phe Asp Glu Val Val Arg Ser Ile Arg Gly Asp Gly Lys Arg Tyr
            180                 185                 190

Val Phe Val Gly Val Pro Cys Phe Val Lys Ser Leu Arg Asn Ile Cys
        195                 200                 205

Leu Asn Asp Ala Val Leu Asn Glu Gln Ile Pro Phe Met Leu Gly Leu
    210                 215                 220

Val Cys Gly His Leu Lys Ser Ser Arg Phe Ala Glu Leu Leu Ala Trp
225                 230                 235                 240

Gln Leu Ser Val Pro Pro Glu Lys Ile Gln Ser Val Asp Phe Arg Val
                245                 250                 255

Lys Asn Asn Ala Ser Thr Val Asn Ser Tyr Asp Phe Gln Val Val Asp
            260                 265                 270

Ser Asn Gln Gln Ala His Ala Lys Thr Ser Asn Thr Met Leu Gly Gly
        275                 280                 285

Asn Trp Gly His Ala Ala Phe Gln Leu Gln Ala Cys Asp Tyr Cys Asp
    290                 295                 300

Asp Ile Phe Ala Glu Thr Ala Asp Ala Val Phe Gly Asp Ala Trp Leu
305                 310                 315                 320

Pro Gln Phe Ser Thr Trp Trp Gln Gly Thr Asn Ile Val Leu Val Arg
                325                 330                 335

Asn Pro Ile Ile Asp Asp Ile Leu Glu Ser Gly Lys Asn Ala Gly Ala
            340                 345                 350

Ile Thr Leu Gly Gly Val Ser Ile Asp Asp Leu Cys Lys Ser Gln Leu
        355                 360                 365

Gly Asn Phe Arg His Arg Lys Asp Gly Leu Ile Val Arg Leu Asp Asp
    370                 375                 380

Asp Leu Lys Gln Gly Lys Lys Val Pro Lys Lys Arg Val Asp Leu Ser
385                 390                 395                 400

Ala Leu Gly Lys Ile Asp Gln Lys Arg Lys Lys Ile Val Arg Leu Arg
                405                 410                 415

Lys Glu Met Ala Val Lys Ser His Glu Tyr Phe Leu Gln Ala Arg Glu
            420                 425                 430

Lys His Asp Leu Ser Leu Tyr Met Lys Pro Met Gly Gln Leu Ala Arg
        435                 440                 445

Asn Met Leu Lys Leu Tyr Arg Asp Pro Phe Met Lys Arg Leu Ile Asn
    450                 455                 460

Arg Leu Lys Arg Tyr Phe
465                 470

<210> 57
<211> 482
<212> PRT
<213> Methylobacillus glycogenes

<400> 57

Met Ser Leu Ser Val Lys Ala Ala Arg Gly Gly Val Val Met Met Ser
1               5                   10                  15

Gly Gln Ile Val Lys Val Ala Leu Gln Phe Gly Ser Leu Ile Ile Leu
            20                  25                  30

Ala Arg Leu Leu Gln Pro Glu Asp Phe Gly Leu Val Ala Met Val Ile
        35                  40                  45

Ala Ile Tyr Gly Ile Cys Asp Ile Leu Leu Asp Phe Gly Leu Ser Thr
    50                  55                  60

Ala Ser Ile Gln Val Gln Asn Ile Thr Lys Ala Gln Ile Ser Asn Leu
65                  70                  75                  80

Phe Trp Val Asn Val Leu Ile Gly Leu Leu Phe Thr Leu Ile Ser Tyr
                85                  90                  95

Phe Ser Ala Ser Leu Leu Ala Ser Phe Tyr Ala Gln Pro Glu Leu Thr
            100                 105                 110

Lys Ile Ala Gln Tyr Val Ser Val Val Phe Leu Phe Asn Gly Ile Ala
        115                 120                 125

Ala Gln Tyr Lys Ala Gln Leu Asn Arg Asn Phe Lys Phe Lys Gln Met
    130                 135                 140

Ala Ile Thr Asp Thr Thr Ala Val Leu Leu Ser Val Cys Ile Gly Val
145                 150                 155                 160

Tyr Phe Ala Tyr Ala Gly His Ser Tyr Trp Ala Ile Val Tyr Gln Gln
                165                 170                 175

Ile Ala Gln Ala Leu Ile Gln Met Leu Leu Tyr Met Leu Phe Gly Ala
            180                 185                 190

Trp Leu Pro Ser Leu Pro Ser Arg His Val Ser Ile Arg His Phe Phe
        195                 200                 205

Lys Phe Gly Trp Gly Leu Ala Gly Ser Gln Leu Met Gly Tyr Phe Ser
    210                 215                 220

Arg Asn Ile Pro Ala Ile Leu Ile Gly Asn Gln Ile Gly Ala Ser Gln
225                 230                 235                 240

Leu Gly Phe Tyr Asp Arg Ala Asn Lys Leu Leu Met Leu Pro Leu Asn
                245                 250                 255

Gln Leu Asn Ala Pro Ser Ser Ser Val Ala Val Pro Val Leu Ser Arg
            260                 265                 270

Leu Ala Val Glu Asp Gln Glu Lys Tyr Asn Arg Phe Leu Leu Phe Gly
        275                 280                 285

Gln Asn Ile Ile Val His Leu Val Val Phe Cys Leu Ala Leu Thr Cys
    290                 295                 300

Cys Gln Thr Glu Arg Leu Val Gln Leu Val Leu Gly Thr Gln Trp Leu
305                 310                 315                 320

Glu Met Val Pro Val Tyr Arg Ala Leu Ser Phe Ala Gly Ile Phe Leu
                325                 330                 335

Ala Leu Ser Tyr Ala Ser Tyr Trp Val Phe Leu Ser Lys Gly Ile Thr
            340                 345                 350

Ala Ser Leu Phe Lys Leu Gly Leu Val Ser Arg Pro Ile Ser Ile Leu
        355                 360                 365

Ile Ile Cys Leu Gly Leu Pro Ala Gly Val Ile Gly Val Ala Tyr Ala
    370                 375                 380

Tyr Ser Ala Ser Leu Leu Phe Thr Trp Leu Leu Gly Val Phe Trp Leu

385                 390                 395                 400

Arg Lys Thr Gly Val Pro Val Lys Ser Met Ile Val Asp Pro Leu Ile
                405                 410                 415

Met Ser Ala Ile Tyr Tyr Ser Ala Ser Phe Ile Ala Asn Tyr Val Val
            420                 425                 430

Asp His Tyr Val Asp Phe Ala Ile Tyr Asn Ile Leu Val Gly Trp Leu
        435                 440                 445

Leu Met Leu Leu Ile Leu Ala Val Phe Tyr Leu Leu Val Pro Lys Phe
    450                 455                 460

Lys Arg Ser Val Lys Lys Leu Phe Glu Val Lys Ser Tyr Ile Arg Asn
465                 470                 475                 480

Lys Gln

<210>  58
<211>  401
<212>  PRT
<213>  Methylobacillus glycogenes

<400>  58

Met Lys Thr Ile Lys Lys Leu Leu Tyr Lys Ile Leu Gly Ser Ala Gly
1               5                   10                  15

Ile Lys Ala Lys Ala Ala Ala Val Ile Asn Leu Leu Phe Gly Arg Val
            20                  25                  30

Ala Arg Ser Thr Asn Asp Ser Val Ser Ile Leu Ile Pro Ala Pro Gly
        35                  40                  45

Gly Gly Asn Ile Gly Asp Gln Ala Met Leu Glu Ser Phe Leu His Asn
    50                  55                  60

Ile Asp Gly Lys Lys Leu Ile Ile Lys Thr Gln Pro Ile Ala Leu Asp
65                  70                  75                  80

Ile Pro Ser His Tyr Ala Val Asn Val Glu Val Ile Glu Met Pro Gly
                85                  90                  95

Leu Ile Tyr Gly His Leu Phe Arg Phe Leu Arg Asp Tyr Val Arg Leu
            100                 105                 110

Arg Lys Ile Met Ala Ser Ala Ser Ala Leu Tyr Val Val Gly Ala Asp

115                 120                 125

Ile Met Asp Gly Gly Tyr Asn Lys Asn Ala Ser Ile Met Arg Ser Lys
    130                 135                 140

Ile Ala Thr Tyr Phe Ala Arg Asn Asn Ile Pro Ser Arg Val Phe Gly
145                 150                 155                 160

Phe Ser Trp Asn Gly Asn Pro Asp Ala Ala Ala Leu Ser Ala Ile Arg
                165                 170                 175

Asn Ala Ala Ala Asn Gly Val Leu Leu Cys Ala Arg Asp Pro Leu Ser
            180                 185                 190

Phe Gln Arg Leu Gln Asp Ala Gly Val Pro Asn Ile Arg Leu Val Ala
        195                 200                 205

Asp Met Val Phe Ala Asn Asp Asn Thr Lys Ser Ala Gly Ile Glu Asp
    210                 215                 220

Ile Leu Gln Phe Cys Ala Gly Lys Gln Ile Ala Leu Val Asn Ala Ser
225                 230                 235                 240

Gly Leu Ile Gly Lys Asn Ile Asp Gln Ile Asp Glu Tyr Ser Lys Val
                245                 250                 255

Ile Asn Lys Leu Met Asp His Asn Phe Asn Ile Val Leu Leu Pro His
            260                 265                 270

Val Ile Arg His Gly Ala Asn Asp Leu Pro Ile Cys Gln Ala Leu Ser
        275                 280                 285

Gly Lys Tyr Pro Asn Ala Phe Leu Val Asn Arg Leu Leu Glu Pro Asp
    290                 295                 300

Gln Val Lys Arg Leu Ala Gln Glu Ala Lys Ile Val Val Thr Gly Arg
305                 310                 315                 320

Met His Leu Ser Ile Met Ser Leu Asn Gln Asn Val Pro Ser Ile Val
                325                 330                 335

Leu Ser Thr Gln Gly Lys Val Asp Gly Leu Met Gln Phe Phe Glu Thr
            340                 345                 350

Glu Lys Leu Ser Ile Asn Pro Glu Lys Gly Phe Ser Asp Leu Ile Cys
        355                 360                 365

Ser Gln Ile Asp Phe Ile Cys Ser Asn Arg Gln Tyr Tyr Gln Asp Leu
    370                 375                 380

Ile Arg Lys Asn Leu Leu Lys Val Lys Glu Leu Ala Ala Gly Asn Phe
385                 390                 395                 400

Asn

<210>  59
<211>  395
<212>  PRT
<213>  Methylobacillus glycogenes

<400>  59

Met Ser Lys Phe Tyr Lys Phe Gln Val Leu Glu Ser Leu Arg Gly Tyr
1               5                   10                  15

Met Ala Trp Trp Val Val Leu Gly His Ala Ile Gln Leu Ser Gly Phe
            20                  25                  30

Trp His Lys Gln Glu Val Asp Glu Phe Phe Thr Gly Gly Gly Ile Ile
        35                  40                  45

Asn Ala Thr Phe Ser Lys Ile Leu Ser Leu Ile Ile Asn Pro Glu Leu
    50                  55                  60

Ala Val Asn Ile Phe Ile Ile Leu Ser Gly Phe Val Ile Thr His Leu
65                  70                  75                  80

Leu Leu Thr Lys Asn Glu Pro Tyr Ser His Phe Ile Thr Arg Arg Phe
                85                  90                  95

Phe Arg Leu Ala Pro Ala Phe Tyr Val Ser Leu Val Ile Ala Ile Leu
            100                 105                 110

Val Val Asn Phe Tyr Lys Val Ala Tyr Thr Gly Leu Ser Phe Ser Gly
        115                 120                 125

Asp Thr Ser Met Arg Leu Ala Arg Leu Val Glu Gln Asp Ser Asn Phe
    130                 135                 140

Leu Leu His Leu Phe Asn His Leu Leu Leu Ile His Ser Val Phe Pro
145                 150                 155                 160

Gln Glu Leu Ile Gln Phe Ser Pro Thr Thr Phe Leu Ala Pro Ala Trp
                165                 170                 175

Ser Ile Ser Leu Glu Trp Gln Phe Tyr Leu Ile Ala Pro Val Leu Ile
        180                 185                 190

Pro Phe Leu Val Lys Ser Glu Lys Ser Leu Ile Val Trp Val Val Leu
    195                 200                 205

Leu Leu Leu Leu Arg Tyr Phe Thr Asn His Val Leu Asp Phe Asn Trp
    210                 215                 220

Gln His Ser Ser Phe Phe Phe Lys Ala Val Asp Tyr Phe Leu Val Gly
225                 230                 235                 240

Ile Leu Ser Arg Leu Ile Ile Glu Arg Leu Gln His Gly Lys Phe Ile
                245                 250                 255

Tyr Asp Val Ala Val Leu Leu Gly Gly Ile Leu Leu Phe Gln Asp Arg
            260                 265                 270

Tyr Cys Ala Val Ile Trp Leu Phe Val Leu Ala Val Ile Phe Tyr Glu
        275                 280                 285

Met Gly Tyr Trp Ser Val Lys Ser Pro Leu Trp Lys Lys Ile Leu Thr
    290                 295                 300

Leu Phe Phe Tyr Asn Lys Val Ile Thr Asn Ile Gly Lys Trp Ser Tyr
305                 310                 315                 320

Ser Thr Tyr Leu Ile His Ile Pro Ile Phe Ser Ile Phe Leu Gly Gly
                325                 330                 335

Tyr Ala Thr Leu Thr Asp Pro Gly Thr Val Thr Gln Gln Ile Ser Leu
            340                 345                 350

Ile Ile Leu Phe Val Ser Phe Pro Val Val Leu Leu Ile Ser Asn Ile
        355                 360                 365

Met Tyr His Asn Ile Glu Leu Pro Ser Met Asn Tyr Ile Ser Ala Lys
    370                 375                 380

Leu Lys Glu Lys Arg Ile Thr Glu Ser Ala Glu
385                 390                 395

<210>  60
<211>  468
<212>  PRT
<213>  Methylobacillus glycogenes

<400>  60

Met Lys Ile Val Pro Val Leu Leu Ser Gly Gly Ser Gly Thr Arg Leu
1               5                   10                  15

Trp Pro Leu Ser Arg Ala Val Leu Pro Lys Gln Leu Leu Pro Leu Ile
            20                  25                  30

Thr Asn Lys Thr Met Leu Gln Glu Thr Leu Leu Arg Ile Ser Arg Trp
        35                  40                  45

Ala Thr Val Thr Asn Pro Val Val Val Cys Gly Asn Asp His Arg Phe
    50                  55                  60

Leu Val Ala Glu Gln Leu Arg Glu Ile Ala Leu Ser Pro Asn Ser Ile
65                  70                  75                  80

Ile Leu Glu Pro Ala Pro Lys Asn Thr Ala Pro Ala Ile Ala Ala Ala
                85                  90                  95

Ala Leu Gln Leu Gln Gly Gln Asp Val Tyr Met Leu Val Leu Pro Ala
            100                 105                 110

Asp His Val Ile Arg Asp Leu Gln Ser Phe Glu His Ala Val Gln Thr
        115                 120                 125

Ala Leu Thr Ala Ala Asp Gln Gly Asn Leu Val Thr Phe Gly Ile Lys
    130                 135                 140

Pro Ala His Pro Glu Thr Gly Tyr Gly Tyr Ile Lys Leu Gly Val Glu
145                 150                 155                 160

Ser Ala Pro Gly Ser Tyr Lys Val Asn Arg Phe Val Glu Lys Pro Asp
                165                 170                 175

Leu Ala Thr Ala Thr Glu Tyr Val Ala Ser Gly Glu Phe Phe Trp Asn
            180                 185                 190

Ser Gly Met Phe Leu Phe Lys Pro Glu Thr Phe Leu Lys Gln Leu Glu
        195                 200                 205

Ile His Ala Pro Glu Ile Thr Ala Ala Val Ser Lys Ala Val Asn Asn
    210                 215                 220

Ser Tyr Lys Asp Leu Asp Phe Val Arg Leu Glu Glu Ser Ser Phe Ala
225                 230                 235                 240

Glu Ser Pro Ser Leu Ser Ile Asp Tyr Ala Val Met Glu Lys Thr Asp
                245                 250                 255

His Ala Val Val Val Pro Ala Asp Ile Gly Trp Ser Asp Val Gly Ser
            260                 265                 270

Trp Thr Ser Leu Gln Glu Val Gln Glu Gln Asp Ala Asn Gly Asn Val
        275                 280                 285

Ala Arg Gly Asp Val Tyr Leu Lys Asn Val Glu Asn Ser Leu Val Arg
    290                 295                 300

Ser Glu Asp Arg Phe Val Ala Ala Ile Gly Val Arg Asp Leu Ile Val
305                 310                 315                 320

Val Glu Thr Ser Asp Ala Val Leu Val Ala Ser Lys Asp Ala Ala Gln
                325                 330                 335

Asp Val Lys Glu Val Val Glu Tyr Leu Lys Leu Asn Gln Arg Ser Glu
            340                 345                 350

His Lys Ile His Pro Arg Ala Tyr Arg Pro Trp Gly Trp Tyr Glu Ser
        355                 360                 365

Ile Asp Leu Gly Glu Arg His Gln Val Lys Arg Ile Met Val Lys Pro
    370                 375                 380

Gly Glu Lys Leu Ser Leu Gln Met His His His Arg Ala Glu His Trp
385                 390                 395                 400

Val Val Val Ser Gly Ser Ala Met Ile Thr Val Asp Gly Asn Thr Lys
                405                 410                 415

Leu Phe Ser Glu Asn Gln Ser Thr Tyr Ile Pro Ile Gly Ser Thr His
            420                 425                 430

Arg Leu Glu Asn Pro Gly Lys Leu Pro Leu His Leu Ile Glu Val Gln
        435                 440                 445

Ser Gly Ser Tyr Leu Gly Glu Asp Asp Ile Val Arg Tyr Glu Asp Thr
    450                 455                 460

Tyr Gly Arg Ser
465

<210> 61
<211> 110
<212> PRT
<213> Methylobacillus glycogenes

<400> 61

Met Leu Thr Asp Glu Tyr Arg Tyr Lys Ile Leu Lys Leu Val Gln Glu
1               5                   10                  15

Lys Pro Asp Ile Ser Gln Arg Glu Leu Ala Lys Ala Leu Gly Ile Ser
            20                  25                  30

Leu Gly Lys Thr Asn Phe Cys Leu Lys Ala Leu Ala Gln Val Gly Leu
        35                  40                  45

Leu Lys Val Ser Asn Phe Arg Asn Ser Lys Asn Lys Leu Ala Tyr Met
    50                  55                  60

Tyr Val Leu Thr Pro Thr Gly Ile Glu His Lys Ala Ala Val Thr Leu
65                  70                  75                  80

Asn Phe Leu Lys Leu Lys Leu Lys Glu Tyr Glu Gln Leu Ala Gly Glu
                85                  90                  95

Ile Glu Glu Leu Arg Ser Glu Ile Asn Ala Lys Val Asn Asp
            100                 105                 110

<210>  62
<211>  362
<212>  PRT
<213>  Methylobacillus glycogenes

<400>  62

Met Leu Lys Lys Val Ala Leu Ile Thr Gly Ile Thr Gly Gln Asp Gly
1               5                   10                  15

Ala Tyr Leu Ala Glu Phe Leu Leu Gly Lys Gly Tyr Val Val His Gly
            20                  25                  30

Ile Lys Arg Arg Ala Ser Leu Phe Asn Thr Asp Arg Ile Asp His Leu
        35                  40                  45

Tyr Gln Asp Pro His Val Asp Asn Gln Asn Phe Ile Leu His Tyr Gly
    50                  55                  60

Asp Leu Thr Asp Ser Thr Asn Leu Ile Arg Ile Ile Gln Gln Val Gln
65                  70                  75                  80

Pro Asp Glu Ile Tyr Asn Leu Ala Ala Met Ser His Val Ala Val Ser
                85                  90                  95

Phe Glu Ser Pro Glu Tyr Thr Ala Asn Ala Asp Gly Leu Gly Thr Leu
            100                 105                 110

Arg Ile Leu Glu Ala Ile Arg Ile Leu Gly Leu Glu Lys Lys Thr Arg
        115                 120                 125

Phe Tyr Gln Ala Ser Thr Ser Glu Leu Tyr Gly Leu Val Gln Glu Thr
    130                 135                 140

Pro Gln Lys Glu Ser Thr Pro Phe Tyr Pro Arg Ser Pro Tyr Ala Val
145                 150                 155                 160

Ala Lys Leu Tyr Ala Tyr Trp Ile Thr Val Asn Tyr Arg Glu Ala Tyr
                165                 170                 175

Gly Ile Tyr Ala Cys Asn Gly Ile Leu Phe Asn His Glu Ser Pro Leu
            180                 185                 190

Arg Gly Glu Thr Phe Val Thr Arg Lys Ile Thr Arg Ala Leu Ala Arg
        195                 200                 205

Ile Lys Leu Gly Leu Gln Asp Cys Leu Tyr Leu Gly Asn Leu Asp Ala
    210                 215                 220

Leu Arg Asp Trp Gly His Ala Lys Asp Tyr Val Glu Met Gln Trp Leu
225                 230                 235                 240

Met Leu Gln Gln Glu Gln Ala Glu Asp Phe Val Ile Ala Thr Gly Val
                245                 250                 255

Gln Tyr Ser Val Arg Asp Phe Val Asn Ala Ala Ala His Glu Leu Asp
            260                 265                 270

Ile Ala Ile Thr Trp Lys Gly Gln Gly Gln Glu Glu Met Gly Tyr Asp
        275                 280                 285

Gly Gln Gly Arg Cys Ile Val Gln Val Asp Pro Arg Tyr Phe Arg Pro
    290                 295                 300

Thr Glu Val Glu Thr Leu Leu Gly Asp Pro Ser Lys Ala Lys Gln Lys
305                 310                 315                 320

Leu Gly Trp Ser Pro Ser Ile Ser Phe Gln Glu Leu Val Ser Glu Met
                325                 330                 335

Val Arg Glu Asp Leu Lys Ser Ala Glu Arg Asp Glu Leu Val Lys Gln
            340                 345                 350

His Gly Phe Ala Ala Tyr Asp Tyr Asn Glu
        355                 360

<210>  63
<211>  313
<212>  PRT
<213>  Methylobacillus glycogenes

<400>  63

Met Asp Lys His Ala Arg Ile Tyr Val Ala Gly His Arg Gly Leu Val
1               5                   10                  15

Gly Ser Ala Ile Val Arg Gly Leu Glu Gly Lys Gly Tyr Ser Asn Leu
            20                  25                  30

Ile Lys Arg Thr His Ala Glu Leu Asp Leu Thr Asp Ala Asn Ala Thr
        35                  40                  45

Glu Ala Phe Phe Ala Gln Tyr Gln Pro Asn Met Phe Ser Trp Arg Gln
    50                  55                  60

Pro Lys Trp Val Ala Trp Pro Ile Thr Leu Thr Arg Gln Ile Leu Ser
65                  70                  75                  80

Val Thr Ile Ser Gln Phe Arg Thr Val Ser Ile Val Pro Gly Ser Met
                85                  90                  95

Glu Ser Ala Tyr Cys Phe Gly Pro Ala Ala Tyr Thr Pro Ser Met Leu
            100                 105                 110

Leu Ser Pro Cys Met Ser Thr Thr Phe Pro Asp Arg Leu Lys Leu Pro
        115                 120                 125

Ile Val His Thr Arg Trp Gln Arg Leu Leu Glu Ser Lys Cys Ala Gly
    130                 135                 140

Val Ile Thr Gly Ser Met Ala Leu Asp Phe Leu Gln Gln Cys Arg Gln
145                 150                 155                 160

Ile Phe Met Gly Leu Ala Ile Thr Ile Thr Cys Arg Ile Val Thr Leu
                165                 170                 175

Ser Leu His Tyr Ala Asn Ser Thr Arg Leu Asn Lys Leu Gly Met Gln
            180                 185                 190

Leu Tyr Arg Ser Gly Val Leu Glu His Pro Gly Ala Asn Phe Phe Thr
        195                 200                 205

Val Arg Ile Trp Gln Met Pro Val Tyr Ser Cys Ile Cys Leu Arg Arg
    210                 215                 220

Asn Ser Ile Leu Cys Trp Glu Val Met Arg Val Leu Leu Val Cys Leu
225                 230                 235                 240

Ile His His Ser Ile Ser Val Leu Glu Ser Ile Phe Gln Tyr Arg Asn
                245                 250                 255

Trp Pro Asn Trp Lys Arg Trp Leu Val Thr Lys Glu Gln Leu Ser Ser
            260                 265                 270

Ile Val Gln Asn Leu Met Ala Arg Cys Val Ser Leu Trp Met Ala Ser
        275                 280                 285

Ser Thr Val Trp Gly Gly Leu Ile Pro Leu Ser Leu Met Met Asp Trp
    290                 295                 300

Lys Arg His Ile Arg Asn Phe Gln Asn
305                 310

<210>  64
<211>  231
<212>  PRT
<213>  Methylobacillus glycogenes

<400>  64

Met Lys Lys Lys Val Phe Ala Leu Ile Pro Ala Arg Ala Gly Ser Lys
1               5                   10                  15

Gly Val Pro Lys Lys Asn Leu Lys Asn Ile Ala Gly Ser Pro Leu Ile
            20                  25                  30

Ala Tyr Thr Ile Asn Ala Ala Lys Lys Ala Lys Asn Val Asp Ala Val
        35                  40                  45

Tyr Val Ser Ser Asp Cys Lys Glu Ile Leu Glu Tyr Ser Lys Ala Gln
    50                  55                  60

Gly Cys Ser Ser Ile Leu Arg Pro Gln Glu Tyr Ala Thr Asp Asp Ala
65                  70                  75                  80

Ser Ala Val Asp Val Val Lys His Phe Ile Ser Ile Glu Asn Ile Glu
                85                  90                  95

Asp Gly Asp Leu Ile Val Tyr Leu Gln Pro Thr Ser Pro Leu Arg Asp
            100                 105                 110

Glu His His Ile Asp Asp Ala Leu Thr Ala Leu Asn Asn Asn Gly Leu
        115                 120                 125

Thr Ser Leu Val Ser Val Glu Val Leu Asp Lys Ser Pro Tyr Lys Ser
    130                 135                 140

Phe Arg Leu Asp Asp Asn Asp Lys Leu Ile Ser Leu Phe Asp Glu Lys
145                 150                 155                 160

Leu Ser Asn Tyr Arg Arg Gln Asp Leu Pro Leu Cys Tyr Ala Pro Asn
                165                 170                 175

Gly Ala Ile Tyr Ile Phe Ser Lys Phe Glu Phe Glu Ala Arg Gly Gly
            180                 185                 190

Phe Pro Ser Asn Gly Ser Val Ala Tyr Ile Met Ser Ala Glu Glu Ser
        195                 200                 205

Val Asp Ile Asp Thr Glu Glu Asp Phe Leu Glu Thr Glu Ala Leu Leu
    210                 215                 220

Arg Lys Lys Tyr Gly Ile Phe
225                 230

<210>  65
<211>  346
<212>  PRT
<213>  Methylobacillus glycogenes

<400>  65

Met Ala Ser Phe Glu Ile Ser Gly Arg Lys Ile Gly Asp Gly His Glu
1               5                   10                  15

Pro Ile Val Ile Ala Glu Ile Gly Ile Asn His Glu Gly Ser Leu Asp
            20                  25                  30

Leu Ala Ile Asp Met Ala Lys Ala Ala Ile Asp Ala Gly Ala Glu Ile
        35                  40                  45

Ile Lys His Gln Thr His Val Val Glu Asp Glu Met Ser Asp Glu Ala
    50                  55                  60

Lys Ser Val Ile Pro Gly Asn Ala Asp Val Ser Ile Tyr Glu Ile Met
65                  70                  75                  80

Gln Arg Cys Ser Leu Ser Glu Asp Asp Glu Tyr Lys Leu Met Gln Tyr
                85                  90                  95

Val Gln Gln Gln Gly Ser Ile Phe Ile Ser Thr Pro Phe Ser Arg Met
            100                 105                 110

Ala Ala Asn Arg Leu Arg Lys Phe Asp Ile Pro Ala Tyr Lys Ile Gly
        115                 120                 125

Ser Gly Glu Cys Asn Asn Tyr Pro Leu Val Arg His Ile Ala Ser Phe
    130                 135                 140

Gly Lys Pro Ile Ile Val Ser Thr Gly Met Asn Ser Ile Glu Ser Ile
145                 150                 155                 160

Ala Pro Thr Val Asn Ile Leu Arg Gln Ala Lys Val Pro Phe Ala Leu
                165                 170                 175

Leu His Cys Thr Asn Ile Tyr Pro Thr Pro Pro Glu Leu Val Arg Leu
            180                 185                 190

Asp Ala Ile Gln Val Ile Lys Asn Ala Tyr Pro Asp Ala Val Val Gly
        195                 200                 205

Leu Ser Asp His Thr Leu Gly Asn Tyr Ala Cys Leu Gly Ala Val Ala
    210                 215                 220

Leu Gly Ala Ser Ile Leu Glu Arg His Phe Thr Asp Ser Met Ser Arg
225                 230                 235                 240

Pro Gly Pro Asp Ile Val Cys Ser Met Asp Pro Ala Ala Leu Gln Asp
                245                 250                 255

Leu Ile Thr Gly Ser Lys Val Ile Phe Ser Ala Lys Gly Ala His Lys
            260                 265                 270

Gly Pro Leu Leu Glu Glu Glu Lys Thr Ile Ala Phe Ala Phe Ala Ser
        275                 280                 285

Val Val Ala Ile Lys Asp Ile Asn Val Gly Asp Val Leu Ser Glu Asp
    290                 295                 300

Asn Ile Trp Leu Arg Arg Pro Gly Gly Gly Asp Phe Asn Ala Ser Asp
305                 310                 315                 320

Tyr Asp Gln Leu Leu Gly Arg Lys Ala Ser Gln Thr Ile Leu Ser Gly
                325                 330                 335

Lys Arg Val Asn Arg Gln Asp Ile Glu Ala
            340                 345

<210>  66

<211>  363
<212>  PRT
<213>  Methylobacillus glycogenes

<400>  66

Met Ser Arg Arg Val Val Phe Leu Thr Gly Thr Arg Ala Asp Phe Gly
1               5                   10                  15

Lys Leu Lys Ser Leu Met Arg Ala Leu Gln Asn Asp Met Gly Phe Asp
            20                  25                  30

Val His Val Phe Ile Thr Gly Met His Met Leu Ser Lys Tyr Gly Tyr
        35                  40                  45

Thr Cys Glu Glu Val Glu Lys Ala Gly Phe Lys Asn Leu Phe Arg Phe
    50                  55                  60

Ile Asn Gln Asn Gln Ser Asp Ser Met Asp His Ile Leu Ala Lys Thr
65                  70                  75                  80

Ile Thr Gly Leu Ser Asp Tyr Val Lys Glu Ile Ser Pro Glu Leu Ile
                85                  90                  95

Val Ile His Gly Asp Arg Val Glu Ala Leu Ala Gly Ala Ala Val Gly
            100                 105                 110

Ala Leu Asn Asn Ile Arg Val Ala His Ile Glu Gly Gly Glu Val Ser
        115                 120                 125

Gly Thr Val Asp Glu Leu Ile Arg His Ala Val Thr Lys Leu Ser His
    130                 135                 140

Gly His Phe Val Ser Asn Glu Gln Ala Lys Asn Arg Leu Ile Gln Leu
145                 150                 155                 160

Gly Glu Ser Glu Glu Ser Ile Phe Val Ile Gly Ser Pro Asp Ile Asp
                165                 170                 175

Val Met Asn Ser Thr Ser Leu Pro Ser Ile Asp Asp Val Arg Glu Arg
            180                 185                 190

Tyr Gly Phe Ser Phe Glu His Tyr Gly Ile Leu Met Tyr His Pro Val
        195                 200                 205

Thr Ser Glu Leu Asp Asp Leu Pro Arg Gln Ala Lys Ala Ile Val Asp
    210                 215                 220

Tyr Ile Ser Glu Ser Ser Leu Pro Phe Ile Val Val Tyr Pro Asn Asn

225                 230                 235                 240

Asp His Gly Ser Asp Ile Ile Leu Asn Glu Tyr Leu Arg Leu Lys Asn
                245                 250                 255

Leu Ser His Val Arg Val Tyr Pro Ser Ile Arg Phe Glu Tyr Phe Leu
            260                 265                 270

Thr Leu Leu Lys Lys Cys Ser Val His His Gly Leu Arg Trp Ser Lys
        275                 280                 285

Arg Gly Ser Ser Phe Arg Arg Ala Ser His Gln Cys Trp Gln Ala Thr
    290                 295                 300

Gln Ser Asn Ser Arg Val Asp Ser Tyr Tyr Cys Thr Cys Gly Phe Pro
305                 310                 315                 320

Thr Cys Cys Ser Thr Ser Cys Arg His Ser Ser Arg Gly Ser Phe Leu
                325                 330                 335

Trp Trp Cys Arg Val Ser Ser Asp Ser Val Glu Cys Phe Leu Gly Gly
            340                 345                 350

His Thr Lys Ala Leu Tyr Arg Glu Cys Ser Val
        355                 360

<210>  67
<211>  245
<212>  PRT
<213>  Methylobacillus glycogenes

<400>  67

Met Arg Leu Ile Ala Arg Leu Asp Val Lys Asn Glu His Val Ile Lys
1               5                   10                  15

Gly Ile His Leu Glu Gly Leu Arg Lys Val Gly Asp Pro Ile Glu Met
            20                  25                  30

Ala Lys Asn Tyr Tyr Ala Gln Gly Ile Asp Glu Ile Val Phe Ile Asp
        35                  40                  45

Ala Val Ala Ser Leu Tyr Asn Arg Asn Asn Leu Leu Ser Val Ile Ala
    50                  55                  60

Lys Ala Ser Glu Glu Val Phe Val Pro Ile Thr Val Gly Gly Gly Leu
65                  70                  75                  80

Arg Ser Leu Ala Asp Val Glu Met Ala Leu Ser Ala Gly Ala Asp Lys

                85                  90                  95

Val Ala Val Asn Thr Ala Ala Ile Leu Asn Pro Lys Phe Ile Thr Glu
            100                 105                 110

Ile Ala Lys Arg Tyr Gly Ser Gln Cys Val Val Ala Ser Ile Gln Ala
        115                 120                 125

Lys Arg Ile Gly Asn Asp Trp Glu Ala Tyr Met Glu Ala Gly Arg Glu
    130                 135                 140

Pro Thr Gly Lys Asn Val Val Ala Trp Ser Lys Glu Leu Glu Asp Leu
145                 150                 155                 160

Gly Ala Gly Glu Ile Leu Leu Thr Ser Ile Asp Gln Asp Gly Thr Lys
                165                 170                 175

Lys Gly Phe Asp Leu Pro Leu Leu Gly Ala Val Asn Thr Ile Val Asn
            180                 185                 190

Ile Pro Val Met Val Ser Gly Gly Cys Gly Asn Pro Asp His Val Gln
        195                 200                 205

Glu Leu Lys Glu Ile Gln Pro Ser Gly Val Cys Leu Ala Ser Val Leu
    210                 215                 220

His Tyr Lys Ile Asn Thr Ile Asp Glu Phe Lys Gln Arg Leu Thr Phe
225                 230                 235                 240

Ile Asn Gly Thr Lys
                245

<210> 68
<211> 212
<212> PRT
<213> Methylobacillus glycogenes

<400> 68

Met Lys Lys Ile Cys Ile Leu Asp Tyr Gly Leu Gly Asn Val Arg Ser
1               5                   10                  15

Val Ser Asn Ala Ile Lys Tyr Val Gly Ala His Pro Val Ile Ser Asn
            20                  25                  30

Thr Pro Glu Ala Leu Lys Asp Ser His Ala Leu Ile Val Pro Gly Val
        35                  40                  45

Gly Ala Phe Ala Gln Gly Met Ser Ala Leu Gly Lys Asp Gly Leu Gln

50                  55                  60

Ala Ser Leu Leu Glu Tyr Ile Lys Ser Gly Lys His Val Leu Gly Ile
65                  70                  75                  80

Cys Leu Gly Met Gln Leu Leu Phe Asp Arg Gly Glu Glu Phe Gly Val
                85                  90                  95

Thr Thr Gly Leu Gly Leu Val Pro Gly Val Val Ser Arg Ile Val Pro
            100                 105                 110

Val Glu Gln Ser Arg Val Arg Leu Pro His Ile Ser Trp Ala Lys Val
        115                 120                 125

Asp Leu Asn Ser Val His Glu Asn Asn Gly Gly Met Phe Asp Gly Leu
    130                 135                 140

Pro Asp Glu Gln Arg Lys Phe Tyr Phe Leu His Ser Tyr Ala Ala Gln
145                 150                 155                 160

Asn Val Pro Asp Arg Tyr Ile Thr Gly Ser Ala Thr Tyr Ser Gly Cys
                165                 170                 175

Thr Phe Val Ala Ser Val Gln Lys Asp Asn Ile Trp Gly Met Gln Phe
            180                 185                 190

His Pro Glu Lys Ser Gly Pro Ser Gly Leu Gln Val Leu Asn Asn Phe
        195                 200                 205

Val Gln Asn Ser
    210

<210>  69
<211>  408
<212>  PRT
<213>  Methylobacillus glycogenes

<400>  69

Met Lys Arg Ser Val Glu Glu Val Val Val Asn Ala Tyr Phe Gly Leu
1               5                   10                  15

Pro Glu Asp Val Val Phe Cys Lys Lys Cys Val Ile Ser Asn Gln Arg
            20                  25                  30

Pro Ser Ser Thr Val Glu Phe Lys His Thr Asn Lys Asp Lys Lys Ala
        35                  40                  45

Thr Ile Gly Phe Gly Ala Asp Gly Ile Cys Asp Ala Cys Arg Tyr Ala

50                  55                  60

Glu Thr Lys Asp Ala Gln Ile Asp Trp Gln Ala Arg Glu Asp Ala Leu
65                  70                  75                  80

Val Arg Leu Leu Asp Gln His Arg Arg Ser Asp Gly Gly Tyr Asp Ile
                85                  90                  95

Val Val Pro Gly Ser Gly Gly Lys Asp Ser Ala Tyr Thr Ser His Ile
            100                 105                 110

Leu Lys Tyr Lys Tyr Gly Met Asn Pro Leu Thr Val Thr Trp Ala Pro
        115                 120                 125

His Lys Tyr Thr Glu Ile Gly Trp Lys Asn Phe Glu Asn Trp Ile His
    130                 135                 140

Lys Gly Gly Leu Asp Asn Ile Leu Phe Thr Pro Asn Gly Lys Leu His
145                 150                 155                 160

Arg Tyr Leu Thr Lys Met Ala Phe Leu Asn Leu Leu His Pro Phe Gln
                165                 170                 175

Pro Phe Ile Val Gly Gln Arg Ile Ile Gly Pro Leu Met Ala Lys Lys
            180                 185                 190

Phe Gly Ile Lys Leu Val Met Tyr Gly Glu Asn Gln Ala Glu Tyr Gly
        195                 200                 205

Asn Lys Thr Glu Glu Asn Tyr Lys Pro Thr Met Asp Lys Asn Phe Phe
    210                 215                 220

Ser Ile Ser Asp Pro Ala Asp Met Val Leu Gly Gly Val Lys Val Gly
225                 230                 235                 240

Asp Ile Ile Lys Asp Gly Glu Tyr Ser Leu Asn Asp Phe Ala Pro Tyr
                245                 250                 255

Ile Pro Pro Ala Ala Glu Asp Leu Glu Arg Asp Gly Ile Glu Val His
            260                 265                 270

Tyr Leu Gly Tyr Tyr Leu Lys Trp Asp Pro Gln Glu Cys Tyr Tyr Tyr
        275                 280                 285

Ala Val Glu His Thr Gly Phe Gln Ala Asn Thr Glu Arg Thr Glu Gly
    290                 295                 300

Thr Tyr Ser Lys Tyr Ser Ser Ile Asp Asp Arg Ile Asp Met Phe His
305                 310                 315                 320

Tyr Phe Thr Thr Leu Ile Lys Phe Gly Ile Gly Arg Ala Thr Tyr Asp
                325                 330                 335

Ala Ala Gln Glu Ile Arg Asn Asp Lys Ile Thr Arg Glu Glu Gly Val
            340                 345                 350

Ala Leu Val His Lys Tyr Asp Gln Glu Phe Pro Lys Lys Tyr Phe Ala
        355                 360                 365

Glu Phe Leu Glu Tyr Ile Glu Ile Thr Glu Ala Gln Phe Trp Glu Thr
    370                 375                 380

Val Asp Arg Phe Arg Ser Pro His Leu Trp Glu Lys Gln Asp Ser Asn
385                 390                 395                 400

Trp Ile Leu Lys His Gln Val Thr
                405

<210>  70
<211>  397
<212>  PRT
<213>  Methylobacillus glycogenes

<400>  70

Met Asp Thr Glu Ala Ser Gly Asp Leu Arg Met Lys Val Leu Gln Ile
1               5                   10                  15

Gly Ser Phe Ser Gly Asn Val Gly Asp Ile Ala Asn His Tyr Gly Phe
            20                  25                  30

Arg Asn Trp Phe Gln Gly Leu Leu Gly Glu Ile Gln Trp Asp Glu Phe
        35                  40                  45

Glu Ile Arg Asn Val Tyr Arg Lys Asn Val Ala Phe Asp Asp Asn Phe
    50                  55                  60

Val Ala Ile Ala Asn Gln Tyr Asp Leu Ile Val Val Gly Gly Gly Asn
65                  70                  75                  80

Phe Phe Glu Leu Trp Val Asp Ser Ser Pro Thr Gly Thr Ser Ile Ser
                85                  90                  95

Ile Glu Ile Glu Val Phe Lys Lys Ile Lys Val Pro Ile Phe Phe Asn
            100                 105                 110

Ala Leu Gly Val Asp Asp Gly Met Gly Cys Ser Asp Glu Ser Leu Ala
        115                 120                 125

Lys Phe Thr Asn Phe Leu Glu Ile Leu Thr Ser Ser Lys Gln Tyr Leu
    130                 135                 140

Val Ser Val Arg Asn Asp Gly Ala Met Gly Thr Leu Arg Asn Arg Ile
145                 150                 155                 160

Ser Asn Asn Pro Met Leu Asp Lys Val Leu Pro Leu Pro Asp Gly Gly
                165                 170                 175

Phe Phe Val Asn Leu Asp Lys Leu Glu Lys Asp Ile Ala Glu Asn Asp
            180                 185                 190

His Glu Asn Thr Gln Ile Thr Ile Gly Ile Asn Leu Ala Gly Asp Met
        195                 200                 205

Leu Glu Thr Arg Phe Pro Gly Gly Ile Asn Gly Tyr Asp Tyr Ser Gly
    210                 215                 220

Phe Ile Ser Glu Phe Thr Glu Thr Leu Ala Glu Met Trp Asn Lys Asn
225                 230                 235                 240

Lys Leu Ile Lys Phe Ile Phe Phe Pro His Ile Tyr Ser Asp Leu Lys
                245                 250                 255

Val Tyr Ala Asp Ile Leu Ser Arg Leu Pro Asp Ala Cys Arg Arg Glu
            260                 265                 270

Asn Val Lys Val Ala Ser Tyr Asp Thr Asn Val Asn Phe Gln Gly Thr
        275                 280                 285

Leu Phe Ser Glu Tyr Ser Lys Cys Thr Leu Ile Leu Gly Met Arg Phe
    290                 295                 300

His Ala Asn Val Val Ala Ile Ala Asn Lys Ile Pro Val Ile Gly Leu
305                 310                 315                 320

Tyr Ser Tyr Lys Gln Ile Lys Glu Leu Phe Tyr Glu Leu Gly Ile Lys
                325                 330                 335

Glu Lys Ile Val Asp Val Arg Gln Ile Gly Phe Lys Asn Glu Leu Leu
            340                 345                 350

Arg Lys Cys Asn Ile Val Ile Glu Asn Ser Ser Ile Ser Arg Asp Glu
        355                 360                 365

Ser Cys Asn Ile Leu Asp Val Val Thr Lys Lys Arg Asp Leu Ile Ala
    370                 375                 380

Pro Arg Val Val Glu Trp Leu Arg Glu His Lys Leu Lys
385                 390                 395

<210>  71
<211>  355
<212>  PRT
<213>  Methylobacillus glycogenes

<400>  71

Met Ile Lys Ser Leu Asp Tyr Leu Arg Ala Ile Ala Ala Leu Thr Val
1               5                   10                  15

Phe Leu Ser His Thr Phe Gln Ile Tyr Trp Leu Pro Thr Ile Gly Leu
            20                  25                  30

Val Ser Trp Lys Tyr Glu Leu Asn His Phe Leu Ser Glu Thr Ala Val
        35                  40                  45

Leu Leu Phe Phe Val Leu Ser Gly Tyr Val Ile Ser Asn Ser Leu His
    50                  55                  60

Ser Asn Thr Gln Arg Asn Gly Gly Arg Leu Ser Val Leu Asp Tyr Ala
65                  70                  75                  80

Ser Ser Arg Leu Leu Arg Ile Tyr Pro Pro Leu Ile Val Ser Ile Ile
                85                  90                  95

Ile Ser Leu Val Ala Tyr Ser Val Ile Val Ile Phe Glu Leu Pro Gly
            100                 105                 110

Ser Val Gly Thr Leu Lys Ala Pro Gly Asp Ile Tyr Gln Ala Arg Asp
        115                 120                 125

Tyr Leu Ser Ile Gly Thr Arg Asp Ile Val Asn Ser Leu Leu Met Asn
    130                 135                 140

Asn Gly Leu Leu Gln Met Asn Gly Pro Leu Trp Ser Leu Cys Ile Glu
145                 150                 155                 160

Ala Lys Leu Tyr Phe Tyr Ala Cys Leu Val Val Ile Ile Phe Ile His
                165                 170                 175

Tyr Lys Gln Lys Asn Leu Leu Asn Phe Ile Ala Asn Thr Gly Phe Leu
            180                 185                 190

Val Leu Ser Val Phe Leu Phe Phe Asn Thr Lys Thr Asp Ile Tyr Tyr
        195                 200                 205

Gly Leu Trp Trp Leu Ser Gly Gly Val Ile Phe Leu Tyr Arg Arg Asn
    210                 215                 220

Phe Ile Lys Phe Met Thr Leu Val Val Val Met Ala Ile Val Val Phe
225                 230                 235                 240

Ala Met Ala Val Ile Gly Gln Lys Phe Val Ser Leu Pro Val Gln Val
                245                 250                 255

Ile Phe Val Tyr Ser Leu Phe Leu Ala Ala Ile Lys Cys Lys Val Pro
            260                 265                 270

Glu Ile Lys Ala Met Asn Ile Leu Ala Asn Thr Ser Tyr Thr Leu Tyr
        275                 280                 285

Ile Val His Phe Pro Met Leu Leu Leu Ser Tyr Ala Ile Phe Ser Tyr
    290                 295                 300

Asn Phe Asp Val Tyr Gly Ser Leu Thr Arg Glu Leu Met Ser Ser Leu
305                 310                 315                 320

Ser Leu Ile Ile Ile Leu Thr Met Ser Ile Tyr Ile Gly Lys Trp Val
                325                 330                 335

Glu Arg Pro Ala Asn Leu Arg Leu Thr Tyr His Arg Leu Leu Arg Lys
            340                 345                 350

Val Lys Val
        355

<210> 72
<211> 559
<212> PRT
<213> Methylobacillus glycogenes

<400> 72

Met Lys Ala Met Phe Tyr Ser Leu His Ala Ala Ile Trp Leu His Ala
1               5                   10                  15

Leu Pro Glu Ser Arg Leu Ala Ser Gln Leu Ser Asn Ala Gly His Glu
            20                  25                  30

Leu Val Tyr Val Thr Cys Asp Lys Ser Phe Thr His Tyr Cys Thr Gly
        35                  40                  45

Met Ser Ala Phe Asn Leu Ala Leu Asn Glu Asp Phe Lys Lys Lys Ser
    50                  55                  60

Ala Ile Cys Glu Leu Cys Ile Asn Asn Ala Asn Lys Ile Thr Lys Ser
65                  70                  75                  80

Phe Asp Cys Lys His Leu Lys Leu His Asp Tyr Ile Asp Gln Lys Thr
                85                  90                  95

Asn Ala Leu Ile Asp Asn Leu Leu Lys Ser Val Thr Glu Asp Asn Phe
            100                 105                 110

Leu Glu Phe His Tyr Glu Asp Ile Pro Val Gly Arg Leu Ala Thr Tyr
        115                 120                 125

Glu Pro Leu Leu Lys Tyr Lys Lys Met Ser Leu Glu Phe Arg Asp Glu
    130                 135                 140

Gln Trp Gln Asp Leu Gln Ile His Leu Arg Asn Ala Leu Val Ser Leu
145                 150                 155                 160

Ile Gly Phe Ile Gln Ile Phe Lys Gln Glu Cys Pro Glu Lys Ile Phe
                165                 170                 175

Phe Tyr Ser Pro Gln Tyr Gly Val Asn Gly Val Cys Ala Glu Tyr Ala
            180                 185                 190

Val Lys Gln Gly Arg Gln Ala Tyr Phe Ile Glu Gly Ser Ser Ser Asn
        195                 200                 205

Ala Glu Arg Tyr Ser Ala Leu Arg Val Trp Glu Trp Ser Lys Phe Lys
    210                 215                 220

Leu Val Asn Pro Ala Leu Gln His Trp Gln Ala Val Lys Asn Leu Val
225                 230                 235                 240

Thr Leu Glu Asp Ile Asp Arg Val Ser Ala His Ile His Glu Leu Phe
                245                 250                 255

Lys Gly Lys Ser Phe Ala Val Phe Ser Ala Pro Lys Thr Gly Asn Phe
            260                 265                 270

Ser Ile Arg Glu Lys Phe Asn Ile Pro Gln Asn Ala Lys Ile Leu Leu
        275                 280                 285

Ala Ser Leu Ser Ser Phe Asp Glu Ala Tyr Ala Ala Phe Val Ile Lys
    290                 295                 300

Gly Phe Pro Glu Arg Lys Val Lys Ser Pro Val Phe Ser Asp Gln Phe
305                 310                 315                 320

Glu Trp Ile Lys Cys Thr Ile Asn Phe Met Lys Ser Arg Pro Asp Asp
                325                 330                 335

Tyr Leu Val Ile Arg Val His Pro Arg Asp Tyr Pro Asn Lys Arg Asp
            340                 345                 350

Ser Asn Gln Ser Glu Gln Ala Leu Ile Trp Glu Lys Ile Leu Gln Asp
        355                 360                 365

Leu Pro Glu Asn Val Lys Leu Asn Ser Pro Asp Gln Asn Ile Ser Leu
    370                 375                 380

Tyr Asp Phe Leu Asp Asp Val His Ala Leu Val Thr Gly Trp Ser Ala
385                 390                 395                 400

Thr Gly Val Glu Ala Leu Leu His Gly Ile Pro Val Ile Thr Tyr Asp
                405                 410                 415

Lys Tyr Leu Pro Ser Tyr Pro Ala Asp Ile His Leu Thr Gly Glu Ser
            420                 425                 430

Glu His Glu Tyr Tyr Asp Asn Leu Ile Lys Val Leu Ser Leu Ala Thr
        435                 440                 445

Ser Thr Asp Asn Thr His Val Val Asn Ala Tyr Arg Trp Leu Ala Val
    450                 455                 460

Ser Phe Ser Leu Gly Thr Val Arg Val Asn Gln Pro Leu Glu Ile Gly
465                 470                 475                 480

Leu Ser Trp Pro Asp Lys Pro Ala Tyr Arg Ile Leu Lys Arg Val Ala
                485                 490                 495

Asn Lys Phe Ser Val Asn Leu Arg Lys Ser Tyr Asp Val Trp Asn Val
            500                 505                 510

Gln Ser Asn Asp Thr Asp Arg Leu Arg Phe Leu Ser Leu Val Leu Asn
        515                 520                 525

Asn Gln Ser Ser Leu Phe Glu Gly Val Ile Ser Asp Val Val Asp Ala
    530                 535                 540

Val Ser Asp Tyr Glu Ile Glu Ala Thr Val Lys Gln His Ser Ile

545                 550                 555

<210>  73
<211>  568
<212>  PRT
<213>  Methylobacillus glycogenes

<400>  73

Met Asn Lys Leu Pro Gln Ala Lys Tyr Ile Phe Lys Leu Leu Lys Phe
1               5                   10                  15

Ala Phe Asn Asn Asn Pro Leu Leu Tyr Leu Ser Val Leu Ile Ser Ile
            20                  25                  30

Leu Ser Ile Gly Ile Glu Leu Leu Ala Met Ala Ser Leu Leu Pro Leu
        35                  40                  45

Phe Glu Leu Val Ser Gly Glu Thr Val Asp Lys His Gly Leu Ile Ser
    50                  55                  60

Lys Ala Leu Ile Ile Leu Gly Phe Thr Val Asp Ala Ala Ser Leu Leu
65                  70                  75                  80

Gln Val Phe Phe Leu Leu Phe Thr Leu Arg Val Val Thr Gln Leu Phe
                85                  90                  95

Ser Gln Ser Leu Thr His Tyr Leu Gly Arg Arg Val Met Ala Gln Leu
            100                 105                 110

Gly Ser Arg Ala Phe Glu Lys Ile Ile Thr Ser Met Ser Ile Asp Lys
        115                 120                 125

Ile Ser Glu Lys Ser Ile Gly Phe Phe Met Gly Leu Ala Gly Asp Glu
    130                 135                 140

Ser Phe Arg Ala Ser Thr Leu Val Val Ala Leu Thr Gln Phe Val Ala
145                 150                 155                 160

Thr Phe Leu Leu Gly Val Met Tyr Phe Ile Ser Ile Ala Ile Tyr Ser
                165                 170                 175

Leu Tyr Ala Ala Glu Met Ile Phe Ile Leu Met Leu Val Ser Ala Val
            180                 185                 190

Phe Phe Ile Trp Ile Leu Lys Ile Ser His Lys Met Gly Val Arg Gln
        195                 200                 205

Ile Glu Gly Ser Arg Lys Ala Asn Ser Ile Phe Met Asp Ala Met Asn

210                 215                 220

Asn Ile Lys Ala Val Arg Ser Leu Ser Ser Glu Lys Tyr Val Ala Gly
225                 230                 235                 240

Leu Tyr Arg Arg Lys Ile Phe Thr Tyr Ala Asn Ile Leu Phe Tyr Leu
                245                 250                 255

Asp Ala Ile Asn Leu Ala Thr Lys Leu Val Pro Ile Ile Phe Leu Leu
            260                 265                 270

Leu Gly Ser Leu Ala Leu Ile Leu Ser Gly Trp Val Ser Val Glu Asn
        275                 280                 285

Leu Gly Met Ala Tyr Ile Val Ser Leu Ile Ala Tyr Leu Met Arg Phe
    290                 295                 300

Phe Pro Val Val Gly Gln Ser Ile Thr Leu Ala Val Arg Ile Val Ala
305                 310                 315                 320

Asp Ala Lys Ser Gly Lys Asp Val Thr Glu Leu Val Ser Asp Glu Ser
                325                 330                 335

Ala Glu Ser Ala Asn Ser Asp Asn Tyr Leu His Lys Ile Asp Thr Ile
            340                 345                 350

Lys Ile Asp Asn Leu Ser Phe Ser Tyr Arg Asp Val Gln Gly Val Arg
        355                 360                 365

Pro Ala Val Leu Asn Ser Val Tyr Tyr Glu Phe Glu Lys Asn Asn Ser
    370                 375                 380

Tyr Ala Leu Val Gly Lys Ser Gly Leu Gly Lys Ser Thr Leu Leu Asp
385                 390                 395                 400

Val Leu Leu Lys Phe Tyr Pro Pro Ala Ser Gly Ser Ile Ser Ile Asp
                405                 410                 415

Gly Ile Ser Thr Glu Asn Ile Pro Gly Ala His Ile Arg His Ser Ile
            420                 425                 430

Val Leu Ile Asn Gln Glu Pro Ala Ile Phe Asp Asp Thr Val Tyr Asn
        435                 440                 445

Asn Ile Cys Met Gly Glu Thr Phe Thr Leu Glu Asp Val Lys Trp Ala
    450                 455                 460

Cys Glu Leu Ala Cys Ile Ser Lys Asp Val Glu Ser Leu Ala Gln Gly
465                 470                 475                 480

Tyr Glu Thr Arg Leu Gln Tyr Gln Gly Lys Asn Leu Ser Gly Gly Gln
                485                 490                 495

Arg Gln Arg Ile Ala Ile Ala Arg Ala Leu Val Arg Lys Pro Asn Val
            500                 505                 510

Leu Ile Leu Asp Glu Ser Thr Thr Ala Leu Asp Lys Ala Thr Gln Glu
        515                 520                 525

Ile Val Ile Arg Asn Leu Met Asn Glu Tyr Glu Asn Lys Ile Ile Ile
    530                 535                 540

Phe Val Ser His Asp Pro Tyr Ile Met Ser Gln Val Asp His Thr Val
545                 550                 555                 560

Asp Leu Ala Leu Ile Asn Lys Val
                565

<210>  74
<211>  232
<212>  PRT
<213>  Methylobacillus glycogenes

<400>  74

Met Ser Ile Ser Ser Ile Ala Leu Lys Ile Phe Asn Tyr Ser Gln Glu
1               5                   10                  15

Ile Phe Ser Phe Tyr Arg Asp Arg Gly Leu Tyr Lys Phe Ser Tyr Ala
            20                  25                  30

Gln Glu Gly Glu Asp Leu Ile Leu Ser Arg Ile Phe Asp Arg Thr Ser
        35                  40                  45

Thr Gly Tyr Tyr Ile Asp Ile Gly Ala His His Pro Val Arg Phe Ser
    50                  55                  60

Asn Thr Tyr Leu Phe Tyr Lys Arg Gly Trp Arg Gly Val Cys Ile Asp
65                  70                  75                  80

Pro Ser His Glu Ala Ile Ala Leu Phe Lys Thr Arg Arg Pro Ala Asp
                85                  90                  95

Ile Ala Leu Glu Met Gly Val Ser Asp His Glu Ser Glu Leu Thr Tyr
            100                 105                 110

His Met Phe Asp Glu Pro Ala Leu Asn Thr Phe Ser Glu Glu Leu Ala
        115                 120                 125

Asn Gln Arg Ile Gln Asn Thr Ala Tyr Lys Arg Val Gly Thr Arg Lys
    130                 135                 140

Val Pro Val Arg Pro Leu Thr Lys Ile Leu Asp Glu Val Leu Leu Pro
145                 150                 155                 160

Asp Thr Glu Ile Asp Phe Met Ser Ile Asp Val Glu Gly Phe Asp Tyr
                165                 170                 175

Gln Val Leu Ala Ser Asn Asp Trp Ser Lys Tyr Arg Pro Arg Ile Val
            180                 185                 190

Leu Ala Glu Ala Leu Asp Asp Val Ala Gly Glu Arg Ile Arg Ser Phe
        195                 200                 205

Met Ile Gln Asn His Tyr Lys Val Ile Ala Arg Thr Phe Asn Thr Glu
    210                 215                 220

Phe Tyr Gln Asp Leu Leu Lys Leu
225                 230

<210> 75
<211> 301
<212> PRT
<213> Methylobacillus glycogenes

<400> 75

Met Ser Asn Leu Ala Pro Val Ala Leu Phe Thr Tyr Asn Arg Pro Asp
1               5                   10                  15

His Thr Lys Arg Thr Ile Glu Ala Leu Lys Asn Asn Thr Leu Ser Ala
            20                  25                  30

Glu Thr Asp Leu Leu Ile Phe Ser Asp Ala Ala Lys Asn Ile Thr Gln
        35                  40                  45

Gln Ser Met Val Asp Ala Val Arg Ala Phe Thr His Thr Val Glu Gly
    50                  55                  60

Phe Lys Ser Val Arg Ile Ile Glu Arg Gln Thr Asn Phe Gly Leu Ala
65                  70                  75                  80

Ala Ser Ile Ile Asp Gly Val Thr Asn Val Leu Ala Glu Tyr Gly Arg
                85                  90                  95

Ile Ile Val Leu Glu Asp Asp Met Val Thr Ser Pro Tyr Phe Leu Gln
                100                 105                 110

Tyr Met Asn Asp Ser Leu Ser Gln Tyr Ala Asp Phe His Glu Val Ala
            115                 120                 125

Ser Ile His Ala Tyr Val Tyr Pro Ile Ala Gly Leu Pro Asp Thr Phe
    130                 135                 140

Phe Leu Arg Gly Ala Asp Cys Trp Gly Trp Ala Thr Trp Arg Asp Arg
145                 150                 155                 160

Trp Asp Trp Phe Glu Pro Asp Gly Ser Lys Leu Phe Ala Gln Leu Lys
                165                 170                 175

Gln Leu Asn Leu Leu Asn Lys Phe Asp Tyr Asn Gly Ser Tyr Pro Phe
                180                 185                 190

Ser Asn Met Leu Arg Asp Gln Ile Ala Gly Thr Asn Asn Ser Trp Ala
            195                 200                 205

Ile Arg Trp His Ala Ser Ala Phe Leu Leu Asn Lys Leu Thr Leu Tyr
    210                 215                 220

Pro Gly Lys Ala Leu Leu Ser Asn Ile Gly His Asp Gly Ser Gly Val
225                 230                 235                 240

His Cys Gly Glu Ser Glu Thr Tyr Ser Ala Glu Leu Arg Ala Asp Arg
                245                 250                 255

Leu Leu Leu Thr Asp Ile Pro Leu Arg Glu Ser Glu Pro Ala Phe Lys
                260                 265                 270

Lys Phe Glu Gln Phe Phe Leu Glu Ser Lys Pro Ser Val Val Arg Lys
            275                 280                 285

Met Met Leu Lys Ala Lys Ser Val Phe Lys Lys Phe Leu
    290                 295                 300

<210>  76
<211>  271
<212>  PRT
<213>  Methylobacillus glycogenes

<400>  76

Met Ser Phe Ile Lys Ile Leu Lys Leu Leu Cys Pro Pro Leu Phe Leu
1               5                   10                  15

His Ile Leu Gln Lys Tyr Arg Arg Arg Asn Phe Ala Ser Ser Phe Ala
            20                  25                  30

Gly Asn Tyr Ser Ser Trp Ala His Ala Lys Gln Asn Ser Gly Gly Tyr
        35                  40                  45

Asp Ala Glu Asn Ile Leu Thr Ile Ile Gln Glu Ser Ala Leu Lys Val
    50                  55                  60

Lys Arg Gly Glu Ala Val Phe Glu Arg Asp Ser Val Cys Phe His His
65                  70                  75                  80

Val Glu Tyr Arg Tyr Ser Ala Leu Ser Gly Leu Leu Tyr Ala Ala Val
                85                  90                  95

Ser Ser Gly Gly Lys Leu Ser Val Leu Asp Phe Gly Gly Ser Leu Ala
            100                 105                 110

Ser Phe Tyr Ser Gln His Ala Ala Ile Leu Ser Ile Val Lys Asp Leu
        115                 120                 125

Arg Trp Gly Val Val Glu Gln Pro His Phe Val Ala Trp Gly Arg Glu
    130                 135                 140

His Leu Thr Thr Thr Asp Leu Lys Phe Tyr Asn Ser Leu Thr Glu Phe
145                 150                 155                 160

Ile Leu Asp Gly Lys Pro Asn Val Ile Phe Leu Ser Ser Val Leu Gln
                165                 170                 175

Tyr Leu Glu His Pro Tyr Gln Leu Leu Glu Glu Leu Leu Arg Leu Asp
            180                 185                 190

Ser Glu Phe Ile Leu Phe Asp Arg Thr Ala Phe Tyr Asp Val Val Asp
        195                 200                 205

Arg Leu Thr Val Gln Thr Val Pro Ala Ser Ile Tyr Pro Ala Ser Tyr
    210                 215                 220

Pro Ala Trp Phe Ile Ser Trp Asp Lys Phe Ile Asn Thr Met Ser Ala
225                 230                 235                 240

Gly Gly Tyr Glu Val Ile Met Glu Phe Pro Cys Ala Asp Leu Thr Asp
                245                 250                 255

Ile Gly Tyr Tyr Lys Gly Leu Phe Phe Lys Arg Lys Ser Val Ser
            260                 265                 270

<210>  77
<211>  332
<212>  PRT
<213>  Methylobacillus glycogenes

<400>  77

Met Asn Asp Glu Leu Tyr Ser Gly Lys Asn Ile Leu Val Thr Gly Gly
1               5                   10                  15

Leu Gly Phe Ile Gly Ser Asn Leu Val Lys Arg Leu Ile Ala His Lys
            20                  25                  30

Ala Lys Val Thr Val Val Asp Ser Leu Leu Pro Leu Tyr Gly Gly Asn
        35                  40                  45

Ala Phe Asn Leu Ser Gly Tyr Glu Ser Leu Val Asn Ile Asn Ile Ser
    50                  55                  60

Asp Val Arg Asp Ser His Ala Met Ala Tyr Leu Ile Lys Gly Lys Asp
65                  70                  75                  80

Tyr Leu Phe Asn Leu Ala Gly Gln Thr Ser His Leu Asp Ser Met Thr
                85                  90                  95

Asp Pro Arg Thr Asp Leu Asp Ile Asn Ala Ala Ala Gln Leu Ser Ile
            100                 105                 110

Leu Glu Ala Cys Arg Gln His Asn Pro Asp Val Arg Ile Val Phe Ala
        115                 120                 125

Ser Thr Arg Gln Leu Tyr Gly Lys Pro Glu Tyr Leu Pro Val Asp Glu
    130                 135                 140

Ser His Pro Ile Gln Pro Val Asp Val Asn Gly Ile Asn Lys Leu Ala
145                 150                 155                 160

Gly Glu Trp Tyr His Ile Leu Tyr Asn Asn Val Tyr Gly Ile Lys Ala
                165                 170                 175

Cys Ala Leu Arg Leu Thr Asn Thr Tyr Gly Pro Gly Met Arg Val Lys
            180                 185                 190

Asp Ala Arg Gln Thr Phe Leu Gly Ile Trp Leu Arg Arg Val Ile Glu
        195                 200                 205

Gly Lys Pro Ile Ala Val Phe Gly Asp Gly Leu Gln Leu Arg Asp Phe
    210                 215                 220

Asn Tyr Val Asp Asp Val Val Asp Ala Leu Leu Leu Ala Ala Ala Asn
225                 230                 235                 240

Thr Glu Ala Asp Gly Gln Ile Phe Asn Leu Gly Ser Asp Glu Val Val
                245                 250                 255

Asn Leu Gln Gln Val Ala Glu Leu Met Thr Gln Phe Gln Pro Gly Ser
            260                 265                 270

Ser Tyr Glu Ile Val Pro Phe Pro Pro Glu Arg Lys Ala Ile Asp Ile
        275                 280                 285

Gly Asp Tyr Tyr Ser Asp Phe Asn Lys Ile Lys Gln Val Leu Gly Trp
    290                 295                 300

Ser Pro Arg Val Gly Leu Gln Gln Gly Leu Ala Ala Ser Leu Ala Tyr
305                 310                 315                 320

Tyr Arg Gln His His Asp Lys Tyr Trp Asp Asn Val
                325                 330

<210>  78
<211>  366
<212>  PRT
<213>  Methylobacillus glycogenes

<400>  78

Met Ile Leu Met Asn Asp Phe Lys Ala Glu Pro Glu Ala Leu Ile Ala
1               5                   10                  15

Ala Gln Leu Gln Ala Val Glu Arg Val Leu Arg Ser Gly Trp Tyr Val
            20                  25                  30

Leu Gly Glu Glu Gly Arg Lys Phe Glu Ser Leu Trp Ser Gln Arg Cys
        35                  40                  45

Gly Thr Ser His Thr Val Gly Val Gly Asn Gly Met Asp Ala Ile Glu
    50                  55                  60

Ile Gly Leu Arg Ala Leu Asp Ile Gly Pro Gly Asp Glu Val Ile Thr
65                  70                  75                  80

Thr Ala Met Thr Ala Phe Ala Ser Val Leu Ala Ile Ile Arg Ala Gly
                85                  90                  95

Ala Thr Pro Val Leu Ala Asp Ile Asp Pro Asp Thr Gly Leu Leu Ser
            100                 105                 110

His Glu Ser Val Lys Arg Cys Ile Asn Ser Arg Thr Lys Ala Val Leu
        115                 120                 125

Leu Val His Leu Tyr Gly Gln Ile Arg Asp Met Gln Ser Trp Leu Asn
    130                 135                 140

Leu Cys Asp Gln His Gln Val Ala Leu Leu Glu Asp Cys Ala Gln Ala
145                 150                 155                 160

His Leu Ala Gln Ala Glu Gly Lys Val Ala Gly Ser Phe Gly Arg Phe
                165                 170                 175

Gly Ala Tyr Ser Phe Tyr Pro Thr Lys Asn Leu Gly Ala Ile Gly Asp
            180                 185                 190

Gly Gly Ala Leu Val Thr Ser Gln Ser Ala Leu Ala Asp Lys Ala Ala
        195                 200                 205

Val Leu Arg Asn Tyr Gly Gln Ser Val Arg Tyr His His Pro Glu Phe
    210                 215                 220

Gly Leu Asn Ser Arg Leu Asp Glu Ile Gln Ala Ala Leu Leu Thr Val
225                 230                 235                 240

Arg Leu Glu Trp Leu Glu Gln Tyr Thr Ser His Arg Gln Ser Ile Ala
                245                 250                 255

Ala Gln Tyr Arg Gln Gln Leu Asn Asn Pro Tyr Val Arg Leu Met Gln
            260                 265                 270

Thr Pro Thr Glu Pro Leu Ala His Val Tyr His Leu Phe Val Val Leu
        275                 280                 285

Thr Glu His Arg Glu Ala Leu Gln Ala His Leu Gln Ala Asn Gly Val
    290                 295                 300

Gln Ala Leu Ile His Tyr Pro Val Pro Ile His His Gln Glu Lys Cys
305                 310                 315                 320

Ser Asp Ile Leu Arg Asp Pro Ala Gly Leu Gln Phe Ser Glu Ala His
                325                 330                 335

Ala Gln Gln Cys Leu Ser Leu Pro Cys His Pro Gln Met Ser Glu Gln
            340                 345                 350

Asp Val Gln Thr Val Ile Ala Ala Val Asn Arg Phe Ala Leu
        355                 360                 365

<210>  79
<211>  313
<212>  PRT
<213>  Methylobacillus glycogenes

<400>  79

Met Glu His Phe Val Thr Leu Phe Asp Ser Lys Phe Leu Pro Gln Gly
1               5                   10                  15

Leu Cys Leu His Glu Ser Leu Ala Arg His Ala Arg Asp Phe His Leu
            20                  25                  30

Trp Ile Val Cys Met Asp Lys Val Val His Glu Gln Leu Glu Lys Leu
        35                  40                  45

Gln Leu Pro His Val Ser Leu Met Arg Leu Ala Asp Phe Glu Ser Glu
    50                  55                  60

Ala Leu Leu Ala Val Lys Pro Gly Arg Thr Lys Gly Glu Tyr Cys Trp
65                  70                  75                  80

Thr Ile Thr Pro Phe Thr Ala Glu Trp Val Phe Ala Gln His Pro Glu
                85                  90                  95

Val Ala Arg Val Thr Tyr Leu Asp Ala Asp Leu Phe Phe Phe Asn Asp
            100                 105                 110

Pro Ala Ile Leu Phe His Glu Phe Glu Gln Ser Gly Lys His Val Met
        115                 120                 125

Ile Thr Glu His Ala Tyr Ala Pro Asp Tyr Ile Gln His Leu Glu Thr
    130                 135                 140

Ser Gly Arg Phe Cys Val Gln Phe Met Val Phe Asp Arg Ser Glu Ala
145                 150                 155                 160

Ser Arg Lys Val Met His Trp Trp Gln Asp Arg Cys Val Glu Trp Cys
                165                 170                 175

Phe Ala Arg Met Glu Asp Gly Lys Phe Gly Asp Gln Lys Tyr Leu Asp
            180                 185                 190

Leu Trp Pro Thr Ile Phe Ala Asp Asp Val His Ile Leu Lys Cys Val
        195                 200                 205

Glu Gln Thr Leu Ala Pro Trp Asn Val Asp Tyr Gln Leu Gln His Gln
    210                 215                 220

Ser Gly Lys Phe Lys Pro Val Phe Tyr His Phe His Ala Phe Arg Ile
225                 230                 235                 240

Ile Lys Phe Asn Leu Val Arg Leu Tyr Glu Gly Tyr Gln Ile Ala Lys
                245                 250                 255

Ser Ser Arg Tyr Leu Tyr Asp Glu Tyr Leu Ala Thr Leu Gln Lys Lys
            260                 265                 270

Ile Gly Met Leu His Arg Phe Ala Ile Pro Leu Pro Leu Leu Pro Glu
        275                 280                 285

Lys Cys Gly Ile Arg Pro Lys Leu Ala Ser Tyr Tyr Thr Trp Ile Arg
    290                 295                 300

Gly Phe Thr Ala Thr Ala Lys Ile Lys
305                 310

<210>  80
<211>  255
<212>  PRT
<213>  Methylobacillus glycogenes

<400>  80

Met Gln Lys Leu Ser Val Ile Thr Ala Thr Tyr Asn Ala Ala Lys Asn
1               5                   10                  15

Leu Pro Asp Leu Val Gln Ser Leu Leu Ser Gln Thr Asp Met Asp Phe
            20                  25                  30

Glu Trp Val Val Ala Asp Gly Gly Ser Ser Asp Glu Thr Ile Ser Tyr
        35                  40                  45

Leu Lys Ser Val Val Gly Ile Asn Leu Leu Leu Ser Ala Gln Gln Asp
    50                  55                  60

Phe Gly Ile Tyr Asp Ala Leu Asn Arg Ala Ile Ser Leu Ser Ser Gly
65                  70                  75                  80

Asp Tyr Tyr Leu Val Leu Gly Ala Asp Asp Ile Leu Tyr Pro His Ala
                85                  90                  95

Val Glu Ala Phe Lys Arg Ala Ala Gly Ile Gly Leu Pro Asp Leu Val
            100                 105                 110

Thr Ala Ser Val Glu Ser Asp His Gly Val Met Ile Ala Gly Ser Gly
        115                 120                 125

Arg Pro Phe Leu Arg Gly Gln Leu Ala Tyr Ile Ser Asn His Ser Val
    130                 135                 140

Gly Thr Met Ile Lys Lys Ser Leu His Asp Arg Phe Gly Tyr Tyr Ser
145                 150                 155                 160

Arg Arg Phe Pro Ile Thr Ala Asp Gln Tyr Phe Ile Lys Arg Val Cys
                165                 170                 175

Gly Ala Ser Glu Thr Val Ile Tyr Lys Ala Asp Phe Val Ala Gly Tyr
            180                 185                 190

Tyr Pro Ala Thr Gly Val Ser Gly Ala Asp Val Pro Gly Thr Met Val
        195                 200                 205

Glu Phe Phe Arg Val Gln Leu Glu Thr Glu Pro Asn Ser Val Leu Gln
    210                 215                 220

Leu Val Leu Phe Ile Leu Arg Leu Ile Arg His Thr Pro Lys Leu Ile
225                 230                 235                 240

Arg Tyr Ala His Tyr Lys Lys Leu Arg Asp Leu Pro Gln His Glu
                245                 250                 255

<210>  81
<211>  360
<212>  PRT
<213>  Methylobacillus glycogenes

<400>  81

Met Asn Ser Asn Val Leu Phe Ile Thr Gly Phe His Pro Gly Gly Met
1               5                   10                  15

Gly Phe Ile Gly Ala Gly Glu Ala Ile Ser Gln Gln His Ile Glu Arg
            20                  25                  30

Leu Ile Lys Gln Gly Arg Lys Val His Val Leu Ala Leu Ala Pro Asn
        35                  40                  45

Tyr Gln Val Ala Ser Pro Glu Val Val Ala Ser Cys Ala Ser Tyr Thr
    50                  55                  60

Thr Leu Thr His Thr Arg Trp Met Thr Phe Ala Ala Leu Trp Arg Ser
65                  70                  75                  80

Arg Gln Gln Gly Ser Leu Leu Cys Pro Trp Leu Phe Thr Arg Val Ser
                85                  90                  95

Pro Gln Ala Glu Arg Phe Ile Arg Gln Leu Leu Asp Gln His Ala Ile
        100                 105                 110

Thr Glu Ile Tyr Leu Asp Phe Pro Ser Cys Leu Gly Phe Ala Pro Thr
        115                 120                 125

Tyr Asn Gln Leu Pro Ile His Tyr Ile Val His Asp Val Leu Ser Gln
    130                 135                 140

Lys Val Glu Arg Arg Pro Leu Leu Lys Trp Leu Lys Asn Gln Val Leu
145                 150                 155                 160

Arg Thr Glu Ser Ser Leu Leu Arg His Val Thr Gln Cys Phe Thr Leu
                165                 170                 175

Ser Leu Lys Asp Lys Leu Arg Cys Glu Glu Ile Gly Tyr Ser Gly Gln
            180                 185                 190

Ile Glu Val Gln Val Pro His Ile Leu Lys Val Gly Val Val Asn Gln
        195                 200                 205

Gly Val Pro Ile Ala Gly Ile Leu Lys Gln Phe Pro Gly Lys Lys Asn
    210                 215                 220

Leu Val Phe Phe Gly Asn Met Gln Arg Ala Glu Asn His Tyr Ser Ile
225                 230                 235                 240

Leu Arg Phe Leu Leu Cys Ser Tyr Pro Gly Ile Trp Leu Glu His Arg
                245                 250                 255

Asp Val Gln Cys Trp Ile Leu Gly Leu Ala Pro Arg Thr Leu Leu Arg
            260                 265                 270

Trp Ile Ser Lys Val Leu Pro Gly Val Arg Val Thr Gly Ala Val Asp
        275                 280                 285

Asp Pro Thr Glu Ala Phe Lys Ala Ala Thr Leu Cys Ile Ala Pro Val
    290                 295                 300

Tyr Tyr Gly Ala Gly Val Lys Ile Lys Val Leu Gln Met Leu Glu Ser
305                 310                 315                 320

Gly Ala Thr Val Ile Ser Thr Pro Val Gly Ala Glu Gly Ile His Gly
                325                 330                 335

Ser Glu Lys Leu Ile Thr Val Glu Ser Ala Arg Leu Ser Ser Thr Ile

            340                 345                 350

Lys Gln Arg Leu Ala Val Glu Ser
        355                 360

<210> 82
<211> 307
<212> PRT
<213> Methylobacillus glycogenes

<400> 82

Met Asn Gln His Glu Leu Pro Ser Val Ala Val Leu Leu Ala Ser Phe
1               5                   10                  15

Asn Gly Arg Ala Trp Ile Ala Glu Gln Leu Ala Ser Ile Leu Ala Gln
            20                  25                  30

Gln Gly Val Lys Leu His Val Phe Ile Ala Asp Asp Leu Ser Thr Asp
        35                  40                  45

Gly Thr Trp Glu Trp Leu Asn Val Glu Ala Ala Cys His Gln Asn Val
    50                  55                  60

Thr Leu Leu Pro His Asn Gln Arg Ala Gly Ser Ala Gly Arg Asn Phe
65                  70                  75                  80

Tyr Arg Leu Ile His Glu Val Asp Leu Asp Gly Phe Asp Phe Val Ala
                85                  90                  95

Phe Ala Asp Gln Asp Asp Val Trp Asp Ser Tyr Lys Leu Ser Arg His
            100                 105                 110

Ala Arg Leu Met Lys Leu His Gln Ala Asp Gly Val Ser Ser Asn Val
        115                 120                 125

Glu Ala Phe Trp Pro Asp Gly Arg Val Met Leu Ile Val Lys Ser Gln
    130                 135                 140

Ala Gln Arg Gln Phe Asp Tyr Leu Phe Glu Ser Ala Gly Pro Gly Cys
145                 150                 155                 160

Thr Phe Leu Met Thr Thr Arg Leu Ala His Gln Val Arg Asn Val Leu
                165                 170                 175

Gln Asp Glu Leu Ser Pro Ala Arg Asp Val Thr Leu His Asp Trp Leu
            180                 185                 190

Thr Tyr Ala Ile Ala Arg Ser Leu Gly Tyr Thr Trp Val Ile Asp Ala

195                 200                 205

Asn Pro Ser Leu Lys Tyr Arg Gln His Gly Asn Asn Val Ile Gly Ala
    210                 215                 220

Asn Val Ser Leu Lys Ala Lys Trp Thr Arg Leu Lys Gln Ile Arg Asp
225                 230                 235                 240

Gly Trp Tyr Arg Gln Gln Val Ile Lys Val Thr Arg Ile Ala Tyr Ala
                245                 250                 255

Phe Ser Gln Arg Glu Asp Leu Gln Arg Leu Leu Val Leu Leu Glu Lys
            260                 265                 270

Pro Ser Leu Gly Asn Arg Leu Gln Leu Leu Pro Tyr Ala Trp His Gly
        275                 280                 285

Arg Arg Ser Val Lys Asp Arg Leu Phe Leu Met Phe Ser Ile Val Phe
    290                 295                 300

Lys Trp Phe
305

<210>  83
<211>  317
<212>  PRT
<213>  Methylobacillus glycogenes

<400>  83

Met Ser Gln Gln His Ile Leu Val Thr Gly Ala Asn Gly Phe Val Gly
1               5                   10                  15

Arg Ser Leu Cys Asn Gln Leu Cys Gln Ser Ala Phe Gln Val Thr Gln
            20                  25                  30

Val Gly Arg Ala Leu Asn Ser Ala Asp Gly Gly Ala Tyr Ser Gln Val
        35                  40                  45

Val Ser Ile Asp Gly Thr Thr Asp Trp Ser Thr Ile Leu Asp Glu Val
    50                  55                  60

Glu Val Val Ile His Leu Ala Ala Arg Val His Val Met Gln Glu Asn
65                  70                  75                  80

Leu Val Asp Pro Ile Val Ala Tyr Trp Gln Thr Asn Val Glu Gly Thr
                85                  90                  95

Leu Asn Leu Ala Arg Gln Ala Leu Asn Ala Gly Val Lys Arg Phe Ile

            100                 105                 110

Phe Val Ser Ser Ile Lys Val Asn Gly Glu Gln Ser Phe Pro Gly Lys
        115                 120                 125

Pro Phe Thr Ala Glu Asp Met Pro Met Pro Glu Asp Ala Tyr Gly Lys
    130                 135                 140

Ser Lys Trp Glu Ala Glu Gln Gly Leu Leu Ala Leu Ala Lys Asp Ser
145                 150                 155                 160

Asn Met Val Val Val Ile Ile Arg Pro Pro Leu Val Tyr Gly Pro Gly
                165                 170                 175

Val Lys Gly Asn Phe Ser Thr Met Ile Lys Leu Leu Gln Thr Gly Leu
            180                 185                 190

Pro Leu Pro Phe Gly Ala Ile Ala Asn Gln Arg Ser Leu Val Ala Ile
        195                 200                 205

Asp Asn Leu Ile His Leu Ile Met Val Cys Ile Asn His Pro Ala Ala
    210                 215                 220

Ala Asn Gln Val Phe Leu Val Ser Asp Gly Glu Asp Leu Ser Thr Thr
225                 230                 235                 240

Ala Leu Leu Arg Arg Leu Ala Leu Ala Ala Gly Val Ser Ser Arg Leu
                245                 250                 255

Ile Pro Phe Pro Ala Ala Trp Val Asn Phe Val Phe Thr Val Leu Gly
            260                 265                 270

Lys Lys Met Met Ala Gly Arg Ile Leu Gly Ser Leu Gln Val Asp Ile
        275                 280                 285

Ser Lys Thr Arg Glu Val Leu Gly Trp Gln Pro Pro Val Thr Val Asp
    290                 295                 300

Gln Gly Leu Arg Asn Cys Phe Pro Thr Lys Ala Glu Pro
305                 310                 315

<210> 84
<211> 183
<212> PRT
<213> Methylobacillus glycogenes

<400> 84

Met Ile Arg Phe Phe Asp Ile Val Phe Ser Leu Leu Gly Leu Val Phe

1               5                   10                  15

Gly Leu Pro Leu Leu Ile Ile Ile Tyr Leu Val Gly Leu Leu Asp Thr
            20                  25                  30

Gly Ser Pro Leu Phe Arg Gln Val Arg Val Gly Arg His Arg Gln Pro
        35                  40                  45

Phe Thr Leu Val Lys Phe Arg Thr Met Lys Val Asn Thr Ala Ser Val
    50                  55                  60

Ala Ser His Leu Ala Ser Ala Ser Ser Ile Thr Trp Leu Gly Ser Phe
65                  70                  75                  80

Leu Arg Ser Thr Lys Leu Asp Glu Leu Pro Gln Leu Trp Asn Val Leu
                85                  90                  95

Lys Gly Glu Met Ser Leu Val Gly Pro Arg Pro Asn Leu Phe Asn Gln
            100                 105                 110

Asp Glu Leu Ile Ala Ala Arg Asp Ala Arg Gly Val Tyr Ala Val Arg
        115                 120                 125

Pro Gly Ile Thr Gly Leu Ala Gln Val Asn Asp Ile Asp Met Ser Met
    130                 135                 140

Pro Lys Leu Leu Ala Glu Thr Asp Ala Ser Met Leu Gln Gln Leu Asn
145                 150                 155                 160

Leu Lys Leu Tyr Phe Lys Leu Leu Ile Gln Thr Val Leu Gly Lys Gly
                165                 170                 175

Arg Gly Asp Arg Val Val Arg
            180

<210>  85
<211>  573
<212>  PRT
<213>  Methylobacillus glycogenes

<400>  85

Met Leu Ala Trp Trp Val Ala Phe Leu Phe Arg Phe Asn Phe Asp Ile
1               5                   10                  15

Pro Ala Pro Phe Ala Glu Ser Met Leu Gln Ser Ala Ala Trp Val Ile
            20                  25                  30

Pro Leu Gln Ala Thr Phe Phe Leu Leu Cys Gly Leu Tyr Arg Gly Val

35                  40                  45

Trp Arg Phe Ala Ser Leu Pro Asp Leu Gln Arg Leu Ile Arg Ala Ile
    50                  55                  60

Gly Val Ala Ala Val Val Val Val Ala Ile Leu Phe Met Phe Gln Pro
65                  70                  75                  80

His Gly Val Val Pro Arg Ser Val Leu Ile Met Asn Pro Met Leu Leu
                85                  90                  95

Leu Leu Leu Met Gly Gly Ser Arg Ile Ala Tyr Arg Ala Trp Lys Glu
            100                 105                 110

His Arg Leu Tyr Gly Ala Ala Gln Ser Ser Gly Lys Pro Val Leu Val
        115                 120                 125

Leu Gly Ala Gly Asp Ala Ala Ile Ala Leu Leu Lys Asp Leu Ser Lys
    130                 135                 140

Ser His Glu Trp Arg Ile Val Gly Leu Leu Asp Asp Lys Gln Ser Met
145                 150                 155                 160

Phe Gly Arg Glu Leu Leu Gly Val Lys Val Leu Gly Lys Leu Ser Asp
                165                 170                 175

Phe Pro Glu Ile Ala Lys Ala Met Ser Val Gln His Val Ile Val Ala
            180                 185                 190

Ile Pro Ser Ala Arg Asn Asn Ala Gly Ala Arg Leu Leu Ile Trp Pro
        195                 200                 205

Val Ser Thr Asn Ser Arg Tyr Leu Pro Cys His His Trp Lys Ile Val
    210                 215                 220

Gly Val Ser Ala Trp Arg Ser Tyr Val Arg Leu Arg Leu Lys Ile Cys
225                 230                 235                 240

Leu Gly Val Ser Leu Trp Cys Trp Thr Met Met Val Cys Thr Ser Ser
                245                 250                 255

Ile Val Arg Trp Leu Trp Ser Leu Ala Arg Ala Ala Arg Leu Val Gln
            260                 265                 270

Asn Phe Ala Asp Ser Tyr Ser Ser Phe Ser Leu Gln Cys Trp Cys Val
        275                 280                 285

Ser Thr Ile Arg Ser Phe Leu Phe Ile Ala Ser Lys Asn Pro Leu Ser
    290                 295                 300

Trp Met Cys Gly Leu Asn Ile Trp Gly Thr Ser Arg Met His Asp Ala
305                 310                 315                 320

Cys Ser Thr Cys Leu Leu Val Ile Ser Gln His Ser Ile Ser Cys Cys
                325                 330                 335

Ser Leu Thr Cys Ala Tyr Asp Gly Lys Pro Arg Cys Arg Val Ile Ile
            340                 345                 350

Gln Cys Ala Gly Tyr Phe His Ser Ser Gln Cys Met Gly Gly Gly Arg
        355                 360                 365

Val Cys Ala Gly Ile His Arg Ser Gly Pro Asn Gln Cys His Gly Gly
    370                 375                 380

Gln Thr Ser Gly Asn Gly Val Ser Arg Phe Ala Gly Gly Asn Arg Asn
385                 390                 395                 400

Pro Leu Cys Gly Ser Ala Phe Arg Gln Cys Ala Gly Gln Gln Trp Cys
                405                 410                 415

Asp Pro Val Arg Ala Asn Cys Ala Trp Trp Ala Ser His Arg Tyr Ser
            420                 425                 430

Ser Arg Asp His Ala Leu Phe His Val Tyr Thr Gly Val Pro Val Gly
        435                 440                 445

His Ala Ser Arg Ile Asp Gly Gly Arg Arg Asn Ile Arg Ala Arg Tyr
    450                 455                 460

Gly Pro His Gln Asp Cys Gly Leu Gly Ala Cys Tyr Asp Val Val Arg
465                 470                 475                 480

Val Gly Gly Gly Arg His Cys Phe Phe Arg Pro Ala Pro Trp Arg Glu
                485                 490                 495

Ala Val Arg Gly Thr Phe Gly Arg Arg Arg Thr His Phe Thr Asp Ala
            500                 505                 510

Ala Arg Ile Ala Tyr Arg Arg Gly Lys Cys Arg Gly Leu Gly Cys Gly
        515                 520                 525

Phe Ala Gln Val Gly Arg Gln Val Ile Gln His Gly Gly Ser Arg Glu
    530                 535                 540

Ala Arg Ala Gln Asp Val Gly Gly Gly Val Cys Arg Tyr Thr Cys Ser
545                 550                 555                 560

Phe Phe Cys Val Asp Ser Arg Val Ile Asp Asn Thr Leu
                565                 570

<210>  86
<211>  291
<212>  PRT
<213>  Methylobacillus glycogenes

<400>  86

Met Thr Lys Lys Ile Thr Lys Ala Val Phe Pro Val Ala Gly Leu Gly
1               5                   10                  15

Thr Arg Phe Leu Pro Ala Thr Lys Ala Ser Pro Lys Glu Met Leu Pro
            20                  25                  30

Ile Val Asp Lys Pro Leu Ile Gln Tyr Ala Ala Glu Glu Ala Val Ala
        35                  40                  45

Ala Gly Ala Thr Glu Leu Ile Phe Ile Ile Gly Arg Asn Lys Arg Ser
    50                  55                  60

Ile Pro Asp His Phe Asp Lys Ser Phe Glu Leu Glu Ala Ser Leu Glu
65                  70                  75                  80

Ala Ser Lys Lys Thr Lys Met Leu Glu Glu Ile Gln Asn Ile Leu Pro
                85                  90                  95

Lys His Val Ser Cys Ile Tyr Ile Arg Gln Ala Glu Ala Leu Gly Leu
            100                 105                 110

Gly His Ala Val Leu Leu Ala Lys Ser Val Val Gly Asn Glu Pro Phe
        115                 120                 125

Ser Val Ile Leu Ala Asp Asp Leu Ile Asp Ala Glu Lys Pro Ala Met
    130                 135                 140

Gln Gln Met Thr Glu Leu Phe Glu Lys Gln Gly Ala Ser Ile Leu Gly
145                 150                 155                 160

Val Glu Asp Val Glu Pro Ser Glu Thr Ala Ser Tyr Gly Ile Val Asp
                165                 170                 175

Ala Val Glu Leu Thr Gly Pro Asp Val Leu Gln Leu Asn Gly Ile Val
            180                 185                 190

Glu Lys Pro Gln Pro Glu Asp Ala Pro Ser Asn Leu Ala Val Val Gly
        195                 200                 205

Arg Tyr Ile Leu Thr Pro Glu Ile Phe Thr Cys Leu Glu Asn Val Lys
    210                 215                 220

Pro Gly Lys Gly Gly Glu Ile Gln Leu Thr Asp Gly Ile Ala Ala Leu
225                 230                 235                 240

Met Glu Gln Gln Lys Ile Leu Ala Tyr Arg Phe Lys Gly Lys Arg Tyr
                245                 250                 255

Asp Cys Gly Ser Lys Leu Gly Tyr Val Lys Ala Asn Val Glu Phe Ser
            260                 265                 270

Leu Lys His Glu Glu Ile Gly Glu Glu Phe Ser Gln Tyr Leu Arg Glu
        275                 280                 285

Leu Thr Lys
    290

<210> 87
<211> 774
<212> DNA
<213> Methylobacillus flagellatus

<400> 87
atgtccttaa ataaaaactt aaacgatatt gagaaaagcc agttaattga gctaatgcat 60

gaatctttga gaattcgctc gcactttgag ctttttctct ggatgcaggg aaaacttcag 120

gttttcctgc cgcatgaaat catgataacg gcttggggag acttttcact tggagtcata 180

tacttcaaca ttgtttcccc cctgccaggc atccgctccg acaagattgc cagtcatgaa 240

atcaatcagt tcctgcaaaa cctgttcaac tattggctga accatgccaa ggcccctttc 300

acgctcgcag cagccgacaa catcctgcat gactgcgaat tctctaacaa agaatcaagc 360

tattacttaa aaaacatgaa atcggcattg gtgcatgcca tcaaggatca ccgtggcagg 420

catgattgcc tttatgtatt gcttagctcc aacctgagct tccccccagc ctctcgtccc 480

atgctggaag cgcttctacc ctatatcgat agcgcattgc ggcagataga acacttgccg 540

gagcagcaac cgacactgat caaggcagaa cccgagccca aggatgaaag cctgaatatc 600

ctttccggcc gcgagctgga gatcatggac tgggtcacca aaggcaaaac caaccaggaa 660

attggcatga ttcttgatat cagcgccttt actgtcaaaa atcatttaca gcgcattttc 720

aaaaaactca atgttatgaa tcgtgcacaa gccgtcgcca aattccgcaa ataa 774

<210> 88

<211>  507
<212>  DNA
<213>  Methylobacillus flagellatus

<400>  88
atgtatcctg atctggtgca tctgggcggt gcggataaat atttcgagga aatcctcgag      60

attgtaaata aaatcaagct gttcggggac ttttccaacg aggaagtgcg ttatttgtgc     120

tcctatatgc agtgctatgc tgcacccagg gactgtcagc tgctgacgga gggcgatcca     180

ggagattact tgctcctgat cctgacggga gaagtgaacg tcatcaagga tattccgaac     240

aagggaatac agaccattgc caaggtcggg gctggcgcca tcattggtga aatgtcaatg     300

attgatggca tgccgcgctc tgccagctgt gttgccagct tgcccactga ctttgccgtg     360

ctgagcaggg atgcgctata tcaattgctg gccaatatgc ccaagctagg caacaaggtg     420

ctgatcaggc tgttgcaatt gctcactgca aggtttcgtg aaagctatga caggattctc     480

cccaaaaccc tgggtgagtt gatctga                                         507


<210>  89
<211>  678
<212>  DNA
<213>  Methylobacillus flagellatus

<400>  89
atgtttttat cccgtttaca tacgccgatc ctgagccgtg tagcgccatt tgcattgtat      60

atgctcttca tggcagtggt ggacttggta gtccggatcg gcttggtgga cgccgatacg     120

cgttggctct atggcatcaa ggtcgggctg gtcgccctat tgctttggta tctacgcaag     180

cagtatgtcg agctccaggg gctagccgcg ctgggagcca ggtcgtggtt gagcgcaatc     240

gccgttggta tgctagtatt tatcgcctgg atcaatctgg atgcgccatg gatgttgatt     300

ggcgatcaat ccgaagggtt cgatccggga agcggaccgg atgcccgtct gctgatggcg     360

acaagagtga tgggggccgc attggtcgtg cccttgatgg aagagctgtt ctggcgttca     420

ttcttgatgc gctggattca caaccaacat ttcctcaatg tcgaccctcg gcaggtaagc     480

gtcctggcct tactagtttc atccgcattg tttgcaatag aacataccga atggtttgcc     540

ggtttagtgg ctggcttggc atacggcttg ctttatattc gtagcggaaa aatatggttg     600

ccgatcatcg ctcatgcggt gacaaatggc atattgggac tttggatcat atataccggt     660

aactggcatt actggtaa                                                   678


<210>  90
<211>  1233
<212>  DNA
<213>  Methylobacillus flagellatus

<400>  90
ttgcggttat ctcaatcaac taacctaata aaaattgctt tggtcgctgg gctaggtatc      60

gagcttgctg gatttgtagt ccctgcttgt gcattagagg gggatgtttt aaagccgtat 120

gccagtctta catattcata tgatgataat ttaagaagat tcgctagtaa gcaattagct 180

caaatgagta ctgggagttc agatacctca gatacaatgc ttgtcaaaag cgctggtatt 240

gtgctggatc atactattag tcgtcaaaaa atgtatgtgg attttagatt aaacaagtcc 300

acatttaata aaaactcaga gttagataat gatggtcgtg acgttattgc caggtgggat 360

tgggagttgg gaaatcgatt ggaaggcaag gttgagtttt ttcataaaaa agctttggtt 420

cctttttcag aattccaacc tctgcaagga accatgttat cgttaaacac aagaactcag 480

gaccgccgct tattggaggc gcgttggatg ctgcatccta gatggcgtgt tagaggggct 540

tttatttact ctgaaatgga gtacggggct gaggctcaaa gggccgctaa tctaaaggaa 600

gatatacagg agatcggatt tgactatcta tctcctacta aaagtatcat cggtgttctg 660

tacagaaata ctaaaggatc aaagccgcag caagcttttc agggatcttt tataagcaat 720

gattatgacc agaatgaatt aaagttgaat gtcgattggg ctgttaccgg aaagtcaagg 780

ttgcagtttc ttggtggttt ggtagatagg cagcatgagc aactgtctca acgggacttt 840

aggggggtta atgctcgggg aaactttaat tggatgactt ctggaaaaac caatttgaat 900

gctgcagttt ggagagaaaa taacgctcag tcatttgtta caacaagtta tacgctgaat 960

cgtggaacat cattaacggc taatttgcag gcaactggta agatagctgt tcaaggcgta 1020

ttgaaatatg aaaaaagaga ttttgagggg gatcaaattt ttggtcaatt taggtcagat 1080

aaagataaga gttttatgct gtcacttatg tataacccag taacatcttt taggataact 1140

acttcattta ttcatagtga tcgagagtca acatctgata tgtttgaatt taggtcaaat 1200

agtgtctcag taacagggca atatgagttt tga 1233

<210> 91
<211> 1386
<212> DNA
<213> Methylobacillus flagellatus

<400> 91
atgacggtaa atgacatacc tttagtcgct tttttcaagc agctgttaga tcctttaatt 60

attctgggga ctttgtatgg ctttacgctt ctttatggtg agccattcaa tggctactac 120

ttaatccttg cgttgcttgc atactttgta tcttcaaatg tctatgagcg cctggatctt 180

taccgcacat ggcgtagtgg aaaagtgttg ggctatattc gtgatatttt gttcggatgg 240

gcaattgttg tcaccatatt ggcatttatc ggatatgccg cagaccttac tgatcagtac 300

catgaggaaa tggtgtatac ctggtttgtg gcggtgccga tcatctttac tgttgcgcat 360

ttgctcatcc gcaaaattgc ctcggatttg cataaaaatg gcgaagttcg ttctgttgtc 420

atagtaggcg ctaatgatat cggtgtccgg ttggtgaatc gcatcaatga agacccttac 480

ctgttcatgg aggtgaaagg tctttttgat gatcgcgaga tatcccggca tcccaaggga 540

atgcgtcatg taccgttgct aggcacaatg gcggacgtgg cggcttatgt gcgtgccaac 600

aatacccaga tcattttcat cagtctgccg atgtccgcgc aaccgcgcat cagacagttg 660

tttgaggagc tgcaagacac gacagcatct gtctattttg tgcccgatat ctatattttt 720

gatctggtgc aggcaaggtt tgatgatgtg gcgggtgtgc ctgtggtggc gatttgtgaa 780

acgccgttta cagggtttaa tagcttaatc aaacggacta gtgacattat tcttgcatcc 840

cttatcctgt tgttgctttc accgatcatg ctgacactgg ctttggctgt gaagctgact 900

tctaaaggcc cggtaatatt caagcagcgt aggtatgggt tgaatggtga ggaaatcatt 960

gtttacaaat tccgctccat gacagtgatg gaagatggtg acaaagtggt gcaggcacaa 1020

aagcgtgacc agcgcctaac accgattggt ggttttctca ggagaaattc ccttgatgag 1080

ttgccacagt ttatcaatgt gttgcagggg cgaatgagta tcgtaggccc gaggcctcac 1140

gccgtggcgc ataatgaaca ataccgcaag ctcatacgtg gctatatgtt gcgccacaag 1200

gtgaagccgg gaattactgg ctgggcccag gtgaatggat tgaggggtga aaccgatacc 1260

ctcgaaaaaa tggaagcgcg gattgaatat gatttggatt atctcaggca ttggtcgctg 1320

gcgttcgatc tctggatcat catccggacg gtgctggtag tgtttaaaaa agataatgcg 1380

tattga 1386

<210> 92
<211> 945
<212> DNA
<213> Methylobacillus flagellatus

<400> 92
ttgaatactg aatatggaag cacaataatg caatggtcga aaatgacgaa aggggcgttg 60

gtcgccttga tgattagtat cgggctgtcg ggctgtggag ataaatctaa tgaaacaaag 120

gctagccaat cgctggcgag agtgaacggc aaggaaataa ccgtccatca actcaatgaa 180

gagcttgctc gggccaatgt ccagccagcg cagaaagaag ctgctaccaa gcagatattg 240

caatcattga ttgaccgtca actgttggag caggctgcct tgaaagcaaa agtagaccgg 300

gacccgaacg tcatgcaagc cattgagcgc gcaaaatccc aaatgattgc ccaggcatac 360

ctccagtcta gggtggctaa cataggcaag ccggacaaaa cagaagttga gaatttctat 420

aagcaaaacc ctcaactatt ttccgagcgc aagctggtgg aaatggaaca aatattgatt 480

gattcacgtt atgtcaatga cgaattcaag tctgcgctcg gtagcttcaa aaccctgcag 540

gaagtcgctg cgtggttgga tgctaaggga atccaatatg accgtggaga agttgcccgc 600

agtgtggcag agctgcctcc acagatttct gagcgcctga aggatgcaag gcgtggccag 660

ctgtttgtga tcggcatggg accgaggacg atgctggtgc tcatggatag cgtaaaggag 720

agcccggtat cactggcaga tgcgacaccg caaattgagc aaatgctctt gaataaaaag 780

cgtaaggaag cgggtgaggc tgaaatcaag cgactacggg ctgaagcaaa aattgaatat 840

ttagacaaat cggccgaagt accacaacaa ccagaaaaag ccgaacagtc aaaaccctct 900

agtgtcgatg atgatattgc tcgcggagta agcggactaa agtag 945

<210> 93
<211> 813
<212> DNA
<213> Methylobacillus flagellatus

<400> 93
gtgtataggt gtagtgccat gactaaattg atcaaatggt ttgtaacggt agtcctgacg 60

attgttgtat tcaatgttaa cgcagcggat tcggatattg ttcttgggcc gggggatgtt 120

ctaaggatat ctgtatttga acagcctgat ctttccttgg aagtaagagt gagtgagtct 180

ggcactatca cctacccgtt gataggcgaa gtcagagtcg gtggagaaac acctgcagct 240

gctgaacgca aaattgccag catgctggaa agcggtggat tcttgaaaaa ccctcacgtg 300

aatatcattg ttgctcagtt gcagagtcag caggtttctg tgctaggtca ggttaacagg 360

ccaggtcgct acccactgga tagtgcaaaa tctcttgcag acgtactggc gttggccggt 420

ggagttgcac ctgatggggg agatttgatt agcttggttc gcaagattga ggggaaaagt 480

gttagagaga taattgattt tgctgaaatg atgcgggatg gcgacttaaa taaaaatatc 540

ttacttacgg caggagatat tatttatgta gatcgagctc ctagattcta tatatatggt 600

gaggttcagc ggccaggaca atatcgtctt gagtatcata tgacagtgct gcaagcactc 660

gctgttggtg gtggccttag tctcagggga acggaaaaag gtattaaggt gaaacggtta 720

gatgatgaag gtggtgttaa gataattaaa gtaaagcatg atgattttat tcagcctaat 780

gatgttgtat atgtacaaga gagtgtattt tag 813

<210> 94
<211> 1410
<212> DNA
<213> Methylobacillus flagellatus

<400> 94
atgagtttta tcagatttat ttctatcatt aaagctagat ataaaattat tatttttact 60

ttatttataa cggtattaac taccgctatt gttacaatat tgttgccaaa aaattataaa 120

gcaacagctt cagtgcttgt taattataaa ggcgctgatc ctgtaacagg tgtaatgctt 180

cctgcgcagt taatgccagg ttatatggca acgcaagtgg atattatttc aagtaaaaac 240

gttgcaaaaa aggtaattga taaactaggt ctggcagatg ttccagccgt aaaagaggac 300

ttttatcaag caacttcagg taaaggtgaa attaaagaat ggcttgcaga tttattgcta 360

aaaaaacttc aagttaaacc atctcgagag agtagtatta ttgatattag cttttttggg 420

gtaaatcccc agtttgcagc tacaatcgca aacgaatttg ctgagtctta tatgcaaact 480

tcaatacaac ttaaagttga gccttctcaa aaagctgctt tgtatttcgt tgagcaattg 540

aaagaactga gagaagatct agctcaagcg caatcgaagc tttcagagta tcagcaagag 600

aaagggattg taagtgttga tgaaagattg gatgtggaaa gagccagatt aaatgagctt 660

tcatctcaat tggttattgc acaagcacaa gttatggaag cgagatctcg ggaaaaaaat 720

tcaagtggaa aagaagctta tgagtctcct gatgtagcag ctaatccaat tatccaaaat 780

cttaaaactg aaattgctag agcagaatca aggtttgcag atgtttcgca gaaatttgaa 840

agaaaccatc ctttttatct aggagctaaa gcagaaattg aaaatcttaa gtctgagcta 900

gatagacaga tcaaatcggt ttcaagtaat gtggcaagta attcaagaat tctgcaacaa 960

cgtgaagcgg aaatacgttt ggctttaaat aatcaaaaag aaaaagtatt acaacttaat 1020

cgagataggg atattttatc tgttttgaca agagaggttg aaaatgctca aaaagcatac 1080

gacatggcta tgcaaaggta tactcaaacg aatattgaag gtcagtcaaa tcaatctgat 1140

atttctttat tgaatcctgc cgtggcacct cttactccca ctagcccaaa tttattgctg 1200

aatattatat tgtcaatcct cttgggtatg tttttaggaa tagggtttgg agttttagct 1260

gagtttgttg atcgaagagt acggactgct gatgacttaa ttgaagtatt acaagcccca 1320

cttcttggga ctattagtaa agaaaaaaga aaactgagat tttttaaaaa tacttcaaac 1380

ctgataactt tagggaaggc tgaattatga 1410

<210> 95
<211> 897
<212> DNA
<213> Methylobacillus flagellatus

<400> 95
atgatttcat ttacaccttc taattcaaac tctgaaaaag tagaaaatgc accattggta 60

aacgaatttt cagaatttac aattggtagt ttattattgc ggatggggaa aattacaccc 120

gaagatgctg aaagaattat gcggttacat aaagagaagg gtttgcgctt tggagaagct 180

gctcaagcat tagggttgat aactgaagat gatattcaac aggttttatc gaggcaattt 240

aactatcctt atttatcacc tgaccaaaac tcatatctta atgagctcgt tgctgcatat 300

cagcctttta gtaacaaagc tgaggttttg agagcagtac gcagtcaatt aatgctgcat 360

tggttttcac cggaacgaaa agcactagct gtttcttcag taagcagcag cgataatgca 420

agctcttttg ttgcaaatct tgcagttgtt tttgcgcaat tgggtgaaaa tacattattg 480

attgacgcta atttacgcta cccacgccag catgagatat acaatctgag taacaaacgt 540

gggttatctg atatcttagc aggaagagcg gggtatgatg taatttcaaa aataaatttt 600

tttgaaaact tatctgtatt aactgccggt acattacctc ctaatcctca agagcttgtt 660

agcaagccaa cattcggaaa tttaattaaa gagttggctt gtgtttatga tgtaatttta 720

ttggatactc ccgaacttag taattattca gatggttatg caattgcttt tactgttgga 780

ggaattgtat tagttgcaag acaaaatcat actcgagtga atgatttatc tattgctgta 840

gaacaatgca agaataatgg tactaaggta attgggtcaa tattggttga tttgtag 897

<210> 96
<211> 888
<212> DNA
<213> Methylobacillus flagellatus

<400> 96
atgaacactg tcgaggtaat cgagcaagat aatcctggta tctgggaggt aatcaatcgg 60

aattggatgc tattattagg tttgttcatt atgtatacgc ctacatattt gaacttatta 120

aatactgtat ggcaatctga agatcaagct catggaccat taattgttgg cgttgtcttg 180

tatttagtat gggtacagag acaccaattg tttaaggccg aaaataatat tataggcagg 240

ccagtaattg gcgggatagt tttagttttt ggtcttttac tctacattct tggccgttct 300

caagatattg caccgttaga tattgcatct caaattcctg tttttgctgg attgttgcta 360

attttccgtg gttattctgg gcttcgagca ttgtggtttc cgttattctt cttgattttt 420

atggtgcctc ttcctggagt ttttcttgat gctgtgacat taccaatgaa gattgcggtc 480

tcatatgtag cagaaaatat actttattgg ataggatatc cgatttcaag aagtggggtg 540

atcctccaaa tcggacaata tcaattactt gttgcggatg catgtgctgg tttgcacact 600

ttaatatctt tggaggcgct gggtttactt tatttaaatg ttgtaaagca cgattcattt 660

attcgtaatg taattcttgg gatattgatt attccaattt cttttaccgc taatgtgatt 720

agagttatgg tattaacttt agttacttat cattttggag atgctgctgg tcagggtttt 780

gttcatggtt ttgctggaat gttacttttt gtagtggctc taattttgat aattagtgtt 840

gatacgctcc tacaatattt ggttaaaaaa tggcagttaa aagcctaa 888

<210> 97
<211> 681
<212> DNA
<213> Methylobacillus flagellatus

<400> 97
atggatggat ttacactgcg aaatttaatt atatttttac tgttattggt agctacttta 60

ggaggagtaa ttcttaaacc tactaagttt gttaatgcag cagaagatat aaatttagaa 120

gcaatgattc ctaaggagtt cgatgagtgg aaaatcgatc ctacgattac tcctatacaa 180

gtgagccctg aggtgcagca aaacctcaat aaagtttatg atcaaactct ttctcggacc 240

tatattaata agcaaggaat gcgaataatg ctttcgattg cttatggggg agaccaaagt 300

cgcggcttgc aagcacataa gcctgaaacc tgctatacag ctcaaggatt taatgttgat 360

ttgttgggta atgccatttt aaattttgat ggatatgaaa ttaaagttag acatttatta 420

gcccagcatg gggcacgata tgagccaata ctttattgga tgcgtgttgg tgaaactaca 480

gtttatggtg gattgggtca aactataaaa aggtttgaat taggtttgca gggatatatt 540

cctgatggac tgctttttag agtttcaagt attagcagag ataatatctc tgcttttaca 600

ctgcaagaag aatttgttaa atctttactg aaaaatgtag atagtaaaag tagaaaaaaa 660

cttataggta cattttcata a 681

<210> 98
<211> 1287
<212> DNA
<213> Methylobacillus flagellatus

<400> 98
atgattaaac ataccgcatt tatggctctt tcgacttttt cacggttgct atcagcggtt 60

gtacttttca ttattcttgc aagattcctt ggtccgagag actttggcat attgatgtac 120

aattttactc ttgcgtcaat agctgtgctt ttagtggaat atggattttc aaatcagttg 180

ttaagagata taggaagata ccctgataat attaatcagt taatggggag ggtttttctt 240

gcaaaactat atctcacagt agtgatgggg gtctgttgcc ttgctggaca ttacttattc 300

tatacaaatg acttagatag agcaatattt tggattttac tgttatcttg tttgctcact 360

tcttttgggg atttttttaa tattgcattc agaggtatcg gaaaatttca tgaagaaaca 420

aaagttgcaa caattggctc tttgttgcat ttcggcttaa tagttttaat ggtaattaat 480

ggggctgagt tattattaat tagtttaggt tttgttttat ctcgttttgt ttattttatt 540

ttgtcgtggc gggcatataa gcgagtggtt ggtgatttaa atactctgtc agcctctcca 600

gttgctgttc taaaagcatt aaaagcagga tttccatatg ccgttgatgc gggtttcact 660

aactttttct accaagttga caccatgctt gtgaaatatt atctgggagc cactggagtt 720

ggtatttacc aagcaggtat gagatttcta aatggtgcaa tgcaatttgc acctgtactc 780

ggaaatgtat ttttacctgt aattgctgcc aagcacacta atactagaga gtttgttgag 840

ctagctaaaa aacttaatct tcagatgctt gctgttggta ctgtgtgctg ggctattttc 900

tggtttgccg gtatgcatat taccgaattt gtttttggtg atgaatatgt agagttaaat 960

tcaatttggc cctatattgg tttgttagtt tttacaagat atcttgctgc gcctcaaggt 1020

gttctattga ctgcttctgg atctcagaca atacgtgtct gggcacaaat ttttgcactt 1080

attattttgc taatttcagc accacttctg atgaaagtga ttggactaaa aggtatggtc 1140

attgctttaa tcatcacatt attaaccttg tttttgatat ttttcagtgc attacggctt 1200

agaaaaatac ctactgggtt tgggggagtt aattttactt tgactgtaat agtatttagt 1260

ctttccattt ttcttttggt atattag                                          1287

<210>  99
<211>  1179
<212>  DNA
<213>  Methylobacillus flagellatus

<400>  99
atgttgaaaa taggcttact ttggcattct actacttcag ataatcttgg tgttggcgct        60

ttaactgaag cacaaatttc tattattaat aaagctgcgg tgcaggcggg ggttgaggtt       120

gaatatcgaa ttttcggcac taatggaaac aaagattact ttgagggcaa ccctttaatt       180

gagcgaggga caaatgtatc actcaaaaga attttgatcg gggcttctga ttatcccaag        240

aaaatccggg aatgcgatgt ggtgtttgat ataggtgaag gtgatagttt tgctgatatt       300

tatggttttt cacgattcct aatacagatt gtgtctaagt tggttgtctt gtttcaacgt       360

aaacctctta ttttaagccc gcaaactata gggccattcg aaagtaggtt taatttaaag       420

gtggccaata atgtaatgca gcggtgcact aaaatatttg taagggatgg attgtcattt       480

ggatacttgc aaaagaataa tttatctgta aatgcagagg aggtaattga cgttgctttt       540

agattgccat ttcaacgccc ttcccgggat tcaagtaaag taaaaattgg actaaatgta       600

tcaggattac tttataacgg tgggtatact gggaagaatc aattcggtct tagtgttaat       660

tatcctgaac ttactagatc actattagat cagtttactc gaatagaagg ttgtgaagta       720

tggcttgttc ctcatgtgct aacgacctca tttcctatag aagacgatgt gaacgtgtgt       780

gttcaattac aagagcttta tccagctgct aagcttgcac catctttttc ttctcctagc       840

caagctaaat cttttatttc agggatggat ttttttcttg gagctcgcat gcatgcttgt       900

atcgcagcat tttcaagtgg agtgcctgtt gtgccattag cgtatagtag gaagtttaat       960

gggctttttt ctgccttgga ttatccttat cttggggatt gtaaggttaa tactaacgag      1020

caaatagttg gtttggttat ggattgtttt aacagtcggg agcagttgaa gtttgcagta      1080

gaagaaggta ataaaattgc agaaaaaaaa ctattgcgtt atgagcagta catagcagaa      1140

ttattaacta gaatttcaaa taaaaagatt gttttataa                             1179

<210>  100
<211>  1023
<212>  DNA
<213>  Methylobacillus flagellatus

<400>  100
atgtatagtt gtaagatgaa aaaatacata attggtttct tgccgctttc tattaaatgg        60

cttctgagat tttgtcttcg aggcccgagt ttattgagag acttttgtta cgatgctaaa       120

cgatatatta aatggtcatc tgtaatttct ttcgagaaag atcgtgaaaa gattcttgcg       180

gttattactg ctacttatca caacatcgag aaaggaatgt ctttacctaa tcccaggccg       240

ttatttggaa gggtagtgat tggacaatta gttggatcag tcgataattt tcatcttaat 300

tttgggcctg atccacagct agatgttccg ataaatgtac tggcgcgtta ttgtcaattt 360

aatagaagtc ttggtcagcc agattctcaa ttagaagtaa tcgttgatcg gttaaagaaa 420

aataatcaga gcactttatt aaggattaat gatggcgggg ttattttgcg ctctaaagct 480

gaggttttag acgcaatatc tggggttaaa gaagattttt ttcttatgcg ccatagtgta 540

aggcagttca ctaaagatcc tatagagcat agtgtaattg aaaaggccgt acgtattgct 600

caaaaaagtc cagcagtttg taatcgacag tctggaaagg tccgggcaat tcttaatcca 660

gaattaatta ggagtacttt acaaattcaa gggggcgcac gagggtttta tgaagaggtt 720

caggctttat tttgtataac agtggatttg tcaaatttta atggggcgga acgttatcaa 780

ggttggattg atgggggaat gtttgcaatg agttttatat atggcttaca tttgcaaggc 840

ctaggaagtt gcgctcttaa ctggagtaaa actaggaaac aagatataga aatgaggaat 900

caccttcgta taccagaaaa cgaattaatt atcatgtttg ttgccgctgg atatttaagg 960

gaatcatttg ttgttccgca atcataccga aaacctttag gtgaagtctt gagttatatt 1020

taa 1023

<210> 101
<211> 1473
<212> DNA
<213> Methylobacillus flagellatus

<400> 101
atgaagaaca attcttctct cttatctttt aaccgtaggg atgctaattt cgttagttta 60

aagataggga gtatattttt tgtaataatg gctgcaatag tggctttgat atttggtttt 120

gtcagtgcta tttttccatg gtggtggata gtagccatta ttctttctat attatttatt 180

tttattggtt ttacatatcc tttagtagct ttaatagctg ttatagcaat ttcttctaga 240

ttaattccag atacattttt gccttcaata agactgctgg gaggcacaat accatcttca 300

gatatggcct tgtttgcaac attcattttt tgttttttta aatacaattc agaatttagc 360

tttacaaagc aagcattgtc acctttggct attcctttat ttgttgtact tttactgttg 420

cttttttctg tatttcgggc ggtgtttgaa attagattgc cattgaaaga tattttaggg 480

gagtcaaggc atttccttta ttggctattg attcccttaa tagctttaac atgctcagat 540

agacaaagat ttaaatttat tgtttttggc tttctttttg tagctttttt attttcaatt 600

ggccagattt tgcaaggttt tctaggtgta aatgtgttcg ggcgcgagct tactagcctt 660

gaaactttgg gaagcattaa tagtgatgtt acacgaagca tgacgtcagg aactcacatt 720

attgtttggg gatttttgtt tgtactggct ttgtttgcta accgaatagg tttttcaaag 780

actcttatta tattgtgtat atttacaggc gctggaattt tcttaaccta tggtcgtgct 840

gtttgggggg ggactttctt agctgttttg ttcttatgct ggaagctagg gacaaaaaaa 900

atgcttagaa gtgtaggact aattgcaatt gttgctgtat ttggtttaag tgctatgcta 960

gctattaagc cgggcgctat tgaaggcgcg atgcagagat tgtttagtgt ggaggaggag 1020

gtttctcatg gatcttctct cggctggagg ttttatgaaa ataaaatggc atgggcggca 1080

attcagcggt cacctttatt aggcattggg cttggtgccc cttataggcc gcctgctaaa 1140

tctgatgtga cagcagagca agtgagatat gttcatagtg cttatatcta tttaacctta 1200

aaaacaggca ttattgtatt catgtcagtc atttttctta tattcaaaat acttcattta 1260

acttacattc aaacgcggtc taatgattta tttatatcaa gtgttgccat agcttctttt 1320

gctgcaatga tagagtattt tttggcctgc attactcagc cagaattaat gcaaaccccg 1380

ggaattgctt ttttggctac tcttgctggt cttatgtgcg cggctaatta tcttaatcaa 1440

aaaaatctta caaaaggaaa gctaacttct taa 1473

<210> 102
<211> 807
<212> DNA
<213> Methylobacillus flagellatus

<400> 102
atgaatgtta aaacatctcc attaattagt attattacgg caacatataa ttcaatgaaa 60

tattttgagg atactaaaaa aagtgtcctt gaaggagatg ttgaagactt tgagtggatt 120

atcgttgatg atggatccac tgatggaacg cggaaatatt tacaagaaat tgatcatcca 180

aatataaaat tgcattttaa aagtattaat gctggtattg aagactcata tagaactggt 240

attgaattgg cgaccggtaa atatttgcta attttagatc acgatgacac tattccattg 300

ggatctttgt caaagcggct tgacttactg gaatcaaatc ccaatgcttt ggctgcattt 360

ggtgctgtgg catatatgga tgagtctggg aggatttata aagaatcacg ttttcctttt 420

gtatcagatg attgcgtact gtcatcacta tttactttat tcggaatctt tattttacca 480

gtataccctt taaagcaagg gtgtgtggtt ttgaggtctt ctttcgttaa aaataatcca 540

gggttatatg acatacaact attcctgcaa gctgctaaat tgggcccagt aatatttatc 600

aagcaagcat gtttaaatta tcgaacattt agaacgcagt tttcaagttc tagaaaaatg 660

cgattgattc gtttttttca atttgtttgg gcaaaatatg cgtttaaatt tctcccgtgg 720

tatataagcc cgttctttgc tatttataaa acaataccag agttctttaa agtattatgg 780

agttttgttt cttcaaaacg gatttaa 807

<210> 103
<211> 969
<212> DNA
<213> Methylobacillus flagellatus

<400>  103
ttgatgacgg attttagttc aaaaaaaaag ttagacttaa gtgttgcaat agtttttgtt      60

aattataata ctagtgacca aattatttct tgtatttact ctcttaggga agagagatat     120

tcaaagatta ttgttgtgga taatgcatct ccggatgatt gtcctgagtg catcgtagaa     180

gagtttccag atgttgtact tattaaaagt gaaaaaaatc ttggctttgg tgagggatgc     240

aatattggaa aagattgggt tatagttaat acagattgtg aatatatatt ttttcttaat     300

ccagacacac tagttgaacc tggattatta caattactgc atgatggatt ttttagccct     360

gaagttggat tggttacccc aagaattact atgatgggtg atgggttgga tatactttgg     420

tatggtgggg ggtatatgaa ctggttaact ggttcggcac gcgtaccagg ttgtggatta     480

agtgctgtat caaaaatagc tctagaagat cgatttgtag agtttgccag tggttgcgct     540

tttatgatta gacgttcagt tttagaagag tgtggcgggt tcgatccacg ctattttatg     600

tacgaagaag acgtcgaatt atctttaaga gtaatttctt atggatataa aatttattat     660

atgccaaaag ctgttgttcg ccatctggct cagggtagcc agggcaaaga ggtagaggct     720

gttggtatgt ttaaaggaag gaatcctaag cttccttttt ttgtttatca aactgcgaaa     780

aatagatttt taactgtttt tactcatgga actgtgctta agaaaggtct atttttaata     840

ggttttatgt tatgggtgtt tcgaaactct tttgaatggg tcagatataa gagacttgat     900

gcatataatg ctttattttt gggcattaaa gattttgtta aatacacata tgtatcaggt     960

aaaaaataa                                                             969

<210>  104
<211>  1149
<212>  DNA
<213>  Methylobacillus flagellatus

<400>  104
gtgaaagaaa ttaacgtcgc ctttgtggag ccagttggta gtcacggtgg tatggactat      60

tatgattctg gactgtgtgc cggtttgtta catcagggag tatctgcaac ttggtatacc     120

tgtgacattt ctaaaccttg tggtaataga cctattttca tgcaacttac ttttgtaaat     180

atttggggaa aggatgctac gtggagaaga ggggctaggt atatcttagg gctttgtagg     240

agcataaagc atgctaaaaa aaattattca aatattgccc attttcattt ttttcatgtg     300

ggtgttttag aatttttaag tgtattattt tttcgcttat ttggatttaa ggttgttgct     360

acagtgcacg atgttgagtc ttttaagccg ggtctaacct tctctatctt gttaaaatgg     420

acttactttc tctgtaatca attaattgtg cacaatcaag ttagtaagga tgagttgatt     480

aatagaagta atgtttctat aaataaaata catgtaattc ctcacggaag ttatattggt     540

ctggttgcac cctcgatgcc aaaagcagat gctcgtcaag ctcttaatgt atcatcagat     600

gaaaaggtaa ttttattttt tggtcaaatc aaagaagtaa aaggtttgga tttgttgatt 660

gaagcgttag gtttggttaa agataaacta aaaccattta agctagtcat tgctggaaaa 720

gtgtggaaag ataatttctc taagtatcaa gaattaataa gtaaaaacgg gctaaatgat 780

ttttgtaaat tggatattag atacatacca gatcaagaga tatcaatgtt ttatagtgcg 840

gcagacttga ttgttctgcc ttatagaaaa atatatcaaa gtggtgtttt attaatggca 900

atgagttatg gaactccagt gttagcttca gaccttccgg gtatggcgca aattataaat 960

gatggggaaa acggattttt gttttctgct ggaaattata ttgatcttgc tgaaaagctt 1020

gtaaaaattt tttcttacca tgacttatct aggattactt tgcaagcaga aaacgatatg 1080

agaactcgct tcagttggga tgagattgcg ataaaaacta tcagtgttta tcagaaaagt 1140

atatcgtaa 1149

<210> 105
<211> 1506
<212> DNA
<213> Methylobacillus flagellatus

<400> 105
atgcaattaa ttaactctcg aaggattgtt ttacttagtt tgttttccct tatgcctttg 60

gttggtattg ctgaagttta taaatctaca gatgtagacg ggagtaccac ttattctgat 120

acccctcaag ctaattcaca aaagataaag gtttttcctt ctgattataa gaagaatgtt 180

aaatctgagg ctgcagctaa taaatccaga agtaattcta tttctgctgc taatgttaca 240

gaagctccta ttgatgagtc tagtagtgat tctaatgtaa ctataaagca ttcaacatct 300

aataaatcta tcaattcaag caatccttct gctgctggtg gctcttcttc tgctggtggc 360

tcttcttctg ctggtggctc ttcttctgct ggtggctctt cttctgctgg tggctcttct 420

tctgctggtg gctcttcttc tgctggtggc tcttcttctg ctggtggctc ttcttctgct 480

ggtggctctt cttctgaaac aactacagct gcttcggtcg ataaggtttc tgcggctttg 540

caaggaccga aagcttttct taattcagac gattttaaag aaaattctta tcgagggttt 600

aatgttcttg gcggaaattt aagttctgct gacatggaag atcttgaaaa gtttaacgtc 660

aatttaattc gaagcgttat tcatttggaa aaatgtaaaa aatgtaatga ttatgaaatt 720

tctccagttg agttgtcgca aatggatttt cttgtggagg caggtcgtaa gttgggattt 780

tatgtaattt ttgcttttga tcctaaacct gctggagatc aggcgatcta ctggaataat 840

caacaattac gagaaagcat taagaataac tgggtaacta ttgctaaaag atacaaaaat 900

gagactgtgg tggcagggtt tgacctgatt aatgagcctg ttgttaggcc tggatcagta 960

tgggaaaagt atgctactga attgattact gccatacgta aagtagatga aaatcatata 1020

atttttctgg aacctagtcc ttgggggctg gcttctagtt tggagcagtt tactcctgtt 1080

ccatttaata atatagttta cagctttcat ttttattctc cccaagaact tactcatcaa 1140

ggattggcgg catacccaca tgcatatata tatcccagtg aaaagtggga taaagaatac 1200

ctaaataatc gactagacat tgctcgtaat tgggctaata aatataatgt tcctctttat 1260

atcggtgaat ttagcattat tcggtgggct ccgtctggtt cgaggttacg ttacttgaca 1320

gatgtagttg agcgctttga gcaagagaaa tggtcatgga catatcatag ctatagagaa 1380

tatagcggat gggatgcgga aatgccagat agtatattca agaatgatgc tattgtcgaa 1440

cgtcaaaaca atgttcctgt acttcagctt ttgatgaaaa attttaataa aaataagaaa 1500

tattag 1506

<210> 106
<211> 1107
<212> DNA
<213> Methylobacillus flagellatus

<400> 106
atgcataaga aaatagcaat attaggaacc agaggaattc cggctgcgca tggtggcttt 60

gaaacatttg cagagcattt ggctatttat cttgaaagaa aagggtggaa agtaacagtt 120

tattgccaag tagatggtaa tgggcctatg tatgaggata tgtggaatgg tataaagcga 180

gtaaatattc cgatatctat ccagggttct ttaggcacta ttctttttga ttggaagtca 240

acgattcatg cttcaaatac ttatcatctt attttaactc ttgggtataa cactgcttta 300

ttctgtgggc tctatagaac taaaggaata actaatctaa ttaatatgga tggaatcgag 360

tggcatcgga aaaaatgggg ggcagttgct aaactctggt tctggattaa tgattgggct 420

ggttcaattt taggcaatca tctgattgct gatcatccgc aaattgcgcg tcatctggaa 480

acaagagtca ggcctagcaa gatcacaatg attccatacg gcgcagataa agtgattgag 540

gcgaatcagg ccgttttgga ggaatatgga cttaagcctt tcagttatgc gatcttaatt 600

gctcgtgcag agcctgaaaa ttctatttta gaggtggtga aagcctggtc cgctaaacct 660

cgaggcatga ggcttgtagt gctaggaaaa tataaccctg accatgctta tcaggtcgcg 720

gttaaagctg ccggctcaga cgaggtgctt tttcctggcg caatttatga taaacacgtg 780

gtcgaatctt tacggtattt ctcaagatta tatattcacg gccaccaagt tggcggcacc 840

aatccttctt tggtggaggc actaggggct ggtaatgcag tattggcgca cgataaccgt 900

tttaaccgct gggtcgctgg agataaggcg gcctactttt ctggtgaaaa ggactgcgaa 960

atcactcttg atgagcttct taatgatgaa gttcggttac gtgagatgag agccggtagc 1020

ttggctcaat acgatacctt atttacctgg gactcagtgt tgcagcaata tgaggccttg 1080

ctgacaaggt tttgccaaa taagtaa 1107

<210> 107

<211>  1440
<212>  DNA
<213>  Methylobacillus flagellatus

<400>  107
atgcagattc aagccgtgat tttatcaggt ggctctggca caaggttgtg gcctctttct      60

cgagaaaact atcccaagca attgctgaca ttgcttgata atgattcttt acttcagtcg     120

acattacgca gggtcgatcc actcattgga accgagtgtt ctccaattct ggtatgtaat     180

gaagaatatc gctttgttat cgctgagcaa ttgcggatga tgggaaggcg tggatctatt     240

ctccttgagc ctgtaggcaa gaacactgcc cctgcgctca ccttggctgc cttagaggct     300

gccagccacg gccaggatcc aatcttactt gtcatgcccg ccgatcacgc tattatcaat     360

gtgcaagact ttcaggatac cgtgaggaca ggggctgaac atgccgctgc tggtaagatc     420

gtcaccttcg gtatcgtgcc cgatagggca gaaactggct atggttatat caagtcaggc     480

gcagtgctgg aaggtttcgg ttctgctttc agtattgagc agtttgttga gaagcctgat     540

gaggctactg ctcagcaata tgtagtaagt gggaattatt tctggaacag cggtctgttc     600

atgatgcgtg ccacggtatg gctgaatgcg attgctaaga ctcgtccaga tatcttatct     660

tcatgtaagc aggcttggga tggtcaacaa cgcgatggcg attttgttcg ggtggacaag     720

cttaaatttc aacaggtacc tagcgattct atagactatg cggtgatgga gcatgtcgct     780

cgtggtgccg ataatctgcc tgcaggcata gtcatccccc tgaatgccgg ctggtcagat     840

gttggggcct gggattcatt cgggaaaatt ttgccgcagg ataacgcggg taactcccat     900

ttaggagatg tgctgttcaa tgattgctcg aatactctag ctgtgtcttc aggtaggctg     960

cttgcatgtg tgggtctgca aaacatcgcg gtgattgaga cagcagatgc ggtgctagtt    1020

atagataaaa gtaaaactca ggatgtgaaa aaaatcgtgg agcagcttaa ggtccagaaa    1080

aggcccgagg ccaagactca ccgcaaggtt caccgcccct ggggctggta tgacagtatt    1140

gatatggggg cacggttcca agtaaaacgt attgtagtga atccaggtgc tacgttatct    1200

cttcaaatgc atcaccatag agccgagcac tggattgtgg tgaccggtac agcgaaggta    1260

acaagaggag atgaggagtt ccttgtttct gaaaaccagt ctacttatat tcctttaggt    1320

gtgacgcata tgctggctaa tccaggaaag cttcctttag aaatgataga ggttcagtct    1380

ggggcttatt tgggtgaaga tgatattgtt cgcttcaatg atcaatatgg acgttcatga    1440


<210>  108
<211>  921
<212>  DNA
<213>  Methylobacillus flagellatus

<400>  108
atggcaactt tcaacaagat cctgcttacc ggcatcaacg gccaggtagg ccatgctctc      60

tatccgaaac tacaaagctt gggagaagtc gttgcacttg accgcagcca acttgaccta     120

tccaaacctg accatatccg ggacgtagtg cgacagacca aaccagacct cataatcaac 180

cctgcagctt atactgctgt ggataaagcg gaatctgagc cggagctggc ctacgccatc 240

aatggcactg ctcccggcat tctggctgaa gaagcagcaa agctaaatgc attgcttgtg 300

cattattcta cggattatgt atttgatggc accaaaacta ccccttacac agaattggat 360

gaaacaaatc cacttagcgt ttatggagcc agcaagttag caggagaaaa agccattcaa 420

gaagtcggag caaatcatct aatcttcagg accagctggg tttatggtgc atatggaaaa 480

aatttcatgc gcaccatatt gagattagct gccgagcgtg actcattgaa tatagtcgca 540

gaccagtttg gcgcaccaac ttctaccgaa agcattgcag atgcaactat tactgcgctg 600

cacgcatggg atgggactca aagaggtgtc tatcacttag tcaatactgg ggagacgagc 660

tggcatggct ttgcactgga aatcattcgc cagtatggag aaatagccca aaagaaaaat 720

ttagcgaaat taaaatccac tgtagaaaat gttgtcggta ttacaactgc agaatacccc 780

acaccagctc atcgccccgc caactccaag atgagcactg aaaaattcat gagcactttc 840

aaaactgaat tagcaccatg ggaattggct ttgcgatcta cagtaaccca cttagcaaca 900

ttctcggtcg aaaatcatta g 921

<210> 109
<211> 549
<212> DNA
<213> Methylobacillus flagellatus

<400> 109
atgaatgtca cgcccaccca tattcctgat gtgatcgtat tcgaacccaa gatattcggt 60

gatgcccggg gattcttttt tgaaagcttc aatcagaaag cctttgagga agccgtcggc 120

aggaaatatg attttgtaca agacaaccac tcaaaatcct caaaaggagt attgagaggg 180

ctacattatc agatccagca agcccaagga aaattggtta gagtagtagc aggtgaagtt 240

tttgacgttg ccgtggatat ccgcaaatct tctccaacct ttggtcaatg ggtagggcaa 300

catctgtcag cagaaaacaa gaaacaaatg tgggtaccac caggttttgc ccacggcttc 360

ctcgtgctgt ccgaaactgc agagtttctc tataagacca cagacttcta cgcgcctgcg 420

catgaacgtt gcctaagctg gaacgatccc gttgttggta ttgaatggcc agatattggc 480

acgcccccgc tcctctcaag taaagatcaa caaggcttag gcctccacca gacagagtta 540

ttcgcataa 549

<210> 110
<211> 879
<212> DNA
<213> Methylobacillus flagellatus

<400> 110

atgagaaaag gcatcatcct cgccggtgga tcgggcaccc ggctttaccc tgtcacccag 60

gcagtctcca agcaactgct gcccgtgtat gacaagccca tgatctacta tcccttgtcc 120

acgctcatgc tggctgggat acgcgacatc ctcctgatct caactccgca ggacacacca 180

cgctttgaac agctattggg tgatggcagt caatggggac tcaatattgc ctatgcagta 240

cagccctccc cagatggatt ggcccaagcc tttatcatcg gcaagaaatt tattggcaat 300

gatccaagtg ccctggtgct tggcgacaac atcttctatg gtcatgacct ggcacatgac 360

ctggcacatg ccgatcaacg gcagcatggc gccactgtat ttgcctaccc tgttcacgac 420

cctgaacgtt atggcgtggt cgagttcaac gatgctggcc aggctatcag cctggaagaa 480

aagcccaaag agccaaaatc ccgctatgcc gttactggac tgtatttcta tgacaaccag 540

gtcgttgaca ttgccgccag cctcaaacca tctcctcgag gcgaacttga aattaccgat 600

gtcaacaaaa tttacctgga aaggcgctcc ctcaatgtgg aattgatggg gcgcggtcat 660

gcctggttgg atacaggaac gcatgaatcc ctgctagaag cctcactatt catcgaaacc 720

atagaaaagc gacaaggcct gaaagtcgcc tgccccgaag aaatcgctta ccgcaaaggt 780

tacatcgatg cagaacaagt agaaaaaatt gcccagccat acctcaagaa tggttacggc 840

caatatttga tccggatgct gaaagaaaag gtcttttaa 879

<210> 111
<211> 1101
<212> DNA
<213> Methylobacillus flagellatus

<400> 111
ttgacgacat taaataacac tatcattgtc actggtggcg caggcttcat cggtggcaac 60

ttcgtactcg actgggtcaa ggcaggacta ggcaccgtca tcaacctgga caaattgacc 120

tatgccggaa atcttgaaaa cctggcagac attgaaggca accccaacca catcttcgta 180

catggcgata ttggtgaccg cgccttggtc gccaagttgc tggcagaaca caaaccccgt 240

gcagtcatca actttgccgc cgaaagccat gttgaccgtt ccatccatgg cccggaagac 300

ttcattcaga ccaatgtagt cggcaccttc cacttactgg aagaagtacg cgcctactgg 360

aatagcgtgg tgaaaaccca cccagaggaa aaagaaaatc ctcagttccg cttcctgcac 420

gtctctacag atgaggtgta tggcacactg ggccccaagg accaagcgtt tacggaaacc 480

accccttacg cccccaatag tccatactcg gcttccaagg cagcctcgga tcatctagtg 540

cgtgcctacc accacactta tggcttgccg gtattgacca ccaactgctc caataactat 600

gggccttacc atttccctga aaaattgatt ccactatgcc tgctcaatgc gcttgctggt 660

aaaccattgc ctatttacgg tgacggccag caaatccgcg actggctatt cgtcaaggat 720

cattgcagcg ccatccgccg cgtactggaa gcaggccagc tcggggaaac ctacaatgtg 780

ggcggttgga acgaaaaggc caatctcgat gtagtacaga ccttatgcag catcctggat 840

gaactcagcc ccaaggcaga tggctcaagc tacaaagacc aaatcacatt cgtcaaggac 900

cgtccaggcc atgaccggcg ctacgcaatc gatgcaagca aactggagcg cgagttgggc 960

tggaagcctg cggaaacctt cgagtcaggc atcagaaaaa ccgtggaatg gtatctgcaa 1020

aaccaggcat gggtgcacaa cgtcaccagt ggagaatacc gcaactgggt cagcaagcaa 1080

tatagcggga gcgcagcatg a 1101

<210> 112
<211> 1005
<212> DNA
<213> Methylobacillus flagellatus

<400> 112
atgttattag taacaggcgg tacaggctac ataggttccc acgcatgtgt ggaactgatt 60

aatgcgggct atgaggtggt ggtgctggat aatttcagca atagccaggc gcatatcgtt 120

gaccgcatca agcagatcac gggcaaggca cccgtattgg tggaagggga tgtgcgggat 180

gaagcactac tgagtgattt attttctagg tacccattcc aggcagtcat gcattttgcc 240

gggctcaagg cggtggggga gtcggtcgcg cagcctttgc gttattacga taacaatgtg 300

gcgggtagtg ttaccttgct gcgcgtcatg caggcgcatg acgtcaaacg attagtcttc 360

agttcgtccg ctactgtgta tggcgatcca gccagtgtgc cgattcgcga ggatttcccg 420

ttgtctgcga ccaatcctta tggggcaagc aagctgatgg tggaggatat cctgcgcgac 480

ttggcagtct ccgatccaga gtggcgtatt gccatcctgc gttatttcaa tcctgtgggc 540

gcgcatgaga gcggcctcat tggtgagaac ccgaatggta tgcccaataa cctcatgcca 600

tacatcaccc aggtagccca ggggcagcgt gaatctcttt ctgtgtttgg cagtgactat 660

ccaaccgtgg atggtacggg cgtgcgcgac tacatccatg tggtcgacct ggtggttggc 720

catttgcgtg ccctggagcg gctggataag gaagctggtg tgttcacatg caatcttggc 780

acgggccaag gatatagcgt gctggaaatg gtcaaggcat ttgaagacgc cagtggccag 840

gctgttccct atcagctggt ggcacgccgg ccgggtgatg tggcggccag ttataccgat 900

cctgcttatg ccgagcaggc attgggttgg aaagcgacgc gtggcatcga gcagatgtgc 960

aaggatagtt ggcattggca gcaaaacctt gcccagagag gctga 1005

<210> 113
<211> 1419
<212> DNA
<213> Methylobacillus flagellatus

<400> 113
atgctgcttc ctgtcattat gtctggcggg gccggttcca gattatggcc ggtttctcgt 60

gaaactctaa ctaagccatt tatcgaatta cctgatggca gaacccttct gcagaagact 120

ttgcaacgta ctgcttgcct ggatggtgtg agcgaagtgc tcacagtaac gaataaggaa 180

tattacttcc tgacacgtga tgtctatgag gcaacagaca ctaatgtttc ccatagctac 240

ttactagaga cgatagggcg caatactgct cctgccattg cagttgctgc actacatgcc 300

gctgcgaaac atggggaaga tgtagtgctg ttggtgatgc cggcagatca tcttattggt 360

aatcaaaaag ttttttcaaa agcagtgact gttgcgacag atatggcggt gaatggctgg 420

atagtcacgt ttggtgtaga gccaagcacg cctgaaacag gttatggcta cattgaaggc 480

ggagagtcaa ttggggactc aggcgcgcgt agagtcgttc gatttgtcga aaagccagat 540

ttggaaactg ctgagaaata tattgcttta ggtcgattta catggaatgc aggtatattc 600

tgttttactg caaaaaccat tattgctgca cttgcggagt atgttccaga agtgcttgag 660

atagcaaaaa agaccctggc atctagtgac tccaaaactg agcctgttgt attgggcgag 720

gagcactttg catcaagtcc agatatttcc atagattatg ccgtcatgga gcgtgcttca 780

aaagtggcgg tagtcgcagc tgattttgac tggagcgaca ttggttcatg gagcgccatg 840

gctgagttgg tccagtcaga tgagcgtggg aatagggtga atggcgaagc tttattggtg 900

gatgctaata attgctatat ccaaagtgat tcccgtcttg tggctggtat aggtattgat 960

aacctgctgg tggtcgatac acccgatgcc ctgcttgtgg ctcatcactc cagagctcag 1020

gatgtcaaaa ccgttgtgaa gcaactcaag cttgagtccc atagtgcggc aaattaccat 1080

cgcaaggtac accgtccatg gggcacatac accgtattgg aagaaggcgc taattttaag 1140

atcaaatgca tcaaggtaaa gcctggtgcc agcctgagct tgcaaatgca ccatcatcgt 1200

agtgagcatt gggttgtagt aagcggtata gctaaagttg tcaacggcga gcgcgagctg 1260

ctcattaatg ccaatgagtc tacttacatt ccagcaggac acaaacaccg tcttgaaaat 1320

ccaggtttgc ttgacctggt gattattgaa gttcaatccg gggaatattt gggcgaagac 1380

gatattgttc gttttcaaga tatctacgga cgcacttga 1419

<210> 114
<211> 795
<212> DNA
<213> Methylobacillus flagellatus

<400> 114
atgatcagca tgttaaaagc cttatggctt tatcgcggct ttgtgttggg cagcgtcaag 60

cgcgagtttc aatccaagta ccgtaattcg ctgctaggcg cagtctggac ggtcttgaac 120

cccctcgcaa tgatcgtagt gtacacagtg attttctcgc aggtaatgaa ggctcgtttg 180

cctggcgtgg acagtacttt tgcgtttagc attcatttat gcgctggcat actcacctgg 240

ggcttgtttg ctgagattgt cgggcgctca cagaccacgt tcatcgataa tgccaacttg 300

ctcaagaaac tgagtttccc ccgtctatgt ctgccagtca tcgtcaccgc caccgcgcta 360

gttaactttg ccattgtttt tgggttgttc acagcgttct tgctgctgat cggtatgttt 420

tcgggttggc cttatttggt gctgatcccg gtattgatac tgatgatcgt ctttgctcta 480

ggcttgggta ttatattggg cgttctcaat gtgtttttcc gcgatgtagg tcagttcttt 540

ggcattgtgc ttagtttctg gttttggctt acgccgattg tctatccggt cagcattctt 600

ccagaacggg tacaagatta tatgcggctt aaccccatgg cgcgcttgat ggaggctttc 660

caaggaatat tggtacaagc aaaatggccg gactggtaca gtctatggcc agtgtttgcg 720

ctgtctatcg tgttgtgcct gctggggctg tctttattcc gcaaacatgc gggcgatatg 780

gtggatgaac tttaa 795

<210> 115
<211> 537
<212> DNA
<213> Methylobacillus flagellatus

<400> 115
gtggattctg caaggagtaa gttttcatat ccagccagga gagacggagg cagctttgcc 60

gtgactgctg ccaagcgttc taatacgagt attaagccta accttgcagt gatcaattgg 120

atgcttgagc aagaagaccg gatgggatta tctaccccaa ggccttatcg tgaatttgag 180

gagcgtgtat atcgtcatcg cgatgatctc aggcgcttaa ttcattcgtt agtagctgac 240

ggcaaaaaaa tatatgggta tggagcttct gccaaaggta atgtagtact acaattttgt 300

ggctttactt ctgaggaaat ccctgccatt gcagaagtaa acgaagaaaa attcggacga 360

gtcactccgg gaacgcatat tcccattatt tcggaaaaag aggcgcgcgc tgccaagccc 420

gactacttct tggtgcttcc atggcatttc aaagatggta tcctgcgccg tgaaaaggac 480

tttctagctg atggcggcag gtttatcttc ccatttcctg agatcgaaat cgtctag 537

<210> 116
<211> 1041
<212> DNA
<213> Methylobacillus flagellatus

<400> 116
atgaccataa aaactgccat cattaccggc atcacaggcc aagatggtgc atatctcgca 60

gaacttctcc ttagtaaagg ctataaggtt tacggtacct tccgccgtac aagctcggtc 120

aacttctggc gtattgaaga gctgggaatt gaaaatgacc caaatcttct tttggttgaa 180

tacgacctga ccgatttgtc agcaagcatc cgtctattgc agagcacagg tgcaacagaa 240

gtgtacaact tagctgcgca aagttttgtg ggcgtgtctt ttgagcaacc tattactacc 300

gcggagatca caggtgtcgg tgcgctcaat cttttggagg ccattcgttt agtaaatccc 360

aatattcgct attaccaagc atccacatcc gaaatgttcg gcaaggtgca ggcgattcca 420

caaacggaag atacgccttt ctatccacgc agtccttatg gtgtcgccaa gctgtatgcg 480

cactggatga ctgttaacta tcgtgaaagc tatggaatct tcggatctag cggtatttta 540

ttcaatcatg agagtccgtt gcgtggtcgc gagttcgtga cgcgcaagat cacggattcg 600

gtggccaaga tcaagctggg gaaacttgat tgcctggagt tgggtaactt ggatgcaaag 660

cgtgattggg gctatgccaa ggattatgtg gaaggcatgt ggcgcatcct gcaggcggat 720

gagccggata cctttgtact ggcgaccaat cgtaccgaga ctgtgcgcga ttttgtaagc 780

atggcgttca agggggctgg cattgctgtt gagttcaagg gcaagggcga ggacgaagtt 840

gccgtggata ctgcaaccgg taagatagtg gttcgggtca atccgaagtt ttaccgtcca 900

gcggaagtgg acctactgat cggtagccca gccaaggcca aggccaagct gggttgggaa 960

ccgaagacaa cgctggagca actctgccag atgatggtcg aggccgatct gcgccgcaac 1020

acggaaggtt tctcattttg a 1041

<210> 117
<211> 867
<212> DNA
<213> Methylobacillus flagellatus

<400> 117
ttgagaattc tgcttactgg tgctgatgga ttcaccgggc gccattttct gacggccgcg 60

aaagcggccg gccatgaggt tgttgcactg agggccgatt tgacggacaa ggctgtattg 120

caacgcgaag ttgaggcggc tcgtccggaa gcagtggttc atctggcagc gatcagtttt 180

gttggtcatg cggatgttag tgccttttat gacgtcaatg tgatcggtac cctgaacctg 240

ttggatgcgc tggctggctt gtccgtgacg ccgcgcagcg tactgctggc aagcagtgcg 300

aatgtgtatg gcaatgcagc gcattcacct atcaccgaag agcaggcgcc ggcgccggtc 360

aatcactatg caatgagcaa gctggccatg gagcacatgg cccgtaccta tctggacaga 420

ctgccgttgt tttttgtgcg gccgttcaat tacacggggc cggggcaggc tgagtctttc 480

gtgattccca agctggtggc gcatttcgcg tgcaaggcgc agaccgtgga actgggcaat 540

ctggatgtgg agcgcgagtt caatgatgtg gctttcgttt gcgaggccta tctgcgtttg 600

ttggacaagg ctgtgccggg tgaggtgttt aatatctgca gcggtgctcc ggtgacattg 660

cgtgcagtaa tcgggatgtt gagggaattg acggcgcatg atatcgaggt caaggtaaat 720

ccgcagttcg tgcggaataa cgagatcaag acattatgcg gtgatccatc aaagctgttc 780

cgtactattg gagaggggag gctggttcct ttagcgctta ccttgaagat catgctggaa 840

cacgctacgc gaaaagaaaa aaaatga 867

<210> 118
<211> 1143
<212> DNA

<213>  Methylobacillus flagellatus

<400>  118
atgatgaagg taggttttgg tgtcaccgtg cttacacgtt gcctggcatt ggggggaatc      60

gatggaattg gtagttatac ccgggagctg atgaaaagac tcgaggctga cccggccgtc      120

gagcttttcc ctatctcgta cgggtattct ccgtcacagt ttcctatcgg ctcacgtgct      180

ccgacgacat ttggacgata tgcgccgctt gctgcagtat cggctgtatc cgggtttccc      240

tttctgggaa cagctcaatt gaaggagcgt gttgatctga ttcatgcgcc tgatcatctt      300

attcccaact acggaaaagt tcctgttatt gcatccatca tggacgcgat tcccttgtcg      360

catccggagt gggtatcccc caagttgcgc aagatgaaaa atgcgttatg gaagcgctct      420

gcgcactggg catcgcatat cattactatc tctgagtatt cgaagcagga aatttcacag      480

catttcggta ttgaggcaca aaagatcagt gccatcccgc ttggcgtgga tgagcggtgg      540

tttcagccgt tggaagaaga ggcgatgaag gtggtattga gacggtacaa tctgcctcaa      600

tactattttc ttgtcgtggg gacgctgcag ccaaggaaga atgtcggccg ggtaatcgag      660

gcatatcggt ccttgcccca agggatacgt aacgaagtgc cgttggtgat tgttggtagg      720

gcagggtggc gatgcgagga tgtggttgaa atgttgactt cgtcttcttc aggaaattct      780

ctgaggtggc tcaagcattt gccggatgcc gacttgcttg cagtgttgaa aggtgcaacg      840

gctttggtct ttccttcgtt gtatgaggga tttggtttgc ccgtagtcga agcttttgcg      900

gctggaactc ccgttattac ttccaatacc acttctcttc cagaggtggc gggcgatgcc      960

gccatgctgg tgaatcctct gaatgtcagc gacattgccg ctgcaatgca atcgatgctg      1020

gaacagccgg aactggcaga gagtttcagg aacaaggggc aagaacgcgc attggcatat      1080

tcgtgggaca aaacagcctc catgacgctt gacgtctata aaaagacgcg agaggctgct      1140

taa                                                                   1143

<210>  119
<211>  1200
<212>  DNA
<213>  Methylobacillus flagellatus

<400>  119
atgatcatcc gttttctggg cgcacagtgg ctcgccgttg gttttgtcgg ggtcatctct      60

ctggcggtca gcattttgat tgcgcggacg cttgggccgg attcatttgg tgtttattcg      120

gttgtcttgt ccggcggagc cattgccgcc attctgcttg atggtggttt cgggcgattg      180

ttgcaaagag aacgcgccca tcccacacct gcattgctca gattcctgcc catattgccg      240

caggctgcct tggggcatgt gattatcgcg gccagcatat tttcgttgat cgctttattg      300

gtactgccca agcatgcgct gacgattgtt gcggttatct ggtttttcgc agcatctgtg      360

accaatcagt tcggtttgaa cattctccgc ggagatgggc ggatggtaag ggatgcctcg      420

tggcagatgg gcaatcgaac cttcacagct gcgtgcgtgt gcattgtcat tctgatggga 480

gctgagaaac cgtggcaggt attctttgcc cagtttgtcg gtgcgaccgc cttcggtttg 540

ctgatcatgc actatctgcg cacgcgcccg ctatggaaga ttcctcctac tctttatgcg 600

gcaaccttgc cattcgtgtg gttggatctc gcgacagtcc tttattttcg agcggacatg 660

ctgttgctgg aatggttaga tgtgtccaag tttgaagtcg ggaaatacgg ggttgcctgc 720

cgcttgattg aggcggtgat tctgcttgct tcgccgctct gtattatttt gttccgcaag 780

tttcgtctgg gtagcgcttc tccccgactg atgttgcgga gaatgctgcc ggtgctggca 840

ggggcgctga tggttggagt gttcttcatg gtgtttttct ggttgtgcag cacgcctttg 900

atcgcggttg catatggcga ggcctatgcg ggagctgatc atattctggc gatactcggc 960

tgcgctttgc tttttatttt gccaaatggc gtgctgaacc aagctgcgct agcgctgggt 1020

atcgaacgtt ggctggcaat gtcggcatct tttgccgcag tgctaaatat tgcaggaaat 1080

ctctggctga ttccccaata tgggttgatt ggagctgcct ggatgactgt tgtgaccgaa 1140

gcggtgttag gctgctgcgt tgcgttaggg ctatggcgcc accgtaatag gagtgtttaa 1200

<210> 120
<211> 1134
<212> DNA
<213> Methylobacillus flagellatus

<400> 120
atgcaggatc ttgccgttgc tcatgtttat cgtacctatt ttcctgaccc tccaggcggg 60

ttgcaggagg caattcgcca gattgccctc acgacgggta tgcagggcgt cgataatacg 120

gtgtttacgc tttctccgca gccgcgtccg gcaatcttgc agcgtccgga ggcttgtgtt 180

gttcgttgcc gctcctgggc ggctccggca tcatgcgatc tgggcggtat cggcgcattt 240

cttgcgtttt ccaggttggc ggaaaaatct gatgtgctac attacctttt tccgtggcca 300

tttgcggatg ttctgcatac ggtggttcgc ccaggttgtc cggccgtcat gacttatatc 360

tcggacgtgg tgcgtcagca gcttttaggg aggttatatg ctcctttgat gtggaaggtg 420

ttgcgccaga tgaggcttgt ggtggcgaat tctccagcgt atgcacgcac cagtcccgtt 480

ctctcccatc ctgagattcg cgaaaaagtc agggttattc cacttggtat tgaggagtct 540

tcttatccta aatcagggga tgaggagatt ctgcagcgtc taggcattgg gaaggatgag 600

ccttattttc tttttattgg cgttcttcgt tattacaaag gcgcgcattt tctggtcaag 660

gcagcaaagc ggataggcgc aaaggtggtg tttgctggcg cggggccgga aggcgcgcgc 720

ctgaaagcat tggctggcga agtcggtgca gagaatgtga tttttgccgg gatggtgact 780

aacgcggaaa aagtaacctt gcttcaatgc tgcagagcgc ttgtgttgcc atcgcatttg 840

cggtctgaag cgtacggcat ggtgctggta gaggctgcca tgttcgggaa gccgctgatt 900

tcttgcgaaa ttggcacagg cacttcatat gtcaaccatc atgaagaaac agggtttgtc 960

gttgagccgg cctctcctga agcattggcg tcggcgatgg cggttttgtt atcagaggat 1020

aggctggcta cagagatggg agctgctgct cgtcagcggt atgagctgct cttttccggc 1080

ccggcattag gaaaggcata tgctgcattg ttccgcgaag ttgacgcttc gtag 1134

<210> 121
<211> 942
<212> DNA
<213> Methylobacillus flagellatus

<400> 121
atgatgcaat tactggtgac aggcgctaca gggtttgtag gtaaaacttt gtgcagtgcg 60

ctaaggcggc agagcttccc cgtaaaagca attgggcgtg cgggcactat ccgggctgac 120

gctttgggtg atcaggttgc ttcaattgac ggtcaaacgg attggtcact gatattggac 180

ggcatcaatg ccgtcatcca tctggcagcc agggtgcacg tcatgcggga acagcttgct 240

gatcccttgg cggcatatcg tgaaacgaat gtcgaaggga cgctgaatct ggcgcgtcag 300

gcggcaaagg ccggagttaa gcggtttgta ttcattagtt ccatcaaggt caacggcgaa 360

cagactctac ctggaaagcc ttttactgcg tacgatgtac cagctcccga agatgcgtac 420

ggtatttcaa agttcgaggc agagcagggt ttgctggccc tggcgcggga gacagggatg 480

gatgtggtgg ttattcgccc tcctttggtt tacgggcctg gagtgaaggg taattttgcc 540

agtatgatga agttggtgaa aagtggaata ccgttgcctc tgggcgccat tcataaccaa 600

cggtctttgg tggcgattga taatttggtg aacctcatcg tcacgtgtat tgaccatccc 660

gcggcagcca accaggtttt tttggtaagc gatggcgagg atttgtccac ttcagatttg 720

ttgcggcgtt tggccaaggc tgcgggcgtt tcctcccgat tgatgccagt gtctgcccgc 780

tggctagaga tgggtttgcg tatgtgtggc aagcgattga tcgcccagcg tctcctggga 840

tcattgcaag tcgatatttc caagacgcgc gagttgttgg ggtgggttcc gccgatctca 900

gtggatgagg gcttgaagcg ctgtttctct accaagggct ga 942

<210> 122
<211> 552
<212> DNA
<213> Methylobacillus flagellatus

<400> 122
ttgatccgat tttttgacat tattttttcg gtgataggct tggtactggg attcccgcta 60

ttgctatcga tttataccct cggcttgttt gatacaggtg ctcccttgtt tcgccaggtg 120

agggttgggc gctaccggca gccgtttact ttggtgaaat ttcggacgat gaaggtggat 180

acggcttctg tggcgagcca tctggcttcc accaattcca tcacccggct cggacggttc 240

ttgcgcagga ccaagctgga tgagctgccc cagctctgga atgtcctgaa gggagaaatg 300

agcctggtgg ggcctcgacc gaacctgttc aatcaggagg agctgatggc cgagcgcgag 360

ttgcgtggcg tttaccatgc gcggccaggg attaccggcc ttgcacaggt gaatgatata 420

gacatgtcga cgccaaggct gcttgccgag accgatgcca ggatgctgcg cgagatgaat 480

gtacggcttt atttcaagtt gctgattcag acggtgctgg gtcgaggaca gggcgaccgg 540

gtcgtgcgct ga 552

<210> 123
<211> 1914
<212> DNA
<213> Methylobacillus flagellatus

<400> 123
gtgctgcatt atttcccctc attcaagtgg tcgaactaca tcagtttgaa gaatccacgc 60

tctttgagcg cttggatata tgacattgtt gctgctgcgc tggcatggtg ggcagccttc 120

ctgttacggt tcaattttga tattccggcc aattttgtcc agagtatgct cgacagcgca 180

gcatgggtga ttccgctgca ggcatttgtg tttctgctgg ctgggttgta caaaggcgtc 240

tggcgttttg ccagcctgcc ggatttgcag cgcttggtgc gcgcgattgc gatcagcgca 300

gtgcttgtgg ttgcgatctt gttcatgttc aagccacacg gcattgttcc tcgctcggtg 360

ctgatcttgg atcctatgct gttgctgctg ttgatgggcg gcagccgctt tgcttaccga 420

gcatggaaag aacatcgcct gtatggttct gcccagtttt ccggaaagcc ggtgctggtg 480

ctcggcgcgg gtgaggcagc gattgccttg ttgaaggatc tttccaaaag ccgtgaatgg 540

catgtcgttg ggttgctgga cgataagaaa accatgcatg gccgagagct actgggcgtc 600

aatgtgctgg ggtcgattgc gcaattgcca cagatcgctg cggatttgtc ggtcaatcat 660

gtgattgtgg ccatgccatc cgcgcgtcat caacgacgca gggaaatcgt cgagctggca 720

agcagaaatg ggctgactgc gctcacggtg ccgtcgtttg atgacctgat gagtggccac 780

ctgagcatgg cacagattcg gccggtcgag gtggaggact tgctggggcg ggagccggtc 840

agtctcgatg gagaggggct ccagcatttg atcgaggggc aggtgttgct gattactggt 900

gcaggtggtt ccatcggttc tgagttatgc cgccaggtgc tgaaataccg tccagcttgc 960

ctggtctgcc tggatcagtc ggagtacgcg ttgtatcggt tggagcagga gtttgacaat 1020

ctggagtttg atacccgcat tgaatacgtg gttggcgatg tcaggaacgc acgccgggtg 1080

gggcaggtgc ttggccgtta tatgcccaaa ctggtgttcc atgctgctgc gtataagcat 1140

gtgcctttga tggagaaccg caatgtggcg gaatcattgt caaataatgt cagcggtact 1200

tacacgctcg cccaggcatg caaaactgca ggggttgcca agttcgtgct gatttccacc 1260

gataaggcgg ttaatcccac caatgtgatg gggccagcaa agcgcctggc ggaaatgata 1320

tgccaaggcc tgcaggagga gtcgggtacg cgttttatcg tggtgcgctt tggcaatgtg 1380

ctgggcagta gtggcagtgt aatccccaag ttccgtgagc agattgcggc gggcgggcca 1440

attaccgtta cccatcccga gatcactcgc tatttcatgt ccatcccgga ggcgtgccag 1500

ctggtcatgc aggctggatt gatgggtggt ggcggggaga tatttgtgct ggatatggga 1560

gaccctgtgc ggattgtgga cttggcccgt gatatgatcc ggttatccgg gcttaatgaa 1620

gatgacattc agattgtata ctctggcttg cggcccggcg aaaagctgta cgaggaactt 1680

ttggctgacg acgagctcac tgtaccaaca ccccacgaca agctacgtat tgccgtggcg 1740

cgaagcgtgg atggtgcttg ggttcaggca ttgctgaaat ggatcgacag ggcatcgctc 1800

atagatgaat ctgtcatcaa gcaagagctg ggcatgtggg tggaggagta tgcaggggat 1860

attcatgcca atccatccat gatgttcccc cccgagtctt caaccataca ttag 1914

<210> 124
<211> 876
<212> DNA
<213> Methylobacillus flagellatus

<400> 124
atggcaaaaa aaattaccaa ggcagtattt ccagtggcag gtttagggac acgtttcctg 60

ccagcaacca aggcgagtcc caaggaaatg ttgccaattg tggataagcc tctgattcag 120

tatgcagcag aggaggctgt ggccgccggt gctaccgagc tgatttttat tatcgggcgc 180

aacaagcgca ccatcccgga tcattttgat aagtccttcg agctggaagc gacgcttgag 240

gcaagcaaga aaaccaaggt gctggaagaa atccgcaata tcctgccaaa aaatgtttcc 300

tgcatctata ttcgccaggc cgaggcgctt ggtttggggc atgcggtatt gcttgccaag 360

tccgtggtcg gcagcgagcc tttctctgtg atcctggccg atgaccttat tgatgccgac 420

aaacctgcca tgcagcagat gacagagctg ttcgaccaag agggtgtcag cattctgggt 480

gtcgaggatg tggagcccag tgagacggca agctatggca ttgtggatgc gacagaggtg 540

agtggcccga atgtgttgca attgaatggc attgtggaaa aaccgcagcc ggaagaagcg 600

ccctccaatc tggctgttgt cggtcggtat attctcacgc ctgaaatttt tgaatgcctg 660

gagaacgtca agccgggcaa gggcggggaa atccagttga ctgatggcat tgccgccttg 720

atggaaaaac agaaaatcct ggcttaccgt ttcgatggga agcgctacga ttgcggcagc 780

aagctgggtt atgtcaaggc gaacgtcgag tattccttga agcatgagga gtttggcaaa 840

gagtttgccc agtacttacg tgatctaacc aagtag 876

<210> 125
<211> 819
<212> DNA
<213> Methylobacillus glycogenes

<400>  125
ttgctacaac aattttctat cggggttaga gagcaccatc acaaggagct cactccagac      60

caggctggcg ctttcctgga ggtggttgac gcatcattgc gtatcaagaa aaaggcccag     120

ttcttttcct ggctacaagg ttattttcaa ttcctgctac cgcatgaagt actactctgt     180

ggtctgtcca taggcgcagg acgtcccttc acgctggaaa ccttcagcag cacgcgctat     240

gtcaccgata cccatacggc cgtgagcatg caggaaactg atggtgtggt gttgcgtgcg     300

attcagtcct ggcaggaaac cagcctgcct gtgttggttg atccttatct acgtcccggc     360

agtttcggta atttcacggt gccggagata gatcaggcca atctacaggc cagcgagcta     420

cgcaatctgg cagcttatgg tctgtcttct gccgatggca gcatttcctc tttcttctgt     480

ttttcacgcg tggccgggga gttgaatggc aaccatgcct atctgctgga attgctggtg     540

ccttatctgc actcggtgct gatgcgcgtt agcgacagtg ctacggcttc tgccgatatg     600

ttgggcgata gctcggtggc ggggctgatc accatgcgcg aaaaggaaat cctgcattgg     660

gtacacacgg gcaagagtaa ctgggaaatt gccaccatcc tgcagatcag ccctttgacg     720

gtaaaaaacc atatgcagaa tattttgcgc aagctggatg tgcaaaatcg cagtcatgcg     780

gcctccaagg ccttcaggct gggactgatt cgtaactaa                            819


<210>  126
<211>  1140
<212>  DNA
<213>  Methylobacillus glycogenes

<400>  126
gtgggttatt ccgaccctgt gctggctcgt gaaggtgatg tcatacggcc ctatgtcagt      60

gcaacatatg tttatgatga caacctgagg cgccgctctg acaaggagtc cgatacgagt     120

gtagtgggcc gggttggcat tattctggat aaggatatta gtcagcagaa tttctacgcc     180

aatatcggtg tgaatgaaac caaatacaac aaaaattcag agttagacaa tacaggtaaa     240

gatattgtcg ctcgatggaa ctgggcagtg ggctcgcaat tccggggcaa ggttgagtac     300

tataaaaacg aggctatgct gccttttgcg gatttcgtgg gtgtggaatt gaatatgcgt     360

attcaggagc gtaaaacttt tgaagcaaac tggcgtttcc attccagttg gcgcgcgcgc     420

tttggtgtgc agcgccagga tgttgagtat gactctcaaa cattgaatgc ggcttctttt     480

caggaaaatt cccagcacgt aggtttggac tatttagctg agtcaggcag taaggttggc     540

ttggtcttca ctaatgggcg tggcacgcgg ccctttgata ggcctgcttt tttcccgggg     600

attggtaccg tgtttgtcaa taatgactac aggcagcaat cactcaaggc ggacatactt     660

tggctttatt ctgccaaaac acgatttgaa tttttggggg ggtatgttga tcgtaaacat     720

gacagcttct cacagcgcga tttttccgga tttaatgccc acgggaacat ggtgtggtcc     780

attaccgaca aaacctcttt caagacagcc gcatggcgcg atgcaaatgc tatatcaatc     840

gtaactgcca gctatgtgat tgatagtggc gcaagtgcaa gcatctccta tagcgcaacg 900

agtaagatcc tgctgtcaac ttcagccagg tatcaacaac taaaatttga aggcgatatc 960

accgggcaag atcaaggtcg taccgacaag aataaggttt tttcttttgg ggtaacgtac 1020

agccctcttg aatcgttaag tttcactact ggtctaaata gaagtttgcg tgactcaacc 1080

tcaccaattt taaactacaa ctcgacaagc atttcgctta ctggtcgtta tgaattctga 1140

<210> 127
<211> 1384
<212> DNA
<213> Methylobacillus glycogenes

<400> 127
atgaccgtaa atgacatccc tttagtagcg tttttcaagc agctgctcga cccgctgatc 60

attcttggta cgctgtatgt gttcaccctg ttgtttggtg agccttttaa tggctactac 120

ctgattctgg cgttgctagc ctactttgtt tcctctaatg tctacgagcg tctggatctt 180

taccgtacct ggcgtagtgg caagatactt ggttatatcc gcgatattct ctttggctgg 240

ggtattgtca tcgctgtgct gatgttcatc ggctatgccg ctgatctatc cagtcagtat 300

ctggatgaga tgatatatgc ctggtttgcc agtgtgccga tgatatttat tgtcacgcat 360

ctggcgatac gcaaaattgc ctcggatttg cacaagaatg gtgaagtcag gtccgtagtg 420

attatcggtg ccaatgatat tggcgtgcgc ttgatcaatc gcatcacgga cgacccgtac 480

ctgtttatgg aggtcaaggg gatctttgac gacagagagg tcgccaggca ccccaagggc 540

atgcgtaatc tgcctttgct tggttccatg tcggaaattg cgccatatgt ccgcgccaat 600

aatacccaga ttattttcat cagcctgccc atgtcggcgc agccacgcat caggcagtta 660

tttgaagaat tgcaagacac cacagcatct atttactttg tgccggatat ctatattttt 720

gatctggtac aagcgcgctt tgatgatgtg gctggtgtgc ctgtggtcgc tattcttgag 780

acgccattca ccggtttcaa cagtttaatc aaacggacta gtgacattat tttggcgtcg 840

cttattctat taatgctgtc tcccatcatg ctgacactgg ctctggccgt gaagttgacc 900

tccccaggcc cggtgatttt caagcagcga cgttatgctg aatggtgagg aaatcattgt 960

ctataaattc cgctccatga aagtaatgga agatggtgac aaggtagtac aggcgcagaa 1020

gcgagatcag cgattgacac cgattggtgg tttcttgcgc aagaactcac tggatgagct 1080

gccgcagttc atcaatgtgt tgcaaggtca gatgagtatc gtggggccta gaccgcatgc 1140

cgtttctcat aatgagcaat atcgcaagct gattcgtggc tatatgctgc ggcacaaggt 1200

caagcctgga ataacaggct gggcgcaggt gaatggttta cgtggtgaaa ctgatacctt 1260

ggacaagatg gagtcccgta tcgagtatga cttggattat ttgcgccatt ggtccctggc 1320

ttttgacttc tggattatcg tcaggacggt gctggttgtg ttcaaaaaag ataacgccta 1380

ctag                                                                  1384

<210>  128
<211>  933
<212>  DNA
<213>  Methylobacillus glycogenes

<400>  128
gtgaataacg gatttgtaac taacttcaag caatccagaa ccctgtgtgt cggtgctgtg      60

attgtgctcg ctttcgcatt gtcgggctgt ggcgacaagc atgaatccaa agccagccag     120

tctctggtta aggtcaatgg caaggaaatc accgtacatc agctgaatca ggagctgggt     180

cgtgccaaca ttcaggagaa tcagcgcgag caggcaacgc agcagattct gactgcattg     240

atagatcgcc agttacttga agaagctgcg ctcaaggcca aggtggatag ggatccaaat     300

gtgatgacca cgattgaacg tgcgaaatca caaatcattg ctcaggcata tctgcaatcc     360

aagttaggca gtgtcgccaa gccaagcaaa acagaaattg agaattttta taacgagaat     420

cctcaattct tcagcaagcg cagaatggtg gaaatggatc aattgttgat tgatactaaa     480

ttcgccaatg atgaattgaa aacagcactt ggcaaactca agaccttgca ggaagtagcc     540

gcatggatgg atgcccataa tattcagtac gatcaaggtc aggtttcacg cagtgttgct     600

gaattgccgc cacagatttc cgagcgcctc aaggccgagg ataagggtaa gttgttcgtg      660

atcggcatgg gccctcgcac catgttgatc atgatgaacg agatcaagga cagcccagtt     720

tcacttgcag aagcaacacc gcaaattgaa aacaatttgc tgaataaggc gcgcaaggaa     780

gttgctgaaa atgaagtcaa gcgcttgaaa gccgatgcca agattgagta catcgataaa     840

aagatggaaa cagcgactga aaccaaggca gccaaaaaga agccaagctc tactgaggat     900

gatatcgcca gtggtgttag tggcttgaaa taa                                  933

<210>  129
<211>  795
<212>  DNA
<213>  Methylobacillus glycogenes

<400>  129
atgaaaaaat taatgacatg gtttttcttg ctgatgctga gtctgacgcc ttttgtaacg      60

catgctgcag aaggtgacat tcttctcggc gctggcgatg tgctgaaaat atcggtgttc     120

gagcagcctg atctgtcact tgaggtcaga gtgagtgaat caggttccat tacttatccc     180

ctgataggtg aagtgcgtgt gggtggtggt acggttgccg ccgccgagca aagaattgcc     240

accatgctgg aaaagggtgg tttccttaag agtgctcacg tcaacatcat cgttgcgcaa     300

ttgcagagcc ttcaagtctc tattctgggt caagtcaatc gcccgggcag ataccctata     360

gatagcgcta aaacgctggc ggatgtgctt gccttggctg gtggtgtatc acctgaaggt     420

ggcgatctga tcacgctggt gcataacgtc gatggtaaag cggtcaggga tcagattgac 480

cttgctgaca tgatgcgtac tggcgatctc agcaagaaca tccgcgtagc tggtggtgac 540

atcatttatg ttgaacgttt cgataaattc tatatctatg gtcaggttca acgtgccagc 600

cattaccgtc tcgaacatga aatgacagta ttgcaagcgc tttctgtagg tggtggctta 660

agccctcgcg gcacagaaaa aggcgtcaag atcaagcgtc gtgatgctag cggccatatt 720

gaagtaatca ctgccaaaca tgatgacctc atcaagccca acgatgtgat ctatgtgcag 780

gaaagtttct tttaa 795

<210> 130
<211> 1389
<212> DNA
<213> Methylobacillus glycogenes

<400> 130
atgagctttt ctcaactttt actgatattc aaagcgcgat ataagatatt gttaagcacc 60

attgttgtgg ccctggtcgt agcaattgcg ctcaatattt tcttgccaaa cagctataag 120

gcaaccacca gtatcctagt caactataaa ggtgtagatc ctgtgactgg tatggctgtc 180

cctgcccagc tgcttccagg ttatatgggt acccaggtgg atatcataac tagcaagaaa 240

attgctaaga gggttgtgac cacattaaag ctcaatgaaa gtagtgaggt tgtggagagt 300

ttcaatgact ccacaggtgg tgaaggtgat atcgttgatt ggttggcgga tttgctgatc 360

tcacgtatcg aagttcaacc tgcgcgcaac agtagtgtgt tgcagctgga gtttgatgga 420

gccaacccca aatttgctgc tgttgttgct aactcctttg ccgaggaata tatcaaggcc 480

actatcgaac tcaaggttga gccttcgaaa aaggcggctg aattttatac cgggcaattg 540

aaggatttgc gtgccgatat tacccgggcg cagcaaaagc tttccgatta ccaacaagat 600

aaaagcatcg tcagcgttga tgagcgtcta gacattgaac gtgccaagct gaatgaactt 660

tcctcgcagt tagtgatgtc acaagggcaa actcttgagg cacagtctcg tcagactggt 720

gcacaaggta gcaatgctta tggatctccg gatgtcgcga gtaatccaat ggttctgcac 780

ctgaaaacag agattgctag gtctgaatca cgcttcgcgg atatctcaca aaaatatgag 840

agaaatcacc cactctatca gggggccaaa gctgagcttg acaatctgaa gtctgaatta 900

agtaagcaaa tccaggcggc taccgtcaat atatcgagca attatagaat ccttaagcaa 960

cgcgagagtg aaatccgcaa tgccttggct gagcaaaaaa ccaaggtcat gctgttgaac 1020

aaggataggg atgcactgac ggtattgacg cgtgaagtcg agggtgcgca acgcgcttac 1080

gaactcgcga ctcagcgata tactcaaata aatcttgagg gacagtctaa tcaagctgat 1140

gtatccatcc ttaatccagc gacaccacca cttaagccat caagcccgaa aaaaatattg 1200

aacgtggcag ttgcgttggt gctgggtaca atttttggct tgttgtttag tttgttggct 1260

gagctcacta atgagcgaat tcgctcagaa agagtgctgg tagagattgt tggcgcaccg 1320

ctgctgggtg tgatcgacaa aaataaaacc cattccagaa ttcagcgcct gctggctaaa 1380

aaaccctaa 1389

<210> 131
<211> 879
<212> DNA
<213> Methylobacillus glycogenes

<400> 131
atgactaatg atgtttccaa tactagttcc attccggcat tgaatgctga tttcaatatc 60

ggtggaatgt tgctcaagat gggtaagatt acaccgcagg atgcggagcg tatcctgcgt 120

gtgcagaagg aaagaggcgt tcgctttggt gaggctgccg agagtttggg cctagtttcc 180

tctgccgata tcaactatgt attatccaag cagtttgcct atccctacct gatggatggt 240

cgcggcgatt tctccgagaa cttggtcgcg gcttatgatc cattcagtat caatgtggag 300

aagtatcgcg ccattcgtag ccagctcatg ctctactggt ttgcacaagg gaaaaagagc 360

ctgtcatttt ccagtgttta cgacaaggac gacaccagtg acttggttgc caatcttggc 420

gtggttttct ctcaactggg agagaaaaca ctcatcattg atgcccatct tgatagccca 480

cggcagaatc gcatttttaa tatcaagagc actgcgggtt tgtcggatat tctggcaggc 540

cgggttacgg attactcctc ttccatcact aggctagtcg agttcgagca tctctccatc 600

ttgtcttctg gcaccattcc acctaatccg cgagagcttc tgggacgtgg tttcctcaag 660

cagttcatca cggcaattgc taatgactac gatgttatcc tggtcgatgc accttccttt 720

gtccgccact ctgaaccgtt gtctgtggca agtgctacgg aaggggtggt gcttcttgcc 780

aagcgtcatg tctccaaagc caatgacttg agcaaagtgg ttgatcagct ttccaagatc 840

agggtgcctg ttgttggcac ggtattgact gaaaactaa 879

<210> 132
<211> 900
<212> DNA
<213> Methylobacillus glycogenes

<400> 132
atgagcttga accagagcat tgcggtcgcc agtacccagc taagaaggta ccagcatgtg 60

ataccactgc tggtaggctt gctgttctta tttgtgccca cctacctgaa cctgacgaac 120

acggtctggc agatgcagga tcaggcgcat ggccccttga tcctgatggt ggtgctgtac 180

ctagtctggg tggacaagga aatactgcta gacacggcaa acttgagaca aagcgggcgt 240

ggtgtgggcc tgttgctgtt tttgtttggc ctgttggtat acatcatcgg gcgttcacaa 300

gatttgctgg tgtttgatgt gggctcgcag attccggtat tggctggctt gctgttgatt 360

ttctataagg ccaaaaccct caagcgcttc tggttcccct tgttcttctt gttcttcatg 420

attccattgc caggctccat gctggatgtt gtgaccttgc cgatgaaggt cgctgtctct 480

tacgtggccg agcatgtgct gtattggttt gattacccca tcgcgcgttc gggtgtgatc 540

attcagattg gtcagtatca gttgctggtg gcggatgctt gtgcaggcat gcataccttg 600

atttcactgg aggccctggg cctgctctac ctcaatatcg tcaggcatga ttccctggcc 660

cgtaacttgt tgctggcctt gctgattatc cctatttcct tctcagcgaa tgtcatacgc 720

gtgattgtgt tgaccttgat cacctactat ttcggggatg aagtggccca gggctttgta 780

catggattcg cgggtatttt gttatttgtc gtggcattaa ccctgattat ttcagtggat 840

agcctgatcc aatactttgt tggcaaacat acacagcgta aggctagatt gcagagttga 900

<210> 133
<211> 687
<212> DNA
<213> Methylobacillus glycogenes

<400> 133
atgagcgtca gattaatcaa tatcctggca gtatgcatgg tggtgagttc tggcctggcg 60

ttggctttga cccctgatct tagctccgtg cgggatgatc cacatcttga aagtttcgtg 120

cctagcagct ttggtcagtg gcaggaagtc aaaaccaatc aggtgcagat ggacctgaca 180

gtcaggagag atggcgaacc cagcatcgat aacccttatg atgaagttct catgaggagt 240

tatgccaatg aaaagggtca ggtcattatg ctggctcttg cctatggcaa attgcagcgg 300

caggaagtca aggtgcatag gcctgaactg tgctatacgg cgcagggttt ctccatcaga 360

aatcagcagg ataccgcagt gaatgtgggg gcaggcgcaa cacccatccc atccaaggcg 420

ttggttgcgg cttcaagaca gcgcgaagag ctggtgcttt actggatcag gataggcgat 480

atgatcagta agagtgcttg ggagacccga ctttatattg tggagcaagg cctcaaacat 540

caactggtag atgggatact ggtcagaacc tcacagatat tgggtgaaga tgatgccgag 600

caatctttgc agctacaaca acagttcctg cacgatctgg tgcaagcagt gcccagggac 660

aagcgtaaat acttgattcc atcataa 687

<210> 134
<211> 1137
<212> DNA
<213> Methylobacillus glycogenes

<400> 134
ttgaagatat tatttctaag tcacactgcc gcagggggcg aatttgtagt tggctcacat 60

caactatcta aacaattcgc cttgcaaggg catgaggttg tccacctcag tccgccagtg 120

acaccagcac atctgacact gttgcagaaa acaccgtttg agcgtgtccg gtttaagcgc 180

tggatgaaag cgggtgatgt catcaaccac gtgaccgaca ttattccttt ctcattaata 240

ccttggggta ttgccacccg cttgggatct ggtagcgata tctttggtta cttcctgaag 300

ttttcagcac tcagacaact ccgtaaacat caattttcta atcctgacgt agtgttgatt 360

gatgagcctc gattaggcta tctactcaaa tatttcccta agtcaaagat cgtataccgc 420

cccactgata tttatcatga aatcagaaat gatcccagta ttgtagatgc tgaaaaaggg 480

ttgatatccc aggtacacgg cttcattgcg acatcagagc ctgtaagaca gcatttgaat 540

cgcctaggtg ttgatgaggt gctattgctg cagaatggtg tagatctaga tcattacctg 600

aattttacaa aatccaaact ggatttgccg cctgagccaa gggcaattta tgctggagct 660

ctggatcatc gctttggttt tgatgtgctg aaaagtgcag ccacaaaaca ccagaaaatt 720

tcatttttat tggctgggcc agtagaggaa aaatacaagg cggcatttag tgatcttgcc 780

aacgtacact ttcttggtca tgtgcaatat aaagcgttgc ccaatctctt gagtcagtgc 840

caaattggtt tattgccgat gtctgatcat ccatccaacc ttggccgtag tcctatgaaa 900

ttgtttgaat atgcagcagt cggacttccc gttgtttcta cctcaacacc agagcttgag 960

cgcagatcat taccctttgt atttttggct gagaatgccc agattttcac agataaactt 1020

ggtgatgttt gcaatgcgga tatcaaaata ttggctcagc aggctcgaga taatgctaag 1080

catgagtctt gggcaagtaa aaccatcaca atactgaact atatttattc aaagtaa 1137

<210> 135
<211> 1263
<212> DNA
<213> Methylobacillus glycogenes

<400> 135
atgaacaaaa ctactgaacc acaagatagt caaaccaatg ttccatggtg gctatcccct 60

catggactat gcttaggctt tcttttgcct atgatcttgt tgatctggtg gttggggtca 120

gtgagatcac caacattaac catacggggt tatatatatt taagttctga atatatagct 180

ctggctgtgc taattgttgt gctgtcagca agtgcagcct ggatgggaag cttcgtcaag 240

acaaaagtca gagtgccgcc agactctcag gttaatatgg ctttgcgggt attaggctat 300

gtagcagttg cagcatatat atactggttc aaagagctga ttatcaatcc tatgaatatt 360

attaatatat ttacagggac aggtttatca agacaggaaa ttggtgcaac gcaagggatt 420

acatcacttg ttaacttcac cccaatattt atttcgattt acatatatgc cagatatatc 480

gccaaattac ctttaagaca agatgttgta ttgttgttct atgcaattgt tttattgaca 540

ttgctgcggg tatttgcctg ggcagagcgt ctggcattaa ttgaaattat tttgccattt 600

gcgttattga taacgcggca acaagtgttc agtaagatta aaattttgca attcttcatt 660

aaggctggtc ccttcgcggg attgcccttg ctggtgcttt actttggtat ggcggaatac 720

ttccgctctt ggagctctac aacttacgcc gggaaatcga gtttttggga ttttgctcta 780

gggcggattg cttcatatta ttacacctcg ctcaataatg gtgcgggaat gctggctact 840

gttaactggc caacatatga attcgaaaat gtgttgaatt ggatgcataa atttccaatt 900

tttggcggcg tgtttcgtta ctacgtagat ttgcacacta tgagaatggg tgagtttctc 960

gctcagtatg gtgatgagga gttcaacaac ccttctggga tattttcagt gatcctggat 1020

ttgggcttgc catttggcat tatctacttc tttataaccg catttcttgc aggaatcttt 1080

tataaaggct atttgagagg caatttttta agtgtaattt tttatccgtt atttttattt 1140

acgttattgg aaattttcag atatccgtat ttaggaacgt cccgagcttt taccgagatt 1200

attggtgtga ttattgcttt ctttatagtt aagggaagga ctgaaaatga tagaaaatcg 1260

tag 1263

<210> 136
<211> 1365
<212> DNA
<213> Methylobacillus glycogenes

<400> 136
atgatagaaa atcgtagaca cctggttaaa tcattattct tgatgctatt ttcatcctgg 60

cttcccaagg tcgcctcggc agcgcctaag gtggtcgggg gcaatgcaga cttcactaga 120

gctttagatg acgcgcttgc tggacgcacg gaagtgctaa gcggttatta tgaaataagt 180

aatcctttaa gattttcact gaagagtggc accgtaaaat ttgcaccaga ttttcagctg 240

aaattcttga atgataaatt aagtggttta gtattttcga gttgcagtga tttatcactc 300

attggtggag atttttcgta caaaaacccc ccagctagaa gaattggaca tggtggtatt 360

tacgagttct ctagatgtac caaagtcatc gttagagatg gattttatta tgacacacca 420

ggtacaggta tcatttttac cttatgtgat gattgtgcag taattaaagc tacggtttgg 480

aattcctggg ctgatgggtt gcactttgca aattgcaata actgcatcgc cttggattat 540

cagtctacca atacagggga tgatgggttg gcgtttgtaa actacaaaaa acatcccgat 600

gcagtaggtg gttatgcgaa caacattgtt atcaatggtt cttatgcacg aggaattgcg 660

attatcggtc aatctggtgt gactatagac aagtttgaaa ttaggcacac ttccgcagaa 720

ggattaaaga ttggaagtga gcaatactat gacactagaa ttccttccaa tgtgacaatc 780

aaaaacggca ttgttgaaga ggcaggtact tggtgcgtgg caaaggcaac tggcaatggt 840

ttcctagtcc aaggtgctac cgataaaatt gttgttgaga atttgactgt caatgcccca 900

cgcgtgcatg gaattgtttt cgacaggcat ggcggtaatg tcaaaatgaa agatatcgtc 960

gtcaagaata caaaagcaag tggcataacc atgatggcgg ataacatcga gttagtgaat 1020

ggggagtttt taaatattgg aacaacacct ctttacgtca ataaagcatt aactgctaat 1080

ttctccaaga ttaatgtcaa taccttcgga ttcgcagata aatcaagttg gcctattttc 1140

tgggctgttg aagctaagaa tgtgttaatt aaccaaatga aagtggagtc caccaccttg 1200

gttagaattg gtgggaaaaa cgttatttca catcagataa ctggggtgca gatcagtggt 1260

gtgcagctta gcggactgct ctctgatggg cttaattcca aaacaacatc ggcggtttct 1320

gctataacat caactgctgt taagtttatt gagcagtgga attag 1365

<210> 137
<211> 1107
<212> DNA
<213> Methylobacillus glycogenes

<400> 137
gtggatgcag gtgcgagatt gaggatttca tggatcgatg tctgccgtgg atttgcaatg 60

ctgttggttg tggttgggca taatcagata ttaatgggcc aaagtctttc tttggtaaaa 120

tttatttatc tttttcatat gccactgttc ttttttatta gtggtataac cctcaagcca 180

gatcaaggat taacgtacta tttcacgcgg gcgattaatg ttattgcggt atattttgta 240

ttttcatttt tagttcttcc ttttgtttta atgaaaaatc cagatgatgg tgttttcaat 300

atagttcttg gtattttgta cggaactgga catacaatag aagttgctcc tctctggttt 360

ataccatgtt tatttttctc tattgttttg gtcggtctcg cattagttat ttcaaatcat 420

tttaataaca ctgatcgtga atcaaataga agtccgattt ttttccttgc attattgttt 480

atcggagttg gtgtttttct cattaactca attcattatt atttaattga taatcttgct 540

tggggtagtc tgaaaaattc aggaatgatt tggagtattg atatagcaat aattggtgct 600

ggctttattc ttctaggtat gtacgcaaag gtatttgttg ataagtacaa aattactgaa 660

atcaaatcaa tatatcttat tgtatctatt gtttgtgcga ttttcgttat gttggggttg 720

tttagttttg gtgttgacgt cgatttgaat tttagaaaaa ttgcgcatta tcctttggct 780

atgctctgca tgatcttagg aatatttttc agtattttgt tttcaatttt tctagataaa 840

tatacctacc tatcaactat cttgagcagt attggtaaat ctacgttatt gattctttta 900

tttcacaatg cgattcaaaa tcatttaatt ttcacaattg gtgattcaaa aatatatagt 960

gctgtgatcg gcattgttat cgcaatattg atacctatag tacttgataa gtttgttgtg 1020

aataaatacg aattgttcag aaaaatagtt tacccaaaac tatctatcaa ccttgcatcc 1080

aagataatgg tagggaaaag attttga 1107

<210> 138
<211> 1098
<212> DNA
<213> Methylobacillus glycogenes

<400> 138
ttgattgtat atattaatgg ccgattttta ggaagacgca taactggggt tgatcgatac 60

gcaatagaag tattaaaaga gttaaataca ctgattgcgg aaaatcatcc cagtgtagct 120

ggtgttgaat ataagattgt tattcctcct cgcatcaaaa ctgacttgca tctaactcat 180

atacctatag tggcttatgg tttttttagt gggcatgcgt gggagcaatt gtccctacta 240

atgttgagtc tgagtagtaa gtcaatggta ttaaatttat gtaatacagg ctcaatcttc 300

gtgcgtagtc aaatcgtcgt cattcacgat gctgcagtgg tgagaattcc tagtacattt 360

tctttagcat ttcgggtgtg gtacaaaaca ctcatgccag tactcggcag agtcgcaaag 420

aaaattgtaa cggtttcaga atattcgaaa aaagacatta ttaacttttt tggtgtacat 480

cctgaaaaag ttcaagttgt tggtgcaggc ggcgagcata ttttgtcgca acagccagac 540

gcaggtattc ttgaaaaatt tcaagttcac ccgggctctt atgtgttggc ggttagtagt 600

catgctccgc acaaaaattt ttctttaacc attgaatctt taaaactttc taagaatgca 660

aatattccgt atttgattgc aggtggggct aatcagaaaa tttttggtga aaattcccag 720

ttagattctt ctaaggtgac gttgctgggt tatgtctcgg ataatgagtt gcgcgcgctt 780

tatgaacatg cgctttgttt tgtttttcca tcactttatg agggcttcgg tcttcctcca 840

ctggaggcga tgaattgcgg ctgccccgta cttgcatcaa atgctgcatc aattccagag 900

gtgtgcagtg atgctgcatt atatttttca ccgcatgatc cgcaggagct ttcaaagctt 960

atagataatg ttgctcatga tgccgatttg agaaagtcgt tagtcaacca aggcttgaag 1020

cgagcaaaag atttttcttg gaaaaattcc gcaatagata ttgcacggat gtgcaaggag 1080

attttcacaa taaagtaa 1098

<210> 139
<211> 1155
<212> DNA
<213> Methylobacillus glycogenes

<400> 139
atgactaata aatatacgag gcattcgaga ttgcacccaa aaaaagtagc tatcgttgtg 60

gattggctta atgaatatgc tggagcagag cgtgttctgg agcagttggt tctttgctat 120

cctgatgcag acatttttgc tgtcattgaa ttcttacctg ataacctgag aggttttatt 180

cagaataaga aggttacgac aacttttatc caaaacttgc ctaaagctaa aacgcattat 240

cgcaattatt tgccactaat gcctttagct attgagcagc tcgatctgtc tcaatatgat 300

ttgattattt ctagttctca cgccgtggcc aagggtgtgc ttactgggcc agatcaagtg 360

catgtaagtt atgtccattc tcccatgaga tatgcgtggg atttgcaaca tcaatacttg 420

cgtgagtcta atttggtttc aggttttaaa tcttggattg tcaaatggtt gctacataaa 480

ttgcgtattt gggatctaag aacggcgaac ggggtagatg tttttatcgc aaactcttct 540

tttattgcta aaagaataaa gaaggtatat ggacgtgagg ctagggttat ttatcctcct 600

gtagatacca acagttttgc attccaggaa aagaaagatg actttttctt gactgcctcg 660

agattggtac cttacaaacg agttgacttg attgtggaag ccttttccaa aatcccagat 720

cagaaattag ttgtcattgg ggatggtcca gacatgtcca aggtgcgtgc aaaagcaggg 780

ccaaatgttg aaatcctcgg ttatcagtct catgctgtgt taagagacta catgcagcgt 840

gcgaaagggt ttgtcttcgc ggcggaggaa gattttggta tttctcctgt tgaggcacaa 900

gcatgtggta ctcccgtgat tgctttcgcc aaaggtggcg ccttggagac agttattcca 960

gaagagcgag gtgatcctgc cactggtttg ttcttccatg agcagacagc tgatgccatc 1020

gtggatgcta ttactagatt taatctactc tctcaaaaga ttagtccatt tgcatgcaga 1080

gagaacgcac tgcgcttttc tcctgatcga tttagaaatg agattatttc aactgtgaat 1140

aaggctctcg attaa 1155

<210> 140
<211> 1167
<212> DNA
<213> Methylobacillus glycogenes

<400> 140
atgaaaatag ctgttgcagg tactgggtat gtgggcttgt cgattgctgt gcttttagca 60

cagaaaaatg aagttgttgc tcttgatatc gtccctgata aagttgacct tatcaacaat 120

aaaatttctc caatcgttga taaagagatt gagcattatc ttaagaatat tccattgaac 180

ctacgtgcga ctacaaacaa gcatgatgca tatgaaaatg cagattttgt agtgattgca 240

acccctacgg actatgatcc ggttaccagc tacttcaata cctcctctgt agaggcagtg 300

atcaaggatg tccaggcgat tgcgcccaac gcgaccatgg tgatcaaatc caccatccct 360

gttggttata ccaaaatgat ttccgaaaag ttgggtgtgg ataacctgat tttctctcct 420

gaattcttgc gtgaaggtaa ggcactttac gacaacctgt atccctcgcg tatcgtcatt 480

ggtgaaattt cggatcgcgc acaagttttt gccgaccttc tcaatgcagg tgctgtgaaa 540

gagaatattc cggtgctatt gaccaatagt gctgaagcgg aggccattaa gctgtttgcc 600

aacaccttcc tcgccatgcg cgtcgcctat ttcaatgagc tggatactta tgccgccacc 660

catggcctga acacacgtca gatcatagat ggggtgtgcc tagacccacg tgtcggtgac 720

cactataaca atccatcgtt tggctatggt ggttactgcc tgcctaagga taccaagcaa 780

ctgctggcca attacgacaa cgtgccgcaa aaactcatca atgccattgt tgatgccaac 840

accacgcgca aggatttcat cgcccagagc atcatcaagc gtgaccccaa ggtggtgggt 900

atctaccgcc tgatcatgaa agcaggctcg gataacttcc gcgccagttc aatccagggc 960

gtgatgaagc gcatcaaggc acgtggtatt aaggtcgtgg tgtttgagcc tgagttgaaa 1020

gaagatcaat tcttcaattc agaggtgatc cgcaatctgg aagagttcaa gaagatttca 1080

gacgtcattg tcgccaatag aagggcgcag gatttgtcag acgttgatga gaaagtatat 1140

acgcgtgatt tgtttggcaa tgactag 1167

<210> 141
<211> 972
<212> DNA
<213> Methylobacillus glycogenes

<400> 141
atgaggatac ttttttcacc tgctacggcg agtaatcagt atgtcgagaa cacgattgcc 60

ctgattaaaa ccctagacgc tacccatgtc gaaacccaag ccagattgaa tgctggattc 120

atcttgcgca atgctttcaa ccgctatgat tgcattatca tcaactggct ggaaaacaac 180

ctggttggaa aaaacaagtc gcttgatata tgggcggtag caaaatttct gttcaagttt 240

ttcatctaca gagtcatggg gagaaagttg atttttgtca ggcacaatat ttatcctcat 300

gccttgaagg gtcggcaggc atccatcgcg acctggatca ttgaagcggc gattaagctc 360

tcaacaatat ccgttgtaca ttcagggcat ctcgaaaatg agaacgcgaa atatgtacct 420

catcctttgt acagggttga ttcgcaggtt gaaggccaag gcagtgatta ttatgtaatt 480

tttggcagga ttgttgcgta taaagccatc cataaattac tcgaaagctg ggggtcgcag 540

cggctaatca tagccggcat tgtcgaggat catgcctacg ctgccagctt gcaatccatc 600

atacagtcca gagggttgag caatgttgaa ctgcgtgctg aattcctctc cgaagccgag 660

gctgaacaac tggtagctaa cagcaagggc ttgattcttt cacattgcga tgccgacatg 720

attgtcagtg gtagtttttt ctttgctgca agtctgggcg tgcctgtttt tgccatggat 780

actccatttt tccagtggct gaagaatgag caaggctatc agggcttgca ggtgttcaca 840

tcactgcagg caatgacggc tgagctagcc actgccgcgg ccgtggaaaa aaatactatt 900

gctgcttcag cccagcgttt atttggcaat gatagggtga gaagtgcatg gggtagtgtg 960

ctccatggtt aa 972

<210> 142
<211> 1413
<212> DNA
<213> Methylobacillus glycogenes

<400> 142
atgaccacga taggtcagga tgttaaccag cctgcaggca ttcctgaagt atttacaatc 60

aatgatgtcg ttgacagagg gctatgcgtt ggatgtggtg cgtgcgcggt tgtagattca 120

aacgtcaagc taacgcgcaa tcaatatggt tccatggtgg ccgccattga tgccaatatc 180

ctgccagggt ctattgccga ccgggtatgc ccattttctg ataacagtct taatgaagat 240

gttgtgggag aacgggtttt tgcgagttgt gaaaattttg atcaacgtac cggttattac 300

accaatttat attatggccg caggttcact gaagctgaat tgctgaaaag cagttctggt 360

gggctgacca gttatgtcgc cattgaactt ctcaagcgtc atcacgtgga tggcattatt 420

catgtgggac gcgaccagtc agatgatcgc gatgctttgt tcaagttcat ggtgtcttac 480

acagaagaag acgccaatgc ctgcaagaaa tcacaatatt attccctgag ttttgatgag 540

gtagtaagaa gcatacgcgg agatggtaag cgttatgtat ttgtgggcgt gccctgcttt 600

gtcaaatctc tgagaaatat ttgtctcaat gatgctgtgc tcaatgagca gattccattc 660

atgctgggct tggtgtgtgg tcatttgaag tcttcgcgct ttgccgaatt actagcgtgg 720

caacttagtg tgccaccgga aaaaatccag agtgtggact ttagagtaaa aaataatgcc 780

tccaccgtga attcctacga tttccaggtg gtggatagca atcagcaagc ccatgcaaaa 840

acctctaata caatgctggg cggcaattgg ggccatgcgg cgtttcaact tcaggcatgc 900

gattattgtg atgatatatt tgccgagaca gcagacgctg tctttggtga tgcatggttg 960

ccgcagttct ccacatggtg gcaaggtacc aatatcgtcc tggtaagaaa ccctatcatc 1020

gacgacattc tggaatcggg gaaaaatgca ggcgccatca ctctgggcgg tgtcagcatt 1080

gatgatttat gcaagtcgca gttgggtaat ttccgccaca gaaaagatgg tttgattgtc 1140

aggttagacg atgacctgaa gcagggcaag aaggtcccca aaaaacgggt tgatctgtct 1200

gcattaggca agattgacca gaagagaaag aaaattgtca ggctcagaaa ggaaatggcc 1260

gtgaaaagtc acgaatattt cctgcaggcc cgagaaaagc acgatctcag cctttatatg 1320

aagcccatgg ggcagttggc gagaaatatg cttaagctgt atagggatcc atttatgaag 1380

cgactgatca ataggctgaa gcgatatttt taa 1413

<210> 143
<211> 1449
<212> DNA
<213> Methylobacillus glycogenes

<400> 143
ttgagtctat ctgtaaaagc cgcgcgcggt ggtgtggtca tgatgtcagg acaaattgtg 60

aaggtagctt tgcagtttgg cagcctgatt atcttggcac gcctgttgca accagaagat 120

tttggtttgg ttgccatggt gattgcgatt tatggcattt gcgacatctt gctcgacttt 180

ggcctttcga cggcgtctat tcaagtccaa aacataacta aagcgcagat cagcaacctg 240

ttctgggtca atgtattgat aggtctgttg tttacgctga tttcatactt ttcagcaagt 300

ttactggcga gcttttatgc tcagccagaa ttaaccaaaa ttgcgcagta tgtttctgtg 360

gtgtttttat tcaatggtat tgccgcacaa tataaagccc agctgaaccg taatttcaaa 420

ttcaaacaga tggcaataac agatacgact gctgttttgc tcagtgtttg tatcggggtc 480

tatttcgcat atgccggaca ttcttactgg gccatcgtat atcagcagat agcgcaggca 540

ttgatacaaa tgcttttgta catgcttttc ggcgcttggc taccttcctt gccttcaaga 600

catgtttcta tcagacattt cttcaaattc ggttgggggc tggctggttc tcaattgatg 660

ggatacttct cgcgcaatat tccagctatc ttgattggta atcaaatagg tgccagccag 720

ttggggtttt acgatagagc caacaaatta ctgatgttgc cgctaaacca gctgaatgcg 780

ccttcatcgt cggttgctgt ccctgtgttg tcacgtttgg cagtggaaga tcaggaaaaa 840

tacaatcgtt ttcttctgtt tggacaaaat atcattgtcc atctcgttgt attttgcctg 900

gccttaacct gctgccagac agagcgcctg gtgcaattgg tgctcgggac acaatggttg 960

gaaatggtgc cggtctaccg ggcactttca tttgcaggga tatttttggc gctcagctat 1020

gccagttact gggtattttt atctaaaggg attactgcct ctctatttaa actaggcttg 1080

gttagcaggc caatctctat tttgattatt tgcctggggc tgcccgctgg tgtgattggc 1140

gtagcgtatg catactcggc aagtctgctc tttacctggc tactcggcgt tttctggctg 1200

aggaaaacgg gcgttcctgt caaaagcatg attgtagatc cactcatcat gtctgccatt 1260

tactatagcg ccagttttat cgccaattac gtggtcgatc attatgtgga ctttgccata 1320

tacaacattc ttgtcggctg gttgctgatg ttgctgatcc tggcagtatt ttatttgttg 1380

gtccccaagt tcaaacgtag cgtgaaaaag ttatttgaag tgaaaagtta tattcggaac 1440

aaacaatga 1449

<210> 144
<211> 1206
<212> DNA
<213> Methylobacillus glycogenes

<400> 144
atgaagacca ttaaaaaatt actatacaaa atcctaggta gtgcaggcat caaggccaag 60

gctgcagcag tcatcaatct cctgtttggt cgtgtggcta gaagcactaa tgacagtgtc 120

tctattctta ttccggcacc aggagggggg aatatcggtg atcaggcaat gctggaatca 180

tttttgcata atattgatgg caaaaaactc attatcaaaa cacagcctat agcattggat 240

attcctagtc actatgcagt gaacgttgaa gtgatcgaaa tgccaggatt gatctacggt 300

catttgttca ggtttttgcg ggattacgta agactgagaa aaataatggc atctgccagc 360

gctttgtatg tagttggcgc agatatcatg gatggtggct ataataaaaa tgcctctatc 420

atgcgctcta aaattgcaac ttatttcgcg cgtaataata ttccatccag ggtgtttggt 480

ttcagttgga atggcaatcc tgatgcagca gcgctttctg ctatcagaaa cgcagctgcc 540

aatggggttt tgctttgtgc gcgagatcct ctttcattcc agcgtctaca agatgcaggg 600

gttcccaata tcaggctggt tgcagatatg gtgtttgcca atgacaacac caaatcagct 660

gggattgaag atattttgca attctgcgca ggcaagcaga ttgctctggt aaatgccagt 720

ggattgattg gcaaaaatat tgatcaaatt gatgaatata gcaaggtaat aaacaagctg 780

atggaccaca attttaatat tgtactattg cctcacgtga tcagacacgg agccaatgac 840

ttgccgattt gtcaggcatt gtcaggcaag tatccgaatg ctttcctagt aaatagactg 900

ttggaaccag atcaggttaa gcgcttagca caagaggcta aaattgtcgt caccggtcgc 960

atgcatcttt ccatcatgag cctgaatcag aatgttccat ctatcgtatt atcgacccaa 1020

ggcaaggttg atgggcttat gcaattcttc gaaactgaaa aactgtcgat taatccggaa 1080

aaaggttttt ccgatcttat ttgttcacaa attgatttta tttgttctaa tcggcaatat 1140

tatcaagacc tgattcgtaa aaacctactt aaggttaagg aactggccgc tgggaatttt 1200

aattag 1206

<210> 145
<211> 1188
<212> DNA
<213> Methylobacillus glycogenes

<400> 145
atgtctaaat tctataaatt tcaggtgtta gaatcactaa gaggttatat ggcatggtgg 60

gttgttttag gtcatgctat ccaactttct ggtttttggc acaagcaaga agtagatgag 120

ttttttacag gagggggcat aattaatgcc actttttcaa aaatactcag tttaatcatc 180

aatccagaac ttgcagtcaa catttttatt attttaagtg gttttgtgat tactcattta 240

ttgcttacta aaaatgagcc atattcacat ttcataacta gacgattttt ccgcttggcg 300

cccgcatttt acgtttcgtt agttatcgcg attttggtag ttaattttta caaggttgca 360

tataccggtc tgagtttttc tggtgatacc tcgatgcgac tcgccaggct tgttgagcaa 420

gatagtaatt ttttattgca tttattcaat catttactcc tcattcatag cgttttcccg 480

caagagctga ttcaattttc acctaccacg tttcttgctc cggcatggag catcagtctt 540

gagtggcagt tctatctaat tgcaccagtc ttaattccat ttttggtgaa gtctgagaag 600

tctttgattg tttgggttgt gttgttgcta ctgctgcgat attttaccaa ccatgtttta 660

gactttaatt ggcaacatag ctccttcttc tttaaagctg tcgattattt cttggtcggc 720

atcttaagca gattaattat cgagcgactg cagcatggca agtttattta tgatgttgcc 780

gtgttgctag gtggaatatt gcttttccag gatagatatt gcgctgttat ctggctgttt 840

gttttggctg tcatatttta tgaaatgggt tattggagtg ttaaatctcc cctatggaaa 900

aaaatcctga cattattttt ctacaataaa gtcatcacga atattggtaa gtggtcttat 960

tctacttacc taatccatat tcctattttc tcaattttct taggtggtta tgcaactctg 1020

actgaccctg gcactgtaac ccagcaaatt tctttgatta tcctatttgt cagttttcca 1080

gtggttttgc tgatttcaaa tattatgtat cacaacatag agctgccatc gatgaattat 1140

atttccgcaa aactaaaaga aaaacgtatc actgaatctg cagagtga 1188

<210>  146
<211>  1407
<212>  DNA
<213>  Methylobacillus glycogenes

<400>  146
atgaaaattg ttcctgtatt actctccggt ggttctggta cacggttatg gccactttct      60

cgagcggtat tacctaagca actgttgccc ttgatcacta ataaaaccat gctgcaggag     120

acccttctca gaatttcaag atgggcgact gtaacaaatc cagttgttgt ttgcggtaac     180

gatcatcgat tcttagtggc tgaacagttg cgcgaaatcg ctttgagtcc taactctatt     240

attctagagc ctgcccccaa gaatactgca ccagctattg ccgctgcagc attgcagcta     300

caaggtcagg atgtttatat gctggtcttg cctgctgatc atgtcatcag ggatctgcaa     360

agttttgaac atgctgtgca aaccgcattg actgccgctg atcaaggtaa cttagtcacc     420

ttcggtatca aacctgctca tcctgaaacc ggttatggct acatcaaatt aggcgttgag     480

agcgcaccag gatcttataa ggtcaatcgt tttgttgaga agccagatct tgccacagca     540

actgaatatg tggcgtcagg tgagtttttc tggaacagtg gcatgttttt attcaagcca     600

gaaactttcc tcaagcaact ggaaatccat gcccccgaaa ttactgctgc agtaagcaag     660

gccgtcaata acagttataa ggatctggat ttcgtcagat tagaagaatc gagttttgct     720

gagtctccgt ccttatccat cgattatgcg gtaatggaaa aaaccgatca tgctgttgta     780

gtgcctgctg atattggttg gagtgacgtt ggctcatgga cgtctctaca agaggtgcaa     840

gagcaggatg ccaatggcaa tgtagctcgt ggcgatgttt acctcaagaa tgtcgagaac     900

agtctggtgc ggagtgagga taggtttgtt gccgcgattg gtgtccgtga tctgatcgtg     960

gtggagacca gtgatgccgt tctggtagct agcaaggatg ctgcacaaga tgtcaaggag    1020

gtagtggaat acctcaagtt aaatcaacgc tctgagcaca agatacatcc acgggcctac    1080

aggccttggg gttggtatga aagtattgat ctgggcgagc gccaccaggt gaaacgtatc    1140

atggtgaagc cgggcgaaaa gctgtccctg caaatgcacc accatcgtgc cgagcattgg    1200

gttgtagtca gtggttcagc catgattacg gtggatggga ataccaagct attcagtgaa    1260

aatcagtcaa cgtacattcc aattggctcc acccatagat tggagaatcc gggcaagttg    1320

ccgctgcact tgatcgaggt tcagtccgga agttatctgg gcgaagatga cattgtgcgt    1380

tacgaggata cctacggccg gtcctga                                        1407


<210>  147
<211>  333
<212>  DNA
<213>  Methylobacillus glycogenes

<400>  147
atgcttacgg atgaataccg atacaagatt ctgaaattgg tgcaggaaaa accagatatc      60

agccaaagag aactggcgaa agcactaggc attagcttgg ggaaaaccaa tttttgtctc 120

aaagccttag cgcaagttgg cttgctcaaa gtatctaatt ttcgcaacag caagaataag 180

ttggcatata tgtatgtcct cacgccgaca ggcatagaac ataaagctgc tgtgacactc 240

aactttctca aactcaagct caaagagtac gaacaattag caggagagat tgaagagtta 300

cgtagtgaga tcaacgctaa ggtaaatgat taa 333

<210> 148
<211> 1089
<212> DNA
<213> Methylobacillus glycogenes

<400> 148
atgttaaaaa aagtagctct aattacaggt atcactggac aagacggagc atatctagct 60

gaatttttac ttggcaaagg ctatgtagta catggcatta agcgccgagc atcacttttc 120

aatacagatc gcattgatca tctgtatcag gaccctcacg tcgataacca gaatttcatc 180

cttcattatg gggatttgac agattccacc aaccttatac gtatcattca gcaggttcag 240

cccgacgaaa tctataacct cgcggcaatg agccacgtgg cagtcagttt tgaaagtcct 300

gaatatacgg ctaacgcgga tggtcttggg actttgcgta ttctggaagc tatccgcatc 360

ttggggctgg aaaagaaaac ccgcttttac caagcctcga cctcagagct ttatggtttg 420

gtgcaggaaa caccgcagaa agaaagcacc ccattttatc cacgcagccc gtatgcggtt 480

gcgaagcttt atgcatattg gattactgtc aattaccgcg aagcttatgg catctacgcc 540

tgtaatggta ttttattcaa tcacgagagc cccttgcgtg gggagacctt tgttacccgt 600

aaaattaccc gtgccttggc gcgcatcaag ctgggcttgc aagattgcct ttatttggga 660

aatctggacg cgttgcgtga ttggggtcac gccaaggatt atgttgaaat gcaatggctg 720

atgttgcaac aggaacaggc agaggatttc gtcattgcga caggtgtgca atacagtgtg 780

cgcgatttcg tcaatgcagc cgcccacgag ctggatattg ccatcacctg gaaaggtcag 840

ggccaagaag agatgggata cgacggtcaa ggcaggtgca ttgtacaggt tgacccgcgc 900

tattttaggc ctaccgaagt tgaaacattg cttggtgacc cgagcaaggc aaagcaaaaa 960

cttggctggt cgcccagcat cagtttccag gagttggttt ctgaaatggt gcgtgaagat 1020

ctcaagagcg cagagcgtga cgaactggtt aagcagcatg gttttgctgc ctatgactat 1080

aacgagtaa 1089

<210> 149
<211> 974
<212> DNA
<213> Methylobacillus glycogenes

<400> 149

atggacaaac acgcacgtat ctatgtggca gggcatcgag gcctagtggg ttccgctatc 60

gttagggggc tagagggcaa gggctatagc aatcttatca agcgtaccca tgcagagctt 120

gacctcacag atgctaatgc cacggaagca ttctttgctc agtatcagcc gaatatgttt 180

tcctggcggc agccaaagtg ggtggcatag tggccaataa cacttacccg gcagatttta 240

tccgtgacaa tctcgcaatt cagaacagtg tgatccatag tgcctggaag catggagtaa 300

agcgcctatt gtttttaggg tccagctgca tataccccaa gcatgctcct cagcccatgc 360

atgagcacca ctttctaacc ggatcgcttg aagctaccaa tcgtgcatac gcgctggcaa 420

agattgctgg agtcgaaatg tgctggagtt ataaccggca gtatggcact cgatttcttg 480

cagcaatgcc gacaaatctt tatgggcctg gcgataacta tcacttgcag aatagtcacg 540

ttatccctgc attgatacgc aaattccacg aggctaaaca agctgggcat gcagctgtat 600

cggtctgggg tactggaaca cccaggcgcg aatttcttta cagtgaggat atggcagatg 660

cctgtatatt cttgctgaat ctgcctgagg agaaattcaa ttctctgctg ggaagtgatg 720

agagtgctac tggtgtgttt gatccaccac tagtcaatat cggtattgga gtcgatattt 780

caatacagga attggccgaa ttggtaaaaa aggtggttgg ttaccaagga gcaattgagt 840

tcgatagttc aaaacctgat ggcacgatgc gtaagcttat ggatgtaagc aagctcaaca 900

gtttggggtg gacttattcc attgagcttg atgatggatt ggaaaagaca tattcggaat 960

ttttaacaaa ataa 974

<210> 150
<211> 696
<212> DNA
<213> Methylobacillus glycogenes

<400> 150
ttgaaaaaaa aagtgtttgc tttgattcct gccagagcag gttctaaagg agtgccaaaa 60

aaaaacctga aaaatatcgc aggttctcca ttaatagctt acacaattaa cgctgctaaa 120

aaagctaaaa atgtcgatgc tgtttatgta tcttctgatt gtaaagaaat attggagtat 180

tcaaaagcac aaggctgcag ttcaatatta aggccgcaag aatacgcaac agatgatgcg 240

tcagctgttg atgttgtcaa gcattttatt agtattgaaa atatagagga tggagattta 300

atcgtttact tacagccaac ctccccatta agagatgaac atcatattga tgatgctttg 360

acggctttga ataataatgg ccttacttct ttggtcagtg tggaagtctt ggataagtcg 420

ccatataagt catttcgtct agatgataat gataaattga tttcattatt tgatgaaaaa 480

ttatctaatt atcgtcgtca agatttgcca ttatgttacg cgccaaatgg tgctatttat 540

atcttttcga aatttgaatt tgaggctaga ggcggtttcc cctccaatgg tagtgttgct 600

tatattatgt ctgctgaaga aagtgttgat attgacaccg aagaagattt tttagaaaca 660

gaagcattat taaggaaaaa atatggcatc ttttga 696

<210> 151
<211> 1041
<212> DNA
<213> Methylobacillus glycogenes

<400> 151
atggcatctt ttgaaatttc tgggcggaaa attggggacg gacatgagcc tatagttatt 60

gcagaaattg gcatcaatca cgagggttca ttagatcttg cgattgatat ggcaaaagct 120

gcaattgatg ctggggcaga gataataaaa catcagacac atgtggttga agatgaaatg 180

tctgatgaag ccaagagtgt tattcctgga aatgcggatg tttcaatcta tgagattatg 240

caacgttgct cattgtcaga ggatgatgaa tataagctca tgcaatatgt gcagcagcaa 300

ggcagtattt ttattagtac gccattctcg agaatggcag caaatcgact gagaaaattt 360

gatataccgg cttataaaat tggatctggc gagtgcaata attatcctct cgtgcggcat 420

attgcttcat ttgggaaacc tattattgtt agtaccggta tgaatagcat tgaaagcatt 480

gcaccaaccg tcaatatatt aaggcaagca aaagttcctt ttgctcttct gcactgcacg 540

aatatttacc ctacccctcc tgagcttgta agacttgatg ctatccaagt cattaaaaat 600

gcatatcctg atgcggttgt gggactttca gatcacaccc ttgggaatta cgcttgccta 660

ggggctgttg cactaggagc ctcaattctt gagcggcatt ttactgactc catgtcacgg 720

ccgggaccag acattgtttg ttcaatggac cctgcagcat tgcaagattt aatcactggt 780

tcaaaagtta ttttttcagc aaaaggtgca cataaaggtc cacttttaga agaagaaaaa 840

actatagctt ttgcttttgc atctgtcgtt gcaattaagg atatcaatgt tggtgatgtc 900

ttaagtgaag ataatatttg gttacgtagg ccaggtggcg gtgattttaa tgcttctgac 960

tatgatcagc tacttggtcg taaagcatcg caaactatac tgagtggcaa aagagttaat 1020

cggcaggata ttgaggcatg a 1041

<210> 152
<211> 1132
<212> DNA
<213> Methylobacillus glycogenes

<400> 152
atgagtaggc gagttgtttt tcttactgga acaagagcag attttggaaa actgaaatcc 60

ctcatgcgag cattacaaaa tgacatggga tttgacgtcc atgtgttcat tactggcatg 120

catatgttgt cgaaatatgg ttatacatgt gaagaagtag aaaaagccgg atttaaaaac 180

ctatttcgct ttatcaatca aaatcaaagc gatagcatgg atcatattct ggccaaaaca 240

attactggtt tatcagatta tgtaaaagaa atttcaccag aactgattgt gattcatggg 300

gatagagttg aagcgttggc cggagccgct gttggcgcat taaataacat tcgagtggca 360

catattgagg gcggagaggt ctctggcact gttgacgagt tgattagaca tgcggtgacc 420

aagctgtccc atgggcattt tgtttccaac gaacaggcaa aaaatcgact tattcagctt 480

ggagaaagtg aagaatccat ttttgttatt ggctccccag atattgatgt tatgaattca 540

acatcgctgc caagcattga tgatgtgagg gaacgttatg gcttctcgtt cgaacattac 600

ggcattttga tgtatcaccc cgttacctct gagttagatg atttgccaag acaagccaaa 660

gccatcgtag attatatttc agaaagctct ctaccattca ttgttgtata tcccaataat 720

gatcatggtt cagacattat tttgaatgag tatttgcgat tgaaaaactt gagccacgtt 780

agagtttatc catccattag atttgaatat tttttaacgc tgctaaaaaa atgcagcgtt 840

catcatgggt aactctagcg ctggagtaag agaggctcct catttcggcg tgccagccat 900

caatgttggt agcaggcaac acaatagagc aacagcagag tcgattctta atactactgc 960

acatgtggat tcccttagac atgctgttca acaagttgca gacattcctc gcgtggcagt 1020

ttctgacttt ggtaatggta atagtgcagg tgagtttcat cagattctgt tgaataatgc 1080

tttttgggat aagggcatac aaaagcactt tatcgatagg agtgcagtgt ga 1132

<210> 153
<211> 738
<212> DNA
<213> Methylobacillus glycogenes

<400> 153
gtgaggttga tcgcaaggct agatgtcaaa aatgaacatg tgattaaagg catacaccta 60

gagggcttgc ggaaagttgg cgatccaatc gagatggcga aaaactatta tgcacaaggc 120

attgatgaga ttgtgtttat agatgcggta gcgagcttgt ataaccgtaa taacctgctg 180

tctgtaatcg ccaaggcttc ggaagaagtt tttgttccta tcactgtagg gggggggctg 240

cgttccttag cagatgtcga gatggctctg agtgccggag cagacaaagt ggcagtgaat 300

acagctgcaa tacttaaccc gaaattcatc acagagattg ccaagcgcta tggcagtcaa 360

tgtgtggttg cctctattca agccaaacgg ataggcaatg actgggaagc ttatatggaa 420

gccgggaggg agcctacggg caaaaatgtt gttgcttgga gcaaggagct ggaagatctt 480

ggtgcaggcg agattttgct aacgtctata gaccaagatg gtacaaaaaa aggatttgac 540

cttccattgc ttggtgcagt taacacgata gtgaacattc cagtcatggt tagtggtggc 600

tgcggtaatc ctgatcatgt gcaagagtta aaagaaatcc agccaagtgg agtttgtctg 660

gcgtcagtat tgcattacaa aatcaatacg atagatgaat tcaaacagcg tctgacattc 720

atcaatggta ctaaataa 738

<210> 154
<211> 639

<212>  DNA
<213>  Methylobacillus glycogenes

<400>  154
atgaagaaaa tatgtattct tgattatggg ctaggcaatg tgcgtagtgt gagtaatgcg      60

atcaagtacg taggtgctca tcctgtaatc tcaaatacgc cagaggcact taaagattct     120

cacgcgttaa tagtgccagg ggttggtgcg tttgctcaag gcatgtctgc attgggtaaa     180

gatggattac aagctagctt gctcgaatat ataaaaagtg gaaaacatgt cttggggatt     240

tgtttgggca tgcaattgct tttcgacagg ggtgaggagt ttggcgttac cactggtctt     300

ggacttgtgc ctggcgtagt ctccagaatt gtacccgtag agcaatcacg agtgaggtta     360

ccgcatatat cctgggcgaa agttgatctc aattccgttc atgaaaataa tggcggcatg     420

tttgatggac tgccagacga gcagcgtaag ttctattttc tgcacagtta tgccgctcaa     480

aatgtacctg atcgatacat cacaggcagc gcgacgtatt ctggatgtac ttttgttgca     540

tcagtgcaga aagacaatat ctggggtatg caatttcacc cagaaaaaag tgggcccagc      600

gggttacagg ttcttaataa ctttgtacaa aacagttaa                            639


<210>  155
<211>  1227
<212>  DNA
<213>  Methylobacillus glycogenes

<400>  155
ttgaaaagaa gtgtggagga agttgtggtt aatgcatatt ttggtttgcc ggaagatgta      60

gtgttttgca agaaatgcgt gatttccaac caaagaccca gttctacagt tgaattcaag     120

cataccaaca aggacaaaaa agcgaccata ggatttggcg ctgatggtat ttgtgatgct     180

tgccgctatg cagaaaccaa ggatgctcaa atagactggc aggcccgcga ggacgctttg     240

gttagattgt tggatcaaca tcgccgtagc gatggtggat atgacattgt cgtgcccggc     300

agtggtggta aagacagtgc atatacctca catatcctta aatataaata tggtatgaat     360

cctttaacag tgacgtgggc gccgcataag tacactgaga ttggttggaa gaattttgaa     420

aactggatcc ataaaggtgg attagacaat attcttttta ccccaaatgg caagttgcac     480

cgctatttaa caaaaatggc atttctgaat ttattgcacc cattccaacc attcattgtt     540

ggccaacgca ttatcgggcc gttgatggct aaaaagtttg gtatcaaatt ggtaatgtat     600

ggtgaaaatc aagctgaata tggcaataag acagaagaaa actataaacc gacaatggac     660

aagaattttt tctctatttc agacccagct gacatggtgt taggtggtgt gaaggttgga     720

gacattatta aagacggtga atacagctta aatgatttcg cgccatatat tccgcctgct     780

gcagaagatc tagagcgcga tggtatcgag gtacattatt tgggctatta cctgaaatgg     840

gatccacaag agtgctatta ctatgcagtc gagcatactg gtttccaggc aaatacggaa     900

cgcactgaag gaacctactc caagtacagt agtatcgatg atcgaataga tatgttccac 960

tactttacta ccttgatcaa attcggcatt ggtagggcaa catatgatgc tgcgcaagaa 1020

attcgcaacg ataaaattac gcgagaagaa ggggttgctc tagttcataa atatgatcaa 1080

gagtttccaa aaaagtactt tgcagaattc cttgaatata tagagatcac cgaagcacag 1140

ttttgggaaa ccgtagatag gtttagatct ccacatttgt gggaaaagca agacagtaat 1200

tggatactga agcatcaggt gacctaa 1227

<210> 156
<211> 1194
<212> DNA
<213> Methylobacillus glycogenes

<400> 156
ttggatactg aagcatcagg tgacctaaga atgaaagtgt tgcagatagg aagtttctcc 60

ggaaatgtcg gtgacattgc caaccattat ggttttcgca attggtttca aggactgctt 120

ggagagattc aatgggatga attcgaaata cgcaatgttt accgaaaaaa tgttgccttt 180

gacgataatt ttgtagctat tgcaaatcag tacgatctta tagtcgtggg aggtggtaat 240

tttttcgagc tatgggttga ttcttctcca accggcacct caatctcaat tgagattgag 300

gtttttaaaa aaataaaagt ccccatcttt ttcaatgcat tgggtgttga tgatggtatg 360

ggatgtagtg atgaaagtct tgcaaagttt actaatttct tagaaatctt aacttcatca 420

aagcaatatt tggtcagtgt tcgtaatgat ggagcaatgg gcacgctcag aaatcggatt 480

tctaataatc ccatgttgga taaagtctta ccattgcctg acggtgggtt ttttgtaaat 540

ctggataagt tagaaaaaga cattgcggaa aatgatcatg aaaacactca gatcaccatc 600

ggcatcaatc tggctggaga tatgttggaa acaaggtttc caggtggaat taatggatat 660

gattactcag gatttatatc cgagttcacc gagactttgg ctgaaatgtg gaataaaaat 720

aaattaataa agtttatttt ttttccacat atttatagtg atcttaaagt atatgccgac 780

atactatcac ggttgcctga tgcatgtaga cgtgaaaatg tcaaagtggc cagttacgat 840

actaatgtga attttcaagg gacattattt tccgaatata gcaaatgtac tttgatactt 900

ggtatgcgat ttcatgccaa tgttgtagcc atagctaata aaattccagt aataggattg 960

tactcataca agcaaattaa agaattgttt tatgagttgg gaataaaaga aaaaattgtt 1020

gatgtcagac aaataggttt taaaaatgaa ttgttgagaa agtgcaatat tgttattgaa 1080

aattcatcta tatctcgtga tgagagctgt aacatacttg atgttgtcac aaaaaaacgt 1140

gatttgattg ctccaagggt agtggagtgg ttacgtgagc ataagcttaa atga 1194

<210> 157
<211> 1068
<212> DNA

<213>  Methylobacillus glycogenes

<400>  157
atgattaaaa gccttgatta tctaagggcg attgcagcac taacggtttt tctcagccac      60

acctttcaaa tttattggtt accaaccatt ggattggtgt cctggaaata tgagttgaat     120

cattttttga gtgaaaccgc ggtcctctta ttttttgtat taagtggcta tgtaatttcg     180

aatagtttgc atagcaatac tcaacgtaat ggagggcgtc tttcagtact tgattacgca     240

tctagcagat tattgcgcat atatcctcct ctcattgtaa gcataataat atctttagtt     300

gcctatagtg tgattgtgat ctttgaacta ccgggatcag tgggtacttt aaaggcacca     360

ggagatatct atcaggcaag agactacttg agcattggta cgcgagacat tgtcaattct     420

ttattgatga ataatggctt gttgcaaatg aatgggccat tatggagttt atgcattgag     480

gctaagctat atttttatgc ttgtttagtt gtaataattt ttattcatta taaacaaaaa     540

aatttgctca attttattgc taatactggt tttctcgtat tatctgtatt cttgtttttt     600

aatacaaaaa ccgatattta ttatggctta tggtggttga gcggtggtgt aattttctta     660

tatcgtcgaa atttcataaa gttcatgact ttggttgttg taatggctat tgtggtgttt     720

gctatggcag tgattggaca gaagttcgtt tctttgccag tgcaagttat atttgtatat     780

tctttatttt tggcagcaat aaaatgcaaa gtgccagaaa ttaaggcaat gaatattttg     840

gccaatactt cgtacacttt atatattgtc catttcccta tgttgttact atcgtacgcc     900

atcttctcat ataactttga tgtgtacggt agcctgacta gagagttgat gtcatcatta     960

tcgctaataa tcatcttaac tatgtcaatt tatattggaa aatgggtaga gcgtccagct    1020

aatttacgtt tgacctatca caggttgttg agaaaagtaa aagtatga                 1068

<210>  158
<211>  1680
<212>  DNA
<213>  Methylobacillus glycogenes

<400>  158
atgaaagcca tgttttattc tttgcatgca gctatttggt tacatgcgct tcctgaaagc      60

cggttggcat cacagctgag taatgccggc catgagttag tatatgtgac ctgtgataaa     120

agctttactc attattgcac tggtatgagt gctttcaatc ttgcattgaa tgaggatttt     180

aaaaagaaaa gtgcaatttg tgagttatgt attaataacg ccaataaaat aaccaagtct     240

tttgattgca agcatcttaa attgcatgat tatattgatc agaaaactaa tgctctgatt     300

gataacctat tgaaaagtgt cacggaagat aatttcctcg aatttcatta tgaagatata     360

ccagtagggc gtttggctac atatgagcca cttcttaagt ataagaaaat gtctttggaa     420

tttagagatg aacaatggca agatctacag attcacctta ggaatgccct ggtatcgtta     480

atcggtttta tccaaatatt caaacaagag tgtccagaaa aaattttctt ttatagtcca     540

cagtatggtg ttaatggtgt gtgtgcagaa tatgcagtaa aacaggggag acaggcatat 600

tttatagaag gtagctctag caatgctgaa cgatatagtg ctttaagggt gtgggagtgg 660

agtaaattta aattagtaaa ccctgcattg cagcattggc aagctgttaa aaatttagta 720

acgcttgaag atattgatag agtctctgca catattcacg aattattcaa aggtaaatct 780

tttgctgttt tttcagcgcc aaaaacagga aatttttcaa tccgtgaaaa attcaatatt 840

ccacagaatg ctaaaattct actcgcctca ttaagtagtt ttgatgaagc gtatgcggct 900

tttgttatca aaggttttcc tgagcgtaaa gttaaaagtc cggttttctc agatcagttt 960

gaatggatta agtgtacaat taattttatg aagtctaggc ctgatgacta tcttgtaatt 1020

agagtacatc cgcgagacta tcctaataaa cgcgattcca atcagtctga gcaagcattg 1080

atttgggaaa aaatacttca ggatctccct gagaatgtta aattaaactc accagatcag 1140

aatatttctt tatacgattt tctcgatgat gtacatgcgc tggtaacagg gtggtctgcc 1200

actggtgtag aagcattgct acatggcata cctgtgatta cctatgataa atacttacct 1260

tcctatccgg cggatataca tctaacggga gagtctgaac atgagtatta tgacaacctt 1320

atcaaagtac tttctttggc aacatcaaca gataatacac atgtagttaa tgcctatcgc 1380

tggctcgcgg tcagtttttc actgggcact gtcagagtta atcagccact ggagattggt 1440

ctgagttggc cggataagcc ggcgtataga atattgaaaa gggtggctaa caagttttca 1500

gttaatttgc gaaaaagcta cgacgtatgg aatgtccagt ccaacgacac agaccgttta 1560

agatttttaa gtctggttct taataatcaa tctagccttt ttgagggtgt aatatctgat 1620

gtggtcgatg ctgtttccga ttatgaaatt gaagctacgg taaaacaaca ttcgatataa 1680

<210> 159
<211> 1707
<212> DNA
<213> Methylobacillus glycogenes

<400> 159
ttgaataaat tgccacaagc aaaatatatt tttaagttat taaaatttgc tttcaataac 60

aacccattgt tgtatttgag tgttttaatt tctatacttt ctataggtat tgaattgttg 120

gcaatggcaa gcctattgcc tttatttgaa cttgtttcag gagaaacagt agataagcat 180

ggtttgattt caaaagctct gattatattg gggtttacag ttgatgctgc cagccttttg 240

caagttttct tcttattgtt cactttgaga gtagtcacgc aactatttag tcaaagcctg 300

acgcactacc ttggaagacg tgtgatggct cagctcggtt ctagggcttt tgaaaaaatc 360

attacttcga tgtcaattga taaaatatct gaaaaaagta taggtttttt tatggggctt 420

gctggagatg aatccttcag ggcgagtact ctggttgttg cattgactca gtttgttgca 480

acatttttac tgggggtaat gtattttatt tctattgcaa tttattcact atatgccgcc 540

gaaatgattt tcattttaat gcttgtgtct gccgtattct tcatttggat tttaaaaata 600

tctcataaga tgggcgtgag gcaaatagaa ggttcaagga aagcaaattc cattttcatg 660

gatgcgatga acaatatcaa agcagtaaga tcactttctt ccgagaagta tgttgccggt 720

ttgtataggc gaaagatttt tacgtatgca aatattcttt tttaccttga tgcaataaat 780

ctggctacga agttagtacc aataatattt ctattactcg gatctttggc tttgatattg 840

agtggctggg tttctgttga aaacttaggg atggcttaca ttgtgtcatt gatagcttat 900

ctcatgcgat ttttcccagt tgttggtcag agtatcactc tagctgttcg cattgttgca 960

gatgcgaaat cgggaaaaga tgtgacggaa ttagtttccg atgaaagtgc cgagagcgct 1020

aattcagaca actatttaca caagatagat acaatcaaga ttgataattt gtcgttttca 1080

taccgtgatg ttcaaggcgt aaggccagcg gttctcaatt ctgtttacta cgagtttgaa 1140

aagaacaact cttatgcttt agtggggaaa tctggtttag gaaagtctac tttgctggat 1200

gttttgctga agttttatcc tcctgcttca ggcagtattt caatagatgg tatttcaaca 1260

gaaaatatcc ctggagcaca tatcaggcat tctattgtac ttattaatca agaacctgcg 1320

atttttgatg atacggtcta caacaatatt tgtatgggcg agacttttac tctagaggac 1380

gtaaaatggg cttgcgaatt agcttgtatt tctaaagatg tggaaagtct tgcgcaaggt 1440

tatgaaacac gtttgcaata tcaaggaaaa aatctttcag gtgggcagcg tcagcgtata 1500

gctattgcca gagcattagt gcgcaaacct aatgtgctga tacttgatga aagtacaaca 1560

gctttagata aggctacaca agaaattgtg atacggaatc ttatgaatga gtatgaaaat 1620

aagatcatta tatttgtcag tcatgatcct tatatcatgt ctcaagtcga tcatacagtt 1680

gatttggcat taatcaataa agtataa 1707

<210> 160
<211> 699
<212> DNA
<213> Methylobacillus glycogenes

<400> 160
atgtctatct caagtattgc tttaaaaata tttaattatt cgcaagagat tttttcattt 60

taccgggata gaggcttata taaattctct tatgcgcaag agggggaaga tttaattctg 120

agtagaattt ttgaccgaac ctctactggg tattatattg atataggtgc tcatcaccca 180

gtaaggttct ccaatactta tttattttat aaacgtggtt ggaggggagt ttgtatagat 240

ccctcacacg aggctattgc tttgtttaaa acacggcgcc cagcagatat tgccttggaa 300

atgggggtat ctgatcatga gtctgagctg acttatcata tgttcgatga gcctgctctc 360

aataccttca gtgaggaatt ggccaatcag cgtatacaaa acactgctta taaacgcgtc 420

ggtacccgca aggtgccagt aagacccttg actaaaattt tggatgaagt gctgctgccg 480

gatactgaaa ttgatttcat gagtatagat gttgaaggct ttgattacca agtgcttgcc 540

tctaacgact ggtcaaagta caggcctcgt atagttctgg cagaagcact tgatgatgtc 600

gctggcgaac gcatacgcag tttcatgatt caaaatcatt acaaagtcat agcgcgtaca 660

ttcaacactg agttttatca agatcttttg aaactgtaa 699

<210> 161
<211> 906
<212> DNA
<213> Methylobacillus glycogenes

<400> 161
atgtccaatc tagcacctgt tgccctgttt acttataacc gccctgacca tactaaaagg 60

acgattgaag cactgaagaa taataccttg tctgctgaaa cggacttgct gattttttct 120

gatgctgcaa aaaatattac acagcaaagc atggtggatg ctgtcagagc gtttactcac 180

actgttgaag gcttcaagtc agtaagaatc atagaacgtc aaacgaattt tgggctagct 240

gcttcgatta ttgatggtgt aacaaatgta cttgcagagt atggtcgcat aatagttctt 300

gaagacgaca tggtcaccag tccatatttt cttcaataca tgaatgatag cttgagtcag 360

tatgctgatt ttcatgaagt tgcaagtatt catgcctatg tttatcccat cgcaggattg 420

cccgatactt tctttttgcg tggtgcagat tgctggggct gggctacatg gagggataga 480

tgggattggt ttgaaccaga tggaagtaag ttgtttgcgc aactgaagca gcttaacttg 540

ctgaataagt ttgattataa cggtagttat ccattttcaa atatgttgcg cgaccagatt 600

gcaggaacca ataattcttg ggcaatacgt tggcatgcta gtgctttctt gcttaataag 660

ttgacgcttt atcctggtaa agctttgctt agtaatattg gacatgatgg atccggtgtg 720

cattgtggag aaagcgaaac atactctgcg gagttgagag ctgaccgatt actattgact 780

gatattccat tgcgcgagtc tgagccggca tttaagaaat ttgagcaatt ttttcttgaa 840

agcaaaccat cagttgttcg caaaatgatg ctgaaggcaa aatccgtatt taaaaaattt 900

ttatga 906

<210> 162
<211> 816
<212> DNA
<213> Methylobacillus glycogenes

<400> 162
atgagtttta tcaagatcct aaaattatta tgccccccac tttttctgca tattctgcag 60

aagtataggc ggcgtaactt tgcctcttcg ttcgccggaa attattcttc ttgggcgcat 120

gctaaacaaa atagtggtgg ctatgatgcg gaaaatattc tcaccatcat tcaagagtca 180

gctctcaaag tcaagcgtgg tgaagcggtt tttgaacgag attcagtatg tttccatcat 240

gttgagtata gatattccgc tttgtctgga ttgctatacg cagctgttag ttctggtggc 300

aagttgagtg tcctggattt tggaggctct ctggcaagtt tttattctca gcatgctgca 360

atattgtcta tagttaagga tttgcgttgg ggagttgttg agcagccaca ttttgttgcc 420

tggggtcgcg agcacctaac cacaactgat cttaaatttt acaatagcct cacagagttt 480

attttggatg gaaaacccaa tgttattttt ctttccagcg tcctgcaata tttggagcat 540

ccataccagc tgttagaaga attattgagg ctagattctg agtttattct tttcgatcgc 600

acggcttttt atgacgttgt tgaccgctta actgtccaga cagtgcctgc atcgatttat 660

ccggcaagtt accctgcctg gttcatttct tgggacaaat ttataaatac tatgagtgca 720

ggagggtatg aggtcatcat ggagtttcct tgtgcagacc tgacggacat aggctattac 780

aaagggcttt ttttcaagcg caagagtgta agttga 816

<210> 163
<211> 999
<212> DNA
<213> Methylobacillus glycogenes

<400> 163
atgaatgatg aactgtactc aggtaagaat attcttgtta ctggtggctt gggttttatt 60

ggttcaaact tggtcaaaag attaattgcg cacaaagcca aggtgaccgt tgtagatagc 120

ctgttacctc tttatggtgg caatgccttt aaccttagtg gctatgaatc gctggtcaat 180

atcaacatca gcgatgtacg tgatagtcat gccatggctt atcttatcaa aggcaaggat 240

taccttttta accttgctgg acaaactagc catttggatt ccatgaccga ccccaggacg 300

gatctggata tcaatgccgc agcacaatta tccattctgg aagcatgccg acagcataat 360

cccgatgtca gaatagtctt cgctagcacc agacagcttt atggcaagcc tgagtattta 420

cctgtagatg agtcacaccc gattcagcca gtggatgtga atggcatcaa taaattggca 480

ggggagtggt atcacattct ctataacaat gtttacggta tcaaagcctg tgcgctacgt 540

ctgacaaata catatggtcc tggcatgcgc gtcaaggatg cccgtcagac ttttcttggg 600

atttggttgc gtagagtgat tgaaggcaag ccaattgcgg tatttggaga tggcttgcaa 660

ttgagggatt tcaattacgt tgatgatgtg gtggatgctc tgttactggc tgccgccaat 720

acagaagcag atgggcaaat atttaatctc ggcagtgatg aggttgtaaa tttacagcaa 780

gtagctgaat taatgacaca atttcagcct ggttccagct atgagattgt gccattccca 840

cctgagagaa aagccattga tatcggtgac tactatagtg atttcaataa aatcaagcag 900

gttttaggtt ggagcccccg tgtgggcttg caacaagggc ttgctgcaag cctggcatac 960

tatcgccagc atcatgataa atattgggac aacgtatga 999

<210> 164

<211>  1101
<212>  DNA
<213>  Methylobacillus glycogenes

<400>  164
atgattttaa tgaatgactt caaggctgaa ccagaagctt tgattgcagc gcagctgcaa      60

gccgtcgagc gtgtgctgcg ctccggttgg tatgtattag gtgaggaggg gcgcaagttt     120

gagtcattgt ggtctcagcg ttgcggcact tctcatacag tgggtgttgg caatggcatg     180

gatgcgattg agataggatt gcgcgcgctg gacattgggc ccggtgatga ggtgattacc     240

actgcgatga cggcttttgc ctctgtgctg gcgatcatac gtgcaggtgc tacgccggtc     300

ttggccgata ttgatcctga cactggctta ttgagtcatg aaagtgtcaa gcgatgcatc     360

aactcgcgaa cgaaagcggt cttgctagtc catctatacg ggcaaatcag ggatatgcaa     420

tcctggctga acttgtgcga tcagcatcaa gtggctttgt tggaagattg tgctcaagca     480

catttggcgc aagcggaagg taaagtggcg gggagttttg gtcgctttgg tgcttacagc     540

ttttatccga ccaaaaacct gggcgccatt ggcgatggag gggctttggt aacatctcaa     600

tctgcgctgg cagacaaggc tgcagtgtta agaaattatg gccaaagtgt gcgttaccat     660

catccagagt ttggcttgaa tagtcgtctt gacgaaattc aggcggcctt gctcacggta     720

aggctagagt ggctggaaca atatacaagt cataggcaat caattgccgc tcaatacagg     780

cagcagttga ataatccata cgtgcgcctc atgcaaactc caacagagcc attagcccat     840

gtctatcatt tgtttgttgt gctaacggag caccgtgaag cattgcaggc tcacttgcag     900

gcaaatggcg ttcaggcatt gattcattat cctgtgccta ttcatcatca ggaaaagtgc     960

agcgatatat tgagagatcc ggctgggctg caatttagtg aggcccatgc acagcaatgc    1020

ctttcgctgc catgtcatcc tcaaatgtct gaacaagatg ttcagaccgt cattgctgca    1080

gtcaataggt ttgcactgta a                                              1101


<210>  165
<211>  942
<212>  DNA
<213>  Methylobacillus glycogenes

<400>  165
atggagcatt ttgtcacact gtttgatagc aagtttctgc ctcaaggttt gtgcctgcat      60

gagtcgctgg cccgccatgc cagggatttt catttatgga tagtctgtat ggataaagtg     120

gtgcatgagc aactggaaaa attgcagttg cctcatgttt cgcttatgcg cttggccgat     180

tttgaaagcg aggcattatt ggccgttaaa ccaggcagaa caaaggggga gtattgctgg     240

accataaccc cattcactgc cgaatgggtg tttgctcaac acccagaggt ggcacgagtc     300

acttatcttg atgcagattt attttttttc aacgatcctg cgatactttt tcatgagttt     360

gaacaaagcg gcaagcatgt gatgattact gagcatgcat atgcacccga ttacattcag     420

catctggaaa cgagtggacg gttttgtgtg caattcatgg tatttgatcg aagtgaagca 480

tcccgtaaag ttatgcattg gtggcaagat cgctgcgtgg agtggtgttt tgcaaggatg 540

gaggatggaa agtttggtga tcagaaatat ttagatttat ggcctacaat ttttgccgat 600

gatgtccaca tattgaagtg cgttgaacaa acgttggcac cttggaatgt agactatcaa 660

cttcagcacc aaagtgggaa attcaagccg gttttttacc actttcatgc attccgcata 720

atcaagttta atcttgtcag actttatgaa gggtaccaga tagctaaaag ttctcgttac 780

ctctatgacg agtatcttgc gactttgcag aaaaaaattg ggatgttgca tcggtttgcc 840

attccgttgc cattgttgcc agaaaaatgt ggtattaggc caaaattggc ttcttattac 900

acatggatca gaggatttac cgctacagct aagatcaaat aa 942

<210> 166
<211> 768
<212> DNA
<213> Methylobacillus glycogenes

<400> 166
atgcaaaaac ttagtgtgat tacggcaaca tataatgccg ctaaaaattt accagattta 60

gtgcaaagcc tgcttagtca aactgatatg gatttcgagt gggtcgttgc agatggcggt 120

tcaagtgacg aaactatatc ttatctaaaa agcgttgtag gcattaatct tttgctgtca 180

gcgcaacagg attttggtat atacgatgca ttaaatcgtg cgatttcgct aagctcaggc 240

gattattatt tggtcttggg tgccgatgat attttgtatc cacatgctgt cgaggcgttt 300

aagcgtgcgg ctggcatagg tctgcctgat ctcgtcaccg caagtgtaga gtcagatcat 360

ggtgtcatga ttgctggaag tggcaggcca tttttgcgcg gacaattggc ttatatttcc 420

aatcattctg tcggtactat gatcaaaaaa agtttgcatg atcgttttgg ttattactcc 480

cgccgattcc ctatcacggc agatcagtat tttatcaagc gtgtttgtgg cgctagcgaa 540

acggtgattt acaaagcaga ttttgtagca ggctattatc cagctacagg cgtgtcagga 600

gcagatgtcc ctgggacgat ggttgaattt ttccgcgtgc aattggaaac cgaacctaat 660

tcagtgctcc agctagtact tttcattttg cgtttgatac gccatacccc caagctcatc 720

cggtatgcac actacaaaaa attgcgcgat ctaccccagc atgaatag 768

<210> 167
<211> 1083
<212> DNA
<213> Methylobacillus glycogenes

<400> 167
atgaatagca acgtattgtt tatcactggc tttcatcctg gaggtatggg ttttattggt 60

gcaggtgagg caatcagtca gcaacatata gagcgactga taaagcaagg aaggaaagtg 120

catgtgctgg cacttgcccc taattatcaa gtcgccagtc ctgaggttgt tgcaagctgt 180

gcttcctaca ccacgcttac gcatacgcga tggatgacgt ttgcagcttt atggcgctca 240

cgccagcagg gaagtctgct ctgcccctgg ttatttacgc gtgtatcacc gcaagctgaa 300

cgttttatcc gtcagttgtt ggaccaacac gccattactg agatctatct tgattttcca 360

tcttgtcttg gcttcgcgcc aacatacaat caattaccca ttcattacat tgttcatgat 420

gtgttgtcgc aaaaagttga gcgacgtcct ttattaaaat ggctgaaaaa tcaagtgttg 480

cgcacggaat cgtccctgct gcgtcatgtc acccagtgtt tcacactttc cctgaaggac 540

aagctgagat gcgaggaaat agggtatagc ggccagatag aggtgcaggt gccgcacatt 600

ctcaaggtag gggttgttaa tcaaggagtg ccaattgcag ggatcctcaa gcaatttcct 660

ggcaagaaaa acctggtttt ttttggcaac atgcagcggg ctgaaaatca ttactccatc 720

ttaagatttt tgttgtgcag ttatcctggc atctggttgg agcatcgcga tgttcagtgc 780

tggatattag gtttggcacc aagaacgcta ttgcgttgga tatcgaaagt gttgcctggt 840

gttagagtca ctggcgctgt tgatgaccca actgaggcgt ttaaggcggc aactttatgt 900

attgcacctg tatattacgg tgctggtgtg aaaatcaaag ttttacaaat gctggaatcg 960

ggagccacag ttatatcgac gccagtcgga gctgagggta ttcacggcag tgaaaaactc 1020

attacggtgg aaagtgctcg tctatcctcc actatcaagc aacgtttggc agttgaatca 1080

tga 1083

<210> 168
<211> 924
<212> DNA
<213> Methylobacillus glycogenes

<400> 168
atgaatcagc atgaactccc cagcgttgca gtgttgttgg ccagtttcaa cggtagggct 60

tggatagcgg agcagcttgc cagtattctt gctcaacagg gtgtcaagct tcatgtgttt 120

attgcggacg acttatccac tgatggcact tgggagtggt tgaatgtaga ggctgcttgt 180

catcaaaacg tgactttgct gccacataat cagcgtgcag gttctgcggg caggaatttt 240

tatcgtttga tccacgaagt tgatctggat ggtttcgatt tcgttgcatt tgcagatcaa 300

gatgatgtgt gggatagcta taagttgagc aggcatgcac gcttgatgaa gctacatcag 360

gctgatggtg tgtcttccaa tgttgaggca ttttggcctg acggcagagt gatgctgatc 420

gttaaatctc aagctcagcg gcagtttgat tatctcttcg aatctgctgg gccaggttgt 480

actttcttga tgactactcg cctcgcacat caagtgcgca atgtgctcca agatgagcta 540

tctccagcgc gggatgttac tttgcatgat tggctaactt atgcaattgc acgttccttg 600

ggatatacct gggtgataga tgctaaccct tctctgaaat atcggcagca tggcaataat 660

gtgattgggg ccaatgtcag tcttaaagcg aaatggacga gattgaaaca aatccgcgat 720

ggctggtatc gccagcaagt cattaaggtg acacgcatcg cttatgcttt ttctcagcgc 780

gaagatttgc agcgcttatt ggttttgctg gagaagcctt cacttggaaa ccgcctgcag 840

ttattgccat atgcctggca tggaaggcga tcagtgaagg atcgtttatt cctgatgttt 900

agtattgtgt ttaagtggtt ttga 924

<210> 169
<211> 954
<212> DNA
<213> Methylobacillus glycogenes

<400> 169
atgagtcagc aacatatttt ggttaccgga gctaatggct ttgtgggtcg atcactatgc 60

aatcaattat gccaatcagc ctttcaagtc acccaggttg gacgtgctct aaatagcgct 120

gatgggggag catatagcca ggttgtgtcg atagatggaa caacggattg gtcgaccatc 180

ttggatgagg ttgaggtagt gatacatctt gctgccagag ttcatgtgat gcaggaaaac 240

ttagttgatc caatcgtggc ttactggcaa accaatgtcg agggcacctt gaatttggcc 300

aggcaagcgc taaatgctgg ggttaaacgc tttatttttg taagttccat caaggttaat 360

ggcgagcaaa gtttcccagg caaaccattt actgcagaag atatgcctat gccagaagat 420

gcctatggca aatccaagtg ggaggcggag cagggcttgc ttgcattggc aaaggattca 480

aatatggttg tggtcattat tcgcccacct ctggtttatg gtcccggtgt aaaaggtaat 540

ttttccacca tgatcaaact gctgcagaca ggcttgccat tgcctttcgg ggcgattgcc 600

aatcaacggt ctttggttgc tatcgataat ttgatccact taattatggt gtgtattaat 660

catccggccg ccgccaatca ggttttctta gtcagtgatg gtgaggattt gtctactacc 720

gctttattgc gtcgcttggc tctagcggct ggcgtatcat ctagattgat tccctttcct 780

gcagcatggg tcaacttcgt ctttactgtt ctgggtaaga aaatgatggc agggagaatt 840

ttagggtctt tgcaggtgga tatcagcaag acacgcgagg tattaggctg gcagcctcct 900

gtgacagttg atcaagggct gcgaaattgt tttccaacta aggctgagcc gtga 954

<210> 170
<211> 552
<212> DNA
<213> Methylobacillus glycogenes

<400> 170
gtgattcgtt tttttgacat tgtattttcc ttgcttggcc tggtattcgg tttgccatta 60

ttgataataa tttatctggt cggcttgctc gatacaggct ctcccttatt ccgccaagtg 120

agggtagggc ggcatcgcca gccatttacg ctggtgaaat tccgcaccat gaaagtcaac 180

acagcatcag ttgccagtca tctggcttct gccagctcta ttacttggtt aggtagtttt 240

ttacgcagta caaagttaga tgaattgccg cagctttgga atgtgctcaa gggcgagatg 300

agcttggtgg ggccgcggcc gaatttgttt aatcaggatg agttgattgc agcgcgtgat 360

gcacggggtg tttatgcagt tcgaccaggc atcaccggac tggctcaggt gaatgacatt 420

gatatgtcta tgccaaagtt gcttgcagag acagatgctt ctatgttgca gcaattaaat 480

ttaaagcttt atttcaagct gttaattcag actgtattgg gcaaggggcg tggtgacagg 540

gtcgtgcggt ga 552

<210> 171
<211> 1816
<212> DNA
<213> Methylobacillus glycogenes

<400> 171
atgcttgcgt ggtgggtagc atttcttttc cgcttcaatt ttgatatccc tgccccattt 60

gccgagagca tgttgcaaag tgcagcttgg gttattcccc tgcaagcaac atttttcctg 120

ctatgcggcc tttaccgcgg tgtatggcgt tttgccagtc tgccggattt acagcggctg 180

attcgagcga ttggggtggc cgcagtagtt gtggtagcaa ttttgtttat gttccagcca 240

catggggttg tgccgcgctc ggtgttgatc atgaatccca tgctgctgct tttgttgatg 300

ggtggcagcc gcattgccta ccgcgcctgg aaagagcatc gactgtatgg cgcggcccaa 360

tcctcaggta agccagtgtt ggtgcttggc gccggggatg ccgcgattgc cttgctcaag 420

gatttatcca agagtcatga gtggcgcata gtgggcttgc ttgatgataa gcaatcaatg 480

ttcggccgtg agttgcttgg tgtaaaagtg ctaggcaaac tcagcgattt tcctgagatc 540

gccaaggcaa tgtctgttca gcatgtgatt gtggccattc cttcggcgcg caacaacgca 600

ggcgccagat tattgatatg gccagtcagc acaaactcaa ggtacttacc gtgccatcac 660

tggaagattt gatgagtggg cgtgtcagca tggcgcagct acgtccggtt gaggttgaag 720

atttgcttgg gcgtgagcct gtggtgttgg acgatgatgg tttgcaccag ttgatcgata 780

gtcaggtggt tatggtcact ggcgcgggcg gctcgattgg ttcagaactt tgccgacagt 840

tactcaagtt tcagccttca atgctggtgt gtttcgacca ttcggagttt tctctttata 900

gcctagagca agaactgacc gctttaaagc tggatgtgcg gattgaatat ctggtagggg 960

acgtcaagaa tgcacgacgc gtgcagcacg tgcttgctag ttatcagcca gcacagtatt 1020

tcatgctgca gcctataaac atgtgcctat gatggaaaac cgtaacgttg cagagtcatt 1080

atccaataat gtgctgggta ctttcacagt agccagtgca tgtaaggcgg cgggcgttaa 1140

taagtttgtg ctggtatcca cagataaagc ggttaaccca accaatgtca tgggggctag 1200

caaacgtctg gctgaaatgg tgtgtcaagg tttgcaggcg gaaacaggaa cccgctttgt 1260

ggtagtgcgt ttcggcaatg tgctgggcag cagtggtagt gtgatcccta agtttaaaga 1320

gcaaattgcg cttggtgggc cagtcaccgt tactcatccc gagatcacgc gttatttcat 1380

gtctatacct gaggcgtgcc agttggtcat gcaagccgga ttgatgggta agggcggaga 1440

aatattcgtg ctagatatgg gtgaccccat caagattgtg gacttggcgc gtgctatgat 1500

taggttgtca gggttggggg atgatgaggt cgacattgtt ttttccggcc tgcgccctgg 1560

cgagaagctg tacgaggaac ttttggcaga cgacgaactc actttaccga cgccgcacga 1620

taaattgcgt atcgccgtgg ctagaagtgc agatgaggcc tgggttgtgg ctttgctcaa 1680

gtgggtagac aagtcatcca gcatggatga ggcagtcgtg aagcaagagc tcaagatgtg 1740

ggtggaggag tatgcaggtg atatacatgc tagtctttct tctgcgttga ctcccgggtc 1800

atcgacaata cattaa 1816

<210> 172
<211> 876
<212> DNA
<213> Methylobacillus glycogenes

<400> 172
atgacaaaaa aaattaccaa ggcagtcttc cccgtggcag gtcttggcac gcgtttcttg 60

ccggccacca aagccagccc caaagagatg ctgcctatcg ttgataaacc tctgattcag 120

tatgccgcgg aagaagcagt tgctgctggt gcaactgaac tgatctttat tattggtcgc 180

aataagcgct ccattcctga tcattttgac aagtcttttg aacttgaagc ttccctagaa 240

gccagcaaga aaaccaagat gcttgaggaa attcagaaca ttctacccaa gcacgtttcc 300

tgcatttata tccgtcaagc tgaagctttg ggcttgggcc acgcggtgtt gctggctaaa 360

tctgtcgtgg gtaatgaacc gttctctgtc attttggcag atgacttgat tgatgctgaa 420

aagcctgcga tgcaacaaat gactgagttg tttgaaaagc aaggagcttc aattcttggt 480

gttgaagatg ttgagccaag cgagacagca agctacggta ttgttgatgc agtggaattg 540

acaggcccag atgtattgca gctgaacggc attgttgaaa agccacagcc tgaagatgca 600

ccttctaacc tggcggtggt tggacgttat attttgactc ctgaaatctt cacctgcctt 660

gagaatgtca agccaggtaa gggtggggaa attcaattga ccgatggtat tgctgcgctg 720

atggagcaac aaaagattct ggcttatcgc ttcaagggta agcgttacga ctgcggcagc 780

aagttgggct atgtcaaagc caatgtcgag ttttcgctta agcatgaaga aattggtgag 840

gagttctcgc agtatctgcg tgaattgacc aagtaa 876

<210> 173
<211> 26858
<212> DNA
<213> Methylobacillus flagellatus

<400> 173

tcagcctctc tgggcaaggt tttgctgcca atgccaacta tccttgcaca tctgctcgat 60

gccacgcgtc gctttccaac ccaatgcctg ctcggcataa gcaggatcgg tataactggc 120

cgccacatca cccggccggc gtgccaccag ctgataggga acagcctggc cactggcgtc 180

ttcaaatgcc ttgaccattt ccagcacgct atatccttgg cccgtgccaa gattgcatgt 240

gaacacacca gcttccttat ccagccgctc cagggcacgc aaatggccaa ccaccaggtc 300

gaccacatgg atgtagtcgc gcacgcccgt accatccacg gttggatagt cactgccaaa 360

cacagaaaga gattcacgct gcccctgggc tacctgggtg atgtatggca tgaggttatt 420

gggcatacca ttcgggttct caccaatgag gccgctctca tgcgcgccca caggattgaa 480

ataacgcagg atggcaatac gccactctgg atcggagact gccaagtcgc gcaggatatc 540

ctccaccatc agcttgcttg ccccataagg attggtcgca gacaacggga aatcctcgcg 600

aatcggcaca ctggctggat cgccatacac agtagcggac gaactgaaga ctaatcgttt 660

gacgtcatgc gcctgcatga cgcgcagcaa ggtaacacta cccgccacat tgttatcgta 720

ataacgcaaa ggctgcgcga ccgactcccc caccgccttg agcccggcaa aatgcatgac 780

tgcctggaat gggtacctag aaaataaatc actcagtagt gcttcatccc gcacatcccc 840

ttccaccaat acgggtgcct tgcccgtgat ctgcttgatg cggtcaacga tatgcgcctg 900

gctattgctg aaattatcca gcaccaccac ctcatagccc gcattaatca gttccacaca 960

tgcgtgggaa cctatgtagc ctgtaccgcc tgttactaat aacattaaaa acttttcctt 1020

tagtccaata gccctattga agcgccacac atatttatag tagctttata aacatagaaa 1080

acttttaaaa aaatctgaat tagaaagtta agctaccatt ctgtgatagc cttcttgtaa 1140

tataaaccag taaataataa tttcaccaca cccgcaaata atccagatat taagtgagtt 1200

aatagattga cgacattaaa taacactatc attgtcactg gtggcgcagg cttcatcggt 1260

ggcaacttcg tactcgactg ggtcaaggca ggactaggca ccgtcatcaa cctggacaaa 1320

ttgacctatg ccggaaatct tgaaaacctg gcagacattg aaggcaaccc caaccacatc 1380

ttcgtacatg gcgatattgg tgaccgcgcc ttggtcgcca agttgctggc agaacacaaa 1440

ccccgtgcag tcatcaactt tgccgccgaa agccatgttg accgttccat ccatggcccg 1500

gaagacttca ttcagaccaa tgtagtcggc accttccact tactggaaga agtacgcgcc 1560

tactggaata gcgtggtgaa aacccaccca gaggaaaaag aaaatcctca gttccgcttc 1620

ctgcacgtct ctacagatga ggtgtatggc acactgggcc ccaaggacca agcgtttacg 1680

gaaaccaccc cttacgcccc caatagtcca tactcggctt ccaaggcagc ctcggatcat 1740

ctagtgcgtg cctaccacca cacttatggc ttgccggtat tgaccaccaa ctgctccaat 1800

aactatgggc cttaccattt ccctgaaaaa ttgattccac tatgcctgct caatgcgctt 1860

gctggtaaac cattgcctat ttacggtgac ggccagcaaa tccgcgactg gctattcgtc 1920

aaggatcatt gcagcgccat ccgccgcgta ctggaagcag gccagctcgg ggaaacctac 1980

aatgtgggcg gttggaacga aaaggccaat ctcgatgtag tacagacctt atgcagcatc 2040

ctggatgaac tcagccccaa ggcagatggc tcaagctaca aagaccaaat cacattcgtc 2100

aaggaccgtc caggccatga ccggcgctac gcaatcgatg caagcaaact ggagcgcgag 2160

ttgggctgga agcctgcgga aaccttcgag tcaggcatca gaaaaaccgt ggaatggtat 2220

ctgcaaaacc aggcatgggt gcacaacgtc accagtggag aataccgcaa ctgggtcagc 2280

aagcaatata gcgggagcgc agcatgagaa aaggcatcat cctcgccggt ggatcgggca 2340

cccggcttta ccctgtcacc caggcagtct ccaagcaact gctgcccgtg tatgacaagc 2400

ccatgatcta ctatcccttg tccacgctca tgctggctgg gatacgcgac atcctcctga 2460

tctcaactcc gcaggacaca ccacgctttg aacagctatt gggtgatggc agtcaatggg 2520

gactcaatat tgcctatgca gtacagccct ccccagatgg attggcccaa gcctttatca 2580

tcggcaagaa atttattggc aatgatccaa gtgccctggt gcttggcgac aacatcttct 2640

atggtcatga cctggcacat gacctggcac atgccgatca acggcagcat ggcgccactg 2700

tatttgccta ccctgttcac gaccctgaac gttatggcgt ggtcgagttc aacgatgctg 2760

gccaggctat cagcctggaa gaaaagccca aagagccaaa atcccgctat gccgttactg 2820

gactgtattt ctatgacaac caggtcgttg acattgccgc cagcctcaaa ccatctcctc 2880

gaggcgaact tgaaattacc gatgtcaaca aaatttacct ggaaaggcgc tccctcaatg 2940

tggaattgat ggggcgcggt catgcctggt tggatacagg aacgcatgaa tccctgctag 3000

aagcctcact attcatcgaa accatagaaa agcgacaagg cctgaaagtc gcctgccccg 3060

aagaaatcgc ttaccgcaaa ggttacatcg atgcagaaca agtagaaaaa attgcccagc 3120

catacctcaa gaatggttac ggccaatatt tgatccggat gctgaaagaa aaggtctttt 3180

aatgaatgtc acgcccaccc atattcctga tgtgatcgta ttcgaaccca agatattcgg 3240

tgatgcccgg ggattctttt ttgaaagctt caatcagaaa gcctttgagg aagccgtcgg 3300

caggaaatat gattttgtac aagacaacca ctcaaaatcc tcaaaaggag tattgagagg 3360

gctacattat cagatccagc aagcccaagg aaaattggtt agagtagtag caggtgaagt 3420

ttttgacgtt gccgtggata tccgcaaatc ttctccaacc tttggtcaat gggtagggca 3480

acatctgtca gcagaaaaca agaaacaaat gtgggtacca ccaggttttg cccacggctt 3540

cctcgtgctg tccgaaactg cagagtttct ctataagacc acagacttct acgcgcctgc 3600

gcatgaacgt tgcctaagct ggaacgatcc cgttgttggt attgaatggc cagatattgg 3660

cacgcccccg ctcctctcaa gtaaagatca acaaggctta ggcctccacc agacagagtt 3720

attcgcataa tggcaacttt caacaagatc ctgcttaccg gcatcaacgg ccaggtaggc 3780

catgctctct atccgaaact acaaagcttg ggagaagtcg ttgcacttga ccgcagccaa 3840

cttgacctat ccaaacctga ccatatccgg gacgtagtgc gacagaccaa accagacctc 3900

ataatcaacc ctgcagctta tactgctgtg gataaagcgg aatctgagcc ggagctggcc 3960

tacgccatca atggcactgc tcccggcatt ctggctgaag aagcagcaaa gctaaatgca 4020

ttgcttgtgc attattctac ggattatgta tttgatggca ccaaaactac cccttacaca 4080

gaattggatg aaacaaatcc acttagcgtt tatggagcca gcaagttagc aggagaaaaa 4140

gccattcaag aagtcggagc aaatcatcta atcttcagga ccagctgggt ttatggtgca 4200

tatggaaaaa atttcatgcg caccatattg agattagctg ccgagcgtga ctcattgaat 4260

atagtcgcag accagtttgg cgcaccaact tctaccgaaa gcattgcaga tgcaactatt 4320

actgcgctgc acgcatggga tgggactcaa agaggtgtct atcacttagt caatactggg 4380

gagacgagct ggcatggctt tgcactggaa atcattcgcc agtatggaga aatagcccaa 4440

aagaaaaatt tagcgaaatt aaaatccact gtagaaaatg ttgtcggtat tacaactgca 4500

gaatacccca caccagctca tcgccccgcc aactccaaga tgagcactga aaaattcatg 4560

agcactttca aaactgaatt agcaccatgg gaattggctt tgcgatctac agtaacccac 4620

ttagcaacat tctcggtcga aaatcattag cattattact aattaatcat taaaactgac 4680

ttcagtcaga ccaaggaaaa ttattcatga acgtccatat tgatcattga agcgaacaat 4740

atcatcttca cccaaataag ccccagactg aacctctatc atttctaaag gaagctttcc 4800

tggattagcc agcatatgcg tcacacctaa aggaatataa gtagactggt tttcagaaac 4860

aaggaactcc tcatctcctc ttgttacctt cgctgtaccg gtcaccacaa tccagtgctc 4920

ggctctatgg tgatgcattt gaagagataa cgtagcacct ggattcacta caatacgttt 4980

tacttggaac cgtgccccca tatcaatact gtcataccag ccccaggggc ggtgaacctt 5040

gcggtgagtc ttggcctcgg gccttttctg gaccttaagc tgctccacga tttttttcac 5100

atcctgagtt ttacttttat ctataactag caccgcatct gctgtctcaa tcaccgcgat 5160

gttttgcaga cccacacatg caagcagcct acctgaagac acagctagag tattcgagca 5220

atcattgaac agcacatctc ctaaatggga gttacccgcg ttatcctgcg gcaaaatttt 5280

cccgaatgaa tcccaggccc caacatctga ccagccggca ttcaggggga tgactatgcc 5340

tgcaggcaga ttatcggcac cacgagcgac atgctccatc accgcatagt ctatagaatc 5400

gctaggtacc tgttgaaatt taagcttgtc cacccgaaca aaatcgccat cgcgttgttg 5460

accatcccaa gcctgcttac atgaagataa gatatctgga cgagtcttag caatcgcatt 5520

cagccatacc gtggcacgca tcatgaacag accgctgttc cagaaataat tcccacttac 5580

tacatattgc tgagcagtag cctcatcagg cttctcaaca aactgctcaa tactgaaagc 5640

agaaccgaaa ccttccagca ctgcgcctga cttgatataa ccatagccag tttctgccct 5700

atcgggcacg ataccgaagg tgacgatctt accagcagcg gcatgttcag cccctgtcct 5760

cacggtatcc tgaaagtctt gcacattgat aatagcgtga tcggcgggca tgacaagtaa 5820

gattggatcc tggccgtggc tggcagcctc taaggcagcc aaggtgagcg caggggcagt 5880

gttcttgcct acaggctcaa ggagaataga tccacgcctt cccatcatcc gcaattgctc 5940

agcgataaca aagcgatatt cttcattaca taccagaatt ggagaacact cggttccaat 6000

gagtggatcg accctgcgta atgtcgactg aagtaaagaa tcattatcaa gcaatgtcag 6060

caattgcttg ggatagtttt ctcgagaaag aggccacaac cttgtgccag agccacctga 6120

taaaatcacg gcttgaatct gcataatttt ctcactggat agaaatataa agggaaagaa 6180

ataggcacag tatctaatta cttatttggc aaaaaccttg tcagcaaggc ctcatattgc 6240

tgcaacactg agtcccaggt aaataaggta tcgtattgag ccaagctacc ggctctcatc 6300

tcacgtaacc gaacttcatc attaagaagc tcatcaagag tgatttcgca gtccttttca 6360

ccagaaaagt aggccgcctt atctccagcg acccagcggt taaaacggtt atcgtgcgcc 6420

aatactgcat taccagcccc tagtgcctcc accaaagaag gattggtgcc gccaacttgg 6480

tggccgtgaa tatataatct tgagaaatac cgtaaagatt cgaccacgtg tttatcataa 6540

attgcgccag gaaaaagcac ctcgtctgag ccggcagctt taaccgcgac ctgataagca 6600

tggtcagggt tatattttcc tagcactaca agcctcatgc ctcgaggttt agcggaccag 6660

gctttcacca cctctaaaat agaattttca ggctctgcac gagcaattaa gatcgcataa 6720

ctgaaaggct taagtccata ttcctccaaa acggcctgat tcgcctcaat cactttatct 6780

gcgccgtatg gaatcattgt gatcttgcta ggcctgactc ttgtttccag atgacgcgca 6840

atttgcggat gatcagcaat cagatgattg cctaaaattg aaccagccca atcattaatc 6900

cagaaccaga gtttagcaac tgccccccat tttttccgat gccactcgat tccatccata 6960

ttaattagat tagttattcc tttagttcta tagagcccac agaataaagc agtgttatac 7020

ccaagagtta aaataagatg ataagtattt gaagcatgaa tcgttgactt ccaatcaaaa 7080

agaatagtgc ctaaagaacc ctggatagat atcggaatat ttactcgctt tataccattc 7140

cacatatcct catacatagg cccattacca tctacttggc aataaactgt tactttccac 7200

ccttttcttt caagataaat agccaaatgc tctgcaaatg tttcaaagcc accatgcgca 7260

gccggaattc ctctggttcc taatattgct attttcttat gcatacaaat acaaactcat 7320

gttaaatgtg attctaatat ttcttatttt tattaaaatt tttcatcaaa agctgaagta 7380

caggaacatt gttttgacgt tcgacaatag catcattctt gaatatacta tctggcattt 7440

ccgcatccca tccgctatat tctctatagc tatgatatgt ccatgaccat ttctcttgct 7500

caaagcgctc aactacatct gtcaagtaac gtaacctcga accagacgga gcccaccgaa 7560

taatgctaaa ttcaccgata taaagaggaa cattatattt attagcccaa ttacgagcaa 7620

tgtctagtcg attatttagg tattctttat cccacttttc actgggatat atatatgcat 7680

gtgggtatgc cgccaatcct tgatgagtaa gttcttgggg agaataaaaa tgaaagctgt 7740

aaactatatt attaaatgga acaggagtaa actgctccaa actagaagcc agcccccaag 7800

gactaggttc cagaaaaatt atatgatttt catctacttt acgtatggca gtaatcaatt 7860

cagtagcata cttttcccat actgatccag gcctaacaac aggctcatta atcaggtcaa 7920

accctgccac cacagtctca tttttgtatc ttttagcaat agttacccag ttattcttaa 7980

tgctttctcg taattgttga ttattccagt agatcgcctg atctccagca ggtttaggat 8040

caaaagcaaa aattacataa aatcccaact tacgacctgc ctccacaaga aaatccattt 8100

gcgacaactc aactggagaa atttcataat cattacattt tttacatttt tccaaatgaa 8160

taacgcttcg aattaaattg acgttaaact tttcaagatc ttccatgtca gcagaactta 8220

atttccgccc aagaacatta aaccctcgat aagaatttcc tttaaaatcg tctgaattaa 8280

gaaaagcttt cggtccttgc aaagccgcag aaaccttatc gaccgaagca gctgtagttg 8340

tttcagaaga agagccacca gcagaagaag agccaccagc agaagaagag ccaccagcag 8400

aagaagagcc accagcagaa gaagagccac cagcagaaga agagccacca gcagaagaag 8460

agccaccagc agaagaagag ccaccagcag aagaagagcc accagcagca gaaggattgc 8520

ttgaattgat agatttatta gatgttgaat gctttatagt tacattagaa tcactactag 8580

actcatcaat aggagcttct gtaacattag cagcagaaat agaattactt ctggatttat 8640

tagctgcagc ctcagattta acattcttct tataatcaga aggaaaaacc tttatctttt 8700

gtgaattagc ttgaggggta tcagaataag tggtactccc gtctacatct gtagatttat 8760

aaacttcagc aataccaacc aaaggcataa gggaaaacaa actaagtaaa acaatccttc 8820

gagagttaat taattgcata gttatggacc tccaggctag atacgatcaa atcacgccac 8880

ttcaaaaaaa cactgtaaat aaaaatccac acctattatt aacggcaaaa taatctaatt 8940

atttagctaa ttttattatt agctttaaaa tttatttttt acaattgaca cctataaaaa 9000

ttaatatata aagattactc ccgaagttaa attaaaaaac tcatacctta ttaaagtcaa 9060

agataatcac tatcaagcta tagcaatgga aaataatcca aataaatata acatacaaaa 9120

gagcctattt actgacatca caattcagaa gatgaaaaaa tcaagaatcc taaccccatt 9180

aagaatttta ctaaacatca ccgcaaaata tttttagatc aaacccgttt accataatct 9240

tagcaaatac acattacgat atacttttct gataaacact gatagttttt atcgcaatct 9300

catcccaact gaagcgagtt ctcatatcgt tttctgcttg caaagtaatc ctagataagt 9360

catggtaaga aaaaattttt acaagctttt cagcaagatc aatataattt ccagcagaaa 9420

acaaaaatcc gttttcccca tcatttataa tttgcgccat acccggaagg tctgaagcta 9480

acactggagt tccataactc attgccatta ataaaacacc actttgatat atttttctat 9540

aaggcagaac aatcaagtct gccgcactat aaaacattga tatctcttga tctggtatgt 9600

atctaatatc caatttacaa aaatcattta gcccgttttt acttattaat tcttgatact 9660

tagagaaatt atctttccac acttttccag caatgactag cttaaatggt tttagtttat 9720

ctttaaccaa acctaacgct tcaatcaaca aatccaaacc ttttacttct ttgatttgac 9780

caaaaaataa aattaccttt tcatctgatg atacattaag agcttgacga gcatctgctt 9840

ttggcatcga gggtgcaacc agaccaatat aacttccgtg aggaattaca tgtattttat 9900

ttatagaaac attacttcta ttaatcaact catccttact aacttgattg tgcacaatta 9960

attgattaca gagaaagtaa gtccatttta acaagataga gaaggttaga cccggcttaa 10020

aagactcaac atcgtgcact gtagcaacaa ccttaaatcc aaataagcga aaaaataata 10080

cacttaaaaa ttctaaaaca cccacatgaa aaaaatgaaa atgggcaata tttgaataat 10140

tttttttagc atgctttatg ctcctacaaa gccctaagat atacctagcc cctcttctcc 10200

acgtagcatc ctttccccaa atatttacaa aagtaagttg catgaaaata ggtctattac 10260

cacaaggttt agaaatgtca caggtatacc aagttgcaga tactccctga tgtaacaaac 10320

cggcacacag tccagaatca taatagtcca taccaccgtg actaccaact ggctccacaa 10380

aggcgacgtt aatttctttc acataacacc caatctgaat aattattttt tacctgatac 10440

atatgtgtat ttaacaaaat ctttaatgcc caaaaataaa gcattatatg catcaagtct 10500

cttatatctg acccattcaa aagagtttcg aaacacccat aacataaaac ctattaaaaa 10560

tagacctttc ttaagcacag ttccatgagt aaaaacagtt aaaaatctat ttttcgcagt 10620

ttgataaaca aaaaaaggaa gcttaggatt ccttccttta aacataccaa cagcctctac 10680

ctctttgccc tggctaccct gagccagatg gcgaacaaca gcttttggca tataataaat 10740

tttatatcca taagaaatta ctcttaaaga taattcgacg tcttcttcgt acataaaata 10800

gcgtggatcg aacccgccac actcttctaa aactgaacgt ctaatcataa aagcgcaacc 10860

actggcaaac tctacaaatc gatcttctag agctattttt gatacagcac ttaatccaca 10920

acctggtacg cgtgccgaac cagttaacca gttcatatac cccccaccat accaaagtat 10980

atccaaccca tcacccatca tagtaattct tggggtaacc aatccaactt cagggctaaa 11040

aaatccatca tgcagtaatt gtaataatcc aggttcaact agtgtgtctg gattaagaaa 11100

aaatatatat tcacaatctg tattaactat aacccaatct tttccaatat tgcatccctc 11160

accaaagcca agattttttt cacttttaat aagtacaaca tctggaaact cttctacgat 11220

gcactcagga caatcatccg gagatgcatt atccacaaca ataatctttg aatatctctc 11280

ttccctaaga gagtaaatac aagaaataat ttggtcacta gtattataat taacaaaaac 11340

tattgcaaca cttaagtcta actttttttt tgaactaaaa tccgtcatca ataagcacca 11400

ataaaaattg caaccatgaa taaagaatcg ttaacatatt tttaaatccg ttttgaagaa 11460

acaaaactcc ataatacttt aaagaactct ggtattgttt tataaatagc aaagaacggg 11520

cttatatacc acgggagaaa tttaaacgca tattttgccc aaacaaattg aaaaaaacga 11580

atcaatcgca tttttctaga acttgaaaac tgcgttctaa atgttcgata atttaaacat 11640

gcttgcttga taaatattac tgggcccaat ttagcagctt gcaggaatag ttgtatgtca 11700

tataaccctg gattattttt aacgaaagaa gacctcaaaa ccacacaccc ttgctttaaa 11760

gggtatactg gtaaaataaa gattccgaat aaagtaaata gtgatgacag tacgcaatca 11820

tctgatacaa aaggaaaacg tgattcttta taaatcctcc cagactcatc catatatgcc 11880

acagcaccaa atgcagccaa agcattggga tttgattcca gtaagtcaag ccgctttgac 11940

aaagatccca atggaatagt gtcatcgtga tctaaaatta gcaaatattt accggtcgcc 12000

aattcaatac cagttctata tgagtcttca ataccagcat taatactttt aaaatgcaat 12060

tttatatttg gatgatcaat ttcttgtaaa tatttccgcg ttccatcagt ggatccatca 12120

tcaacgataa tccactcaaa gtcttcaaca tctccttcaa ggacactttt tttagtatcc 12180

tcaaaatatt tcattgaatt atatgttgcc gtaataatac taattaatgg agatgtttta 12240

acattcatct ttaagttctc atattatttt atataattat gactatatat atattccaat 12300

ttacacttta agaagttagc tttccttttg taagattttt ttgattaaga taattagccg 12360

cgcacataag accagcaaga gtagccaaaa aagcaattcc cggggtttgc attaattctg 12420

gctgagtaat gcaggccaaa aaatactcta tcattgcagc aaaagaagct atggcaacac 12480

ttgatataaa taaatcatta gaccgcgttt gaatgtaagt taaatgaagt attttgaata 12540

taagaaaaat gactgacatg aatacaataa tgcctgtttt taaggttaaa tagatataag 12600

cactatgaac atatctcact tgctctgctg tcacatcaga tttagcaggc ggcctataag 12660

gggcaccaag cccaatgcct aataaaggtg accgctgaat tgccgcccat gccattttat 12720

tttcataaaa cctccagccg agagaagatc catgagaaac ctcctcctcc acactaaaca 12780

atctctgcat cgcgccttca atagcgcccg gcttaatagc tagcatagca cttaaaccaa 12840

atacagcaac aattgcaatt agtcctacac ttctaagcat tttttttgtc cctagcttcc 12900

agcataagaa caaaacagct aagaaagtcc ccccccaaac agcacgacca taggttaaga 12960

aaattccagc gcctgtaaat atacacaata taataagagt ctttgaaaaa cctattcggt 13020

tagcaaacaa agccagtaca aacaaaaatc cccaaacaat aatgtgagtt cctgacgtca 13080

tgcttcgtgt aacatcacta ttaatgcttc ccaaagtttc aaggctagta agctcgcgcc 13140

cgaacacatt tacacctaga aaaccttgca aaatctggcc aattgaaaat aaaaaagcta 13200

caaaaagaaa gccaaaaaca ataaatttaa atctttgtct atctgagcat gttaaagcta 13260

ttaagggaat caatagccaa taaaggaaat gccttgactc ccctaaaata tctttcaatg 13320

gcaatctaat ttcaaacacc gcccgaaata cagaaaaaag caacagtaaa agtacaacaa 13380

ataaaggaat agccaaaggt gacaatgctt gctttgtaaa gctaaattct gaattgtatt 13440

taaaaaaaca aaaaatgaat gttgcaaaca aggccatatc tgaagatggt attgtgcctc 13500

ccagcagtct tattgaaggc aaaaatgtat ctggaattaa tctagaagaa attgctataa 13560

cagctattaa agctactaaa ggatatgtaa aaccaataaa aataaataat atagaaagaa 13620

taatggctac tatccaccac catggaaaaa tagcactgac aaaaccaaat atcaaagcca 13680

ctattgcagc cattattaca aaaaatatac tccctatctt taaactaacg aaattagcat 13740

ccctacggtt aaaagataag agagaagaat tgttcttcat aatgacgcat tagccttgtg 13800

aaattttaat ggtaccttct acctaatatt taatatatat taaatataac tcaagacttc 13860

acctaaaggt tttcggtatg attgcggaac aacaaatgat tcccttaaat atccagcggc 13920

aacaaacatg ataattaatt cgttttctgg tatacgaagg tgattcctca tttctatatc 13980

ttgtttccta gttttactcc agttaagagc gcaacttcct aggccttgca aatgtaagcc 14040

atatataaaa ctcattgcaa acattccccc atcaatccaa ccttgataac gttccgcccc 14100

attaaaattt gacaaatcca ctgttataca aaataaagcc tgaacctctt cataaaaccc 14160

tcgtgcgccc ccttgaattt gtaaagtact cctaattaat tctggattaa gaattgcccg 14220

gacctttcca gactgtcgat tacaaactgc tggacttttt tgagcaatac gtacggcctt 14280

ttcaattaca ctatgctcta taggatcttt agtgaactgc cttacactat ggcgcataag 14340

aaaaaaatct tctttaaccc cagatattgc gtctaaaacc tcagctttag agcgcaaaat 14400

aaccccgcca tcattaatcc ttaataaagt gctctgatta tttttcttta accgatcaac 14460

gattacttct aattgagaat ctggctgacc aagacttcta ttaaattgac aataacgcgc 14520

cagtacattt atcggaacat ctagctgtgg atcaggccca aaattaagat gaaaattatc 14580

gactgatcca actaattgtc caatcactac ccttccaaat aacggcctgg gattaggtaa 14640

agacattcct ttctcgatgt tgtgataagt agcagtaata accgcaagaa tcttttcacg 14700

atctttctcg aaagaaatta cagatgacca tttaatatat cgtttagcat cgtaacaaaa 14760

gtctctcaat aaactcgggc ctcgaagaca aaatctcaga agccatttaa tagaaagcgg 14820

caagaaacca attatgtatt ttttcatctt acaactatac attctctaaa atatgtgttc 14880

aacttagtaa atttgacttt taacttataa aacaatcttt ttatttgaaa ttctagttaa 14940

taattctgct atgtactgct cataacgcaa tagttttttt tctgcaattt tattaccttc 15000

ttctactgca aacttcaact gctcccgact gttaaaacaa tccataacca aaccaactat 15060

ttgctcgtta gtattaacct tacaatcccc aagataagga taatccaagg cagaaaaaag 15120

cccattaaac ttcctactat acgctaatgg cacaacaggc actccacttg aaaatgctgc 15180

gatacaagca tgcatgcgag ctccaagaaa aaaatccatc cctgaaataa aagatttagc 15240

ttggctagga gaagaaaaag atggtgcaag cttagcagct ggataaagct cttgtaattg 15300

aacacacacg ttcacatcgt cttctatagg aaatgaggtc gttagcacat gaggaacaag 15360

ccatacttca caaccttcta ttcgagtaaa ctgatctaat agtgatctag taagttcagg 15420

ataattaaca ctaagaccga attgattctt cccagtatac ccaccgttat aaagtaatcc 15480

tgatacattt agtccaattt ttactttact tgaatcccgg gaagggcgtt gaaatggcaa 15540

tctaaaagca acgtcaatta cctcctctgc atttacagat aaattattct tttgcaagta 15600

tccaaatgac aatccatccc ttacaaatat tttagtgcac cgctgcatta cattattggc 15660

cacctttaaa ttaaacctac tttcgaatgg ccctatagtt tgcgggctta aaataagagg 15720

tttacgttga aacaagacaa ccaacttaga cacaatctgt attaggaatc gtgaaaaacc 15780

ataaatatca gcaaaactat caccttcacc tatatcaaac accacatcgc attcccggat 15840

tttcttggga taatcagaag ccccgatcaa aattcttttg agtgatacat ttgtccctcg 15900

ctcaattaaa gggttgccct caaagtaatc tttgtttcca ttagtgccga aaattcgata 15960

ttcaacctca acccccgcct gcaccgcagc tttattaata atagaaattt gtgcttcagt 16020

taaagcgcca acaccaagat tatctgaagt agtagaatgc caaagtaagc ctattttcaa 16080

catgactaat ataccaaaag aaaaatggaa agactaaata ctattacagt caaagtaaaa 16140

ttaactcccc caaacccagt aggtattttt ctaagccgta atgcactgaa aaatatcaaa 16200

aacaaggtta ataatgtgat gattaaagca atgaccatac cttttagtcc aatcactttc 16260

atcagaagtg gtgctgaaat tagcaaaata ataagtgcaa aaatttgtgc ccagacacgt 16320

attgtctgag atccagaagc agtcaataga acaccttgag gcgcagcaag atatcttgta 16380

aaaactaaca aaccaatata gggccaaatt gaatttaact ctacatattc atcaccaaaa 16440

acaaattcgg taatatgcat accggcaaac cagaaaatag cccagcacac agtaccaaca 16500

gcaagcatct gaagattaag ttttttagct agctcaacaa actctctagt attagtgtgc 16560

ttggcagcaa ttacaggtaa aaatacattt ccgagtacag gtgcaaattg cattgcacca 16620

tttagaaatc tcatacctgc ttggtaaata ccaactccag tggctcccag ataatatttc 16680

acaagcatgg tgtcaacttg gtagaaaaag ttagtgaaac ccgcatcaac ggcatatgga 16740

aatcctgctt ttaatgcttt tagaacagca actggagagg ctgacagagt atttaaatca 16800

ccaaccactc gcttatatgc ccgccacgac aaaataaaat aaacaaaacg agataaaaca 16860

aaacctaaac taattaataa taactcagcc ccattaatta ccattaaaac tattaagccg 16920

aaatgcaaca aagagccaat tgttgcaact tttgtttctt catgaaattt tccgatacct 16980

ctgaatgcaa tattaaaaaa atccccaaaa gaagtgagca aacaagataa cagtaaaatc 17040

caaaatattg ctctatctaa gtcatttgta tagaataagt aatgtccagc aaggcaacag 17100

acccccatca ctactgtgag atatagtttt gcaagaaaaa ccctccccat taactgatta 17160

atattatcag ggtatcttcc tatatctctt aacaactgat ttgaaaatcc atattccact 17220

aaaagcacag ctattgacgc aagagtaaaa ttgtacatca atatgccaaa gtctctcgga 17280

ccaaggaatc ttgcaagaat aatgaaaagt acaaccgctg atagcaaccg tgaaaaagtc 17340

gaaagagcca taaatgcggt atgtttaatc ataaattgct acatttttat tattaaagtt 17400

aggttaaaaa aacattgaat tttatgaaaa tgtacctata agtttttttc tacttttact 17460

atctacattt ttcagtaaag atttaacaaa ttcttcttgc agtgtaaaag cagagatatt 17520

atctctgcta atacttgaaa ctctaaaaag cagtccatca ggaatatatc cctgcaaacc 17580

taattcaaac ctttttatag tttgacccaa tccaccataa actgtagttt caccaacacg 17640

catccaataa agtattggct catatcgtgc cccatgctgg gctaataaat gtctaacttt 17700

aatttcatat ccatcaaaat ttaaaatggc attacccaac aaatcaacat taaatccttg 17760

agctgtatag caggtttcag gcttatgtgc ttgcaagccg cgactttggt ctcccccata 17820

agcaatcgaa agcattattc gcattccttg cttattaata taggtccgag aaagagtttg 17880

atcataaact ttattgaggt tttgctgcac ctcagggctc acttgtatag gagtaatcgt 17940

aggatcgatt ttccactcat cgaactcctt aggaatcatt gcttctaaat ttatatcttc 18000

tgctgcatta acaaacttag taggtttaag aattactcct cctaaagtag ctaccaataa 18060

cagtaaaaat ataattaaat ttcgcagtgt aaatccatcc attagacagg ccttattaag 18120

agtttaggct tttaactgcc attttttaac caaatattgt aggagcgtat caacactaat 18180

tatcaaaatt agagccacta caaaaagtaa cattccagca aaaccatgaa caaaaccctg 18240

accagcagca tctccaaaat gataagtaac taaagttaat accataactc taatcacatt 18300

agcggtaaaa gaaattggaa taatcaatat cccaagaatt acattacgaa taaatgaatc 18360

gtgctttaca acatttaaat aaagtaaacc cagcgcctcc aaagatatta aagtgtgcaa 18420

accagcacat gcatccgcaa caagtaattg atattgtccg atttggagga tcaccccact 18480

tcttgaaatc ggatatccta tccaataaag tatattttct gctacatatg agaccgcaat 18540

cttcattggt aatgtcacag catcaagaaa aactccagga agaggcacca taaaaatcaa 18600

gaagaataac ggaaaccaca atgctcgaag cccagaataa ccacggaaaa ttagcaacaa 18660

tccagcaaaa acaggaattt gagatgcaat atctaacggt gcaatatctt gagaacggcc 18720

aagaatgtag agtaaaagac caaaaactaa aactatcccg ccaattactg gcctgcctat 18780

aatattattt tcggccttaa acaattggtg tctctgtacc catactaaat acaagacaac 18840

gccaacaatt aatggtccat gagcttgatc ttcagattgc catacagtat ttaataagtt 18900

caaatatgta ggcgtataca taatgaacaa acctaataat agcatccaat tccgattgat 18960

tacctcccag ataccaggat tatcttgctc gattacctcg acagtgttca tatctacaaa 19020

tcaaccaata ttgacccaat taccttagta ccattattct tgcattgttc tacagcaata 19080

gataaatcat tcactcgagt atgattttgt cttgcaacta atacaattcc tccaacagta 19140

aaagcaattg cataaccatc tgaataatta ctaagttcgg gagtatccaa taaaattaca 19200

tcataaacac aagccaactc tttaattaaa tttccgaatg ttggcttgct aacaagctct 19260

tgaggattag gaggtaatgt accggcagtt aatacagata agttttcaaa aaaatttatt 19320

tttgaaatta catcataccc cgctcttcct gctaagatat cagataaccc acgtttgtta 19380

ctcagattgt atatctcatg ctggcgtggg tagcgtaaat tagcgtcaat caataatgta 19440

ttttcaccca attgcgcaaa aacaactgca agatttgcaa caaaagagct tgcattatcg 19500

ctgctgctta ctgaagaaac agctagtgct tttcgttccg gtgaaaacca atgcagcatt 19560

aattgactgc gtactgctct caaaacctca gctttgttac taaaaggctg atatgcagca 19620

acgagctcat taagatatga gttttggtca ggtgataaat aaggatagtt aaattgcctc 19680

gataaaacct gttgaatatc atcttcagtt atcaacccta atgcttgagc agcttctcca 19740

aagcgcaaac ccttctcttt atgtaaccgc ataattcttt cagcatcttc gggtgtaatt 19800

ttccccatcc gcaataataa actaccaatt gtaaattctg aaaattcgtt taccaatggt 19860

gcattttcta ctttttcaga gtttgaatta gaaggtgtaa atgaaatcat aattcagcct 19920

tccctaaagt tatcaggttt gaagtatttt taaaaaatct cagttttctt ttttctttac 19980

taatagtccc aagaagtggg gcttgtaata cttcaattaa gtcatcagca gtccgtactc 20040

ttcgatcaac aaactcagct aaaactccaa accctattcc taaaaacata cccaagagga 20100

ttgacaatat aatattcagc aataaatttg ggctagtggg agtaagaggt gccacggcag 20160

gattcaataa agaaatatca gattgatttg actgaccttc aatattcgtt tgagtatacc 20220

tttgcatagc catgtcgtat gctttttgag cattttcaac ctctcttgtc aaaacagata 20280

aaatatccct atctcgatta agttgtaata ctttttcttt ttgattattt aaagccaaac 20340

gtatttccgc ttcacgttgt tgcagaattc ttgaattact tgccacatta cttgaaaccg 20400

atttgatctg tctatctagc tcagacttaa gattttcaat ttctgcttta gctcctagat 20460

aaaaaggatg gtttctttca aatttctgcg aaacatctgc aaaccttgat tctgctctag 20520

caatttcagt tttaagattt tggataattg gattagctgc tacatcagga gactcataag 20580

cttcttttcc acttgaattt ttttcccgag atctcgcttc cataacttgt gcttgtgcaa 20640

taaccaattg agatgaaagc tcatttaatc tggctctttc cacatccaat ctttcatcaa 20700

cacttacaat ccctttctct tgctgatact ctgaaagctt cgattgcgct tgagctagat 20760

cttctctcag ttctttcaat tgctcaacga aatacaaagc agctttttga gaaggctcaa 20820

ctttaagttg tattgaagtt tgcatataag actcagcaaa ttcgtttgcg attgtagctg 20880

caaactgggg atttacccca aaaaagctaa tatcaataat actactctct cgagatggtt 20940

taacttgaag tttttttagc aataaatctg caagccattc tttaatttca cctttacctg 21000

aagttgcttg ataaaagtcc tcttttacgg ctggaacatc tgccagacct agtttatcaa 21060

ttaccttttt tgcaacgttt ttacttgaaa taatatccac ttgcgttgcc atataacctg 21120

gcattaactg cgcaggaagc attacacctg ttacaggatc agcgccttta taattaacaa 21180

gcactgaagc tgttgcttta taattttttg gcaacaatat tgtaacaata gcggtagtta 21240

ataccgttat aaataaagta aaaataataa ttttatatct agctttaatg atagaaataa 21300

atctgataaa actcatctta gattaccttt ttgattattc agctgttatt caaaaaacaa 21360

actaaaatac actctcttgt acatatacaa catcattagg ctgaataaaa tcatcatgct 21420

ttactttaat tatcttaaca ccaccttcat catctaaccg tttcacctta ataccttttt 21480

ccgttcccct gagactaagg ccaccaccaa cagcgagtgc ttgcagcact gtcatatgat 21540

actcaagacg atattgtcct ggccgctgaa cctcaccata tatatagaat ctaggagctc 21600

gatctacata aataatatct cctgccgtaa gtaagatatt tttatttaag tcgccatccc 21660

gcatcatttc agcaaaatca attatctctc taacactttt cccctcaatc ttgcgaacca 21720

agctaatcaa atctccccca tcaggtgcaa ctccaccggc caacgccagt acgtctgcaa 21780

gagattttgc actatccagt gggtagcgac ctggcctgtt aacctgacct agcacagaaa 21840

cctgctgact ctgcaactga gcaacaatga tattcacgtg agggtttttc aagaatccac 21900

cgctttccag catgctggca attttgcgtt cagcagctgc aggtgtttct ccaccgactc 21960

tgacttcgcc tatcaacggg taggtgatag tgccagactc actcactctt acttccaagg 22020

aaagatcagg ctgttcaaat acagatatcc ttagaacatc ccccggccca agaacaatat 22080

ccgaatccgc tgcgttaaca ttgaatacaa caatcgtcag gactaccgtt acaaaccatt 22140

tgatcaattt agtcatggca ctacacctat acacgtttta tagaattact actttagtcc 22200

gcttactccg cgagcaatat catcatcgac actagagggt tttgactgtt cggctttttc 22260

tggttgttgt ggtacttcgg ccgatttgtc taaatattca atttttgctt cagcccgtag 22320

tcgcttgatt tcagcctcac ccgcttcctt acgcttttta ttcaagagca tttgctcaat 22380

ttgcggtgtc gcatctgcca gtgataccgg gctctccttt acgctatcca tgagcaccag 22440

catcgtcctc ggtcccatgc cgatcacaaa cagctggcca cgccttgcat ccttcaggcg 22500

ctcagaaatc tgtggaggca gctctgccac actgcgggca acttctccac ggtcatattg 22560

gattccctta gcatccaacc acgcagcgac ttcctgcagg gttttgaagc taccgagcgc 22620

agacttgaat tcgtcattga cataacgtga atcaatcaat atttgttcca tttccaccag 22680

cttgcgctcg gaaaatagtt gagggttttg cttatagaaa ttctcaactt ctgttttgtc 22740

cggcttgcct atgttagcca ccctagactg gaggtatgcc tgggcaatca tttgggattt 22800

tgcgcgctca atggcttgca tgacgttcgg gtcccggtct acttttgctt tcaaggcagc 22860

ctgctccaac agttgacggt caatcaatga ttgcaatatc tgcttggtag cagcttcttt 22920

ctgcgctggc tggacattgg cccgagcaag ctcttcattg agttgatgga cggttatttc 22980

cttgccgttc actctcgcca gcgattggct agcctttgtt tcattagatt tatctccaca 23040

gcccgacagc ccgatactaa tcatcaaggc gaccaacgcc cctttcgtca ttttcgacca 23100

ttgcattatt gtgcttccat attcagtatt caaaatccag tctcctcaaa attcagccaa 23160

caccatcaat acgcattatc ttttttaaac actaccagca ccgtccggat gatgatccag 23220

agatcgaacg ccagcgacca atgcctgaga taatccaaat catattcaat ccgcgcttcc 23280

attttttcga gggtatcggt ttcacccctc aatccattca cctgggccca gccagtaatt 23340

cccggcttca ccttgtggcg caacatatag ccacgtatga gcttgcggta ttgttcatta 23400

tgcgccacgg cgtgaggcct cgggcctacg atactcattc gcccctgcaa cacattgata 23460

aactgtggca actcatcaag ggaatttctc ctgagaaaac caccaatcgg tgttaggcgc 23520

tggtcacgct tttgtgcctg caccactttg tcaccatctt ccatcactgt catggagcgg 23580

aatttgtaaa caatgatttc ctcaccattc aacccatacc tacgctgctt gaatattacc 23640

gggcctttag aagtcagctt cacagccaaa gccagtgtca gcatgatcgg tgaaagcaac 23700

aacaggataa gggatgcaag aataatgtca ctagtccgtt tgattaagct attaaaccct 23760

gtaaacggcg tttcacaaat cgccaccaca ggcacacccg ccacatcatc aaaccttgcc 23820

tgcaccagat caaaaatata gatatcgggc acaaaataga cagatgctgt cgtgtcttgc 23880

agctcctcaa acaactgtct gatgcgcggt tgcgcggaca tcggcagact gatgaaaatg 23940

atctgggtat tgttggcacg cacataagcc gccacgtccg ccattgtgcc tagcaacggt 24000

acatgacgca ttcccttggg atgccgggat atctcgcgat catcaaaaag acctttcacc 24060

tccatgaaca ggtaagggtc ttcattgatg cgattcacca accggacacc gatatcatta 24120

gcgcctacta tgacaacaga acgaacttcg ccatttttat gcaaatccga ggcaattttg 24180

cggatgagca aatgcgcaac agtaaagatg atcggcaccg ccacaaacca ggtatacacc 24240

atttcctcat ggtactgatc agtaaggtct gcggcatatc cgataaatgc caatatggtg 24300

acaacaattg cccatccgaa caaaatatca cgaatatagc ccaacacttt tccactacgc 24360

catgtgcggt aaagatccag gcgctcatag acatttgaag atacaaagta tgcaagcaac 24420

gcaaggatta agtagtagcc attgaatggc tcaccataaa gaagcgtaaa gccatacaaa 24480

gtccccagaa taattaaagg atctaacagc tgcttgaaaa aagcgactaa aggtatgtca 24540

tttaccgtca tttatagctc ttcaaaactc atattgccct gttactgaga cactatttga 24600

cctaaattca aacatatcag atgttgactc tcgatcacta tgaataaatg aagtagttat 24660

cctaaaagat gttactgggt tatacataag tgacagcata aaactcttat ctttatctga 24720

cctaaattga ccaaaaattt gatccccctc aaaatctctt ttttcatatt tcaatacgcc 24780

ttgaacagct atcttaccag ttgcctgcaa attagccgtt aatgatgttc cacgattcag 24840

cgtataactt gttgtaacaa atgactgagc gttattttct ctccaaactg cagcattcaa 24900

attggttttt ccagaagtca tccaattaaa gtttccccga gcattaaccc ccctaaagtc 24960

ccgttgagac agttgctcat gctgcctatc taccaaacca ccaagaaact gcaaccttga 25020

ctttccggta acagcccaat cgacattcaa ctttaattca ttctggtcat aatcattgct 25080

tataaaagat ccctgaaaag cttgctgcgg ctttgatcct ttagtatttc tgtacagaac 25140

accgatgata cttttagtag gagatagata gtcaaatccg atctcctgta tatcttcctt 25200

tagattagcg gccctttgag cctcagcccc gtactccatt tcagagtaaa taaaagcccc 25260

tctaacacgc catctaggat gcagcatcca acgcgcctcc aataagcggc ggtcctgagt 25320

tcttgtgttt aacgataaca tggttccttg cagaggttgg aattctgaaa aaggaaccaa 25380

agctttttta tgaaaaaact caaccttgcc ttccaatcga tttcccaact cccaatccca 25440

cctggcaata acgtcacgac catcattatc taactctgag tttttattaa atgtggactt 25500

gtttaatcta aaatccacat acattttttg acgactaata gtatgatcca gcacaatacc 25560

agcgcttttg acaagcattg tatctgaggt atctgaactc ccagtactca tttgagctaa 25620

ttgcttacta gcgaatcttc ttaaattatc atcatatgaa tatgtaagac tggcatacgg 25680

ctttaaaaca tccccctcta atgcacaagc agggactaca aatccagcaa gctcgatacc 25740

tagcccagcg accaaagcaa tttttattag gttagttgat tgagataacc gcaaagaagt 25800

gtttccccaa tgattaccag taatgccagt taccggtata tatgatccaa agtcccaata 25860

tgccatttgt caccgcatga gcgatgatcg gcaaccatat ttttccgcta cgaatataaa 25920

gcaagccgta tgccaagcca gccactaaac cggcaaacca ttcggtatgt tctattgcaa 25980

acaatgcgga tgaaactagt aaggccagga cgcttacctg ccgagggtcg acattgagga 26040

aatgttggtt gtgaatccag cgcatcaaga atgaacgcca gaacagctct tccatcaagg 26100

gcacgaccaa tgcggccccc atcactcttg tcgccatcag cagacgggca tccggtccgc 26160

ttcccggatc gaacccttcg gattgatcgc caatcaacat ccatggcgca tccagattga 26220

tccaggcgat aaatactagc ataccaacgg cgattgcgct caaccacgac ctggctccca 26280

gcgcggctag cccctggagc tcgacatact gcttgcgtag ataccaaagc aatagggcga 26340

ccagcccgac cttgatgcca tagagccaac gcgtatcggc gtccaccaag ccgatccgga 26400

ctaccaagtc caccactgcc atgaagagca tatacaatgc aaatggcgct acacggctca 26460

ggatcggcgt atgtaaacgg gataaaaaca tatgttgcaa attgaattca attggtcatc 26520

atacaaaact ttggtcacaa atgacacaaa ttctcattta ggtagtctgt tcggactaaa 26580

aaaaataagt cggcatcaag ccgacttatc gaatgctact gagctactgc gatagattta 26640

tgacaaatcc ctgcttttac ggcggcgcgc caccaatccc atcaacccca tgccgatcag 26700

catcaatgca tattcggatg gctcaggcac agcagccaca cggaaaccgc ttagccagat 26760

ttcctgaaac tggcggccgc cttcgttgct ggaagcgccc tggatgatgc ctatggcatt 26820

gctgccaagt tcaaacgtgt agcgggaggc atccacgc 26858

<210> 174
<211> 26858
<212> DNA
<213> Methylobacillus flagellatus

<400> 174
gcgtggatgc ctcccgctac acgtttgaac ttggcagcaa tgccataggc atcatccagg 60

gcgcttccag caacgaaggc ggccgccagt ttcaggaaat ctggctaagc ggtttccgtg 120

tggctgctgt gcctgagcca tccgaatatg cattgatgct gatcggcatg gggttgatgg 180

gattggtggc gcgccgccgt aaaagcaggg atttgtcata aatctatcgc agtagctcag 240

tagcattcga taagtcggct tgatgccgac ttattttttt tagtccgaac agactaccta 300

aatgagaatt tgtgtcattt gtgaccaaag ttttgtatga tgaccaattg aattcaattt 360

gcaacatatg tttttatccc gtttacatac gccgatcctg agccgtgtag cgccatttgc 420

attgtatatg ctcttcatgg cagtggtgga cttggtagtc cggatcggct tggtggacgc 480

cgatacgcgt tggctctatg gcatcaaggt cgggctggtc gccctattgc tttggtatct 540

acgcaagcag tatgtcgagc tccaggggct agccgcgctg ggagccaggt cgtggttgag 600

cgcaatcgcc gttggtatgc tagtatttat cgcctggatc aatctggatg cgccatggat 660

gttgattggc gatcaatccg aagggttcga tccgggaagc ggaccggatg cccgtctgct 720

gatggcgaca agagtgatgg gggccgcatt ggtcgtgccc ttgatggaag agctgttctg 780

gcgttcattc ttgatgcgct ggattcacaa ccaacatttc ctcaatgtcg accctcggca 840

ggtaagcgtc ctggccttac tagtttcatc cgcattgttt gcaatagaac ataccgaatg 900

gtttgccggt ttagtggctg gcttggcata cggcttgctt tatattcgta gcggaaaaat 960

atggttgccg atcatcgctc atgcggtgac aaatggcata ttgggacttt ggatcatata 1020

taccggtaac tggcattact ggtaatcatt ggggaaacac ttctttgcgg ttatctcaat 1080

caactaacct aataaaaatt gctttggtcg ctgggctagg tatcgagctt gctggatttg 1140

tagtccctgc ttgtgcatta gagggggatg ttttaaagcc gtatgccagt cttacatatt 1200

catatgatga taatttaaga agattcgcta gtaagcaatt agctcaaatg agtactggga 1260

gttcagatac ctcagataca atgcttgtca aaagcgctgg tattgtgctg gatcatacta 1320

ttagtcgtca aaaaatgtat gtggatttta gattaaacaa gtccacattt aataaaaact 1380

cagagttaga taatgatggt cgtgacgtta ttgccaggtg ggattgggag ttgggaaatc 1440

gattggaagg caaggttgag ttttttcata aaaaagcttt ggttcctttt tcagaattcc 1500

aacctctgca aggaaccatg ttatcgttaa acacaagaac tcaggaccgc cgcttattgg 1560

aggcgcgttg gatgctgcat cctagatggc gtgttagagg ggcttttatt tactctgaaa 1620

tggagtacgg ggctgaggct caaagggccg ctaatctaaa ggaagatata caggagatcg 1680

gatttgacta tctatctcct actaaaagta tcatcggtgt tctgtacaga aatactaaag 1740

gatcaaagcc gcagcaagct tttcagggat cttttataag caatgattat gaccagaatg 1800

aattaaagtt gaatgtcgat tgggctgtta ccggaaagtc aaggttgcag tttcttggtg 1860

gtttggtaga taggcagcat gagcaactgt ctcaacggga ctttaggggg gttaatgctc 1920

ggggaaactt taattggatg acttctggaa aaaccaattt gaatgctgca gtttggagag 1980

aaaataacgc tcagtcattt gttacaacaa gttatacgct gaatcgtgga acatcattaa 2040

cggctaattt gcaggcaact ggtaagatag ctgttcaagg cgtattgaaa tatgaaaaaa 2100

gagattttga gggggatcaa atttttggtc aatttaggtc agataaagat aagagtttta 2160

tgctgtcact tatgtataac ccagtaacat cttttaggat aactacttca tttattcata 2220

gtgatcgaga gtcaacatct gatatgtttg aatttaggtc aaatagtgtc tcagtaacag 2280

ggcaatatga gttttgaaga gctataaatg acggtaaatg acataccttt agtcgctttt 2340

ttcaagcagc tgttagatcc tttaattatt ctggggactt tgtatggctt tacgcttctt 2400

tatggtgagc cattcaatgg ctactactta atccttgcgt tgcttgcata ctttgtatct 2460

tcaaatgtct atgagcgcct ggatctttac cgcacatggc gtagtggaaa agtgttgggc 2520

tatattcgtg atattttgtt cggatgggca attgttgtca ccatattggc atttatcgga 2580

tatgccgcag accttactga tcagtaccat gaggaaatgg tgtatacctg gtttgtggcg 2640

gtgccgatca tctttactgt tgcgcatttg ctcatccgca aaattgcctc ggatttgcat 2700

aaaaatggcg aagttcgttc tgttgtcata gtaggcgcta atgatatcgg tgtccggttg 2760

gtgaatcgca tcaatgaaga cccttacctg ttcatggagg tgaaaggtct ttttgatgat 2820

cgcgagatat cccggcatcc caagggaatg cgtcatgtac cgttgctagg cacaatggcg 2880

gacgtggcgg cttatgtgcg tgccaacaat acccagatca ttttcatcag tctgccgatg 2940

tccgcgcaac cgcgcatcag acagttgttt gaggagctgc aagacacgac agcatctgtc 3000

tattttgtgc ccgatatcta tatttttgat ctggtgcagg caaggtttga tgatgtggcg 3060

ggtgtgcctg tggtggcgat ttgtgaaacg ccgtttacag ggtttaatag cttaatcaaa 3120

cggactagtg acattattct tgcatccctt atcctgttgt tgctttcacc gatcatgctg 3180

acactggctt tggctgtgaa gctgacttct aaaggcccgg taatattcaa gcagcgtagg 3240

tatgggttga atggtgagga aatcattgtt tacaaattcc gctccatgac agtgatggaa 3300

gatggtgaca aagtggtgca ggcacaaaag cgtgaccagc gcctaacacc gattggtggt 3360

tttctcagga gaaattccct tgatgagttg ccacagttta tcaatgtgtt gcaggggcga 3420

atgagtatcg taggcccgag gcctcacgcc gtggcgcata atgaacaata ccgcaagctc 3480

atacgtggct atatgttgcg ccacaaggtg aagccgggaa ttactggctg ggcccaggtg 3540

aatggattga ggggtgaaac cgataccctc gaaaaaatgg aagcgcggat tgaatatgat 3600

ttggattatc tcaggcattg gtcgctggcg ttcgatctct ggatcatcat ccggacggtg 3660

ctggtagtgt ttaaaaaaga taatgcgtat tgatggtgtt ggctgaattt tgaggagact 3720

ggattttgaa tactgaatat ggaagcacaa taatgcaatg gtcgaaaatg acgaaagggg 3780

cgttggtcgc cttgatgatt agtatcgggc tgtcgggctg tggagataaa tctaatgaaa 3840

caaaggctag ccaatcgctg gcgagagtga acggcaagga aataaccgtc catcaactca 3900

atgaagagct tgctcgggcc aatgtccagc cagcgcagaa agaagctgct accaagcaga 3960

tattgcaatc attgattgac cgtcaactgt tggagcaggc tgccttgaaa gcaaaagtag 4020

accgggaccc gaacgtcatg caagccattg agcgcgcaaa atcccaaatg attgcccagg 4080

catacctcca gtctagggtg gctaacatag gcaagccgga caaaacagaa gttgagaatt 4140

tctataagca aaaccctcaa ctattttccg agcgcaagct ggtggaaatg gaacaaatat 4200

tgattgattc acgttatgtc aatgacgaat tcaagtctgc gctcggtagc ttcaaaaccc 4260

tgcaggaagt cgctgcgtgg ttggatgcta agggaatcca atatgaccgt ggagaagttg 4320

cccgcagtgt ggcagagctg cctccacaga tttctgagcg cctgaaggat gcaaggcgtg 4380

gccagctgtt tgtgatcggc atggaccgag gacgatgctg gtgctcatgg gatagcgtaa 4440

aggagagccc ggtatcactg gcagatgcga caccgcaaat tgagcaaatg ctcttgaata 4500

aaaagcgtaa ggaagcgggt gaggctgaaa tcaagcgact acgggctgaa gcaaaaattg 4560

aatatttaga caaatcggcc gaagtaccac aacaaccaga aaaagccgaa cagtcaaaac 4620

cctctagtgt cgatgatgat attgctcgcg gagtaagcgg actaaagtag taattctata 4680

aaacgtgtat aggtgtagtg ccatgactaa attgatcaaa tggtttgtaa cggtagtcct 4740

gacgattgtt gtattcaatg ttaacgcagc ggattcggat attgttcttg ggccggggga 4800

tgttctaagg atatctgtat ttgaacagcc tgatctttcc ttggaagtaa gagtgagtga 4860

gtctggcact atcacctacc cgttgatagg cgaagtcaga gtcggtggag aaacacctgc 4920

agctgctgaa cgcaaaattg ccagcatgct ggaaagcggt ggattcttga aaaaccctca 4980

cgtgaatatc attgttgctc agttgcagag tcagcaggtt tctgtgctag gtcaggttaa 5040

caggccaggt cgctacccac tggatagtgc aaaatctctt gcagacgtac tggcgttggc 5100

cggtggagtt gcacctgatg gggagatttt gattagcttg gttcgcaaga ttgaggggaa 5160

aagtgttaga gagataattg attttgctga aatgatgcgg gatggcgact taaataaaaa 5220

tatcttactt acggcaggag atattattta tgtagatcga gctcctagat tctatatata 5280

tggtgaggtt cagcggccag gacaatatcg tcttgagtat catatgacag tgctgcaagc 5340

actcgctgtt ggtggtggcc ttagtctcag gggaacggaa aaaggtatta aggtgaaacg 5400

gttagatgat gaaggtggtg ttaagataat taaagtaaag catgatgatt ttattcagcc 5460

taatgatgtt gtatatgtac aagagagtgt attttagttt gttttttgaa taacagctga 5520

ataatcaaaa aggtaatcta agatgagttt tatcagattt atttctatca ttaaagctag 5580

atataaaatt attattttta ctttatttat aacggtatta actaccgcta ttgttacaat 5640

attgttgcca aaaaattata aagcaacagc ttcagtgctt gttaattata aaggcgctga 5700

tcctgtaaca ggtgtaatgc ttcctgcgca gttaatgcca ggttatatgg caacgcaagt 5760

ggatattatt tcaagtaaaa acgttgcaaa aaaggtaatt gataaactag gtctggcaga 5820

tgttccagcc gtaaaagagg acttttatca agcaacttca ggtaaaggtg aaattaaaga 5880

atggcttgca gatttattgc taaaaaaact tcaagttaaa ccatctcgag agagtagtat 5940

tattgatatt agcttttttg gggtaaatcc ccagtttgca gctacaatcg caaacgaatt 6000

tgctgagtct tatatgcaaa cttcaataca acttaaagtt gagccttctc aaaaagctgc 6060

tttgtatttc gttgagcaat tgaaagaact gagagaagat ctagctcaag cgcaatcgaa 6120

gctttcagag tatcagcaag agaaagggat tgtaagtgtt gatgaaagat tggatgtgga 6180

aagagccaga ttaaatgagc tttcatctca attggttatt gcacaagcac aagttatgga 6240

agcgagatct cgggaaaaaa attcaagtgg aaaagaagct tatgagtctc ctgatgtagc 6300

agctaatcca attatccaaa atcttaaaac tgaaattgct agagcagaat caaggtttgc 6360

agatgtttcg cagaaatttg aaagaaacca tcctttttat ctaggagcta aagcagaaat 6420

tgaaaatctt aagtctgagc tagatagaca gatcaaatcg gtttcaagta atgtggcaag 6480

taattcaaga attctgcaac aacgtgaagc ggaaatacgt ttggctttaa ataatcaaaa 6540

agaaaaagta ttacaactta atcgagatag ggatatttta tctgttttga caagagaggt 6600

tgaaaatgct caaaaagcat acgacatggc tatgcaaagg tatactcaaa cgaatattga 6660

aggtcagtca aatcaatctg atatttcttt attgaatcct gccgtggcac ctcttactcc 6720

cactagccca aatttattgc tgaatattat attgtcaatc ctcttgggta tgtttttagg 6780

aatagggttt ggagttttag ctgagtttgt tgatcgaaga gtacggactg ctgatgactt 6840

aattgaagta ttacaagccc cacttcttgg gactattagt aaagaaaaaa gaaaactgag 6900

attttttaaa aatacttcaa acctgataac tttagggaag gctgaattat gatttcattt 6960

acaccttcta attcaaactc tgaaaaagta gaaaatgcac cattggtaaa cgaattttca 7020

gaatttacaa ttggtagttt attattgcgg atggggaaaa ttacacccga agatgctgaa 7080

agaattatgc ggttacataa agagaagggt ttgcgctttg gagaagctgc tcaagcatta 7140

gggttgataa ctgaagatga tattcaacag gttttatcga ggcaatttaa ctatccttat 7200

ttatcacctg accaaaactc atatcttaat gagctcgttg ctgcatatca gccttttagt 7260

aacaaagctg aggttttgag agcagtacgc agtcaattaa tgctgcattg gttttcaccg 7320

gaacgaaaag cactagctgt ttcttcagta agcagcagcg ataatgcaag ctcttttgtt 7380

gcaaatcttg cagttgtttt tgcgcaattg ggtgaaaata cattattgat tgacgctaat 7440

ttacgctacc cacgccagca tgagatatac aatctgagta acaaacgtgg gttatctgat 7500

atcttagcag gaagagcggg gtatgatgta atttcaaaaa taaatttttt tgaaaactta 7560

tctgtattaa ctgccggtac attacctcct aatcctcaag agcttgttag caagccaaca 7620

ttcggaaatt taattaaaga gttggcttgt gtttatgatg taattttatt ggatactccc 7680

gaacttagta attattcaga tggttatgca attgctttta ctgttggagg aattgtatta 7740

gttgcaagac aaaatcatac tcgagtgaat gatttatcta ttgctgtaga acaatgcaag 7800

aataatggta ctaaggtaat tgggtcaata ttggttgatt tgtagatatg aacactgtcg 7860

aggtaatcga gcaagataat cctggtatct gggaggtaat caatcggaat tggatgctat 7920

tattaggttt gttcattatg tatacgccta catatttgaa cttattaaat actgtatggc 7980

aatctgaaga tcaagctcat ggaccattaa ttgttggcgt tgtcttgtat ttagtatggg 8040

tacagagaca ccaattgttt aaggccgaaa ataatattat aggcaggcca gtaattggcg 8100

ggatagtttt agtttttggt cttttactct acattcttgg ccgttctcaa gatattgcac 8160

cgttagatat tgcatctcaa attcctgttt ttgctggatt gttgctaatt ttccgtggtt 8220

attctgggct tcgagcattg tggtttccgt tattcttctt gatttttatg gtgcctcttc 8280

ctggagtttt tcttgatgct gtgacattac caatgaagat tgcggtctca tatgtagcag 8340

aaaatatact ttattggata ggatatccga tttcaagaag tggggtgatc ctccaaatcg 8400

gacaatatca attacttgtt gcggatgcat gtgctggttt gcacacttta atatctttgg 8460

aggcgctggg tttactttat ttaaatgttg taaagcacga ttcatttatt cgtaatgtaa 8520

ttcttgggat attgattatt ccaatttctt ttaccgctaa tgtgattaga gttatggtat 8580

taactttagt tacttatcat tttggagatg ctgctggtca gggttttgtt catggttttg 8640

ctggaatgtt actttttgta gtggctctaa ttttgataat tagtgttgat acgctcctac 8700

aatatttggt taaaaaatgg cagttaaaag cctaaactct taataaggcc tgtctaatgg 8760

atggatttac actgcgaaat ttaattatat ttttactgtt attggtagct actttaggag 8820

gagtaattct taaacctact aagtttgtta atgcagcaga agatataaat ttagaagcaa 8880

tgattcctaa ggagttcgat gagtggaaaa tcgatcctac gattactcct atacaagtga 8940

gccctgaggt gcagcaaaac ctcaataaag tttatgatca aactctttct cggacctata 9000

ttaataagca aggaatgcga ataatgcttt cgattgctta tgggggagac caaagtcgcg 9060

gcttgcaagc acataagcct gaaacctgct atacagctca aggatttaat gttgatttgt 9120

tgggtaatgc cattttaaat tttgatggat atgaaattaa agttagacat ttattagccc 9180

agcatggggc acgatatgag ccaatacttt attggatgcg tgttggtgaa actacagttt 9240

atggtggatt gggtcaaact ataaaaaggt ttgaattagg tttgcaggga tatattcctg 9300

atggactgct ttttagagtt tcaagtatta gcagagataa tatctctgct tttacactgc 9360

aagaagaatt tgttaaatct ttactgaaaa atgtagatag taaaagtaga aaaaaactta 9420

taggtacatt ttcataaaat tcaatgtttt tttaacctaa ctttaataat aaaaatgtag 9480

caatttatga ttaaacatac cgcatttatg gctctttcga ctttttcacg gttgctatca 9540

gcggttgtac ttttcattat tcttgcaaga ttccttggtc cgagagactt tggcatattg 9600

atgtacaatt ttactcttgc gtcaatagct gtgcttttag tggaatatgg attttcaaat 9660

cagttgttaa gagatatagg aagataccct gataatatta atcagttaat ggggagggtt 9720

tttcttgcaa aactatatct cacagtagtg atgggggtct gttgccttgc tggacattac 9780

ttattctata caaatgactt agatagagca atattttgga ttttactgtt atcttgtttg 9840

ctcacttctt ttggggattt ttttaatatt gcattcagag gtatcggaaa atttcatgaa 9900

gaaacaaaag ttgcaacaat tggctctttg ttgcatttcg gcttaatagt tttaatggta 9960

attaatgggg ctgagttatt attaattagt ttaggttttg ttttatctcg ttttgtttat 10020

tttattttgt cgtggcgggc atataagcga gtggttggtg atttaaatac tctgtcagcc 10080

tctccagttg ctgttctaaa agcattaaaa gcaggatttc catatgccgt tgatgcgggt 10140

ttcactaact ttttctacca agttgacacc atgcttgtga aatattatct gggagccact 10200

ggagttggta tttaccaagc aggtatgaga tttctaaatg gtgcaatgca atttgcacct 10260

gtactcggaa atgtattttt acctgtaatt gctgccaagc acactaatac tagagagttt 10320

gttgagctag ctaaaaaact taatcttcag atgcttgctg ttggtactgt gtgctgggct 10380

attttctggt ttgccggtat gcatattacc gaatttgttt ttggtgatga atatgtagag 10440

ttaaattcaa tttggcccta tattggtttg ttagttttta caagatatct tgctgcgcct 10500

caaggtgttc tattgactgc ttctggatct cagacaatac gtgtctgggc acaaattttt 10560

gcacttatta ttttgctaat ttcagcacca cttctgatga aagtgattgg actaaaaggt 10620

atggtcattg ctttaatcat cacattatta accttgtttt tgatattttt cagtgcatta 10680

cggcttagaa aaatacctac tgggtttggg ggagttaatt ttactttgac tgtaatagta 10740

tttagtcttt ccatttttct tttggtatat tagtcatgtt gaaaataggc ttactttggc 10800
attctactac ttcagataat cttggtgttg gcgctttaac tgaagcacaa atttctatta 10860
ttaataaagc tgcggtgcag gcgggggttg aggttgaata tcgaattttc ggcactaatg 10920
gaaacaaaga ttactttgag ggcaaccctt taattgagcg agggacaaat gtatcactca 10980
aaagaatttt gatcggggct tctgattatc ccaagaaaat ccgggaatgc gatgtggtgt 11040
ttgatatagg tgaaggtgat agttttgctg atatttatgg tttttcacga ttcctaatac 11100
agattgtgtc taagttggtt gtcttgtttc aacgtaaacc tcttatttta agcccgcaaa 11160
ctatagggcc attcgaaagt aggtttaatt taaaggtggc caataatgta atgcagcggt 11220
gcactaaaat atttgtaagg gatggattgt catttggata cttgcaaaag aataatttat 11280
ctgtaaatgc agaggaggta attgacgttg cttttagatt gccatttcaa cgcccttccc 11340
gggattcaag taaagtaaaa attggactaa atgtatcagg attactttat aacggtgggt 11400
atactgggaa gaatcaattc ggtcttagtg ttaattatcc tgaacttact agatcactat 11460
tagatcagtt tactcgaata gaaggttgtg aagtatggct tgttcctcat gtgctaacga 11520
cctcatttcc tatagaagac gatgtgaacg tgtgtgttca attacaagag ctttatccag 11580
ctgctaagct tgcaccatct ttttcttctc ctagccaagc taaatctttt atttcaggga 11640
tggatttttt tcttggagct cgcatgcatg cttgtatcgc agcattttca agtggagtgc 11700
ctgttgtgcc attagcgtat agtaggaagt ttaatgggct tttttctgcc ttggattatc 11760
cttatcttgg ggattgtaag gttaatacta acgagcaaat agttggtttg gttatggatt 11820
gttttaacag tcgggagcag ttgaagtttg cagtagaaga aggtaataaa attgcagaaa 11880
aaaaactatt gcgttatgag cagtacatag cagaattatt aactagaatt tcaaataaaa 11940
agattgtttt ataagttaaa agtcaaattt actaagttga acacatattt tagagaatgt 12000
atagttgtaa gatgaaaaaa tacataattg gtttcttgcc gctttctatt aaatggcttc 12060
tgagattttg tcttcgaggc ccgagtttat tgagagactt ttgttacgat gctaaacgat 12120
atattaaatg gtcatctgta atttctttcg agaaagatcg tgaaaagatt cttgcggtta 12180
ttactgctac ttatcacaac atcgagaaag gaatgtcttt acctaatccc aggccgttat 12240
ttggaagggt agtgattgga caattagttg gatcagtcga taattttcat cttaattttg 12300
ggcctgatcc acagctagat gttccgataa atgtactggc gcgttattgt caatttaata 12360
gaagtcttgg tcagccagat tctcaattag aagtaatcgt tgatcggtta aagaaaaata 12420
atcagagcac tttattaagg attaatgatg gcggggttat tttgcgctct aaagctgagg 12480
ttttagacgc aatatctggg gttaaagaag atttttttct tatgcgccat agtgtaaggc 12540
agttcactaa agatcctata gagcatagtg taattgaaaa ggccgtacgt attgctcaaa 12600

aaagtccagc agtttgtaat cgacagtctg gaaaggtccg ggcaattctt aatccagaat 12660

taattaggag tactttacaa attcaagggg gcgcacgagg gttttatgaa gaggttcagg 12720

ctttattttg tataacagtg gatttgtcaa attttaatgg ggcggaacgt tatcaaggtt 12780

ggattgatgg gggaatgttt gcaatgagtt ttatatatgg cttacatttg caaggcctag 12840

gaagttgcgc tcttaactgg agtaaaacta ggaaacaaga tatagaaatg aggaatcacc 12900

ttcgtatacc agaaaacgaa ttaattatca tgtttgttgc cgctggatat ttaagggaat 12960

catttgttgt tccgcaatca taccgaaaac ctttaggtga agtcttgagt tatatttaat 13020

atatattaaa tattaggtag aaggtaccat taaaatttca caaggctaat gcgtcattat 13080

gaagaacaat tcttctctct tatcttttaa ccgtagggat gctaatttcg ttagtttaaa 13140

gatagggagt atattttttg taataatggc tgcaatagtg gctttgatat ttggttttgt 13200

cagtgctatt tttccatggt ggtggatagt agccattatt ctttctatat tatttatttt 13260

tattggtttt acatatcctt tagtagcttt aatagctgtt atagcaattt cttctagatt 13320

aattccagat acatttttgc cttcaataag actgctggga ggcacaatac catcttcaga 13380

tatggccttg tttgcaacat tcattttttg tttttttaaa tacaattcag aatttagctt 13440

tacaaagcaa gcattgtcac ctttggctat tcctttattt gttgtacttt tactgttgct 13500

tttttctgta tttcgggcgg tgtttgaaat tagattgcca ttgaaagata ttttagggga 13560

gtcaaggcat ttcctttatt ggctattgat tcccttaata gctttaacat gctcagatag 13620

acaaagattt aaatttattg tttttggctt tctttttgta gcttttttat tttcaattgg 13680

ccagattttg caaggttttc taggtgtaaa tgtgttcggg cgcgagctta ctagccttga 13740

aactttggga agcattaata gtgatgttac acgaagcatg acgtcaggaa ctcacattat 13800

tgtttgggga tttttgtttg tactggcttt gtttgctaac cgaataggtt tttcaaagac 13860

tcttattata ttgtgtatat ttacaggcgc tggaattttc ttaacctatg gtcgtgctgt 13920

ttgggggggg actttcttag ctgttttgtt cttatgctgg aagctaggga caaaaaaaat 13980

gcttagaagt gtaggactaa ttgcaattgt tgctgtattt ggtttaagtg ctatgctagc 14040

tattaagccg ggcgctattg aaggcgcgat gcagagattg tttagtgtgg aggaggaggt 14100

ttctcatgga tcttctctcg gctggaggtt ttatgaaaat aaaatggcat gggcggcaat 14160

tcagcggtca cctttattag gcattgggct tggtgcccct tataggccgc ctgctaaatc 14220

tgatgtgaca gcagagcaag tgagatatgt tcatagtgct tatatctatt taaccttaaa 14280

aacaggcatt attgtattca tgtcagtcat ttttcttata ttcaaaatac ttcatttaac 14340

ttacattcaa acgcggtcta atgatttatt tatatcaagt gttgccatag cttcttttgc 14400

tgcaatgata gagtattttt tggcctgcat tactcagcca gaattaatgc aaaccccggg 14460

aattgctttt ttggctactc ttgctggtct tatgtgcgcg gctaattatc ttaatcaaaa 14520

aaatcttaca aaaggaaagc taacttctta aagtgtaaat tggaatatat atatagtcat 14580

aattatataa aataatatga gaacttaaag atgaatgtta aaacatctcc attaattagt 14640

attattacgg caacatataa ttcaatgaaa tattttgagg atactaaaaa aagtgtcctt 14700

gaaggagatg ttgaagactt tgagtggatt atcgttgatg atggatccac tgatggaacg 14760

cggaaatatt tacaagaaat tgatcatcca aatataaaat tgcattttaa aagtattaat 14820

gctggtattg aagactcata tagaactggt attgaattgg cgaccggtaa atatttgcta 14880

attttagatc acgatgacac tattccattg ggatctttgt caaagcggct tgacttactg 14940

gaatcaaatc ccaatgcttt ggctgcattt ggtgctgtgg catatatgga tgagtctggg 15000

aggatttata aagaatcacg ttttcctttt gtatcagatg attgcgtact gtcatcacta 15060

tttactttat tcggaatctt tattttacca gtataccctt taaagcaagg gtgtgtggtt 15120

ttgaggtctt ctttcgttaa aaataatcca gggttatatg acatacaact attcctgcaa 15180

gctgctaaat tgggcccagt aatatttatc aagcaagcat gtttaaatta tcgaacattt 15240

agaacgcagt tttcaagttc tagaaaaatg cgattgattc gtttttttca atttgtttgg 15300

gcaaaatatg cgtttaaatt tctcccgtgg tatataagcc cgttctttgc tatttataaa 15360

acaataccag agttctttaa agtattatgg agttttgttt cttcaaaacg gatttaaaaa 15420

tatgttaacg attctttatt catggttgca atttttattg gtgcttattg atgacggatt 15480

ttagttcaaa aaaaaagtta gacttaagtg ttgcaatagt ttttgttaat tataatacta 15540

gtgaccaaat tatttcttgt atttactctc ttagggaaga gagatattca aagattattg 15600

ttgtggataa tgcatctccg gatgattgtc ctgagtgcat cgtagaagag tttccagatg 15660

ttgtacttat taaaagtgaa aaaaatcttg gctttggtga gggatgcaat attggaaaag 15720

attgggttat agttaataca gattgtgaat atatattttt tcttaatcca gacacactag 15780

ttgaacctgg attattacaa ttactgcatg atggattttt tagccctgaa gttggattgg 15840

ttaccccaag aattactatg atgggtgatg ggttggatat actttggtat ggtggggggt 15900

atatgaactg gttaactggt tcggcacgcg taccaggttg tggattaagt gctgtatcaa 15960

aaatagctct agaagatcga tttgtagagt ttgccagtgg ttgcgctttt atgattagac 16020

gttcagtttt agaagagtgt ggcgggttcg atccacgcta ttttatgtac gaagaagacg 16080

tcgaattatc tttaagagta atttcttatg gatataaaat ttattatatg ccaaaagctg 16140

ttgttcgcca tctggctcag ggtagccagg gcaaagaggt agaggctgtt ggtatgttta 16200

aaggaaggaa tcctaagctt cctttttttg tttatcaaac tgcgaaaaat agatttttaa 16260

ctgtttttac tcatggaact gtgcttaaga aaggtctatt tttaataggt tttatgttat 16320

gggtgtttcg aaactctttt gaatgggtca gatataagag acttgatgca tataatgctt 16380

tatttttggg cattaaagat tttgttaaat acacatatgt atcaggtaaa aaataattat 16440

tcagattggg tgttatgtga aagaaattaa cgtcgccttt gtggagccag ttggtagtca 16500

cggtggtatg gactattatg attctggact gtgtgccggt ttgttacatc agggagtatc 16560

tgcaacttgg tatacctgtg acatttctaa accttgtggt aatagaccta ttttcatgca 16620

acttactttt gtaaatattt ggggaaagga tgctacgtgg agaagagggg ctaggtatat 16680

cttagggctt tgtaggagca taaagcatgc taaaaaaaat tattcaaata ttgcccattt 16740

tcattttttt catgtgggtg ttttagaatt tttaagtgta ttattttttc gcttatttgg 16800

atttaaggtt gttgctacag tgcacgatgt tgagtctttt aagccgggtc taaccttctc 16860

tatcttgtta aaatggactt actttctctg taatcaatta attgtgcaca atcaagttag 16920

taaggatgag ttgattaata gaagtaatgt ttctataaat aaaatacatg taattcctca 16980

cggaagttat attggtctgg ttgcaccctc gatgccaaaa gcagatgctc gtcaagctct 17040

taatgtatca tcagatgaaa aggtaatttt attttttggt caaatcaaag aagtaaaagg 17100

tttggatttg ttgattgaag cgttaggttt ggttaaagat aaactaaaac catttaagct 17160

agtcattgct ggaaaagtgt ggaaagataa tttctctaag tatcaagaat taataagtaa 17220

aaacgggcta aatgattttt gtaaattgga tattagatac ataccagatc aagagatatc 17280

aatgttttat agtgcggcag acttgattgt tctgccttat agaaaaatat atcaaagtgg 17340

tgttttatta atggcaatga gttatggaac tccagtgtta gcttcagacc ttccgggtat 17400

ggcgcaaatt ataaatgatg gggaaaacgg attttgtttt tctgctggaa attatattga 17460

tcttgctgaa aagcttgtaa aaattttttc ttaccatgac ttatctagga ttactttgca 17520

agcagaaaac gatatgagaa ctcgcttcag ttgggatgag attgcgataa aaactatcag 17580

tgtttatcag aaaagtatat cgtaatgtgt atttgctaag attatggtaa acgggtttga 17640

tctaaaaata ttttgcggtg atgtttagta aaattcttaa tggggttagg attcttgatt 17700

ttttcatctt ctgaattgtg atgtcagtaa ataggctctt ttgtatgtta tatttatttg 17760

gattattttc cattgctata gcttgatagt gattatcttt gactttaata aggtatgagt 17820

tttttaattt aacttcggga gtaatcttta tatattaatt tttataggtg tcaattgtaa 17880

aaaataaatt ttaaagctaa taataaaatt agctaaataa ttagattatt ttgccgttaa 17940

taataggtgt ggatttttat ttacagtgtt tttttgaagt ggcgtgattt gatcgtatct 18000

agcctggagg tccataacta tgcaattaat taactctcga aggattgttt tacttagttt 18060

gttttccctt atgcctttgg ttggtattgc tgaagtttat aaatctacag atgtagacgg 18120

gagtaccact tattctgata cccctcaagc taattcacaa aagataaagg tttttccttc 18180

tgattataag aagaatgtta aatctgaggc tgcagctaat aaatccagaa gtaattctat 18240

ttctgctgct aatgttacag aagctcctat tgatgagtct agtagtgatt ctaatgtaac 18300

tataaagcat tcaacatcta ataaatctat caattcaagc aatccttctg ctgctggtgg 18360

ctcttcttct gctggtggct cttcttctgc tggtggctct tcttctgctg gtggctcttc 18420

ttctgctggt ggctcttctt ctgctggtgg ctcttcttct gctggtggct cttcttctgc 18480

tggtggctct tcttctgctg gtggctcttc ttctgaaaca actacagctg cttcggtcga 18540

taaggtttct gcggctttgc aaggaccgaa agcttttctt aattcagacg attttaaaga 18600

aaattcttat cgaggtttta atgttcttgg gcggaaatta agttctgctg acatggaaga 18660

tcttgaaaag tttaacgtca atttaattcg aagcgttatt catttggaaa aatgtaaaaa 18720

atgtaatgat tatgaaattt ctccagttga gttgtcgcaa atggattttc ttgtggaggc 18780

aggtcgtaag ttgggatttt atgtaatttt tgcttttgat cctaaacctg ctggagatca 18840

ggcgatctac tggaataatc aacaattacg agaaagcatt aagaataact gggtaactat 18900

tgctaaaaga tacaaaaatg agactgtggt ggcagggttt gacctgatta atgagcctgt 18960

tgttaggcct ggatcagtat gggaaaagta tgctactgaa ttgattactg ccatacgtaa 19020

agtagatgaa aatcatataa tttttctgga acctagtcct tgggggctgg cttctagttt 19080

ggagcagttt actcctgttc catttaataa tatagtttac agctttcatt tttattctcc 19140

ccaagaactt actcatcaag gattggcggc atacccacat gcatatatat atcccagtga 19200

aaagtgggat aaagaatacc taaataatcg actagacatt gctcgtaatt gggctaataa 19260

atataatgtt cctctttata tcggtgaatt tagcattatt cggtgggctc cgtctggttc 19320

gaggttacgt tacttgacag atgtagttga gcgctttgag caagagaaat ggtcatggac 19380

atatcatagc tatagagaat atagcggatg ggatgcggaa atgccagata gtatattcaa 19440

gaatgatgct attgtcgaac gtcaaaacaa tgttcctgta cttcagcttt tgatgaaaaa 19500

ttttaataaa aataagaaat attagaatca catttaacat gagtttgtat ttgtatgcat 19560

aagaaaatag caatattagg aaccagagga attccggctg cgcatggtgg ctttgaaaca 19620

tttgcagagc atttggctat ttatcttgaa agaaaagggt ggaaagtaac agtttattgc 19680

caagtagatg gtaatgggcc tatgtatgag gatatgtgga atggtataaa gcgagtaaat 19740

attccgatat ctatccaggg ttctttaggc actattcttt ttgattggaa gtcaacgatt 19800

catgcttcaa atacttatca tcttatttta actcttgggt ataacactgc tttattctgt 19860

gggctctata gaactaaagg aataactaat ctaattaata tggatggaat cgagtggcat 19920

cggaaaaaat ggggggcagt tgctaaactc tggttctgga ttaatgattg ggctggttca 19980

attttaggca atcatctgat tgctgatcat ccgcaaattg cgcgtcatct ggaaacaaga 20040

gtcaggccta gcaagatcac aatgattcca tacggcgcag ataaagtgat tgaggcgaat 20100

caggccgttt tggaggaata tggacttaag cctttcagtt atgcgatctt aattgctcgt 20160

gcagagcctg aaaattctat tttagaggtg gtgaaagcct ggtccgctaa acctcgaggc 20220

atgaggcttg tagtgctagg aaaatataac cctgaccatg cttatcaggt cgcggttaaa 20280

gctgccggct cagacgaggt gctttttcct ggcgcaattt atgataaaca cgtggtcgaa 20340

tctttacggt atttctcaag attatatatt cacggccacc aagttggcgg caccaatcct 20400

tctttggtgg aggcactagg ggctggtaat gcagtattgg cgcacgataa ccgttttaac 20460

cgctgggtcg ctggagataa ggcggcctac ttttctggtg aaaaggactg cgaaatcact 20520

cttgatgagc ttcttaatga tgaagttcgg ttacgtgaga tgagagccgg tagcttggct 20580

caatacgata ccttatttac ctgggactca gtgttgcagc aatatgaggc cttgctgaca 20640

aggtttttgc caaataagta attagatact gtgcctattt ctttcccttt atatttctat 20700

ccagtgagaa aattatgcag attcaagccg tgattttatc aggtggctct ggcacaaggt 20760

tgtggcctct ttctcgagaa aactatccca agcaattgct gacattgctt gataatgatt 20820

ctttacttca gtcgacatta cgcagggtcg atccactcat tggaaccgag tgttctccaa 20880

ttctggtatg taatgaagaa tatcgctttg ttatcgctga gcaattgcgg atgatgggaa 20940

ggcgtggatc tattctcctt gagcctgtag gcaagaacac tgcccctgcg ctcaccttgg 21000

ctgccttaga ggctgccagc cacggccagg atccaatctt acttgtcatg cccgccgatc 21060

acgctattat caatgtgcaa gactttcagg ataccgtgag gacaggggct gaacatgccg 21120

ctgctggtaa gatcgtcacc ttcggtatcg tgcccgatag ggcagaaact ggctatggtt 21180

atatcaagtc aggcgcagtg ctggaaggtt tcggttctgc tttcagtatt gagcagtttg 21240

ttgagaagcc tgatgaggct actgctcagc aatatgtagt aagtgggaat tatttctgga 21300

acagcggtct gttcatgatg cgtgccacgg tatggctgaa tgcgattgct aagactcgtc 21360

cagatatctt atcttcatgt aagcaggctt gggatggtca acaacgcgat ggcgattttg 21420

ttcgggtgga caagcttaaa tttcaacagg tacctagcga ttctatagac tatgcggtga 21480

tggagcatgt cgctcgtggt gccgataatc tgcctgcagg catagtcatc cccctgaatg 21540

ccggctggtc agatgttggg gcctgggatt cattcgggaa aattttgccg caggataacg 21600

cgggtaactc ccatttagga gatgtgctgt tcaatgattg ctcgaatact ctagctgtgt 21660

cttcaggtag gctgcttgca tgtgtgggtc tgcaaaacat cgcggtgatt gagacagcag 21720

atgcggtgct agttatagat aaaagtaaaa ctcaggatgt gaaaaaaatc gtggagcagc 21780

ttaaggtcca gaaaaggccc gaggccaaga ctcaccgcaa ggttcaccgc ccctggggct 21840

ggtatgacag tattgatatg ggggcacggt tccaagtaaa acgtattgta gtgaatccag 21900

gtgctacgtt atctcttcaa atgcatcacc atagagccga gcactggatt gtggtgaccg 21960

gtacagcgaa ggtaacaaga ggagatgagg agttccttgt ttctgaaaac cagtctactt 22020

atattccttt aggtgtgacg catatgctgg ctaatccagg aaagcttcct ttagaaatga 22080

tagaggttca gtctggggct tatttgggtg aagatgatat tgttcgcttc aatgatcaat 22140

atggacgttc atgaataatt tcccttggtc tgactgaagt cagttttaat gattaattag 22200

taataatgct aatgattttc gaccgagaat gttgctaagt gggttactgt agatcgcaaa 22260

gccaattccc atggtgctaa ttcagttttg aaagtgctca tgaatttttc agtgctcatc 22320

ttggagttgg cggggcgatg agctggtgtg gggtattctg cagttgtaat accgacaaca 22380

ttttctacag tggattttaa tttcgctaaa tttttctttt gggctatttc tccatactgg 22440

cgaatgattt ccagtgcaaa gccatgccag ctcgtctccc cagtattgac taagtgatag 22500

acacctcttt gagtcccatc ccatgcgtgc agcgcagtaa tagttgcatc tgcaatgctt 22560

tcggtagaag ttggtgcgcc aaactggtct gcgactatat tcaatgagtc acgctcggca 22620

gctaatctca atatggtgcg catgaaattt tttccatatg caccataaac ccagctggtc 22680

ctgaagatta gatgatttgc tccgacttct tgaatggctt tttctcctgc taacttgctg 22740

gctccataaa cgctaagtgg atttgtttca tccaattctg tgtaaggggt agttttggtg 22800

ccatcaaata cataatccgt agaataatgc acaagcaatg catttagctt tgctgcttct 22860

tcagccagaa tgccgggagc agtgccattg atggcgtagg ccagctccgg ctcagattcc 22920

gctttatcca cagcagtata agctgcaggg ttgattatga ggtctggttt ggtctgtcgc 22980

actacgtccc ggatatggtc aggtttggat aggtcaagtt ggctgcggtc aagtgcaacg 23040

acttctccca agctttgtag tttcggatag agagcatggc ctacctggcc gttgatgccg 23100

gtaagcagga tcttgttgaa agttgccatt atgcgaataa ctctgtctgg tggaggccta 23160

agccttgttg atctttactt gagaggagcg ggggcgtgcc aatatctggc cattcaatac 23220

caacaacggg atcgttccag cttaggcaac gttcatgcgc aggcgcgtag aagtctgtgg 23280

tcttatagag aaactctgca gtttcggaca gcacgaggaa gccgtgggca aaacctggtg 23340

gtacccacat ttgtttcttg ttttctgctg acagatgttg ccctacccat tgaccaaagg 23400

ttggagaaga tttgcggata tccacggcaa cgtcaaaaac ttcacctgct actactctaa 23460

ccaattttcc ttgggcttgc tggatctgat aatgtagccc tctcaatact ccttttgagg 23520

attttgagtg gttgtcttgt acaaaatcat atttcctgcc gacggcttcc tcaaaggctt 23580

tctgattgaa gctttcaaaa aagaatcccc gggcatcacc gaatatcttg ggttcgaata 23640

cgatcacatc aggaatatgg gtgggcgtga cattcattaa aagacctttt ctttcagcat 23700

ccggatcaaa tattggccgt aaccattctt gaggtatggc tgggcaattt tttctacttg 23760

ttctgcatcg atgtaacctt tgcggtaagc gatttcttcg gggcaggcga ctttcaggcc 23820

ttgtcgcttt tctatggttt cgatgaatag tgaggcttct agcagggatt catgcgttcc 23880

tgtatccaac caggcatgac cgcgccccat caattccaca ttgagggagc gcctttccag 23940

gtaaattttg ttgacatcgg taatttcaag ttcgcctcga ggagatggtt tgaggctggc 24000

ggcaatgtca acgacctggt tgtcatagaa atacagtcca gtaacggcat agcgggattt 24060

tggctctttg ggcttttctt ccaggctgat agcctggcca gcatcgttga actcgaccac 24120

gccataacgt tcagggtcgt gaacagggta ggcaaataca gtggcgccat gctgccgttg 24180

atcggcatgt gccaggtcat gtgccaggtc atgaccatag aagatgttgt cgccaagcac 24240

cagggcactt ggatcattgc caataaattt cttgccgatg ataaaggctt gggccaatcc 24300

atctggggag ggctgtactg cataggcaat attgagtccc cattgactgc catcacccaa 24360

tagctgttca aagcgtggtg tgtcctgcgg agttgagatc aggaggatgt cgcgtatccc 24420

agccagcatg agcgtggaca agggatagta gatcatgggc ttgtcataca cgggcagcag 24480

ttgcttggag actgcctggg tgacagggta aagccgggtg cccgatccac cggcgaggat 24540

gatgcctttt ctcatgctgc gctcccgcta tattgcttgc tgacccagtt gcggtattct 24600

ccactggtga cgttgtgcac ccatgcctgg ttttgcagat accattccac ggtttttctg 24660

atgcctgact cgaaggtttc cgcaggcttc cagcccaact cgcgctccag tttgcttgca 24720

tcgattgcgt agcgccggtc atggcctgga cggtccttga cgaatgtgat ttggtctttg 24780

tagcttgagc catctgcctt ggggctgagt tcatccagga tgctgcataa ggtctgtact 24840

acatcgagat tggccttttc gttccaaccg cccacattgt aggtttcccc gagctggcct 24900

gcttccagta cgcggcggat ggcgctgcaa tgatccttga cgaatagcca gtcgcggatt 24960

tgctggccgt caccgtaaat aggcaatggt ttaccagcaa gcgcattgag caggcatagt 25020

ggaatcaatt tttcagggaa atggtaaggc ccatagttat tggagcagtt ggtggtcaat 25080

accggcaagc cataagtgtg gtggtaggca cgcactagat gatccgaggc tgccttggaa 25140

gccgagtatg gactattggg ggcgtaaggg gtggtttccg taaacgcttg gtccttgggg 25200

cccagtgtgc catacacctc atctgtagag acgtgcagga agcggaactg aggatttctt 25260

ttttcctctg ggtgggtttt caccacgcta ttccagtagg cgcgtacttc ttccagtaag 25320

tggaaggtgc cgactacatt ggtctgaatg aagtcttccg ggccatggat ggaacggtca 25380

acatggcttt cggcggcaaa gttgatgact gcacggggtt tgtgttctgc cagcaacttg 25440

gcgaccaagg cgcggtcacc aatatcgcca tgtacgaaga tgtggttggg gttgccttca 25500

atgtctgcca ggttttcaag atttccggca taggtcaatt tgtccaggtt gatgacggtg 25560

cctagtcctg ccttgaccca gtcgagtacg aagttgccac cgatgaagcc tgcgccacca 25620

gtgacaatga tagtgttatt taatgtcgtc aatctattaa ctcacttaat atctggatta 25680

tttgcgggtg tggtgaaatt attatttact ggtttatatt acaagaaggc tatcacagaa 25740

tggtagctta actttctaat tcagattttt ttaaaagttt tctatgttta taaagctact 25800

ataaatatgt gtggcgcttc aatagggcta ttggactaaa ggaaaagttt ttaatgttat 25860

tagtaacagg cggtacaggc tacataggtt cccacgcatg tgtggaactg attaatgcgg 25920

gctatgaggt ggtggtgctg gataatttca gcaatagcca ggcgcatatc gttgaccgca 25980

tcaagcagat cacgggcaag gcacccgtat tggtggaagg ggatgtgcgg gatgaagcac 26040

tactgagtga tttattttct aggtacccat tccaggcagt catgcatttt gccgggctca 26100

aggcggtggg ggagtcggtc gcgcagcctt tgcgttatta cgataacaat gtggcgggta 26160

gtgttacctt gctgcgcgtc atgcaggcgc atgacgtcaa acgattagtc ttcagttcgt 26220

ccgctactgt gtatggcgat ccagccagtg tgccgattcg cgaggatttc ccgttgtctg 26280

cgaccaatcc ttatgggcca agcaagctga tggtggagga tatcctgcgc gacttggcag 26340

tctccgatcc agagtggcgt attgccatcc tgcgttattt caatcctgtg ggcgcgcatg 26400

agagcggcct cattggtgag aacccgaatg gtatgcccaa taacctcatg ccatacatca 26460

cccaggtagc ccaggggcag cgtgaatctc tttctgtgtt tggcagtgac tatccaaccg 26520

tggatggtac gggcgtgcgc gactacatcc atgtggtcga cctggtggtt ggccatttgc 26580

gtgccctgga gcggctggat aaggaagctg gtgtgttcac atgcaatctt ggcacgggcc 26640

aaggatatag cgtgctggaa atggtcaagg catttgaaga cgccagtggc caggctgttc 26700

cctatcagct ggtggcacgc cggccgggtg atgtggcggc cagttatacc gatcctgctt 26760

atgccgagca ggcattgggt tggaaagcga cgcgtggcat cgagcagatg tgcaaggata 26820

gttggcattg gcagcaaaac cttgcccaga gaggctga 26858

<210> 175
<211> 13733
<212> DNA
<213> Methylobacillus flagellatus

<400> 175
gtgaggagac tccttgatga acgtagttta aacatactct tgtaatccaa gagtttaact 60

tgggatgaga tattctgaat tggttgaagc gtaatctttt aaaattttgg ggatgtgtgt 120

tatttagtat tatgattttt atgatgtttt tttattgtta aatattgttt ttagtagcaa 180

tattttttat ataattagat ttcatattga atgctcaaga tttaatttaa ttcagtattt 240

aaaactcttt aggtataatg agccaatttt ataaaaaagt tttttaatgc tgcttcctgt 300

cattatgtct ggcggggccg gttccagatt atggccggtt tctcgtgaaa ctctaactaa 360

gccatttatc gaattacctg atggcagaac ccttctgcag aagactttgc aacgtactgc 420

ttgcctggat ggtgtgagcg aagtgctcac agtaacgaat aaggaatatt acttcctgac 480

acgtgatgtc tatgaggcaa cagacactaa tgtttcccat agctacttac tagagacgat 540

agggcgcaat actgctcctg ccattgcagt tgctgcacta catgccgctg cgaaacatgg 600

ggaagatgta gtgctgttgg tgatgccggc agatcatctt attggtaatc aaaaagtttt 660

ttcaaaagca gtgactgttg cgacagatat ggcggtgaat ggctggatag tcacgtttgg 720

tgtagagcca agcacgcctg aaacaggtta tggctacatt gaaggcggag agtcaattgg 780

ggactcaggc gcgcgtagag tcgttcgatt tgtcgaaaag ccagatttgg aaactgctga 840

gaaatatatt gctttaggtc gatttacatg gaatgcaggt atattctgtt ttactgcaaa 900

aaccattatt gctgcacttg cggagtatgt tccagaagtg cttgagatag caaaaaagac 960

cctggcatct agtgactcca aaactgagcc tgttgtattg ggcgaggagc actttgcatc 1020

aagtccagat atttccatag attatgccgt catggagcgt gcttcaaaag tggcggtagt 1080

cgcagctgat tttgactgga gcgacattgg ttcatggagc gccatggctg agttggtcca 1140

gtcagatgag cgtgggaata gggtgaatgg cgaagcttta ttggtggatg ctaataattg 1200

ctatatccaa agtgattccc gtcttgtggc tggtataggt attgataacc tgctggtggt 1260

cgatacaccc gatgccctgc ttgtggctca tcactccaga gctcaggatg tcaaaaccgt 1320

tgtgaagcaa ctcaagcttg agtcccatag tgcggcaaat taccatcgca aggtacaccg 1380

tccatggggc acatacaccg tattggaaga aggcgctaat tttaagatca aatgcatcaa 1440

ggtaaagcct ggtgccagcc tgagcttgca aatgcaccat catcgtagtg agcattgggt 1500

tgtagtaagc ggtatagcta aagttgtcaa cggcgagcgc gagctgctca ttaatgccaa 1560

tgagtctact tacattccag caggacacaa acaccgtctt gaaaatccag gtttgcttga 1620

cctggtgatt attgaagttc aatccgggga atatttgggc gaagacgata ttgttcgttt 1680

tcaagatatc tacggacgca cttgatgatc agcatgttaa aagccttatg gctttatcgc 1740

ggctttgtgt tgggcagcgt caagcgcgag tttcaatcca agtaccgtaa ttcgctgcta 1800

ggcgcagtct ggacggtctt gaaccccctc gcaatgatcg tagtgtacac agtgattttc 1860

tcgcaggtaa tgaaggctcg tttgcctggc gtggacagta cttttgcgtt tagcattcat 1920

ttatgcgctg gcatactcac ctggggcttg tttgctgaga ttgtcgggcg ctcacagacc 1980

acgttcatcg ataatgccaa cttgctcaag aaactgagtt tcccccgtct atgtctgcca 2040

gtcatcgtca ccgccaccgc gctagttaac tttgccattg tttttgggtt gttcacagcg 2100

ttcttgctgc tgatcggtat gttttcgggt tggccttatt tggtgctgat cccggtattg 2160

atactgatga tcgtctttgc tctaggcttg ggtattatat tgggcgttct caatgtgttt 2220

ttccgcgatg taggtcagtt ctttggcatt gtgcttagtt tctggttttg gcttacgccg 2280

attgtctatc cggtcagcat tcttccagaa cgggtacaag attatatgcg gcttaacccc 2340

atggcgcgct tgatggaggc tttccaagga atattggtac aagcaaaatg gccggactgg 2400

tacagtctat ggccagtgtt tgcgctgtct atcgtgttgt gcctgctggg gctgtcttta 2460

ttccgcaaac atgcgggcga tatggtggat gaactttaat gggtactatt gttgtcacca 2520

acctgggtaa ggcctacaag caatacccca gtcgctggtc tcggttgttg gagtggatag 2580

atctgaggcg taagccgcac caccaattga agtggattct gcaaggagta agttttcata 2640

tccagccagg agagacggag gcagctttgc cgtgactgct gccaagcgtt ctaatacgag 2700

tattaagcct aaccttgcag tgatcaattg gatgcttgag caagaagacc ggatgggatt 2760

atctacccca aggccttatc gtgaatttga ggagcgtgta tatcgtcatc gcgatgatct 2820

caggcgctta attcattcgt tagtagctga cggcaaaaaa atatatgggt atggagcttc 2880

tgccaaaggt aatgtagtac tacaattttg tggctttact tctgaggaaa tccctgccat 2940

tgcagaagta aacgaagaaa aattcggacg agtcactccg ggaacgcata ttcccattat 3000

ttcggaaaaa gaggcgcgcg ctgccaagcc cgactacttc ttggtgcttc catggcattt 3060

caaagatggt atcctgcgcc gtgaaaagga ctttctagct gatggcggca ggtttatctt 3120

cccatttcct gagatcgaaa tcgtctagtt tccaaaaaaa gatgaaggaa aataataaga 3180

taatttagac gctgttctaa cgaatccatt atttacatac catatataca tatacagtaa 3240

gactttaaat acataaggca taatacgatg accataaaaa ctgccatcat taccggcatc 3300

acaggccaag atggtgcata tctcgcagaa cttctcctta gtaaaggcta taaggtttac 3360

ggtaccttcc gccgtacaag ctcggtcaac ttctggcgta ttgaagagct gggaattgaa 3420

aatgacccaa atcttctttt ggttgaatac gacctgaccg atttgtcagc aagcatccgt 3480

ctattgcaga gcacaggtgc aacagaagtg tacaacttag ctgcgcaaag ttttgtgggc 3540

gtgtcttttg agcaacctat tactaccgcg gagatcacag gtgtcggtgc gctcaatctt 3600

ttggaggcca ttcgtttagt aaatcccaat attcgctatt accaagcatc cacatccgaa 3660

atgttcggca aggtgcaggc gattccacaa acggaagata cgcctttcta tccacgcagt 3720

ccttatggtg tcgccaagct gtatgcgcac tggatgactg ttaactatcg tgaaagctat 3780

ggaatcttcg gatctagcgg tattttattc aatcatgaga gtccgttgcg tggtcgcgag 3840

ttcgtgacgc gcaagatcac ggattcggtg gccaagatca agctggggaa acttgattgc 3900

ctggagttgg gtaacttgga tgcaaagcgt gattggggct atgccaagga ttatgtggaa 3960

ggcatgtggc gcatcctgca ggcggatgag ccggatacct ttgtactggc gaccaatcgt 4020

accgagactg tgcgcgattt tgtaagcatg gcgttcaagg gggctggcat tgctgttgag 4080

ttcaagggca agggcgagga cgaagttgcc gtggatactg caaccggtaa gatagtggtt 4140

cgggtcaatc cgaagtttta ccgtccagcg gaagtggacc tactgatcgg tagcccagcc 4200

aaggccaagg ccaagctggg ttgggaaccg aagacaacgc tggagcaact ctgccagatg 4260

atggtcgagg ccgatctgcg ccgcaacacg gaaggtttct cattttgaga attctgctta 4320

ctggtgctga tggattcacc gggcgccatt ttctgacggc cgcgaaagcg gccggccatg 4380

aggttgttgc actgagggcc gatttgacgg acaaggctgt attgcaacgc gaagttgagg 4440

cggctcgtcc ggaagcagtg gttcatctgg cagcgatcag ttttgttggt catgcggatg 4500

ttagtgcctt ttatgacgtc aatgtgatcg gtaccctgaa cctgttggat gcgctggctg 4560

gcttgtccgt gacgccgcgc agcgtactgc tggcaagcag tgcgaatgtg tatggcaatg 4620

cagcgcattc acctatcacc gaagagcagg cgccggcgcc ggtcaatcac tatgcaatga 4680

gcaagctggc catggagcac atggcccgta cctatctgga cagactgccg ttgttttttg 4740

tgcggccgtt caattacacg gggccggggc aggctgagtc tttcgtgatt cccaagctgg 4800

tggcgcattt cgcgtgcaag gcgcagaccg tggaactggg caatctggat gtggagcgcg 4860

agttcaatga tgtggctttc gtttgcgagg cctatctgcg tttgttggac aaggctgtgc 4920

cgggtgaggt gtttaatatc tgcagcggtg ctccggtgac attgcgtgca gtaatcggga 4980

tgttgaggga attgacggcg catgatatcg aggtcaaggt aaatccgcag ttcgtgcgga 5040

ataacgagat caagacatta tgcggtgatc catcaaagct gttccgtact attggagagg 5100

ggaggctggt tcctttagcg cttaccttga agatcatgct ggaacacgct acgcgaaaag 5160

aaaaaaaatg atgaaggtag gttttggtgt caccgtgctt acacgttgcc tggcattggg 5220

gggaatcgat ggaattggta gttatacccg ggagctgatg aaaagactcg aggctgaccc 5280

ggccgtcgag cttttcccta tctcgtacgg gtattctccg tcacagtttc ctatcggctc 5340

acgtgctccg acgacatttg gacgatatgc gccgcttgct gcagtatcgg ctgtatccgg 5400

gtttcccttt ctgggaacag ctcaattgaa ggagcgtgtt gatctgattc atgcgcctga 5460

tcatcttatt cccaactacg gaaaagttcc tgttattgca tccatcatgg acgcgattcc 5520

cttgtcgcat ccggagtggg tatcccccaa gttgcgcaag atgaaaaatg cgttatggaa 5580

gcgctctgcg cactgggcat cgcatatcat tactatctct gagtattcga agcaggaaat 5640

ttcacagcat ttcggtattg aggcacaaaa gatcagtgcc atcccgcttg gcgtggatga 5700

gcggtggttt cagccgttgg aagaagaggc gatgaaggtg gtattgagac ggtacaatct 5760

gcctcaatac tattttcttg tcgtggggac gctgcagcca aggaagaatg tcggccgggt 5820

aatcgaggca tatcggtcct tgccccaagg gatacgtaac gaagtgccgt tggtgattgt 5880

tggtagggca gggtggcgat gcgaggatgt ggttgaaatg ttgacttcgt cttcttcagg 5940

aaattctctg aggtggctca agcatttgcc ggatgccgac ttgcttgcag tgttgaaagg 6000

tgcaacggct ttggtctttc cttcgttgta tgaggatttt ggtttgcccg tagtcgaagc 6060

ttttgcggct ggaactcccg ttattacttc caataccact tctcttccag aggtggcggg 6120

cgatgccgcc atgctggtga atcctctgaa tgtcagcgac attgccgctg caatgcaatc 6180

gatgctggaa cagccggaac tggcagagag tttcaggaac aaggggcaag aacgcgcatt 6240

ggcatattcg tgggacaaaa cagcctccat gacgcttgac gtctataaaa agacgcgaga 6300

ggctgcttaa catcaacgca ccgtcaaaca tcgcactcct tcccttttta tgatcatccg 6360

ttttctgggc gcacagtggc tcgccgttgg ttttgtcggg gtcatctctc tggcggtcag 6420

cattttgatt gcgcggacgc ttgggccgga ttcatttggt gtttattcgg ttgtcttgtc 6480

cggcggagcc attgccgcca ttctgcttga tggtggtttc gggcgattgt tgcaaagaga 6540

acgcgcccat cccacacctg cattgctcag attcctgccc atattgccgc aggctgcctt 6600

ggggcatgtg attatcgcgg ccagcatatt ttcgttgatc gctttattgg tactgcccaa 6660

gcatgcgctg acgattgttg cggttatctg gtttttcgca gcatctgtga ccaatcagtt 6720

cggtttgaac attctccgcg gagatgggcg gatggtaagg gatgcctcgt ggcagatggg 6780

caatcgaacc ttcacagctg cgtgcgtgtg cattgtcatt ctgatgggag ctgagaaacc 6840

gtggcaggta ttctttgccc agtttgtcgg tgcgaccgcc ttcggtttgc tgatcatgca 6900

ctatctgcgc acgcgcccgc tatggaagat tcctcctact ctttatgcgg caaccttgcc 6960

attcgtgtgg ttggatctcg cgacagtcct ttattttcga gcggacatgc tgttgctgga 7020

atggttagat gtgtccaagt ttgaagtcgg gaaatacggg gttgcctgcc gcttgattga 7080

ggcggtgatt ctgcttgctt cgccgctctg tattattttg ttccgcaagt ttcgtctggg 7140

tagcgcttct ccccgactga tgttgcggag aatgctgccg gtgctggcag gggcgctgat 7200

ggttggagtg ttcttcatgg tgtttttctg gttgtgcagc acgcctttga tcgcggttgc 7260

atatggcgag gcctatgcgg gagctgatca tattctggcg atactcggct gcgctttgct 7320

ttttattttg ccaaatggcg tgctgaacca agctgcgcta gcgctgggta tcgaacgttg 7380

gctggcaatg tcggcatctt ttgccgcagt gctaaatatt gcaggaaatc tctggctgat 7440

tccccaatat gggttgattg gagctgcctg gatgactgtt gtgaccgaag cggtgttagg 7500

ctgctgcgtt gcgttagggc tatggcgcca ccgtaatagg agtgtttaat gcaggatctt 7560

gccgttgctc atgtttatcg tacctatttt cctgaccctc caggcgggtt gcaggaggca 7620

attcgccaga ttgccctcac gacgggtatg cagggcgtcg ataatacggt gtttacgctt 7680

tctccgcagc cgcgtccggc aatcttgcag cgtccggagg cttgtgttgt tcgttgccgc 7740

tcctgggcgg ctccggcatc atgcgatctg ggcggtatcg gcgcatttct tgcgttttcc 7800

aggttggcgg aaaaatctga tgtgctacat tacctttttc cgtggccatt tgcggatgtt 7860

ctgcatacgg tggttcgccc aggttgtccg gccgtcatga cttatatctc ggacgtggtg 7920

cgtcagcagc ttttagggag gttatatgct cctttgatgt ggaaggtgtt gcgccagatg 7980

aggcttgtgg tggcgaattc tccagcgtat gcacgcacca gtcccgttct ctcccatcct 8040

gagattcgcg aaaaagtcag ggttattcca cttggtattg aggagtcttc ttatcctaaa 8100

tcaggggatg aggagattct gcagcgtcta ggcattggga aggatgagcc ttattttctt 8160

tttattggcg ttcttcgtta ttacaaaggc gcgcattttc tggtcaaggc agcaaagcgg 8220

ataggcgcaa aggtggtgtt tgctggcgcg gggccggaag gcgcgcgcct gaaagcattg 8280

gctggcgaag tcggtgcaga gaatgtgatt tttgccggga tggtgactaa cgcggaaaaa 8340

gtaaccttgc ttcaatgctg cagagcgctt gtgttgccat cgcatttgcg gtctgaagcg 8400

tacggcatgg tgctggtaga ggctgccatg ttcgggaagc cgctgatttc ttgcgaaatt 8460

ggcacaggca cttcatatgt caaccatcat gaagaaacag ggtttgtcgt tgagccggcc 8520

tctcctgaag cattggcgtc ggcgatggcg gttttgttat cagaggatag gctggctaca 8580

gagatgggag ctgctgctcg tcagcggtat gagctgctct tttccggccc ggcattagga 8640

aaggcatatg ctgcattgtt ccgcgaagtt gacgcttcgt agcagagtgc gtcatgatga 8700

agtagccaga gttgtaacgt ttttgggatt atcctgttgg ttttcttgat tgttaataca 8760

tgatgcaatt actggtgaca ggcgctacag ggtttgtagg taaaactttg tgcagtgcgc 8820

taaggcggca gagcttcccc gtaaaagcaa ttgggcgtgc gggcactatc cgggctgacg 8880

ctttgggtga tcaggttgct tcaattgacg gtcaaacgga ttggtcactg atattggacg 8940

gcatcaatgc cgtcatccat ctggcagcca gggtgcacgt catgcgggaa cagcttgctg 9000

atcccttggc ggcatatcgt gaaacgaatg tcgaagggac gctgaatctg gcgcgtcagg 9060

cggcaaaggc cggagttaag cggtttgtat tcattagttc catcaaggtc aacggcgaac 9120

agactctacc tggaaagcct tttactgcgt acgatgtacc agctcccgaa gatgcgtacg 9180

gtatttcaaa gttcgaggca gagcagggtt tgctggccct ggcgcgggag acagggatgg 9240

atgtggtggt tattcgccct cctttggttt acgggcctgg agtgaagggt aattttgcca 9300

gtatgatgaa gttggtgaaa agtggaatac cgttgcctct gggcgccatt cataaccaac 9360

ggtctttggt ggcgattgat aatttggtga acctcatcgt cacgtgtatt gaccatcccg 9420

cggcagccaa ccaggttttt ttggtaagcg atggcgagga tttgtccact tcagatttgt 9480

tgcggcgttt ggccaaggct gcgggcgttt cctcccgatt gatgccagtg tctgcccgct 9540

ggctagagat gggtttgcgt atgtgtggca agcgattgat cgcccagcgt ctcctgggat 9600

cattgcaagt cgatatttcc aagacgcgcg agttgttggg gtgggttccg ccgatctcag 9660

tggatgaggg cttgaagcgc tgtttctcta ccaagggctg attcttgatc cgattttttg 9720

acattatttt ttcggtgata ggcttggtac tgggattccc gctattgcta tcgatttata 9780

ccctcggctt gtttgataca ggtgctccct tgtttcgcca ggtgagggtt gggcgctacc 9840

ggcagccgtt tactttggtg aaatttcgga cgatgaaggt ggatacggct tctgtggcga 9900

gccatctggc ttccaccaat tccatcaccc ggctcggacg gttcttgcgc aggaccaagc 9960

tggatgagct gccccagctc tggaatgtcc tgaagggaga aatgagcctg gtggggcctc 10020

gaccgaacct gttcaatcag gaggagctga tggccgagcg cgagttgcgt ggcgtttacc 10080

atgcgcggcc agggattacc ggccttgcac aggtgaatga tatagacatg tcgacgccaa 10140

ggctgcttgc cgagaccgat gccaggatgc tgcgcgagat gaatgtacgg ctttatttca 10200

agttgctgat tcagacggtg ctgggtcgag gacagggcga ccgggtcgtg cgctgagctc 10260

ggcaggaacc aatgcctggc tacaaagtgt tgatccatga ttggtcgtca ctgtcccgtg 10320

gtttttagta gaatgttatt cattttcact ttaggctggg cgcgtgctgc attatttccc 10380

ctcattcaag tggtcgaact acatcagttt gaagaatcca cgctctttga gcgcttggat 10440

atatgacatt gttgctgctg cgctggcatg gtgggcagcc ttcctgttac ggttcaattt 10500

tgatattccg gccaattttg tccagagtat gctcgacagc gcagcatggg tgattccgct 10560

gcaggcattt gtgtttctgc tggctgggtt gtacaaaggc gtctggcgtt ttgccagcct 10620

gccggatttg cagcgcttgg tgcgcgcgat tgcgatcagc gcagtgcttg tggttgcgat 10680

cttgttcatg ttcaagccac acggcattgt tcctcgctcg gtgctgatct tggatcctat 10740

gctgttgctg ctgttgatgg gcggcagccg ctttgcttac cgagcatgga aagaacatcg 10800

cctgtatggt tctgcccagt tttccggaaa gccggtgctg gtgctcggcg cgggtgaggc 10860

agcgattgcc ttgttgaagg atctttccaa aagccgtgaa tggcatgtcg ttgggttgct 10920

ggacgataag aaaaccatgc atggccgaga gctactgggc gtcaatgtgc tggggtcgat 10980

tgcgcaattg ccacagatcg ctgcggattt gtcggtcaat catgtgattg tggccatgcc 11040

atccgcgcgt catcaacgac gcagggaaat cgtcgagctg gcaagcagaa atgggctgac 11100

tgcgctcacg gtgccgtcgt ttgatgacct gatgagtggc cacctgagca tggcacagat 11160

tcggccggtc gaggtggagg acttgctggg gcgggagccg gtcagtctcg atggagaggg 11220

gctccagcat ttgatcgagg ggcaggtgtt gctgattact ggtgcaggtg gttccatcgg 11280

ttctgagtta tgccgccagg tgctgaaata ccgtccagct tgcctggtct gcctggatca 11340

gtcggagtac gcgttgtatc ggttggagca ggagtttgac aatctggagt ttgatacccg 11400

cattgaatac gtggttggcg atgtcaggaa cgcacgccgg gtggggcagg tgcttggccg 11460

ttatatgccc aaactggtgt tccatgctgc tgcgtataag catgtgcctt tgatggagaa 11520

ccgcaatgtg gcggaatcat tgtcaaataa tgtcagcggt acttacacgc tcgcccaggc 11580

atgcaaaact gcaggggttg ccaagttcgt gctgatttcc accgataagg cggttaatcc 11640

caccaatgtg atgggggcca gcaagcgcct ggcggaaatg atatgccaag gcctgcagga 11700

ggagtcgggt acgcgtttta tcgtggtgcg ctttggcaat gtgctgggca gtagtggcag 11760

tgtaatcccc aagttccgtg agcagattgc ggcgggcggg ccaattaccg ttacccatcc 11820

cgagatcact cgctatttca tgtccatccc ggaggcgtgc cagctggtca tgcaggctgg 11880

attgatgggt ggtggcgggg agatatttgt gctggatatg ggagaccctg tgcggattgt 11940

ggacttggcc cgtgatatga tccggttatc cgggcttaat gaagatgaca ttcagattgt 12000

atactctggc ttgcggcccg gcgaaaagct gtacgaggaa cttttggctg acgacgagct 12060

cactgtacca acaccccacg acaagctacg tattgccgtg gcgcgaagcg tggatggtgc 12120

ttgggttcag gcattgctga aatggatcga cagggcatcg ctcatagatg aatctgtcat 12180

caagcaagag ctgggcatgt gggtggagga gtatgcaggg gatattcatg ccaatccatc 12240

catgatgttc ccccccgagt cttcaaccat acattagaag aaagatagat agaaatggca 12300

aaaaaaatta ccaaggcagt atttccagtg gcaggtttag ggacacgttt cctgccagca 12360

accaaggcga gtcccaagga aatgttgcca attgtggata agcctctgat tcagtatgca 12420

gcagaggagg ctgtggccgc cggtgctacc gagctgattt ttattatcgg gcgcaacaag 12480

cgcaccatcc cggatcattt tgataagtcc ttcgagctgg aagcgacgct tgaggcaagc 12540

aagaaaacca aggtgctgga agaaatccgc aatatcctgc caaaaaatgt ttcctgcatc 12600

tatattcgcc aggccgaggc gcttggtttg gggcatgcgg tattgcttgc caagtccgtg 12660

gtcggcagcg agcctttctc tgtgatcctg gccgatgacc ttattgatgc cgacaaacct 12720

gccatgcagc agatgacaga gctgttcgac caagagggtg tcagcattct gggtgtcgag 12780

gatgtggagc ccagtgagac ggcaagctat ggcattgtgg atgcgacaga ggtgagtggc 12840

ccgaatgtgt tgcaattgaa tggcattgtg gaaaaaccgc agccggaaga agcgccctcc 12900

aatctggctg ttgtcggtcg gtatattctc acgcctgaaa tttttgaatg cctggagaac 12960

gtcaagccgg gcaagggcgg ggaaatccag ttgactgatg gcattgccgc cttgatggaa 13020

aaacagaaaa tcctggctta ccgtttcgat gggaagcgct acgattgcgg cagcaagctg 13080

ggttatgtca aggcgaacgt cgagtattcc ttgaagcatg aggagtttgg caaagagttt 13140

gcccagtact tacgtgatct aaccaagtag cagtcgtggt ggtacttgag ggtaaccccg 13200

atctcattct gcagcgatcc acgctggtct cttctgaaga ggaagtgaag caggcggtga 13260

cgagggtggc gcaggaaatc actgccacgc taaggcagga ccaacctctc gtcctgtgtg 13320

tcatgggagg cggggtggta ttcaccgggc aattattgcc acagctggtt ttccctttgg 13380

agttcggtta tgtgcaggcc acccgctatc acaaccggac acatggcgat gcccagctgg 13440

tgtggaaaat gaagcccggg gaggaggtgc gcggccgtac cgttttattg ctcgatgata 13500

ttctcgatga gggggtcacg ctggcagaaa tcaaggcgca gtgtctggca ctgggtgcca 13560

gcaaggttgt cgttgcggtc ttggttgaaa aaatgctggg gcgggtcaag ccgatcaggg 13620

ctgatttcgt cggactggag gtgccggacc agtatgtatt cggatgcgga atggatgcct 13680

atgggtggtg gcgtaatttg ccggccattt atgccatgca aggggaagct tga 13733

<210> 176
<211> 13733
<212> DNA

<213>  Methylobacillus flagellatus

<400>  176
tcaagcttcc ccttgcatgg cataaatggc cggcaaatta cgccaccacc cataggcatc      60

cattccgcat ccgaatacat actggtccgg cacctccagt ccgacgaaat cagccctgat     120

cggcttgacc cgccccagca ttttttcaac caagaccgca acgacaacct tgctggcacc     180

cagtgccaga cactgcgcct tgatttctgc cagcgtgacc ccctcatcga gaatatcatc     240

gagcaataaa acggtacggc cgcgcacctc ctccccgggc ttcattttcc acaccagctg     300

ggcatcgcca tgtgtccggt tgtgatagcg ggtggcctgc acataaccga actccaaagg     360

gaaaaccagc tgtggcaata attgcccggt gaataccacc ccgcctccca tgacacacag     420

gacgagaggt tggtcctgcc ttagcgtggc agtgatttcc tgcgccaccc tcgtcaccgc     480

ctgcttcact tcctcttcag aagagaccag cgtggatcgc tgcagaatga gatcggggtt     540

accctcaagt accaccacga ctgctacttg gttagatcac gtaagtactg ggcaaactct     600

ttgccaaact cctcatgctt caaggaatac tcgacgttcg ccttgacata acccagcttg     660

ctgccgcaat cgtagcgctt cccatcgaaa cggtaagcca ggattttctg tttttccatc     720

aaggcggcaa tgccatcagt caactggatt tccccgccct tgcccggctt gacgttctcc     780

aggcattcaa aaatttcagg cgtgagaata taccgaccga caacagccag attggagggc     840

gcttcttccg gctgcggttt ttccacaatg ccattcaatt gcaacacatt cgggccactc     900

acctctgtcg catccacaat gccatagctt gccgtctcac tgggctccac atcctcgaca     960

cccagaatgc tgacaccctc ttggtcgaac agctctgtca tctgctgcat ggcaggtttg    1020

tcggcatcaa taaggtcatc ggccaggatc acagagaaag gctcgctgcc gaccacggac    1080

ttggcaagca ataccgcatg ccccaaacca agcgcctcgg cctggcgaat atagatgcag    1140

gaaacatttt ttggcaggat attgcggatt tcttccagca ccttggtttt cttgcttgcc    1200

tcaagcgtcg cttccagctc gaaggactta tcaaaatgat ccgggatggt gcgcttgttg    1260

cgcccgataa taaaaatcag ctcggtagca ccggcggcca cagcctcctc tgctgcatac    1320

tgaatcagag gcttatccac aattggcaac atttccttgg gactcgcctt ggttgctggc    1380

aggaaacgtg tccctaaacc tgccactgga aatactgcct tggtaatttt ttttgccatt    1440

tctatctatc tttcttctaa tgtatggttg aagactcggg ggggaacatc atggatggat    1500

tggcatgaat atcccctgca tactcctcca cccacatgcc cagctcttgc ttgatgacag    1560

attcatctat gagcgatgcc ctgtcgatcc atttcagcaa tgcctgaacc caagcaccat    1620

ccacgcttcg cgccacggca atacgtagct tgtcgtgggg tgttggtaca gtgagctcgt    1680

cgtcagccaa aagttcctcg tacagctttt cgccgggccg caagccagag tatacaatct    1740

gaatgtcatc ttcattaagc ccggataacc ggatcatatc acgggccaag tccacaatcc    1800

gcacagggtc tcccatatcc agcacaaata tctccccgcc accacccatc aatccagcct 1860

gcatgaccag ctggcacgcc tccgggatgg acatgaaata gcgagtgatc tcgggatggg 1920

taacggtaat tggcccgccc gccgcaatct gctcacggaa cttggggatt acactgccac 1980

tactgcccag cacattgcca aagcgcacca cgataaaacg cgtacccgac tcctcctgca 2040

ggccttggca tatcatttcc gccaggcgct tgctggcccc catcacattg gtgggattaa 2100

ccgccttatc ggtggaaatc agcacgaact tggcaacccc tgcagttttg catgcctggg 2160

cgagcgtgta agtaccgctg acattatttg acaatgattc cgccacattg cggttctcca 2220

tcaaaggcac atgcttatac gcagcagcat ggaacaccag tttgggcata taacggccaa 2280

gcacctgccc cacccggcgt gcgttcctga catcgccaac cacgtattca atgcgggtat 2340

caaactccag attgtcaaac tcctgctcca accgatacaa cgcgtactcc gactgatcca 2400

ggcagaccag gcaagctgga cggtatttca gcacctggcg gcataactca gaaccgatgg 2460

aaccacctgc accagtaatc agcaacacct gcccctcgat caaatgctgg agcccctctc 2520

catcgagact gaccggctcc cgccccagca agtcctccac ctcgaccggc cgaatctgtg 2580

ccatgctcag gtggccactc atcaggtcat caaacgacgg caccgtgagc gcagtcagcc 2640

catttctgct tgccagctcg acgatttccc tgcgtcgttg atgacgcgcg gatggcatgg 2700

ccacaatcac atgattgacc gacaaatccg cagcgatctg tggcaattgc gcaatcgacc 2760

ccagcacatt gacgcccagt agctctcggc catgcatggt tttcttatcg tccagcaacc 2820

caacgacatg ccattcacgg cttttggaaa gatccttcaa caaggcaatc gctgcctcac 2880

ccgcgccgag caccagcacc ggctttccgg aaaactgggc agaaccatac aggcgatgtt 2940

ctttccatgc tcggtaagca aagcggctgc cgcccatcaa cagcagcaac agcataggat 3000

ccaagatcag caccgagcga ggaacaatgc cgtgtggctt gaacatgaac aagatcgcaa 3060

ccacaagcac tgcgctgatc gcaatcgcgc gcaccaagcg ctgcaaatcc ggcaggctgg 3120

caaaacgcca gacgcctttg tacaacccag ccagcagaaa cacaaatgcc tgcagcggaa 3180

tcacccatgc tgcgctgtcg agcatactct ggacaaaatt ggccggaata tcaaaattga 3240

accgtaacag gaaggctgcc caccatgcca gcgcagcagc aacaatgtca tatatccaag 3300

cgctcaaaga gcgtggattc ttcaaactga tgtagttcga ccacttgaat gaggggaaat 3360

aatgcagcac gcgcccagcc taaagtgaaa atgaataaca ttctactaaa aaccacggga 3420

cagtgacgac caatcatgga tcaacacttt gtagccaggc attggttcct gccgagctca 3480

gcgcacgacc cggtcgccct gtcctcgacc cagcaccgtc tgaatcagca acttgaaata 3540

aagccgtaca ttcatctcgc gcagcatcct ggcatcggtc tcggcaagca gccttggcgt 3600

cgacatgtct atatcattca cctgtgcaag gccggtaatc cctggccgcg catggtaaac 3660

gccacgcaac tcgcgctcgg ccatcagctc ctcctgattg aacaggttcg gtcgaggccc 3720

caccaggctc atttctccct tcaggacatt ccagagctgg ggcagctcat ccagcttggt 3780

cctgcgcaag aaccgtccga gccgggtgat ggaattggtg gaagccagat ggctcgccac 3840

agaagccgta tccaccttca tcgtccgaaa tttcaccaaa gtaaacggct gccggtagcg 3900

cccaaccctc acctggcgaa acaagggagc acctgtatca aacaagccga gggtataaat 3960

cgatagcaat agcgggaatc ccagtaccaa gcctatcacc gaaaaaataa tgtcaaaaaa 4020

tcggatcaag aatcagccct tggtagagaa acagcgcttc aagccctcat ccactgagat 4080

cggcggaacc caccccaaca actcgcgcgt cttggaaata tcgacttgca atgatcccag 4140

gagacgctgg gcgatcaatc gcttgccaca catacgcaaa cccatctcta gccagcgggc 4200

agacactggc atcaatcggg aggaaacgcc cgcagccttg gccaaacgcc gcaacaaatc 4260

tgaagtggac aaatcctcgc catcgcttac caaaaaaacc tggttggctg ccgcgggatg 4320

gtcaatacac gtgacgatga ggttcaccaa attatcaatc gccaccaaag accgttggtt 4380

atgaatggcg cccagaggca acggtattcc acttttcacc aacttcatca tactggcaaa 4440

attacccttc actccaggcc cgtaaaccaa aggagggcga ataaccacca catccatccc 4500

tgtctcccgc gccagggcca gcaaaccctg ctctgcctcg aactttgaaa taccgtacgc 4560

atcttcggga gctggtacat cgtacgcagt aaaaggcttt ccaggtagag tctgttcgcc 4620

gttgaccttg atggaactaa tgaatacaaa ccgcttaact ccggcctttg ccgcctgacg 4680

cgccagattc agcgtccctt cgacattcgt ttcacgatat gccgccaagg gatcagcaag 4740

ctgttcccgc atgacgtgca ccctggctgc cagatggatg acggcattga tgccgtccaa 4800

tatcagtgac caatccgttt gaccgtcaat tgaagcaacc tgatcaccca aagcgtcagc 4860

ccggatagtg cccgcacgcc caattgcttt tacggggaag ctctgccgcc ttagcgcact 4920

gcacaaagtt ttacctacaa accctgtagc gcctgtcacc agtaattgca tcatgtatta 4980

acaatcaaga aaaccaacag gataatccca aaaacgttac aactctggct acttcatcat 5040

gacgcactct gctacgaagc gtcaacttcg cggaacaatg cagcatatgc ctttcctaat 5100

gccgggccgg aaaagagcag ctcataccgc tgacgagcag cagctcccat ctctgtagcc 5160

agcctatcct ctgataacaa aaccgccatc gccgacgcca atgcttcagg agaggccggc 5220

tcaacgacaa accctgtttc ttcatgatgg ttgacatatg aagtgcctgt gccaatttcg 5280

caagaaatca gcggcttccc gaacatggca gcctctacca gcaccatgcc gtacgcttca 5340

gaccgcaaat gcgatggcaa cacaagcgct ctgcagcatt gaagcaaggt tactttttcc 5400

gcgttagtca ccatcccggc aaaaatcaca ttctctgcac cgacttcgcc agccaatgct 5460

ttcaggcgcg cgccttccgg ccccgcgcca gcaaacacca cctttgcgcc tatccgcttt 5520

gctgccttga ccagaaaatg cgcgcctttg taataacgaa gaacgccaat aaaaagaaaa 5580

taaggctcat ccttcccaat gcctagacgc tgcagaatct cctcatcccc tgatttagga 5640

taagaagact cctcaatacc aagtggaata accctgactt tttcgcgaat ctcaggatgg 5700

gagagaacgg gactggtgcg tgcatacgct ggagaattcg ccaccacaag cctcatctgg 5760

cgcaacacct tccacatcaa aggagcatat aacctcccta aaagctgctg acgcaccacg 5820

tccgagatat aagtcatgac ggccggacaa cctgggcgaa ccaccgtatg cagaacatcc 5880

gcaaatggcc acggaaaaag gtaatgtagc acatcagatt tttccgccaa cctggaaaac 5940

gcaagaaatg cgccgatacc gcccagatcg catgatgccg gagccgccca ggagcggcaa 6000

cgaacaacac aagcctccgg acgctgcaag attgccggac gcggctgcgg agaaagcgta 6060

aacaccgtat tatcgacgcc ctgcataccc gtcgtgaggg caatctggcg aattgcctcc 6120

tgcaacccgc ctggagggtc aggaaaatag gtacgataaa catgagcaac ggcaagatcc 6180

tgcattaaac actcctatta cggtggcgcc atagccctaa cgcaacgcag cagcctaaca 6240

ccgcttcggt cacaacagtc atccaggcag ctccaatcaa cccatattgg ggaatcagcc 6300

agagatttcc tgcaatattt agcactgcgg caaaagatgc cgacattgcc agccaacgtt 6360

cgatacccag cgctagcgca gcttggttca gcacgccatt tggcaaaata aaaagcaaag 6420

cgcagccgag tatcgccaga atatgatcag ctcccgcata ggcctcgcca tatgcaaccg 6480

cgatcaaagg cgtgctgcac aaccagaaaa acaccatgaa gaacactcca accatcagcg 6540

cccctgccag caccggcagc attctccgca acatcagtcg gggagaagcg ctacccagac 6600

gaaacttgcg gaacaaaata atacagagcg gcgaagcaag cagaatcacc gcctcaatca 6660

agcggcaggc aaccccgtat ttcccgactt caaacttgga cacatctaac cattccagca 6720

acagcatgtc cgctcgaaaa taaaggactg tcgcgagatc caaccacacg aatggcaagg 6780

ttgccgcata aagagtagga ggaatcttcc atagcgggcg cgtgcgcaga tagtgcatga 6840

tcagcaaacc gaaggcggtc gcaccgacaa actgggcaaa gaatacctgc cacggtttct 6900

cagctcccat cagaatgaca atgcacacgc acgcagctgt gaaggttcga ttgcccatct 6960

gccacgaggc atcccttacc atccgcccat ctccgcggag aatgttcaaa ccgaactgat 7020

tggtcacaga tgctgcgaaa aaccagataa ccgcaacaat cgtcagcgca tgcttgggca 7080

gtaccaataa agcgatcaac gaaaatatgc tggccgcgat aatcacatgc cccaaggcag 7140

cctgcggcaa tatgggcagg aatctgagca atgcaggtgt gggatgggcg cgttctcttt 7200

gcaacaatcg cccgaaacca ccatcaagca gaatggcggc aatggctccg ccggacaaga 7260

caaccgaata aacaccaaat gaatccggcc caagcgtccg cgcaatcaaa atgctgaccg 7320

ccagagagat gaccccgaca aaaccaacgg cgagccactg tgcgcccaga aaacggatga 7380

tcataaaaag ggaaggagtg cgatgtttga cggtgcgttg atgttaagca gcctctcgcg 7440

tctttttata gacgtcaagc gtcatggagg ctgttttgtc ccacgaatat gccaatgcgc 7500

gttcttgccc cttgttcctg aaactctctg ccagttccgg ctgttccagc atcgattgca 7560

ttgcagcggc aatgtcgctg acattcagag gattcaccag catggcggca tcgcccgcca 7620

cctctggaag agaagtggta ttggaagtaa taacgggagt tccagccgca aaagcttcga 7680

ctacgggcaa accaaatccc tcatacaacg aaggaaagac caaagccgtt gcacctttca 7740

acactgcaag caagtcggca tccggcaaat gcttgagcca cctcagagaa tttcctgaag 7800

aagacgaagt caacatttca accacatcct cgcatcgcca ccctgcccta ccaacaatca 7860

ccaacggcac ttcgttacgt atcccttggg gcaaggaccg atatgcctcg attacccggc 7920

cgacattctt ccttggctgc agcgtcccca cgacaagaaa atagtattga ggcagattgt 7980

accgtctcaa taccaccttc atcgcctctt cttccaacgg ctgaaaccac cgctcatcca 8040

cgccaagcgg gatggcactg atcttttgtg cctcaatacc gaaatgctgt gaaatttcct 8100

gcttcgaata ctcagagata gtaatgatat gcgatgccca gtgcgcagag cgcttccata 8160

acgcattttt catcttgcgc aacttggggg atacccactc cggatgcgac aagggaatcg 8220

cgtccatgat ggatgcaata acaggaactt ttccgtagtt gggaataaga tgatcaggcg 8280

catgaatcag atcaacacgc tccttcaatt gagctgttcc cagaaaggga aacccggata 8340

cagccgatac tgcagcaagc ggcgcatatc gtccaaatgt cgtcggagca cgtgagccga 8400

taggaaactg tgacggagaa tacccgtacg agatagggaa aagctcgacg gccgggtcag 8460

cctcgagtct tttcatcagc tcccgggtat aactaccaat tccatcgatt ccccccaatg 8520

ccaggcaacg tgtaagcacg gtgacaccaa aacctacctt catcattttt tttcttttcg 8580

cgtagcgtgt tccagcatga tcttcaaggt aagcgctaaa ggaaccagcc tcccctctcc 8640

aatagtacgg aacagctttg atggatcacc gcataatgtc ttgatctcgt tattccgcac 8700

gaactgcgga tttaccttga cctcgatatc atgcgccgtc aattccctca acatcccgat 8760

tactgcacgc aatgtcaccg gagcaccgct gcagatatta aacacctcac ccggcacagc 8820

cttgtccaac aaacgcagat aggcctcgca aacgaaagcc acatcattga actcgcgctc 8880

cacatccaga ttgcccagtt ccacggtctg cgccttgcac gcgaaatgcg ccaccagctt 8940

gggaatcacg aaagactcag cctgccccgg ccccgtgtaa ttgaacggcc gcacaaaaaa 9000

caacggcagt ctgtccagat aggtacgggc catgtgctcc atggccagct tgctcattgc 9060

atagtgattg accggcgccg gcgcctgctc ttcggtgata ggtgaatgcg ctgcattgcc 9120

atacacattc gcactgcttg ccagcagtac gctgcgcggc gtcacggaca agccagccag 9180

cgcatccaac aggttcaggg taccgatcac attgacgtca taaaaggcac taacatccgc 9240

atgaccaaca aaactgatcg ctgccagatg aaccactgct tccggacgag ccgcctcaac 9300

ttcgcgttgc aatacagcct tgtccgtcaa atcggccctc agtgcaacaa cctcatggcc 9360

ggccgctttc gcggccgtca gaaaatggcg cccggtgaat ccatcagcac cagtaagcag 9420

aattctcaaa atgagaaacc ttccgtgttg cggcgcagat cggcctcgac catcatctgg 9480

cagagttgct ccagcgttgt cttcggttcc caacccagct tggccttggc cttggctggg 9540

ctaccgatca gtaggtccac ttccgctgga cggtaaaact tcggattgac ccgaaccact 9600

atcttaccgg ttgcagtatc cacggcaact tcgtcctcgc ccttgccctt gaactcaaca 9660

gcaatgccag cccccttgaa cgccatgctt acaaaatcgc gcacagtctc ggtacgattg 9720

gtcgccagta caaaggtatc cggctcatcc gcctgcagga tgcgccacat gccttccaca 9780

taatccttgg catagcccca atcacgcttt gcatccaagt tacccaactc caggcaatca 9840

agtttcccca gcttgatctt ggccaccgaa tccgtgatct tgcgcgtcac gaactcgcga 9900

ccacgcaacg gactctcatg attgaataaa ataccgctag atccgaagat tccatagctt 9960

tcacgatagt taacagtcat ccagtgcgca tacagcttgg cgacaccata aggactgcgt 10020

ggatagaaag gcgtatcttc cgtttgtgga atcgcctgca ccttgccgaa catttcggat 10080

gtggatgctt ggtaatagcg aatattggga tttactaaac gaatggcctc caaaagattg 10140

agcgcaccga cacctgtgat ctccgcggta gtaataggtt gctcaaaaga cacgcccaca 10200

aaactttgcg cagctaagtt gtacacttct gttgcacctg tgctctgcaa tagacggatg 10260

cttgctgaca aatcggtcag gtcgtattca accaaaagaa gatttgggtc attttcaatt 10320

cccagctctt caatacgcca gaagttgacc gagcttgtac ggcggaaggt accgtaaacc 10380

ttatagcctt tactaaggag aagttctgcg agatatgcac catcttggcc tgtgatgccg 10440

gtaatgatgg cagtttttat ggtcatcgta ttatgcctta tgtatttaaa gtcttactgt 10500

atatgtatat atggtatgta aataatggat tcgttagaac agcgtctaaa ttatcttatt 10560

attttccttc atcttttttt ggaaactaga cgatttcgat ctcaggaaat gggaagataa 10620

acctgccgcc atcagctaga aagtcctttt cacggcgcag gataccatct ttgaaatgcc 10680

atggaagcac caagaagtag tcgggcttgg cagcgcgcgc ctctttttcc gaaataatgg 10740

gaatatgcgt tcccggagtg actcgtccga atttttcttc gtttacttct gcaatggcag 10800

ggatttcctc agaagtaaag ccacaaaatt gtagtactac attacctttg gcagaagctc 10860

catacccata tatttttttg ccgtcagcta ctaacgaatg aattaagcgc ctgagatcat 10920

cgcgatgacg atatacacgc tcctcaaatt cacgataagg ccttggggta gataatccca 10980

tccggtcttc ttgctcaagc atccaattga tcactgcaag gttaggctta atactcgtat 11040

tagaacgctt ggcagcagtc acggcaaagc tgcctccgtc tctcctggct ggatatgaaa 11100

acttactcct tgcagaatcc acttcaattg gtggtgcggc ttacgcctca gatctatcca 11160

ctccaacaac cgagaccagc gactggggta ttgcttgtag gccttaccca ggttggtgac 11220

aacaatagta cccattaaag ttcatccacc atatcgcccg catgtttgcg gaataaagac 11280

agccccagca ggcacaacac gatagacagc gcaaacactg gccatagact gtaccagtcc 11340

ggccattttg cttgtaccaa tattccttgg aaagcctcca tcaagcgcgc catggggtta 11400

agccgcatat aatcttgtac ccgttctgga agaatgctga ccggatagac aatcggcgta 11460

agccaaaacc agaaactaag cacaatgcca aagaactgac ctacatcgcg gaaaaacaca 11520

ttgagaacgc ccaatataat acccaagcct agagcaaaga cgatcatcag tatcaatacc 11580

gggatcagca ccaaataagg ccaacccgaa aacataccga tcagcagcaa gaacgctgtg 11640

aacaacccaa aaacaatggc aaagttaact agcgcggtgg cggtgacgat gactggcaga 11700

catagacggg ggaaactcag tttcttgagc aagttggcat tatcgatgaa cgtggtctgt 11760

gagcgcccga caatctcagc aaacaagccc caggtgagta tgccagcgca taaatgaatg 11820

ctaaacgcaa aagtactgtc cacgccaggc aaacgagcct tcattacctg cgagaaaatc 11880

actgtgtaca ctacgatcat tgcgaggggg ttcaagaccg tccagactgc gcctagcagc 11940

gaattacggt acttggattg aaactcgcgc ttgacgctgc ccaacacaaa gccgcgataa 12000

agccataagg cttttaacat gctgatcatc aagtgcgtcc gtagatatct tgaaaacgaa 12060

caatatcgtc ttcgcccaaa tattccccgg attgaacttc aataatcacc aggtcaagca 12120

aacctggatt ttcaagacgg tgtttgtgtc ctgctggaat gtaagtagac tcattggcat 12180

taatgagcag ctcgcgctcg ccgttgacaa ctttagctat accgcttact acaacccaat 12240

gctcactacg atgatggtgc atttgcaagc tcaggctggc accaggcttt accttgatgc 12300

atttgatctt aaaattagcg ccttcttcca atacggtgta tgtgccccat ggacggtgta 12360

ccttgcgatg gtaatttgcc gcactatggg actcaagctt gagttgcttc acaacggttt 12420

tgacatcctg agctctggag tgatgagcca caagcagggc atcgggtgta tcgaccacca 12480

gcaggttatc aatacctata ccagccacaa gacgggaatc actttggata tagcaattat 12540

tagcatccac caataaagct tcgccattca ccctattccc acgctcatct gactggacca 12600

actcagccat ggcgctccat gaaccaatgt cgctccagtc aaaatcagct gcgactaccg 12660

ccacttttga agcacgctcc atgacggcat aatctatgga aatatctgga cttgatgcaa 12720

agtgctcctc gcccaataca acaggctcag ttttggagtc actagatgcc agggtctttt 12780

ttgctatctc aagcacttct ggaacatact ccgcaagtgc agcaataatg gtttttgcag 12840

taaaacagaa tatacctgca ttccatgtaa atcgacctaa agcaatatat ttctcagcag 12900

tttccaaatc tggcttttcg acaaatcgaa cgactctacg cgcgcctgag tccccaattg 12960

actctccgcc ttcaatgtag ccataacctg tttcaggcgt gcttggctct acaccaaacg 13020

tgactatcca gccattcacc gccatatctg tcgcaacagt cactgctttt gaaaaaactt 13080

tttgattacc aataagatga tctgccggca tcaccaacag cactacatct tccccatgtt 13140

tcgcagcggc atgtagtgca gcaactgcaa tggcaggagc agtattgcgc cctatcgtct 13200

ctagtaagta gctatgggaa acattagtgt ctgttgcctc atagacatca cgtgtcagga 13260

agtaatattc cttattcgtt actgtgagca cttcgctcac accatccagg caagcagtac 13320

gttgcaaagt cttctgcaga agggttctgc catcaggtaa ttcgataaat ggcttagtta 13380

gagtttcacg agaaaccggc cataatctgg aaccggcccc gccagacata atgacaggaa 13440

gcagcattaa aaaacttttt tataaaattg gctcattata cctaaagagt tttaaatact 13500

gaattaaatt aaatcttgag cattcaatat gaaatctaat tatataaaaa atattgctac 13560

taaaaacaat atttaacaat aaaaaaacat cataaaaatc ataatactaa ataacacaca 13620

tcccaaaaat tttaaaagat tacgcttcaa ccaattcaga atatctcatc ccaagttaaa 13680

ctcttggatt acaagagtat gtttaaacta cgttcatcaa ggagtctcct cac 13733

<210> 177
<211> 26292
<212> DNA
<213> Methylobacillus glycogenes

<400> 177
attgatgctg gttggtctgg gactgctggg ctttgctgca cgtcgtcgcc aagcttaatt 60

aagctgagca cgtaaatgca cgcgcttatc aggcgtgcat ttagtcatgc taagagtaaa 120

tgaataagtc ggcttcatgc cgacttattc attttgggta ttgtgttggt gcctattcct 180

gaagttgtag cacatactgg gaatggcgag cgaaggtgtg acaagacttg tcataaatct 240

atagcagttg cacagtagca tccgcaaggc cattcatgca gtgagtgagt ctgatttagt 300

ccatagggac tattgaaatg agaatgcctg tcatttggcg atttgatttt gtatcatgag 360

cgtatttcaa actaaggcac atgcatgaac agggcatcac tggggccgat aagtagccgt 420

gttattccct tcgcggtcta tatgctattc atggcagggg tagacctgat cggcgcagtt 480

gtgcctgagg gttttgatct gcgatggtta tatggagtca aggttggctt ggttgcctac 540

ttgctttgga cattgcgcaa acactatatc gagcttggtg gcttgtcgtt gcttagtttg 600

aagtcgtggt tgagcgcaat cagcattggt gtgctagtat ttgtcgcttg gattaacctg 660

gatgccccgt ggatgttact gggcgggcca tcgcaaggct tcaatccggg gcagggtgtc 720

gaagcgtgtc tcctaatggt gacaagaatt gtcggtgctg ccctagtagt gcctctgatg 780

gaagagttgt tctggcgttc tttcctgatg cgctggatac aaaatcagga cttcctcaag 840

gtggagccgg ccacggttgg tgctgtggca ttactggttt cttcactact ttttgccata 900

gagcataacc tatggtttgc tggtctggtt gcggggctgg catacggatt gctttacatc 960

cggagcagaa caatatggat gccgatcatc gcgcatgcgg tgaccaacgg tgtattggga 1020

gtttggatta tctataccgg taactggcat tactggtagc cattggggaa gtatttcttt 1080

gcgactaaca gactctactc attataaaaa gctggttatc acggcagtca taggttggaa 1140

tgcagtgggt tattccgacc ctgtgctggc tcgtgaaggt gatgtcatac ggccctatgt 1200

cagtgcaaca tatgtttatg atgacaacct gaggcgccgc tctgacaagg agtccgatac 1260

gagtgtagtg ggccgggttg gcattattct ggataaggat attagtcagc agaatttcta 1320

cgccaatatc ggtgtgaatg aaaccaaata caacaaaaat tcagagttag acaatacagg 1380

taaagatatt gtcgctcgat ggaactgggc agtgggctcg caattccggg gcaaggttga 1440

gtactataaa aacgaggcta tgctgccttt tgcggatttt cgtggtgtgg aattgaatat 1500

gcgtattcag gagcgtaaaa cttttgaagc aaactggcgt ttccattcca gttggcgcgc 1560

gcgctttggt gtgcagcgcc aggatgttga gtatgactct caaacattga atgcggcttc 1620

ttttcaggaa aattcccagc acgtaggttt ggactattta gctgagtcag gcagtaaggt 1680

tggcttggtc ttcactaatg ggcgtggcac gcggcccttt gataggcctg cttttttccc 1740

ggggattggt accgtgtttg tcaataatga ctacaggcag caatcactca aggcggacat 1800

actttggctt tattctgcca aaacacgatt tgaatttttg ggggggtatg ttgatcgtaa 1860

acatgacagc ttctcacagc gcgatttttc cggatttaat gcccacggga acatggtgtg 1920

gtccattacc gacaaaacct ctttcaagac agccgcatgg cgcgatgcaa atgctatatc 1980

aatcgtaact gccagctatg tgattgatag tggcgcaagt gcaagcatct cctatagcgc 2040

aacgagtaag atcctgctgt caacttcagc caggtatcaa caactaaaat ttgaaggcga 2100

tatcaccggg caagatcaag gtcgtaccga caagaataag gttttttctt ttggggtaac 2160

gtacagccct cttgaatcgt taagtttcac tactggtcta aatagaagtt tgcgtgactc 2220

aacctcacca attttaaact acaactcgac aagcatttcg cttactggtc gttatgaatt 2280

ctgatggtgt aagataaatg accgtaaatg acatcccttt agtagcgttt ttcaagcagc 2340

tgctcgaccc gctgatcatt cttggtacgc tgtatgtgtt caccctgttg tttggtgagc 2400

cttttaatgg ctactacctg attctggcgt tgctagccta ctttgtttcc tctaatgtct 2460

acgagcgtct ggatctttac cgtacctggc gtagtggcaa gatacttggt tatatccgcg 2520

atattctctt tggctggggt attgtcatcg ctgtgctgat gttcatcggc tatgccgctg 2580

atctatccag tcagtatctg gatgagatga tatatgcctg gtttgccagt gtgccgatga 2640

tatttattgt cacgcatctg gcgatacgca aaattgcctc ggatttgcac aagaatggtg 2700

aagtcaggtc cgtagtgatt atcggtgcca atgatattgg cgtgcgcttg atcaatcgca 2760

tcacggacga cccgtacctg tttatggagg tcaaggggat ctttgacgac agagaggtcg 2820

ccaggcaccc caagggcatg cgtaatctgc ctttgcttgg ttccatgtcg gaaattgcgc 2880

catatgtccg cgccaataat acccagatta ttttcatcag cctgcccatg tcggcgcagc 2940

cacgcatcag gcagttattt gaagaattgc aagacaccac agcatctatt tactttgtgc 3000

cggatatcta tatttttgat ctggtacaag cgcgctttga tgatgtggct ggtgtgcctg 3060

tggtcgctat tcttgagacg ccattcaccg gtttcaacag tttaatcaaa cggactagtg 3120

acattatttt ggcgtcgctt attctattaa tgctgtctcc catcatgctg acactggctc 3180

tggccgtgaa gttgacctcc ccaggcccgg tgattttcaa gcagcgacgt tatgctgaat 3240

ggtgaggaaa tcattgtcta taaattccgc tccatgaaag taatggaaga tggtgacaag 3300

gtagtacagg cgcagaagcg agatcagcga ttgacaccga ttggtggttt cttgcgcaag 3360

aactcactgg atgagctgcc gcagttcatc aatgtgttgc aaggtcagat gagtatcgtg 3420

gggcctagac cgcatgccgt ttctcataat gagcaatatc gcaagctgat tcgtggctat 3480

atgctgcggc acaaggtcaa gcctggaata acaggctggg cgcaggtgaa tggtttacgt 3540

ggtgaaactg ataccttgga caagatggag tcccgtatcg agtatgactt ggattatttg 3600

cgccattggt ccctggcttt tgacttctgg attatcgtca ggacggtgct ggttgtgttc 3660

aaaaaagata acgcctacta ggttgtttaa atagatttga ggagattgga aagtgaataa 3720

cggatttgta actaacttca agcaatccag aaccctgtgt gtcggtgctg tgattgtgct 3780

cgctttcgca ttgtcgggct gtggcgacaa gcatgaatcc aaagccagcc agtctctggt 3840

taaggtcaat ggcaaggaaa tcaccgtaca tcagctgaat caggagctgg gtcgtgccaa 3900

cattcaggag aatcagcgcg agcaggcaac gcagcagatt ctgactgcat tgatagatcg 3960

ccagttactt gaagaagctg cgctcaaggc caaggtggat agggatccaa atgtgatgac 4020

cacgattgaa cgtgcgaaat cacaaatcat tgctcaggca tatctgcaat ccaagttagg 4080

cagtgtcgcc aagccaagca aaacagaaat tgagaatttt tataacgaga atcctcaatt 4140

cttcagcaag cgcagaatgg tggaaatgga tcaattgttg attgatacta aattcgccaa 4200

tgatgaattg aaaacagcac ttggcaaact caagaccttg caggaagtag ccgcatggat 4260

ggatgcccat aatattcagt acgatcaagg tcaggtttca cgcagtgttg ctgaattgcc 4320

gccacagatt tccgagcgcc tcaaggccga ggataagggt aagttgttcg tgatcggcat 4380

gggccctcgc accatgttga tcatgatgaa cgagatcaag gacagcccag tttcacttgc 4440

agaagcaaca ccgcaaattg aaaacaattt gctgaataag gcgcgcaagg aagttgctga 4500

aaatgaagtc aagcgcttga aagccgatgc caagattgag tacatcgata aaaagatgga 4560

aacagcgact gaaaccaagg cagccaaaaa gaagccaagc tctactgagg atgatatcgc 4620

cagtggtgtt agtggcttga aataagtact ccccatcctt aatatcagga cgatagtaaa 4680

atgaaaaaat taatgacatg gtttttcttg ctgatgctga gtctgacgcc ttttgtaacg 4740

catgctgcag aaggtgacat tcttctcggc gctggcgatg tgctgaaaat atcggtgttc 4800

gagcagcctg atctgtcact tgaggtcaga gtgagtgaat caggttccat tacttatccc 4860

ctgataggtg aagtgcgtgt gggtggtggt acggttgccg ccgccgagca aagaattgcc 4920

accatgctgg aaaagggtgg tttccttaag agtgctcacg tcaacatcat cgttgcgcaa 4980

ttgcagagcc ttcaagtctc tattctgggt caagtcaatc gcccgggcag ataccctata 5040

gatagcgcta aaacgctggc ggatgtgctt gccttggctg gtggtgtatc acctgaaggt 5100

ggcgatctga tcacgctggt gcataacgtc gatggtaaag cggtcaggga tcagattgac 5160

cttgctgaca tgatgcgtac tggcgatctc agcaagaaca tccgcgtagc tggtggtgac 5220

atcatttatg ttgaacgttt cgataaattc tatatctatg gtcaggttca acgtgccagc 5280

cattaccgtc tcgaacatga aatgacagta ttgcaagcgc tttctgtagg tggtggctta 5340

agccctcgcg gcacagaaaa aggcgtcaag atcaagcgtc gtgatgctag cggccatatt 5400

gaagtaatca ctgccaaaca tgatgacctc atcaagccca acgatgtgat ctatgtgcag 5460

gaaagtttct tttaatagat tgcagatttt aataaatact tgataggcaa ggttaatcta 5520

atgagctttt ctcaactttt actgatattc aaagcgcgat ataagatatt gttaagcacc 5580

attgttgtgg ccctggtcgt agcaattgcg ctcaatattt tcttgccaaa cagctataag 5640

gcaaccacca gtatcctagt caactataaa ggtgtagatc ctgtgactgg tatggctgtc 5700

cctgcccagc tgcttccagg ttatatgggt acccaggtgg atatcataac tagcaagaaa 5760

attgctaaga gggttgtgac cacattaaag ctcaatgaaa gtagtgaggt tgtggagagt 5820

ttcaatgact ccacaggtgg tgaaggtgat atcgttgatt ggttggcgga tttgctgatc 5880

tcacgtatcg aagttcaacc tgcgcgcaac agtagtgtgt tgcagctgga gtttgatgga 5940

gccaacccca aatttgctgc tgttgttgct aactcctttg ccgaggaata tatcaaggcc 6000

actatcgaac tcaaggttga gccttcgaaa aaggcggctg aattttatac cgggcaattg 6060

aaggatttgc gtgccgatat tacccgggcg cagcaaaagc tttccgatta ccaacaagat 6120

aaaagcatcg tcagcgttga tgagcgtcta gacattgaac gtgccaagct gaatgaactt 6180

tcctcgcagt tagtgatgtc acaagggcaa actcttgagg cacagtctcg tcagactggt 6240

gcacaaggta gcaatgctta tggatctccg gatgtcgcga gtaatccaat ggttctgcac 6300

ctgaaaacag agattgctag gtctgaatca cgcttcgcgg atatctcaca aaaatatgag 6360

agaaatcacc cactctatca gggggccaaa gctgagcttg acaatctgaa gtctgaatta 6420

agtaagcaaa tccaggcggc taccgtcaat atatcgagca attatagaat ccttaagcaa 6480

cgcgagagtg aaatccgcaa tgccttggct gagcaaaaaa ccaaggtcat gctgttgaac 6540

aaggataggg atgcactgac ggtattgacg cgtgaagtcg agggtgcgca acgcgcttac 6600

gaactcgcga ctcagcgata tactcaaata aatcttgagg gacagtctaa tcaagctgat 6660

gtatccatcc ttaatccagc gacaccacca cttaagccat caagcccgaa aaaaatattg 6720

aacgtggcag ttgcgttggt gctgggtaca atttttggct tgttgtttag tttgttggct 6780

gagctcacta atgagcgaat tcgctcagaa agagtgctgg tagagattgt tggcgcaccg 6840

ctgctgggtg tgatcgacaa aaataaaacc cattccagaa ttcagcgcct gctggctaaa 6900

aaaccctaat ttgtgtggat atagacatga ctaatgatgt ttccaatact agttccattc 6960

cggcattgaa tgctgatttc aatatcggtg gaatgttgct caagatgggt aagattacac 7020

cgcaggatgc ggagcgtatc ctgcgtgtgc agaaggaaag aggcgttcgc tttggtgagg 7080

ctgccgagag tttgggccta gtttcctctg ccgatatcaa ctatgtatta tccaagcagt 7140

ttgcctatcc ctacctgatg gatggtcgcg gcgatttctc cgagaacttg gtcgcggctt 7200

atgatccatt cagtatcaat gtggagaagt atcgcgccat tcgtagccag ctcatgctct 7260

actggtttgc acaagggaaa aagagcctgt cattttccag tgtttacgac aaggacgaca 7320

ccagtgactt ggttgccaat cttggcgtgg ttttctctca actgggagag aaaacactca 7380

tcattgatgc ccatcttgat agcccacggc agaatcgcat ttttaatatc aagagcactg 7440

cgggtttgtc ggatattctg gcaggccggg ttacggatta ctcctcttcc atcactaggc 7500

tagtcgagtt cgagcatctc tccatcttgt cttctggcac cattccacct aatccgcgag 7560

agcttctggg acgtggtttc ctcaagcagt tcatcacggc aattgctaat gactacgatg 7620

ttatcctggt cgatgcacct tcctttgtcc gccactctga accgttgtct gtggcaagtg 7680

ctacggaagg ggtggtgctt cttgccaagc gtcatgtctc caaagccaat gacttgagca 7740

aagtggttga tcagctttcc aagatcaggg tgcctgttgt tggcacggta ttgactgaaa 7800

actaaaatga gcttgaacca gagcattgcg gtcgccagta cccagctaag aaggtaccag 7860

catgtgatac cactgctggt aggcttgctg ttcttatttg tgcccaccta cctgaacctg 7920

acgaacacgg tctggcagat gcaggatcag gcgcatggcc ccttgatcct gatggtggtg 7980

ctgtacctag tctgggtgga caaggaaata ctgctagaca cggcaaactt gagacaaagc 8040

gggcgtggtg tgggcctgtt gctgtttttg tttggcctgt tggtatacat catcgggcgt 8100

tcacaagatt tgctggtgtt tgatgtgggc tcgcagattc cggtattggc tggcttgctg 8160

ttgattttct ataaggccaa aaccctcaag cgcttctggt tccccttgtt cttcttgttc 8220

ttcatgattc cattgccagg ctccatgctg gatgttgtga ccttgccgat gaaggtcgct 8280

gtctcttacg tggccgagca tgtgctgtat tggtttgatt accccatcgc gcgttcgggt 8340

gtgatcattc agattggtca gtatcagttg ctggtggcgg atgcttgtgc aggcatgcat 8400

accttgattt cactggaggc cctgggcctg ctctacctca atatcgtcag gcatgattcc 8460

ctggcccgta acttgttgct ggccttgctg attatcccta tttccttctc agcgaatgtc 8520

atacgcgtga ttgtgttgac cttgatcacc tactatttcg gggatgaagt gggccagggc 8580

tttgtacatg gattcgcggg tattttgtta tttgtcgtgg cattaaccct gattatttca 8640

gtggatagcc tgatccaata ctttgttggc aaacatacac agcgtaaggc tagattgcag 8700

agttgatatg agcgtcagat taatcaatat cctggcagta tgcatggtgg tgagttctgg 8760

cctggcgttg gctttgaccc ctgatcttag ctccgtgcgg gatgatccac atcttgaaag 8820

tttcgtgcct agcagctttg gtcagtggca ggaagtcaaa accaatcagg tgcagatgga 8880

cctgacagtc aggagagatg gcgaacccag catcgataac ccttatgatg aagttctcat 8940

gaggagttat gccaatgaaa agggtcaggt cattatgctg gctcttgcct atggcaaatt 9000

gcagcggcag gaagtcaagg tgcataggcc tgaactgtgc tatacggcgc agggtttctc 9060

catcagaaat cagcaggata ccgcagtgaa tgtgggggca ggcgcaacac ccatcccatc 9120

caaggcgttg gttgcggctt caagacagcg cgaagagctg gtgctttact ggatcaggat 9180

aggcgatatg atcagtaaga gtgcttggga gacccgactt tatattgtgg agcaaggcct 9240

caaacatcaa ctggtagatg ggatactggt cagaacctca cagatattgg gtgaagatga 9300

tgccgagcaa tctttgcagc tacaacaaca gttcctgcac gatctggtgc aagcagtgcc 9360

cagggacaag cgtaaatact tgattccatc ataattttat agccttgtgt ttccattagg 9420

ataatggaga tcgagtaaag gatttgagca taaaagctgg gtatgctagc aaacagccag 9480

caggggaatt aacaacatat cttaataaca gcgcaccagc tctgcggcgc aagtgcgttg 9540

gcatatcctt gggtgttaaa cctaatctgg gatgactttt ttggtctctg caacaacagg 9600

cgcttgagcc atctcgtcaa ataatgggga ttgggtttga agatattatt tctaagtcac 9660

actgccgcag ggggcgaatt tgtagttggc tcacatcaac tatctaaaca attcgccttg 9720

caagggcatg aggttgtcca cctcagtccg ccagtgacac cagcacatct gacactgttg 9780

cagaaaacac cgtttgagcg tgtccggttt aagcgctgga tgaaagcggg tgatgtcatc 9840

aaccacgtga ccgacattat tcctttctca ttaatacctt ggggtattgc cacccgcttg 9900

ggatctggta gcgatatctt tggttacttc ctgaagtttt cagcactcag acaactccgt 9960

aaacatcaat tttctaatcc tgacgtagtg ttgattgatg agcctcgatt aggctatcta 10020

ctcaaatatt tccctaagtc aaagatcgta taccgcccca ctgatattta tcatgaaatc 10080

agaaatgatc ccagtattgt agatgctgaa aaagggttga tatcccaggt acacggcttc 10140

attgcgacat cagagcctgt aagacagcat ttgaatcgcc taggtgttga tgaggtgcta 10200

ttgctgcaga atggtgtaga tctagatcat tacctgaatt ttacaaaatc caaactggat 10260

ttgccgcctg agccaagggc aatttatgct ggagctctgg atcatcgctt tggttttgat 10320

gtgctgaaaa gtgcagccac aaaacaccag aaaatttcat ttttattggc tgggccagta 10380

gaggaaaaat acaaggcggc atttagtgat cttgccaacg tacactttct tggtcatgtg 10440

caatataaag cgttgcccaa tctcttgagt cagtgccaaa ttggtttatt gccgatgtct 10500

gatcatccat ccaaccttgg ccgtagtcct atgaaattgt ttgaatatgc agcagtcgga 10560

cttcccgttg tttctacctc aacaccagag cttgagcgca gatcattacc ctttgtattt 10620

ttggctgaga atgcccagat tttcacagat aaacttggtg atgtttgcaa tgcggatatc 10680

aaaatattgg ctcagcaggc tcgagataat gctaagcatg agtcttgggc aagtaaaacc 10740

atcacaatac tgaactatat ttattcaaag taaagcaatg aacaaaacta ctgaaccaca 10800

agatagtcaa accaatgttc catggtggct atcccctcat ggactatgct taggctttct 10860

tttgcctatg atcttgttga tctggtggtt ggggtcagtg agatcaccaa cattaaccat 10920

acggggttat atatatttaa gttctgaata tatagctctg gctgtgctaa ttgttgtgct 10980

gtcagcaagt gcagcctgga tgggaagctt cgtcaagaca aaagtcagag tgccgccaga 11040

ctctcaggtt aatatggctt tgcgggtatt aggctatgta gcagttgcag catatatata 11100

ctggttcaaa gagctgatta tcaatcctat gaatattatt aatatattta cagggacagg 11160

tttatcaaga caggaaattg gtgcaacgca agggattaca tcacttgtta acttcacccc 11220

aatatttatt tcgatttaca tatatgccag atatatcgcc aaattacctt taagacaaga 11280

tgttgtattg ttgttctatg caattgtttt attgacattg ctgcgggtat ttgcctgggc 11340

agagcgtctg gcattaattg aaattatttt gccatttgcg ttattgataa cgcggcaaca 11400

agtgttcagt aagattaaaa ttttgcaatt cttcattaag gctggtccct tcgcgggatt 11460

gcccttgctg gtgctttact ttggtatggc ggaatacttc cgctcttgga gctctacaac 11520

ttacgccggg aaatcgagtt tttgggattt tgctctaggg cggattgctt catattatta 11580

cacctcgctc aataatggtg cgggaatgct ggctactgtt aactggccaa catatgaatt 11640

cgaaaatgtg ttgaattgga tgcataaatt tccaattttt ggcggcgtgt ttcgttacta 11700

cgtagatttg cacactatga gaatgggtga gtttctcgct cagtatggtg atgaggagtt 11760

caacaaccct tctgggatat tttcagtgat cctggatttg ggcttgccat ttggcattat 11820

ctacttcttt ataaccgcat ttcttgcagg aatcttttat aaaggctatt tgagaggcaa 11880

ttttttaagt gtaatttttt atccgttatt ttttattacg ttattggaaa ttttcagata 11940

tccgtattta ggaacgtccc gagcttttac cgagattatt ggtgtgatta ttgctttctt 12000

tatagttaag ggaaggactg aaaatgatag aaaatcgtag acacctggtt aaatcattat 12060

tcttgatgct attttcatcc tggcttccca aggtcgcctc ggcagcgcct aaggtggtcg 12120

ggggcaatgc agacttcact agagctttag atgacgcgct tgctggacgc acggaagtgc 12180

taagcggtta ttatgaaata agtaatcctt taagattttc actgaagagt ggcaccgtaa 12240

aatttgcacc agattttcag ctgaaattct tgaatgataa attaagtggt ttagtatttt 12300

cgagttgcag tgatttatca ctcattggtg gagatttttc gtacaaaaac cccccagcta 12360

gaagaattgg acatggtggt atttacgagt tctctagatg taccaaagtc atcgttagag 12420

atggatttta ttatgacaca ccaggtacag gtatcatttt taccttatgt gatgattgtg 12480

cagtaattaa agctacggtt tggaattcct gggctgatgg gttgcacttt gcaaattgca 12540

ataactgcat cgccttggat tatcagtcta ccaatacagg ggatgatggg ttggcgtttg 12600

taaactacaa aaaacatccc gatgcagtag gtggttatgc gaacaacatt gttatcaatg 12660

gttcttatgc acgaggaatt gcgattatcg gtcaatctgg tgtgactata gacaagtttg 12720

aaattaggca cacttccgca gaaggattaa agattggaag tgagcaatac tatgacacta 12780

gaattccttc caatgtgaca atcaaaaacg gcattgttga agaggcaggt acttggtgcg 12840

tggcaaaggc aactggcaat ggtttcctag tccaaggtgc taccgataaa attgttgttg 12900

agaatttgac tgtcaatgcc ccacgcgtgc atggaattgt tttcgacagg catggcggta 12960

atgtcaaaat gaaagatatc gtcgtcaaga atacaaaagc aagtggcata accatgatgg 13020

cggataacat cgagttagtg aatggggagt ttttaaatat tggaacaaca cctctttacg 13080

tcaataaagc attaactgct aatttctcca agattaatgt caataccttc ggattcgcag 13140

ataaatcaag ttggcctatt ttctgggctg ttgaagctaa gaatgtgtta attaaccaaa 13200

tgaaagtgga gtccaccacc ttggttagaa ttggtgggaa aaacgttatt tcacatcaga 13260

taactggggt gcagatcagt ggtgtgcagc ttagcggact gctctctgat gggcttaatt 13320

ccaaaacaac atcggcggtt tctgctataa catcaactgc tgttaagttt attgagcagt 13380

ggaattagca agtggatgca ggtgcgagat tgaggatttc atggatcgat gtctgccgtg 13440

gatttgcaat gctgttggtt gtggttgggc ataatcagat attaatgggc caaagtcttt 13500

ctttggtaaa atttatttat ctttttcata tgccactgtt cttttttatt agtggtataa 13560

ccctcaagcc agatcaagga ttaacgtact atttcacgcg ggcgattaat gttattgcgg 13620

tatattttgt attttcattt ttagttcttc cttttgtttt aatgaaaaat ccagatgatg 13680

gtgttttcaa tatagttctt ggtattttgt acggaactgg acatacaata gaagttgctc 13740

ctctctggtt tataccatgt ttatttttct ctattgtttt ggtcggtctc gcattagtta 13800

tttcaaatca ttttaataac actgatcgtg aatcaaatag aagtccgatt tttttccttg 13860

cattattgtt tatcggagtt ggtgtttttc tcattaactc aattcattat tatttaattg 13920

ataatcttgc ttggggtagt ctgaaaaatt caggaatgat ttggagtatt gatatagcaa 13980

taattggtgc tggctttatt cttctaggta tgtacgcaaa ggtatttgtt gataagtaca 14040

aaattactga aatcaaatca atatatctta ttgtatctat tgtttgtgcg atttcgttta 14100

tgttggggtt gtttagtttt ggtgttgacg tcgatttgaa ttttagaaaa attgcgcatt 14160

atcctttggc tatgctctgc atgatcttag gaatattttt cagtattttg ttttcaattt 14220

ttctagataa atatacctac ctatcaacta tcttgagcag tattggtaaa tctacgttat 14280

tgattctttt atttcacaat gcgattcaaa atcatttaat tttcacaatt ggtgattcaa 14340

aaatatatag tgctgtgatc ggcattgtta tcgcaatatt gatacctata gtacttgata 14400

agtttgttgt gaataaatac gaattgttca gaaaaatagt ttacccaaaa ctatctatca 14460

accttgcatc caagataatg gtagggaaaa gattttgatt gtatatatta atggccgatt 14520

tttaggaaga cgcataactg gggttgatcg atacgcaata gaagtattaa aagagttaaa 14580

tacactgatt gcggaaaatc atcccagtgt agctggtgtt gaatataaga ttgttattcc 14640

tcctcgcatc aaaactgact tgcatctaac tcatatacct atagtggctt atggtttttt 14700

tagtgggcat gcgtgggagc aattgtccct actaatgttg agtctgagta gtaagtcaat 14760

ggtattaaat ttatgtaata caggctcaat cttcgtgcgt agtcaaatcg tcgtcattca 14820

cgatgctgca gtggtgagaa ttcctagtac attttcttta gcatttcggg tgtggtacaa 14880

aacactcatg ccagtactcg gcagagtcgc aaagaaaatt gtaacggttt cagaatattc 14940

gaaaaaagac attattaact tttttggtgt acatcctgaa aaagttcaag ttgttggtgc 15000

aggcggcgag catattttgt cgcaacagcc agacgcaggt attcttgaaa aatttcaagt 15060

tcacccgggc tcttatgtgt tggcggttag tagtcatgct ccgcacaaaa atttttcttt 15120

aaccattgaa tctttaaaac tttctaagaa tgcaaatatt ccgtatttga ttgcaggtgg 15180

ggctaatcag aaaatttttg gtgaaaattc ccagttagat tcttctaagg tgacgttgct 15240

gggttatgtc tcggataatg agttgcgcgc gctttatgaa catgcgcttt gttttgtttt 15300

tccatcactt tatgagggct tcggtcttcc tccactggag gcgatgaatt gcggctgccc 15360

cgtacttgca tcaaatgctg catcaattcc agaggtgtgc agtgatgctg cattatattt 15420

ttcaccgcat gatccgcagg agctttcaaa gcttatagat aatgttgctc atgatgccga 15480

tttgagaaag tcgttagtca accaaggctt gaagcgagca aaagattttt cttggaaaaa 15540

ttccgcaata gatattgcac ggatgtgcaa ggagattttc acaataaagt aatagtatta 15600

tctgccaagc tgaatttttc tttataaagt ctaggccagg catgttgatt attaactaga 15660

ttatgactaa taaatatacg aggcattcga gattgcaccc aaaaaaagta gctatcgttg 15720

tggattggct taatgaatat gctggagcag agcgtgttct ggagcagttg gttctttgct 15780

atcctgatgc agacattttt gctgtcattg aattcttacc tgataacctg agaggtttta 15840

ttcagaataa gaaggttacg acaactttta tccaaaactt gcctaaagct aaaacgcatt 15900

atcgcaatta tttgccacta atgcctttag ctattgagca gctcgatctg tctcaatatg 15960

atttgattat ttctagttct cacgccgtgg ccaagggtgt gcttactggg ccagatcaag 16020

tgcatgtaag ttatgtccat tctcccatga gatatgcgtg ggatttgcaa catcaatact 16080

tgcgtgagtc taatttggtt tcaggtttta aatcttggat tgtcaaatgg ttgctacata 16140

aattgcgtat ttgggatcta agaacggcga acggggtaga tgtttttatc gcaaactctt 16200

cttttattgc taaaagaata aagaaggtat atggacgtga ggctagggtt atttatcctc 16260

ctgtagatac caacagtttt gcattccagg aaaagaaaga tgactttttc ttgactgcct 16320

cgagattggt accttacaaa cgagttgact tgattgtgga agccttttcc aaaatcccag 16380

atcagaaatt agttgtcatt ggggatggtc cagacatgtc caaggtgcgt gcaaaagcag 16440

ggccaaatgt tgaaatcctc ggttatcagt ctcatgctgt gttaagagac tacatgcagc 16500

gtgcgaaagg gtttgtcttc gcggcggagg aagattttgg tatttctcct gttgaggcac 16560

aagcatgtgg tactcccgtg attgctttcg ccaaaggtgg cgccttggag acagttattc 16620

cagaagagcg aggtgatcct gccactggtt tgttcttcca tgagcagaca gctgatgcca 16680

tcgtggatgc tattactaga tttaatctac tctctcaaaa gattagtcca tttgcatgca 16740

gagagaacgc actgcgcttt tctcctgatc gatttagaaa tgagattatt tcaactgtga 16800

ataaggctct cgattaatta ttttctctta cgacatatag taagttctca tatttaattt 16860

tataggatag ttgctatgaa aatagctgtt gcaggtactg ggtatgtggg cttgtcgatt 16920

gctgtgcttt tagcacagaa aaatgaagtt gttgctcttg atatcgtccc tgataaagtt 16980

gaccttatca acaataaaat ttctccaatc gttgataaag agattgagca ttatcttaag 17040

aatattccat tgaacctacg tgcgactaca aacaagcatg atgcatatga aaatgcagat 17100

tttgtagtga ttgcaacccc tacggactat gatccggtta ccagctactt caatacctcc 17160

tctgtagagg cagtgatcaa ggatgtccag gcgattgcgc ccaacgcgac catggtgatc 17220

aaatccacca tccctgttgg ttataccaaa atgatttccg aaaagttggg tgtggataac 17280

ctgattttct ctcctgaatt cttgcgtgaa ggtaaggcac tttacgacaa cctgtatccc 17340

tcgcgtatcg tcattggtga aatttcggat cgcgcacaag tttttgccga ccttctcaat 17400

gcaggtgctg tgaaagagaa tattccggtg ctattgacca atagtgctga agcggaggcc 17460

attaagctgt ttgccaacac cttcctcgcc atgcgcgtcg cctatttcaa tgagctggat 17520

acttatgccg ccacccatgg cctgaacaca cgtcagatca tagatggggt gtgcctagac 17580

ccacgtgtcg gtgaccacta taacaatcca tcgtttggct atggtggtta ctgcctgcct 17640

aaggatacca agcaactgct ggccaattac gacaacgtgc cgcaaaaact catcaatgcc 17700

attgttgatg ccaacaccac gcgcaaggat ttcatcgccc agagcatcat caagcgtgac 17760

cccaaggtgg tgggtatcta ccgcctgatc atgaaagcag gctcggataa cttccgcgcc 17820

agttcaatcc agggcgtgat gaagcgcatc aaggcacgtg gtattaaggt cgtggtgttt 17880

gagcctgagt tgaaagaaga tcaattcttc aattcagagg tgatccgcaa tctggaagag 17940

ttcaagaaga tttcagacgt cattgtcgcc aatagaaggg cgcaggattt gtcagacgtt 18000

gatgagaaag tatatacgcg tgatttgttt ggcaatgact agccagattg aaaaggtgtt 18060

gggttaagga tatgaggata cttttttcac ctgctacggc gagtaatcag tatgtcgaga 18120

acacgattgc cctgattaaa accctagacg ctacccatgt cgaaacccaa gccagattga 18180

atgctggatt catcttgcgc aatgctttca accgctatga ttgcattatc atcaactggc 18240

tggaaaacaa cctggttgga aaaaacaagt cgcttgatat atgggcggta gcaaaatttc 18300

tgttcaagtt tttcatctac agagtcatgg ggagaaagtt gatttttgtc aggcacaata 18360

tttatcctca tgccttgaag ggtcggcagg catccatcgc gacctggatc attgaagcgg 18420

cgattaagct ctcaacaata tccgttgtac attcagggca tctcgaaaat gagaacgcga 18480

aatatgtacc tcatcctttg tacagggttg attcgcaggt tgaaggccaa ggcagtgatt 18540

attatgtaat ttttggcagg attgttgcgt ataaagccat ccataaatta ctcgaaagct 18600

gggggtcgca gcggctaatc atagccggca ttgtcgagga tcatgcctac gctgccagct 18660

tgcaatccat catacagtcc agagggttga gcaatgttga actgcgtgct gaattcctct 18720

ccgaagccga ggctgaacaa ctggtagcta acagcaaggg cttgattctt tcacattgcg 18780

atgccgacat gattgtcagt ggtagttttt tctttgctgc aagtctgggc gtgcctgttt 18840

ttgccatgga tactccattt ttccagtggc tgaagaatga gcaaggctat cagggcttgc 18900

aggtgttcac atcactgcag gcaatgacgg ctgagctagc cactgccgcg gccgtggaaa 18960

aaaatactat tgctgcttca gcccagcgtt tatttggcaa tgatagggtg agaagtgcat 19020

ggggtagtgt gctccatggt taattcaata ccttatcttt tgtctcactt aatattttga 19080

ttgaattgca acaatgagca aattgatgac cacgataggt caggatgtta accagcctgc 19140

aggcattcct gaagtattta caatcaatga tgtcgttgac agagggctat gcgttggatg 19200

tggtgcgtgc gcggttgtag attcaaacgt caagctaacg cgcaatcaat atggttccat 19260

ggtggccgcc attgatgcca atatcctgcc agggtctatt gccgaccggg tatgcccatt 19320

ttctgataac agtcttaatg aagatgttgt gggagaacgg gtttttgcga gttgtgaaaa 19380

ttttgatcaa cgtaccggtt attacaccaa tttatattat ggccgcaggt tcactgaagc 19440

tgaattgctg aaaagcagtt ctggtgggct gaccagttat gtcgccattg aacttctcaa 19500

gcgtcatcac gtggatggca ttattcatgt gggacgcgac cagtcagatg atcgcgatgc 19560

tttgttcaag ttcatggtgt cttacacaga agaagacgcc aatgcctgca agaaatcaca 19620

atattattcc ctgagttttg atgaggtagt aagaagcata cgcggagatg gtaagcgtta 19680

tgtatttgtg ggcgtgccct gctttgtcaa atctctgaga aatatttgtc tcaatgatgc 19740

tgtgctcaat gagcagattc cattcatgct gggcttggtg tgtggtcatt tgaagtcttc 19800

gcgctttgcc gaattactag cgtggcaact tagtgtgcca ccggaaaaaa tccagagtgt 19860

ggactttaga gtaaaaaata atgcctccac cgtgaattcc tacgatttcc aggtggtgga 19920

tagcaatcag caagcccatg caaaaacctc taatacaatg ctgggcggca attgggggca 19980

tgcggcgttt caacttcagg catgcgatta ttgtgatgat atatttgccg agacagcaga 20040
cgctgtcttt ggtgatgcat ggttgccgca gttctccaca tggtggcaag gtaccaatat 20100
cgtcctggta agaaacccta tcatcgacga cattctggaa tcggggaaaa atgcaggcgc 20160
catcactctg ggcggtgtca gcattgatga tttatgcaag tcgcagttgg gtaatttccg 20220
ccacagaaaa gatggtttga ttgtcaggtt agacgatgac ctgaagcagg gcaagaaggt 20280
ccccaaaaaa cgggttgatc tgtctgcatt aggcaagatt gaccagaaga gaaagaaaat 20340
tgtcaggctc agaaaggaaa tggccgtgaa aagtcacgaa tatttcctgc aggcccgaga 20400
aaagcacgat ctcagccttt atatgaagcc catggggcag ttggcgagaa atatgcttaa 20460
gctgtatagg gatccattta tgaagcgact gatcaatagg ctgaagcgat atttttaata 20520
ttgccttagg tcaatgcgtt atcactcatt aatctaattt ttgaatggtc tagggatttg 20580
agtctatctg taaaagccgc gcgcggtggt gtggtcatga tgtcaggaca aattgtgaag 20640
gtagctttgc agtttggcag cctgattatc ttggcacgcc tgttgcaacc agaagatttt 20700
ggtttggttg ccatggtgat tgcgatttat ggcatttgcg acatcttgct cgactttggc 20760
ctttcgacgg cgtctattca agtccaaaac ataactaaag cgcagatcag caacctgttc 20820
tgggtcaatg tattgatagg tctgttgttt acgctgattt catacttttc agcaagttta 20880
ctggcgagct tttatgctca gccagaatta accaaaattg cgcagtatgt ttctgtggtg 20940
tttttattca atggtattgc cgcacaatat aaagcccagc tgaaccgtaa tttcaaattc 21000
aaacagatgg caataacaga tacgactgct gttttgctca gtgtttgtat cggggtctat 21060
ttcgcatatg ccggacattc ttactgggcc atcgtatatc agcagatagc gcaggcattg 21120
atacaaatgc ttttgtacat gcttttcggc gcttggctac cttccttgcc ttcaagacat 21180
gtttctatca gacatttctt caaattcggt tgggggctgg ctggttctca attgatggga 21240
tacttctcgc gcaatattcc agctatcttg attggtaatc aaataggtgc cagccagttg 21300
gggttttacg atagagccaa caaattactg atgttgccgc taaaccagct gaatgcgcct 21360
tcatcgtcgg ttgctgtccc tgtgttgtca cgtttggcag tggaagatca ggaaaaatac 21420
aatcgttttc ttctgtttgg acaaaatatc attgtccatc tcgttgtatt ttgcctggcc 21480
ttaacctgct gccagacaga gcgcctggtg caattggtgc tcgggacaca atggttggaa 21540
atggtgccgg tctaccgggc actttcattt gcagggatat ttttggcgct cagctatgcc 21600
agttactggg tatttttatc taaagggatt actgcctctc tatttaaact aggcttggtt 21660
agcaggccaa tctctatttt gattatttgc ctggggctgc ccgctggtgt gattggcgta 21720
gcgtatgcat actcggcaag tctgctcttt acctggctac tcggcgtttt ctggctgagg 21780
aaaacgggcg ttcctgtcaa aagcatgatt gtagatccac tcatcatgtc tgccatttac 21840
tatagcgcca gttttatcgc caattacgtg gtcgatcatt atgtggactt tgccatatac 21900

aacattcttg tcggctggtt gctgatgttg ctgatcctgg cagtatttta tttgttggtc 21960

cccaagttca aacgtagcgt gaaaaagtta tttgaagtga aaagttatat tcggaacaaa 22020

caatgaagac cattaaaaaa ttactataca aaatcctagg tagtgcaggc atcaaggcca 22080

aggctgcagc agtcatcaat ctcctgtttg gtcgtgtggc tagaagcact aatgacagtg 22140

tctctattct tattccggca ccaggagggg ggaatatcgg tgatcaggca atgctggaat 22200

catttttgca taatattgat ggcaaaaaac tcattatcaa aacacagcct atagcattgg 22260

atattcctag tcactatgca gtgaacgttg aagtgatcga aatgccagga ttgatctacg 22320

gtcatttgtt caggtttttg cgggattacg taagactgag aaaaataatg gcatctgcca 22380

gcgctttgta tgtagttggc gcagatatca tggatggtgg ctataataaa aatgcctcta 22440

tcatgcgctc taaaattgca acttatttcg cgcgtaataa tattccatcc agggtgtttg 22500

gtttcagttg gaatggcaat cctgatgcag cagcgctttc tgctatcaga aacgcagctg 22560

ccaatggggt tttgctttgt gcgcgagatc ctctttcatt ccagcgtcta caagatgcag 22620

gggttcccaa tatcaggctg gttgcagata tggtgtttgc caatgacaac accaaatcag 22680

ctgggattga agatattttg caattctgcg caggcaagca gattgctctg gtaaatgcca 22740

gtggattgat tggcaaaaat attgatcaaa ttgatgaata tagcaaggta ataaacaagc 22800

tgatggacca caattttaat attgtactat tgcctcacgt gatcagacac ggagccaatg 22860

acttgccgat ttgtcaggca ttgtcaggca agtatccgaa tgctttccta gtaaatagac 22920

tgttggaacc agatcaggtt aagcgcttag cacaagaggc taaaattgtc gtcaccggtc 22980

gcatgcatct ttccatcatg agcctgaatc agaatgttcc atctatcgta ttatcgaccc 23040

aaggcaaggt tgatgggctt atgcaattct tcgaaactga aaaactgtcg attaatccgg 23100

aaaaaggttt ttccgatctt atttgttcac aaattgattt tatttgttct aatcggcaat 23160

attatcaaga cctgattcgt aaaaacctac ttaaggttaa ggaactggcc gctgggaatt 23220

ttaattagtg tgataattat gacttatcat cgggctgctt tcagggtgca aaatagttag 23280

attccccagt taatattcta taataatgtt tatatgtatt aatggttttc attgcatttt 23340

atatctatga tatatgcaca ctaatgttgg tgtaattttc aatatttatt gattagcagg 23400

cacatcataa tttattgcaa gcttttacta taatgatagt gatgaactgt cgaagtgttt 23460

tggattatgt tcacactatg tctccaggga ttatatgtct aaattctata aatttcaggt 23520

gttagaatca ctaagaggtt atatggcatg gtgggttgtt ttaggtcatg ctatccaact 23580

ttctggtttt tggcacaagc aagaagtaga tgagtttttt acaggagggg gcataattaa 23640

tgccactttt tcaaaaatac tcagtttaat catcaatcca gaacttgcag tcaacatttt 23700

tattatttta agtggttttg tgattactca tttattgctt actaaaaatg agccatattc 23760

acatttcata actagacgat ttttccgctt ggcgcccgca ttttacgttt cgttagttat 23820

cgcgattttg gtagttaatt tttacaaggt tgcatatacc ggtctgagtt tttctggtga 23880

tacctcgatg cgactcgcca ggcttgttga gcaagatagt aattttttat tgcatttatt 23940

caatcattta ctcctcattc atagcgtttt cccgcaagag ctgattcaat tttcacctac 24000

cacgtttctt gctccggcat ggagcatcag tcttgagtgg cagttctatc taattgcacc 24060

agtcttaatt ccatttttgg tgaagtctga gaagtctttg attgtttggg ttgtgttgtt 24120

gctactgctg cgatatttta ccaaccatgt tttagacttt aattggcaac atagctcctt 24180

cttctttaaa gctgtcgatt atttcttggt cggcatctta agcagattaa ttatcgagcg 24240

actgcagcat ggcaagttta tttatgatgt tgccgtgttg ctaggtggaa tattgctttt 24300

ccaggataga tattgcgctg ttatctggct gtttgttttg gctgtcatat tttatgaaat 24360

gggttattgg agtgttaaat ctcccctatg gaaaaaaatc ctgacattat ttttctacaa 24420

taaagtcatc acgaatattg gtaagtggtc ttattctact tacctaatcc atattcctat 24480

tttctcaatt ttcttaggtg gttatgcaac tctgactgac cctggcactg taacccagca 24540

aatttctttg attatcctat ttgtcagttt tccagtggtt ttgctgattt caaatattat 24600

gtatcacaac atagagctgc catcgatgaa ttatatttcc gcaaaactaa aagaaaaacg 24660

tatcactgaa tctgcagagt gatggttaat tctcttattg catgctggaa taattattct 24720

ttcaatgtga gtgccatatt tttttaatgt tattaattta tcgttttaga gattttaatt 24780

atattttcta tttgattttt taatgcaaga tttttatcta ttaaacaatt aattattgat 24840

tggaatgaaa aatgaaaatt gttcctgtat tactctccgg tggttctggt acacggttat 24900

ggccactttc tcgagcggta ttacctaagc aactgttgcc cttgatcact aataaaacca 24960

tgctgcagga gacccttctc agaatttcaa gatgggcgac tgtaacaaat ccagttgttg 25020

tttgcggtaa cgatcatcga ttcttagtgg ctgaacagtt gcgcgaaatc gctttgagtc 25080

ctaactctat tattctagag cctgccccca agaatactgc accagctatt gccgctgcag 25140

cattgcagct acaaggtcag gatgtttata tgctggtctt gcctgctgat catgtcatca 25200

gggatctgca aagttttgaa catgctgtgc aaaccgcatt gactgccgct gatcaaggta 25260

acttagtcac cttcggtatc aaacctgctc atcctgaaac cggttatggc tacatcaaat 25320

taggcgttga gagcgcacca ggatcttata aggtcaatcg ttttgttgag aagccagatc 25380

ttgccacagc aactgaatat gtggcgtcag gtgagttttt ctggaacagt ggcatgtttt 25440

tattcaagcc agaaactttc ctcaagcaac tggaaatcca tgcccccgaa attactgctg 25500

cagtaagcaa ggccgtcaat aacagttata aggatctgga tttcgtcaga ttagaagaat 25560

cgagttttgc tgagtctccg tccttatcca tcgattatgc ggtaatggaa aaaaccgatc 25620

atgctgttgt agtgcctgct gatattggtt ggagtgacgt tggctcatgg acgtctctac 25680

aagaggtgca agagcaggat gccaatggca atgtagctcg tggcgatgtt tacctcaaga 25740

atgtcgagaa cagtctggtg cggagtgagg ataggtttgt tgccgcgatt ggtgtccgtg 25800

atctgatcgt ggtggagacc agtgatgccg ttctggtagc tagcaaggat gctgcacaag 25860

atgtcaagga ggtagtggaa tacctcaagt taaatcaacg ctctgagcac aagatacatc 25920

cacgggccta caggccttgg ggttggtatg aaagtattga tctgggcgag cgccaccagg 25980

tgaaacgtat catggtgaag ccgggcgaaa agctgtccct gcaaatgcac caccatcgtg 26040

ccgagcattg ggttgtagtc agtggttcag ccatgattac ggtggatggg aataccaagc 26100

tattcagtga aaatcagtca acgtacattc caattggctc cacccataga ttggagaatc 26160

cgggcaagtt gccgctgcac ttgatcgagg ttcagtccgg aagttatctg ggcgaagatg 26220

acattgtgcg ttacgaggat acctacggcc ggtcctgata agaaaagcgt cgtcctcagg 26280

ctagtgtcat gc 26292

<210> 178
<211> 26292
<212> DNA
<213> Methylobacillus glycogenes

<400> 178
gcatgacact agcctgagga cgacgctttt cttatcagga ccggccgtag gtatcctcgt 60

aacgcacaat gtcatcttcg cccagataac ttccggactg aacctcgatc aagtgcagcg 120

gcaacttgcc cggattctcc aatctatggg tggagccaat tggaatgtac gttgactgat 180

tttcactgaa tagcttggta ttcccatcca ccgtaatcat ggctgaacca ctgactacaa 240

cccaatgctc ggcacgatgg tggtgcattt gcagggacag cttttcgccc ggcttcacca 300

tgatacgttt cacctggtgg cgctcgccca gatcaatact ttcataccaa ccccaaggcc 360

tgtaggcccg tggatgtatc ttgtgctcag agcgttgatt taacttgagg tattccacta 420

cctccttgac atcttgtgca gcatccttgc tagctaccag aacggcatca ctggtctcca 480

ccacgatcag atcacggaca ccaatcgcgg caacaaacct atcctcactc cgcaccagac 540

tgttctcgac attcttgagg taaacatcgc cacgagctac attgccattg gcatcctgct 600

cttgcacctc ttgtagagac gtccatgagc caacgtcact ccaaccaata tcagcaggca 660

ctacaacagc atgatcggtt ttttccatta ccgcataatc gatggataag gacggagact 720

cagcaaaact cgattcttct aatctgacga aatccagatc cttataactg ttattgacgg 780

ccttgcttac tgcagcagta atttcggggg catggatttc cagttgcttg aggaaagttt 840

ctggcttgaa taaaaacatg ccactgttcc agaaaaactc acctgacgcc acatattcag 900

ttgctgtggc aagatctggc ttctcaacaa aacgattgac cttataagat cctggtgcgc 960

tctcaacgcc taatttgatg tagccataac cggtttcagg atgagcaggt ttgataccga 1020

aggtgactaa gttaccttga tcagcggcag tcaatgcggt ttgcacagca tgttcaaaac 1080

tttgcagatc cctgatgaca tgatcagcag gcaagaccag catataaaca tcctgacctt 1140

gtagctgcaa tgctgcagcg gcaatagctg gtgcagtatt cttgggggca ggctctagaa 1200

taatagagtt aggactcaaa gcgatttcgc gcaactgttc agccactaag aatcgatgat 1260

cgttaccgca aacaacaact ggatttgtta cagtcgccca tcttgaaatt ctgagaaggg 1320

tctcctgcag catggtttta ttagtgatca agggcaacag ttgcttaggt aataccgctc 1380

gagaaagtgg ccataaccgt gtaccagaac caccggagag taatacagga acaattttca 1440

tttttcattc caatcaataa ttaattgttt aatagataaa aatcttgcat taaaaaatca 1500

aatagaaaat ataattaaaa tctctaaaac gataaattaa taacattaaa aaaatatggc 1560

actcacattg aaagaataat tattccagca tgcaataaga gaattaacca tcactctgca 1620

gattcagtga tacgtttttc ttttagtttt gcggaaatat aattcatcga tggcagctct 1680

atgttgtgat acataatatt tgaaatcagc aaaaccactg gaaaactgac aaataggata 1740

atcaaagaaa tttgctgggt tacagtgcca gggtcagtca gagttgcata accacctaag 1800

aaaattgaga aaataggaat atggattagg taagtagaat aagaccactt accaatattc 1860

gtgatgactt tattgtagaa aaataatgtc aggatttttt tccatagggg agatttaaca 1920

ctccaataac ccatttcata aaatatgaca gccaaaacaa acagccagat aacagcgcaa 1980

tatctatcct ggaaaagcaa tattccacct agcaacacgg caacatcata aataaacttg 2040

ccatgctgca gtcgctcgat aattaatctg cttaagatgc cgaccaagaa ataatcgaca 2100

gctttaaaga agaaggagct atgttgccaa ttaaagtcta aaacatggtt ggtaaaatat 2160

cgcagcagta gcaacaacac aacccaaaca atcaaagact tctcagactt caccaaaaat 2220

ggaattaaga ctggtgcaat tagatagaac tgccactcaa gactgatgct ccatgccgga 2280

gcaagaaacg tggtaggtga aaattgaatc agctcttgcg ggaaaacgct atgaatgagg 2340

agtaaatgat tgaataaatg caataaaaaa ttactatctt gctcaacaag cctggcgagt 2400

cgcatcgagg tatcaccaga aaaactcaga ccggtatatg caaccttgta aaaattaact 2460

accaaaatcg cgataactaa cgaaacgtaa aatgcgggcg ccaagcggaa aaatcgtcta 2520

gttatgaaat gtgaatatgg ctcattttta gtaagcaata aatgagtaat cacaaaacca 2580

cttaaaataa taaaaatgtt gactgcaagt tctggattga tgattaaact gagtattttt 2640

gaaaaagtgg cattaattat gccccctcct gtaaaaaact catctacttc ttgcttgtgc 2700

caaaaaccag aaagttggat agcatgacct aaaacaaccc accatgccat ataacctctt 2760

agtgattcta acacctgaaa tttatagaat ttagacatat aatccctgga gacatagtgt 2820

gaacataatc caaaacactt cgacagttca tcactatcat tatagtaaaa gcttgcaata 2880

aattatgatg tgcctgctaa tcaataaata ttgaaaatta caccaacatt agtgtgcata 2940

tatcatagat ataaaatgca atgaaaacca ttaatacata taaacattat tatagaatat 3000

taactgggga atctaactat tttgcaccct gaaagcagcc cgatgataag tcataattat 3060

cacactaatt aaaattccca gcggccagtt ccttaacctt aagtaggttt ttacgaatca 3120

ggtcttgata atattgccga ttagaacaaa taaaatcaat ttgtgaacaa ataagatcgg 3180

aaaaaccttt ttccggatta atcgacagtt tttcagtttc gaagaattgc ataagcccat 3240

caaccttgcc ttgggtcgat aatacgatag atggaacatt ctgattcagg ctcatgatgg 3300

aaagatgcat gcgaccggtg acgacaattt tagcctcttg tgctaagcgc ttaacctgat 3360

ctggttccaa cagtctattt actaggaaag cattcggata cttgcctgac aatgcctgac 3420

aaatcggcaa gtcattggct ccgtgtctga tcacgtgagg caatagtaca atattaaaat 3480

tgtggtccat cagcttgttt attaccttgc tatattcatc aatttgatca atatttttgc 3540

caatcaatcc actggcattt accagagcaa tctgcttgcc tgcgcagaat tgcaaaatat 3600

cttcaatccc agctgatttg gtgttgtcat tggcaaacac catatctgca accagcctga 3660

tattgggaac ccctgcatct tgtagacgct ggaatgaaag aggatctcgc gcacaaagca 3720

aaaccccatt ggcagctgcg tttctgatag cagaaagcgc tgctgcatca ggattgccat 3780

tccaactgaa accaaacacc ctggatggaa tattattacg cgcgaaataa gttgcaattt 3840

tagagcgcat gatagaggca tttttattat agccaccatc catgatatct gcgccaacta 3900

catacaaagc gctggcagat gccattattt ttctcagtct tacgtaatcc cgcaaaaacc 3960

tgaacaaatg accgtagatc aatcctggca tttcgatcac ttcaacgttc actgcatagt 4020

gactaggaat atccaatgct ataggctgtg ttttgataat gagttttttg ccatcaatat 4080

tatgcaaaaa tgattccagc attgcctgat caccgatatt cccccctcct ggtgccggaa 4140

taagaataga gacactgtca ttagtgcttc tagccacacg accaaacagg agattgatga 4200

ctgctgcagc cttggccttg atgcctgcac tacctaggat tttgtatagt aattttttaa 4260

tggtcttcat tgtttgttcc gaatataact tttcacttca aataactttt tcacgctacg 4320

tttgaacttg gggaccaaca aataaaatac tgccaggatc agcaacatca gcaaccagcc 4380

gacaagaatg ttgtatatgg caaagtccac ataatgatcg accacgtaat tggcgataaa 4440

actggcgcta tagtaaatgg cagacatgat gagtggatct acaatcatgc ttttgacagg 4500

aacgcccgtt ttcctcagcc agaaaacgcc gagtagccag gtaaagagca gacttgccga 4560

gtatgcatac gctacgccaa tcacaccagc gggcagcccc aggcaaataa tcaaaataga 4620

gattggcctg ctaaccaagc ctagtttaaa tagagaggca gtaatccctt tagataaaaa 4680

tacccagtaa ctggcatagc tgagcgccaa aaatatccct gcaaatgaaa gtgcccggta 4740

gaccggcacc atttccaacc attgtgtccc gagcaccaat tgcaccaggc gctctgtctg 4800

gcagcaggtt aaggccaggc aaaatacaac gagatggaca atgatatttt gtccaaacag 4860

aagaaaacga ttgtattttt cctgatcttc cactgccaaa cgtgacaaca cagggacagc 4920

aaccgacgat gaaggcgcat tcagctggtt tagcggcaac atcagtaatt tgttggctct 4980

atcgtaaaac cccaactggc tggcacctat ttgattacca atcaagatag ctggaatatt 5040

gcgcgagaag tatcccatca attgagaacc agccagcccc caaccgaatt tgaagaaatg 5100

tctgatagaa acatgtcttg aaggcaagga aggtagccaa gcgccgaaaa gcatgtacaa 5160

aagcatttgt atcaatgcct gcgctatctg ctgatatacg atggcccagt aagaatgtcc 5220

ggcatatgcg aaatagaccc cgatacaaac actgagcaaa acagcagtcg tatctgttat 5280

tgccatctgt ttgaatttga aattacggtt cagctgggct ttatattgtg cggcaatacc 5340

attgaataaa aacaccacag aaacatactg cgcaattttg gttaattctg gctgagcata 5400

aaagctcgcc agtaaacttg ctgaaaagta tgaaatcagc gtaaacaaca gacctatcaa 5460

tacattgacc cagaacaggt tgctgatctg cgctttagtt atgttttgga cttgaataga 5520

cgccgtcgaa aggccaaagt cgagcaagat gtcgcaaatg ccataaatcg caatcaccat 5580

ggcaaccaaa ccaaaatctt ctggttgcaa caggcgtgcc aagataatca ggctgccaaa 5640

ctgcaaagct accttcacaa tttgtcctga catcatgacc acaccaccgc gcgcggcttt 5700

tacagataga ctcaaatccc tagaccattc aaaaattaga ttaatgagtg ataacgcatt 5760

gacctaaggc aatattaaaa atatcgcttc agcctattga tcagtcgctt cataaatgga 5820

tccctataca gcttaagcat atttctcgcc aactgcccca tgggcttcat ataaaggctg 5880

agatcgtgct tttctcgggc ctgcaggaaa tattcgtgac ttttcacggc catttccttt 5940

ctgagcctga caattttctt tctcttctgg tcaatcttgc ctaatgcaga cagatcaacc 6000

cgttttttgg ggaccttctt gccctgcttc aggtcatcgt ctaacctgac aatcaaacca 6060

tcttttctgt ggcggaaatt acccaactgc gacttgcata aatcatcaat gctgacaccg 6120

cccagagtga tggcgcctgc atttttcccc gattccagaa tgtcgtcgat gatagggttt 6180

cttaccagga cgatattggt accttgccac catgtggaga actgcggcaa ccatgcatca 6240

ccaaagacag cgtctgctgt ctcggcaaat atatcatcac aataatcgca tgcctgaagt 6300

tgaaacgccg catggcccca attgccgccc agcattgtat tagaggtttt tgcatgggct 6360

tgctgattgc tatccaccac ctggaaatcg taggaattca cggtggaggc attatttttt 6420

actctaaagt ccacactctg gattttttcc ggtggcacac taagttgcca cgctagtaat 6480

tcggcaaagc gcgaagactt caaatgacca cacaccaagc ccagcatgaa tggaatctgc 6540

tcattgagca cagcatcatt gagacaaata tttctcagag atttgacaaa gcagggcacg 6600

cccacaaata cataacgctt accatctccg cgtatgcttc ttactacctc atcaaaactc 6660

agggaataat attgtgattt cttgcaggca ttggcgtctt cttctgtgta agacaccatg 6720

aacttgaaca aagcatcgcg atcatctgac tggtcgcgtc ccacatgaat aatgccatcc 6780

acgtgatgac gcttgagaag ttcaatggcg acataactgg tcagcccacc agaactgctt 6840

ttcagcaatt cagcttcagt gaacctgcgg ccataatata aattggtgta ataaccggta 6900

cgttgatcaa aattttcaca actcgcaaaa acccgttctc ccacaacatc ttcattaaga 6960

ctgttatcag aaaatgggca tacccggtcg gcaatagacc ctggcaggat attggcatca 7020

atggcggcca ccatggaacc atattgattg cgcgttagct tgacgtttga atctacaacc 7080

gcgcacgcac cacatccaac gcatagccct ctgtcaacga catcattgat tgtaaatact 7140

tcaggaatgc ctgcaggctg gttaacatcc tgacctatcg tggtcatcaa tttgctcatt 7200

gttgcaattc aatcaaaata ttaagtgaga caaaagataa ggtattgaat taaccatgga 7260

gcacactacc ccatgcactt ctcaccctat cattgccaaa taaacgctgg gctgaagcag 7320

caatagtatt tttttccacg gccgcggcag tggctagctc agccgtcatt gcctgcagtg 7380

atgtgaacac ctgcaagccc tgatagcctt gctcattctt cagccactgg aaaaatggag 7440

tatccatggc aaaaacaggc acgcccagac ttgcagcaaa gaaaaaacta ccactgacaa 7500

tcatgtcggc atcgcaatgt gaaagaatca agcccttgct gttagctacc agttgttcag 7560

cctcggcttc ggagaggaat tcagcacgca gttcaacatt gctcaaccct ctggactgta 7620

tgatggattg caagctggca gcgtaggcat gatcctcgac aatgccggct atgattagcc 7680

gctgcgaccc ccagctttcg agtaatttat ggatggcttt atacgcaaca atcctgccaa 7740

aaattacata ataatcactg ccttggcctt caacctgcga atcaaccctg tacaaaggat 7800

gaggtacata tttcgcgttc tcattttcga gatgccctga atgtacaacg gatattgttg 7860

agagcttaat cgccgcttca atgatccagg tcgcgatgga tgcctgccga cccttcaagg 7920

catgaggata aatattgtgc ctgacaaaaa tcaactttct ccccatgact ctgtagatga 7980

aaaacttgaa cagaaatttt gctaccgccc atatatcaag cgacttgttt tttccaacca 8040

ggttgttttc cagccagttg atgataatgc aatcatagcg gttgaaagca ttgcgcaaga 8100

tgaatccagc attcaatctg gcttgggttt cgacatgggt agcgtctagg gttttaatca 8160

gggcaatcgt gttctcgaca tactgattac tcgccgtagc aggtgaaaaa agtatcctca 8220

tatccttaac ccaacacctt ttcaatctgg ctagtcattg ccaaacaaat cacgcgtata 8280

tactttctca tcaacgtctg acaaatcctg cgcccttcta ttggcgacaa tgacgtctga 8340

aatcttcttg aactcttcca gattgcggat cacctctgaa ttgaagaatt gatcttcttt 8400

caactcaggc tcaaacacca cgaccttaat accacgtgcc ttgatgcgct tcatcacgcc 8460

ctggattgaa ctggcgcgga agttatccga gcctgctttc atgatcaggc ggtagatacc 8520

caccaccttg gggtcacgct tgatgatgct ctgggcgatg aaatccttgc gcgtggtgtt 8580

ggcatcaaca atggcattga tgagtttttg cggcacgttg tcgtaattgg ccagcagttg 8640

cttggtatcc ttaggcaggc agtaaccacc atagccaaac gatggattgt tatagtggtc 8700

accgacacgt gggtctaggc acaccccatc tatgatctga cgtgtgttca ggccatgggt 8760

ggcggcataa gtatccagct cattgaaata ggcgacgcgc atggcgagga aggtgttggc 8820

aaacagctta atggcctccg cttcagcact attggtcaat agcaccggaa tattctcttt 8880

cacagcacct gcattgagaa ggtcggcaaa aacttgtgcg cgatccgaaa tttcaccaat 8940

gacgatacgc gagggataca ggttgtcgta aagtgcctta ccttcacgca agaattcagg 9000

agagaaaatc aggttatcca cacccaactt ttcggaaatc attttggtat aaccaacagg 9060

gatggtggat ttgatcacca tggtcgcgtt gggcgcaatc gcctggacat ccttgatcac 9120

tgcctctaca gaggaggtat tgaagtagct ggtaaccgga tcatagtccg taggggttgc 9180

aatcactaca aaatctgcat tttcatatgc atcatgcttg tttgtagtcg cacgtaggtt 9240

caatggaata ttcttaagat aatgctcaat ctctttatca acgattggag aaattttatt 9300

gttgataagg tcaactttat cagggacgat atcaagagca acaacttcat ttttctgtgc 9360

taaaagcaca gcaatcgaca agcccacata cccagtacct gcaacagcta ttttcatagc 9420

aactatccta taaaattaaa tatgagaact tactatatgt cgtaagagaa aataattaat 9480

cgagagcctt attcacagtt gaaataatct catttctaaa tcgatcagga gaaaagcgca 9540

gtgcgttctc tctgcatgca aatggactaa tcttttgaga gagtagatta aatctagtaa 9600

tagcatccac gatggcatca gctgtctgct catggaagaa caaaccagtg gcaggatcac 9660

ctcgctcttc tggaataact gtctccaagg cgccaccttt ggcgaaagca atcacgggag 9720

taccacatgc ttgtgcctca acaggagaaa taccaaaatc ttcctccgcc gcgaagacaa 9780

accctttcgc acgctgcatg tagtctctta acacagcatg agactgataa ccgaggattt 9840

caacatttgg ccctgctttt gcacgcacct tggacatgtc tggaccatcc ccaatgacaa 9900

ctaatttctg atctgggatt ttggaaaagg cttccacaat caagtcaact cgtttgtaag 9960

gtaccaatct cgaggcagtc aagaaaaagt catctttctt ttcctggaat gcaaaactgt 10020

tggtatctac aggaggataa ataaccctag cctcacgtcc atataccttc tttattcttt 10080

tagcaataaa agaagagttt gcgataaaaa catctacccc gttcgccgtt cttagatccc 10140

aaatacgcaa tttatgtagc aaccatttga caatccaaga tttaaaacct gaaaccaaat 10200

tagactcacg caagtattga tgttgcaaat cccacgcata tctcatggga gaatggacat 10260

aacttacatg cacttgatct ggcccagtaa gcacaccctt ggccacggcg tgagaactag 10320

aaataatcaa atcatattga gacagatcga gctgctcaat agctaaaggc attagtggca 10380

aataattgcg ataatgcgtt ttagctttag gcaagttttg gataaaagtt gtcgtaacct 10440

tcttattctg aataaaacct ctcaggttat caggtaagaa ttcaatgaca gcaaaaatgt 10500

ctgcatcagg atagcaaaga accaactgct ccagaacacg ctctgctcca gcatattcat 10560

taagccaatc cacaacgata gctacttttt ttgggtgcaa tctcgaatgc ctcgtatatt 10620

tattagtcat aatctagtta ataatcaaca tgcctggcct agactttata aagaaaaatt 10680

cagcttggca gataatacta ttactttatt gtgaaaatct ccttgcacat ccgtgcaata 10740

tctattgcgg aatttttcca agaaaaatct tttgctcgct tcaagccttg gttgactaac 10800

gactttctca aatcggcatc atgagcaaca ttatctataa gctttgaaag ctcctgcgga 10860

tcatgcggtg aaaaatataa tgcagcatca ctgcacacct ctggaattga tgcagcattt 10920

gatgcaagta cggggcagcc gcaattcatc gcctccagtg gaggaagacc gaagccctca 10980

taaagtgatg gaaaaacaaa acaaagcgca tgttcataaa gcgcgcgcaa ctcattatcc 11040

gagacataac ccagcaacgt caccttagaa gaatctaact gggaattttc accaaaaatt 11100

ttctgattag ccccacctgc aatcaaatac ggaatatttg cattcttaga aagttttaaa 11160

gattcaatgg ttaaagaaaa atttttgtgc ggagcatgac tactaaccgc caacacataa 11220

gagcccgggt gaacttgaaa tttttcaaga atacctgcgt ctggctgttg cgacaaaata 11280

tgctcgccgc ctgcaccaac aacttgaact ttttcaggat gtacaccaaa aaagttaata 11340

atgtcttttt tcgaatattc tgaaaccgtt acaatttctt ttgcgactct gccgagtact 11400

ggcatgagtg ttttgtacca cacccgaaat gctaaagaaa atgtactagg aattctcacc 11460

actgcagcat cgtgaatgac gacgatttga ctacgcacga agattgagcc tgtattacat 11520

aaatttaata ccattgactt actactcaga ctcaacatta gtagggacaa ttgctcccac 11580

gcatgcccac taaaaaaacc ataagccact ataggtatat gagttagatg caagtcagtt 11640

ttgatgcgag gaggaataac aatcttatat tcaacaccag ctacactggg atgattttcc 11700

gcaatcagtg tatttaactc ttttaatact tctattgcgt atcgatcaac cccagttatg 11760

cgtcttccta aaaatcggcc attaatatat acaatcaaaa tcttttccct accattatct 11820

tggatgcaag gttgatagat agttttgggt aaactatttt tctgaacaat tcgtatttat 11880

tcacaacaaa cttatcaagt actataggta tcaatattgc gataacaatg ccgatcacag 11940

cactatatat ttttgaatca ccaattgtga aaattaaatg attttgaatc gcattgtgaa 12000

ataaaagaat caataacgta gatttaccaa tactgctcaa gatagttgat aggtaggtat 12060

atttatctag aaaaattgaa aacaaaatac tgaaaaatat tcctaagatc atgcagagca 12120

tagccaaagg ataatgcgca atttttctaa aattcaaatc gacgtcaaca ccaaaactaa 12180

acaaccccaa cataacgaaa atcgcacaaa caatagatac aataagatat attgatttga 12240

tttcagtaat tttgtactta tcaacaaata cctttgcgta catacctaga agaataaagc 12300

cagcaccaat tattgctata tcaatactcc aaatcattcc tgaatttttc agactacccc 12360

aagcaagatt atcaattaaa taataatgaa ttgagttaat gagaaaaaca ccaactccga 12420

taaacaataa tgcaaggaaa aaaatcggac ttctatttga ttcacgatca gtgttattaa 12480

aatgatttga aataactaat gcgagaccga ccaaaacaat agagaaaaat aaacatggta 12540

taaaccagag aggagcaact tctattgtat gtccagttcc gtacaaaata ccaagaacta 12600

tattgaaaac accatcatct ggatttttca ttaaaacaaa aggaagaact aaaaatgaaa 12660

atacaaaata taccgcaata acattaatcg cccgcgtgaa atagtacgtt aatccttgat 12720

ctggcttgag ggttatacca ctaataaaaa agaacagtgg catatgaaaa agataaataa 12780

attttaccaa agaaagactt tggcccatta atatctgatt atgcccaacc acaaccaaca 12840

gcattgcaaa tccacggcag acatcgatcc atgaaatcct caatctcgca cctgcatcca 12900

cttgctaatt ccactgctca ataaacttaa cagcagttga tgttatagca gaaaccgccg 12960

atgttgtttt ggaattaagc ccatcagaga gcagtccgct aagctgcaca ccactgatct 13020

gcaccccagt tatctgatgt gaaataacgt ttttcccacc aattctaacc aaggtggtgg 13080

actccacttt catttggtta attaacacat tcttagcttc aacagcccag aaaataggcc 13140

aacttgattt atctgcgaat ccgaaggtat tgacattaat cttggagaaa ttagcagtta 13200

atgctttatt gacgtaaaga ggtgttgttc caatatttaa aaactcccca ttcactaact 13260

cgatgttatc cgccatcatg gttatgccac ttgcttttgt attcttgacg acgatatctt 13320

tcattttgac attaccgcca tgcctgtcga aaacaattcc atgcacgcgt ggggcattga 13380

cagtcaaatt ctcaacaaca attttatcgg tagcaccttg gactaggaaa ccattgccag 13440

ttgcctttgc cacgcaccaa gtacctgcct cttcaacaat gccgtttttg attgtcacat 13500

tggaaggaat tctagtgtca tagtattgct cacttccaat ctttaatcct tctgcggaag 13560

tgtgcctaat ttcaaacttg tctatagtca caccagattg accgataatc gcaattcctc 13620

gtgcataaga accattgata acaatgttgt tcgcataacc acctactgca tcgggatgtt 13680

ttttgtagtt tacaaacgcc aacccatcat cccctgtatt ggtagactga taatccaagg 13740

cgatgcagtt attgcaattt gcaaagtgca acccatcagc ccaggaattc caaaccgtag 13800

ctttaattac tgcacaatca tcacataagg taaaaatgat acctgtacct ggtgtgtcat 13860

aataaaatcc atctctaacg atgactttgg tacatctaga gaactcgtaa ataccaccat 13920

gtccaattct tctagctggg gggtttttgt acgaaaaatc tccaccaatg agtgataaat 13980

cactgcaact cgaaaatact aaaccactta atttatcatt caagaatttc agctgaaaat 14040

ctggtgcaaa ttttacggtg ccactcttca gtgaaaatct taaaggatta cttatttcat 14100

aataaccgct tagcacttcc gtgcgtccag caagcgcgtc atctaaagct ctagtgaagt 14160

ctgcattgcc cccgaccacc ttaggcgctg ccgaggcgac cttgggaagc caggatgaaa 14220

atagcatcaa gaataatgat ttaaccaggt gtctacgatt ttctatcatt ttcagtcctt 14280

cccttaacta taaagaaagc aataatcaca ccaataatct cggtaaaagc tcgggacgtt 14340

cctaaatacg gatatctgaa aatttccaat aacgtaataa aaaataacgg ataaaaaatt 14400

acacttaaaa aattgcctct caaatagcct ttataaaaga ttcctgcaag aaatgcggtt 14460

ataaagaagt agataatgcc aaatggcaag cccaaatcca ggatcactga aaatatccca 14520

gaagggttgt tgaactcctc atcaccatac tgagcgagaa actcacccat tctcatagtg 14580

tgcaaatcta cgtagtaacg aaacacgccg ccaaaaattg gaaatttatg catccaattc 14640

aacacatttt cgaattcata tgttggccag ttaacagtag ccagcattcc cgcaccatta 14700

ttgagcgagg tgtaataata tgaagcaatc cgccctagag caaaatccca aaaactcgat 14760

ttcccggcgt aagttgtaga gctccaagag cggaagtatt ccgccatacc aaagtaaagc 14820

accagcaagg gcaatcccgc gaagggacca gccttaatga agaattgcaa aattttaatc 14880

ttactgaaca cttgttgccg cgttatcaat aacgcaaatg gcaaaataat ttcaattaat 14940

gccagacgct ctgcccaggc aaatacccgc agcaatgtca ataaaacaat tgcatagaac 15000

aacaatacaa catcttgtct taaaggtaat ttggcgatat atctggcata tatgtaaatc 15060

gaaataaata ttggggtgaa gttaacaagt gatgtaatcc cttgcgttgc accaatttcc 15120

tgtcttgata aacctgtccc tgtaaatata ttaataatat tcataggatt gataatcagc 15180

tctttgaacc agtatatata tgctgcaact gctacatagc ctaatacccg caaagccata 15240

ttaacctgag agtctggcgg cactctgact tttgtcttga cgaagcttcc catccaggct 15300

gcacttgctg acagcacaac aattagcaca gccagagcta tatattcaga acttaaatat 15360

atataacccc gtatggttaa tgttggtgat ctcactgacc ccaaccacca gatcaacaag 15420

atcataggca aaagaaagcc taagcatagt ccatgagggg atagccacca tggaacattg 15480

gtttgactat cttgtggttc agtagttttg ttcattgctt tactttgaat aaatatagtt 15540

cagtattgtg atggttttac ttgcccaaga ctcatgctta gcattatctc gagcctgctg 15600

agccaatatt ttgatatccg cattgcaaac atcaccaagt ttatctgtga aaatctgggc 15660

attctcagcc aaaaatacaa agggtaatga tctgcgctca agctctggtg ttgaggtaga 15720

aacaacggga agtccgactg ctgcatattc aaacaatttc ataggactac ggccaaggtt 15780

ggatggatga tcagacatcg gcaataaacc aatttggcac tgactcaaga gattgggcaa 15840

cgctttatat tgcacatgac caagaaagtg tacgttggca agatcactaa atgccgcctt 15900

gtatttttcc tctactggcc cagccaataa aaatgaaatt ttctggtgtt ttgtggctgc 15960

acttttcagc acatcaaaac caaagcgatg atccagagct ccagcataaa ttgcccttgg 16020

ctcaggcggc aaatccagtt tggattttgt aaaattcagg taatgatcta gatctacacc 16080

attctgcagc aatagcacct catcaacacc taggcgattc aaatgctgtc ttacaggctc 16140

tgatgtcgca atgaagccgt gtacctggga tatcaaccct ttttcagcat ctacaatact 16200

gggatcattt ctgatttcat gataaatatc agtggggcgg tatacgatct ttgacttagg 16260

gaaatatttg agtagatagc ctaatcgagg ctcatcaatc aacactacgt caggattaga 16320

aaattgatgt ttacggagtt gtctgagtgc tgaaaacttc aggaagtaac caaagatatc 16380

gctaccagat cccaagcggg tggcaatacc ccaaggtatt aatgagaaag gaataatgtc 16440

ggtcacgtgg ttgatgacat cacccgcttt catccagcgc ttaaaccgga cacgctcaaa 16500

cggtgttttc tgcaacagtg tcagatgtgc tggtgtcact ggcggactga ggtggacaac 16560

ctcatgccct tgcaaggcga attgtttaga tagttgatgt gagccaacta caaattcgcc 16620

ccctgcggca gtgtgactta gaaataatat cttcaaaccc aatccccatt atttgacgag 16680

atggctcaag cgcctgttgt tgcagagacc aaaaaagtca tcccagatta ggtttaacac 16740

ccaaggatat gccaacgcac ttgcgccgca gagctggtgc gctgttatta agatatgttg 16800

ttaattcccc tgctggctgt ttgctagcat acccagcttt tatgctcaaa tcctttactc 16860

gatctccatt atcctaatgg aaacacaagg ctataaaatt atgatggaat caagtattta 16920

cgcttgtccc tgggcactgc ttgcaccaga tcgtgcagga actgttgttg tagctgcaaa 16980

gattgctcgg catcatcttc acccaatatc tgtgaggttc tgaccagtat cccatctacc 17040

agttgatgtt tgaggccttg ctccacaata taaagtcggg tctcccaagc actcttactg 17100

atcatatcgc ctatcctgat ccagtaaagc accagctctt cgcgctgtct tgaagccgca 17160

accaacgcct tggatgggat gggtgttgcg cctgccccca cattcactgc ggtatcctgc 17220

tgatttctga tggagaaacc ctgcgccgta tagcacagtt caggcctatg caccttgact 17280

tcctgccgct gcaatttgcc ataggcaaga gccagcataa tgacctgacc cttttcattg 17340

gcataactcc tcatgagaac ttcatcataa gggttatcga tgctgggttc gccatctctc 17400

ctgactgtca ggtccatctg cacctgattg gttttgactt cctgccactg accaaagctg 17460

ctaggcacga aactttcaag atgtggatca tcccgcacgg agctaagatc aggggtcaaa 17520

gccaacgcca ggccagaact caccaccatg catactgcca ggatattgat taatctgacg 17580

ctcatatcaa ctctgcaatc tagccttacg ctgtgtatgt ttgccaacaa agtattggat 17640

caggctatcc actgaaataa tcagggttaa tgccacgaca aataacaaaa tacccgcgaa 17700

tccatgtaca aagccctggc ccacttcatc cccgaaatag taggtgatca aggtcaacac 17760

aatcacgcgt atgacattcg ctgagaagga aatagggata atcagcaagg ccagcaacaa 17820

gttacgggcc agggaatcat gcctgacgat attgaggtag agcaggccca gggcctccag 17880

tgaaatcaag gtatgcatgc ctgcacaagc atccgccacc agcaactgat actgaccaat 17940

ctgaatgatc acacccgaac gcgcgatggg gtaatcaaac caatacagca catgctcggc 18000

cacgtaagag acagcgacct tcatcggcaa ggtcacaaca tccagcatgg agcctggcaa 18060

tggaatcatg aagaacaaga agaacaaggg gaaccagaag cgcttgaggg ttttggcctt 18120

atagaaaatc aacagcaagc cagccaatac cggaatctgc gagcccacat caaacaccag 18180

caaatcttgt gaacgcccga tgatgtatac caacaggcca aacaaaaaca gcaacaggcc 18240

cacaccacgc ccgctttgtc tcaagtttgc cgtgtctagc agtatttcct tgtccaccca 18300

gactaggtac agcaccacca tcaggatcaa ggggccatgc gcctgatcct gcatctgcca 18360

gaccgtgttc gtcaggttca ggtaggtggg cacaaataag aacagcaagc ctaccagcag 18420

tggtatcaca tgctggtacc ttcttagctg ggtactggcg accgcaatgc tctggttcaa 18480

gctcatttta gttttcagtc aataccgtgc caacaacagg caccctgatc ttggaaagct 18540

gatcaaccac tttgctcaag tcattggctt tggagacatg acgcttggca agaagcacca 18600

ccccttccgt agcacttgcc acagacaacg gttcagagtg gcggacaaag gaaggtgcat 18660

cgaccaggat aacatcgtag tcattagcaa ttgccgtgat gaactgcttg aggaaaccac 18720

gtcccagaag ctctcgcgga ttaggtggaa tggtgccaga agacaagatg gagagatgct 18780

cgaactcgac tagcctagtg atggaagagg agtaatccgt aacccggcct gccagaatat 18840

ccgacaaacc cgcagtgctc ttgatattaa aaatgcgatt ctgccgtggg ctatcaagat 18900

gggcatcaat gatgagtgtt ttctctccca gttgagagaa aaccacgcca agattggcaa 18960

ccaagtcact ggtgtcgtcc ttgtcgtaaa cactggaaaa tgacaggctc tttttccctt 19020

gtgcaaacca gtagagcatg agctggctac gaatggcgcg atacttctcc acattgatac 19080

tgaatggatc ataagccgcg accaagttct cggagaaatc gccgcgacca tccatcaggt 19140

agggataggc aaactgcttg gataatacat agttgatatc ggcagaggaa actaggccca 19200

aactctcggc agcctcacca aagcgaacgc ctctttcctt ctgcacacgc aggatacgct 19260

ccgcatcctg cggtgtaatc ttacccatct tgagcaacat tccaccgata ttgaaatcag 19320

cattcaatgc cggaatggaa ctagtattgg aaacatcatt agtcatgtct atatccacac 19380

aaattagggt tttttagcca gcaggcgctg aattctggaa tgggttttat ttttgtcgat 19440

cacacccagc agcggtgcgc caacaatctc taccagcact ctttctgagc gaattcgctc 19500

attagtgagc tcagccaaca aactaaacaa caagccaaaa attgtaccca gcaccaacgc 19560

aactgccacg ttcaatattt ttttcgggct tgatggctta agtggtggtg tcgctggatt 19620

aaggatggat acatcagctt gattagactg tccctcaaga tttatttgag tatatcgctg 19680

agtcgcgagt tcgtaagcgc gttgcgcacc ctcgacttca cgcgtcaata ccgtcagtgc 19740

atccctatcc ttgttcaaca gcatgacctt ggttttttgc tcagccaagg cattgcggat 19800

ttcactctcg cgttgcttaa ggattctata attgctcgat atattgacgg tagccgcctg 19860

gatttgctta cttaattcag acttcagatt gtcaagctca gctttggccc cctgatagag 19920

tgggtgattt ctctcatatt tttgtgagat atccgcgaag cgtgattcag acctagcaat 19980

ctctgttttc aggtgcagaa ccattggatt actcgcgaca tccggagatc cataagcatt 20040

gctaccttgt gcaccagtct gacgagactg tgcctcaaga gtttgccctt gtgacatcac 20100

taactgcgag gaaagttcat tcagcttggc acgttcaatg tctagacgct catcaacgct 20160

gacgatgctt ttatcttgtt ggtaatcgga aagcttttgc tgcgcccggg taatatcggc 20220

acgcaaatcc ttcaattgcc cggtataaaa ttcagccgcc tttttcgaag gctcaacctt 20280

gagttcgata gtggccttga tatattcctc ggcaaaggag ttagcaacaa cagcagcaaa 20340

tttggggttg gctccatcaa actccagctg caacacacta ctgttgcgcg caggttgaac 20400

ttcgatacgt gagatcagca aatccgccaa ccaatcaacg atatcacctt caccacctgt 20460

ggagtcattg aaactctcca caacctcact actttcattg agctttaatg tggtcacaac 20520

cctcttagca attttcttgc tagttatgat atccacctgg gtacccatat aacctggaag 20580

cagctgggca gggacagcca taccagtcac aggatctaca cctttatagt tgactaggat 20640

actggtggtt gccttatagc tgtttggcaa gaaaatattg agcgcaattg ctacgaccag 20700

ggccacaaca atggtgctta acaatatctt atatcgcgct ttgaatatca gtaaaagttg 20760

agaaaagctc attagattaa ccttgcctat caagtattta ttaaaatctg caatctatta 20820

aaagaaactt tcctgcacat agatcacatc gttgggcttg atgaggtcat catgtttggc 20880

agtgattact tcaatatggc cgctagcatc acgacgcttg atcttgacgc ctttttctgt 20940

gccgcgaggg cttaagccac cacctacaga aagcgcttgc aatactgtca tttcatgttc 21000

gagacggtaa tggctggcac gttgaacctg accatagata tagaatttat cgaaacgttc 21060

aacataaatg atgtcaccac cagctacgcg gatgttcttg ctgagatcgc cagtacgcat 21120

catgtcagca aggtcaatct gatccctgac cgctttacca tcgacgttat gcaccagcgt 21180

gatcagatcg ccaccttcag gtgatacacc accagccaag gcaagcacat ccgccagcgt 21240

tttagcgcta tctatagggt atctgcccgg gcgattgact tgacccagaa tagagacttg 21300

aaggctctgc aattgcgcaa cgatgatgtt gacgtgagca ctcttaagga aaccaccctt 21360

ttccagcatg gtggcaattc tttgctcggc ggcggcaacc gtaccaccac ccacacgcac 21420

ttcacctatc aggggataag taatggaacc tgattcactc actctgacct caagtgacag 21480

atcaggctgc tcgaacaccg atattttcag cacatcgcca gcgccgagaa gaatgtcacc 21540

ttctgcagca tgcgttacaa aaggcgtcag actcagcatc agcaagaaaa accatgtcat 21600

taattttttc attttactat cgtcctgata ttaaggatgg ggagtactta tttcaagcca 21660

ctaacaccac tggcgatatc atcctcagta gagcttggct tctttttggc tgccttggtt 21720

tcagtcgctg tttccatctt tttatcgatg tactcaatct tggcatcggc tttcaagcgc 21780

ttgacttcat tttcagcaac ttccttgcgc gccttattca gcaaattgtt ttcaatttgc 21840

ggtgttgctt ctgcaagtga aactgggctg tccttgatct cgttcatcat gatcaacatg 21900

gtgcgagggc ccatgccgat cacgaacaac ttacccttat cctcggcctt gaggcgctcg 21960

gaaatctgtg gcggcaattc agcaacactg cgtgaaacct gaccttgatc gtactgaata 22020

ttatgggcat ccatccatgc ggctacttcc tgcaaggtct tgagtttgcc aagtgctgtt 22080

ttcaattcat cattggcgaa tttagtatca atcaacaatt gatccatttc caccattctg 22140

cgcttgctga agaattgagg attctcgtta taaaaattct caatttctgt tttgcttggc 22200

ttggcgacac tgcctaactt ggattgcaga tatgcctgag caatgatttg tgatttcgca 22260

cgttcaatcg tggtcatcac atttggatcc ctatccacct tggccttgag cgcagcttct 22320

tcaagtaact ggcgatctat caatgcagtc agaatctgct gcgttgcctg ctcgcgctga 22380

ttctcctgaa tgttggcacg acccagctcc tgattcagct gatgtacggt gatttccttg 22440

ccattgacct taaccagaga ctggctggct ttggattcat gcttgtcgcc acagcccgac 22500

aatgcgaaag cgagcacaat cacagcaccg acacacaggg ttctggattg cttgaagtta 22560

gttacaaatc cgttattcac tttccaatct cctcaaatct atttaaacaa cctagtaggc 22620

gttatctttt ttgaacacaa ccagcaccgt cctgacgata atccagaagt caaaagccag 22680

ggaccaatgg cgcaaataat ccaagtcata ctcgatacgg gactccatct tgtccaaggt 22740

atcagtttca ccacgtaaac cattcacctg cgcccagcct gttattccag gcttgacctt 22800

gtgccgcagc atatagccac gaatcagctt gcgatattgc tcattatgag aaacggcatg 22860

cggtctaggc cccacgatac tcatctgacc ttgcaacaca ttgatgaact gcggcagctc 22920

atccagtgag ttcttgcgca agaaaccacc aatcggtgtc aatcgctgat ctcgcttctg 22980

cgcctgtact accttgtcac catcttccat tactttcatg gagcggaatt tatagacaat 23040

gatttcctca ccattcagca taacgtcgct gcttgaaaat caccgggcct ggggaggtca 23100

acttcacggc cagagccagt gtcagcatga tgggagacag cattaataga ataagcgacg 23160

ccaaaataat gtcactagtc cgtttgatta aactgttgaa accggtgaat ggcgtctcaa 23220

gaatagcgac cacaggcaca ccagccacat catcaaagcg cgcttgtacc agatcaaaaa 23280

tatagatatc cggcacaaag taaatagatg ctgtggtgtc ttgcaattct tcaaataact 23340

gcctgatgcg tggctgcgcc gacatgggca ggctgatgaa aataatctgg gtattattgg 23400

cgcggacata tggcgcaatt tccgacatgg aaccaagcaa aggcagatta cgcatgccct 23460

tggggtgcct ggcgacctct ctgtcgtcaa agatcccctt gacctccata aacaggtacg 23520

ggtcgtccgt gatgcgattg atcaagcgca cgccaatatc attggcaccg ataatcacta 23580

cggacctgac ttcaccattc ttgtgcaaat ccgaggcaat tttgcgtatc gccagatgcg 23640

tgacaataaa tatcatcggc acactggcaa accaggcata tatcatctca tccagatact 23700

gactggatag atcagcggca tagccgatga acatcagcac agcgatgaca ataccccagc 23760

caaagagaat atcgcggata taaccaagta tcttgccact acgccaggta cggtaaagat 23820

ccagacgctc gtagacatta gaggaaacaa agtaggctag caacgccaga atcaggtagt 23880

agccattaaa aggctcacca aacaacaggg tgaacacata cagcgtacca agaatgatca 23940

gcgggtcgag cagctgcttg aaaaacgcta ctaaagggat gtcatttacg gtcatttatc 24000

ttacaccatc agaattcata acgaccagta agcgaaatgc ttgtcgagtt gtagtttaaa 24060

attggtgagg ttgagtcacg caaacttcta tttagaccag tagtgaaact taacgattca 24120

agagggctgt acgttacccc aaaagaaaaa accttattct tgtcggtacg accttgatct 24180

tgcccggtga tatcgccttc aaattttagt tgttgatacc tggctgaagt tgacagcagg 24240

atcttactcg ttgcgctata ggagatgctt gcacttgcgc cactatcaat cacatagctg 24300

gcagttacga ttgatatagc atttgcatcg cgccatgcgg ctgtcttgaa agaggttttg 24360

tcggtaatgg accacaccat gttcccgtgg gcattaaatc cggaaaaatc gcgctgtgag 24420

aagctgtcat gtttacgatc aacatacccc cccaaaaatt caaatcgtgt tttggcagaa 24480

taaagccaaa gtatgtccgc cttgagtgat tgctgcctgt agtcattatt gacaaacacg 24540

gtaccaatcc ccgggaaaaa agcaggccta tcaaagggcc gcgtgccacg cccattagtg 24600

aagaccaagc caaccttact gcctgactca gctaaatagt ccaaacctac gtgctgggaa 24660

ttttcctgaa aagaagccgc attcaatgtt tgagagtcat actcaacatc ctggcgctgc 24720

acaccaaagc gcgcgcgcca actggaatgg aaacgccagt ttgcttcaaa agttttacgc 24780

tcctgaatac gcatattcaa ttccacacca cgaaaatccg caaaaggcag catagcctcg 24840

tttttatagt actcaacctt gccccggaat tgcgagccca ctgcccagtt ccatcgagcg 24900

acaatatctt tacctgtatt gtctaactct gaatttttgt tgtatttggt ttcattcaca 24960

ccgatattgg cgtagaaatt ctgctgacta atatccttat ccagaataat gccaacccgg 25020

cccactacac tcgtatcgga ctccttgtca gagcggcgcc tcaggttgtc atcataaaca 25080

tatgttgcac tgacataggg ccgtatgaca tcaccttcac gagccagcac agggtcggaa 25140

taacccactg cattccaacc tatgactgcc gtgataacca gctttttata atgagtagag 25200

tctgttagtc gcaaagaaat acttccccaa tggctaccag taatgccagt taccggtata 25260

gataatccaa actcccaata caccgttggt caccgcatgc gcgatgatcg gcatccatat 25320

tgttctgctc cggatgtaaa gcaatccgta tgccagcccc gcaaccagac cagcaaacca 25380

taggttatgc tctatggcaa aaagtagtga agaaaccagt aatgccacag caccaaccgt 25440

ggccggctcc accttgagga agtcctgatt ttgtatccag cgcatcagga aagaacgcca 25500

gaacaactct tccatcagag gcactactag ggcagcaccg acaattcttg tcaccattag 25560

gagacacgct tcgacaccct gccccggatt gaagccttgc gatggcccgc ccagtaacat 25620

ccacggggca tccaggttaa tccaagcgac aaatactagc acaccaatgc tgattgcgct 25680

caaccacgac ttcaaactaa gcaacgacaa gccaccaagc tcgatatagt gtttgcgcaa 25740

tgtccaaagc aagtaggcaa ccaagccaac cttgactcca tataaccatc gcagatcaaa 25800

accctcaggc acaactgcgc cgatcaggtc tacccctgcc atgaatagca tatagaccgc 25860

gaagggaata acacggctac ttatcggccc cagtgatgcc ctgttcatgc atgtgcctta 25920

gtttgaaata cgctcatgat acaaaatcaa atcgccaaat gacaggcatt ctcatttcaa 25980

tagtccctat ggactaaatc agactcactc actgcatgaa tggccttgcg gatgctactg 26040

tgcaactgct atagatttat gacaagtctt gtcacacctt cgctcgccat tcccagtatg 26100

tgctacaact tcaggaatag gcaccaacac aatacccaaa atgaataagt cggcatgaag 26160

ccgacttatt catttactct tagcatgact aaatgcacgc ctgataagcg cgtgcattta 26220

cgtgctcagc ttaattaagc ttggcgacga cgtgcagcaa agcccagcag tcccagacca 26280

accagcatca at 26292

<210> 179
<211> 26076
<212> DNA
<213> Methylobacillus glycogenes

<400> 179
atgttaaaaa aagtagctct aattacaggt atcactggac aagacggagc atatctagct 60

gaatttttac ttggcaaagg ctatgtagta catggcatta agcgccgagc atcacttttc 120

aatacagatc gcattgatca tctgtatcag gaccctcacg tcgataacca gaatttcatc 180

cttcattatg gggatttgac agattccacc aaccttatac gtatcattca gcaggttcag 240

cccgacgaaa tctataacct cgcggcaatg agccacgtgg cagtcagttt tgaaagtcct 300

gaatatacgg ctaacgcgga tggtcttggg actttgcgta ttctggaagc tatccgcatc 360

ttggggctgg aaaagaaaac ccgcttttac caagcctcga cctcagagct ttatggtttg 420

gtgcaggaaa caccgcagaa agaaagcacc ccattttatc cacgcagccc gtatgcggtt 480

gcgaagcttt atgcatattg gattactgtc aattaccgcg aagcttatgg catctacgcc 540

tgtaatggta ttttattcaa tcacgagagc cccttgcgtg gggagacctt tgttacccgt 600

aaaattaccc gtgccttggc gcgcatcaag ctgggcttgc aagattgcct ttatttggga 660

aatctggacg cgttgcgtga ttggggtcac gccaaggatt atgttgaaat gcaatggctg 720

atgttgcaac aggaacaggc agaggatttc gtcattgcga caggtgtgca atacagtgtg 780

cgcgatttcg tcaatgcagc cgcccacgag ctggatattg ccatcacctg gaaaggtcag 840

ggccaagaag agatgggata cgacggtcaa ggcaggtgca ttgtacaggt tgacccgcgc 900

tattttaggc ctaccgaagt tgaaacattg cttggtgacc cgagcaaggc aaagcaaaaa 960

cttggctggt cgcccagcat cagtttccag gagttggttt ctgaaatggt gcgtgaagat 1020

ctcaagagcg cagagcgtga cgaactggtt aagcagcatg gttttgctgc ctatgactat 1080

aacgagtaac ctgagcctat ggacaaacac gcacgtatct atgtggcagg gcatcgaggc 1140

ctagtgggtt ccgctatcgt tagggggcta gagggcaagg gctatagcaa tcttatcaag 1200

cgtacccatg cagagcttga cctcacagat gctaatgcca cggaagcatt ctttgctcag 1260

tatcagccga atatgttttc ctggcggcag ccaaagtggg tggcatagtg gccaataaca 1320

cttacccggc agattttatc cgtgacaatc tcgcaattca gaacagtgtg atccatagtg 1380

cctggaagca tggagtaaag cgcctattgt ttttagggtc cagctgcata taccccaagc 1440

atgctcctca gcccatgcat gagcaccact ttctaaccgg atcgcttgaa gctaccaatc 1500

gtgcatacgc gctggcaaag attgctggag tcgaaatgtg ctggagttat aaccggcagt 1560

atggcactcg atttcttgca gcaatgccga caaatcttta tgggcctggc gataactatc 1620

acttgcagaa tagtcacgtt atccctgcat tgatacgcaa attccacgag gctaaacaag 1680

ctgggcatgc agctgtatcg gtctggggta ctggaacacc caggcgcgaa tttctttaca 1740

gtgaggatat ggcagatgcc tgtatattct tgctgaatct gcctgaggag aaattcaatt 1800

ctctgctggg aagtgatgag agtgctactg gtgtgtttga tccaccacta gtcaatatcg 1860

gtattggagt cgatatttca atacaggaat tggccgaatt ggtaaaaaag gtggttggtt 1920

accaaggagc aattgagttc gatagttcaa aacctgatgg cacgatgcgt aagcttatgg 1980

atgtaagcaa gctcaacagt ttggggtgga cttattccat tgagcttgat gatggattgg 2040

aaaagacata ttcggaattt ttaacaaaat aaatctgagt ttattaattt atttctgata 2100

tcgaattgat attatcctca aaaatagcgt ttagataatt tatataacct cgataatttt 2160

taaaatttaa tgttcaattt tgaaaaaaaa agtgtttgct ttgattcctg ccagagcagg 2220

ttctaaagga gtgccaaaaa aaaacctgaa aaatatcgca ggttctccat taatagctta 2280

cacaattaac gctgctaaaa aagctaaaaa tgtcgatgct gtttatgtat cttctgattg 2340

taaagaaata ttggagtatt caaaagcaca aggctgcagt tcaatattaa ggccgcaaga 2400

atacgcaaca gatgatgcgt cagctgttga tgttgtcaag cattttatta gtattgaaaa 2460

tatagaggat ggagatttaa tcgtttactt acagccaacc tccccattaa gagatgaaca 2520

tcatattgat gatgctttga cggctttgaa taataatggc cttacttctt tggtcagtgt 2580

ggaagtcttg gataagtcgc catataagtc atttcgtcta gatgataatg ataaattgat 2640

ttcattattt gatgaaaaat tatctaatta tcgtcgtcaa gatttgccat tatgttacgc 2700

gccaaatggt gctatttata tcttttcgaa atttgaattt gaggctagag gcggtttccc 2760

ctccaatggt agtgttgctt atattatgtc tgctgaagaa agtgttgata ttgacaccga 2820

agaagatttt ttagaaacag aagcattatt aaggaaaaaa tatggcatct tttgaaattt 2880

ctgggcggaa aattggggac ggacatgagc ctatagttat tgcagaaatt ggcatcaatc 2940

acgagggttc attagatctt gcgattgata tggcaaaagc tgcaattgat gctggggcag 3000

agataataaa acatcagaca catgtggttg aagatgaaat gtctgatgaa gccaagagtg 3060

ttattcctgg aaatgcggat gtttcaatct atgagattat gcaacgttgc tcattgtcag 3120

aggatgatga atataagctc atgcaatatg tgcagcagca aggcagtatt tttattagta 3180

cgccattctc gagaatggca gcaaatcgac tgagaaaatt tgatataccg gcttataaaa 3240

ttggatctgg cgagtgcaat aattatcctc tcgtgcggca tattgcttca tttgggaaac 3300

ctattattgt tagtaccggt atgaatagca ttgaaagcat tgcaccaacc gtcaatatat 3360

taaggcaagc aaaagttcct tttgctcttc tgcactgcac gaatatttac cctacccctc 3420

ctgagcttgt aagacttgat gctatccaag tcattaaaaa tgcatatcct gatgcggttg 3480

tgggactttc agatcacacc cttgggaatt acgcttgcct aggggctgtt gcactaggag 3540

cctcaattct tgagcggcat tttactgact ccatgtcacg gccgggacca gacattgttt 3600

gttcaatgga ccctgcagca ttgcaagatt taatcactgg ttcaaaagtt attttttcag 3660

caaaaggtgc acataaaggt ccacttttag aagaagaaaa aactatagct tttgcttttg 3720

catctgtcgt tgcaattaag gatatcaatg ttggtgatgt cttaagtgaa gataatattt 3780

ggttacgtag gccaggtggc ggtgatttta atgcttctga ctatgatcag ctacttggtc 3840

gtaaagcatc gcaaactata ctgagtggca aaagagttaa tcggcaggat attgaggcat 3900

gagtaggcga gttgtttttc ttactggaac aagagcagat tttggaaaac tgaaatccct 3960

catgcgagca ttacaaaatg acatgggatt tgacgtccat gtgttcatta ctggcatgca 4020

tatgttgtcg aaatatggtt atacatgtga agaagtagaa aaagccggat ttaaaaacct 4080

atttcgcttt atcaatcaaa atcaaagcga tagcatggat catattctgg ccaaaacaat 4140

tactggttta tcagattatg taaaagaaat ttcaccagaa ctgattgtga ttcatgggga 4200

tagagttgaa gcgttggccg gagccgctgt tggcgcatta aataacattc gagtggcaca 4260

tattgagggc ggagaggtct ctggcactgt tgacgagttg attagacatg cggtgaccaa 4320

gctgtcccat gggcattttg tttccaacga acaggcaaaa aatcgactta ttcagcttgg 4380

agaaagtgaa gaatccattt ttgttattgg ctccccagat attgatgtta tgaattcaac 4440

atcgctgcca agcattgatg atgtgaggga acgttatggc ttctcgttcg aacattacgg 4500

cattttgatg tatcaccccg ttacctctga gttagatgat ttgccaagac aagccaaagc 4560

catcgtagat tatatttcag aaagctctct accattcatt gttgtatatc ccaataatga 4620

tcatggttca gacattattt tgaatgagta tttgcgattg aaaaacttga gccacgttag 4680

agtttatcca tccattagat ttgaatattt tttaacgctg ctaaaaaaat gcagcgttca 4740

tcatgggtaa ctctagcgct ggagtaagag aggctcctca tttcggcgtg ccagccatca 4800

atgttggtag caggcaacac aatagagcaa cagcagagtc gattcttaat actactgcac 4860

atgtggattc ccttagacat gctgttcaac aagttgcaga cattcctcgc gtggcagttt 4920

ctgactttgg taatggtaat agtgcaggtg agtttcatca gattctgttg aataatgctt 4980

tttgggataa gggcatacaa aagcacttta tcgataggag tgcagtgtga ggttgatcgc 5040

aaggctagat gtcaaaaatg aacatgtgat taaaggcata cacctagagg gcttgcggaa 5100

agttggcgat ccaatcgaga tggcgaaaaa ctattatgca caaggcattg atgagattgt 5160

gtttatagat gcggtagcga gcttgtataa ccgtaataac ctgctgtctg taatcgccaa 5220

ggcttcggaa gaagtttttg ttcctatcac tgtagggggg gggctgcgtt ccttagcaga 5280

tgtcgagatg gctctgagtg ccggagcaga caaagtggca gtgaatacag ctgcaatact 5340

taacccgaaa ttcatcacag agattgccaa gcgctatggc agtcaatgtg tggttgcctc 5400

tattcaagcc aaacggatag gcaatgactg ggaagcttat atggaagccg ggagggagcc 5460

tacgggcaaa aatgttgttg cttggagcaa ggagctggaa gatcttggtg caggcgagat 5520

tttgctaacg tctatagacc aagatggtac aaaaaaagga tttgaccttc cattgcttgg 5580

tgcagttaac acgatagtga acattccagt catggttagt ggtggctgcg gtaatcctga 5640

tcatgtgcaa gagttaaaag aaatccagcc aagtggagtt tgtctggcgt cagtattgca 5700

ttacaaaatc aatacgatag atgaattcaa acagcgtctg acattcatca atggtactaa 5760

ataaatgaag aaaatatgta ttcttgatta tgggctaggc aatgtgcgta gtgtgagtaa 5820

tgcgatcaag tacgtaggtg ctcatcctgt aatctcaaat acgccagagg cacttaaaga 5880

ttctcacgcg ttaatagtgc caggggttgg tgcgtttgct caaggcatgt ctgcattggg 5940

taaagatgga ttacaagcta gcttgctcga atatataaaa agtggaaaac atgtcttggg 6000

gatttgtttg ggcatgcaat tgcttttcga caggggtgag gagtttggcg ttaccactgg 6060

tcttggactt gtgcctggcg tagtctccag aattgtaccc gtagagcaat cacgagtgag 6120

gttaccgcat atatcctggg cgaaagttga tctcaattcc gttcatgaaa ataatggcgg 6180

catgtttgat ggactgccag acgagcagcg taagttctat tttctgcaca gttatgccgc 6240

tcaaaatgta cctgatcgat acatcacagg cagcgcgacg tattctggat gtacttttgt 6300

tgcatcagtg cagaaagaca atatctgggg tatgcaattt cacccagaaa aaagtggccc 6360

cagcgggtta caggttctta ataactttgt acaaaacagt taattgaaaa gaagtgtgga 6420

ggaagttgtg gttaatgcat attttggttt gccggaagat gtagtgtttt gcaagaaatg 6480

cgtgatttcc aaccaaagac ccagttctac agttgaattc aagcatacca acaaggacaa 6540

aaaagcgacc ataggatttg gcgctgatgg tatttgtgat gcttgccgct atgcagaaac 6600

caaggatgct caaatagact ggcaggcccg cgaggacgct ttggttagat tgttggatca 6660

acatcgccgt agcgatggtg gatatgacat tgtcgtgccc ggcagtggtg gtaaagacag 6720

tgcatatacc tcacatatcc ttaaatataa atatggtatg aatcctttaa cagtgacgtg 6780

ggcgccgcat aagtacactg agattggttg gaagaatttt gaaaactgga tccataaagg 6840

tggattagac aatattcttt ttaccccaaa tggcaagttg caccgctatt taacaaaaat 6900

ggcatttctg aatttattgc acccattcca accattcatt gttggccaac gcattatcgg 6960

gccgttgatg gctaaaaagt ttggtatcaa attggtaatg tatggtgaaa atcaagctga 7020

atatggcaat aagacagaag aaaactataa accgacaatg gacaagaatt ttttctctat 7080

ttcagaccca gctgacatgg tgttaggtgg tgtgaaggtt ggagacatta ttaaagacgg 7140

tgaatacagc ttaaatgatt tcgcgccata tattccgcct gctgcagaag atctagagcg 7200

cgatggtatc gaggtacatt atttgggcta ttacctgaaa tgggatccac aagagtgcta 7260

ttactatgca gtcgagcata ctggtttcca ggcaaatacg gaacgcactg aaggaaccta 7320

ctccaagtac agtagtatcg atgatcgaat agatatgttc cactacttta ctaccttgat 7380

caaattcggc attggtaggg caacatatga tgctgcgcaa gaaattcgca acgataaaat 7440

tacgcgagaa gaaggggttg ctctagttca taaatatgat caagagtttc caaaaaagta 7500

ctttgcagaa ttccttgaat atatagagat caccgaagca cagttttggg aaaccgtaga 7560

taggtttaga tctccacatt tgtgggaaaa gcaagacagt aattggatac tgaagcatca 7620

ggtgacctaa gaatgaaagt gttgcagata ggaagtttct ccggaaatgt cggtgacatt 7680

gccaaccatt atggttttcg caattggttt caaggactgc ttggagagat tcaatgggat 7740

gaattcgaaa tacgcaatgt ttaccgaaaa aatgttgcct ttgacgataa ttttgtagct 7800

attgcaaatc agtacgatct tatagtcgtg ggaggtggta attttttcga gctatgggtt 7860

gattcttctc caaccggcac ctcaatctca attgagattg aggtttttaa aaaaataaaa 7920

gtccccatct ttttcaatgc attgggtgtt gatgatggta tgggatgtag tgatgaaagt 7980

cttgcaaagt ttactaattt cttagaaatc ttaacttcat caaagcaata tttggtcagt 8040

gttcgtaatg atggagcaat gggcacgctc agaaatcgga tttctaataa tcccatgttg 8100

gataaagtct taccattgcc tgacggtggg ttttttgtaa atctggataa gttagaaaaa 8160

gacattgcgg aaaatgatca tgaaaacact cagatcacca tcggcatcaa tctggctgga 8220

gatatgttgg aaacaaggtt tccaggtgga attaatggat atgattactc aggatttata 8280

tccgagttca ccgagacttt ggctgaaatg tggaataaaa ataaattaat aaagtttatt 8340

ttttttccac atatttatag tgatcttaaa gtatatgccg acatactatc acggttgcct 8400

gatgcatgta gacgtgaaaa tgtcaaagtg gccagttacg atactaatgt gaattttcaa 8460

gggacattat tttccgaata tagcaaatgt actttgatac ttggtatgcg atttcatgcc 8520

aatgttgtag ccatagctaa taaaattcca gtaataggat tgtactcata caagcaaatt 8580

aaagaattgt tttatgagtt gggaataaaa gaaaaaattg ttgatgtcag acaaataggt 8640

tttaaaaatg aattgttgag aaagtgcaat attgttattg aaaattcatc tatatctcgt 8700

gatgagagct gtaacatact tgatgttgtc acaaaaaaac gtgatttgat tgctccaagg 8760

gtagtggagt ggttacgtga gcataagctt aaatgattaa ttgttgtaat tgtatgctta 8820

gttattatga ttaaaagcct tgattatcta agggcgattg cagcactaac ggtttttctc 8880

agccacacct ttcaaattta ttggttacca accattggat tggtgtcctg gaaatatgag 8940

ttgaatcatt ttttgagtga aaccgcggtc ctcttatttt ttgtattaag tggctatgta 9000

atttcgaata gtttgcatag caatactcaa cgtaatggag ggcgtctttc agtacttgat 9060

tacgcatcta gcagattatt gcgcatatat cctcctctca ttgtaagcat aataatatct 9120

ttagttgcct atagtgtgat tgtgatcttt gaactaccgg gatcagtggg tactttaaag 9180

gcaccaggag atatctatca ggcaagagac tacttgagca ttggtacgcg agacattgtc 9240

aattctttat tgatgaataa tggcttgttg caaatgaatg ggccattatg gagtttatgc 9300

attgaggcta agctatattt ttatgcttgt ttagttgtaa taatttttat tcattataaa 9360

caaaaaaatt tgctcaattt tattgctaat actggttttc tcgtattatc tgtattcttg 9420

ttttttaata caaaaaccga tatttattat ggcttatggt ggttgagcgg tggtgtaatt 9480

ttcttatatc gtcgaaattt cataaagttc atgactttgg ttgttgtaat ggctattgtg 9540

gtgtttgcta tggcagtgat tggacagaag ttcgtttctt tgccagtgca agttatattt 9600

gtatattctt tatttttggc agcaataaaa tgcaaagtgc cagaaattaa ggcaatgaat 9660

attttggcca atacttcgta cactttatat attgtccatt tccctatgtt gttactatcg 9720

tacgccatct tctcatataa ctttgatgtg tacggtagcc tgactagaga gttgatgtca 9780

tcattatcgc taataatcat cttaactatg tcaatttata ttggaaaatg ggtagagcgt 9840

ccagctaatt tacgtttgac ctatcacagg ttgttgagaa aagtaaaagt atgaaagcca 9900

tgttttattc tttgcatgca gctatttggt tacatgcgct tcctgaaagc cggttggcat 9960

cacagctgag taatgccggc catgagttag tatatgtgac ctgtgataaa agctttactc 10020

attattgcac tggtatgagt gctttcaatc ttgcattgaa tgaggatttt aaaaagaaaa 10080

gtgcaatttg tgagttatgt attaataacg ccaataaaat aaccaagtct tttgattgca 10140

agcatcttaa attgcatgat tatattgatc agaaaactaa tgctctgatt gataacctat 10200

tgaaaagtgt cacggaagat aatttcctcg aatttcatta tgaagatata ccagtagggc 10260

gtttggctac atatgagcca cttcttaagt ataagaaaat gtctttggaa tttagagatg 10320

aacaatggca agatctacag attcacctta ggaatgccct ggtatcgtta atcggtttta 10380

tccaaatatt caaacaagag tgtccagaaa aaattttctt ttatagtcca cagtatggtg 10440

ttaatggtgt gtgtgcagaa tatgcagtaa aacaggggag acaggcatat tttatagaag 10500

gtagctctag caatgctgaa cgatatagtg ctttaagggt gtgggagtgg agtaaattta 10560

aattagtaaa ccctgcattg cagcattggc aagctgttaa aaatttagta acgcttgaag 10620

atattgatag agtctctgca catattcacg aattattcaa aggtaaatct tttgctgttt 10680

tttcagcgcc aaaaacagga aatttttcaa tccgtgaaaa attcaatatt ccacagaatg 10740

ctaaaattct actcgcctca ttaagtagtt ttgatgaagc gtatgcggct tttgttatca 10800

aaggttttcc tgagcgtaaa gttaaaagtc cggttttctc agatcagttt gaatggatta 10860

agtgtacaat taattttatg aagtctaggc ctgatgacta tcttgtaatt agagtacatc 10920

cgcgagacta tcctaataaa cgcgattcca atcagtctga gcaagcattg atttgggaaa 10980

aaatacttca ggatctccct gagaatgtta aattaaactc accagatcag aatatttctt 11040

tatacgattt tctcgatgat gtacatgcgc tggtaacagg gtggtctgcc actggtgtag 11100

aagcattgct acatggcata cctgtgatta cctatgataa atacttacct tcctatccgg 11160

cggatataca tctaacggga gagtctgaac atgagtatta tgacaacctt atcaaagtac 11220

tttctttggc aacatcaaca gataatacac atgtagttaa tgcctatcgc tggctcgcgg 11280

tcagtttttc actgggcact gtcagagtta atcagccact ggagattggt ctgagttggc 11340

cggataagcc ggcgtataga atattgaaaa gggtggctaa caagttttca gttaatttgc 11400

gaaaaagcta cgacgtatgg aatgtccagt ccaacgacac agaccgttta agatttttaa 11460

gtctggttct taataatcaa tctagccttt ttgagggtgt aatatctgat gtggtcgatg 11520

ctgtttccga ttatgaaatt gaagctacgg taaaacaaca ttcgatataa gttaattatc 11580

tgatatttaa tacaatttat atttagtaac acttaccatt aaaatgaaag ttgttgaata 11640

aattgccaca agcaaaatat atttttaagt tattaaaatt tgctttcaat aacaacccat 11700

tgttgtattt gagtgtttta atttctatac tttctatagg tattgaattg ttggcaatgg 11760

caagcctatt gcctttattt gaacttgttt caggagaaac agtagataag catggtttga 11820

tttcaaaagc tctgattata ttgggggtta cagttgatgc tgccagcctt ttgcaagttt 11880

tcttcttatt gttcactttg agagtagtca cgcaactatt tagtcaaagc ctgacgcact 11940

accttggaag acgtgtgatg gctcagctcg gttctagggc ttttgaaaaa atcattactt 12000

cgatgtcaat tgataaaata tctgaaaaaa gtataggttt ttttatgggg cttgctggag 12060

atgaatcctt cagggcgagt actctggttg ttgcattgac tcagtttgtt gcaacatttt 12120

tactgggggt aatgtatttt atttctattg caatttattc actatatgcc gccgaaatga 12180

ttttcatttt aatgcttgtg tctgccgtat tcttcatttg gattttaaaa atatctcata 12240

agatgggcgt gaggcaaata gaaggttcaa ggaaagcaaa ttccattttc atggatgcga 12300
tgaacaatat caaagcagta agatcacttt cttccgagaa gtatgttgcc ggtttgtata 12360
ggcgaaagat ttttacgtat gcaaatattc ttttttacct tgatgcaata aatctggcta 12420
cgaagttagt accaataata tttctattac tcggatcttt ggctttgata ttgagtggct 12480
gggtttctgt tgaaaactta gggatggctt acattgtgtc attgatagct tatctcatgc 12540
gatttttccc agttgttggt cagagtatca ctctagctgt tcgcattgtt gcagatgcga 12600
aatcgggaaa agatgtgacg gaattagttt ccgatgaaag tgccgagagc gctaattcag 12660
acaactattt acacaagata gatacaatca agattgataa tttgtcgttt tcataccgtg 12720
atgttcaagg cgtaaggcca gcggttctca attctgttta ctacgagttt gaaaagaaca 12780
actcttatgc tttagtgggg aaatctggtt taggaaagtc tactttgctg gatgttttgc 12840
tgaagtttta tcctcctgct tcaggcagta tttcaataga tggtatttca acagaaaata 12900
tccctggagc acatatcagg cattctattg tacttattaa tcaagaacct gcgatttttg 12960
atgatacggt ctacaacaat atttgtatgg gcgagacttt tactctagag gacgtaaaat 13020
gggcttgcga attagcttgt atttctaaag atgtggaaag tcttgcgcaa ggttatgaaa 13080
cacgtttgca atatcaagga aaaaatcttt caggtggcca gcgtcagcgt atagctattg 13140
ccagagcatt agtgcgcaaa cctaatgtgc tgatacttga tgaaagtaca acagctttag 13200
ataaggctac acaagaaatt gtgatacgga atcttatgaa tgagtatgaa aataagatca 13260
ttatatttgt cagtcatgat ccttatatca tgtctcaagt cgatcataca gttgatttgg 13320
cattaatcaa taaagtataa atttatgtct atctcaagta ttgctttaaa aatatttaat 13380
tattcgcaag agattttttc attttaccgg gatagaggct tatataaatt ctcttatgcg 13440
caagaggggg aagatttaat tctgagtaga atttttgacc gaacctctac tgggtattat 13500
attgatatag gtgctcatca cccagtaagg ttctccaata cttatttatt ttataaacgt 13560
ggttggaggg gagtttgtat agatccctca cacgaggcta ttgctttgtt taaaacacgg 13620
cgcccagcag atattgcctt ggaaatgggg gtatctgatc atgagtctga gctgacttat 13680
catatgttcg atgagcctgc tctcaatacc ttcagtgagg aattggccaa tcagcgtata 13740
caaaacactg cttataaacg cgtcggtacc cgcaaggtgc cagtaagacc cttgactaaa 13800
attttggatg aagtgctgct gccggatact gaaattgatt tcatgagtat agatgttgaa 13860
ggctttgatt accaagtgct tgcctctaac gactggtcaa agtacaggcc tcgtatagtt 13920
ctggcagaag cacttgatga tgtcgctggc gaacgcatac gcagtttcat gattcaaaat 13980
cattacaaag tcatagcgcg tacattcaac actgagtttt atcaagatct tttgaaactg 14040
taagtaacca tgtccaatct agcacctgtt gccctgttta cttataaccg ccctgaccat 14100
actaaaagga cgattgaagc actgaagaat aataccttgt ctgctgaaac ggacttgctg 14160

attttttctg atgctgcaaa aaatattaca cagcaaagca tggtggatgc tgtcagagcg 14220

tttactcaca ctgttgaagg cttcaagtca gtaagaatca tagaacgtca aacgaatttt 14280

gggctagctg cttcgattat tgatggtgta acaaatgtac ttgcagagta tggtcgcata 14340

atagttcttg aagacgacat ggtcaccagt ccatattttc ttcaatacat gaatgatagc 14400

ttgagtcagt atgctgattt tcatgaagtt gcaagtattc atgcctatgt ttatcccatc 14460

gcaggattgc ccgatacttt ctttttgcgt ggtgcagatt gctggggctg ggctacatgg 14520

agggatagat gggattggtt tgaaccagat ggaagtaagt tgtttgcgca actgaagcag 14580

cttaacttgc tgaataagtt tgattataac ggtagttatc cattttcaaa tatgttgcgc 14640

gaccagattg caggaaccaa taattcttgg gcaatacgtt ggcatgctag tgctttcttg 14700

cttaataagt tgacgcttta tcctggtaaa gctttgctta gtaatattgg acatgatgga 14760

tccggtgtgc attgtggaga aagcgaaaca tactctgcgg agttgagagc tgaccgatta 14820

ctattgactg atattccatt gcgcgagtct gagccggcat ttaagaaatt tgagcaattt 14880

tttcttgaaa gcaaaccatc agttgttcgc aaaatgatgc tgaaggcaaa atccgtattt 14940

aaaaaatttt tatgagtttt atcaagatcc taaaattatt atgcccccca ctttttctgc 15000

atattctgca gaagtatagg cggcgtaact ttgcctcttc gttcgccgga aattattctt 15060

cttgggcgca tgctaaacaa aatagtggtg gctatgatgc ggaaaatatt ctcaccatca 15120

ttcaagagtc agctctcaaa gtcaagcgtg gtgaagcggt ttttgaacga gattcagtat 15180

gtttccatca tgttgagtat agatattccg ctttgtctgg attgctatac gcagctgtta 15240

gttctggtgg caagttgagt gtcctggatt ttggaggctc tctggcaagt ttttattctc 15300

agcatgctgc aatattgtct atagttaagg atttgcgttg gggagttgtt gagcagccac 15360

attttgttgc ctggggtcgc gagcacctaa ccacaactga tcttaaattt tacaatagcc 15420

tcacagagtt tattttggat ggaaaaccca atgttatttt tctttccagc gtcctgcaat 15480

atttggagca tccataccag ctgttagaag aattattgag gctagattct gagtttattc 15540

ttttcgatcg cacggctttt tatgacgttg ttgaccgctt aactgtccag acagtgcctg 15600

catcgattta tccggcaagt taccctgcct ggttcatttc ttgggacaaa tttataaata 15660

ctatgagtgc aggagggtat gaggtcatca tggagtttcc ttgtgcagac ctgacggaca 15720

taggctatta caaagggctt tttttcaagc gcaagagtgt aagttgattt tctaacaagc 15780

aattgttcat ctttgagtta ctgtgtctta tttcttctaa tgtacgggtt gattctcttg 15840

ttgtgaaatt gttaaggtaa ttgtatgaat gatgaactgt actcaggtaa gaatattctt 15900

gttactggtg gcttgggttt tattggttca aacttggtca aaagattaat tgcgcacaaa 15960

gccaaggtga ccgttgtaga tagcctgtta cctctttatg gtggcaatgc ctttaacctt 16020

agtggctatg aatcgctggt caatatcaac atcagcgatg tacgtgatag tcatgccatg 16080

gcttatctta tcaaaggcaa ggattacctt tttaaccttg ctggacaaac tagccatttg 16140

gattccatga ccgaccccag gacggatctg gatatcaatg ccgcagcaca attatccatt 16200

ctggaagcat gccgacagca taatcccgat gtcagaatag tcttcgctag caccagacag 16260

ctttatggca agcctgagta tttacctgta gatgagtcac acccgattca gccagtggat 16320

gtgaatggca tcaataaatt ggcaggggag tggtatcaca ttctctataa caatgtttac 16380

ggtatcaaag cctgtgcgct acgtctgaca aatacatatg gtcctggcat gcgcgtcaag 16440

gatgcccgtc agacttttct tgggatttgg ttgcgtagag tgattgaagg caagccaatt 16500

gcggtatttg gagatggctt gcaattgagg gatttcaatt acgttgatga tgtggtggat 16560

gctctgttac tggctgccgc caatacagaa gcagatgggc aaatatttaa tctcggcagt 16620

gatgaggttg taaatttaca gcaagtagct gaattaatga cacaatttca gcctggttcc 16680

agctatgaga ttgtgccatt cccacctgag agaaaagcca ttgatatcgg tgactactat 16740

agtgatttca ataaaatcaa gcaggtttta ggttggagcc cccgtgtggg cttgcaacaa 16800

gggcttgctg caagcctggc atactatcgc cagcatcatg ataaatattg ggacaacgta 16860

tgattttaat gaatgacttc aaggctgaac cagaagcttt gattgcagcg cagctgcaag 16920

ccgtcgagcg tgtgctgcgc tccggttggt atgtattagg tgaggagggg cgcaagtttg 16980

agtcattgtg gtctcagcgt tgcggcactt ctcatacagt gggtgttggc aatggcatgg 17040

atgcgattga gataggattg cgcgcgctgg acattgggcc cggtgatgag gtgattacca 17100

ctgcgatgac ggcttttgcc tctgtgctgg cgatcatacg tgcaggtgct acgccggtct 17160

tggccgatat tgatcctgac actggcttat tgagtcatga aagtgtcaag cgatgcatca 17220

actcgcgaac gaaagcggtc ttgctagtcc atctatacgg gcaaatcagg gatatgcaat 17280

cctggctgaa cttgtgcgat cagcatcaag tggctttgtt ggaagattgt gctcaagcac 17340

atttggcgca agcggaaggt aaagtggcgg ggagttttgg tcgctttggt gcttacagct 17400

tttatccgac caaaaacctg ggcgccattg gcgatggagg ggctttggta acatctcaat 17460

ctgcgctggc agacaaggct gcagtgttaa gaaattatgg ccaaagtgtg cgttaccatc 17520

atccagagtt tggcttgaat agtcgtcttg acgaaattca ggcggccttg ctcacggtaa 17580

ggctagagtg gctggaacaa tatacaagtc ataggcaatc aattgccgct caatacaggc 17640

agcagttgaa taatccatac gtgcgcctca tgcaaactcc aacagagcca ttagcccatg 17700

tctatcattt gtttgttgtg ctaacggagc accgtgaagc attgcaggct cacttgcagg 17760

caaatggcgt tcaggcattg attcattatc ctgtgcctat tcatcatcag gaaaagtgca 17820

gcgatatatt gagagatccg gctgggctgc aatttagtga ggcccatgca cagcaatgcc 17880

tttcgctgcc atgtcatcct caaatgtctg aacaagatgt tcagaccgtc attgctgcag 17940

tcaataggtt tgcactgtaa acatggagca ttttgtcaca ctgtttgata gcaagtttct 18000

gcctcaaggt ttgtgcctgc atgagtcgct ggcccgccat gccagggatt ttcatttatg 18060

gatagtctgt atggataaag tggtgcatga gcaactggaa aaattgcagt tgcctcatgt 18120

ttcgcttatg cgcttggccg attttgaaag cgaggcatta ttggccgtta aaccaggcag 18180

aacaaagggg gagtattgct ggaccataac cccattcact gccgaatggg tgtttgctca 18240

acacccagag gtggcacgag tcacttatct tgatgcagat ttattttttt tcaacgatcc 18300

tgcgatactt tttcatgagt ttgaacaaag cggcaagcat gtgatgatta ctgagcatgc 18360

atatgcaccc gattacattc agcatctgga aacgagtgga cggttttgtg tgcaattcat 18420

ggtatttgat cgaagtgaag catcccgtaa agttatgcat tggtggcaag atcgctgcgt 18480

ggagtggtgt tttgcaagga tggaggatgg aaagtttggt gatcagaaat atttagattt 18540

atggcctaca atttttgccg atgatgtcca catattgaag tgcgttgaac aaacgttggc 18600

accttggaat gtagactatc aacttcagca ccaaagtggg aaattcaagc cggtttttta 18660

ccactttcat gcattccgca taatcaagtt taatcttgtc agactttatg aagggtacca 18720

gatagctaaa agttctcgtt acctctatga cgagtatctt gcgactttgc agaaaaaaat 18780

tgggatgttg catcggtttg ccattccgtt gccattgttg ccagaaaaat gtggtattag 18840

gccaaaattg gcttcttatt acacatggat cagaggattt accgctacag ctaagatcaa 18900

ataaaatgca aaaacttagt gtgattacgg caacatataa tgccgctaaa aatttaccag 18960

atttagtgca aagcctgctt agtcaaactg atatggattt cgagtgggtc gttgcagatg 19020

gcggttcaag tgacgaaact atatcttatc taaaaagcgt tgtaggcatt aatcttttgc 19080

tgtcagcgca acaggatttt ggtatatacg atgcattaaa tcgtgcgatt tcgctaagct 19140

caggcgatta ttatttggtc ttgggtgccg atgatatttt gtatccacat gctgtcgagg 19200

cgtttaagcg tgcggctggc ataggtctgc ctgatctcgt caccgcaagt gtagagtcag 19260

atcatggtgt catgattgct ggaagtggca ggccattttt gcgcggacaa ttggcttata 19320

tttccaatca ttctgtcggt actatgatca aaaaaagttt gcatgatcgt tttggttatt 19380

actcccgccg attccctatc acggcagatc agtattttat caagcgtgtt tgtggcgcta 19440

gcgaaacggt gatttacaaa gcagattttg tagcaggcta ttatccagct acaggcgtgt 19500

caggagcaga tgtccctggg acgatggttg aatttttccg cgtgcaattg gaaaccgaac 19560

ctaattcagt gctccagcta gtacttttca ttttgcgttt gatacgccat acccccaagc 19620

tcatccggta tgcacactac aaaaaattgc gcgatctacc ccagcatgaa tagcaacgta 19680

ttgtttatca ctggctttca tcctggaggt atgggtttta ttggtgcagg tgaggcaatc 19740

agtcagcaac atatagagcg actgataaag caaggaagga aagtgcatgt gctggcactt 19800

gcccctaatt atcaagtcgc cagtcctgag gttgttgcaa gctgtgcttc ctacaccacg 19860

cttacgcata cgcgatggat gacgtttgca gctttatggc gctcacgcca gcagggaagt 19920

ctgctctgcc cctggttatt tacgcgtgta tcaccgcaag ctgaacgttt tatccgtcag 19980

ttgttggacc aacacgccat tactgagatc tatcttgatt ttccatcttg tcttggcttc 20040

gcgccaacat acaatcaatt acccattcat tacattgttc atgatgtgtt gtcgcaaaaa 20100

gttgagcgac gtcctttatt aaaatggctg aaaaatcaag tgttgcgcac ggaatcgtcc 20160

ctgctgcgtc atgtcaccca gtgtttcaca ctttccctga aggacaagct gagatgcgag 20220

gaaatagggt atagcggcca gatagaggtg caggtgccgc acattctcaa ggtaggggtt 20280

gttaatcaag gagtgccaat tgcagggatc ctcaagcaat ttcctggcaa gaaaaacctg 20340

gttttttttg gcaacatgca gcgggctgaa aatcattact ccatcttaag atttttgttg 20400

tgcagttatc ctggcatctg gttggagcat cgcgatgttc agtgctggat attaggtttg 20460

gcaccaagaa cgctattgcg ttggatatcg aaagtgttgc ctggtgttag agtcactggc 20520

gctgttgatg acccaactga ggcgtttaag gcggcaactt tatgtattgc acctgtatat 20580

tacggtgctg gtgtgaaaat caaagtttta caaatgctgg aatcgggagc cacagttata 20640

tcgacgccag tcggagctga gggtattcac ggcagtgaaa aactcattac ggtggaaagt 20700

gctcgtctat cctccactat caagcaacgt ttggcagttg aatcatgaat cagcatgaac 20760

tccccagcgt tgcagtgttg ttggccagtt tcaacggtag ggcttggata gcggagcagc 20820

ttgccagtat tcttgctcaa cagggtgtca agcttcatgt gtttattgcg gacgacttat 20880

ccactgatgg cacttgggag tggttgaatg tagaggctgc ttgtcatcaa aacgtgactt 20940

tgctgccaca taatcagcgt gcaggttctg cgggcaggaa tttttatcgt ttgatccacg 21000

aagttgatct ggatggtttc gatttcgttg catttgcaga tcaagatgat gtgtgggata 21060

gctataagtt gagcaggcat gcacgcttga tgaagctaca tcaggctgat ggtgtgtctt 21120

ccaatgttga ggcattttgg cctgacggca gagtgatgct gatcgttaaa tctcaagctc 21180

agcggcagtt tgattatctc ttcgaatctg ctgggccagg ttgtactttc ttgatgacta 21240

ctcgcctcgc acatcaagtg cgcaatgtgc tccaagatga gctatctcca gcgcgggatg 21300

ttactttgca tgattggcta acttatgcaa ttgcacgttc cttgggatat acctgggtga 21360

tagatgctaa cccttctctg aaatatcggc agcatggcaa taatgtgatt ggggccaatg 21420

tcagtcttaa agcgaaatgg acgagattga aacaaatccg cgatggctgg tatcgccagc 21480

aagtcattaa ggtgacacgc atcgcttatg ctttttctca gcgcgaagat ttgcagcgct 21540

tattggtttt gctggagaag ccttcacttg gaaaccgcct gcagttattg ccatatgcct 21600

ggcatggaag gcgatcagtg aaggatcgtt tattcctgat gtttagtatt gtgtttaagt 21660

ggttttgaac gatgagtcag caacatattt tggttaccgg agctaatggc tttgtgggtc 21720

gatcactatg caatcaatta tgccaatcag cctttcaagt cacccaggtt ggacgtgctc 21780

taaatagcgc tgatggggga gcatatagcc aggttgtgtc gatagatgga acaacggatt 21840

ggtcgaccat cttggatgag gttgaggtag tgatacatct tgctgccaga gttcatgtga 21900

tgcaggaaaa cttagttgat ccaatcgtgg cttactggca aaccaatgtc gagggcacct 21960

tgaatttggc caggcaagcg ctaaatgctg gggttaaacg ctttattttt gtaagttcca 22020

tcaaggttaa tggcgagcaa agtttcccag gcaaaccatt tactgcagaa gatatgccta 22080

tgccagaaga tgcctatggc aaatccaagt gggaggcgga gcagggcttg cttgcattgg 22140

caaaggattc aaatatggtt gtggtcatta ttcgcccacc tctggtttat ggtcccggtg 22200

taaaaggtaa tttttccacc atgatcaaac tgctgcagac aggcttgcca ttgcctttcg 22260

gggcgattgc caatcaacgg tctttggttg ctatcgataa tttgatccac ttaattatgg 22320

tgtgtattaa tcatccggcc gccgccaatc aggttttctt agtcagtgat ggtgaggatt 22380

tgtctactac cgctttattg cgtcgcttgg ctctagcggc tggcgtatca tctagattga 22440

ttccctttcc tgcagcatgg gtcaacttcg tctttactgt tctgggtaag aaaatgatgg 22500

cagggagaat tttagggtct ttgcaggtgg atatcagcaa gacacgcgag gtattaggct 22560

ggcagcctcc tgtgacagtt gatcaaggcc tgcgaaattg ttttccaact aaggctgagc 22620

cgtgattcgt ttttttgaca ttgtattttc cttgcttggc ctggtattcg gtttgccatt 22680

attgataata atttatctgg tcggcttgct cgatacaggc tctcccttat tccgccaagt 22740

gagggtaggg cggcatcgcc agccatttac gctggtgaaa ttccgcacca tgaaagtcaa 22800

cacagcatca gttgccagtc atctggcttc tgccagctct attacttggt taggtagttt 22860

tttacgcagt acaaagttag atgaattgcc gcagctttgg aatgtgctca agggcgagat 22920

gagcttggtg gggccgcggc cgaatttgtt taatcaggat gagttgattg cagcgcgtga 22980

tgcacggggt gtttatgcag ttcgaccagg catcaccgga ctggctcagg tgaatgacat 23040

tgatatgtct atgccaaagt tgcttgcaga gacagatgct tctatgttgc agcaattaaa 23100

tttaaagctt tatttcaagc tgttaattca gactgtattg ggcaaggggc gtggtgacag 23160

ggtcgtgcgg tgatctaccg ccatgccagt taggcgagtg ctattttcgt tctgccaacg 23220

cagcactata gtttttcagt aaaatacatt tttttcattt taggccgtgc gcgtgcatac 23280

ccccccccat ttatttcgtt ggcctaattt catcagtttg aaaaacccgc gctcaatcag 23340

tgcctgggtg catgacatca ttgccgccat gcttgcgtgg tgggtagcat ttcttttccg 23400

cttcaatttt gatatccctg ccccatttgc cgagagcatg ttgcaaagtg cagcttgggt 23460

tattcccctg caagcaacat ttttcctgct atgcggcctt taccgcggtg tatggcgttt 23520

tgccagtctg ccggatttac agcggctgat tcgagcgatt ggggtggccg cagtagttgt 23580

ggtagcaatt ttgtttatgt tccagccaca tggggttgtg ccgcgctcgg tgttgatcat 23640

gaatcccatg ctgctgcttt tgttgatggg tggcagccgc attgcctacc gcgcctggaa 23700

agagcatcga ctgtatggcg cggcccaatc ctcaggtaag ccagtgttgg tgcttggcgc 23760

cggggatgcc gcgattgcct tgctcaagga tttatccaag agtcatgagt ggcgcatagt 23820

gggcttgctt gatgataagc aatcaatgtt cggccgtgag ttgcttggtg taaaagtgct 23880

aggcaaactc agcgattttc ctgagatcgc caaggcaatg tctgttcagc atgtgattgt 23940

ggccattcct tcggcgcgca acaacgcagg cgccagatta ttgatatggc cagtcagcac 24000

aaactcaagg tacttaccgt gccatcactg gaagatttga tgagtgggcg tgtcagcatg 24060

gcgcagctac gtccggttga ggttgaagat ttgcttgggc gtgagcctgt ggtgttggac 24120

gatgatggtt tgcaccagtt gatcgatagt caggtggtta tggtcactgg cgcgggcggc 24180

tcgattggtt cagaactttg ccgacagtta ctcaagtttc agccttcaat gctggtgtgt 24240

ttcgaccatt cggagttttc tctttatagc ctagagcaag aactgaccgc tttaaagctg 24300

gatgtgcgga ttgaatatct ggtaggggac gtcaagaatg cacgacgcgt gcagcacgtg 24360

cttgctagtt atcagccagc acagtatttc atgctgcagc ctataaacat gtgcctatga 24420

tggaaaaccg taacgttgca gagtcattat ccaataatgt gctgggtact ttcacagtag 24480

ccagtgcatg taaggcggcg ggcgttaata agtttgtgct ggtatccaca gataaagcgg 24540

ttaacccaac caatgtcatg gggctagcaa aacgtctggc tgaaatggtg tgtcaaggtt 24600

tgcaggcgga aacaggaacc cgctttgtgg tagtgcgttt cggcaatgtg ctgggcagca 24660

gtggtagtgt gatccctaag tttaaagagc aaattgcgct tggtgggcca gtcaccgtta 24720

ctcatcccga gatcacgcgt tatttcatgt ctatacctga ggcgtgccag ttggtcatgc 24780

aagccggatt gatgggtaag ggcggagaaa tattcgtgct agatatgggt gaccccatca 24840

agattgtgga cttggcgcgt gctatgatta ggttgtcagg gttgggggat gatgaggtcg 24900

acattgtttt ttccggcctg cgccctggcg agaagctgta cgaggaactt ttggcagacg 24960

acgaactcac tttaccgacg ccgcacgata aattgcgtat cgccgtggct agaagtgcag 25020

atgaggcctg ggttgtggct ttgctcaagt gggtagacaa gtcatccagc atggatgagg 25080

cagtcgtgaa gcaagagctc aagatgtggg tggaggagta tgcaggtgat atacatgcta 25140

gtctttcttc tgcgttgact cccgggtcat cgacaataca ttaaaagaaa gacgattgag 25200

atgacaaaaa aaattaccaa ggcagtcttc cccgtggcag gtcttggcac gcgtttcttg 25260

ccggccacca aagccagccc caaagagatg ctgcctatcg ttgataaacc tctgattcag 25320

tatgccgcgg aagaagcagt tgctgctggt gcaactgaac tgatctttat tattggtcgc 25380

aataagcgct ccattcctga tcattttgac aagtcttttg aacttgaagc ttccctagaa 25440

gccagcaaga aaaccaagat gcttgaggaa attcagaaca ttctacccaa gcacgtttcc 25500

tgcatttata tccgtcaagc tgaagctttg ggcttgggcc acgcggtgtt gctggctaaa 25560

tctgtcgtgg gtaatgaacc gttctctgtc attttggcag atgacttgat tgatgctgaa 25620

aagcctgcga tgcaacaaat gactgagttg tttgaaaagc aaggagcttc aattcttggt 25680

gttgaagatg ttgagccaag cgagacagca agctacggta ttgttgatgc agtggaattg 25740

acaggcccag atgtattgca gctgaacggc attgttgaaa agccacagcc tgaagatgca 25800

ccttctaacc tggcggtggt tggacgttat attttgactc ctgaaatctt cacctgcctt 25860

gagaatgtca agccaggtaa gggtggggaa attcaattga ccgatggtat tgctgcgctg 25920

atggagcaac aaaagattct ggcttatcgc ttcaagggta agcgttacga ctgcggcagc 25980

aagttgggct atgtcaaagc caatgtcgag ttttcgctta agcatgaaga aattggtgag 26040

gagttctcgc agtatctgcg tgaattgacc aagtaa 26076

<210> 180
<211> 26076
<212> DNA
<213> Methylobacillus glycogenes

<400> 180
ttacttggtc aattcacgca gatactgcga gaactcctca ccaatttctt catgcttaag 60

cgaaaactcg acattggctt tgacatagcc caacttgctg ccgcagtcgt aacgcttacc 120

cttgaagcga taagccagaa tcttttgttg ctccatcagc gcagcaatac catcggtcaa 180

ttgaatttcc ccacccttac ctggcttgac attctcaagg caggtgaaga tttcaggagt 240

caaaatataa cgtccaacca ccgccaggtt agaaggtgca tcttcaggct gtggcttttc 300

aacaatgccg ttcagctgca atacatctgg gcctgtcaat tccactgcat caacaatacc 360

gtagcttgct gtctcgcttg gctcaacatc ttcaacacca agaattgaag ctccttgctt 420

ttcaaacaac tcagtcattt gttgcatcgc aggcttttca gcatcaatca agtcatctgc 480

caaaatgaca gagaacggtt cattacccac gacagattta gccagcaaca ccgcgtggcc 540

caagcccaaa gcttcagctt gacggatata aatgcaggaa acgtgcttgg gtagaatgtt 600

ctgaatttcc tcaagcatct tggttttctt gctggcttct agggaagctt caagttcaaa 660

agacttgtca aaatgatcag gaatggagcg cttattgcga ccaataataa agatcagttc 720

agttgcacca gcagcaactg cttcttccgc ggcatactga atcagaggtt tatcaacgat 780

aggcagcatc tctttggggc tggctttggt ggccggcaag aaacgcgtgc caagacctgc 840

cacggggaag actgccttgg taattttttt tgtcatctca atcgtctttc ttttaatgta 900

ttgtcgatga cccgggagtc aacgcagaag aaagactagc atgtatatca cctgcatact 960

cctccaccca catcttgagc tcttgcttca cgactgcctc atccatgctg gatgacttgt 1020

ctacccactt gagcaaagcc acaacccagg cctcatctgc acttctagcc acggcgatac 1080

gcaatttatc gtgcggcgtc ggtaaagtga gttcgtcgtc tgccaaaagt tcctcgtaca 1140

gcttctcgcc agggcgcagg ccggaaaaaa caatgtcgac ctcatcatcc cccaaccctg 1200

acaacctaat catagcacgc gccaagtcca caatcttgat ggggtcaccc atatctagca 1260

cgaatatttc tccgccctta cccatcaatc cggcttgcat gaccaactgg cacgcctcag 1320

gtatagacat gaaataacgc gtgatctcgg gatgagtaac ggtgactggc ccaccaagcg 1380

caatttgctc tttaaactta gggatcacac taccactgct gcccagcaca ttgccgaaac 1440

gcactaccac aaagcgggtt cctgtttccg cctgcaaacc ttgacacacc atttcagcca 1500

gacgtttgct agcccccatg acattggttg ggttaaccgc tttatctgtg gataccagca 1560

caaacttatt aacgcccgcc gccttacatg cactggctac tgtgaaagta cccagcacat 1620

tattggataa tgactctgca acgttacggt tttccatcat aggcacatgt ttataggctg 1680

cagcatgaaa tactgtgctg gctgataact agcaagcacg tgctgcacgc gtcgtgcatt 1740

cttgacgtcc cctaccagat attcaatccg cacatccagc tttaaagcgg tcagttcttg 1800

ctctaggcta taaagagaaa actccgaatg gtcgaaacac accagcattg aaggctgaaa 1860

cttgagtaac tgtcggcaaa gttctgaacc aatcgagccg cccgcgccag tgaccataac 1920

cacctgacta tcgatcaact ggtgcaaacc atcatcgtcc aacaccacag gctcacgccc 1980

aagcaaatct tcaacctcaa ccggacgtag ctgcgccatg ctgacacgcc cactcatcaa 2040

atcttccagt gatggcacgg taagtacctt gagtttgtgc tgactggcca tatcaataat 2100

ctggcgcctg cgttgttgcg cgccgaagga atggccacaa tcacatgctg aacagacatt 2160

gccttggcga tctcaggaaa atcgctgagt ttgcctagca cttttacacc aagcaactca 2220

cggccgaaca ttgattgctt atcatcaagc aagcccacta tgcgccactc atgactcttg 2280

gataaatcct tgagcaaggc aatcgcggca tccccggcgc caagcaccaa cactggctta 2340

cctgaggatt gggccgcgcc atacagtcga tgctctttcc aggcgcggta ggcaatgcgg 2400

ctgccaccca tcaacaaaag cagcagcatg ggattcatga tcaacaccga gcgcggcaca 2460

accccatgtg gctggaacat aaacaaaatt gctaccacaa ctactgcggc caccccaatc 2520

gctcgaatca gccgctgtaa atccggcaga ctggcaaaac gccatacacc gcggtaaagg 2580

ccgcatagca ggaaaaatgt tgcttgcagg ggaataaccc aagctgcact ttgcaacatg 2640

ctctcggcaa atggggcagg gatatcaaaa ttgaagcgga aaagaaatgc tacccaccac 2700

gcaagcatgg cggcaatgat gtcatgcacc caggcactga ttgagcgcgg gtttttcaaa 2760

ctgatgaaat taggccaacg aaataaatgg gggggggtat gcacgcgcac ggcctaaaat 2820

gaaaaaaatg tattttactg aaaaactata gtgctgcgtt ggcagaacga aaatagcact 2880

cgcctaactg gcatggcggt agatcaccgc acgaccctgt caccacgccc cttgcccaat 2940

acagtctgaa ttaacagctt gaaataaagc tttaaattta attgctgcaa catagaagca 3000

tctgtctctg caagcaactt tggcatagac atatcaatgt cattcacctg agccagtccg 3060

gtgatgcctg gtcgaactgc ataaacaccc cgtgcatcac gcgctgcaat caactcatcc 3120

tgattaaaca aattcggccg cggccccacc aagctcatct cgcccttgag cacattccaa 3180

agctgcggca attcatctaa ctttgtactg cgtaaaaaac tacctaacca agtaatagag 3240

ctggcagaag ccagatgact ggcaactgat gctgtgttga ctttcatggt gcggaatttc 3300

accagcgtaa atggctggcg atgccgccct accctcactt ggcggaataa gggagagcct 3360

gtatcgagca agccgaccag ataaattatt atcaataatg gcaaaccgaa taccaggcca 3420

agcaaggaaa atacaatgtc aaaaaaacga atcacggctc agccttagtt ggaaaacaat 3480

ttcgcagccc ttgatcaact gtcacaggag gctgccagcc taatacctcg cgtgtcttgc 3540

tgatatccac ctgcaaagac cctaaaattc tccctgccat cattttctta cccagaacag 3600

taaagacgaa gttgacccat gctgcaggaa agggaatcaa tctagatgat acgccagccg 3660

ctagagccaa gcgacgcaat aaagcggtag tagacaaatc ctcaccatca ctgactaaga 3720

aaacctgatt ggcggcggcc ggatgattaa tacacaccat aattaagtgg atcaaattat 3780

cgatagcaac caaagaccgt tgattggcaa tcgccccgaa aggcaatggc aagcctgtct 3840

gcagcagttt gatcatggtg gaaaaattac cttttacacc gggaccataa accagaggtg 3900

ggcgaataat gaccacaacc atatttgaat cctttgccaa tgcaagcaag ccctgctccg 3960

cctcccactt ggatttgcca taggcatctt ctggcatagg catatcttct gcagtaaatg 4020

gtttgcctgg gaaactttgc tcgccattaa ccttgatgga acttacaaaa ataaagcgtt 4080

taaccccagc atttagcgct tgcctggcca aattcaaggt gccctcgaca ttggtttgcc 4140

agtaagccac gattggatca actaagtttt cctgcatcac atgaactctg gcagcaagat 4200

gtatcactac ctcaacctca tccaagatgg tcgaccaatc cgttgttcca tctatcgaca 4260

caacctggct atatgctccc ccatcagcgc tatttagagc acgtccaacc tgggtgactt 4320

gaaaggctga ttggcataat tgattgcata gtgatcgacc cacaaagcca ttagctccgg 4380

taaccaaaat atgttgctga ctcatcgttc aaaaccactt aaacacaata ctaaacatca 4440

ggaataaacg atccttcact gatcgccttc catgccaggc atatggcaat aactgcaggc 4500

ggtttccaag tgaaggcttc tccagcaaaa ccaataagcg ctgcaaatct tcgcgctgag 4560

aaaaagcata agcgatgcgt gtcaccttaa tgacttgctg gcgataccag ccatcgcgga 4620

tttgtttcaa tctcgtccat ttcgctttaa gactgacatt ggccccaatc acattattgc 4680

catgctgccg atatttcaga gaagggttag catctatcac ccaggtatat cccaaggaac 4740

gtgcaattgc ataagttagc caatcatgca aagtaacatc ccgcgctgga gatagctcat 4800

cttggagcac attgcgcact tgatgtgcga ggcgagtagt catcaagaaa gtacaacctg 4860

gcccagcaga ttcgaagaga taatcaaact gccgctgagc ttgagattta acgatcagca 4920

tcactctgcc gtcaggccaa aatgcctcaa cattggaaga cacaccatca gcctgatgta 4980

gcttcatcaa gcgtgcatgc ctgctcaact tatagctatc ccacacatca tcttgatctg 5040

caaatgcaac gaaatcgaaa ccatccagat caacttcgtg gatcaaacga taaaaattcc 5100

tgcccgcaga acctgcacgc tgattatgtg gcagcaaagt cacgttttga tgacaagcag 5160

cctctacatt caaccactcc caagtgccat cagtggataa gtcgtccgca ataaacacat 5220

gaagcttgac accctgttga gcaagaatac tggcaagctg ctccgctatc caagccctac 5280

cgttgaaact ggccaacaac actgcaacgc tggggagttc atgctgattc atgattcaac 5340

tgccaaacgt tgcttgatag tggaggatag acgagcactt tccaccgtaa tgagtttttc 5400

actgccgtga ataccctcag ctccgactgg cgtcgatata actgtggctc ccgattccag 5460

catttgtaaa actttgattt tcacaccagc accgtaatat acaggtgcaa tacataaagt 5520

tgccgcctta aacgcctcag ttgggtcatc aacagcgcca gtgactctaa caccaggcaa 5580

cactttcgat atccaacgca atagcgttct tggtgccaaa cctaatatcc agcactgaac 5640

atcgcgatgc tccaaccaga tgccaggata actgcacaac aaaaatctta agatggagta 5700

atgattttca gcccgctgca tgttgccaaa aaaaaccagg tttttcttgc caggaaattg 5760

cttgaggatc cctgcaattg gcactccttg attaacaacc cctaccttga gaatgtgcgg 5820

cacctgcacc tctatctggc cgctataccc tatttcctcg catctcagct tgtccttcag 5880

ggaaagtgtg aaacactggg tgacatgacg cagcagggac gattccgtgc gcaacacttg 5940

atttttcagc cattttaata aaggacgtcg ctcaactttt tgcgacaaca catcatgaac 6000

aatgtaatga atgggtaatt gattgtatgt tggcgcgaag ccaagacaag atggaaaatc 6060

aagatagatc tcagtaatgg cgtgttggtc caacaactga cggataaaac gttcagcttg 6120

cggtgataca cgcgtaaata accaggggca gagcagactt ccctgctggc gtgagcgcca 6180

taaagctgca aacgtcatcc atcgcgtatg cgtaagcgtg gtgtaggaag cacagcttgc 6240

aacaacctca ggactggcga cttgataatt aggggcaagt gccagcacat gcactttcct 6300

tccttgcttt atcagtcgct ctatatgttg ctgactgatt gcctcacctg caccaataaa 6360

acccatacct ccaggatgaa agccagtgat aaacaatacg ttgctattca tgctggggta 6420

gatcgcgcaa ttttttgtag tgtgcatacc ggatgagctt gggggtatgg cgtatcaaac 6480

gcaaaatgaa aagtactagc tggagcactg aattaggttc ggtttccaat tgcacgcgga 6540

aaaattcaac catcgtccca gggacatctg ctcctgacac gcctgtagct ggataatagc 6600

ctgctacaaa atctgctttg taaatcaccg tttcgctagc gccacaaaca cgcttgataa 6660

aatactgatc tgccgtgata gggaatcggc gggagtaata accaaaacga tcatgcaaac 6720

tttttttgat catagtaccg acagaatgat tggaaatata agccaattgt ccgcgcaaaa 6780

atggcctgcc acttccagca atcatgacac catgatctga ctctacactt gcggtgacga 6840

gatcaggcag acctatgcca gccgcacgct taaacgcctc gacagcatgt ggatacaaaa 6900

tatcatcggc acccaagacc aaataataat cgcctgagct tagcgaaatc gcacgattta 6960

atgcatcgta tataccaaaa tcctgttgcg ctgacagcaa aagattaatg cctacaacgc 7020

tttttagata agatatagtt tcgtcacttg aaccgccatc tgcaacgacc cactcgaaat 7080

ccatatcagt ttgactaagc aggctttgca ctaaatctgg taaattttta gcggcattat 7140

atgttgccgt aatcacacta agtttttgca ttttatttga tcttagctgt agcggtaaat 7200

cctctgatcc atgtgtaata agaagccaat tttggcctaa taccacattt ttctggcaac 7260

aatggcaacg gaatggcaaa ccgatgcaac atcccaattt ttttctgcaa agtcgcaaga 7320

tactcgtcat agaggtaacg agaactttta gctatctggt acccttcata aagtctgaca 7380

agattaaact tgattatgcg gaatgcatga aagtggtaaa aaaccggctt gaatttccca 7440

ctttggtgct gaagttgata gtctacattc caaggtgcca acgtttgttc aacgcacttc 7500

aatatgtgga catcatcggc aaaaattgta ggccataaat ctaaatattt ctgatcacca 7560

aactttccat cctccatcct tgcaaaacac cactccacgc agcgatcttg ccaccaatgc 7620

ataactttac gggatgcttc acttcgatca aataccatga attgcacaca aaaccgtcca 7680

ctcgtttcca gatgctgaat gtaatcgggt gcatatgcat gctcagtaat catcacatgc 7740

ttgccgcttt gttcaaactc atgaaaaagt atcgcaggat cgttgaaaaa aaataaatct 7800

gcatcaagat aagtgactcg tgccacctct gggtgttgag caaacaccca ttcggcagtg 7860

aatggggtta tggtccagca atactccccc tttgttctgc ctggtttaac ggccaataat 7920

gcctcgcttt caaaatcggc caagcgcata agcgaaacat gaggcaactg caatttttcc 7980

agttgctcat gcaccacttt atccatacag actatccata aatgaaaatc cctggcatgg 8040

cgggccagcg actcatgcag gcacaaacct tgaggcagaa acttgctatc aaacagtgtg 8100

acaaaatgct ccatgtttac agtgcaaacc tattgactgc agcaatgacg gtctgaacat 8160

cttgttcaga catttgagga tgacatggca gcgaaaggca ttgctgtgca tgggcctcac 8220

taaattgcag cccagccgga tctctcaata tatcgctgca cttttcctga tgatgaatag 8280

gcacaggata atgaatcaat gcctgaacgc catttgcctg caagtgagcc tgcaatgctt 8340

cacggtgctc cgttagcaca acaaacaaat gatagacatg ggctaatggc tctgttggag 8400

tttgcatgag gcgcacgtat ggattattca actgctgcct gtattgagcg gcaattgatt 8460

gcctatgact tgtatattgt tccagccact ctagccttac cgtgagcaag gccgcctgaa 8520

tttcgtcaag acgactattc aagccaaact ctggatgatg gtaacgcaca ctttggccat 8580

aatttcttaa cactgcagcc ttgtctgcca gcgcagattg agatgttacc aaagcccctc 8640

catcgccaat ggcgcccagg tttttggtcg gataaaagct gtaagcacca aagcgaccaa 8700

aactccccgc cactttacct tccgcttgcg ccaaatgtgc ttgagcacaa tcttccaaca 8760

aagccacttg atgctgatcg cacaagttca gccaggattg catatccctg atttgcccgt 8820

atagatggac tagcaagacc gctttcgttc gcgagttgat gcatcgcttg acactttcat 8880

gactcaataa gccagtgtca ggatcaatat cggccaagac cggcgtagca cctgcacgta 8940

tgatcgccag cacagaggca aaagccgtca tcgcagtggt aatcacctca tcaccgggcc 9000

caatgtccag cgcgcgcaat cctatctcaa tcgcatccat gccattgcca acacccactg 9060

tatgagaagt gccgcaacgc tgagaccaca atgactcaaa cttgcgcccc tcctcaccta 9120

atacatacca accggagcgc agcacacgct cgacggcttg cagctgcgct gcaatcaaag 9180

cttctggttc agccttgaag tcattcatta aaatcatacg ttgtcccaat atttatcatg 9240

atgctggcga tagtatgcca ggcttgcagc aagcccttgt tgcaagccca cacgggggct 9300

ccaacctaaa acctgcttga ttttattgaa atcactatag tagtcaccga tatcaatggc 9360

ttttctctca ggtgggaatg gcacaatctc atagctggaa ccaggctgaa attgtgtcat 9420

taattcagct acttgctgta aatttacaac ctcatcactg ccgagattaa atatttgccc 9480

atctgcttct gtattggcgg cagccagtaa cagagcatcc accacatcat caacgtaatt 9540

gaaatccctc aattgcaagc catctccaaa taccgcaatt ggcttgcctt caatcactct 9600

acgcaaccaa atcccaagaa aagtctgacg ggcatccttg acgcgcatgc caggaccata 9660

tgtatttgtc agacgtagcg cacaggcttt gataccgtaa acattgttat agagaatgtg 9720

ataccactcc cctgccaatt tattgatgcc attcacatcc actggctgaa tcgggtgtga 9780

ctcatctaca ggtaaatact caggcttgcc ataaagctgt ctggtgctag cgaagactat 9840

tctgacatcg ggattatgct gtcggcatgc ttccagaatg gataattgtg ctgcggcatt 9900

gatatccaga tccgtcctgg ggtcggtcat ggaatccaaa tggctagttt gtccagcaag 9960

gttaaaaagg taatccttgc ctttgataag ataagccatg gcatgactat cacgtacatc 10020

gctgatgttg atattgacca gcgattcata gccactaagg ttaaaggcat tgccaccata 10080

aagaggtaac aggctatcta caacggtcac cttggctttg tgcgcaatta atcttttgac 10140

caagtttgaa ccaataaaac ccaagccacc agtaacaaga atattcttac ctgagtacag 10200

ttcatcattc atacaattac cttaacaatt tcacaacaag agaatcaacc cgtacattag 10260

aagaaataag acacagtaac tcaaagatga acaattgctt gttagaaaat caacttacac 10320

tcttgcgctt gaaaaaaagc cctttgtaat agcctatgtc cgtcaggtct gcacaaggaa 10380

actccatgat gacctcatac cctcctgcac tcatagtatt tataaatttg tcccaagaaa 10440

tgaaccaggc agggtaactt gccggataaa tcgatgcagg cactgtctgg acagttaagc 10500

ggtcaacaac gtcataaaaa gccgtgcgat cgaaaagaat aaactcagaa tctagcctca 10560

ataattcttc taacagctgg tatggatgct ccaaatattg caggacgctg gaaagaaaaa 10620

taacattggg ttttccatcc aaaataaact ctgtgaggct attgtaaaat ttaagatcag 10680

ttgtggttag gtgctcgcga ccccaggcaa caaaatgtgg ctgctcaaca actccccaac 10740

gcaaatcctt aactatagac aatattgcag catgctgaga ataaaaactt gccagagagc 10800

ctccaaaatc caggacactc aacttgccac cagaactaac agctgcgtat agcaatccag 10860

acaaagcgga atatctatac tcaacatgat ggaaacatac tgaatctcgt tcaaaaaccg 10920

cttcaccacg cttgactttg agagctgact cttgaatgat ggtgagaata ttttccgcat 10980

catagccacc actattttgt ttagcatgcg cccaagaaga ataatttccg gcgaacgaag 11040

aggcaaagtt acgccgccta tacttctgca gaatatgcag aaaaagtggg ggcataaata 11100

attttaggat cttgataaaa ctcataaaaa ttttttaaat acggattttg ccttcagcat 11160

cattttgcga acaactgatg gtttgctttc aagaaaaaat tgctcaaatt tcttaaatgc 11220

cggctcagac tcgcgcaatg gaatatcagt caatagtaat cggtcagctc tcaactccgc 11280

agagtatgtt tcgctttctc cacaatgcac accggatcca tcatgtccaa tattactaag 11340

caaagcttta ccaggataaa gcgtcaactt attaagcaag aaagcactag catgccaacg 11400

tattgcccaa gaattattgg ttcctgcaat ctggtcgcgc aacatatttg aaaatggata 11460

actaccgtta taatcaaact tattcagcaa gttaagctgc ttcagttgcg caaacaactt 11520

acttccatct ggttcaaacc aatcccatct atccctccat gtagcccagc cccagcaatc 11580

tgcaccacgc aaaaagaaag tatcgggcaa tcctgcgatg ggataaacat aggcatgaat 11640

acttgcaact tcatgaaaat cagcatactg actcaagcta tcattcatgt attgaagaaa 11700

atatggactg gtgaccatgt cgtcttcaag aactattatg cgaccatact ctgcaagtac 11760

atttgttaca ccatcaataa tcgaagcagc tagcccaaaa ttcgtttgac gttctatgat 11820

tcttactgac ttgaagcctt caacagtgtg agtaaacgct ctgacagcat ccaccatgct 11880

ttgctgtgta atattttttg cagcatcaga aaaaatcagc aagtccgttt cagcagacaa 11940

ggtattattc ttcagtgctt caatcgtcct tttagtatgg tcagggcggt tataagtaaa 12000

cagggcaaca ggtgctagat tggacatggt tacttacagt ttcaaaagat cttgataaaa 12060

ctcagtgttg aatgtacgcg ctatgacttt gtaatgattt tgaatcatga aactgcgtat 12120

gcgttcgcca gcgacatcat caagtgcttc tgccagaact atacgaggcc tgtactttga 12180

ccagtcgtta gaggcaagca cttggtaatc aaagccttca acatctatac tcatgaaatc 12240

aatttcagta tccggcagca gcacttcatc caaaatttta gtcaagggtc ttactggcac 12300

cttgcgggta ccgacgcgtt tataagcagt gttttgtata cgctgattgg ccaattcctc 12360

actgaaggta ttgagagcag gctcatcgaa catatgataa gtcagctcag actcatgatc 12420

agataccccc atttccaagg caatatctgc tgggcgccgt gttttaaaca aagcaatagc 12480

ctcgtgtgag ggatctatac aaactcccct ccaaccacgt ttataaaata aataagtatt 12540

ggagaacctt actgggtgat gagcacctat atcaatataa tacccagtag aggttcggtc 12600

aaaaattcta ctcagaatta aatcttcccc ctcttgcgca taagagaatt tatataagcc 12660

tctatcccgg taaaatgaaa aaatctcttg cgaataatta aatattttta aagcaatact 12720

tgagatagac ataaatttat actttattga ttaatgccaa atcaactgta tgatcgactt 12780

gagacatgat ataaggatca tgactgacaa atataatgat cttattttca tactcattca 12840

taagattccg tatcacaatt tcttgtgtag ccttatctaa agctgttgta ctttcatcaa 12900

gtatcagcac attaggtttg cgcactaatg ctctggcaat agctatacgc tgacgctgcc 12960

cacctgaaag attttttcct tgatattgca aacgtgtttc ataaccttgc gcaagacttt 13020

ccacatcttt agaaatacaa gctaattcgc aagcccattt tacgtcctct agagtaaaag 13080

tctcgcccat acaaatattg ttgtagaccg tatcatcaaa aatcgcaggt tcttgattaa 13140

taagtacaat agaatgcctg atatgtgctc cagggatatt ttctgttgaa ataccatcta 13200

ttgaaatact gcctgaagca ggaggataaa acttcagcaa aacatccagc aaagtagact 13260

ttcctaaacc agatttcccc actaaagcat aagagttgtt cttttcaaac tcgtagtaaa 13320

cagaattgag aaccgctggc cttacgcctt gaacatcacg gtatgaaaac gacaaattat 13380

caatcttgat tgtatctatc ttgtgtaaat agttgtctga attagcgctc tcggcacttt 13440

catcggaaac taattccgtc acatcttttc ccgatttcgc atctgcaaca atgcgaacag 13500

ctagagtgat actctgacca acaactggga aaaatcgcat gagataagct atcaatgaca 13560

caatgtaagc catccctaag ttttcaacag aaacccagcc actcaatatc aaagccaaag 13620

atccgagtaa tagaaatatt attggtacta acttcgtagc cagatttatt gcatcaaggt 13680

aaaaaagaat atttgcatac gtaaaaatct ttcgcctata caaaccggca acatacttct 13740

cggaagaaag tgatcttact gctttgatat tgttcatcgc atccatgaaa atggaatttg 13800

ctttccttga accttctatt tgcctcacgc ccatcttatg agatattttt aaaatccaaa 13860

tgaagaatac ggcagacaca agcattaaaa tgaaaatcat ttcggcggca tatagtgaat 13920

aaattgcaat agaaataaaa tacattaccc ccagtaaaaa tgttgcaaca aactgagtca 13980

atgcaacaac cagagtactc gccctgaagg attcatctcc agcaagcccc ataaaaaaac 14040

ctatactttt ttcagatatt ttatcaattg acatcgaagt aatgattttt tcaaaagccc 14100

tagaaccgag ctgagccatc acacgtcttc caaggtagtg cgtcaggctt tgactaaata 14160

gttgcgtgac tactctcaaa gtgaacaata agaagaaaac ttgcaaaagg ctggcagcat 14220

caactgtaaa ccccaatata atcagagctt ttgaaatcaa accatgctta tctactgttt 14280

ctcctgaaac aagttcaaat aaaggcaata ggcttgccat tgccaacaat tcaataccta 14340

tagaaagtat agaaattaaa acactcaaat acaacaatgg gttgttattg aaagcaaatt 14400

ttaataactt aaaaatatat tttgcttgtg gcaatttatt caacaacttt cattttaatg 14460

gtaagtgtta ctaaatataa attgtattaa atatcagata attaacttat atcgaatgtt 14520

gttttaccgt agcttcaatt tcataatcgg aaacagcatc gaccacatca gatattacac 14580

cctcaaaaag gctagattga ttattaagaa ccagacttaa aaatcttaaa cggtctgtgt 14640

cgttggactg gacattccat acgtcgtagc tttttcgcaa attaactgaa aacttgttag 14700

ccaccctttt caatattcta tacgccggct tatccggcca actcagacca atctccagtg 14760

gctgattaac tctgacagtg cccagtgaaa aactgaccgc gagccagcga taggcattaa 14820

ctacatgtgt attatctgtt gatgttgcca aagaaagtac tttgataagg ttgtcataat 14880

actcatgttc agactctccc gttagatgta tatccgccgg ataggaaggt aagtatttat 14940

cataggtaat cacaggtatg ccatgtagca atgcttctac accagtggca gaccaccctg 15000

ttaccagcgc atgtacatca tcgagaaaat cgtataaaga aatattctga tctggtgagt 15060

ttaatttaac attctcaggg agatcctgaa gtattttttc ccaaatcaat gcttgctcag 15120

actgattgga atcgcgttta ttaggatagt ctcgcggatg tactctaatt acaagatagt 15180

catcaggcct agacttcata aaattaattg tacacttaat ccattcaaac tgatctgaga 15240

aaaccggact tttaacttta cgctcaggaa aacctttgat aacaaaagcc gcatacgctt 15300

catcaaaact acttaatgag gcgagtagaa ttttagcatt ctgtggaata ttgaattttt 15360

cacggattga aaaatttcct gtttttggcg ctgaaaaaac agcaaaagat ttacctttga 15420

ataattcgtg aatatgtgca gagactctat caatatcttc aagcgttact aaatttttaa 15480

cagcttgcca atgctgcaat gcagggttta ctaatttaaa tttactccac tcccacaccc 15540

ttaaagcact atatcgttca gcattgctag agctaccttc tataaaatat gcctgtctcc 15600

cctgttttac tgcatattct gcacacacac cattaacacc atactgtgga ctataaaaga 15660

aaattttttc tggacactct tgtttgaata tttggataaa accgattaac gataccaggg 15720

cattcctaag gtgaatctgt agatcttgcc attgttcatc tctaaattcc aaagacattt 15780

tcttatactt aagaagtggc tcatatgtag ccaaacgccc tactggtata tcttcataat 15840

gaaattcgag gaaattatct tccgtgacac ttttcaatag gttatcaatc agagcattag 15900

ttttctgatc aatataatca tgcaatttaa gatgcttgca atcaaaagac ttggttattt 15960

tattggcgtt attaatacat aactcacaaa ttgcactttt ctttttaaaa tcctcattca 16020

atgcaagatt gaaagcactc ataccagtgc aataatgagt aaagctttta tcacaggtca 16080

catatactaa ctcatggccg gcattactca gctgtgatgc caaccggctt tcaggaagcg 16140

catgtaacca aatagctgca tgcaaagaat aaaacatggc tttcatactt ttacttttct 16200

caacaacctg tgataggtca aacgtaaatt agctggacgc tctacccatt ttccaatata 16260

aattgacata gttaagatga ttattagcga taatgatgac atcaactctc tagtcaggct 16320

accgtacaca tcaaagttat atgagaagat ggcgtacgat agtaacaaca tagggaaatg 16380

gacaatatat aaagtgtacg aagtattggc caaaatattc attgccttaa tttctggcac 16440

tttgcatttt attgctgcca aaaataaaga atatacaaat ataacttgca ctggcaaaga 16500

aacgaacttc tgtccaatca ctgccatagc aaacaccaca atagccatta caacaaccaa 16560

agtcatgaac tttatgaaat ttcgacgata taagaaaatt acaccaccgc tcaaccacca 16620

taagccataa taaatatcgg tttttgtatt aaaaaacaag aatacagata atacgagaaa 16680

accagtatta gcaataaaat tgagcaaatt tttttgttta taatgaataa aaattattac 16740

aactaaacaa gcataaaaat atagcttagc ctcaatgcat aaactccata atggcccatt 16800

catttgcaac aagccattat tcatcaataa agaattgaca atgtctcgcg taccaatgct 16860

caagtagtct cttgcctgat agatatctcc tggtgccttt aaagtaccca ctgatcccgg 16920

tagttcaaag atcacaatca cactataggc aactaaagat attattatgc ttacaatgag 16980

aggaggatat atgcgcaata atctgctaga tgcgtaatca agtactgaaa gacgccctcc 17040

attacgttga gtattgctat gcaaactatt cgaaattaca tagccactta atacaaaaaa 17100

taagaggacc gcggtttcac tcaaaaaatg attcaactca tatttccagg acaccaatcc 17160

aatggttggt aaccaataaa tttgaaaggt gtggctgaga aaaaccgtta gtgctgcaat 17220

cgcccttaga taatcaaggc ttttaatcat aataactaag catacaatta caacaattaa 17280

tcatttaagc ttatgctcac gtaaccactc cactaccctt ggagcaatca aatcacgttt 17340

ttttgtgaca acatcaagta tgttacagct ctcatcacga gatatagatg aattttcaat 17400

aacaatattg cactttctca acaattcatt tttaaaacct atttgtctga catcaacaat 17460

tttttctttt attcccaact cataaaacaa ttctttaatt tgcttgtatg agtacaatcc 17520

tattactgga attttattag ctatggctac aacattggca tgaaatcgca taccaagtat 17580

caaagtacat ttgctatatt cggaaaataa tgtcccttga aaattcacat tagtatcgta 17640

actggccact ttgacatttt cacgtctaca tgcatcaggc aaccgtgata gtatgtcggc 17700

atatacttta agatcactat aaatatgtgg aaaaaaaata aactttatta atttattttt 17760

attccacatt tcagccaaag tctcggtgaa ctcggatata aatcctgagt aatcatatcc 17820

attaattcca cctggaaacc ttgtttccaa catatctcca gccagattga tgccgatggt 17880

gatctgagtg ttttcatgat cattttccgc aatgtctttt tctaacttat ccagatttac 17940

aaaaaaccca ccgtcaggca atggtaagac tttatccaac atgggattat tagaaatccg 18000

atttctgagc gtgcccattg ctccatcatt acgaacactg accaaatatt gctttgatga 18060

agttaagatt tctaagaaat tagtaaactt tgcaagactt tcatcactac atcccatacc 18120

atcatcaaca cccaatgcat tgaaaaagat ggggactttt atttttttaa aaacctcaat 18180

ctcaattgag attgaggtgc cggttggaga agaatcaacc catagctcga aaaaattacc 18240

acctcccacg actataagat cgtactgatt tgcaatagct acaaaattat cgtcaaaggc 18300

aacatttttt cggtaaacat tgcgtatttc gaattcatcc cattgaatct ctccaagcag 18360

tccttgaaac caattgcgaa aaccataatg gttggcaatg tcaccgacat ttccggagaa 18420

acttcctatc tgcaacactt tcattcttag gtcacctgat gcttcagtat ccaattactg 18480

tcttgctttt cccacaaatg tggagatcta aacctatcta cggtttccca aaactgtgct 18540

tcggtgatct ctatatattc aaggaattct gcaaagtact tttttggaaa ctcttgatca 18600

tatttatgaa ctagagcaac cccttcttct cgcgtaattt tatcgttgcg aatttcttgc 18660

gcagcatcat atgttgccct accaatgccg aatttgatca aggtagtaaa gtagtggaac 18720

atatctattc gatcatcgat actactgtac ttggagtagg ttccttcagt gcgttccgta 18780

tttgcctgga aaccagtatg ctcgactgca tagtaatagc actcttgtgg atcccatttc 18840

aggtaatagc ccaaataatg tacctcgata ccatcgcgct ctagatcttc tgcagcaggc 18900

ggaatatatg gcgcgaaatc atttaagctg tattcaccgt ctttaataat gtctccaacc 18960

ttcacaccac ctaacaccat gtcagctggg tctgaaatag agaaaaaatt cttgtccatt 19020

gtcggtttat agttttcttc tgtcttattg ccatattcag cttgattttc accatacatt 19080

accaatttga taccaaactt tttagccatc aacggcccga taatgcgttg gccaacaatg 19140

aatggttgga atgggtgcaa taaattcaga aatgccattt ttgttaaata gcggtgcaac 19200

ttgccatttg gggtaaaaag aatattgtct aatccacctt tatggatcca gttttcaaaa 19260

ttcttccaac caatctcagt gtacttatgc ggcgcccacg tcactgttaa aggattcata 19320

ccatatttat atttaaggat atgtgaggta tatgcactgt ctttaccacc actgccgggc 19380

acgacaatgt catatccacc atcgctacgg cgatgttgat ccaacaatct aaccaaagcg 19440

tcctcgcggg cctgccagtc tatttgagca tccttggttt ctgcatagcg gcaagcatca 19500

caaataccat cagcgccaaa tcctatggtc gcttttttgt ccttgttggt atgcttgaat 19560

tcaactgtag aactgggtct ttggttggaa atcacgcatt tcttgcaaaa cactacatct 19620

tccggcaaac caaaatatgc attaaccaca acttcctcca cacttctttt caattaactg 19680

ttttgtacaa agttattaag aacctgtaac ccgctgggcc cacttttttc tgggtgaaat 19740

tgcatacccc agatattgtc tttctgcact gatgcaacaa aagtacatcc agaatacgtc 19800

gcgctgcctg tgatgtatcg atcaggtaca ttttgagcgg cataactgtg cagaaaatag 19860

aacttacgct gctcgtctgg cagtccatca aacatgccgc cattattttc atgaacggaa 19920

ttgagatcaa ctttcgccca ggatatatgc ggtaacctca ctcgtgattg ctctacgggt 19980

acaattctgg agactacgcc aggcacaagt ccaagaccag tggtaacgcc aaactcctca 20040

cccctgtcga aaagcaattg catgcccaaa caaatcccca agacatgttt tccacttttt 20100

atatattcga gcaagctagc ttgtaatcca tctttaccca atgcagacat gccttgagca 20160

aacgcaccaa cccctggcac tattaacgcg tgagaatctt taagtgcctc tggcgtattt 20220

gagattacag gatgagcacc tacgtacttg atcgcattac tcacactacg cacattgcct 20280

agcccataat caagaataca tattttcttc atttatttag taccattgat gaatgtcaga 20340

cgctgtttga attcatctat cgtattgatt ttgtaatgca atactgacgc cagacaaact 20400

ccacttggct ggatttcttt taactcttgc acatgatcag gattaccgca gccaccacta 20460

accatgactg gaatgttcac tatcgtgtta actgcaccaa gcaatggaag gtcaaatcct 20520

ttttttgtac catcttggtc tatagacgtt agcaaaatct cgcctgcacc aagatcttcc 20580

agctccttgc tccaagcaac aacatttttg cccgtaggct ccctcccggc ttccatataa 20640

gcttcccagt cattgcctat ccgtttggct tgaatagagg caaccacaca ttgactgcca 20700

tagcgcttgg caatctctgt gatgaatttc gggttaagta ttgcagctgt attcactgcc 20760

actttgtctg ctccggcact cagagccatc tcgacatctg ctaaggaacg cagccccccc 20820

cctacagtga taggaacaaa aacttcttcc gaagccttgg cgattacaga cagcaggtta 20880

ttacggttat acaagctcgc taccgcatct ataaacacaa tctcatcaat gccttgtgca 20940

taatagtttt tcgccatctc gattggatcg ccaactttcc gcaagccctc taggtgtatg 21000

cctttaatca catgttcatt tttgacatct agccttgcga tcaacctcac actgcactcc 21060

tatcgataaa gtgcttttgt atgcccttat cccaaaaagc attattcaac agaatctgat 21120

gaaactcacc tgcactatta ccattaccaa agtcagaaac tgccacgcga ggaatgtctg 21180

caacttgttg aacagcatgt ctaagggaat ccacatgtgc agtagtatta agaatcgact 21240

ctgctgttgc tctattgtgt tgcctgctac caacattgat ggctggcacg ccgaaatgag 21300

gagcctctct tactccagcg ctagagttac ccatgatgaa cgctgcattt ttttagcagc 21360

gttaaaaaat attcaaatct aatggatgga taaactctaa cgtggctcaa gtttttcaat 21420

cgcaaatact cattcaaaat aatgtctgaa ccatgatcat tattgggata tacaacaatg 21480

aatggtagag agctttctga aatataatct acgatggctt tggcttgtct tggcaaatca 21540

tctaactcag aggtaacggg gtgatacatc aaaatgccgt aatgttcgaa cgagaagcca 21600

taacgttccc tcacatcatc aatgcttggc agcgatgttg aattcataac atcaatatct 21660

ggggagccaa taacaaaaat ggattcttca ctttctccaa gctgaataag tcgatttttt 21720

gcctgttcgt tggaaacaaa atgcccatgg gacagcttgg tcaccgcatg tctaatcaac 21780

tcgtcaacag tgccagagac ctctccgccc tcaatatgtg ccactcgaat gttatttaat 21840

gcgccaacag cggctccggc caacgcttca actctatccc catgaatcac aatcagttct 21900

ggtgaaattt cttttacata atctgataaa ccagtaattg ttttggccag aatatgatcc 21960

atgctatcgc tttgattttg attgataaag cgaaataggt ttttaaatcc ggctttttct 22020

acttcttcac atgtataacc atatttcgac aacatatgca tgccagtaat gaacacatgg 22080

acgtcaaatc ccatgtcatt ttgtaatgct cgcatgaggg atttcagttt tccaaaatct 22140

gctcttgttc cagtaagaaa aacaactcgc ctactcatgc ctcaatatcc tgccgattaa 22200

ctcttttgcc actcagtata gtttgcgatg ctttacgacc aagtagctga tcatagtcag 22260

aagcattaaa atcaccgcca cctggcctac gtaaccaaat attatcttca cttaagacat 22320

caccaacatt gatatcctta attgcaacga cagatgcaaa agcaaaagct atagtttttt 22380

cttcttctaa aagtggacct ttatgtgcac cttttgctga aaaaataact tttgaaccag 22440

tgattaaatc ttgcaatgct gcagggtcca ttgaacaaac aatgtctggt cccggccgtg 22500

acatggagtc agtaaaatgc cgctcaagaa ttgaggctcc tagtgcaaca gcccctaggc 22560

aagcgtaatt cccaagggtg tgatctgaaa gtcccacaac cgcatcagga tatgcatttt 22620

taatgacttg gatagcatca agtcttacaa gctcaggagg ggtagggtaa atattcgtgc 22680

agtgcagaag agcaaaagga acttttgctt gccttaatat attgacggtt ggtgcaatgc 22740

tttcaatgct attcataccg gtactaacaa taataggttt cccaaatgaa gcaatatgcc 22800

gcacgagagg ataattattg cactcgccag atccaatttt ataagccggt atatcaaatt 22860

ttctcagtcg atttgctgcc attctcgaga atggcgtact aataaaaata ctgccttgct 22920

gctgcacata ttgcatgagc ttatattcat catcctctga caatgagcaa cgttgcataa 22980

tctcatagat tgaaacatcc gcatttccag gaataacact cttggcttca tcagacattt 23040

catcttcaac cacatgtgtc tgatgtttta ttatctctgc cccagcatca attgcagctt 23100

ttgccatatc aatcgcaaga tctaatgaac cctcgtgatt gatgccaatt tctgcaataa 23160

ctataggctc atgtccgtcc ccaattttcc gcccagaaat ttcaaaagat gccatatttt 23220

ttccttaata atgcttctgt ttctaaaaaa tcttcttcgg tgtcaatatc aacactttct 23280

tcagcagaca taatataagc aacactacca ttggagggga aaccgcctct agcctcaaat 23340

tcaaatttcg aaaagatata aatagcacca tttggcgcgt aacataatgg caaatcttga 23400

cgacgataat tagataattt ttcatcaaat aatgaaatca atttatcatt atcatctaga 23460

cgaaatgact tatatggcga cttatccaag acttccacac tgaccaaaga agtaaggcca 23520

ttattattca aagccgtcaa agcatcatca atatgatgtt catctcttaa tggggaggtt 23580

ggctgtaagt aaacgattaa atctccatcc tctatatttt caatactaat aaaatgcttg 23640

acaacatcaa cagctgacgc atcatctgtt gcgtattctt gcggccttaa tattgaactg 23700

cagccttgtg cttttgaata ctccaatatt tctttacaat cagaagatac ataaacagca 23760

tcgacatttt tagctttttt agcagcgtta attgtgtaag ctattaatgg agaacctgcg 23820

atatttttca ggtttttttt tggcactcct ttagaacctg ctctggcagg aatcaaagca 23880

aacacttttt ttttcaaaat tgaacattaa attttaaaaa ttatcgaggt tatataaatt 23940

atctaaacgc tatttttgag gataatatca attcgatatc agaaataaat taataaactc 24000

agatttattt tgttaaaaat tccgaatatg tcttttccaa tccatcatca agctcaatgg 24060

aataagtcca ccccaaactg ttgagcttgc ttacatccat aagcttacgc atcgtgccat 24120

caggttttga actatcgaac tcaattgctc cttggtaacc aaccaccttt tttaccaatt 24180

cggccaattc ctgtattgaa atatcgactc caataccgat attgactagt ggtggatcaa 24240

acacaccagt agcactctca tcacttccca gcagagaatt gaatttctcc tcaggcagat 24300

tcagcaagaa tatacaggca tctgccatat cctcactgta aagaaattcg cgcctgggtg 24360

ttccagtacc ccagaccgat acagctgcat gcccagcttg tttagcctcg tggaatttgc 24420

gtatcaatgc agggataacg tgactattct gcaagtgata gttatcgcca ggcccataaa 24480

gatttgtcgg cattgctgca agaaatcgag tgccatactg ccggttataa ctccagcaca 24540

tttcgactcc agcaatcttt gccagcgcgt atgcacgatt ggtagcttca agcgatccgg 24600

ttagaaagtg gtgctcatgc atgggctgag gagcatgctt ggggtatatg cagctggacc 24660

ctaaaaacaa taggcgcttt actccatgct tccaggcact atggatcaca ctgttctgaa 24720

ttgcgagatt gtcacggata aaatctgccg ggtaagtgtt attggccact atgccaccca 24780

ctttggctgc cgccaggaaa acatattcgg ctgatactga gcaaagaatg cttccgtggc 24840

attagcatct gtgaggtcaa gctctgcatg ggtacgcttg ataagattgc tatagccctt 24900

gccctctagc cccctaacga tagcggaacc cactaggcct cgatgccctg ccacatagat 24960

acgtgcgtgt ttgtccatag gctcaggtta ctcgttatag tcataggcag caaaaccatg 25020

ctgcttaacc agttcgtcac gctctgcgct cttgagatct tcacgcacca tttcagaaac 25080

caactcctgg aaactgatgc tgggcgacca gccaagtttt tgctttgcct tgctcgggtc 25140

accaagcaat gtttcaactt cggtaggcct aaaatagcgc gggtcaacct gtacaatgca 25200

cctgccttga ccgtcgtatc ccatctcttc ttggccctga cctttccagg tgatggcaat 25260

atccagctcg tgggcggctg cattgacgaa atcgcgcaca ctgtattgca cacctgtcgc 25320

aatgacgaaa tcctctgcct gttcctgttg caacatcagc cattgcattt caacataatc 25380

cttggcgtga ccccaatcac gcaacgcgtc cagatttccc aaataaaggc aatcttgcaa 25440

gcccagcttg atgcgcgcca aggcacgggt aattttacgg gtaacaaagg tctccccacg 25500

caaggggctc tcgtgattga ataaaatacc attacaggcg tagatgccat aagcttcgcg 25560

gtaattgaca gtaatccaat atgcataaag cttcgcaacc gcatacgggc tgcgtggata 25620

aaatggggtg ctttctttct gcggtgtttc ctgcaccaaa ccataaagct ctgaggtcga 25680

ggcttggtaa aagcgggttt tcttttccag ccccaagatg cggatagctt ccagaatacg 25740

caaagtccca agaccatccg cgttagccgt atattcagga ctttcaaaac tgactgccac 25800

gtggctcatt gccgcgaggt tatagatttc gtcgggctga acctgctgaa tgatacgtat 25860

aaggttggtg gaatctgtca aatccccata atgaaggatg aaattctggt tatcgacgtg 25920

agggtcctga tacagatgat caatgcgatc tgtattgaaa agtgatgctc ggcgcttaat 25980

gccatgtact acatagcctt tgccaagtaa aaattcagct agatatgctc cgtcttgtcc 26040

agtgatacct gtaattagag ctactttttt taacat 26076

<210> 181
<211> 4093
<212> DNA
<213> Methylobacillus glycogenes

<400> 181
gtgaataacg gatttgtaac taacttcaag caatccagaa ccctgtgtgt cggtgctgtg 60

attgtgctcg ctttcgcatt gtcgggctgt ggcgacaagc atgaatccaa agccagccag 120

tctctggtta aggtcaatgg caaggaaatc accgtacatc agctgaatca ggagctgggt 180

cgtgccaaca ttcaggagaa tcagcgcgag caggcaacgc agcagattct gactgcattg 240

atagatcgcc agttacttga agaagctgcg ctcaaggcca aggtggatag ggatccaaat 300

gtgatgacca cgattgaacg tgcgaaatca caaatcattg ctcaggcata tctgcaatcc 360

aagttaggca gtgtcgccaa gccaagcaaa acagaaattg agaattttta taacgagaat 420

cctcaattct tcagcaagcg cagaatggtg gaaatggatc aattgttgat tgatactaaa 480

ttcgccaatg atgaattgaa aacagcactt ggcaaactca agaccttgca ggaagtagcc 540

gcatggatgg atgcccataa tattcagtac gatcaaggtc aggtttcacg cagtgttgct 600

gaattgccgc cacagatttc cgagcgcctc aaggccgagg ataagggtaa gttgttcgtg 660

atcggcatgg gccctcgcac catgttgatc atgatgaacg agatcaagga cagcccagtt 720

tcacttgcag aagcaacacc gcaaattgaa aacaatttgc tgaataaggc gcgcaaggaa 780

gttgctgaaa atgaagtcaa gcgcttgaaa gccgatgcca agattgagta catcgataaa 840

aagatggaaa cagcgactga aaccaaggca gccaaaaaga agccaagctc tactgaggat 900

gatatcgcca gtggtgttag tggcttgaaa taagtactcc ccatccttaa tatcaggacg 960

atagtaaaat gaaaaaatta atgacatggt ttttcttgct gatgctgagt ctgacgcctt 1020

ttgtaacgca tgctgcagaa ggtgacattc ttctcggcgc tggcgatgtg ctgaaaatat 1080

cggtgttcga gcagcctgat ctgtcacttg aggtcagagt gagtgaatca ggttccatta 1140

cttatcccct gataggtgaa gtgcgtgtgg gtggtggtac ggttgccgcc gccgagcaaa 1200

gaattgccac catgctggaa aagggtggtt tccttaagag tgctcacgtc aacatcatcg 1260
ttgcgcaatt gcagagcctt caagtctcta ttctgggtca agtcaatcgc ccgggcagat 1320
accctataga tagcgctaaa acgctggcgg atgtgcttgc cttggctggt ggtgtatcac 1380
ctgaaggtgg cgatctgatc acgctggtgc ataacgtcga tggtaaagcg gtcagggatc 1440
agattgacct tgctgacatg atgcgtactg gcgatctcag caagaacatc cgcgtagctg 1500
gtggtgacat catttatgtt gaacgtttcg ataaattcta tatctatggt caggttcaac 1560
gtgccagcca ttaccgtctc gaacatgaaa tgacagtatt gcaagcgctt tctgtaggtg 1620
gtggcttaag ccctcgcggc acagaaaaag gcgtcaagat caagcgtcgt gatgctagcg 1680
gccatattga agtaatcact gccaaacatg atgacctcat caagcccaac gatgtgatct 1740
atgtgcagga aagtttcttt taatagattg cagattttaa taaatacttg ataggcaagg 1800
ttaatctaat gagcttttct caacttttac tgatattcaa agcgcgatat aagatattgt 1860
taagcaccat tgttgtggcc ctggtcgtag caattgcgct caatattttc ttgccaaaca 1920
gctataaggc aaccaccagt atcctagtca actataaagg tgtagatcct gtgactggta 1980
tggctgtccc tgcccagctg cttccaggtt atatgggtac ccaggtggat atcataacta 2040
gcaagaaaat tgctaagagg gttgtgacca cattaaagct caatgaaagt agtgaggttg 2100
tggagagttt caatgactcc acaggtggtg aaggtgatat cgttgattgg ttggcggatt 2160
tgctgatctc acgtatcgaa gttcaacctg cgcgcaacag tagtgtgttg cagctggagt 2220
ttgatggagc caaccccaaa tttgctgctg ttgttgctaa ctcctttgcc gaggaatata 2280
tcaaggccac tatcgaactc aaggttgagc cttcgaaaaa ggcggctgaa ttttataccg 2340
ggcaattgaa ggatttgcgt gccgatatta cccgggcgca gcaaaagctt tccgattacc 2400
aacaagataa aagcatcgtc agcgttgatg agcgtctaga cattgaacgt gccaagctga 2460
atgaactttc ctcgcagtta gtgatgtcac aagggcaaac tcttgaggca cagtctcgtc 2520
agactggtgc acaaggtagc aatgcttatg gatctccgga tgtcgcgagt aatccaatgg 2580
ttctgcacct gaaaacagag attgctaggt ctgaatcacg cttcgcggat atctcacaaa 2640
aatatgagag aaatcaccca ctctatcagg gggccaaagc tgagcttgac aatctgaagt 2700
ctgaattaag taagcaaatc caggcggcta ccgtcaatat atcgagcaat tatagaatcc 2760
ttaagcaacg cgagagtgaa atccgcaatg ccttggctga gcaaaaaacc aaggtcatgc 2820
tgttgaacaa ggatagggat gcactgacgg tattgacgcg tgaagtcgag ggtgcgcaac 2880
gcgcttacga actcgcgact cagcgatata ctcaaataaa tcttgaggga cagtctaatc 2940
aagctgatgt atccatcctt aatccagcga caccaccact taagccatca agcccgaaaa 3000
aaatattgaa cgtggcagtt gcgttggtgc tgggtacaat ttttggcttg ttgtttagtt 3060
tgttggctga gctcactaat gagcgaattc gctcagaaag agtgctggta gagattgttg 3120

gcgcaccgct gctgggtgtg atcgacaaaa ataaaaccca ttccagaatt cagcgcctgc 3180

tggctaaaaa accctaattt gtgtggatat agacatgact aatgatgttt ccaatactag 3240

ttccattccg gcattgaatg ctgatttcaa tatcggtgga atgttgctca agatgggtaa 3300

gattacaccg caggatgcgg agcgtatcct gcgtgtgcag aaggaaagag gcgttcgctt 3360

tggtgaggct gccgagagtt tgggcctagt ttcctctgcc gatatcaact atgtattatc 3420

caagcagttt gcctatccct acctgatgga tggtcgcggc gatttctccg agaacttggt 3480

cgcggcttat gatccattca gtatcaatgt ggagaagtat cgcgccattc gtagccagct 3540

catgctctac tggtttgcac aagggaaaaa gagcctgtca ttttccagtg tttacgacaa 3600

ggacgacacc agtgacttgg ttgccaatct tggcgtggtt ttctctcaac tgggagagaa 3660

aacactcatc attgatgccc atcttgatag cccacggcag aatcgcattt ttaatatcaa 3720

gagcactgcg ggtttgtcgg atattctggc aggccgggtt acggattact cctcttccat 3780

cactaggcta gtcgagttcg agcatctctc catcttgtct tctggcacca ttccacctaa 3840

tccgcgagag cttctgggac gtggtttcct caagcagttc atcacggcaa ttgctaatga 3900

ctacgatgtt atcctggtcg atgcaccttc ctttgtccgc cactctgaac cgttgtctgt 3960

ggcaagtgct acggaagggg tggtgcttct tgccaagcgt catgtctcca aagccaatga 4020

cttgagcaaa gtggttgatc agctttccaa gatcagggtg cctgttgttg gcacggtatt 4080

gactgaaaac taa 4093

<210> 182
<211> 2434
<212> DNA
<213> Methylobacillus glycogenes

<400> 182
atgcttacgg atgaataccg atacaagatt ctgaaattgg tgcaggaaaa accagatatc 60

agccaaagag aactggcgaa agcactaggc attagcttgg ggaaaaccaa tttttgtctc 120

aaagccttag cgcaagttgg cttgctcaaa gtatctaatt ttcgcaacag caagaataag 180

ttggcatata tgtatgtcct cacgccgaca ggcatagaac ataaagctgc tgtgacactc 240

aactttctca aactcaagct caaagagtac gaacaattag caggagagat tgaagagtta 300

cgtagtgaga tcaacgctaa ggtaaatgat taagggcgga gacagttcct taggaaaaac 360

atatgttaaa aaaagtagct ctaattacag gtatcactgg acaagacgga gcatatctag 420

ctgaattttt acttggcaaa ggctatgtag tacatggcat taagcgccga gcatcacttt 480

tcaatacaga tcgcattgat catctgtatc aggaccctca cgtcgataac cagaatttca 540

tccttcatta tggggatttg acagattcca ccaaccttat acgtatcatt cagcaggttc 600

agcccgacga aatctataac ctcgcggcaa tgagccacgt ggcagtcagt tttgaaagtc 660

ctgaatatac ggctaacgcg gatggtcttg ggactttgcg tattctggaa gctatccgca 720

tcttggggct ggaaaagaaa acccgctttt accaagcctc gacctcagag ctttatggtt 780

tggtgcagga aacaccgcag aaagaaagca ccccatttta tccacgcagc ccgtatgcgg 840

ttgcgaagct ttatgcatat tggattactg tcaattaccg cgaagcttat ggcatctacg 900

cctgtaatgg tattttattc aatcacgaga gccccttgcg tggggagacc tttgttaccc 960

gtaaaattac ccgtgccttg gcgcgcatca agctgggctt gcaagattgc ctttatttgg 1020

gaaatctgga cgcgttgcgt gattggggtc acgccaagga ttatgttgaa atgcaatggc 1080

tgatgttgca acaggaacag gcagaggatt tcgtcattgc gacaggtgtg caatacagtg 1140

tgcgcgattt cgtcaatgca gccgcccacg agctggatat tgccatcacc tggaaaggtc 1200

agggccaaga agagatggga tacgacggtc aaggcaggtg cattgtacag gttgacccgc 1260

gctattttag gcctaccgaa gttgaaacat tgcttggtga cccgagcaag gcaaagcaaa 1320

aacttggctg gtcgcccagc atcagtttcc aggagttggt ttctgaaatg gtgcgtgaag 1380

atctcaagag cgcagagcgt gacgaactgg ttaagcagca tggttttgct gcctatgact 1440

ataacgagta acctgagcct atggacaaac acgcacgtat ctatgtggca gggcatcgag 1500

gcctagtggg ttccgctatc gttagggggc tagagggcaa gggctatagc aatcttatca 1560

agcgtaccca tgcagagctt gacctcacag atgctaatgc cacggaagca ttctttgctc 1620

agtatcagcc gaatatgttt tcctggcggc agccaaagtg ggtggcatag tggccaataa 1680

cacttacccg gcagatttta tccgtgacaa tctcgcaatt cagaacagtg tgatccatag 1740

tgcctggaag catggagtaa agcgcctatt gtttttaggg tccagctgca tataccccaa 1800

gcatgctcct cagcccatgc atgagcacca ctttctaacc ggatcgcttg aagctaccaa 1860

tcgtgcatac gcgctggcaa agattgctgg agtcgaaatg tgctggagtt ataaccggca 1920

gtatggcact cgatttcttg cagcaatgcc gacaaatctt tatgggcctg gcgataacta 1980

tcacttgcag aatagtcacg ttatccctgc attgatacgc aaattccacg aggctaaaca 2040

agctgggcat gcagctgtat cggtctgggg tactggaaca cccaggcgcg aatttcttta 2100

cagtgaggat atggcagatg cctgtatatt cttgctgaat ctgcctgagg agaaattcaa 2160

ttctctgctg ggaagtgatg agagtgctac tggtgtgttt gatccaccac tagtcaatat 2220

cggtattgga gtcgatattt caatacagga attggccgaa ttggtaaaaa aggtggttgg 2280

ttaccaagga gcaattgagt tcgatagttc aaaacctgat ggcacgatgc gtaagcttat 2340

ggatgtaagc aagctcaaca gtttggggtg gacttattcc attgagcttg atgatggatt 2400

ggaaaagaca tattcggaat ttttaacaaa ataa 2434

<210> 183

<211>  4120
<212>  DNA
<213>  Methylobacillus flagellatus

<400>  183
ttgaatactg aatatggaag cacaataatg caatggtcga aaatgacgaa aggggcgttg      60

gtcgccttga tgattagtat cgggctgtcg ggctgtggag ataaatctaa tgaaacaaag     120

gctagccaat cgctggcgag agtgaacggc aaggaaataa ccgtccatca actcaatgaa     180

gagcttgctc gggccaatgt ccagccagcg cagaaagaag ctgctaccaa gcagatattg     240

caatcattga ttgaccgtca actgttggag caggctgcct tgaaagcaaa agtagaccgg     300

gacccgaacg tcatgcaagc cattgagcgc gcaaaatccc aaatgattgc ccaggcatac     360

ctccagtcta gggtggctaa cataggcaag ccggacaaaa cagaagttga gaatttctat     420

aagcaaaacc ctcaactatt ttccgagcgc aagctggtgg aaatggaaca aatattgatt     480

gattcacgtt atgtcaatga cgaattcaag tctgcgctcg gtagcttcaa aaccctgcag     540

gaagtcgctg cgtggttgga tgctaaggga atccaatatg accgtggaga agttgcccgc     600

agtgtggcag agctgcctcc acagatttct gagcgcctga aggatgcaag gcgtggccag     660

ctgtttgtga tcggcatggg accgaggacg atgctggtgc tcatggatag cgtaaaggag     720

agcccggtat cactggcaga tgcgacaccg caaattgagc aaatgctctt gaataaaaag     780

cgtaaggaag cgggtgaggc tgaaatcaag cgactacggg ctgaagcaaa aattgaatat     840

ttagacaaat cggccgaagt accacaacaa ccagaaaaag ccgaacagtc aaaaccctct     900

agtgtcgatg atgatattgc tcgcggagta agcggactaa agtagtaatt ctataaaacg     960

tgtataggtg tagtgccatg actaaattga tcaaatggtt tgtaacggta gtcctgacga    1020

ttgttgtatt caatgttaac gcagcggatt cggatattgt tcttgggccg ggggatgttc    1080

taaggatatc tgtatttgaa cagcctgatc tttccttgga agtaagagtg agtgagtctg    1140

gcactatcac ctacccgttg ataggcgaag tcagagtcgg tggagaaaca cctgcagctg    1200

ctgaacgcaa aattgccagc atgctggaaa gcggtggatt cttgaaaaac cctcacgtga    1260

atatcattgt tgctcagttg cagagtcagc aggtttctgt gctaggtcag gttaacaggc    1320

caggtcgcta cccactggat agtgcaaaat ctcttgcaga cgtactggcg ttggccggtg    1380

gagttgcacc tgatggggga gatttgatta gcttggttcg caagattgag gggaaaagtg    1440

ttagagagat aattgatttt gctgaaatga tgcgggatgg cgacttaaat aaaaatatct    1500

tacttacggc aggagatatt atttatgtag atcgagctcc tagattctat atatatggtg    1560

aggttcagcg gccaggacaa tatcgtcttg agtatcatat gacagtgctg caagcactcg    1620

ctgttggtgg tggccttagt ctcaggggaa cggaaaaagg tattaaggtg aaacggttag    1680

atgatgaagg tggtgttaag ataattaaag taaagcatga tgattttatt cagcctaatg    1740

atgttgtata tgtacaagag agtgtatttt agtttgtttt ttgaataaca gctgaataat 1800

caaaaaggta atctaagatg agttttatca gatttatttc tatcattaaa gctagatata 1860

aaattattat ttttacttta tttataacgg tattaactac cgctattgtt acaatattgt 1920

tgccaaaaaa ttataaagca acagcttcag tgcttgttaa ttataaaggc gctgatcctg 1980

taacaggtgt aatgcttcct gcgcagttaa tgccaggtta tatggcaacg caagtggata 2040

ttatttcaag taaaaacgtt gcaaaaaagg taattgataa actaggtctg gcagatgttc 2100

cagccgtaaa agaggacttt tatcaagcaa cttcaggtaa aggtgaaatt aaagaatggc 2160

ttgcagattt attgctaaaa aaacttcaag ttaaaccatc tcgagagagt agtattattg 2220

atattagctt ttttggggta aatccccagt ttgcagctac aatcgcaaac gaatttgctg 2280

agtcttatat gcaaacttca atacaactta aagttgagcc ttctcaaaaa gctgctttgt 2340

atttcgttga gcaattgaaa gaactgagag aagatctagc tcaagcgcaa tcgaagcttt 2400

cagagtatca gcaagagaaa gggattgtaa gtgttgatga aagattggat gtggaaagag 2460

ccagattaaa tgagctttca tctcaattgg ttattgcaca agcacaagtt atggaagcga 2520

gatctcggga aaaaaattca agtggaaaag aagcttatga gtctcctgat gtagcagcta 2580

atccaattat ccaaaatctt aaaactgaaa ttgctagagc agaatcaagg tttgcagatg 2640

tttcgcagaa atttgaaaga aaccatcctt tttatctagg agctaaagca gaaattgaaa 2700

atcttaagtc tgagctagat agacagatca aatcggtttc aagtaatgtg gcaagtaatt 2760

caagaattct gcaacaacgt gaagcggaaa tacgtttggc tttaaataat caaaaagaaa 2820

aagtattaca acttaatcga gatagggata ttttatctgt tttgacaaga gaggttgaaa 2880

atgctcaaaa agcatacgac atggctatgc aaaggtatac tcaaacgaat attgaaggtc 2940

agtcaaatca atctgatatt tctttattga atcctgccgt ggcacctctt actcccacta 3000

gcccaaattt attgctgaat attatattgt caatcctctt gggtatgttt ttaggaatag 3060

ggtttggagt tttagctgag tttgttgatc gaagagtacg gactgctgat gacttaattg 3120

aagtattaca agccccactt cttgggacta ttagtaaaga aaaaagaaaa ctgagatttt 3180

ttaaaaatac ttcaaacctg ataactttag ggaaggctga attatgattt catttacacc 3240

ttctaattca aactctgaaa aagtagaaaa tgcaccattg gtaaacgaat tttcagaatt 3300

tacaattggt agtttattat tgcggatggg gaaaattaca cccgaagatg ctgaaagaat 3360

tatgcggtta cataaagaga agggtttgcg ctttggagaa gctgctcaag cattagggtt 3420

gataactgaa gatgatattc aacaggtttt atcgaggcaa tttaactatc cttatttatc 3480

acctgaccaa aactcatatc ttaatgagct cgttgctgca tatcagcctt ttagtaacaa 3540

agctgaggtt ttgagagcag tacgcagtca attaatgctg cattggtttt caccggaacg 3600

aaaagcacta gctgtttctt cagtaagcag cagcgataat gcaagctctt ttgttgcaaa 3660

tcttgcagtt gtttttgcgc aattgggtga aaatacatta ttgattgacg ctaatttacg 3720

ctacccacgc cagcatgaga tatacaatct gagtaacaaa cgtgggttat ctgatatctt 3780

agcaggaaga gcggggtatg atgtaatttc aaaaataaat ttttttgaaa acttatctgt 3840

attaactgcc ggtacattac ctcctaatcc tcaagagctt gttagcaagc caacattcgg 3900

aaatttaatt aaagagttgg cttgtgttta tgatgtaatt ttattggata ctcccgaact 3960

tagtaattat tcagatggtt atgcaattgc ttttactgtt ggaggaattg tattagttgc 4020

aagacaaaat catactcgag tgaatgattt atctattgct gtagaacaat gcaagaataa 4080

tggtactaag gtaattgggt caatattggt tgatttgtag 4120

<210> 184
<211> 13447
<212> DNA
<213> Methylobacillus flagellatus

<400> 184
atgctgcttc ctgtcattat gtctggcggg gccggttcca gattatggcc ggtttctcgt 60

gaaactctaa ctaagccatt tatcgaatta cctgatggca gaacccttct gcagaagact 120

ttgcaacgta ctgcttgcct ggatggtgtg agcgaagtgc tcacagtaac gaataaggaa 180

tattacttcc tgacacgtga tgtctatgag gcaacagaca ctaatgtttc ccatagctac 240

ttactagaga cgatagggcg caatactgct cctgccattg cagttgctgc actacatgcc 300

gctgcgaaac atggggaaga tgtagtgctg ttggtgatgc cggcagatca tcttattggt 360

aatcaaaaag ttttttcaaa agcagtgact gttgcgacag atatggcggt gaatggctgg 420

atagtcacgt ttggtgtaga gccaagcacg cctgaaacag gttatggcta cattgaaggc 480

ggagagtcaa ttggggactc aggcgcgcgt agagtcgttc gatttgtcga aaagccagat 540

ttggaaactg ctgagaaata tattgcttta ggtcgattta catggaatgc aggtatattc 600

tgttttactg caaaaaccat tattgctgca cttgcggagt atgttccaga agtgcttgag 660

atagcaaaaa agaccctggc atctagtgac tccaaaactg agcctgttgt attgggcgag 720

gagcactttg catcaagtcc agatatttcc atagattatg ccgtcatgga gcgtgcttca 780

aaagtggcgg tagtcgcagc tgattttgac tggagcgaca ttggttcatg gagcgccatg 840

gctgagttgg tccagtcaga tgagcgtggg aatagggtga atggcgaagc tttattggtg 900

gatgctaata attgctatat ccaaagtgat tcccgtcttg tggctggtat aggtattgat 960

aacctgctgg tggtcgatac acccgatgcc ctgcttgtgg ctcatcactc cagagctcag 1020

gatgtcaaaa ccgttgtgaa gcaactcaag cttgagtccc atagtgcggc aaattaccat 1080

cgcaaggtac accgtccatg gggcacatac accgtattgg aagaaggcgc taattttaag 1140

atcaaatgca tcaaggtaaa gcctggtgcc agcctgagct tgcaaatgca ccatcatcgt 1200

agtgagcatt gggttgtagt aagcggtata gctaaagttg tcaacggcga gcgcgagctg 1260

ctcattaatg ccaatgagtc tacttacatt ccagcaggac acaaacaccg tcttgaaaat 1320

ccaggtttgc ttgacctggt gattattgaa gttcaatccg ggaatatttt gggcgaagac 1380

gatattgttc gttttcaaga tatctacgga cgcacttgat gatcagcatg ttaaaagcct 1440

tatggcttta tcgcggcttt gtgttgggca gcgtcaagcg cgagtttcaa tccaagtacc 1500

gtaattcgct gctaggcgca gtctggacgg tcttgaaccc cctcgcaatg atcgtagtgt 1560

acacagtgat tttctcgcag gtaatgaagg ctcgtttgcc tggcgtggac agtacttttg 1620

cgtttagcat tcatttatgc gctggcatac tcacctgggg cttgtttgct gagattgtcg 1680

ggcgctcaca gaccacgttc atcgataatg ccaacttgct caagaaactg agtttccccc 1740

gtctatgtct gccagtcatc gtcaccgcca ccgcgctagt taactttgcc attgtttttg 1800

ggttgttcac agcgttcttg ctgctgatcg gtatgttttc gggttggcct tatttggtgc 1860

tgatcccggt attgatactg atgatcgtct ttgctctagg cttgggtatt atattgggcg 1920

ttctcaatgt gtttttccgc gatgtaggtc agttctttgg cattgtgctt agtttctggt 1980

tttggcttac gccgattgtc tatccggtca gcattcttcc agaacgggta caagattata 2040

tgcggcttaa ccccatggcg cgcttgatgg aggctttcca aggaatattg gtacaagcaa 2100

aatggccgga ctggtacagt ctatggccag tgtttgcgct gtctatcgtg ttgtgcctgc 2160

tggggctgtc tttattccgc aaacatgcgg gcgatatggt ggatgaactt taatgggtac 2220

tattgttgtc accaacctgg gtaaggccta caagcaatac cccagtcgct ggtctcggtt 2280

gttggagtgg atagatctga ggcgtaagcc gcaccaccaa ttgaagtgga ttctgcaagg 2340

agtaagtttt catatccagc caggagagac ggaggcagct ttgccgtgac tgctgccaag 2400

cgttctaata cgagtattaa gcctaacctt gcagtgatca attggatgct tgagcaagaa 2460

gaccggatgg gattatctac cccaaggcct tatcgtgaat ttgaggagcg tgtatatcgt 2520

catcgcgatg atctcaggcg cttaattcat tcgttagtag ctgacggcaa aaaaatatat 2580

gggtatggag cttctgccaa aggtaatgta gtactacaat tttgtggctt tacttctgag 2640

gaaatccctg ccattgcaga agtaaacgaa gaaaaattcg gacgagtcac tccgggaacg 2700

catattccca ttatttcgga aaaagaggcg cgcgctgcca agcccgacta cttcttggtg 2760

cttccatggc atttcaaaga tggtatcctg cgccgtgaaa aggactttct agctgatggc 2820

ggcaggttta tcttcccatt tcctgagatc gaaatcgtct agtttccaaa aaaagatgaa 2880

ggaaaataat aagataattt agacgctgtt ctaacgaatc cattatttac ataccatata 2940

tacatataca gtaagacttt aaatacataa ggcataatac gatgaccata aaaactgcca 3000

tcattaccgg catcacaggc caagatggtg catatctcgc agaacttctc cttagtaaag 3060

gctataaggt ttacggtacc ttccgccgta caagctcggt caacttctgg cgtattgaag 3120

agctgggaat tgaaaatgac ccaaatcttc ttttggttga atacgacctg accgatttgt 3180

cagcaagcat ccgtctattg cagagcacag gtgcaacaga agtgtacaac ttagctgcgc 3240

aaagttttgt gggcgtgtct tttgagcaac ctattactac cgcggagatc acaggtgtcg 3300

gtgcgctcaa tcttttggag gccattcgtt tagtaaatcc caatattcgc tattaccaag 3360

catccacatc cgaaatgttc ggcaaggtgc aggcgattcc acaaacggaa gatacgcctt 3420

tctatccacg cagtccttat ggtgtcgcca agctgtatgc gcactggatg actgttaact 3480

atcgtgaaag ctatggaatc ttcggatcta gcggtatttt attcaatcat gagagtccgt 3540

tgcgtggtcg cgagttcgtg acgcgcaaga tcacggattc ggtggccaag atcaagctgg 3600

ggaaacttga ttgcctggag ttgggtaact tggatgcaaa gcgtgattgg ggctatgcca 3660

aggattatgt ggaaggcatg tggcgcatcc tgcaggcgga tgagccggat acctttgtac 3720

tggcgaccaa tcgtaccgag actgtgcgcg attttgtaag catggcgttc aagggggctg 3780

gcattgctgt tgagttcaag ggcaagggcg aggacgaagt tgccgtggat actgcaaccg 3840

gtaagatagt ggttcgggtc aatccgaagt tttaccgtcc agcggaagtg gacctactga 3900

tcggtagccc agccaaggcc aaggccaagc tgggttggga accgaagaca acgctggagc 3960

aactctgcca gatgatggtc gaggccgatc tgcgccgcaa cacggaaggt ttctcatttt 4020

gagaattctg cttactggtg ctgatggatt caccgggcgc cattttctga cggccgcgaa 4080

agcggccggc catgaggttg ttgcactgag ggccgatttg acggacaagg ctgtattgca 4140

acgcgaagtt gaggcggctc gtccggaagc agtggttcat ctggcagcga tcagttttgt 4200

tggtcatgcg gatgttagtg ccttttatga cgtcaatgtg atcggtaccc tgaacctgtt 4260

ggatgcgctg gctggcttgt ccgtgacgcc gcgcagcgta ctgctggcaa gcagtgcgaa 4320

tgtgtatggc aatgcagcgc attcacctat caccgaagag caggcgccgg cgccggtcaa 4380

tcactatgca atgagcaagc tggccatgga gcacatggcc cgtacctatc tggacagact 4440

gccgttgttt tttgtgcggc cgttcaatta cacggggccg gggcaggctg agtctttcgt 4500

gattcccaag ctggtggcgc atttcgcgtg caaggcgcag accgtggaac tgggcaatct 4560

ggatgtggag cgcgagttca atgatgtggc tttcgtttgc gaggcctatc tgcgtttgtt 4620

ggacaaggct gtgccgggtg aggtgtttaa tatctgcagc ggtgctccgg tgacattgcg 4680

tgcagtaatc gggatgttga gggaattgac ggcgcatgat atcgaggtca aggtaaatcc 4740

gcagttcgtg cggaataacg agatcaagac attatgcggt gatccatcaa agctgttccg 4800

tactattgga gaggggaggc tggttccttt agcgcttacc ttgaagatca tgctggaaca 4860

cgctacgcga aaagaaaaaa aatgatgaag gtaggttttg gtgtcaccgt gcttacacgt 4920

tgcctggcat tggggggaat cgatggaatt ggtagttata cccgggagct gatgaaaaga 4980

ctcgaggctg acccggccgt cgagcttttc cctatctcgt acgggtattc tccgtcacag 5040

tttcctatcg gctcacgtgc tccgacgaca tttggacgat atgcgccgct tgctgcagta 5100

tcggctgtat ccgggtttcc ctttctggga acagctcaat tgaaggagcg tgttgatctg 5160

attcatgcgc ctgatcatct tattcccaac tacggaaaag ttcctgttat tgcatccatc 5220

atggacgcga ttcccttgtc gcatccggag tgggtatccc ccaagttgcg caagatgaaa 5280

aatgcgttat ggaagcgctc tgcgcactgg gcatcgcata tcattactat ctctgagtat 5340

tcgaagcagg aaatttcaca gcatttcggt attgaggcac aaaagatcag tgccatcccg 5400

cttggcgtgg atgagcggtg gtttcagccg ttggaagaag aggcgatgaa ggtggtattg 5460

agacggtaca atctgcctca atactatttt cttgtcgtgg ggacgctgca gccaaggaag 5520

aatgtcggcc gggtaatcga ggcatatcgg tccttgcccc aagggatacg taacgaagtg 5580

ccgttggtga ttgttggtag ggcagggtgg cgatgcgagg atgtggttga aatgttgact 5640

tcgtcttctt caggaaattc tctgaggtgg ctcaagcatt tgccggatgc cgacttgctt 5700

gcagtgttga aaggtgcaac ggctttggtc tttccttcgt tgtatgaggg atttggtttg 5760

cccgtagtcg aagcttttgc ggctggaact cccgttatta cttccaatac cacttctctt 5820

ccagaggtgg cgggcgatgc cgccatgctg gtgaatcctc tgaatgtcag cgacattgcc 5880

gctgcaatgc aatcgatgct ggaacagccg gaactggcag agagtttcag gaacaagggg 5940

caagaacgcg cattggcata ttcgtgggac aaaacagcct ccatgacgct tgacgtctat 6000

aaaaagacgc gagaggctgc ttaacatcaa cgcaccgtca aacatcgcac tccttccctt 6060

tttatgatca tccgttttct gggcgcacag tggctcgccg ttggttttgt cggggtcatc 6120

tctctggcgg tcagcatttt gattgcgcgg acgcttgggc cggattcatt tggtgtttat 6180

tcggttgtct tgtccggcgg agccattgcc gccattctgc ttgatggtgg tttcgggcga 6240

ttgttgcaaa gagaacgcgc ccatcccaca cctgcattgc tcagattcct gcccatattg 6300

ccgcaggctg ccttggggca tgtgattatc gcggccagca tattttcgtt gatcgcttta 6360

ttggtactgc ccaagcatgc gctgacgatt gttgcggtta tctggttttt cgcagcatct 6420

gtgaccaatc agttcggttt gaacattctc cgcggagatg ggcggatggt aagggatgcc 6480

tcgtggcaga tgggcaatcg aaccttcaca gctgcgtgcg tgtgcattgt cattctgatg 6540

ggagctgaga aaccgtggca ggtattcttt gcccagtttg tcggtgcgac cgccttcggt 6600

ttgctgatca tgcactatct gcgcacgcgc ccgctatgga agattcctcc tactctttat 6660

gcggcaacct tgccattcgt gtggttggat ctcgcgacag tcctttattt tcgagcggac 6720

atgctgttgc tggaatggtt agatgtgtcc aagtttgaag tcgggaaata cggggttgcc 6780

tgccgcttga ttgaggcggt gattctgctt gcttcgccgc tctgtattat tttgttccgc 6840

aagtttcgtc tgggtagcgc ttctccccga ctgatgttgc ggagaatgct gccggtgctg 6900

gcaggggcgc tgatggttgg agtgttcttc atggtgtttt tctggttgtg cagcacgcct 6960

ttgatcgcgg ttgcatatgg cgaggcctat gcgggagctg atcatattct ggcgatactc 7020

ggctgcgctt tgctttttat tttgccaaat ggcgtgctga accaagctgc gctagcgctg 7080

ggtatcgaac gttggctggc aatgtcggca tcttttgccg cagtgctaaa tattgcagga 7140

aatctctggc tgattcccca atatgggttg attggagctg cctggatgac tgttgtgacc 7200

gaagcggtgt taggctgctg cgttgcgtta gggctatggc gccaccgtaa taggagtgtt 7260

taatgcagga tcttgccgtt gctcatgttt atcgtaccta ttttcctgac cctccaggcg 7320

ggttgcagga ggcaattcgc cagattgccc tcacgacggg tatgcagggc gtcgataata 7380

cggtgtttac gctttctccg cagccgcgtc cggcaatctt gcagcgtccg gaggcttgtg 7440

ttgttcgttg ccgctcctgg gcggctccgg catcatgcga tctgggcggt atcggcgcat 7500

ttcttgcgtt ttccaggttg gcggaaaaat ctgatgtgct acattacctt tttccgtggc 7560

catttgcgga tgttctgcat acggtggttc gcccaggttg tccggccgtc atgacttata 7620

tctcggacgt ggtgcgtcag cagcttttag ggaggttata tgctcctttg atgtggaagg 7680

tgttgcgcca gatgaggctt gtggtggcga attctccagc gtatgcacgc accagtcccg 7740

ttctctccca tcctgagatt cgcgaaaaag tcagggttat tccacttggt attgaggagt 7800

cttcttatcc taaatcaggg gatgaggaga ttctgcagcg tctaggcatt gggaaggatg 7860

agccttattt tctttttatt ggcgttcttc gttattacaa aggcgcgcat tttctggtca 7920

aggcagcaaa gcggataggc gcaaaggtgg tgtttgctgg cgcggggccg gaaggcgcgc 7980

gcctgaaagc attggctggc gaagtcggtg cagagaatgt gatttttgcc gggatggtga 8040

ctaacgcgga aaaagtaacc ttgcttcaat gctgcagagc gcttgtgttg ccatcgcatt 8100

tgcggtctga agcgtacggc atggtgctgg tagaggctgc catgttcggg aagccgctga 8160

tttcttgcga aattggcaca ggcacttcat atgtcaacca tcatgaagaa acagggtttg 8220

tcgttgagcc ggcctctcct gaagcattgg cgtcggcgat ggcggttttg ttatcagagg 8280

ataggctggc tacagagatg ggagctgctg ctcgtcagcg gtatgagctg ctcttttccg 8340

gcccggcatt aggaaaggca tatgctgcat tgttccgcga agttgacgct tcgtagcaga 8400

gtgcgtcatg atgaagtagc cagagttgta acgtttttgg gattatcctg ttggttttct 8460

tgattgttaa tacatgatgc aattactggt gacaggcgct acagggtttg taggtaaaac 8520

tttgtgcagt gcgctaaggc ggcagagctt ccccgtaaaa gcaattgggc gtgcgggcac 8580

tatccgggct gacgctttgg gtgatcaggt tgcttcaatt gacggtcaaa cggattggtc 8640

actgatattg gacggcatca atgccgtcat ccatctggca gccagggtgc acgtcatgcg 8700

ggaacagctt gctgatccct tggcggcata tcgtgaaacg aatgtcgaag ggacgctgaa 8760

tctggcgcgt caggcggcaa aggccggagt taagcggttt gtattcatta gttccatcaa 8820

ggtcaacggc gaacagactc tacctggaaa gccttttact gcgtacgatg taccagctcc 8880

cgaagatgcg tacggtattt caaagttcga ggcagagcag ggtttgctgg ccctggcgcg 8940

ggagacaggg atggatgtgg tggttattcg ccctcctttg gtttacggcc ctggagtgaa 9000

gggtaatttt gccagtatga tgaagttggt gaaaagtgga ataccgttgc ctctgggcgc 9060

cattcataac caacggtctt tggtggcgat tgataatttg gtgaacctca tcgtcacgtg 9120

tattgaccat cccgcggcag ccaaccaggt ttttttggta agcgatggcg aggatttgtc 9180

cacttcagat ttgttgcggc gtttggccaa ggctgcgggc gtttcctccc gattgatgcc 9240

agtgtctgcc cgctggctag agatgggttt gcgtatgtgt ggcaagcgat tgatcgccca 9300

gcgtctcctg ggatcattgc aagtcgatat ttccaagacg cgcgagttgt tggggtgggt 9360

tccgccgatc tcagtggatg agggcttgaa gcgctgtttc tctaccaagg gctgattctt 9420

gatccgattt tttgacatta ttttttcggt gataggcttg gtactgggat tcccgctatt 9480

gctatcgatt tataccctcg gcttgtttga tacaggtgct cccttgtttc gccaggtgag 9540

ggttgggcgc taccggcagc cgtttacttt ggtgaaattt cggacgatga aggtggatac 9600

ggcttctgtg gcgagccatc tggcttccac caattccatc acccggctcg gacggttctt 9660

gcgcaggacc aagctggatg agctgcccca gctctggaat gtcctgaagg gagaaatgag 9720

cctggtgggg cctcgaccga acctgttcaa tcaggaggag ctgatggccg agcgcgagtt 9780

gcgtggcgtt taccatgcgc ggccagggat taccggcctt gcacaggtga atgatataga 9840

catgtcgacg ccaaggctgc ttgccgagac cgatgccagg atgctgcgcg agatgaatgt 9900

acggctttat ttcaagttgc tgattcagac ggtgctgggt cgaggacagg gcgaccgggt 9960

cgtgcgctga gctcggcagg aaccaatgcc tggctacaaa gtgttgatcc atgattggtc 10020

gtcactgtcc cgtggttttt agtagaatgt tattcatttt cactttaggc tgggcgcgtg 10080

ctgcattatt tcccctcatt caagtggtcg aactacatca gtttgaagaa tccacgctct 10140

ttgagcgctt ggatatatga cattgttgct gctgcgctgg catggtgggc agccttcctg 10200

ttacggttca attttgatat tccggccaat tttgtccaga gtatgctcga cagcgcagca 10260

tgggtgattc cgctgcaggc atttgtgttt ctgctggctg ggttgtacaa aggcgtctgg 10320

cgttttgcca gcctgccgga tttgcagcgc ttggtgcgcg cgattgcgat cagcgcagtg 10380

cttgtggttg cgatcttgtt catgttcaag ccacacggca ttgttcctcg ctcggtgctg 10440

atcttggatc ctatgctgtt gctgctgttg atgggcggca gccgctttgc ttaccgagca 10500

tggaaagaac atcgcctgta tggttctgcc cagttttccg gaaagccggt gctggtgctc 10560

ggcgcgggtg aggcagcgat tgccttgttg aaggatcttt ccaaaagccg tgaatggcat 10620

gtcgttgggt tgctggacga taagaaaacc atgcatggcc gagagctact gggcgtcaat 10680

gtgctggggt cgattgcgca attgccacag atcgctgcgg atttgtcggt caatcatgtg 10740

attgtggcca tgccatccgc gcgtcatcaa cgacgcaggg aaatcgtcga gctggcaagc 10800

agaaatgggc tgactgcgct cacggtgccg tcgtttgatg acctgatgag tggccacctg 10860

agcatggcac agattcggcc ggtcgaggtg gaggacttgc tggggcggga gccggtcagt 10920

ctcgatggag aggggctcca gcatttgatc gaggggcagg tgttgctgat tactggtgca 10980

ggtggttcca tcggttctga gttatgccgc caggtgctga aataccgtcc agcttgcctg 11040

gtctgcctgg atcagtcgga gtacgcgttg tatcggttgg agcaggagtt tgacaatctg 11100

gagtttgata cccgcattga atacgtggtt ggcgatgtca ggaacgcacg ccgggtgggg 11160

caggtgcttg gccgttatat gcccaaactg gtgttccatg ctgctgcgta taagcatgtg 11220

cctttgatgg agaaccgcaa tgtggcggaa tcattgtcaa ataatgtcag cggtacttac 11280

acgctcgccc aggcatgcaa aactgcaggg gttgccaagt tcgtgctgat ttccaccgat 11340

aaggcggtta atcccaccaa tgtgatgggg gccagcaagc gcctggcgga aatgatatgc 11400

caaggcctgc aggaggagtc gggtacgcgt tttatcgtgg tgcgctttgg caatgtgctg 11460

ggcagtagtg gcagtgtaat ccccaagttc cgtgagcaga ttgcggcggg cgggccaatt 11520

accgttaccc atcccgagat cactcgctat ttcatgtcca tcccggaggc gtgccagctg 11580

gtcatgcagg ctggattgat gggtggtggc ggggagatat ttgtgctgga tatgggagac 11640

cctgtgcgga ttgtggactt ggcccgtgat atgatccggt tatccgggct taatgaagat 11700

gacattcaga ttgtatactc tggcttgcgg cccggcgaaa agctgtacga ggaacttttg 11760

gctgacgacg agctcactgt accaacaccc cacgacaagc tacgtattgc cgtggcgcga 11820

agcgtggatg gtgcttgggt tcaggcattg ctgaaatgga tcgacagggc atcgctcata 11880

gatgaatctg tcatcaagca agagctgggc atgtgggtgg aggagtatgc aggggatatt 11940

catgccaatc catccatgat gttccccccc gagtcttcaa ccatacatta gaagaaagat 12000

agatagaaat ggcaaaaaaa attaccaagg cagtatttcc agtggcaggt ttagggacac 12060

gtttcctgcc agcaaccaag gcgagtccca aggaaatgtt gccaattgtg gataagcctc 12120

tgattcagta tgcagcagag gaggctgtgg ccgccggtgc taccgagctg atttttatta 12180

tcgggcgcaa caagcgcacc atcccggatc attttgataa gtccttcgag ctggaagcga 12240

cgcttgaggc aagcaagaaa accaaggtgc tggaagaaat ccgcaatatc ctgccaaaaa 12300

atgtttcctg catctatatt cgccaggccg aggcgcttgg tttggggcat gcggtattgc 12360

ttgccaagtc cgtggtcggc agcgagcctt tctctgtgat cctggccgat gaccttattg 12420

atgccgacaa acctgccatg cagcagatga cagagctgtt cgaccaagag ggtgtcagca 12480

ttctgggtgt cgaggatgtg gagcccagtg agacggcaag ctatggcatt gtggatgcga 12540

cagaggtgag tggcccgaat gtgttgcaat tgaatggcat tgtggaaaaa ccgcagccgg 12600

aagaagcgcc ctccaatctg gctgttgtcg gtcggtatat tctcacgcct gaaatttttg 12660

aatgcctgga gaacgtcaag ccgggcaagg gcggggaaat ccagttgact gatggcattg 12720

ccgccttgat ggaaaaacag aaaatcctgg cttaccgttt cgatgggaag cgctacgatt 12780

gcggcagcaa gctgggttat gtcaaggcga acgtcgagta ttccttgaag catgaggagt 12840

ttggcaaaga gtttgcccag tacttacgtg atctaaccaa gtagcagtcg tggtggtact 12900

tgagggtaac cccgatctca ttctgcagcg atccacgctg gtctcttctg aagaggaagt 12960

gaagcaggcg gtgacgaggg tggcgcagga aatcactgcc acgctaaggc aggaccaacc 13020

tctcgtcctg tgtgtcatgg gaggcggggt ggtattcacc gggcaattat tgccacagct 13080

ggttttccct ttggagttcg gttatgtgca ggccacccgc tatcacaacc ggacacatgg 13140

cgatgcccag ctggtgtgga aaatgaagcc cggggaggag gtgcgcggcc gtaccgtttt 13200

attgctcgat gatattctcg atgagggggt cacgctggca gaaatcaagg cgcagtgtct 13260

ggcactgggt gccagcaagg ttgtcgttgc ggtcttggtt gaaaaaatgc tggggcgggt 13320

caagccgatc agggctgatt tcgtcggact ggaggtgccg gaccagtatg tattcggatg 13380

cggaatggat gcctatgggt ggtggcgtaa tttgccggcc atttatgcca tgcaagggga 13440

agcttga 13447

## Description

### Technical field of the invention

The present invention generally relates to the biotechnology engineering, and specifically to genetically modified bacteria of the genus *Methylobacillus* which have improved properties making them particularly useful in large scale methanol fermentations. More specifically, the present invention provides a bacterium of the genus *Methylobacillus* which has been modified to have a decreased production of exopolysaccharides (EPS) compared to an otherwise identical bacterium that does not carry said modification. The present invention further provides a method for producing a biochemical compound using a genetically modified bacterium of the present invention.

### Background of the invention

Methanol fermentation has the potential to replace oil-derived chemical production. To realize this, efficient methylotrophic (consuming one-carbon compounds such as methanol or methane or multi-carbon compounds that contain no carbon-carbon bonds) strains must be developed. Several bacteria capable of utilizing methanol as the sole source of carbon for growth and product formation have been identified. Some examples include *Bacillus methanolicus, Methylobacterium extorquens, Methylobacillus glycogenes* and *Methylobacillus flagellatus.* Methylotrophic bacteria can be divided into two broad groups based on their methanol assimilation pathways. The first step is always the conversion of methanol into formaldehyde, catalyzed by a methanol dehydrogenase enzyme. In the first group, formaldehyde reacts with Ribulose-monophosphate (RuMP) to form C6-compounds that are then metabolized further. Members of this group are commonly referred to as "RuMP cycle methylotrophs". The second group, which includes M. extorquens, assimilate formaldehyde by reacting it with glycine, forming serine in the pathway known as the serine cycle. Both groups of methylotrophic bacteria have been used for the production of various biochemicals from methanol. However, the RuMP cycle is more energy efficient than the serine cycle, resulting in faster growth and biomass formation.

Methylotrophic microorganisms can be found in extremely diverse environments, such as oceans, soil, plant rhizosphere and even sewage water. Since they must be capable of surviving these diverse and often harsh environments, methylotrophic bacteria often tend to be quite robust and resistant to different types of stress, all while growing efficiently on minimal nutrients. Strains from the RuMP cycle-utilizing Methylobacillus genus possess a number of traits desired in biochemical productions trains; they grow rapidly and reproducibly in minimal mineral media and they are insensitive to fluctuations in temperature and methanol concentration. Furthermore, they are very amenable to genetic manipulation, unlocking the potential for metabolic engineering of complex pathways.

Many methylotrophs, including *Methylobacilli* produce large amounts of exopolysaccharides (EPS), which confer multiple advantages to the organisms in their natural environment, such as protection from dessication, biofilm scaffold formation, energy reserves etc. In fed-batch fermentations, Mehtylobacillus biomass can consist of up to 30% EPS.

EPS production in Methylobacilli specifically is relatively unstudied. Research has only been performed on a single strain, Methylobacillus sp. strain 12S, an obligate methylotroph isolated from soil, which was found to synthesize a novel EPS termed methanolan (Yoshida et al., 2000). While the gene cluster responsible for methanolan synthesis in *Methylobacillus* sp. strain 12S has been identified and characterized (Yoshida et al., 2003), no such work has been done in other *Methylobacilli.* Based on bioinformatic analysis and sequence similarities, an EPS gene cluster has been predicted in *Methylobacillus flagellatus* strain KT (Chistoserdova et al., 2007) and *Methylobacillus glycogenes* (Hattori et al., 2020), though the genes themselves have never been characterized.

### Summary of the invention

The object of the present invention is to overcome certain drawbacks in methanol-based bioprocesses. This is achieved by the present inventors who have engineered different genetically modified bacteria of the genus *Methylobacillus* having a decreased production of exopolysaccharides (EPS).

The present inventors studied general EPS production in *Methylobacilli.* Based on bioinformatic analysis of the genome of *M. flagellatus* and *M. glycogenes*, the present inventors identified two putative EPS-producing gene clusters. Disruption of both EPS clusters resulted in complete abolishment of EPS production.

An unexpected and surprising observation in the modified *M. flagellatus* and *M. glycogenes* strains was their behavior in growth cultures. When abolishing the expression of one or more endogenous polypeptides involved in the production of exopolysaccharides (EPS) in the *Methylobacillus* bacteria, the culture showed a number of surprising and unexpected properties beneficial to the development of methanol-based bioprocesses. Unexpectedly, the excessive culture foaming in both shake flasks and bioreactors was reduced. Moreover, while cell clumping was observed for unmodified parent strains, this was reduced or even absent in the culture of modified *Methylobacilli.* Centrifugation and filtration of the biomass was also improved when EPS production was eliminated. These combined characteristics significantly improve the bioprocess, strain handling, and downstream processing, making modified Methylobacillus bacteria suitable for large-scale methanol fermentations. Removal of EPS synthesis unexpectedly also improved the methanol tolerance of *M. flagellatus* and other *Methylobacillus species.*

Based on the foregoing finds, the present invention provides in a first aspect a genetically engineered bacterium of the genus *Methylobacillus* which has been modified to have a decreased production of exopolysaccharides (EPS) compared to an otherwise identical bacterium that does not carry said modification.

The present invention further provides a method for the production of a biochemical compound comprising cultivating a bacterium of the present invention under suitable culture conditions in a culture medium comprising a reduced one-carbon compound, such as methanol, or a multi-carbon compound that contains no carbon-carbon bonds, such as dimethylamine.

The present invention may be summarized by the following items:
1. A genetically engineered bacterium of the genus *Methylobacillus* which has been modified to have a decreased production of exopolysaccharides (EPS) compared to an otherwise identical bacterium that does not carry said modification.
2. The bacterium according to item 1, which has been modified to have a decreased expression and/or function (e.g. activity) of at least one endogenous polypeptide involved in the production of exopolysaccharides (EPS) in said bacterium compared to an otherwise identical bacterium that does not carry said modification (reference bacterium).
3. The bacterium according to item 1 or 2, which has been modified to have a decreased expression of at least one endogenous polypeptide involved in the production of exopolysaccharides (EPS) in said bacterium compared to an otherwise identical bacterium that does not carry said modification (reference bacterium).
4. The bacterium according to any one of items 1 to 3, which has been modified to have a decrease expression of at least two endogenous polypeptides involved in the production of exopolysaccharides (EPS) in said bacterium compared to an otherwise identical bacterium that does not carry said modification (reference bacterium).
5. The bacterium according to any one of items 1 to 4, which has been modified to have a decrease expression of at least three endogenous polypeptides involved in the production of exopolysaccharides (EPS) in said bacterium compared to an otherwise identical bacterium that does not carry said modification (reference bacterium).
6. The bacterium according to any one of items 1 to 5, which has been modified to have a decrease expression of at least four endogenous polypeptides involved in the production of exopolysaccharides (EPS) in said bacterium compared to an otherwise identical bacterium that does not carry said modification (reference bacterium).
7. The bacterium according to any one of items 1 to 6, which has been modified to have a decrease expression of at least five endogenous polypeptides involved in the production of exopolysaccharides (EPS) in said bacterium compared to an otherwise identical bacterium that does not carry said modification (reference bacterium).
8. The bacterium according to any one of items 1 to 7, which has been modified to have a decrease expression of at least six endogenous polypeptides involved in the production of exopolysaccharides (EPS) in said bacterium compared to an otherwise identical bacterium that does not carry said modification (reference bacterium).
9. The bacterium according to any one of items 1 to 8, which has been modified to have a decrease expression of all endogenous polypeptides involved in the production of exopolysaccharides (EPS) in said bacterium compared to an otherwise identical bacterium that does not carry said modification (reference bacterium).
10. The bacterium according to any one of items 2 to 9, wherein the at least one polypeptide involved in the production of exopolysaccharides (EPS) in said bacterium is selected from the group consisting of a) polypeptides comprising an amino acid sequence set forth in any one of SEQ ID NO: 1 to 38 and b) polypeptides comprising an amino acid sequence having at least 70% sequence identity with any one of SEQ ID NO: 1 to 38 and having the same functional property as the reference polypeptide.
11. The bacterium according to any one of items 2 to 10, wherein the at least one polypeptide involved in the production of exopolysaccharides (EPS) in said bacterium is selected from the group consisting of a) polypeptides comprising an amino acid sequence set forth in any one of SEQ ID NO: 1 to 26 and b) polypeptides comprising an amino acid sequence having at least 70% sequence identity with any one of SEQ ID NO: 1 to 26 and having the same functional property as the reference polypeptide.
12. The bacterium according to any one of items 2 to 10, wherein the at least one polypeptide involved in the production of exopolysaccharides (EPS) in said bacterium is selected from the group consisting of a) polypeptides comprising an amino acid sequence set forth in any one of SEQ ID NO: 27 to 38 and b) polypeptides comprising an amino acid sequence having at least 70% sequence identity with any one of SEQ ID NO: 27 to 38 and having the same functional property as the reference polypeptide.
13. The bacterium according to any one of items 2 to 12, wherein the at least one polypeptide involved in the production of exopolysaccharides (EPS) in said bacterium is selected from the group consisting of a) polypeptides comprising an amino acid sequence set forth in any one of SEQ ID NO: 39 to 86 and b) polypeptides comprising an amino acid sequence having at least 70% sequence identity with any one of SEQ ID NO: 39 to 86 and having the same functional property as the reference polypeptide.
14. The bacterium according to any one of items 2 to 13, wherein the at least one polypeptide involved in the production of exopolysaccharides (EPS) in said bacterium is selected from the group consisting of a) polypeptides comprising an amino acid sequence set forth in any one of SEQ ID NO: 39 to 60 and b) polypeptides comprising an amino acid sequence having at least 70% sequence identity with any one of SEQ ID NO: 39 to 60 and having the same functional property as the reference polypeptide.
15. The bacterium according to any one of items 2 to 13, wherein the at least one polypeptide involved in the production of exopolysaccharides (EPS) in said bacterium is selected from the group consisting of a) polypeptides comprising an amino acid sequence set forth in any one of SEQ ID NO: 61 to 86 and b) polypeptides comprising an amino acid sequence having at least 70% sequence identity with any one of SEQ ID NO: 61 to 86 and having the same functional property as the reference polypeptide.
16. The bacterium according to any one of items 2 to 15, wherein the at least one polypeptide involved in the production of exopolysaccharides (EPS) in said bacterium is selected from the group consisting a polypeptide having peptidyl-prolyl cis-trans isomerase activity, a polypeptide capable of acting as a polysaccharide exporter; a polypeptide acting as a chain length determinant protein; and a polypeptide having protein-tyrosine kinase activity.
17. The bacterium according to item 16, wherein the polypeptide having peptidyl-prolyl cis-trans isomerase activity comprises an amino acid sequence having at least 70% sequence identity to SEQ ID NO: 6, the polypeptide capable of acting as a polysaccharide exporter comprises an amino acid sequence having at least 70% sequence identity to SEQ ID NO: 7, the polypeptide acting as a chain length determinant protein comprises an amino acid sequence having at least 70% sequence identity to SEQ ID NO: 8 and the polypeptide having protein-tyrosine kinase activity comprises an amino acid sequence having at least 70% sequence identity to SEQ ID NO: 9.
18. The bacterium according to item 16, wherein the polypeptide having peptidyl-prolyl cis-trans isomerase activity comprises an amino acid sequence having at least 70% sequence identity to SEQ ID NO: 42, the polypeptide capable of acting as a polysaccharide exporter comprises an amino acid sequence having at least 70% sequence identity to SEQ ID NO: 43, the polypeptide acting as a chain length determinant protein comprises an amino acid sequence having at least 70% sequence identity to SEQ ID NO: 44 and the polypeptide having protein-tyrosine kinase activity comprises an amino acid sequence having at least 70% sequence identity to SEQ ID NO: 45.
19. The bacterium according to any one of items 2 to 18, wherein the expression of the at least one polypeptide involved in the production of exopolysaccharides (EPS) in said bacterium is decreased by at least 50%, such as by at least 60%, at least 65%, at least 70%, at least 75%, at least 80%, at least 85%, at least 90%, at least 95% or at least 100% compared to the otherwise identical bacterium.
20. The bacterium according to any one of items 2 to 19, wherein the expression of the at least one polypeptide involved in the production of exopolysaccharides (EPS) in said bacterium is abolished compared to the otherwise identical bacterium.
21. The bacterium according to any one of items 2 to 20, wherein the endogenous gene/s encoding said polypeptide/s involved in the production of exopolysaccharides (EPS) in said bacterium has/have been inactivated.
22. The bacterium according to any one of items 2 to 21, wherein the endogenous gene/s encoding said polypeptides/s involved in the production of exopolysaccharides (EPS) in said bacterium has/have been inactivated by deletion of part of or the entire gene sequence.
23. The bacterium according to any one of items 2 to 22, wherein the endogenous gene/s encoding said polypeptide/s involved in the production of exopolysaccharides (EPS) in said bacterium has/have been inactivated by introducing or expressing in the bacterium a respective rare-cutting endonuclease able to selectively inactivating by DNA cleavage the endogenous gene encoding said polypeptide.
24. The bacterium according to item 23, wherein said rare-cutting endonucleases are selected from the group consisting of a transcription activator-like effector (TALE) nuclease, meganuclease, zing-finger nuclease (ZFN) and RNA-guided endonuclease.
25. The bacterium according to item 24, wherein the RNA-guided endonuclease is a catalytically inactive Cas9 protein.
26. The bacterium according to item 25, which comprises (e.g., expresses) at least one single guide RNA (sgRNA) specifically hybridizing (e.g. binding) under cellular conditions with the genomic DNA encoding said enzyme(s).
27. The bacterium according to any one of items 2 to 21, wherein the expression of the polypeptide(s) involved in the production of exopolysaccharides (EPS) in said bacterium is decreased (e.g., inhibited) by transcriptional and/or translational repression of the endogenous gene(s) encoding said polypeptide(s).
28. The bacterium according to any one of items 2 to 21, wherein the expression of the polypeptide(s) involved in the production of exopolysaccharides (EPS) in said bacterium is decreased (e.g., inhibited) by introducing or expressing in the bacterium at least one inhibitory nucleic acid molecule that specifically hybridizes (e.g. binds) under cellular conditions with cellular mRNA and/or genomic DNA encoding said polypeptide(s).
29. The bacterium according to item 28, wherein the inhibitory nucleic acid molecule is an antisense oligonucleotide, ribozyme or interfering RNA (RNAi) molecule.
30. The bacterium according to item 29, wherein the interfering RNA molecule is a micro RNA (miRNA), small interfering RNA (siRNA) or short hairpin RNA (shRNA).
31. The bacterium according to any one of items 2 to 30, wherein the endogenous polypeptide or polypeptides involved in the production of exopolysaccharides (EPS) in said bacterium is/are encoded by a gene or genes included in a first EPS gene cluster and/or second EPS gene cluster.
32. The bacterium according to item 31, wherein the first EPS gene cluster includes genes defined by or orthologous to the open reading frames (ORFs) found in SEQ ID NO: 174 or 177.
33. The bacterium according to item 31 or 32, wherein the first EPS gene cluster includes the genes *epsD, epsE, epsF, epsG, epsB, epsL, epsH, epsl, epsJ* and *epsS,* or orthologs thereof.
34. The bacterium according to any one of items 31 to 33, wherein the first EPS gene cluster includes the genes *epsD, epsE, epsF* and *epsG*, or orthologs thereof.
35. The bacterium according to any one of items 31 to 34, wherein the first EPS gene cluster includes a nucleotide sequence which has at least 50% sequence identity with the nucleotide sequence of SEQ ID NO: 174 or 177.
36. The bacterium according to any one of items 31 to 34, wherein the first EPS gene cluster includes a nucleotide sequence which has at least 50% sequence identity with the nucleotide sequence of SEQ ID NO: 174.
37. The bacterium according to any one of items 31 to 36, wherein at least one gene in said first EPS gene cluster is inactivated, e.g., by deletion of part of or the entire gene sequence.
38. The bacterium according to any one of items 31 to 37, wherein at least one gene selected from *epsD, epsE, epsF, epsG, epsB, epsL, epsH, epsl, epsJ* and *epsS,* or ortholog thereof, is inactivated, e.g., by deletion of part of or the entire gene sequence.
39. The bacterium according to any one of items 31 to 38, wherein at least one gene selected from *epsD, epsE, epsF* and *epsG,* or ortholog thereof, is inactivated, e.g., by deletion of part of or the entire gene sequence.
40. The bacterium according to any one of items 31 to 39, wherein the genes *epsD, epsE, epsF and epsG,* or orthologs thereof, are inactivated, e.g., by deletion of part of or the entire gene sequence.
41. The bacterium according to item 39 or 40, wherein the gene *epsD* encodes a polypeptide comprising an amino acid sequence having at least 70% sequence identity to SEQ ID NO: 6, wherein the gene *epsE* encodes a polypeptide comprising an amino acid sequence having at least 70% sequence identity to SEQ ID NO: 7, wherein the gene *epsF* encodes a polypeptide comprising an amino acid sequence having at least 70% sequence identity to SEQ ID NO: 8, and wherein the gene *epsG* encodes a polypeptide comprising an amino acid sequence having at least 70% sequence identity to SEQ ID NO: 9.
42. The bacterium according to item 41, wherein the gene *epsD* comprises a nucleotide sequence having at least 70% sequence identity with SEQ ID NO: 92, the gene *epsE* comprises a nucleotide sequence having at least 70% sequence identity with SEQ ID NO: 93, the gene *epsF* comprises an nucleotide sequence having at least 70% sequence identity with SEQ ID NO: 94, and the gene *epsG* comprises an nucleotide sequence having at least 70% sequence identity with SEQ ID NO: 95.
43. The bacterium according to item 39 or 40, wherein the gene *epsD* encodes a polypeptide comprising an amino acid sequence having at least 70% sequence identity to SEQ ID NO: 42, wherein the gene *epsE* encodes a polypeptide comprising an amino acid sequence having at least 70% sequence identity to SEQ ID NO: 43, wherein the gene *epsF* encodes a polypeptide comprising an amino acid sequence having at least 70% sequence identity to SEQ ID NO: 44, and wherein the gene *epsG* encodes a polypeptide comprising an amino acid sequence having at least 70% sequence identity to SEQ ID NO: 45.
44. The bacterium according to item 43, wherein the *epsD* gene comprises a nucleotide sequence having at least 70% sequence identity with SEQ ID NO: 128, the gene *epsE* comprises a nucleotide sequence having at least 70% sequence identity with SEQ ID NO: 129, the gene *epsF* comprises an nucleotide sequence having at least 70% sequence identity with SEQ ID NO: 130, and the gene *epsG* comprises an nucleotide sequence having at least 70% sequence identity with SEQ ID NO: 131.
45. The bacterium according to any one of items 31 to 44, wherein the first EPS gene cluster is inactivated.
46. The bacterium according to any one of items 31 to 45, wherein the first EPS gene cluster is inactivated by deletion of part of or the entire sequence of said cluster.
47. The bacterium according to any one of items 31 to 45, wherein the first EPS gene cluster is inactivated by modification of (e.g., by introducing at least one mutation in) the promoter and/or ribosome binding site region resulting in the lack of gene expression.
48. The bacterium according to any one of items 31 to 47, wherein the second EPS gene cluster includes genes defined by or orthologous to the open reading frames (ORFs) found in SEQ ID NO: 175 or 179.
49. The bacterium according to any one of items 31 to 48, wherein the second EPS gene cluster includes a nucleotide sequence which has at least 50% sequence identity with the nucleotide sequence of 175 or 179.
50. The bacterium according to any one of items 31 to 48, wherein the second EPS gene cluster includes a nucleotide sequence which has at least 50% sequence identity with the nucleotide sequence of 175.
51. The bacterium according to any one of items 31 to 50, wherein at least one gene in said second EPS gene cluster is inactivated, e.g., by deletion of part of or the entire gene sequence.
52. The bacterium according to any one of items 31 to 51, wherein the second EPS gene cluster is inactivated.
53. The bacterium according to any one of items 31 to 52, wherein the second EPS gene cluster is inactivated by deletion of part of or the entire sequence of said cluster.
54. The bacterium according to any one of items 31 to 52, wherein the second EPS gene cluster is inactivated by modification of (e.g., by introducing at least one mutation in) the promoter and/or ribosome binding site region resulting in the lack of gene expression.
55. The bacterium according to any one of 1 to 52, wherein all endogenous genes encoding enzymes involved in the production of exopolysaccharides (EPS) in said bacterium are inactivated, e.g., deleted.
56. The bacterium according to any one of items 1 to 55, wherein the bacterium is selected from *Methylobacillus flagellatus, Methylobacillus glycogenes, Methylobacillus pratensis, Methylobacillus rhizosphaerae, Methylobacillus gramineus, Methylobacillus arboreus, Methylobacillus caricics, Methylobacillus methilovorans and Methylobacillus sp..*
57. The bacterium according to any one of items 1 to 56, wherein the bacterium is selected from *Methylobacillus flagellatus* and *Methylobacillus glycogenes.*
58. The bacterium according to any one of items 1 to 55, wherein the bacterium is *Methylobacillus flagellatus.*
59. The bacterium according to any one of items 1 to 55, wherein the bacterium is *Methylobacillus glycogenes.*
60. A method for the production of a biochemical compound comprising cultivating a bacterium according to any one of items 1 to 59 under suitable culture conditions in a culture medium comprising a reduced one-carbon compound, such as methanol, or a multi-carbon compound that contains no carbon-carbon bonds, such as dimethylamine.
61. The method according to item 60, wherein the culture medium comprises methanol.
62. The method according to item 60 or 61, wherein the cultivation is performed in a bioreactor.
63. The method according to any one of items 60 to 62, wherein the biochemical compound is selected from organic acids, amino acids, fatty acids and derivatives thereof.

### Brief description of the figures

**Figure 1****:** Sugar content of hydrolyzed *M. flagellatus* shake-flask supernatant. Dark gray bars represent the total glucose in mg/L and the light gray bars represent total monomeric sugars. Error bars are the standard deviation of technical replicates.
**Figure 2****:** Measured viscosity of bioreactor broth produced by different strains of *Methylobacillus flagellatus.*
**Figure 3****:** Photo of wild type M. flagellatus and M. glycogenes cultures after 24 hours of incubation. Wild type ABME 5 and 6 on the left, followed by a single-cluster disruption in *M. flagellatus* and finally a double disruption.
**Figure 4****:** Plot of antifoam addition (y axis) during two representative bioreactor fermentations of ABME 6 and ABME 131 in relation to fermentation time (x axis).
**Figure 5****:** Phase-contrast microscopy images of bioreactor fermentation broth of wild type *Methylobacillus flagellatus* (left) vs the double-disruption strain ABME 131 (right).
**Figure 6****:** Improved growth of the EPS free ABME131 compared to the parent strain.

The present invention is now described in more detail below.

### Detailed description of the invention

Unless specifically defined herein, all technical and scientific terms used have the same meaning as commonly understood by a skilled artisan in the fields of biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory).

### Bacterium of the invention

As indicated above, the present invention is based on the unexpected and surprising finding that certain drawbacks in methanol-based bioprocesses can be overcome by down-regulating the production of exopolysaccharides (EPS) in bacteria of the genus *Methylobacillus.*

The present invention thus provides in a first aspect a genetically engineered bacterium of the genus *Methylobacillus* which has been modified to have a decreased production of exopolysaccharides (EPS) compared to an otherwise identical bacterium that does not carry said modification.

More specifically, the present invention provides a genetically engineered bacterium of the genus *Methylobacillus* which has been modified to have a decreased expression and/or activity of at least one endogenous polypeptide involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least one, such as at least two, endogenous polypeptide involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least three, such as at least four, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least five, such as at least six, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least seven, such as at least eight, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least nine, such as at least ten, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least eleven, such as at least twelve, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least thirteen, such as at least fourteen, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of at least fifteen, such as at least sixteen, endogenous polypeptides involved in the production of exopolysaccharides (EPS) in said bacterium compared to an otherwise identical bacterium that does not carry said modification (reference bacterium).

According to some embodiments, the genetically engineered bacterium of the invention has been modified to have a decreased expression of all endogenous polypeptides involved in the production of exopolysaccharides (EPS) in said bacterium compared to an otherwise identical bacterium that does not carry said modification (reference bacterium).

According to some embodiments, the at least one polypeptide involved in the production of exopolysaccharides (EPS) in said bacterium is selected from the group consisting of a) polypeptides comprising an amino acid sequence set forth in any one of SEQ ID NO: 1 to 38 and b) polypeptides comprising an amino acid sequence having at least 70%%, such as at least 75%, at least 80%, at least 85%, at least 90% or at least 95%, sequence identity with any one of SEQ ID NO: 1 to 38 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 1 to 26 and b) polypeptides comprising an amino acid sequence having at least 70%%, such as at least 75%, at least 80%, at least 85%, at least 90% or at least 95%, sequence identity with any one of SEQ ID NO: 1 to 26 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 27 to 38 and b) polypeptides comprising an amino acid sequence having at least 70%%, such as at least 75%, at least 80%, at least 85%, at least 90% or at least 95%, sequence identity with any one of SEQ ID NO: 27 to 38 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 39 to 86 and b) polypeptides comprising an amino acid sequence having at least 70%, such as at least 75%, at least 80%, at least 85%, at least 90% or at least 95%, sequence identity with any one of SEQ ID NO: 39 to 86 and having the same functional property as the reference polypeptide.

According to some embodiments, the the at least one polypeptide involved in the production of exopolysaccharides (EPS) in said bacterium is selected from the group consisting of a) polypeptides comprising an amino acid sequence set forth in any one of SEQ ID NO: 39 to 60 and b) polypeptides comprising an amino acid sequence having at least 70%, such as at least 75%, at least 80%, at least 85%, at least 90% or at least 95%, sequence identity with any one of SEQ ID NO: 39 to 60 and having the same functional property as the reference polypeptide.

According to some embodiments, the at least one enzyme involved in the production of exopolysaccharides (EPS) in said bacterium is selected from the group consisting of a) polypeptides comprising an amino acid sequence set forth in any one of SEQ ID NO: 61 to 86 and b) polypeptides comprising an amino acid sequence having at least 70%%, such as at least 75%, at least 80%, at least 85%, at least 90% or at least 95%, sequence identity with any one of SEQ ID NO: 61 to 86 and having the same functional property as the reference polypeptide.

According to some embodiments, the at least one polypeptide involved in the production of exopolysaccharides (EPS) in said bacterium is selected from the group consisting a polypeptide having peptidyl-prolyl cis-trans isomerase activity, a polypeptide capable of acting as a polysaccharide exporter; a polypeptide acting as a chain length determinant protein; and a polypeptide having protein-tyrosine kinase activity.

According to some embodiments, the polypeptide having peptidyl-prolyl cis-trans isomerase activity comprises an amino acid sequence having at least 70% sequence identity to SEQ ID NO: 6.

According to some embodiments, the polypeptide capable of acting as a polysaccharide exporter comprises an amino acid sequence having at least 70% sequence identity to SEQ ID NO: 7.

According to some embodiments, the polypeptide acting as a chain length determinant protein comprises an amino acid sequence having at least 70% sequence identity to SEQ ID NO: 8.

According to some embodiments, the polypeptide having protein-tyrosine kinase activity comprises an amino acid sequence having at least 70% sequence identity to SEQ ID NO: 9.

According to some embodiments, the polypeptide having peptidyl-prolyl cis-trans isomerase activity comprises an amino acid sequence having at least 70% sequence identity to SEQ ID NO: 42.

According to some embodiments, the polypeptide capable of acting as a polysaccharide exporter comprises an amino acid sequence having at least 70% sequence identity to SEQ ID NO: 43.

According to some embodiments, the polypeptide acting as a chain length determinant protein comprises an amino acid sequence having at least 70% sequence identity to SEQ ID NO: 44.

According to some embodiments, the polypeptide having protein-tyrosine kinase activity comprises an amino acid sequence having at least 70% sequence identity to SEQ ID NO: 45.

The expression level of the endogenous polypeptide(s) may, for example, be decreased by at least 50%, such as by at least 60%, at least 65%, at least 70%, at least 75%, at least 80%, at least 85%, at least 90%, at least 95% or at least 100% compared to the otherwise identical bacterium.

A decrease of the expression of the endogenous polypeptide(s) may be achieved by any suitable means known in the art. For example, the expression may be decrease by inactivating the endogenous gene(s) encoding said polypeptide(s) involved in the production of exopolysaccharides (EPS) in the bacterium, such as by deletion of part of or the entire gene sequence.

According to some embodiments, the expression of the at least one polypeptide involved in the production of exopolysaccharides (EPS) in said bacterium is abolished compared to the otherwise identical bacterium.

According to some embodiments, the endogenous gene/s encoding said polypeptide/s involved in the production of exopolysaccharides (EPS) in the bacterium has/have been inactivated, such as by deletion of part of or the entire gene sequence.

According to some embodiments, the endogenous gene/s encoding said polypeptide/s involved in the production of exopolysaccharides (EPS) in said bacterium has/have been inactivated by introducing or expressing in the bacterium a rare-cutting endonuclease able to selectively inactivating by DNA cleavage the endogenous gene encoding said enzyme. A rare-cutting endonuclease to be used in accordance of the present invention to inactivate the endogenous gene may, for instance, be a transcription activator-like effector (TALE) nuclease, meganuclease, zing-finger nuclease (ZFN), or RNA-guided endonuclease.

One way to inactivate an endogenous gene encoding a polypeptide involved in the production of exopolysaccharides (EPS) in the bacterium is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

According to some embodiments, the expression of an endogenous polypeptide involved in the production of exopolysaccharides (EPS) in the bacterium is decreased by way of inhibition.

Inhibition of the expression of said polypeptide may be achieved by any suitable means known in the art. For example, the expression may be inhibited by gene silencing techniques involving the use of inhibitory nucleic acid molecules, such as antisense oligonucleotides, ribozymes or interfering RNA (RNAi) molecules, such as microRNA (miRNA), small interfering RNA (siRNA) or short hairpin RNA (shRNA).

According to some embodiments, the expression of an endogenous polypeptide involved in the production of exopolysaccharides (EPS) in the bacterium is decreased (e.g., inhibited) by transcriptional and/or translational repression of the endogenous gene encoding said polypeptide.

According to some embodiments, the expression of an endogenous polypeptide involved in the production of exopolysaccharides (EPS) in the bacterium is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypeptide in question. Depending on the target, transcription of the encoding genomic DNA and/or translation of the encoding mRNA is/are inhibited.

According to some embodiments, the inhibitory nucleic acid molecule is an antisense oligonucleotide, ribozyme or interfering RNA (RNAi) molecule. Preferably, such nucleic acid molecule comprises at least 10 consecutive nucleotides of the complement of the cellular mRNA and/or genomic DNA encoding the polypeptide in question.

According to some embodiments, the inhibitory nucleic acid is an antisense oligonucleotide. Such antisense oligonucleotide is a nucleic acid molecule (either DNA or RNA) which specifically hybridizes (e.g. binds) under cellular conditions with the cellular mRNA and/or genomic DNA encoding the enzyme in question.

According to some embodiments, the inhibitory nucleic acid molecule is a ribozyme, such as a hammerhead ribozyme. A ribozyme molecule is designed to catalytically cleave the mRNA transcript to prevent translation of the polypeptide in question.

According to some embodiments, the inhibitory nucleic acid molecule is an interfering RNA (RNAi) molecule. RNA interference is a biological process in which RNA molecules inhibit expression, typically causing the destruction of specific mRNA. Exemplary types of RNAi molecules include microRNA (miRNA), small interfering RNA (siRNA) and short hairpin RNA (shRNA). According to some embodiments, the RNAi molecule is a miRNA. According to some embodiments, the RNAi molecule is a siRNA. According to some embodiments, the RNAi molecule is a shRNA.

According to some embodiments, bacterium of the invention has been modified to have a decreased function (e.g. activity) of at least one polypeptide involved in the production of exopolysaccharides (EPS) in the bacterium compared to an otherwise identical microorganism that does not carry said modification (reference bacterium).

A decrease of the function (e.g. activity) of the at least one endogenous polypeptide may be achieved by any suitable means known in the art. For example, if the polypeptide is an enzyme the activity may be decrease by introducing one or more mutations in the active site of the enzyme resulting in the reduction or loss of activity. Thus, according to some embodiments, the activity of the at least one endogenous enzyme involved in the production of exopolysaccharides (EPS) in the bacterium is decreased by at least one active-site mutation resulting in the reduction or loss of activity. The at least one active-site mutation may, for example, be at least one non-conservative amino acid substitution.

As noted above, the present inventors have identified two putative EPS gene clusters in the genome of *Methylobacilli.* The endogenous polypeptide or polypeptides involved in the production of exopolysaccharides (EPS) is/are thus encoded by a gene or genes included in a first EPS gene cluster and/or second EPS gene cluster.

According to some embodiments, the endogenous polypeptide or polypeptides involved in the production of exopolysaccharides (EPS) in said bacterium is/are encoded by a gene or genes included in a first EPS gene cluster and/or second EPS gene cluster.

According to some embodiments, the first EPS gene cluster includes genes defined by or orthologous to the open reading frames (ORFs) found in SEQ ID NO: 174 or 177.

According to some embodiments, the first EPS gene cluster includes the genes *epsD, epsE, epsF, epsG, epsB, epsL, epsH, epsl, epsJ* and *epsS,* or orthologs thereof.

According to some embodiments, the first EPS gene cluster includes the genes *epsD, epsE, epsF* and *epsG,* or orthologs thereof.

According to some embodiments, the first EPS gene cluster includes a nucleotide sequence which has at least 50%, such as at least 55%, sequence identity with the nucleotide sequence of SEQ ID NO: 174 or 177.

According to some embodiments, the first EPS gene cluster includes a nucleotide sequence which has at least 60%, such as at least 65%, sequence identity with the nucleotide sequence of SEQ ID NO: 174 or 177.

According to some embodiments, the first EPS gene cluster includes a nucleotide sequence which has at least 70%, such as at least 75%, sequence identity with the nucleotide sequence of SEQ ID NO: 174 or 177.

According to some embodiments, the first EPS gene cluster includes a nucleotide sequence which has at least 80%, such as at least 85%, sequence identity with the nucleotide sequence of SEQ ID NO: 174 or 177.

According to some embodiments, the first EPS gene cluster includes a nucleotide sequence which has at least 90%, such as at least 93%, sequence identity with the nucleotide sequence of SEQ ID NO: 174 or 177.

According to some embodiments, the first EPS gene cluster includes a nucleotide sequence which has at least 95%, such as at least 97%, sequence identity with the nucleotide sequence of SEQ ID NO: 174 or 177.

According to some embodiments, the first EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of SEQ ID NO: 174.

According to some embodiments, the first EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of SEQ ID NO: 177.

According to some embodiments, the second EPS gene cluster includes genes defined by or orthologous to the open reading frames (ORFs) found in SEQ ID NO: 175 or 179.

According to some embodiments, the second EPS gene cluster includes a nucleotide sequence which has at least 50%, such as at least 55%, sequence identity with the nucleotide sequence of 175 or 179.

According to some embodiments, the second EPS gene cluster includes a nucleotide sequence which has at least 60%, such as at least 65%, sequence identity with the nucleotide sequence of 175 or 179.

According to some embodiments, the second EPS gene cluster includes a nucleotide sequence which has at least 70%, such as at least 75%, sequence identity with the nucleotide sequence of 175 or 179.

According to some embodiments, the second EPS gene cluster includes a nucleotide sequence which has at least 80%, such as at least 85%, sequence identity with the nucleotide sequence of 175 or 179.

According to some embodiments, the second EPS gene cluster includes a nucleotide sequence which has at least 90%, such as at least 85%, sequence identity with the nucleotide sequence of 175 or 179.

According to some embodiments, the second EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of 175.

According to some embodiments, the second EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of 179.

According to some embodiments, at least one gene in said first EPS gene cluster is inactivated, e.g., by deletion of part of or the entire gene sequence.

According to some embodiments, the at least one gene is selected from *epsD, epsE, epsF, epsG*, *epsB, epsL, epsH, epsI, epsJ* and *epsS,* or ortholog thereof.

According to some embodiments, the at least one gene is selected from *epsD, epsE, epsF* and *epsG,* or ortholog thereof.

According to some embodiments, the genes *epsD, epsE, epsF* and *epsG,* or orthologs thereof, are inactivated, e.g., by deletion of part of or the entire gene sequence.

According to some embodiments, the gene *epsD* encodes a polypeptide comprising an amino acid sequence having at least 70%, such as at least 75%, sequence identity to SEQ ID NO: 6, the gene *epsE* encodes a polypeptide comprising an amino acid sequence having at least 70%, such as at least 75%, sequence identity to SEQ ID NO: 7, the gene *epsF* encodes a polypeptide comprising an amino acid sequence having at least 70%, such as at least 75%, sequence identity to SEQ ID NO: 8, and the gene *epsG* encodes a polypeptide comprising an amino acid sequence having at least 70%, such as at least 75%, sequence identity to SEQ ID NO: 9.

According to some embodiments, the gene *epsD* encodes a polypeptide comprising an amino acid sequence having at least 80%, such as at least 85%, sequence identity to SEQ ID NO: 6, the gene *epsE* encodes a polypeptide comprising an amino acid sequence having at least 80%, such as at least 85%, sequence identity to SEQ ID NO: 7, the gene *epsF* encodes a polypeptide comprising an amino acid sequence having at least 80%, such as at least 85, sequence identity to SEQ ID NO: 8, and the gene *epsG* encodes a polypeptide comprising an amino acid sequence having at least 80%, such as at least 85%, sequence identity to SEQ ID NO: 9.

According to some embodiments, the gene *epsD* encodes a polypeptide comprising an amino acid sequence having at least 90%, such as at least 95%, sequence identity to SEQ ID NO: 6, the gene *epsE* encodes a polypeptide comprising an amino acid sequence having at least 90%, such as at least 95%, sequence identity to SEQ ID NO: 7, the gene *epsF* encodes a polypeptide comprising an amino acid sequence having at least 90%, such as at least 95, sequence identity to SEQ ID NO: 8, and the gene *epsG* encodes a polypeptide comprising an amino acid sequence having at least 90%, such as at least 95%, sequence identity to SEQ ID NO: 9.

According to some embodiments, the *epsD* gene comprises a nucleotide sequence having at least 70%, such as at least 75%, sequence identity with SEQ ID NO: 92, the gene *epsE* comprises a nucleotide sequence having at least 70%, such as at least 75%, sequence identity with SEQ ID NO: 93, the gene *epsF* comprises an nucleotide sequence having at least 70%, such as at least 75% sequence identity with SEQ ID NO: 94, and the gene *epsG* comprises an nucleotide sequence having at least 70%, such as at least 75%, sequence identity with SEQ ID NO: 95.

According to some embodiments, the *epsD* gene comprises a nucleotide sequence having at least 80%, such as at least 85%, sequence identity with SEQ ID NO: 92, the gene *epsE* comprises a nucleotide sequence having at least 80%, such as at least 85%, sequence identity with SEQ ID NO: 93, the gene *epsF* comprises an nucleotide sequence having at least 80%, such as at least 85% sequence identity with SEQ ID NO: 94, and the gene *epsG* comprises an nucleotide sequence having at least 80%, such as at least 85%, sequence identity with SEQ ID NO: 95.

According to some embodiments, the *epsD* gene comprises a nucleotide sequence having at least 90%, such as at least 95%, sequence identity with SEQ ID NO: 92, the gene *epsE* comprises a nucleotide sequence having at least 90%, such as at least 95%, sequence identity with SEQ ID NO: 93, the gene *epsF* comprises an nucleotide sequence having at least 90%, such as at least 95% sequence identity with SEQ ID NO: 94, and the gene *epsG* comprises an nucleotide sequence having at least 90%, such as at least 95% sequence identity with SEQ ID NO: 95.

According to some embodiments, the gene *epsD* encodes a polypeptide comprising an amino acid sequence having at least 70%, such as at least 75%, sequence identity to SEQ ID NO: 42, the gene *epsE* encodes a polypeptide comprising an amino acid sequence having at least 70%, such as at least 75%, sequence identity to SEQ ID NO: 43, the gene epsFencodes a polypeptide comprising an amino acid sequence having at least 70%, such as at least 75%, sequence identity to SEQ ID NO: 44, and the gene *epsG* encodes a polypeptide comprising an amino acid sequence having at least 70%, such as at least 75%, sequence identity to SEQ ID NO: 45.

According to some embodiments, the gene *epsD* encodes a polypeptide comprising an amino acid sequence having at least 80%, such as at least 85%, sequence identity to SEQ ID NO: 42, the gene *epsE* encodes a polypeptide comprising an amino acid sequence having at least 80%, such as at least 85%, sequence identity to SEQ ID NO: 43, the gene e*psF* encodes a polypeptide comprising an amino acid sequence having at least 80%, such as at least 85%, sequence identity to SEQ ID NO: 44, and the gene *epsG* encodes a polypeptide comprising an amino acid sequence having at least 80%, such as at least 85%, sequence identity to SEQ ID NO: 45.

According to some embodiments, the gene *epsD* encodes a polypeptide comprising an amino acid sequence having at least 90%, such as at least 95%, sequence identity to SEQ ID NO: 42, the gene *epsE* encodes a polypeptide comprising an amino acid sequence having at least 90%, such as at least 95%, sequence identity to SEQ ID NO: 43, the gene epsFencodes a polypeptide comprising an amino acid sequence having at least 90%, such as at least 95%, sequence identity to SEQ ID NO: 44, and the gene *epsG* encodes a polypeptide comprising an amino acid sequence having at least 90%, such as at least 95%, sequence identity to SEQ ID NO: 45.

According to some embodiments, the gene *epsD* comprises a nucleotide sequence having at least 70%, such as at least 75%, sequence identity with SEQ ID NO: 128, the gene *epsE* comprises a nucleotide sequence having at least 70%, such as at least 75%, sequence identity with SEQ ID NO: 129, the gene *epsF* comprises an nucleotide sequence having at least 70%, such as at least 75%, sequence identity with SEQ ID NO: 130, and the gene *epsG* comprises an nucleotide sequence having at least 70%, such as at least 75%, sequence identity with SEQ ID NO: 131.

According to some embodiments, the gene *epsD* comprises a nucleotide sequence having at least 80%, such as at least 85%, sequence identity with SEQ ID NO: 128, the gene *epsE* comprises a nucleotide sequence having at least 80%, such as at least 85%, sequence identity with SEQ ID NO: 129, the gene *epsF* comprises an nucleotide sequence having at least 80%, such as at least 85%, sequence identity with SEQ ID NO: 130, and the gene *epsG* comprises an nucleotide sequence having at least 80%, such as at least 85%, sequence identity with SEQ ID NO: 131.

According to some embodiments, the gene *epsD* comprises a nucleotide sequence having at least 90%, such as at least 95%, sequence identity with SEQ ID NO: 128, the gene *epsE* comprises a nucleotide sequence having at least 90%, such as at least 95%, sequence identity with SEQ ID NO: 129, the gene *epsF* comprises an nucleotide sequence having at least 90%, such as at least 95%, sequence identity with SEQ ID NO: 130, and the gene *epsG* comprises an nucleotide sequence having at least 90%, such as at least 95%, sequence identity with SEQ ID NO: 131.

According to some embodiments, the genetically engineered bacterium of the invention has been modified to inactivate the first EPS gene cluster.

According to some embodiments, the genetically engineered bacterium of the invention has been (further) modified to inactivate the second EPS gene cluster.

According to some embodiments, the genetically engineered bacterium of the invention has been modified to inactivate the first EPS gene cluster and second EPS gene cluster.

According to some embodiments, the first EPS gene cluster is inactivated by deletion of part of or the entire sequence of said cluster.

According to some embodiments, the first EPS gene cluster is inactivated by deletion of the entire coding sequence of said cluster.

According to some embodiments, the first EPS gene cluster is inactivated by deletion of the entire sequence of said cluster.

According to some embodiments, the first EPS gene cluster is inactivated by modification of (e.g., by introducing at least one mutation in) the promoter and/or ribosome binding site region resulting in the lack of gene expression.

According to some embodiments, the second EPS gene cluster is inactivated by deletion of part of or the entire sequence of said cluster.

According to some embodiments, the second EPS gene cluster is inactivated by deletion of the entire coding sequence of said cluster.

According to some embodiments, the second EPS gene cluster is inactivated by deletion of the entire sequence of said cluster.

According to some embodiments, the second EPS gene cluster is inactivated by modification of (e.g., by introducing at least one mutation in) the promoter and/or ribosome binding site region resulting in the lack of gene expression.

According to some embodiments, the genetically engineered bacterium of the invention has been modified to delete a nucleotide sequence having at least 70%, such as at least 75%, sequence identity with SEQ ID NO: 181 or 183. According to some embodiments, the genetically engineered bacterium of the invention has been modified to delete a nucleotide sequence having at least 80%, such as at least 85%, sequence identity with SEQ ID NO: 181 or 183. According to some embodiments, the genetically engineered bacterium of the invention has been modified to delete a nucleotide sequence having at least 90%, such as at least 95%, sequence identity with SEQ ID NO: 181 or 183.

According to some embodiments, the genetically engineered bacterium of the invention has been modified to delete a nucleotide sequence having at least 70%, such as at least 75%, sequence identity with SEQ ID NO: 182 or 184. According to some embodiments, the genetically engineered bacterium of the invention has been modified to delete a nucleotide sequence having at least 80%, such as at least 85%, sequence identity with SEQ ID NO: 182 or 184. According to some embodiments, the genetically engineered bacterium of the invention has been modified to delete a nucleotide sequence having at least 90%, such as at least 95%, sequence identity with SEQ ID NO: 182 or 184.

According to some embodiments, the genetically engineered bacterium of the invention has been modified to delete a nucleotide sequence having at least 70%, such as at least 75%, sequence identity with SEQ ID NO: 181 and to delete a nucleotide sequence having at least 70%, such as at least 75%, sequence identity with SEQ ID NO: 182. According to some embodiments, the genetically engineered bacterium of the invention has been modified to delete a nucleotide sequence having at least 80%, such as at least 85%, sequence identity with SEQ ID NO: 181 and to delete a nucleotide sequence having at least 80%, such as at least 85%, sequence identity with SEQ ID NO: 182. According to some embodiments, the genetically engineered bacterium of the invention has been modified to delete a nucleotide sequence having at least 90%, such as at least 95%, sequence identity with SEQ ID NO: 181 and to delete a nucleotide sequence having at least 90%, such as at least 95%, sequence identity with SEQ ID NO: 182.

According to some embodiments, the genetically engineered bacterium of the invention has been modified to delete a nucleotide sequence having at least 70%, such as at least 75%, sequence identity with SEQ ID NO: 183 and to delete a nucleotide sequence having at least 70%, such as at least 75%, sequence identity with SEQ ID NO: 184. According to some embodiments, the genetically engineered bacterium of the invention has been modified to delete a nucleotide sequence having at least 80%, such as at least 85%, sequence identity with SEQ ID NO: 183 and to delete a nucleotide sequence having at least 80%, such as at least 85%, sequence identity with SEQ ID NO: 184. According to some embodiments, the genetically engineered bacterium of the invention has been modified to delete a nucleotide sequence having at least 90%, such as at least 95%, sequence identity with SEQ ID NO: 183 and to delete a nucleotide sequence having at least 90%, such as at least 95%, sequence identity with SEQ ID NO: 184.

According to some embodiments, the genetically engineered bacterium of the invention has been modified to delete all endogenous genes encoding polypeptides involved in the production of exopolysaccharides (EPS) in said bacterium.

A bacterium in accordance with the present invention can be produced from any suitable bacterium of the genus *Methylobacillus. Methylobacillus* is a genus of Gram-negative methylotrophic bacteria.

According to some embodiments, the bacterium of the present invention is selected from *Methylobacillus flagellatus, Methylobacillus glycogenes, Methylobacillus pratensis and Methylobacillus rhizosphaerae.*

According to some embodiments, the bacterium of the present invention is selected from *Methylobacillus flagellates* and *Methylobacillus glycogenes.*

According to some embodiments, the bacterium of the present invention is *Methylobacillus flagellatus.*

According to some embodiments, the bacterium of the present invention is *Methylobacillus glycogenes.*

### Method of the invention

The present invention also provides methods for producing a biochemical compound comprising cultivating a bacterium according to the invention under suitable culture conditions in a culture medium comprising a reduced one-carbon compound, such as methanol, or a multi-carbon compound that contain no carbon-carbon bonds, such as dimethylamine.

The culture medium employed may be any conventional medium suitable for culturing a bacterium cell in question, and may be composed according to the principles of the prior art. The medium will usually contain all nutrients necessary for the growth and survival of the respective bacterium, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as E. coli cells, including minimal media and rich media such as Luria Broth (LB), M9 media, M17 media, SA media, MOPS media, Terrific Broth, YT and others.

The carbon source may be any suitable carbon substrate known in the art, and in particularly any carbon substrate commonly used in the cultivation of methylotrophic bacteria and/or fermentation. A carbon source of particular interest is a reduced one-carbon compound, such as methanol or methylamine, or a multi-carbon compound that contains no carbon-carbon bonds, such as dimethylamine. Thus, according to some embodiments, the culture medium comprises methanol as a carbon source. The concentration of methanol in the culture medium may generally be in the range from about 0,5% w/v to about 4 % w/v, such as from about 2% w/v to about 4 % w/v. According to some embodiments, the concentration of methanol in the culture medium is in the range from about 2,5% w/v to about 3,5% w/v.

As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used.

The cultivation can be preferably performed under aerobic conditions, such as by a shaking culture, by a stirring culture or in a bioreactor with aeration, at a temperature of about 20 to about 45 °C, such as about 30 to 38 °C, preferably at about 37°C. The pH of the culture is usually above 5, such as in a range from about 6 to about 8, preferably from about 6.5 to about 7.5, more preferably from about 6.8 to about 7.2. The pH of the culture can be adjusted with ammonia, calcium carbonate, various acids, various bases, and buffers. The cultivation may be carried out for a period in the range from 10 to 70 h, preferably in a range from 24 to 60 h, more preferably in a range from 36 to 50 h.

After cultivation, solids such as cells can be removed from the culture medium by centrifugation or membrane filtration. The biochemical compound can be collected by conventional method for isolation and purification chemical compounds from a medium. Well-known purification procedures include, but are not limited to, centrifugation or filtration, precipitation, ion exchange, chromatographic methods such as e.g. ion exchange chromatography or gel filtration chromatography, and crystallization methods. The method may further comprise collecting the biochemical compound from the culture medium.

The biochemical compound produced by the method of the present invention may be any desired compound obtained through the cultivation of a bacterium of the present invention. Non-limiting examples include organic acids, amino acids, fatty acids and derivatives thereof. Non-limiting examples of organic acids include fumarate, glycolate, succinate, malate, malonate, lactates and derivatives thereof. Non-limiting examples of amino acids include glutamate, lysine, methionine, tryptophan, phenylalanine and derivatives thereof.

The present invention thus provides a biochemical compound obtainable by a method as detailed herein.

### Certain other definitions

"Polypeptide" and "protein" are used interchangeably herein to denote a polymer of at least two amino acids covalently linked by an amide bond, regardless of length or post- translational modification (e.g., glycosylation, phosphorylation, lipidation, myristilation, ubiquitination, etc.). Included within this definition are D- and L-amino acids, and mixtures of D- and L-amino acids.

"Nucleic acid" or "polynucleotide" are used interchangeably herein to denote a polymer of at least two nucleic acid monomer units or bases (e.g., adenine, cytosine, guanine, thymine) covalently linked by a phosphodiester bond, regardless of length or base modification.

"Recombinant" or "non-naturally occurring" when used with reference to, e.g., a host cell, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant bacterial cells expressing genes that are not found within the native (non-recombinant) form of the cell or express native genes that are otherwise expressed at a different level.

"Heterologous" or "exogenous" as used herein in the context of a gene or nucleic acid molecule refer to a gene or nucleic acid molecule (i.e. DNA or RNA molecule) that does not occur naturally as part of the genome of the bacterium in which it is present or which is found in a location or locations in the genome that differ from that in which it occurs in nature. Thus, a "heterologous" or "exogenous" gene or nucleic acid molecule is not endogenous to the bacterium and has been exogenously introduced into the microorganism. A "heterologous" gene or nucleic acid molecule DNA molecule may be from a different organism, a different species, a different genus or a different kingdom, as the host DNA.

"Heterologous" as used herein in the context of a polypeptide means that a polypeptide is normally not found in or made (i.e. expressed) by the host microorganism, but derived from a different organism, a different species, a different genus or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

By "decreased expression" of a gene it is meant that the amount of the transcription product, respectively the amount of the polypeptide (e.g., enzyme) encoded by said gene produced by the modified bacterium is decreased compared to an otherwise identical bacterium that does not carry said modification. More particularly, by "decreased expression" of a gene it is meant that the amount of the transcription product, respectively the amount of the polypeptide (e.g., enzyme) encoded by said gene produced by the modified bacterium is decreased by at least 10%, such as at least 20%, at least 30%, at least 40%, at least 50% at least 60%, at least 70%, at least 80%, at least 90% or at least 100%, compared to an otherwise identical bacterium that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry or Western Blotting and the like.

Expression of a gene can be decreased by introducing a mutation into the gene in the genome of the bacterium so that the intracellular activity of the polypeptide encoded by the gene is decreased as compared to an otherwise identical bacterium that does not carry said mutation. Mutations which result in a decreased expression of the gene include the replacement of one nucleotide or more to cause an amino acid substitution in the polypeptide encoded by the gene (missense mutation), introduction of a stop codon (nonsense mutation), deletion or insertion of nucleotides to cause a frame shift, insertion of a drug-resistance gene, or deletion of a part of the gene or the entire gene (Qiu and Goodman, 1997; Kwon et al., 2000). Expression can also be decreased by modifying an expression regulating sequence such as the promoter, the Shine-Dalgarno (SD) sequence, etc. Expression of the gene can also be decreased by gene replacement (Datsenko and Wanner, 2000), such as the "lambda-red mediated gene replacement". The lambda-red mediated gene replacement is a particularly suitable method to inactive one or more genes as described herein.

"Inactivating", "inactivation" and "inactivated", when used in the context of a gene or gene cluster, means that the gene or gene cluster in question no longer expresses a functional protein. It is possible that the modified DNA region is unable to naturally express the gene or gene cluster due to the deletion of a part of or the entire sequence of the gene or gene cluster, the shifting of the reading frame of the gene or gene cluster, the introduction of missense/nonsense mutation(s), or the modification of the regulatory region of the gene or gene cluster, including sequences controlling gene expression, such as a promoter, enhancer, attenuator, ribosome- binding site, etc. Preferably, a gene or gene cluster of interest is inactivated by deletion of a part of or the entire sequence of the gene or gene cluster, such as by gene replacement. Inactivation may also be accomplished by introducing or expressing a rare-cutting endonuclease able to selectively inactivating by DNA cleavage, preferably by double-strand break, the gene or gene cluster of interest. A "rare-cutting endonuclease" within the context of the present invention includes transcription activator-like effector (TALE) nucleases, meganucleases, zing-finger nucleases (ZFN), and RNA-guided endonucleases.

The presence or absence of a gene or gene cluster in the genome of a bacterium can be detected by well-known methods, including PCR, Southern blotting, and the like. In addition, the level of gene expression can be estimated by measuring the amount of mRNA transcribed from the gene or gene cluster using various well-known methods, including Northern blotting, quantitative RT-PCR, and the like. The amount of the polypeptide encoded by the gene or gene cluster can be measured by well-known methods, including SDS-PAGE followed by an immunoblotting assay (Western blotting analysis), and the like.

As used herein, "decreased", "decreasing" or "decrease of" expression of a polypeptide (such as an enzyme as described herein) means that the expression of said polypeptide in a modified bacterium is reduced compared to the expression of said polypeptide in an otherwise identical bacterium that does not carry said modification (control). The expression of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). More particularly, "decreased", "decreasing" or "decrease of" expression of a polypeptide means that the amount of the polypeptide in the modified bacterium is reduced by at least about 10 %, and preferably by at least about 20%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the amount of said polypeptide in an otherwise identical bacterium that does not carry said modification (control). The expression or amount of a polypeptide in a bacterium can be determined by any suitable means know in the art, including techniques such as ELISA, Immunohistochemistry, Western Blotting or Flow Cytometry.

As used herein, "abolished" expression of a polypeptide (such as an enzyme as described herein) means that the expression of said polypeptide in a modified bacterium is not detectable compared to the expression of said polypeptide in an otherwise identical bacterium that does not carry said modification (control).

As used herein, "decreased", "decreasing" or "decrease of" activity of a polypeptide (such as an enzyme as described herein) means that the catalytic activity of said polypeptide in a modified bacterium is reduced compared to the catalytic activity of said polypeptide in an otherwise identical bacterium that does not carry said modification (control). The activity of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). The activity of a polypeptide in a bacterium can be determined by any suitable protein and enzyme activity assay.

"Expression" includes any step involved in the production of a polypeptide (e.g., encoded enzyme) including, but not limited to, transcription, post-transcriptional modification, translation, post-translational modification, and secretion.

As used herein, "regulatory region" of a gene or gene cluster refers to a nucleic acid sequence that affect the expression of a coding sequence. Regulatory regions are known in the art and include, but are not limited to, promoters, enhancers, transcription terminators, polyadenylation sites, matrix attachment regions and/or other elements that regulate expression of a coding sequence.

"Substitution" or "substituted" refers to modification of the polypeptide by replacing one amino acid residue with another, for instance the replacement of an Serine residue with a Glycine or Alanine residue in a polypeptide sequence is an amino acid substitution. When used with reference to a polynucleotide, "substitution" or "substituted" refers to modification of the polynucleotide by replacing one nucleotide with another, for instance the replacement of a cytosine with a thymine in a polynucleotide sequence is a nucleotide substitution.

"Conservative substitution", when used with reference to a polypeptide, refers to a substitution of an amino acid residue with a different residue having a similar side chain, and thus typically involves substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

As used herein, "vector" refers to a nucleic acid molecule capable of transporting another nucleic acid molecule to which it has been linked. One type of vector is a "plasmid", which refers to a circular double stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a genome of a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

"Percentage of sequence identity," "% sequence identity" and "percent identity" refers to sequence identity between a nucleotide sequence and a reference nucleotide sequence or between an amino acid sequence and a reference amino acid sequence. Sequence identity can be determined by comparing a position in each sequence which may be aligned for purposes of comparison. When a position in the compared sequence is occupied by the same base or amino acid, then the molecules are identical at that position. A degree of identity between nucleotide or amino acid sequences is a function of the number of identical or matching nucleotides or amino acids at positions shared by the nucleotide or amino acid sequences, respectively. Various alignment algorithms and/or programs may be used to calculate the identity between two sequences, including FASTA or BLAST which are available as a part of the GCG sequence analysis package (University of Wisconsin, Madison, Wis.), and can be used with default settings.

As used herein, the term "about" means plus or minus 10% of the numerical value of the number with which it is being used.

Where a numerical limit or range is stated herein, the endpoints are included. Also, all values and sub ranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps or components but do not preclude the addition of one or more additional features, steps, components or groups thereof.

Having generally described this invention, a further understanding can be obtained by reference to certain specific examples, which are provided herein for purposes of illustration only, and are not intended to be limiting unless otherwise specified.

### Examples

### Example 1: Identification of EPS-producing genes in Methylobacillus flagellatus and Methylobacillus glycogenes

Several genomic loci were chosen for disruption based on bioinformatic analysis. The genetic locations of two EPS-associated gene clusters in *Methylobacillus flagellatus* have been published previously (Chistoserdova *et al.,* 2007). To annotate genes in the first *Methylobacillus flagellatus* EPS-producing gene cluster (SEQ ID NOs: 87-112), homologues for genes from the published *Methylobacillus sp.* 12S methanolan synthesis cluster (Yoshida et al., 2000) were compared to the *Methylobacillus flagellatus genome* via an NCBI BLAST protein search, using a custom written script in the Python programming language. Top hits were selected based on a computed combination-score consisting of E-value, query coverage and sequence identity. A single top hit for each gene was then manually located in the genome sequence and annotated. Locations of these top hits coincided with the location of the published first cluster. Since a clear homologue was not found for each gene, all genes between the most distant homologues were considered to be part of the cluster. Most such non-annotated genes coded for sugar and polysaccharide related genes. The same procedure was used to identify the completely non-annotated EPS cluster in *Methylobacillus glycogenes* (SEQ ID NOs: 125-146).

A second EPS-related cluster was predicted in the genome sequence of *Methylobacillus flagellatus* (SEQ ID NOs: 113-124). A BLAST search comparing these genes to the methanolan cluster was performed, finding no clear homologues. The protein product of each individual gene in this cluster was identified via a NCBI protein BLAST search against all non-redundant protein sequences and the top hit for each was chosen as described above. The protein sequence of each gene from this *Methylobacillus flagellatus* protein cluster was cross-searched in the *Methylobacillus glycogenes* genome using NCBI protein BLAST. The top hits were found to form a similar cluster in *Methylobacillus glycogenes* (SEQ ID NOs: 147-172).

The identified open reading frames (ORFs) in each EPS-related gene cluster are shown in Tables 1 to 4 below.

**Table 1: ORFs of EPS gene cluster 1 in M. flagellatus**

| **Amino acid sequence SEQ ID NO** | **Nucleotide sequence SEQ ID NO** | **S12 homolog** | **Predicted function/ property** |
|---|---|---|---|
| 1 | 87 | *espA* | Transcriptional regulator, LuxR family |
| 2 | 88 | *espK* | Cyclic nucleotide-binding domain (cNMP-BD)protein |
| 3 | 89 | N/A | Unknown |
| 4 | 90 | *epsL* | Exosortase B-associated extracellular polysaccharide biosynthesis transporter EpsL |
| 5 | 91 | *epsB* | Transferase enzyme epsB family |
| 6 | 92 | *epsD* | Peptidyl-prolyl cis-trans isomerase, EpsD family |
| 7 | 93 | *epsE* | Polysaccharide export protein EpsE |
| 8 | 94 | *epsF* | Chain length determinant protein EpsF |
| 9 | 95 | *epsG* | Chain length determinant protein tyrosine kinase EpsG |
| 10 | 96 | *epsH* | Exosortase B |
| 11 | 97 | *epsI* | Epsl family protein |
| 12 | 98 | N/A | Polysaccharide biosynthesis protein |
| 13 | 99 | N/A | Polysaccharide pyruvyl transferase |
| 14 | 100 | N/A | Nitroreductase |
| 15 | 101 | N/A | O-antigen polymerase |
| 16 | 102 | *epsJ* | Glycosyl transferase, family 2 |
| 17 | 103 | N/A | Glycosyl transferase, family 2 |
| 18 | 104 | N/A | Glycosyl transferase, group 1 |
| 19 | 105 | N/A | Glycoside hydrolase, family 5 |
| 20 | 106 | N/A | Glycosyltransferase, RfaG |
| 21 | 107 | N/A | Mannose-1-phosphate guanylyltransferase (GDP) |
| 22 | 108 | N/A | dTDP-4-dehydrorhamnose reductase |
| 23 | 109 | N/A | dTDP-4-dehydrorhamnose 3,5-epimerase |
| 24 | 110 | N/A | Glucose-1-phosphate thymidylyltransferase |
| 25 | 111 | N/A | dTDP-glucose 4,6-dehydratase |
| 26 | 112 | *epsS* | UDP-galactose 4-epimerase |

**Table 2: ORFs of EPS gene cluster 2 in M. flagellatus**

| **Amino acid sequence SEQ ID NO** | **Nucleotide sequence SEQ ID NO** | **S12 homolog** | **Predicted function/ property** |
|---|---|---|---|
| 27 | 113 | N/A | Mannose-6-phosphate isomerase, type 2 |
| 28 | 114 | N/A | ABC transporter permease |
| 29 | 115 | N/A | NDP-hexose 3-C-methyltransferase TylCIII |
| 30 | 116 | N/A | GDP-mannose 4,6-dehydratase |
| 31 | 117 | N/A | NAD-dependent epimerase/dehydratase |
| 32 | 118 | N/A | Glycosyl transferase |
| 33 | 119 | N/A | Polysaccharide export protein |
| 34 | 120 | N/A | Glycosyl transferase |
| 35 | 121 | N/A | NAD-dependent epimerase/dehydratase |
| 36 | 122 | N/A | Sugar transferase |
| 37 | 123 | N/A | Polysaccharide biosynthesis protein CapD |
| 38 | 124 | N/A | UDP-glucose pyrophosphorylase |

**Table 3: ORFs of EPS gene cluster 2 in M. glycogenes**

| **Amino acid sequence SEQ ID NO** | **Nucleotide sequence SEQ ID NO** | **S12 homolog** | **Predicted function/ property** |
|---|---|---|---|
| 39 | 125 | *epsA* | Transcriptional regulator, LuxR family |
| 40 | 126 | *epsL* | Exosortase B-associated extracellular polysaccharide biosynthesis transporter EpsL |
| 41 | 127 | N/A | U ndeca prenyl-phosphate gl ucosephosphotra nsferase |
| 42 | 128 | *epsD* | Peptidyl-prolyl cis-trans isomerase, EpsD family |
| 43 | 129 | *epsE* | Polysaccharide export protein EpsE |
| 44 | 130 | *epsF* | Chain length determinant protein EpsF |
| 45 | 131 | *epsG* | Chain length determinant protein tyrosine kinase EpsG |
| 46 | 132 | *epsH* | Exosortase B |
| 47 | 133 | *epsI* | Epsl family protein |
| 48 | 134 | *epsB* | Glycosyltransferase |
| 49 | 135 | N/A | Oligosaccharide repeat unit polymerase |
| 50 | 136 | N/A | Right-handed parallel beta-helix repeat-containing protein |
| 51 | 137 | N/A | Acyltransferase family protein |
| 52 | 138 | N/A | Glycosyltransferase family 4 protein |
| 53 | 139 | N/A | Glycosyltransferase family 4 protein |
| 54 | 140 | N/A | Nucleotide sugar dehydrogenase |
| 55 | 141 | *epsR* | Unknown |
| 56 | 142 | N/A | Unknown |
| 57 | 143 | N/A | Oligosaccharide flippase family protein |
| 58 | 144 | N/A | Polysaccharide pyruvyl transferase family protein |
| 59 | 145 | N/A | Acyltransferase |
| 60 | 146 | *epsQ* | mannose-1-phosphate guanylyltransferase/mannose-6-phosphate isomerase |

**Table 4: ORFs of EPS gene cluster 2 in M. glycogenes**

| **Amino acid sequence SEQ ID NO** | **Nucleotide sequence SEQ ID NO** | **S12 homolog** | **Predicted function/ property** |
|---|---|---|---|
| 61 | 147 | N/A | MarR family EPS-associated transcriptional regulator |
| 62 | 148 | N/A | GDP-mannose 4,6-dehydratase |
| 63 | 149 | N/A | NAD-dependent epimerase/dehydratase family protein |
| 64 | 150 | N/A | Unknown |
| 65 | 151 | N/A | Polyhydroxyalkanoate synthesis repressor PhaR |
| 66 | 152 | N/A | UDP-N-acetylglucosamine 2-epimerase (hydrolyzing) neuC |
| 67 | 153 | N/A | Imidazole glycerol phosphate synthase subunit HisF |
| 68 | 154 | N/A | Imidazole glycerol phosphate synthase subunit HisH |
| 69 | 155 | N/A | N-acetyl sugar amidotransferase |
| 70 | 156 | N/A | Polysaccharide pyruvyl transferase family protein |
| 71 | 157 | N/A | Acyltransferase |
| 72 | 158 | N/A | Unknown |
| 73 | 159 | N/A | ABC transporter ATP-binding protein |
| 74 | 160 | N/A | FkbM family methyltransferase |
| 75 | 161 | N/A | Glycosyltransferase |
| 76 | 162 | N/A | Methyltransferase, TIGR04325 family |
| 77 | 163 | N/A | NAD-dependent epimerase/dehydratase family protein |
| 78 | 164 | N/A | DegT/DnrJ/EryC1/StrS family aminotransferase |
| 79 | 165 | N/A | Glycosyltransferase |
| 80 | 166 | *epsJ* | Glycosyltransferase |
| 81 | 167 | N/A | Glycosyltransferase |
| 82 | 168 | N/A | Glycosyltransferase |
| 83 | 169 | N/A | SDR family oxidoreductase |
| 84 | 170 | N/A | Sugar transferase |
| 85 | 171 | N/A | SDR family NAD(P)-dependent oxidoreductase |
| 86 | 172 | *epsT* | UTP--glucose-1-phosphate uridylyltransferase GalU |

**Table 5: Full sequences of clusters**

| **SEQ ID NO** | **Organism** | **Cluster** | **Strand** |
|---|---|---|---|
| 173 | *M. flagellatus* | 1 | top |
| 174 | *M. flagellatus* | 1 | bottom |
| 175 | *M. flagellatus* | 2 | top |
| 176 | *M. flagellatus* | 2 | bottom |
| 177 | M. glycogenes | 1 | top |
| 178 | M. glycogenes | 1 | bottom |
| 179 | M. glycogenes | 2 | top |
| 180 | M. glycogenes | 2 | bottom |

ORFs annotated as *epsD, epsE, epsF* and *epsG* (SEQ ID NO: 6-9 in M. *flagellatus,* SEQ ID NO: 42-45 in *M*. *glycogenes*) were chosen for deletion, based on their similarity to EPS-production essential genes in *Methylobacillus sp.* strain 12 (Yoshida *et al.,* 2003). Since the second EPS cluster had no clear homologues in other characterized EPS clusters, all 12 ORFs in *M. flagellatus* (SEQ ID NO: 27-38) and all 26 ORFs in *Methylobacillus glycogenes* (SEQ ID NO: 61-86) were chosen for deletion.

### Example 2: DNA deletions

Modified strains of ABME 5 and ABME 6 were preparing in which either one EPS-producing cluster or both EPS-producing clusters are deleted. The genome edits were achieved by introducing a linear DNA fragment consisting a kanamycin or rifampicin resistance cassette flanked by 2 kb long sequences upstream and downstream of the integration sites. DNA fragments were transformed into ABME 5 and ABMR 6 by electroporation. Transformations were plated onto selective PM7 plates containing 20 g/L agar, 50 µg/mL kanamycin, or 5 µg/mL rifampicin for 24-48 h. ABME 5-derived strains were incubated at 30°C. ABME 6-derived strains were cultivated at 37°C. Correct clones were confirmed by colony PCR. The strains prepared by DNA deletion are listed in Table 6.

**Table 6: List of strains prepared with genomic integration.**

| **Strain** | **Species** | **Genotype** | **Deleted sequences** |
|---|---|---|---|
| ABME 5 | *Methylobacillus glycogenes* DSM 5685 | Wild type | |
| ABME 76 | *Methylobacillus glycogenes* DSM 5685 | epsDEFG :: kanR | SEQ ID NO: 181 |
| ABME 190TH | *Methylobacillus glycogenes* DSM 5685 | EPS2 :: kanR | SEQ ID NO: 182 |
| ABME 191TH | *Methylobacillus glycogenes* DSM 5685 | epsDEFG :: kanR, EPS2 :: rifR | SEQ ID NO: 181 and SEQ ID NO: 182 |
| ABME 6 | *Methylobacillus flagellatus* DSM 6875 | Wild type | |
| ABME 80 | *Methylobacillus flagellatus* DSM 6875 | EPS2 :: kanR | SEQ ID NO: 184 |
| ABME 82 | *Methylobacillus flagellatus* DSM 6875 | epsDEFG :: kanR | SEQ ID NO: 183 |
| ABME 131 | *Methylobacillus flagellatus* DSM 6875 | epsDEFG :: kanR, EPS2 :: rifR | SEQ ID NO: 183 and SEQ ID NO: 184 |
| ABME 145 | *Methylobacillus sp.* DSM 1758 | Wild type | |

### Example 3: Measurements of total EPS in shake flask cultures before and after EPS gene disruption in Methylobacillus flagellatus

To evaluate the effects of deleting EPS-producing genes in ABME 6, three modified ABME 6 strains (ABME 80, ABME 82 and ABME 131) were prepared as described in Example 3 and tested for EPS production.

Confirmed transformants were grown overnight on PM7 + 50 µg/mL kanamycin agar plates. Single colonies were resuspended in 25 mL PM7 medium + 25 µg/mL kanamycin in baffled 250 mL shake flasks, and incubated overnight at 200 rpm. Next day, 50 mL PM7 medium + 25 µg/mL kanamycin in baffled 250 mL shake flasks was inoculated with 2.5 mL overnight culture. All ABME 5-derived strains were cultivated at 30°C, and all ABME 6-derived strains were cultivated at 37°C. The cultures were then chilled on ice, aliquoted into 2 mL Eppendorf tubes and centrifuged for 15 minutes at 15 000 rpm on a standard tabletop centrifuge at 4 °C. When a layer of EPS was visible above the cell pellet, it was mixed back into the supernatant. The culture supernatants were stored for EPS hydrolysis. Hydrolysis was performed by adding half the volume of the supernatant of 6M TFA (trifluoroacetic acid), resulting in a final TFA concentration of 3M. Tubes were placed into a thermoblock heated to 120 °C and incubated for 6h. After hydrolysis, the liquid (including TFA) was evaporated at 80 °C until completely dry. The dry mass was resuspended in 0.5 M NaOH (at the same volume as the original culture aliquot) and analyzed for monomeric sugars on an HPLC.

**Table 7: Composition of PM7 medium used for shake-flask experiments**

| **Compound** | **Per 1L** |
|---|---|
| MeOH | 10 g |
| KH2PO4 | 0.65 g |
| NA2HPO4 | 1.05 g |
| MgSO4 ^{∗} 7H2O | 0.1 g |
| NH4Cl | 0.2 g |
| (NH4)2SO4 | 2g |
| TE1 1000X | 1 mL |
| d-Biotin | 0.1 mg |
| Thiamine^{∗}HCl | 0.1 mg |
| Riboflavin | 0.1 mg |
| Pyridoxine^{∗}HCl | 0.1 mg |
| Pantothenate | 0.1 mg |
| Nicotinamide | 0.1 mg |
| p-Aminobenzoic acid | 0.02 mg |
| Folic acid | 0.01 mg |
| Vitamin B12 | 0.01 mg |
| Lipoic acid | 0.01 mg |
| CaCl2 ^{∗} 2H2O | 3.3 mg |
| FeSO4 ^{∗} 7H2O | 1.3 mg |
| MnSO4 ^{∗} H2O | 0.1 mg |
| ZnSO4 ^{∗} 7H2O | 0.13 mg |
| CuSO4 ^{∗} 5H2O | 0.04 mg |
| Na2MoO4 ^{∗} 2H2O | 0.04 mg |
| CoCl2 ^{∗} 6H2O | 0.04 mg |
| H3BO3 | 0.03 mg |
| pH | 7.0 |
| Agar (for solid media) | 20 g |

ABME 6 produced a visible slime layer on top of the cell pellet after centrifugation, amounting to ^{∼} 1 g/L total monomeric sugars after hydrolysis. The single-cluster disruptions (ABME 80 and ABME 82) had no visible slime layer, but still produced ^{∼} 500 mg/L total sugars. Interestingly, the glucose to total sugars ratio is inverted depending on which gene cluster was disrupted, most likely due to two different types of EPS being produced. When both clusters were disrupted (strain ABME 131), sugars were below the detection limit.

### Example 4: Measurements of viscosity in reactor cultures of Methylobacillus flagellatus before and after EPS gene disruption

To assess the change in fermentation broth viscosity before and after the disruption of EPS production, the strains were compared in 5-liter bioreactor fermentations under oxygen limitation. The bioreactor seed cultures were always prepared as follows. 25 mL of PM7 medium in 250 mL baffled shake-flasks were inoculated with 250 uL of strain cryo-stock with the stock OD adjusted to 5, to produce a starting OD of 0.05. The cultures were incubated overnight at 200 rpm 37°C. In the morning, 200 mL of PM7 medium in 2L baffled shake-flasks was inoculated with 10% (20 mL) of the overnight culture and grown until OD ^{∼}1 (+/- 0.2). The culture was then used to inoculate a 2.5 L starting volume of sterile PM3 medium in 5-L bioreactors (8% inoculum). The pH was kept at 7 by automatic addition of NH₄OH and the temperature was kept at 37 °C.

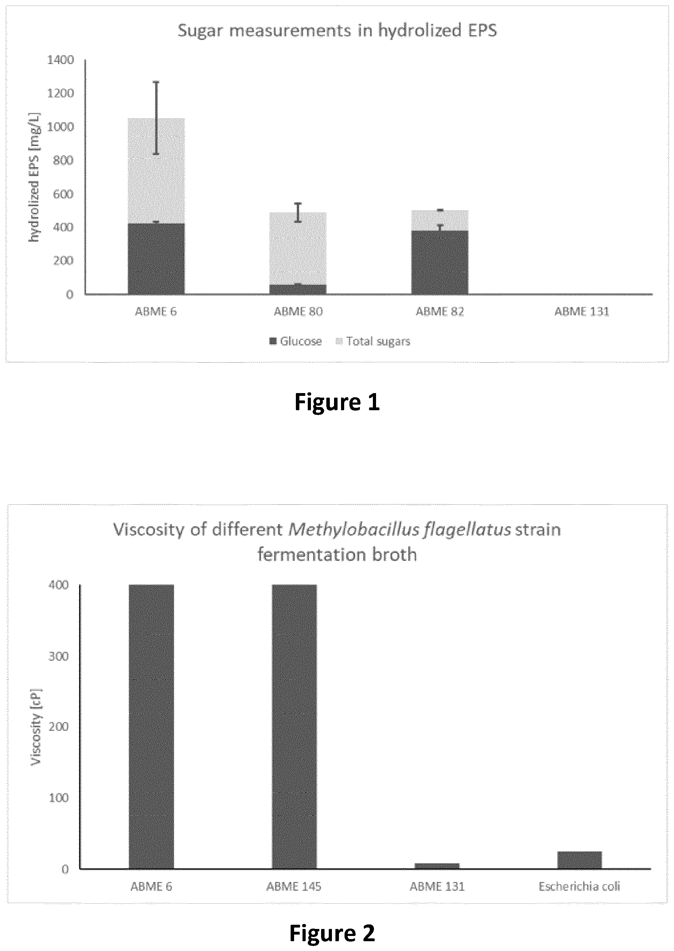

For oxygen limited processes, methanol was fed automatically to maintain a concentration between 3 and 6 g/L. Reactor aeration and stirring were kept at maximum, though the oxygen concentration always dropped to 0% during the process. The broth was sampled regularly for OD, mineral and metabolite measurements. Viscosity was measured at the end of the process and the results are summarized in **Figure 2**. Wild type *M. flagellatus* fermentation broth was highly viscous at the time of sampling at 400 centipoise. A different species of *Methylobacillus*, ABME 145 was measured as well and produced a similarly viscous broth of 422 centipoise. The double disruption produced a visibly less viscous broth, which could be centrifuged and filtered using standard tabletop equipment - something that was not possible with the previous samples. The viscosimeter confirmed this assessment, measuring in at 35 cP or a nearly 10-fold reduction in viscosity. A representative sample of *Escherichia coli* fermentation broth was also measured for comparison.

### Example 5: Measurements of reduced foaming in shake flasks before and after EPS gene elimination in Methylobacillus flagellates

We observed that the EPS-deficient strains showed significantly less foaming. To compare foam formation of ABME 6 against EPS-deficient mutant strains (ABME 82 and ABME 131), the strains were culture as described in Example 3.

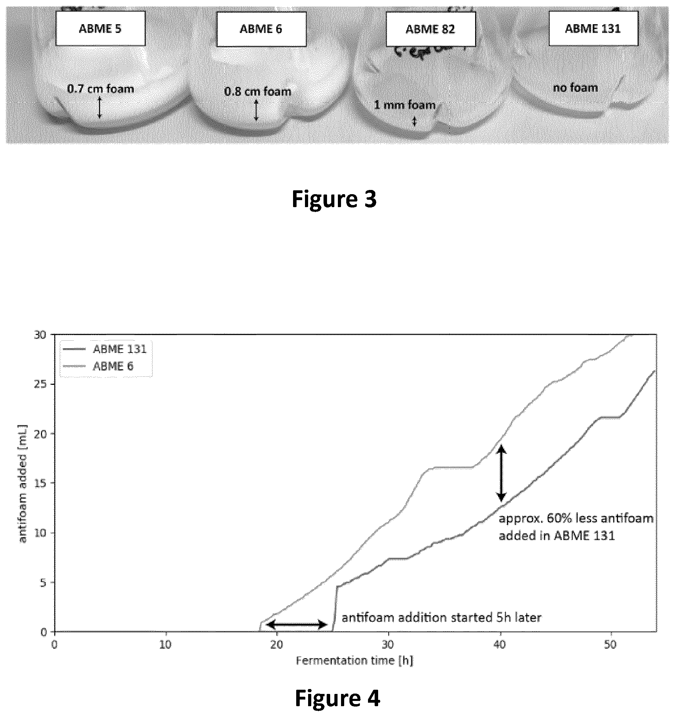

To measure foam formation flasks were taken off the shaker and the liquid was allowed to settle for 5 minutes. The foam layer on top of the liquid was measured with a tape measure (**Figure 3**). Wild type cultures produced 0.5 - 1 cm of foam layer on top of the liquid while the single-cluster disruptions only accumulated ^{∼}1 mm of foam at the liquid meniscus. No foam at all was visible in the double-disruption cultures.

### Example 6: Measurements of reduced foaming in bioreactor cultivation before and after EPS gene disruption in Methylobacillus flagellatus

To assess the change in fermentation broth foaming before and after the disruption of EPS production, the strains were compared in 5-liter bioreactor fermentations. The amount of foam formation was approximated by the amount of added anti-foaming agent (SAG 5693). The fermentations were carried out under carbon limitation.

The reactors were inoculated as described in Example 5. To achieve carbon limitation, 50% methanol was fed to the reactor automatically at a dynamically-calculated rate that that maintained pO2-value at approximately 30% at the highest aeration and stirring settings. The added methanol was consumed immediately and kept below detection levels. Antifoam was added automatically when foam reached a detector placed above the fermentation broth meniscus. ABME 6 and ABME 131 were tested in this way. Antifoam addition started 5h later in the case of ABME 131. Approximately 60% less antifoam was added in the ABME 131 fermentation, depending on the fermentation time (**Figure 4**).

### Example 7: Microscope images of Methylobacillus flagellatus cell aggregates before and after the EPS gene disruption

To assess the change in cell clumping foaming before and after the disruption of EPS production, the strains were cultured in 5-liter bioreactor fermentations under oxygen limitation as described in Example 3.

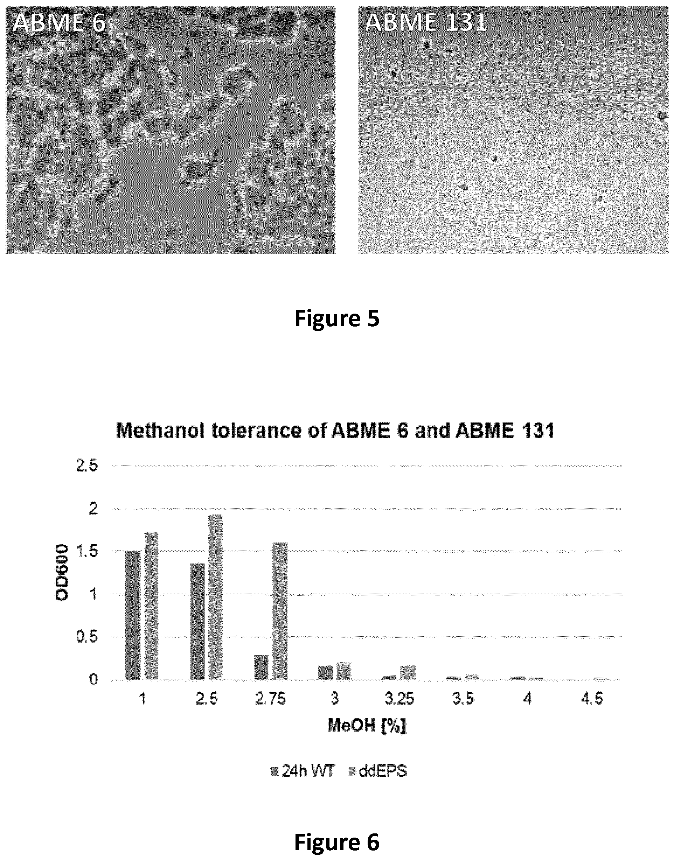

Samples of broth were observed by phase-constrast microscopy on a Zeiss Axioplan 2 microscope through a Zeiss Ph2 Plan-NEOFLUAR 40x objective (NA 0.75) and recorded on a sCMOS camera. Cells of ABME 6 formed large clumps, and were immobilized in the EPS matrix (**Figure 5**). In the case of the double-disruption strain ABME 131, cells floated individually in solution, with minimal clumping.

### Example 8: Improved methanol tolerance of EPS-free M. flagellates

Tolerance to high methanol concentrations is a critical parameter for efficient bioprocesses. To confirm that methanol tolerance was not reduced in strains that cannot produced EPS, growth at increasing methanol concentrations was tested in ABME 6 and its EPS-producing derivative strain ABME 131.

The strains were tested in shake flasks as described in Example 4. To test methanol tolerance, the initial methanol concentration was increased up to 4.5 %. OD600 was measured after 24h.

Surprisingly, the methanol tolerance of ABME 131 was higher compared to ABME 6. For example, at 2.75 % initial methanol concentration, the EPS-free ABME 131 grew to an OD600 of 1.6, whereas the wild-type ABME 6 only grew to an OD600 of 0.3. The difference in growth was most striking at 2.75 %. However improved growth of ABME 131 compared to ABME 6 was apparent at all methanol concentration (**Figure 6**).

### List of references cited in the description

Chistoserdova, L. et al. (2007) 'Genome of Methylobacillus flagellatus, Molecular Basis for Obligate Methylotrophy, and Polyphyletic Origin of Methylotrophy', Journal of Bacteriology. American Society for Microbiology Journals, 189(11): 4020-4027.
Hattori, M. et al. (2020) Methylobacillus glycogenes JCM 2850, whole genome shotgun sequencing p - Nucleotide - NCBI*.*
Yoshida, T. et al. (2000) 'Saccharide production from methanol by transposon 5 mutants derived from the extracellular polysaccharide-producing bacterium Methylobacillus sp. strain 12S', Applied Microbiology and Biotechnology. doi: 10.1007/s002530000407.
Yoshida, T. et al. (2003) 'Genes involved in the synthesis of the exopolysaccharide methanolan by the obligate methylotroph Methylobacillus sp. strain 12S', Microbiology. Microbiology Society, pp. 431-444. doi: 10.1099/mic.0.25913-0.
Qui Z and Goodman MF: The Escherichia coli polB locus is identical to dinA, the structural gene for DNA polymerase II. Characterization of Pol II purified from a polB mutant. J Biol Chem. 1997, 272(13): 8611-8617.
Kwon DH, Peña JA, Osato MS, Fox JG, Graham DY, Versalovic J: Frameshift mutations in rdxA and metronidazole resistance in North American Helicobacter pylori isolates. J Antimicrob Chemother 2000, 46(5): 793-796
Datsenko KA, Wanner BL: One-step inactivation of chromosomal genes in Escherichia coli K-12 using PCR products. Proc Natl Acad Sci U S A 2000, 97:6640-6645.

## Claims

1. A genetically engineered bacterium of the genus *Methylobacillus* which has been modified to have a decreased production of exopolysaccharides (EPS) compared to an otherwise identical bacterium that does not carry said modification.

2. The bacterium according to claim 1, which has been modified to have a decreased expression and/or function of at least one endogenous polypeptide involved in the production of exopolysaccharides (EPS) in said bacterium compared to an otherwise identical bacterium that does not carry said modification.

3. The bacterium according to claim 2, wherein the endogenous polypeptide or polypeptides involved in the production of exopolysaccharides (EPS) in said bacterium is/are encoded by a gene or genes included in a first EPS gene cluster and/or second EPS gene cluster.

4. The bacterium according to claim 3, wherein the first EPS gene cluster includes genes defined by or orthologous to the open reading frames (ORFs) found in SEQ ID NO: 174 or 177.

5. The bacterium according to claim 3 or 4, wherein at least one gene in said first EPS gene cluster is inactivated, e.g., by deletion of part of or the entire gene sequence.

6. The bacterium according to any one of claims 3 to 5, wherein the first EPS gene cluster includes the genes *epsD, epsE, epsF* and *epsG*, or orthologs thereof.

7. The bacterium according to claim 6, wherein at least one gene selected from *epsD, epsE, epsF* and *epsG,* or ortholog thereof, is inactivated, e.g., by deletion of part of or the entire gene sequence.

8. The bacterium according to claim 6 or 7, wherein the genes *epsD, epsE, epsF and epsG,* or orthologs thereof, are inactivated, e.g., by deletion of part of or the entire gene sequence.

9. The bacterium according to any one of claims 6 to 8, wherein the gene *epsD* encodes a polypeptide comprising an amino acid sequence having at least 70% sequence identity to SEQ ID NO: 6, wherein the gene *epsE* encodes a polypeptide comprising an amino acid sequence having at least 70% sequence identity to SEQ ID NO: 7, wherein the gene *epsF* encodes a polypeptide comprising an amino acid sequence having at least 70% sequence identity to SEQ ID NO: 8, and wherein the gene *epsG* encodes a polypeptide comprising an amino acid sequence having at least 70% sequence identity to SEQ ID NO: 9; or wherein the gene *epsD* encodes a polypeptide comprising an amino acid sequence having at least 70% sequence identity to SEQ ID NO: 42, wherein the gene *epsE* encodes a polypeptide comprising an amino acid sequence having at least 70% sequence identity to SEQ ID NO: 43, wherein the gene *epsF* encodes a polypeptide comprising an amino acid sequence having at least 70% sequence identity to SEQ ID NO: 44, and wherein the gene *epsG* encodes a polypeptide comprising an amino acid sequence having at least 70% sequence identity to SEQ ID NO: 45.

10. The bacterium according to any one of claims 3 to 9, wherein the second EPS gene cluster includes genes defined by or orthologous to the open reading frames (ORFs) found in SEQ ID NO: 175 or 179.

11. The bacterium according to any one of claims 3 to 10, wherein the first EPS gene cluster is inactivated.

12. The bacterium according to any one of claims 3 to 11, wherein the second EPS gene cluster is inactivated.

13. The bacterium according to claim 11 or 12, wherein the first EPS gene cluster and/or the second EPS gene cluster is inactivated by deletion of part of or the entire sequence of said cluster or by modification of, e.g., by introducing at least one mutation in, the promoter and/or ribosome binding site region resulting in the lack of gene expression.

14. The bacterium according to any one of claims 1 to 13, wherein the bacterium is selected from *Methylobacillus flagellatus* and *Methylobacillus glycogenes.*

15. A method for the production of a biochemical compound comprising cultivating a bacterium according to any one of claims 1 to 14 under suitable culture conditions in a culture medium comprising a reduced one-carbon compound, such as methanol, or a multi-carbon compound that contains no carbon-carbon bonds, such as dimethylamine.
